(19) Europäisches Patentamt
European Patent Office
Office européen des brevets

(11) **EP 1 724 268 A1**

(12) # EUROPEAN PATENT APPLICATION

published in accordance with Art. 158(3) EPC

(43) Date of publication:
**22.11.2006 Bulletin 2006/47**

(21) Application number: **05719280.9**

(22) Date of filing: **18.02.2005**

(51) Int Cl.:
*C07D 401/14* (2006.01) *C07D 409/14* (2006.01)
*C07D 405/14* (2006.01) *C07D 417/14* (2006.01)
*C07D 471/04* (2006.01) *C07D 495/04* (2006.01)
*C07D 519/00* (2006.01) *C07D 215/22* (2006.01)
*C07D 239/88* (2006.01) *C07D 401/12* (2006.01)
*C07D 405/12* (2006.01) *C07D 413/12* (2006.01)
*A61K 31/4375* (2006.01) *A61K 31/444* (2006.01)
*A61P 1/04* (2006.01) *A61P 1/16* (2006.01)

(86) International application number:
**PCT/JP2005/002610**

(87) International publication number:
**WO 2005/080377 (01.09.2005 Gazette 2005/35)**

(84) Designated Contracting States:
**AT BE BG CH CY CZ DE DK EE ES FI FR GB GR HU IE IS IT LI LT LU MC NL PL PT RO SE SI SK TR**

(30) Priority: **20.02.2004 JP 2004045383**

(71) Applicant: **KIRIN BEER KABUSHIKI KAISHA**
**Chuo-Ku,**
**Tokyo (JP)**

(72) Inventors:
- **SHIMIZU, Kiyoshi;2c/o Pharmaceutical Research Labo**
  **Takasaki-shi, Gunma;3701295 (JP)**
- **SHIMIZU, Toshiyuki;**
  **c/o Pharmaceutical Research La**
  **Takasaki-shi, Gunma;3701295 (JP)**
- **KAWAKAMI, Kazuki;2c/o Pharmaceutical Research Labo**
  **Takasaki-shi, Gunma;3701295 (JP)**
- **NAKOJI, Masayoshi;**
  **c/o Pharmaceutical Research Lab**
  **Takasaki-shi, Gunma;3701295 (JP)**
- **SAKAI, Teruyuki;**
  **c/o Pharmaceutical Research Labor**
  **Takasaki-shi, Gunma;3701295 (JP)**

(74) Representative: **HOFFMANN EITLE**
**Patent- und Rechtsanwälte**
**Arabellastrasse 4**
**81925 München (DE)**

(54) **COMPOUND HAVING TGF-BETA INHIBITORY ACTIVITY AND PHARMACEUTICAL COMPOSITION CONTAINING SAME**

(57) The present invention provides compounds of formula (I) or compounds of formula (II) and pharmaceutically acceptable salts or solvates thereof. An objective of the present invention is to provide compounds having TGFβ inhibitory activity.

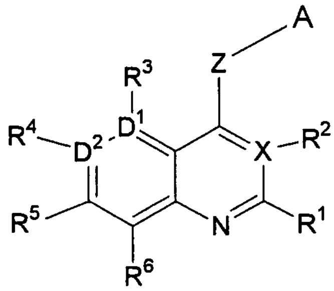
(I)

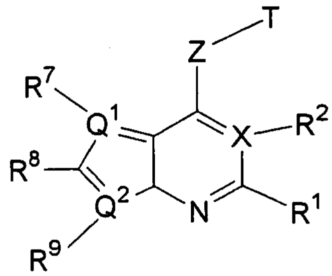
(II)

EP 1 724 268 A1

## Description

BACKGROUND OF THE INVENTION

Field of Invention

The present invention relates to compounds having TGFβ inhibitory activity. The present invention also relates to a pharmaceutical composition useful for the prevention or treatment of diseases for which TGFβ inhibition is effective therapeutically.

Background Art

TGFβ (transforming growth factor-β) is an important cytokine for living organisms, which regulates growth and differentiation of cells, and repair and regeneration of tissue after its disorder. Disruption of its signal is known to cause onset and progress of various diseases.
Regarding the relationship between TGFβ and diseases, the fibrosis of organs or tissues is well known. The fibrosis of an organ or a tissue arise from excessive accumulation of extracellular matrix proteins within the organ, as a defence mechanism against damage of the organ by some cause. The extracelluar matrix protein refers to a substance surrounding cells of the tissue. For example, fibrotic proteins such as collagen and elastin, glycoconjugates such as proteoglycan, and glycoproteins such as fibronectin and laminin are mentioned as major extracellular matrix proteins.
When the level of fibrosis of an organ is low, the organ can be recovered to a normal state without leaving any repair scar. On the other hand, when the level of disorder of the organ is large or when the disorder continues, the fibrosis causes damage to the innate function of the organ. Further, the damage causes new fibrosis to create a vicious cycle. Ultimately, this causes organ failure and, in the worst case, sometimes leads to death.
TGFβ is known to play an important role in the accumulation of the extracellular matrix proteins.
For example, the administration of TGFβ to normal animals is known to cause fibrosis in various tissues (International Review of Experimental Pathology, 34B: 43-67, 1993). Further, rapid fibrosis of tissues is also observed in trans-genetic mice for TGFβ1 and normal animals with gene-transfer of a TGFβ1 gene, (Proc. Natl. Acad. Sci. USA, 92: 2572-2576, 1995; Laboratory Investigation, 74: 991-1003, 1995).
TGFβ is considered to be involved in the fibrosis of tissues via the following mechanism.

1) TGFβ highly increases the level of extracellular matrix proteins such as fibronectin in cells (Journal of Biological Chemistry, 262: 6443-6446, 1987), collagen (Proc. Natl. Acad. Sci. USA, 85 1105-1108, 1988), and proteoglycan (Journal of Biological Chemistry, 263: 3039-3045, 1988).
2) TGFβ reduces the expression of degradative enzymes for extracellular matrix proteins (Journal of Biological Chemistry, 263: 16999-17005, 1988) and, in addition, highly promotes the expression of an inhibitor against the degradative enzymes for extracellular matrix proteins (Cancer Research, 49: 2553-2553, 1989). Consequently, the degradation of the extracellular matrix proteins is suppressed.
3) Further, TGFβ increases the expression of integrin as a receptor for an extracellular matrix proteins and promotes the deposition of the matrix proteins on cells (Journal of Biological Chemistry, 263: 4586-4592, 1988).
4) Furthermore, TGFβ proliferates cells which produce extracellular matrix proteins (American Journal of Physiology, 264: F199-F205, 1993).

TGFβ is known to be mainly involved in the fibrosis of organs such as kidney, liver, lung, heart, bone marrow, and skin.
For example, in the analysis of expression of TGFβ1, an increase in expression of TGFβ1 is observed in diseases such as human acute renal diseases, chronic renal diseases, diabetic nephropathy, renal allograft rejection, HIV nephropathy, hepatic fibrosis, cirrhosis, pulmonary fibrosis, scleroderma, and keloid (New Engl. J. Med., 331, 1286-1292, 1994), and the expression is known to correlate with the expression of extracellular matirix proteins.
Further, it has been shown that the administration of a soluble form of TGFβ type II receptor or an anti-TGFβ neutralizating antibody can inhibit fibrosis and pathology in pathologic animal models of renal disease, diabetic nephropathy, hepatic fibrosis, pulmonary fibrosis, and scleroderma (Nature, 346: 371-374, 1990; Journal of the British Thoracic Society, 54: 805-812, 1999; Journal of Immunology, 163: 5693-5699, 1999; Human Gene Therapy, 11: 33-42, 2000; Proc. Natl. Acad. Sci. USA., 97: 8015-20, 2000).
These facts show that the inhibition of TGFβ is useful for the prophylaxis and therapy for the diseases involving fibrosis including chronic renal diseases.
Further, TGFβ is also known to be involved in restenosis and arteriosclerosis.
In restenosis model animals, an increase in expression of TGFβ1 and its receptor is observed in a disordered blood

vessel, which suggests that TGFβ1 may be involved in the formation of new intima after balloon angioplasty injury (Clinical and Experimental Pharmacology and Physiology 23: 193-200, 1996).
For arteriosclerosis, a high level of expression of TGFβ1 is observed in non-foam macrophage infiltrated in an affected region in which active matrix synthesis takes place(American Journal of Physiology 146: 1140-1149, 1995), which suggests that the non-foam macrophage participates in extracellular matrix protein synthesis within an arteriosclerosis region through TGFβ1.
Further, in a migration test using cells, TGFβ1 is also reported to be a potent stimulating factor for migration of smooth muscle cells causative of arteriosclerosis and vascular restenosis (Biochem Biophys Res Commun., 169: 725-729, 1990).

TGFβ1 is also involved in wound repair.
For example, an experiment using a neutralizing antibody against TGFβ1 demonstrates that the inhibition of TGFβ1 suppresses excessive scar formation after the injury and is useful for functional recovery. Specifically, it is also known that the administration of a neutralizing antibody against TGFβ1 or TGFβ2 to rats can suppress scar formation and further promotes dermal cell construction via the suppression of dermal fibronectin and collagen deposition and a reduction in the number of monocytes and macrophages (Journal of Cellular Science 108: 985-1002, 1995). In other tissues, the acceleration of cure by the administration of an anti-TGFβ-neutralizing antibody is observed in a rabbit corneal injury model and a rat gastric ulcer model (Cornea 16: 177-187, 1997; An international Journal of gastroenterology & Hepatology 39: 172-175, 1996).

Further, TGFβ1 is also involved in peritoneal adhesion.
For example, it is suggested that the inhibition of TGFβ is effective for suppressing peritoneal adhesion and subdermal fibrous adhesion after surgery (J. Surg. Res., 65: 135-138, 1996).
It is reported in a number of articles that the administration of an anti-TGFβ neutralizing antibody or a soluble form of TGFβ type II receptor into model animals for cancer is also effective for suppressing tumor growth and cancer metastasis (Journal of Clinical Investigation, 92: 2569-76, 1993; Clinical Cancer Research 7: 2931-2940, 2001; Cancer Res. 59: 2210-6, 1999; Journal of Clinical Investigation 109: 1551-1559, 2002; Journal of Clinical Investigation 109: 1607-1615, 2002).

Tumors is considered to acquire tumor growth potential and metastatic ability by inducing angiogenesis on host side and by lowering immunity of the host side, through TGFβ produced by the tumors themselve, on the basis of studies using an anti-TGFβ neutralizing antibody. Thus, the inhibition of TGFβ is considered to be effective for suppressing cancer metastasis and cancer cell growth.

It is also reported that an anti-TGFβ1 neutralizing antibody is effective in ex vivo expansion of hematopoietic stem cells (Experimental Hematology 26: 374-381, 1998).
Further, TGFβ1 is known to have growth inhibitory activity against a number of other cell as well as against hematopoietic stem cells.
Accordingly, the inhibition of TGFβ is expected to be effective for ex vivo expansion of a number of cells including hematopoietic stem cells.

SUMMARY OF THE INVENTION

The present inventors have now found that a certain group of naphthyridine derivatives, quinoline derivatives, quinazoline derivatives, thienopyridine derivatives, and thienopyrimidine derivatives have inhibitory activity against TGFβ1. The present invention has been made based on such finding.
An object of the present invention is to provide compounds having potent TGFβ inhibitory activity.

In one embodiment of the present invention, there are provided compounds of formula (I) or pharmaceutically acceptable salts or solvates thereof:

[Chemical formula 1]

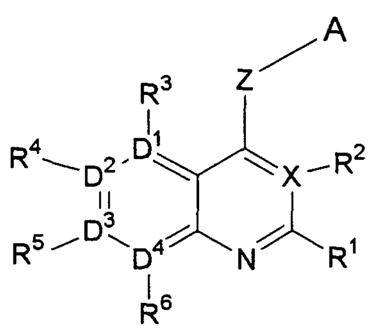

(I)

wherein
A represents a group of formula (a):

[Chemical formula 2]

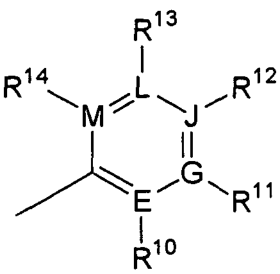

(a)

Z represents -O-, -N(-$R^Z$)-, -S-, or -C(=O)- wherein $R^Z$ represents a hydrogen atom or unsubstituted C1-4 alkyl, $D^1$, $D^2$, $D^3$, $D^4$, X, E, G, J, L, and M, which may be the same or different, represent C or N, $R^1$ to $R^6$ and $R^{10}$ to $R^{14}$, which may be the same or different, represent

(1) a hydrogen atom;
(2) a halogen atom;
(3) hydroxyl;
(4) cyano group;
(5) nitro group;
(6) C1-6 alkyl;
(7) C2-6 alkenyl;
(8) C2-6 alkynyl;
(9) C1-6 alkoxy;
(10) C1-6 alkylthio;
wherein (6) C1-6 alkyl, (7) C2-6 alkenyl, (8) C2-6 alkynyl, (9) C1-6 alkoxy, and (10) C1-6 alkylthio are optionally substituted by

(I) hydroxyl,
(II) a halogen atom,
(III) C1-4 alkoxy,
(IV) an oxygen atom,
(V) a saturated or unsaturated three- to nine-membered carbocyclic or heterocyclic group wherein the carbocyclic or heterocyclic group is optionally substituted by C1-4 alkyl, C1-4 alkoxy, a halogen atom, or a five- or six-membered carbocyclic or heterocyclic group, and the C1-4 alkyl group is optionally substituted by hydroxyl or phenyl,
(VI) amino group wherein one or two hydrogen atoms in the amino group are optionally substituted by C1-4 alkyl or a five- or six-membered carbocyclic or heterocyclic group, and the C1-4 alkyl group is optionally substituted by hydroxyl or C1-4 alkoxy,
(VII) -NHCONHR$^{VII}$ wherein R$^{VII}$ represents C1-4 alkyl,
(VIII) -OCOR$^{VIII}$ wherein R$^{VIII}$ represents C1-6 alkyl optionally substituted by amino group, or
(IX) -NSO$_2$R$^{IX}$ wherein R$^{IX}$ represents C1-4 alkyl;

(11) -NR$^a$R$^b$;
(12) -CO-OR$^c$;
(13) -CO-NR$^d$R$^e$;
wherein, in groups (11) to (13), $R^a$, $R^b$, $R^c$, $R^d$, and $R^e$, which may be the same or different, represent a hydrogen atom or C1-4 alkyl, and the C1-4 alkyl group is optionally substituted by

(a) hydroxyl,

(b) a halogen atom,
(c) C1-4 alkoxy,
(d) a saturated or unsaturated three-to nine-membered carbocyclic or heterocyclic group optionally substituted by C1-4 alkyl, C1-4 alkoxy, or a halogen atom, or
(e) amino group wherein one or two hydrogen atoms in the amino group are optionally substituted by C1-4 alkyl, and
$R^d$ and $R^e$ together may combine with the carbon atoms to which they are attached represent a saturated three- to nine-membered heterocyclic group, and the heterocyclic group is optionally substituted by C1-4 alkyl, C1-4 alkoxy, or a halogen atom or may contain one or more additional heteroatoms;

(14) a saturated or unsaturated three- to nine-membered carbocyclic group;
(15) a saturated or unsaturated three- to nine-membered heterocyclic group;
(16) a bicyclic saturated or unsaturated eight- to twelve-membered carbocyclic or heterocyclic group;
(17) $-OCOR^k$ wherein $R^k$ represents C1-4 alkyl; or
(18) $-OSO_2R^L$ wherein $R^L$ represents C1-4 alkyl,

wherein (14) carbocyclic group, (15) heterocyclic group, and (16) bicyclic carbocyclic or heterocyclic group are optionally substituted by (i) hydroxyl, (ii) a halogen atom, (iii) cyano group, (iv) nitro group, (v) amino group
wherein one or two hydrogen atoms in the amino group are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl, and
$R^{10}$ and $R^{11}$, $R^{11}$ and $R^{12}$, $R^{12}$ and $R^{13}$, and $R^{13}$ and $R^{14}$ together may combine with the carbon atoms to which they are attached represent a saturated or unsaturated five- or six-membered carbocyclic or heterocyclic group, and the carbocyclic or heterocyclic group is optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^9R^h$ wherein $R^f$, $R^9$, and $R^h$, which may be the same or different, represent a hydrogen atom or C1-4 alkyl,
provided that, when $D^1$, $D^2$, $D^3$, $D^4$, X, E, G, J, L, and M represent a nitrogen atom, groups $R^2$ to $R^6$ and $R^{10}$ to $R^{14}$ which attach to the nitrogen atom are absent, and
provided that, when all of $D^1$, $D^2$, $D^3$, and $D^4$ represent a carbon atom,

I) at least one of $R^4$ and $R^5$ represents (4) cyano group, (5) nitro group, (12) $-CO-OR^c$, (13) $-CO-NR^dR^e$ wherein any one of $R^d$ and $R^e$ represents optionally substituted C1-4 alkyl, (14) carbocyclic group, (15) heterocyclic group
wherein the heterocyclic group contains at least one substituent, or (16) bicyclic carbocyclic group or heterocyclic group, or
II) L represents a nitrogen atom, E, G, J, and M represent a carbon atom, $R^{10}$ represents a hydrogen atom, and $R^{14}$ represents (6) C1-6 alkyl group, (14) carbocyclic group, (15) heterocyclic group, or (16) bicyclic carbocyclic group or heterocyclic group.

In another embodiment of the present invention, formula (I) may also be defined as follow:

That is, in formula (I),
A represents a group of formula (a),
Z represents -O-, $-N(-R^Z)-$, -S-, or -C(=O)- wherein $R^Z$ represents a hydrogen atom or unsubstituted C1-4 alkyl,
$D^1$, $D^2$, $D^3$, $D^4$, X, E, G, J, L, and M, which may be the same or different, represent C or N,
$R^1$ to $R^6$ and $R^{10}$ to $R^{14}$, which may be the same or different, represent

(1) a hydrogen atom;
(2) a halogen atom;
(3) hydroxyl;
(4) cyano group;
(5) nitro group;
(6) C1-6 alkyl;
(7) C2-6 alkenyl;
(8) C2-6 alkynyl;
(9) C1-6 alkoxy;
(10) C1-6 alkylthio;

wherein (6) C1-6 alkyl, (7) C2-6 alkenyl, (8) C2-6 alkynyl, (9) C1-6 alkoxy, and (10) C1-6 alkylthio are optionally substituted by

(I) hydroxyl,
(II) a halogen atom,
(III) C1-4 alkoxy,
(IV) an oxygen atom,
(V) a saturated or unsaturated three- to nine-membered carbocyclic or heterocyclic group optionally substituted by C1-4 alkyl, C1-4 alkoxy, or a halogen atom, or
(VI) amino group wherein one or two hydrogen atoms in the amino group are optionally substituted by C1-4 alkyl, and the C1-4 alkyl group is optionally substituted by hydroxyl or C1-4 alkoxy,

(11) $-NR^aR^b$;
(12) $-CO-OR^c$;
(13) $-CO-NR^dR^e$;
wherein, in groups (11) to (13), $R^a$, $R^b$, $R^c$, $R^d$, and $R^e$, which may be the same or different, represent a hydrogen atom or C1-4 alkyl, and the C1-4 alkyl group is optionally substituted by

(a) hydroxyl,
(b) a halogen atom,
(c) C1-4 alkoxy,
(d) a saturated or unsaturated three- to nine-membered carbocyclic or heterocyclic group optionally substituted by C1-4 alkyl, C1-4 alkoxy, or a halogen atom, or
(e) amino group wherein one or two hydrogen atoms in the amino group are optionally substituted by C1-4 alkyl, and
$R^d$ and $R^e$ together may combine with the carbon atoms to which they are attached represent a saturated three- to nine-membered heterocyclic group, and the heterocyclic group is optionally substituted by C1-4 alkyl, C1-4 alkoxy, or a halogen atom or may contain one or more additional heteroatoms;

(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group;

wherein (14) carbocyclic group, (15) heterocyclic group, and (16) bicyclic carbocyclic or heterocyclic group are optionally substituted by (i) hydroxyl, (ii) a halogen atom, (iii) cyano group, (iv) nitro group, (v) amino group
wherein one or two hydrogen atoms in the amino group are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl, and
$R^{10}$ and $R^{11}$, $R^{11}$ and $R^{12}$, $R^{12}$ and $R^{13}$, and $R^{13}$ and $R^{14}$ together may combine with the carbon atoms to which they are attached represent a saturated or unsaturated five- or six-membered carbocyclic or heterocyclic group, and the carbocyclic or heterocyclic group is optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl,
provided that, when $D^1$, $D^2$, $D^3$, $D^4$, X, E, G, J, L, and M represent a nitrogen atom, groups $R^2$ to $R^6$ and $R^{10}$ to $R^{14}$ which attach to the nitrogen atom are absent, and
provided that, when all of $D^1$, $D^2$, $D^3$, and $D^4$ represent a carbon atom,

I) at least one of $R^4$ and $R^5$ represents (4) cyano group, (5) nitro group, (12) $-CO-OR^c$, (13) $-CO-NR^dR^e$
wherein any one of $R^d$ and $R^e$ represents optionally substituted C1-4 alkyl, (14) carbocyclic group, (15) heterocyclic group
wherein the heterocyclic group contains at least one substituent, or (16) bicyclic carbocyclic group or heterocyclic group, or
II) L represents a nitrogen atom, E, G, J, and M represent a carbon atom, $R^{10}$ represents a hydrogen atom, and $R^{14}$ represents (6) C1-6 alkyl group, (14) carbocyclic group, (15) heterocyclic group, or (16) bicyclic carbocyclic group or heterocyclic group.

In one preferred embodiment of the present invention, in formula (I), at least one of $D^1$ to $D^4$ represents a

nitrogen atom.

In another preferred embodiment of the present invention, in formula (I),
all of $D^1$ to $D^4$ represent a carbon atom,
$R^1$ and $R^2$ represent a hydrogen atom,
$R^3$ and $R^6$, which may be the same or different, represent a hydrogen atom, a halogen atom, or C1-4 alkyl, and
$R^4$ and $R^5$, which may be the same or different, represent

(4) cyano group;
(5) nitro group;
(12) -CO-OR$^c$;
(13) -CO-NR$^d$R$^e$;
wherein, in groups (12) and (13), $R^c$, $R^d$, and $R^e$, which may be the same or different, represent a hydrogen atom or, C1-4 alkyl, provided that at least one of $R^d$ and $R^e$ represents C1-4 alkyl, and the C1-4 alkyl group is optionally substituted by

(a) hydroxyl,
(b) a halogen atom,
(c) C1-4 alkoxy,
(d) a saturated or unsaturated three-to nine-membered carbocyclic or heterocyclic group optionally substituted by C1-4 alkyl, C1-4 alkoxy, or a halogen atom, or
(e) amino group wherein one or two hydrogen atoms in the amino group are optionally substituted by C1-4 alkyl,

(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group;
wherein (14) carbocyclic group, (15) heterocyclic group, and (16) bicyclic carbocyclic or heterocyclic group are optionally substituted by (i) hydroxyl, (ii) a halogen atom, (iii) cyano group, (iv) nitro group, (v) amino group wherein one or two hydrogen atoms in the amino group are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein $R^f$, $R^g$, and $R^h$, which may be the same or different, represent a hydrogen atom or C1-4 alkyl, and
(15) heterocyclic group contains at least one substituent.

In one embodiment of the present invention, there are provided compounds of formula (II) or pharmaceutically acceptable salts or solvates thereof:

[Chemical formula 3]

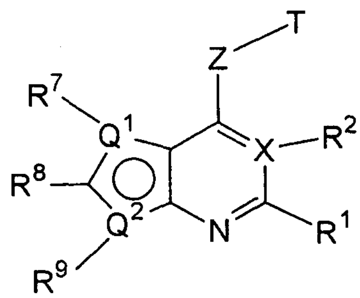

(II)

wherein
T represents a saturated or unsaturated five- or six-membered carbocyclic or heterocyclic group, wherein group T is optionally substituted by groups (2) to (16):

(2) a halogen atom;
(3) hydroxyl;
(4) cyano group;
(5) nitro group;
(6) C1-6 alkyl;
(7) C2-6 alkenyl;

(8) C2-6 alkynyl;
(9) C1-6 alkoxy;
(10) C1-6 alkylthio;
wherein (6) C1-6 alkyl, (7) C2-6 alkenyl, (8) C2-6 alkynyl, (9) C1-6 alkoxy, and (10) C1-6 alkylthio are optionally substituted by

(I) hydroxyl,
(II) a halogen atom,
(III) C1-4 alkoxy,
(IV) an oxygen atom,
(V) a saturated or unsaturated three- to nine-membered carbocyclic or heterocyclic group optionally substituted by C1-4 alkyl, C1-4 alkoxy, or a halogen atom, or
(VI) amino group wherein one or two hydrogen atoms in the amino group are optionally substituted by C1-4 alkyl, and the C1-4 alkyl group is optionally substituted by hydroxyl or C1-4 alkoxy,

(11) $-NR^aR^b$;
(12) $-CO-OR^c$;
(13) $-CO-NR^dR^e$;
wherein, in groups (11) to (13), $R^a$, $R^b$, $R^c$, $R^d$, and $R^e$, which may be the same or different, represent a hydrogen atom or C1-4 alkyl, and the C1-4 alkyl group is optionally substituted by

(a) hydroxyl,
(b) a halogen atom,
(c) C1-4 alkoxy,
(d) a saturated or unsaturated three- to nine-membered carbocyclic or heterocyclic group optionally substituted by C1-4 alkyl, C1-4 alkoxy, or a halogen atom, or
(e) amino group wherein one or two hydrogen atoms in the amino group are optionally substituted by C1-4 alkyl, and
$R^d$ and $R^e$ together may combine with the carbon atoms to which they are attached to represent a saturated three- to nine-membered heterocyclic group, and the heterocyclic group is optionally substituted by C1-4 alkyl, C1-4 alkoxy, or a halogen atom or may contain one or more additional heteroatoms;

(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group;

wherein (14) carbocyclic group, (15) heterocyclic group, and (16) bicyclic carbocyclic or heterocyclic group are optionally substituted by (i) hydroxyl, (ii) a halogen atom, (iii) cyano group, (iv) nitro group, (v) amino group wherein one or two hydrogen atoms in the amino group are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl,
two adjacent substituents on group T together may combine with the carbon atoms to which they are attached represent a saturated or unsaturated five- or six-membered carbocyclic or heterocyclic group, and the carbocyclic or heterocyclic group is optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl,
**[0019]** $Q^1$ and $Q^2$, which may be the same or different, represent C, S, O, or N,
X represents C or N,
Z represents -O-, $-N(-R^Z)-$, -S-, or -C(=O)- wherein $R^Z$ represents a hydrogen atom or unsubstituted C1-4 alkyl,
$R^1$, $R^2$, and $R^7$ to $R^9$, which may be the same or different, represent,

(1) a hydrogen atom;
(2) a halogen atom;
(3) hydroxyl;
(4) cyano group;
(5) nitro group;
(6) C1-6 alkyl;

(7) C2-6 alkenyl;
(8) C2-6 alkynyl;
(9) C1-6 alkoxy;
(10) C1-6 alkylthio;
wherein (6) C1-6 alkyl, (7) C2-6 alkenyl, (8) C2-6 alkynyl, (9) C1-6 alkoxy, and (10) C1-6 alkylthio are optionally substituted by

(I) hydroxyl,
(II) a halogen atom,
(III) C1-4 alkoxy,
(IV) an oxygen atom,
(V) a saturated or unsaturated three- to nine-membered carbocyclic or heterocyclic group optionally substituted by C1-4 alkyl, C1-4 alkoxy, or a halogen atom, or
(VI) amino group wherein one or two hydrogen atoms in the amino group are optionally substituted by C1-4 alkyl, and the C1-4 alkyl group is optionally substituted by hydroxyl or C1-4 alkoxy;

(11) $-NR^aR^b$;
(12) $-CO-OR^c$;
(13) $-CO-NR^dR^e$;
wherein, in groups (11) to (13), $R^a$, $R^b$, $R^c$, $R^d$, and $R^e$, which may be the same or different, represent a hydrogen atom or C1-4 alkyl, and C1-4 alkyl is optionally substituted by

(a) hydroxyl,
(b) a halogen atom,
(c) C1-4 alkoxy,
(d) a saturated or unsaturated three- to nine-membered carbocyclic or heterocyclic group optionally substituted by C1-4 alkyl, C1-4 alkoxy, or a halogen atom, or
(e) amino group wherein one or two hydrogen atoms in the amino groups are optionally substituted by C1-4 alkyl, and
$R^d$ and $R^e$ together may combine with the carbon atoms to which they are attached represent a saturated three- to nine-membered heterocyclic group, and the heterocyclic group is optionally substituted by C1-4 alkyl, C1-4 alkoxy, or a halogen atom and may contain one or more additional heteroatoms;

(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,
wherein (14) carbocyclic group, (15) heterocyclic group, and (16) bicyclic carbocyclic or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl, and
the five-membered ring part containing $Q^1$ and $Q^2$ in formula (II) represents an aromatic ring,
provided that, when X represents a nitrogen atom, $R^2$ is absent, and
when $Q^1$ and $Q^2$ represent an oxygen atom or a sulfur atom, $R^7$ and $R^9$ which attach to the oxygen atom or the sulfur atom are absent, and, when both $Q^1$ and $Q^2$ represent a nitrogen atom, any one of $R^7$ and $R^9$ is absent.

The compounds according to the present invention can be used for the treatment and prevention of diseases for which TGFβ inhibition is effective therapeutically or prophylactically.
Diseases for which TGFβ inhibition is effective therapeutically or prophylactically include, for example, chronic renal disease, acute renal disease, hepatic fibrosis, cirrhosis, pulmonary fibrosis, scleroderma, wound healing, arthritis, congestive cardiac disease, ulcer, ocular disorder, corneal problem, diabetic nephropathy, peritoneal sclerosis, arteriosclerosis, peritoneal adhesions, and subdermal adhesion and, further, malignant tumors.
Accordingly, the compounds according to the present invention can be useful for the prevention or treatment of these diseases. The compounds according to the present invention can also be useful for ex vivo expansion of hematopoietic stem cells.
The pharmaceutical composition according to the present invention comprises a compound of formula (I) or (II) according to the present invention as an active component.
The TGFβ inhibitor according to the present invention comprises a compound according to the present invention.

The method for treating or preventing a disease for which TGFβ inhibition according to the present invention is effective therapeutically or prophylactically, comprises the step of administering a therapeutically or prophylactically effective amount of a compound of formula (I) or (II) or a pharmaceutically acceptable salt or solvate thereof into a patient requiring the treatment or prevention of the disease for which TGFβ inhibition is effective therapeutically or prophylactically.

The method for ex vivo expansion according to the present invention comprises the step of adding a compound of formula (I) or (II) or a pharmaceutically acceptable salt or solvate thereof into intended cells in vitro to amplify the cells, at an amount effective for promoting cell growth.

DETAILED DESCRIPTION OF THE INVENTION

Compounds according to invention

The term "alkyl," "alkoxy," "alkenyl," "alkynyl," and "alkylthio" as used herein as a group or a part of a group respectively mean straight chain or branched chain alkyl, alkoxy, alkenyl, alkynyl, and alkylthio.

Accordingly, in the present specification, for example, "C1-6 alkyl" and "C1-6 alkoxy" as a group or a part of a group respectively mean straight chain or branched chain alkyl and alkoxy having 1 to 6 carbon atoms.

"C1-6 alkyl" is preferably C1-4 alkyl, more preferably C1-3 alkyl, still more preferably C1-2 alkyl. "C1-4 alkyl" is preferably C1-3 alkyl, more preferably C1-2 alkyl.

"C1-6 alkoxy" is preferably C1-4 alkoxy, more preferably C1-3 alkoxy, still more preferably C1-2 alkoxy. "C1-4 alkoxy" is preferably C1-3 alkoxy, more preferably C1-2 alkoxy.

"C2-6 alkenyl" is preferably C2-5 alkenyl, more preferably C2-4 alkenyl, still more preferably C2-3 alkenyl. "C2-4 alkenyl" is preferably C2-3 alkenyl, more preferably C2 alkenyl.

"C2-6 alkynyl" is preferably C2-5 alkynyl, more preferably C2-4 alkynyl, still more preferably C2-3 alkynyl. "C2-4 alkynyl" is preferably C2-3 alkynyl, more preferably C2 alkynyl.

"C1-6 alkylthio" is preferably C1-4 alkylthio, more preferably C1-3 alkylthio, still more preferably C1-2 alkylthio. "C1-4 alkylthio" is preferably C1-3 alkylthio, more preferably C1-2 alkylthio.

Examples of C1-6 alkyl include methyl, ethyl, n-propyl, isopropyl, n-butyl, i-butyl, s-butyl, t-butyl, n-pentyl, and n-hexyl.

Examples of C1-6 alkoxy include methoxy, ethoxy, n-propoxy, i-propoxy, n-butoxy, i-butoxy, s-butoxy, and t-butoxy.

Examples of C2-6 alkenyl include allyl, butenyl, pentenyl, and hexenyl.

Examples of C2-6 alkynyl include 2-propynyl, butynyl, pentynyl, and hexynyl.

Examples of C1-6 alkylthio include methylthio, ethylthio, n-propylthio, isopropylthio, n-butylthio, i-butylthio, and s-butylthio.

In the present specification, alkyl "optionally substituted by" means either alkyl in which one or more hydrogen atoms on the alkyl group may be substituted by one or more substituents (which may be the same or different), or unsubstituted alkyl. It will be apparent to a person having ordinary skill in the art that the maximum number of substituents may be determined depending upon the number of substitutable hydrogen atoms on alkyl. This is true of groups that contain a group which is other than alkyl and is substitutable, for example, carbocyclic groups such as alkylthio, alkoxy, alkenyl, alkynyl, and phenyl, heterocyclic groups such as pyridyl, or bicyclic groups such as naphthyl.

Further, C1-6 alkyl optionally substituted by "an oxygen atom" mainly means such a state that one oxygen atom may be substituted for two hydrogen atoms on an identical carbon atom on alkyl, that is, the case where a ketone may be formed. This, however, does not exclude the case where a cyclic ether structure such as oxirane is formed. Accordingly, examples of C1-6 alkyl optionally substituted by "an oxygen atom" include ethan-1-one, propan-1-one, propan-2-one, butan-1-one, and butan-2-one. This is true of alkenyl, alkynyl, alkoxy, and alkylthio.

The term "halogen atom" as used herein means a fluorine, chlorine, bromine, or iodine atom.

The term "unsaturated carbocylic ring" and "unsaturated heterocyclic ring" means a carbocyclic or heterocyclic ring containing one or more unsaturated bonds such as a double bond.

The "saturated or unsaturated three- to nine-membered carbocyclic group" is preferably a saturated or unsaturated five- to seven-membered carbocyclic group, more preferably a saturated or unsaturated five- or six-membered carbocyclic group. Examples of saturated or unsaturated three- to nine-membered carbocyclic rings include phenyl, cyclopropyl, cyclobutyl, cyclopentyl, cyclohexyl, and cycloheptyl.

The "saturated or unsaturated three- to nine-membered heterocyclic group" contains one or more heteroatoms selected from an oxygen atom, a nitrogen atom, and a sulfur atom. The saturated or unsaturated three- to nine-membered heterocyclic group is preferably a heterocyclic ring containing one, two or three heteroatoms and the remaining ring atoms being a carbon atom. The saturated or unsaturated three- to nine-membered heterocyclic group is preferably a saturated or unsaturated five- to seven-membered heterocyclic group, more preferably a saturated or unsaturated five- or six-membered heterocyclic group. Examples of saturated or unsaturated three- to nine-membered heterocyclic groups

include thienyl, pyridyl, 1,2,3-triazolyl, thiazolyl, imidazolyl, isoxazolyl, pyrazolyl, piperazinyl, piperazino, piperidyl, piperidino, morpholinyl, morpholino, homopiperazinyl, homopiperazino, thiomorpholinyl, thiomorpholino, tetrahydropyrrolyl, and azepanyl.

When the carbocyclic or heterocyclic group is substituted by two alkyl groups, the two alkyl groups together may form an alkylene chain, preferably a C1-3 alkylene chain. Azabicyclo[2.2.2]octanyl, bicyclo[2.2.2]octanyl, and norbornanyl may be mentioned as the carbocyclic or heterocyclic group having the above crosslinked structure.

The "bicyclic saturated or unsaturated eight- to twelve-membered carbocyclic or heterocyclic group" is preferably bicyclic saturated or unsaturated nine- or ten-membered carbocyclic or heterocyclic group. When the bicyclic group is a heterocyclic group, the bicyclic group contains one or more heteroatoms selected from oxygen, nitrogen, and sulfur atoms. Examples of such bicyclic groups include indolizine, indole, isoindole, benzofuran, benzothiophene, indazole, benzimidazole, benzothiazole, purine, quinoline, isoquinoline, cinnoline, phthalazine, quinazoline, quinoxaline, naphthyridine, and naphthalene.

In one preferred embodiment of the present invention, in formula (I), at least one of $D^1$ to $D^4$ represents a nitrogen atom. In this case, more preferably when at least one of $D^1$ and $D^2$ is a nitrogen atom, both $D^3$ and $D^4$ represent a carbon atom.
Still more preferably, $D^1$ represents a nitrogen atom, and all of $D^2$ to $D^4$ represent a carbon atom, or
$D^2$ represents a nitrogen atom, and all of $D^1$, $D^3$, and $D^4$ represent a carbon atom. In these cases, more preferably, X represents a carbon atom.

In formula (II), $Q^1$ and $Q^2$ are preferably selected from carbon and sulfur atoms. More preferably, any one of $Q^1$ and $Q^2$ represents a sulfur atom, and the other represents a carbon atom or a sulfur atom. Still more preferably,
$Q^1$ represents a sulfur atom, and $Q^2$ represents a carbon atom, or
$Q^1$ represents a carbon atom, and $Q^2$ represents a sulfur atom. Still more preferably,
when $Q^1$ represents a carbon atom and, at the same time, $Q^2$ represents a sulfur atom, X represents a nitrogen atom, or
when $Q^1$ represents a sulfur atom and, at the same time, $Q^2$ represents a carbon atom, X represents a nitrogen atom, or
when $Q^1$ represents a sulfur atom and, at the same time, $Q^2$ represents a carbon atom, X represents a carbon atom.

Preferably, Z represents -O-, -NH-, -S-, or -C(=O)-, more preferably -O- or -NH-, still more preferably -O-.

Preferably, $R^1$, $R^2$, and $R^3$ independently represent a hydrogen atom, a halogen atom, or C1-4 alkyl, more preferably a hydrogen atom, a halogen atom, or methyl, still more preferably a hydrogen atom.

In one preferred embodiment of the present invention, when X represents a carbon atom, both $R^1$ and $R^2$ represent a hydrogen atom.
In this case, more preferably, when $D^1$ represents a nitrogen atom and, at the same time, all of $D^2$ to $D^4$ represent a carbon atom, $R^6$ represents a hydrogen atom.
Alternatively, in this case, more preferably when $D^2$ represents a nitrogen atom and, at the same time, all of $D^1$, $D^3$, and $D^4$ represent a carbon atom, $R^3$ represents a hydrogen atom or a halogen atom while $R^6$ represents a hydrogen atom.

$R^4$ and $R^5$, and $R^7$ to $R^9$, which may be the same or different, preferably represent

(1) a hydrogen atom ;
(2) a halogen atom;
(3) hydroxyl;
(6) C1-6 alkyl;
(9) C1-6 alkoxy;
(12) -CO-OR$^c$;
(13) -CO-NR$^d$R$^e$;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,
wherein these groups are optionally substituted as defined above.

More preferably, $R^4$ and $R^5$, and $R^7$ to $R^9$, which may be the same or different, represent

(1) a hydrogen atom;
(2) a halogen atom;
(6') C1-4 alkyl optionally substituted by a halogen atom;
(9') C1-4 alkoxy optionally substituted by a halogen atom;
(12') -CO-OR$^c$;
(13') -CO-NR$^d$R$^e$;
wherein, in groups (12') and (13'), $R^c$, $R^d$, and $R^e$, which may be the same or different, represent a hydrogen atom or C1-2 alkyl, and the C1-2 alkyl group is optionally substituted by

(a) hydroxyl,
(b) a halogen atom,
(d') a saturated or an unsaturated five- or six-membered carbocyclic or heterocyclic group, or
(e) amino;

(14') a saturated or unsaturated five- or six-membered carbocyclic group; or
(15') a saturated or unsaturated five- or six-membered heterocyclic group,
wherein (14') carbocyclic group and (15') heterocyclic group are optionally substituted by (i) hydroxyl, (ii) a halogen atom, (v') amino group, (vi') C1-2 alkyl, or (ix') C1-2 alkoxy.

Still more preferably, $R^4$ and $R^5$, and $R^7$ to $R^9$, which may be the same or different, represent

(1) a hydrogen atom;
(2) a halogen atom;
(6) C1-6 alkyl; or
(9) C1-6 alkoxy;

wherein (6) C1-6 alkyl and (9) C1-6 alkoxy are optionally substituted as defined above.
More preferably, $R^4$ and $R^5$, and $R^7$ to $R^9$, which may be the same or different, represent a hydrogen atom, a halogen atom, or C1-4 alkyl.
In one more preferred embodiment of the present invention, $R^4$ and $R^5$ represent a hydrogen atom.
In another embodiment of the present invention,
$R^4$ represents optionally substituted C1-6 alkoxy, and $R^5$ represents a hydrogen atom, a halogen atom, or $-CO-NH_2$. More preferably, $R^4$ represents C1-6 alkoxy substituted by hydroxyl, still more preferably $-O(CH_2)m1-OH$ wherein m1 is an integer of 2 to 4, still more preferably $-OC_2H_5-OH$.
In a more preferred embodiment of the present invention, $R^7$ and $R^9$ represent a hydrogen atom, a halogen atom, or C1-4 alkyl, still more preferably a hydrogen atom, a halogen atom, or methyl.
In still more preferred embodiment of the present invention, $R^8$ represents
a hydrogen atom,
a halogen atom,
C1-4 alkyl,
a saturated or unsaturated six-membered carbocyclic group, or
a saturated or unsaturated six-membered heterocyclic group,
wherein the carbocyclic or heterocyclic group is optionally substituted by (i) hydroxyl, (ii) a halogen atom, (v') amino group, (vi') C1-2 alkyl, or (ix') C1-2 alkoxy.
In a still more preferred embodiment of the present invention, $R^7$ and $R^9$ represent a hydrogen atom, a halogen atom, or C1-4 alkyl. In this case,
$R^8$ represents
a hydrogen atom,
a halogen atom,
C1-4 alkyl,
a saturated or unsaturated six-membered carbocyclic group, or
a saturated or unsaturated six-membered heterocyclic group,
wherein the carbocyclic and heterocyclic groups are optionally substituted by (i) hydroxyl, (ii) a halogen atom, (v') amino group, (vi') C1-2 alkyl, or (ix') C1-2 alkoxy.
In one preferred embodiment of the present invention, when X represents a carbon atom, both $R^1$ and $R^2$ represent a hydrogen atom.
In this case, more preferably, when $Q^1$ represents a sulfur atom and, at the same time, $Q^2$ represents a carbon atom, $R^9$ represents a hydrogen atom.
Alternatively, in this case, more preferably, when $Q^1$ represents a carbon atom and, at the same time, $Q^2$ represents a sulfur atom, $R^7$ represents a hydrogen atom.
In another embodiment of the present invention, $R^4$ and $R^5$, and $R^7$ to $R^9$ independently may represent group $-OR^X$
wherein $R^X$ represents a hydrogen atom or $-(CH_2)m-R^{aX}$ wherein $R^{aX}$ represents a hydrogen atom, a halogen atom, hydroxyl, a saturated or unsaturated three- to six-membered carbocyclic or heterocyclic group, C1-4 alkoxy, C1-4 alkoxycarbonyl, or $-NR^{bX}R^{cX}$ wherein $R^{bx}$ and $R^{cx}$, which may be the same or different, represent a hydrogen atom, or C1-6 alkyl optionally substituted by hydroxyl, an oxygen atom, amino group, a nitrogen atom, or C1-4 alkyl, and $R^{bX}$ and $R^{cX}$ together may combine with nitrogen atoms to which they are attached represent a saturated or unsaturated five- or six-membered heterocyclic group wherein the heterocyclic group may contain one or more heteroatoms. The heterocyclic group is optionally substituted by C1-4 alkyl optionally substituted by hydroxyl; hydroxyl; an oxygen atom; aminocarbonyl; C1-4 alkoxy; C1-4 alkoxycarbonyl; or a saturated or unsaturated five- or six-membered heterocyclic group. The heterocyclic group may condense with another saturated or unsaturated five- or six-membered carbocyclic or heterocyclic group to form a bicyclic group. m is an integer of 1 to 6. The alkyl chain part $-(CH_2)m-$ in this group is optionally substituted by hydroxyl; an oxygen atom; $-OR^{dX}$ wherein $R^{dX}$ represents C1-4 alkyl or C1-4 alkylcarbonyl; or C1-4 alkyl optionally substituted by hydroxyl or a halogen atom.

In still another preferred embodiment of the present invention, $R^4$ and $R^5$, and $R^7$ to $R^9$ independently may represent group $-OR^m$ wherein $R^m$ represents groups of formulae (i) to (vi):

(i) a group of formula (i):

[Chemical formula 4]

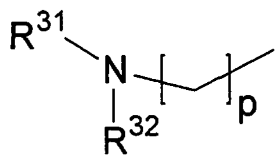

(i)

wherein
$R^{31}$ and $R^{32}$, which may be the same or different, represent
a hydrogen atom, or
C1-6 alkyl optionally substituted by hydroxyl, an oxygen atom, amino group, or a nitrogen atom,
$R^{31}$ and $R^{32}$ together may combine with the nitrogen atom to which they are attached represent a saturated or unsaturated five- or six-membered heterocyclic group wherein the heterocyclic group may contain one or more additional heteroatoms and is optionally substituted by C1-4 alkyl optionally substituted by hydroxyl; hydroxyl; an oxygen atom; aminocarbonyl; C1-4 alkoxy; C1-4 alkoxycarbonyl; or a saturated or unsaturated five- or six-membered heterocyclic group, and, further, the heterocyclic group optionally formed by combining $R^{31}$ with $R^{32}$ may condense with another saturated or unsaturated five- or six-membered carbocyclic or heterocyclic group to represent a bicyclic group, p is an integer of 2 to 4, preferably 2 or 3, and the alkyl chain part in this group is optionally substituted by hydroxyl or group $-OR^i$ wherein $R^i$ represents C1-4 alkyl or C1-4 alkylcarbonyl;

(ii) a group of formula (ii):

[Chemical formula 5]

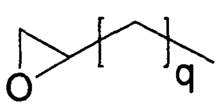

(ii)

wherein q is an integer of 1 to 4, preferably 1 or 2;

(iii) a group of formula (iii):

[Chemical formula 6]

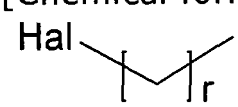

(iii)

wherein Hal represents a halogen atom, preferably a fluorine atom or a chlorine atom, and

r is an integer of 2 to 4, preferably 2 or 3;

(iv) a group of formula (iv):

[Chemical formula 7]

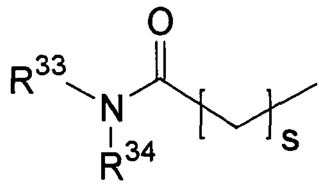

(iv)

wherein

$R^{33}$ and $R^{34}$, which may be the same or different, represent

a hydrogen atom or

$C_{1-6}$ alkyl optionally substituted by hydroxyl,

$R^{33}$ and $R^{34}$ may combine with the nitrogen atom to which they are attached represent a saturated or unsaturated five- or six-membered heterocyclic group wherein the heterocyclic group may contain one or more additional heteroatoms and is optionally substituted by C1-4 alkyl optionally substituted by hydroxyl; or a saturated or unsaturated five- or six-membered heterocyclic group, and, further, the heterocyclic group optionally formed by combining $R^{33}$ with $R^{34}$ may condense with another saturated or unsaturated five- or six-membered carbocyclic or heterocyclic group to represent a bicyclic group, and

s is an integer of 0 to 3, preferably 0 to 2;

(v) a group of formula (v):

[Chemical formula 8]

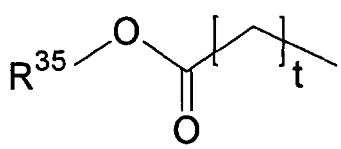

(v)

wherein

$R^{35}$ represents C1-4 alkyl, and

t is an integer of 0 to 3, preferably 0 to 2; and

(vi) a group of formula (vi):

[Chemical formula 9]

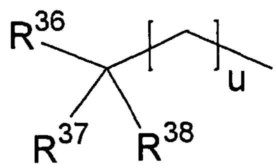

(vi)

wherein

$R^{36}$, $R^{37}$ and $R^{38}$, which may be the same or different, represent

a hydrogen atom,

C1-4 alkoxycarbonyl, or

C1-4 alkyl optionally substituted by hydroxyl or a halogen atom, and

u is an integer of 0 to 4, preferably 0 to 2, more preferably 0 or 1.

Preferably, $R^6$ represents a hydrogen atom, a halogen atom, or C1-4 alkyl, more preferably a hydrogen atom, a halogen atom, or methyl, still more preferably a hydrogen atom.

In a preferred embodiment of the present invention, in formula (I), when $R^1$ and $R^2$ represent a hydrogen atom, $R^3$ and $R^6$ represent a hydrogen atom, a halogen atom, or C1-4 alkyl. More preferably, all of $R^1$, $R^2$, $R^3$, and $R^6$ represent a hydrogen atom.

In one preferred embodiment of the present invention, in formula (I), when $R^3$ represents a hydrogen atom, Z represents -O-.

In a preferred embodiment of the present invention, in formula (II), both $R^1$ and $R^2$ represent a hydrogen atom.

In formula (I), when group A may represent a group of formula (a), preferably, in formula (a), any one of E, G, J, L, and M represents a nitrogen atom, and all the other groups represent a carbon atom. More preferably, L represents a nitrogen atom, and E, G, J and M represent a carbon atom.

In one preferred embodiment of the present invention, in formula (I), group A represents a group of formula (a-1) or (a-2):

[Chemical formula 10]

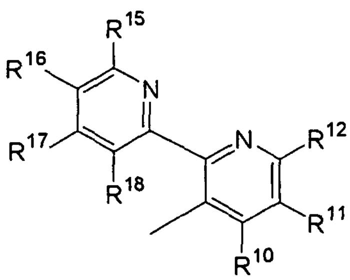
(a-1)

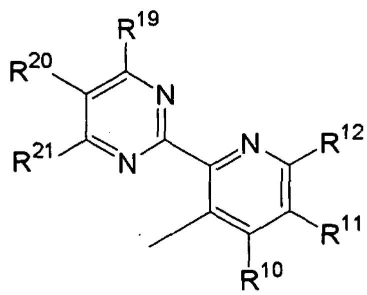
(a-2)

wherein

$R^{10}$ to $R^{12}$ are as defined above, and

$R^{15}$ to $R^{18}$ and $R^{19}$ to $R^{21}$, which may be the same or different, represent (0) a hydrogen atom, (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein $R^f$, $R^g$, and $R^h$, which may be the same or different, represent a hydrogen atom or C1-4 alkyl.

In another preferred embodiment of the present invention, in formula (I), group A represents a group of formula (a-3):

[Chemical formula 11]

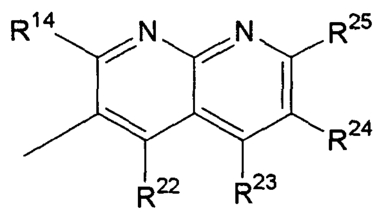
(a-3)

wherein

$R^{14}$ is as defined above, and

$R^{22}$ to $R^{25}$, which may be the same or different, represent (0) a hydrogen atom, (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^9$R$^h$ wherein $R^f$, $R^9$, and $R^h$, which may be the same or different, represent a hydrogen atom

or C1-4 alkyl.

In formula (II), preferably, group T represents a unsaturated six-membered carbocyclic or heterocyclic group optionally substituted by groups (2) to (16), more preferably an unsaturated six-membered heterocyclic group optionally substituted by groups (2) to (16). In this case, the heteroatom in the heterocyclic group is preferably a nitrogen atom. When the two adjacent substituents on group T together combine with the carbon atoms to which they are attached represent a saturated or unsaturated five- or six-membered carbocyclic or heterocyclic group, bicyclic groups which may be formed by group T include, for example, indolizine, indole, isoindole, benzofuran, benzothiophene, indazole, benzimidazole, benzothiazole, purine, quinoline, isoquinoline, cinnoline, phthalazine, quinazoline, quinoxaline, naphthyridine, and naphthalene.

In formula (II), more preferably, group T represents a group of formula (a):

[Chemical formula 12]

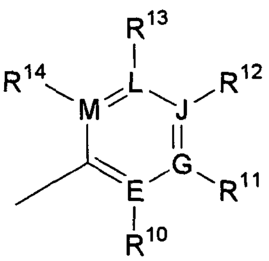

(a)

wherein

E, G, J, L, and M, which may be the same or different, represent C or N, and $R^{10}$ to $R^{14}$, which may be the same or different, represent

(1) a hydrogen atom;
(2) a halogen atom;
(3) hydroxyl;
(4) cyano group;
(5) nitro group;
(6) C1-6 alkyl;
(7) C2-6 alkenyl;
(8) C2-6 alkynyl;
(9) C1-6 alkoxy;
(10) C1-6 alkylthio;

wherein (6) C1-6 alkyl, (7) C2-6 alkenyl, (8) C2-6 alkynyl, (9) C1-6 alkoxy, and (10) C1-6 alkylthio are optionally substituted by

(I) hydroxyl,
(II) a halogen atom,
(III) C1-4 alkoxy,
(IV) an oxygen atom,
(V) a saturated or unsaturated three- to nine-membered carbocyclic or heterocyclic group optionally substituted by C1-4 alkyl, C1-4 alkoxy, or a halogen atom, or
(VI) amino group wherein one or two hydrogen atoms in the amino group are optionally substituted by C1-4 alkyl, and the C1-4 alkyl group is optionally substituted by hydroxyl or C1-4 alkoxy,

(11) $-NR^aR^b$;
(12) $-CO-OR^c$;
(13) $-CO-NR^dR^e$;

wherein, in groups (11) to (13), $R^a$, $R^b$, $R^c$, $R^d$, and $R^e$, which may be the same or different, represent a hydrogen atom or C1-4 alkyl, and the C1-4 alkyl group is optionally substituted by

(a) hydroxyl,
(b) a halogen atom,

(c) C1-4 alkoxy,

(d) a saturated or unsaturated three- to nine-membered carbocyclic or heterocyclic group optionally substituted by C1-4 alkyl, C1-4 alkoxy, or a halogen atom, or

(e) amino group wherein one or two hydrogen atoms in the amino group are optionally substituted by C1-4 alkyl, and

$R^d$ and $R^e$ together may combine with the carbon atoms to which they are attached represent a saturated three- to nine-membered heterocyclic group, and the heterocyclic group is optionally substituted by C1-4 alkyl, C1-4 alkoxy, or a halogen atom or may contain one or more additional heteroatoms;

(14) a saturated or unsaturated three- to nine-membered carbocyclic group;

(15) a saturated or unsaturated three- to nine-membered heterocyclic group; or

(16) a bicyclic saturated or unsaturated eight- to twelve-membered carbocyclic or heterocyclic group;

wherein (14) carbocyclic group, (15) heterocyclic group, and (16) bicyclic carbocyclic or heterocyclic group are optionally substituted by (i) hydroxyl, (ii) a halogen atom, (iii) cyano group, (iv) nitro group, (v) amino group wherein one or two hydrogen atoms in the amino group are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein $R^f$, $R^g$, and $R^h$, which may be the same or different, represent a hydrogen atom or C1-4 alkyl, and

$R^{10}$ and $R^{11}$, $R^{11}$ and $R^{12}$, $R^{12}$ and $R^{13}$, and $R^{13}$ and $R^{14}$ together may combine with the carbon atoms to which they are attached represent a saturated or unsaturated five- or six-membered carbocyclic or heterocyclic group, and the carbocyclic or heterocyclic group is optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein $R^f$, $R^g$, and $R^h$, which may be the same or different, represent a hydrogen atom or C1-4 alkyl,

provided that, when E, G, J, L, and M represent a nitrogen atom, groups $R^{10}$ to $R^{14}$ which attach to the nitrogen atom are absent.

In formula (II), when group T may represent a group of formula (a), preferably, in formula (a), any one of E, G, J, L, and M represents a nitrogen atom, and all the other groups represent a carbon atom. More preferably, L represents a nitrogen atom, and E, G, J and M represent a carbon atom.

In a further preferred embodiment of the present invention, in formula (II), group T represents a group of formula (a-1) or (a-2) wherein the substituents are as defined above. In another preferred embodiment of the present invention, in formula (II), group T represents a group of formula (a-3) wherein the substituents are as defined above.

In the group of formula (a-1) or (a-2) in formula (I) and formula (II), preferably, $R^{15}$ to $R^{18}$ and $R^{19}$ to $R^{21}$ are selected from the group consisting of a hydrogen atom, a halogen atom, C1-4 alkyl, and C1-4 alkoxy, more preferably selected from the group consisting of a hydrogen atom, a halogen atom, methyl, and methoxy. Still more preferably, all of these groups represent a hydrogen atom.

In the group of formula (a-3), preferably, $R^{22}$ to $R^{25}$ are selected from the group consisting of a hydrogen atom, a halogen atom, C1-4 alkyl, and C1-4 alkoxy, more preferably selected from the group consisting of a hydrogen atom, a halogen atom, and C1-4 alkyl, still more preferably selected from the group consisting of a hydrogen atom, a halogen atom, and methyl. Further preferably, all of $R^{22}$ to $R^{25}$ represent a hydrogen atom.

Preferably, $R^{10}$ represents a hydrogen atom, a halogen atom, or methyl, more preferably a hydrogen atom.

In a preferred embodiment of the present invention, $R^{11}$ and $R^{12}$ independently are selected from the group consisting of a hydrogen atom, a halogen atom, and C1-4 alkyl, more preferably selected from the group consisting of a hydrogen atom and C1-4 alkyl. More preferably, at least one of R11 and R12 represents C1-4 alkyl and the other represents a hydrogen atom or C1-4 alkyl, still more preferably are selected from the group consisting of a hydrogen atom, methyl, and ethyl. More preferably, both $R^{11}$ and $R^{12}$ represent methyl. Alternatively, $R^{11}$ represents a hydrogen atom, and $R^{12}$ represents methyl or ethyl.

In a more preferred embodiment of the present invention, when $R^{11}$ and $R^{12}$ are selected from the group consisting of a hydrogen atom, a halogen atom, C1-4 alkyl, and C1-4 alkoxy, preferably when selected from the group consisting of a hydrogen atom and C1-4 alkyl, $R^{10}$ represents a hydrogen atom. Still more preferably, in the group of formula (a-1) or (a-2), $R^{15}$ to $R^{18}$ and $R^{19}$ to $R^{21}$ are selected from the group consisting of a hydrogen atom, a halogen atom, C1-4 alkyl, and C1-4 alkoxy. Most preferably, in the group of formula (a-1) or (a-2), all of $R^{15}$ to $R^{18}$ and $R^{19}$ to $R^{21}$ represent a hydrogen atom.

In another preferred embodiment of the present invention, $R^{11}$ and $R^{12}$ together combine with the carbon atoms to which they are attached represent a saturated or unsaturated five- or six-membered carbocyclic or heterocyclic group. In this case, preferably, $R^{10}$ represents a hydrogen atom. More preferably, $R^{11}$ and $R^{12}$ together combine with the carbon atoms to which they are attached represent an unsaturated six-membered carbocyclic or heterocyclic group. Still more

preferably, $R^{11}$ and $R^{12}$ together combine with the carbon atoms to which they are attached represent an unsaturated six-membered heterocyclic group.

When $R^{10}$, $R^{11}$ and $R^{12}$ are as defined above, in a preferred embodiment of the present invention, Z represents -O-, X represents a carbon atom, and $R^1$ to $R^3$ and $R^6$ represent a hydrogen atom.

When $R^{13}$ is present, $R^{13}$ preferably represents a hydrogen atom, a halogen atom, or methyl, more preferably a hydrogen atom.

Preferably, $R^{14}$ represents
optionally substituted C1-4 alkyl or
an optionally substituted saturated or unsaturated five- or six-membered carbocyclic or heterocyclic group. Here regarding the C1-4 alkyl group, the term "optionally substituted" means that, as with "(6) C1-6 alkyl" in formula (I) or formula (II), the alkyl group may be substituted. Further, the term "optionally substituted" in the carbocyclic or heterocyclic group means that, as with "(14) carbocyclic group" or "(15) heterocyclic group" in formula (I) or (II), the carbocyclic or heterocyclic group may be substituted.

When $R^{11}$ and $R^{12}$ are selected from the group consisting of a hydrogen atom, a halogen atom, C1-4 alkyl, and C1-4 alkoxy, preferably selected from the group consisting of a hydrogen atom and C1-4 alkyl, preferably $R^{14}$ represents an optionally substituted saturated or unsaturated five- or six-membered carbocyclic or heterocyclic group. In this case, more preferably, $R^{14}$ represents an optionally substituted unsaturated six-membered heterocyclic group, still more preferably 2-pyridyl or 2,6-pyrimidyl.

When $R^{11}$ and $R^{12}$ together combine with the carbon atoms to which they are attached represent a saturated or unsaturated five- or six-membered carbocyclic or heterocyclic group, preferably, $R^{14}$ represents optionally substituted C1-4 alkyl or an optionally substituted saturated or unsaturated five- or six-membered carbocyclic or heterocyclic group. The C1-4 alkyl group which may be represented by $R^{11}$ and $R^{12}$ is preferably unsubstituted C1-4 alkyl. The carbocyclic or heterocyclic group which may be represented by $R^{11}$ and $R^{12}$ is preferably an optionally substituted unsaturated six-membered carbocyclic or heterocyclic group, more preferably phenyl optionally substituted by hydroxyl, a halogen atom, amino group, C1-4 alkyl, or C1-4 alkoxy, still more preferably phenyl optionally substituted by (i) hydroxyl, (ii) a halogen atom, (v') amino group, (vi') C1-2 alkyl, or (ix') C1-2 alkoxy.

When A represents a group of formula (a-3),
$R^{14}$ preferably represents
optionally substituted C1-4 alkyl,
an optionally substituted, saturated or unsaturated five- or six-membered carbocyclic or heterocyclic group, or
an optionally substituted bicyclic saturated or unsaturated nine- or ten-membered carbocyclic or heterocyclic group. In a more preferred embodiment, $R^{14}$ represents unsubstituted C1-4 alkyl. In another preferred embodiment, $R^{14}$ represents an optionally substituted unsaturated six-membered carbocyclic or heterocyclic group, more preferably phenyl optionally substituted by hydroxyl, a halogen atom, amino group, C1-4 alkyl, or C1-4 alkoxy, still more preferably phenyl optionally substituted by (i) hydroxyl, (ii) a halogen atom, (v') amino group, (vi') C1-2 alkyl, or (ix') C1-2 alkoxy.

In a preferred embodiment of the present invention, the compound of formula (I) may be a compound of formula (100):

[Chemical formula 13]

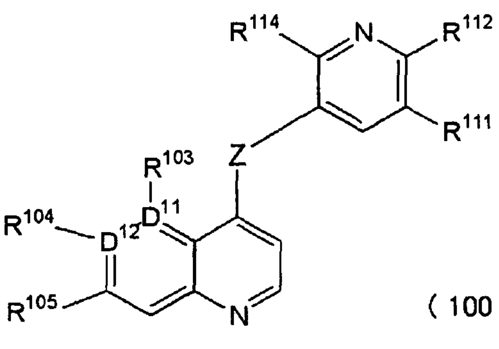

(100)

wherein
Z represents -O-, -NH-, -S-, or -C(=O)-, preferably -O- or -NH-, more preferably -O-,
any one of $D^{11}$ and $D^{12}$ represents a nitrogen atom, and the other represents a carbon atom,
$R^{103}$ represents a hydrogen atom or a halogen atom, preferably a hydrogen atom,
$R^{104}$ and $R^{105}$, which may be the same or different, represent

(1) a hydrogen atom ;
(2) a halogen atom;
(3) hydroxyl;
(6) C1-6 alkyl;
(9) C1-6 alkoxy;
(12) -CO-$OR^c$;
(13) -CO-$NR^dR^e$;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,
wherein these groups are optionally substituted as in the definition of formula (I), $R^{111}$ and $R^{112}$, which may be the same or different, are selected from the group consisting of a hydrogen atom, C1-4 alkyl, and C1-4 alkoxy, preferably selected from the group consisting of a hydrogen atom and C1-4 alkyl, more preferably selected from the group consisting of a hydrogen atom, methyl, and ethyl,
$R^{114}$ represents
(14") a saturated or unsaturated five- or six-membered carbocyclic group;
(15") a saturated or unsaturated five- or six-membered heterocyclic group; or
(16") a bicyclic saturated or unsaturated nine- or ten-membered carbocyclic or heterocyclic group;
wherein (14") carbocyclic group, (15") heterocyclic group, and (16") bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-$OR^f$, or (xii) -CO-$NR^gR^h$ wherein $R^f$, $R^g$, and $R^h$, which may be the same or different, represent a hydrogen atom or C1-4 alkyl.

In formula (100), preferably, when Z represents -O-, $R^{103}$ represents a hydrogen atom.
In formula (100), preferably, $R^{104}$ and $R^{105}$ represent a hydrogen atom, a halogen atom, or C1-4 alkyl, more preferably a hydrogen atom, a halogen atom, or methyl, still more preferably a hydrogen atom.
In formula (100), preferably, both $R^{111}$ and $R^{112}$ represent methyl, or alternatively $R^{111}$ represents a hydrogen atom while $R^{112}$ represents ethyl.
In formula (100), preferably, $R^{114}$ represents a group of formula (a-4) or formula (a-5):

[Chemical formula 14]

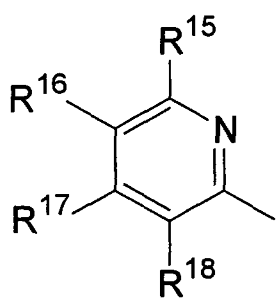
(a-4)
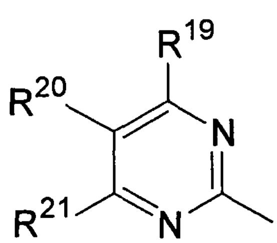
(a-5)

wherein
$R^{15}$ to $R^{18}$ and $R^{19}$ to $R^{21}$, which may be the same or different, represent a group selected from the group consisting of a hydrogen atom, a halogen atom, C1-4 alkyl, and C1-4 alkoxy, preferably selected from the group consisting of a hydrogen atom, a halogen atom, C1-2 alkyl, and C1-2 alkoxy, and, more preferably, all of $R^{15}$ to $R^{18}$ and $R^{19}$ to $R^{21}$ represent a hydrogen atom.
In a preferred embodiment of the present invention, the compound of formula (I) may be a compound of formula (200):

[Chemical formula 15]

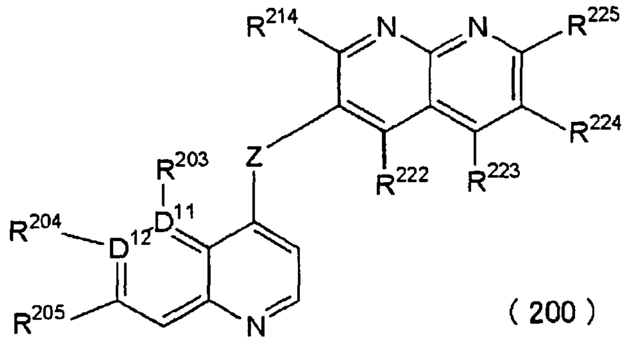

(200)

wherein

Z represents -O-, -NH-, -S-, or -C(=O)-, preferably -O- or -NH-, more preferably -O-,
any one of $D^{11}$ and $D^{12}$ represents a nitrogen atom, and the other represents a carbon atom,
$R^{203}$ represents a hydrogen atom or a halogen atom, preferably a hydrogen atom,
$R^{204}$ and $R^{205}$, which may be the same or different, represent

(1) a hydrogen atom ;
(2) a halogen atom;
(3) hydroxyl;
(6) C1-6 alkyl;
(9) C1-6 alkoxy;
(12) -CO-$OR^c$;
(13) -CO-$NR^dR^e$;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,
wherein these groups are optionally substituted as defined in formula (I),
$R^{222}$ to $R^{225}$, which may be the same or different, represent a group selected from the group consisting of a hydrogen atom, a halogen atom, C1-4 alkyl, and C1-4 alkoxy, preferably a group selected from the group consisting of a hydrogen atom, a halogen atom, and C1-4 alkyl, more preferably a hydrogen atom, a halogen atom, or methyl, still more preferably a hydrogen atom, and
$R^{214}$ represents
unsubstituted C1-4 alkyl,
an optionally substituted unsaturated six-membered carbocyclic or heterocyclic group, or
an optionally substituted, bicyclic saturated or unsaturated nine- or ten-membered carbocyclic or heterocyclic group.

In formula (200), preferably, when Z represents -O- and, at the same time, $R^{203}$ represents a hydrogen atom, all of $R^{222}$ to $R^{225}$ represent a hydrogen atom.

In formula (200), preferably, $R^{204}$ and $R^{205}$ represent a hydrogen atom, a halogen atom, or C1-4 alkyl, more preferably a hydrogen atom, a halogen atom, or methyl, still more preferably a hydrogen atom.

In a preferred embodiment of the present invention, in formula (200), $R^{214}$ represents phenyl optionally substituted by hydroxyl, a halogen atom, amino group, C1-4 alkyl, or C1-4 alkoxy, more preferably phenyl optionally substituted by (i) hydroxyl, (ii) a halogen atom, (v') amino group, (vi') C1-2 alkyl, or (ix') C1-2 alkoxy, still more preferably phenyl optionally substituted by (i) hydroxyl, (ii) a halogen atom, (v') amino group, (vi') C1-2 alkyl, or (ix') C1-2 alkoxy, still more preferably unsubstituted phenyl.

In another preferred embodiment of the present invention, in formula (200), $R^{214}$ represents methyl or ethyl.

In still another embodiment of the present invention, in formula (I), when all of $D^1$ to $D^4$ represent a carbon atom, requirement I) or II) should be satisfied:

I) any one of $R^4$ and $R^5$ represents (4) cyano group, (5) nitro group, (12) -CO-$OR^c$, (13) -CO-$NR^dR^e$ wherein any one of $R^d$ and $R^e$ represents optionally substituted C1-4 alkyl, (14) carbocyclic group, (15) heterocyclic group wherein the heterocyclic group contains at least one substituent, or (16) bicyclic carbocyclic or heterocyclic group; and

II) L represents a nitrogen atom, E, G, J, and M represent a carbon atom, $R^{10}$ represents a hydrogen atom, and $R^{14}$ represents (6) C1-6 alkyl group, (14) carbocyclic group,(15) heterocyclic group, or (16) bicyclic carbocyclic or heterocyclic group.

In another preferred embodiment of the present invention, in formula (I), when all of $D^1$ to $D^4$ represent a carbon atom, at least one of $R^4$ and $R^5$ represents (4) cyano group, (5) nitro group, (12)-CO-OR$^c$, (14) carbocyclic group, (15) heterocyclic group containing at least one substituent, or (16) bicyclic carbocyclic or heterocyclic group.

In a further preferred embodiment of the present invention, in formula (I), when all of $D^1$ to $D^4$ represent a carbon atom,
$R^1$ and $R^2$ represent a hydrogen atom, and
$R^4$ and $R^5$, which may be the same or different, represent a hydrogen atom, a halogen atom, or C1-4 alkyl, and
$R^4$ and $R^5$, which may be the same or different, represent

(4) cyano group;
(5) nitro group;
(12) -CO-OR$^c$;
(13) -CO-NR$^d$R$^e$;
wherein, in groups (12) and (13), $R^c$, $R^d$, and $R^e$, which may be the same or different, represent a hydrogen atom or C1-4 alkyl, provided that at least one of $R^d$ and $R^e$ represents C1-4 alkyl, and the C1-4 alkyl group is optionally substituted by

(a) hydroxyl,
(b) a halogen atom,
(c) C1-4 alkoxy,
(d) a saturated or unsaturated three- to nine-membered carbocyclic or heterocyclic group optionally substituted by C1-4 alkyl, C1-4 alkoxy, or a halogen atom, or
(e) amino group wherein one or two hydrogen atoms in the amino groups are optionally substituted by C1-4 alkyl,

(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,
wherein (14) carbocyclic group, (15) heterocyclic group, and (16) bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein $R^f$, $R^g$, and $R^h$, which may be the same or different, represent a hydrogen atom or C1-4 alkyl, and (15) heterocyclic group contains at least one substituent. In this case, more preferably, $R^4$ and $R^5$ represent
-CO-NR$^d$R$^e$ wherein $R^d$ and $R^e$, which may be the same or different, represent a hydrogen atom or C1-2 alkyl optionally substituted by hydroxyl, a halogen atom, phenyl, or amino group; or
phenyl optionally substituted by (i) hydroxyl, (ii) a halogen atom, (v') amino group, (vi') C1-2 alkyl, or (ix') C1-2 alkoxy.

In another preferred embodiment of the present invention, in formula (I), when all of $D^1$ to $D^4$ represent a carbon atom, at least one of $R^4$ and $R^5$ represents
an unsaturated five- or six-membered carbocyclic or heterocyclic group wherein the carbocyclic or heterocyclic group is optionally substituted by C1-4 alkyl, C1-4 alkoxy, a halogen atom ,amino group, or hydroxyl), or
a bicyclic unsaturated nine- or ten-membered carbocyclic or heterocyclic group wherein the carbocyclic or heterocyclic group is optionally substituted by C1-4 alkyl, C1-4 alkoxy, a halogen atom, amino group, or hydroxyl.

In still another preferred embodiment of the present invention, in formula (I), when all of $D^1$ to $D^4$ represent a carbon atom, $R^1$, $R^2$, $R^3$, $R^4$, $R^5$, and $R^6$ are as defined above. In this case, L represents a nitrogen atom, and E, G, J and M represent a carbon atom. Further, in this case, $R^{14}$ represents (16) bicyclic carbocyclic or heterocyclic group in which these cyclic groups are optionally substituted as defined in formula (I).

In a further preferred embodiment of the present invention, in formula (I), when all of $D^1$ to $D^4$ represent a carbon atom, $R^1$, $R^2$, $R^3$, $R^4$, $R^5$, and $R^6$ are as defined above, L represents a nitrogen atom, and E, G, J and M represent a carbon atom. In this case, $R^{14}$ represents

(6) C1-6 alkyl;
wherein the alkyl group is optionally substituted as defined in formula (I),
(14'') a saturated or unsaturated five- or six-membered carbocyclic group;

(15") a saturated or unsaturated five- or six-membered heterocyclic group; or
(16") a bicyclic saturated or unsaturated nine- or ten-membered carbocyclic or heterocyclic group;
wherein (14") carbocyclic group, (15") heterocyclic group, and (16") bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-$OR^f$, or (xii) -CO-$NR^gR^h$ wherein $R^f$, $R^g$, and $R^h$, which may be the same or different, represent a hydrogen atom or C1-4 alkyl.

In another preferred embodiment of the present invention, in formula (I), when both $D^1$ and $D^2$ represent a carbon atom, the compound of formula (I) may be a compound of formula (300):

[Chemical formula 16]

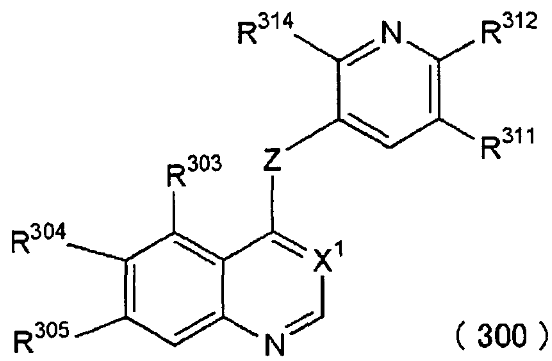

(300)

wherein
$X^1$ represents CH or N,
Z represents -O-, -NH-, -S-, or -C(=O)-, preferably -O- or -NH-, more preferably -O-,
$R^{303}$ represents a hydrogen atom, a halogen atom, or C1-4 alkyl, preferably a hydrogen atom, a halogen atom, or methyl, more preferably a hydrogen atom,
$R^{304}$ and $R^{305}$, which may be the same or different, represent

(1) a hydrogen atom;
(2) a halogen atom;
(3) hydroxyl;
(6) C1-6 alkyl;
(9) C1-6 alkoxy;
(12) -CO-$OR^c$;
(13) -CO-$NR^dR^e$;
(14) a saturated or unsaturated three- to nine-membered carbocyclic group;
(15) a saturated or unsaturated three- to nine-membered heterocyclic group;
(16) a bicyclic saturated or unsaturated eight- to twelve-membered carbocyclic or heterocyclic group;
(17) -$OCOR^k$ wherein $R^k$ represents C1-4 alkyl; or
(18) -$OSO_2R^L$ wherein $R^L$ represents C1-4 alkyl,
wherein these groups are optionally substituted as defined in formula (I),
at least one of $R^{311}$ and $R^{312}$ represents C1-4 alkyl and the other represents a hydrogen atom or C1-4 alkyl, and $R^{314}$ represents an unsaturated six-membered heterocyclic group wherein the heterocyclic group is optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-$OR^f$, or (xii) -CO-$NR^gR^h$ wherein $R^f$, $R^g$, and $R^h$, which may be the same or different, represent a hydrogen atom or C1-4 alkyl.

In formula (300), preferably, when Z represents -O-, $R^{303}$ represents a hydrogen atom and $R^{314}$ represents a

group of formula (a-4) or formula (a-5). In this case, in formula (a-4) or formula (a-5), preferably, $R^{15}$ to $R^{18}$ and $R^{19}$ to $R^{21}$ are selected from the group consisting of a hydrogen atom, a halogen atom, methyl and methoxy.

In a preferred embodiment of the present invention, in formula (300),
Z represents -O-,
$R^{303}$ represents a hydrogen atom, and
$R^{304}$ represents

(1) a hydrogen atom;
(2) a halogen atom;
(3) hydroxyl;
(9) C1-6 alkoxy;
(17) $-OCOR^k$ wherein $R^k$ represents C1-4 alkyl; or
(18) $-OSO_2R^L$ wherein $R^L$ represents C1-4 alkyl,
wherein these groups are optionally substituted as defined in claim 1,
$R^{305}$ represents a hydrogen atom, a halogen atom, or $-CO-NH_2$, and
$R^{314}$ represents a group of formula (a-4) or (a-5).

In formula (300), preferably, $R^{15}$ to $R^{18}$ and $R^{19}$ to $R^{21}$ represent a hydrogen atom.

In formula (300), preferably, $X^1$ represents CH.

In formula (300), preferably, $R^{304}$ represents C1-6 alkoxy which is optionally substituted as defined in claim 1, more preferably C1-6 alkoxy substituted by hydroxyl, still more preferably $-O(CH_2)m1-OH$ wherein m1 is an integer of 2 to 4. Still more preferably, $R^{304}$ represents $-OC_2H_5-OH$.
Prerferably, $R^{305}$ represents a hydrogen atom, a fluorine atom, or $-CO-NH_2$.

In formula (300), preferably, $R^{311}$ and $R^{312}$, which may be the same or different, represent C1-4 alkyl. More preferably, both $R^{311}$ and $R^{312}$ represent methyl. Alternatively, $R^{311}$ represents a hydrogen atom and, at the same time, $R^{312}$ represents C1-4 alkyl. Preferably, $R^{311}$ represents a hydrogen atom, and, at the same time, $R^{312}$ represents methyl or ethyl.

Preferred compounds of formula (300) are selected, for example, from compounds 181, 188, 192, 200, 202, and 205.

In another preferred embodiment of the present invention, in formula (I), when both $D^1$ and $D^2$ represent a carbon atom, the compund of formula (I) may be a compound of formula (400):

[Chemical formula 17]

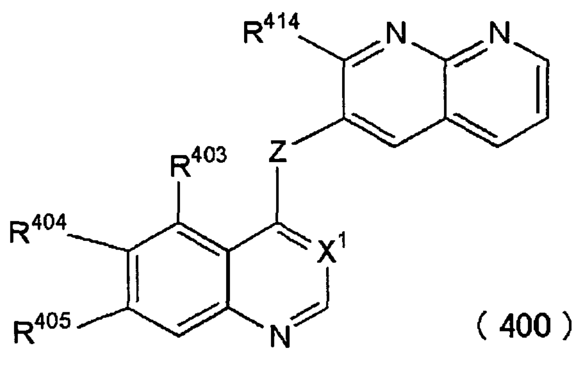

(400)

wherein
$X^1$ represents CH or N,
Z represents -O-, -NH-, -S-, or -C(=O)-, preferably -O- or -NH-, more preferably -O-,
$R^{403}$ represents a hydrogen atom, a halogen atom, or C1-4 alkyl, preferably a hydrogen atom, a halogen atom, or methyl, more preferably a hydrogen atom,
$R^{404}$ and $R^{405}$, which may be the same or different, represent

(1) a hydrogen atom;
(2) a halogen atom;
(3) hydroxyl;
(6) C1-6 alkyl;

(9) C1-6 alkoxy;
(12) -CO-OR$^c$;
(13) -CO-NR$^d$R$^e$;
(14) a saturated or unsaturated three- to nine-membered carbocyclic group;
(15) a saturated or unsaturated three- to nine-membered heterocyclic group;
(16) a bicyclic saturated or unsaturated eight- to twelve-membered carbocyclic or heterocyclic group;
(17) -OCOR$^k$ wherein R$^k$ represents C1-4 alkyl; or
(18) -OSO$_2$R$^L$ wherein R$^L$ represents C1-4 alkyl,
wherein these groups are optionally substituted as defined in formula (I),
R$^{414}$ represents
(6) C1-6 alkyl;
wherein the alkyl group is optionally substituted as defined in formula (I),
(14") a saturated or unsaturated five- or six-membered carbocyclic group;
(15") a saturated or unsaturated five- or six-membered heterocyclic group; or
(16") a bicyclic saturated or unsaturated nine- or ten-membered carbocyclic or heterocyclic group;
wherein (14") carbocyclic group, (15") heterocyclic group, and (16") bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein R$^f$, R$^g$, and R$^h$, which may be the same or different, represent a hydrogen atom or C1-4 alkyl.

In formula (400), preferably, when Z represents -O-, R$^{403}$ represents a hydrogen atom, and
R$^{414}$ represents
an unsaturated five- or six-membered carbocyclic or heterocyclic group wherein the carbocyclic or heterocyclic group is optionally substituted by C1-4 alkyl, C1-4 alkoxy, a halogen atom, amino group, or hydroxyl, or
a bicyclic unsaturated nine- or ten-membered carbocyclic or heterocyclic group wherein the carbocyclic or heterocyclic group is optionally substituted by C1-4 alkyl, C1-4 alkoxy, a halogen atom, amino group, or hydroxyl. In this case, more preferably, R$^{414}$ represents phenyl optionally substituted by (i) hydroxyl, (ii) a halogen atom, (v') amino group, (vi') C1-2 alkyl, or (ix') C1-2 alkoxy.

In a preferred embodiment of the present invention, in formula (400),
X$^1$ represents CH,
Z represents -O-,
R$^{403}$ represents a hydrogen atom,
any one of R$^{404}$ and R$^{405}$ represents C1-4 alkoxy substituted by hydroxyl, and the other represents unsubstituted C1-4 alkoxy, and
R$^{414}$ represents phenyl.

For example, compound 178 may be mentioned as preferred compounds of formula (400).

In a preferred embodiment of the present invention, the compound of formula (II) may be a compound of formula (500):

[Chemical formula 18]

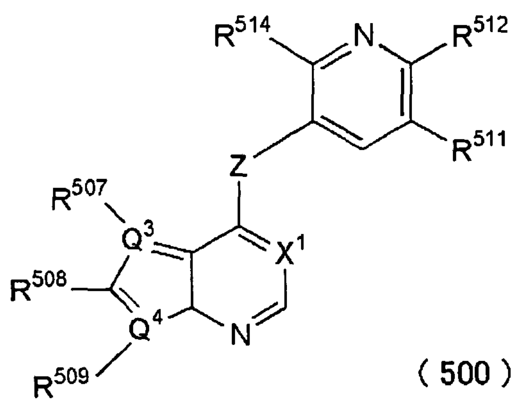

(500)

wherein
$X^1$ represents CH or N,
Z represents -O-, -NH-, -S-, or -C(=O)-, preferably -O- or -NH-, more preferably -O-,
any one of $Q^3$ and $Q^4$ represents a sulfur atom, and the other represents a carbon atom,
$R^{507}$ to $R^{509}$, which may be the same or different, represent

(1) a hydrogen atom;
(2) a halogen atom;
(3) hyd roxyl;
(6) C1-6 alkyl;
(9) C1-6 alkoxy;
(12) -CO-$OR^c$;
(13) -CO-$NR^dR^e$;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group
wherein these groups are optionally substituted as defined in formula (II),
$R^{511}$ and $R^{512}$, which may be the same or different, are selected from the group consisting of a hydrogen atom and C1-4 alkyl, preferably selected from the group consisting of a hydrogen atom, methyl, and ethyl,
$R^{514}$ represents
(14") a saturated or unsaturated five- or six-membered carbocyclic group;
(15") a saturated or unsaturated five- or six-membered heterocyclic group; or
(16") a bicyclic saturated or unsaturated nine- or ten-membered carbocyclic or heterocyclic group;
wherein (14") carbocyclic group, (15") heterocyclic group, and (16") bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-$OR^f$, or (xii) -CO-$NR^9R^h$ wherein $R^f$, $R^9$, and $R^h$, which may be the same or different, represent a hydrogen atom or C1-4 alkyl.

In formula (500), preferably,
when $Q^3$ represents a sulfur atom, $Q^4$ represents a carbon atom, or
when $Q^3$ represents a carbon atom, $Q^4$ represents a sulfur atom.
In formula (500), both $R^{511}$ and $R^{512}$ represent methyl, or alternatively, $R^{511}$ represents a hydrogen atom while $R^{512}$ represents ethyl. In this case, preferably, Z represents -O-.
In formula (500), preferably, $R^{514}$ represents a group of formula (a-4) or formula (a-5). In formula (a-4) or formula (a-5), $R^{15}$ to $R^{18}$ and $R^{19}$ to $R^{21}$, which may be the same or different, are selected from the group consisting of a hydrogen atom, a halogen atom, C1-4 alkyl, and C1-4 alkoxy. Preferably, all of $R^{15}$ to $R^{18}$ and $R^{19}$ to $R^{21}$ represent a hydrogen atom.
Preferably, $R^{507}$ and $R^{509}$ represent a hydrogen atom, a halogen atom, or C1-4 alkyl, more preferably a hydrogen atom, a halogen atom, or methyl, still more preferably a hydrogen atom.
Preferably, $R^{508}$ represents
a hydrogen atom,
a halogen atom,
C1-4 alkyl, or
a saturated or unsaturated six-membered carbocyclic or,
a saturated or unsaturated six-membered heterocyclic group,
wherein said carbocyclic or heterocyclic group is optionally substituted by (i) hydroxyl, (ii) a halogen atom, (v') amino group, (vi') C1-2 alkyl, or (ix') C1-2 alkoxy.
In formula (500), preferably,
$R^{507}$ and $R^{509}$ represent a hydrogen atom, a halogen atom, or C1-4 alkyl, and, in this case,
$R^{508}$ represents
a hydrogen atom ,
a halogen atom,
C1-4 alkyl,
a saturated or unsaturated six-membered carbocyclic group, or
a saturated or unsaturated six-membered heterocyclic group
wherein the carbocyclic and heterocyclic groups are optionally substituted by (i) hydroxyl, (ii) a halogen atom, (v') amino group, (vi') C1-2 alkyl, or (ix') C1-2 alkoxy.

In a preferred embodiment of the present invention, in formula (500),
$Q^3$ represents a sulfur atom,
$Q^4$ represents a carbon atom,
$R^{508}$ represents
a hydrogen atom,
C1-4 alkyl, or
a saturated or unsaturated six-membered carbocyclic or heterocyclic group,
wherein said carbocyclic or heterocyclic group is optionally substituted by (i) hydroxyl, (ii) a halogen atom, (v') amino group, (vi') C1-2 alkyl, or (ix') C1-2 alkoxy, and
$R^{509}$ represents a hydrogen atom. In this case, more preferably, $R^{508}$ represents a hydrogen atom or phenyl.

In a preferred embodiment of the present invention, in formula (500),
$Q^3$ represents a carbon atom,
$Q^4$ represents a sulfur atom, and
$R^{507}$ and $R^{508}$ are selected from the group consisting of a hydrogen atom and C1-4 alkyl. In this case, more preferably,
both $R^{507}$ and $R^{508}$ represent a hydrogen atom or
both $R^{507}$ and $R^{508}$ represent methyl or
$R^{507}$ represents methyl while $R^{508}$ represents a hydrogen atom.

In a preferred embodiment of the present invention, the compound of formula (II) may be a compound of formula (600):

[Chemical formula 19]

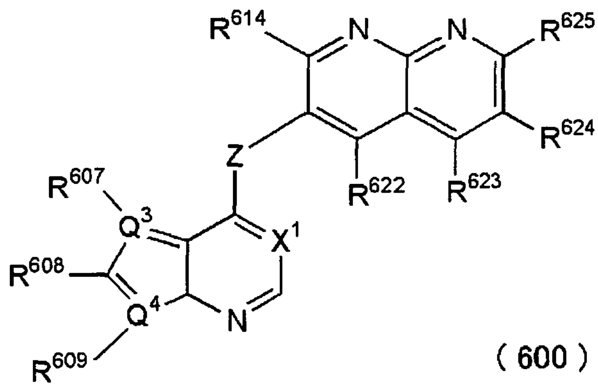

(600)

wherein
$X^1$ represents CH or N,
Z represents -O-, -NH-, -S-, or -C(=O)-,
any one of $Q^3$ and $Q^4$ represents a sulfur atom, and the other represents a carbon atom,
$R^{607}$ to $R^{609}$, which may be the same or different, represent

(1) a hydrogen atom;
(2) a halogen atom;
(3) hydroxyl;
(6) C1-6 alkyl;
(9) C1-6 alkoxy;
(12) -CO-OR$^c$;
(13) -CO-NR$^d$R$^e$;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,
wherein these groups are optionally substituted as defined in formula (II),
$R^{622}$ to $R^{625}$, which may be the same or different, are selected from the group consisting of a hydrogen atom, a halogen atom, C1-4 alkyl, and C1-4 alkoxy, and
$R^{614}$ represents
unsubstituted C1-4 alkyl,
an optionally substituted unsaturated six-membered carbocyclic or heterocyclic group, or

an optionally substituted bicyclic saturated or unsaturated nine- or ten-membered carbocyclic or heterocyclic group.

In formula (600), preferably,
when $Q^3$ represents a sulfur atom, $Q^4$ represents a carbon atom, or
when $Q^3$ represents a carbon atom, $Q^4$ represents a sulfur atom.
In formula (600), when Z represents -O-, preferably, all of $R^{622}$ to $R^{625}$ represent a hydrogen atom.
In a preferred embodiment of the present invention, in formula (600), $R^{614}$ represents phenyl optionally substituted by hydroxyl, a halogen atom, amino group, C1-4 alkyl, or C1-4 alkoxy, more preferably phenyl optionally substituted by (i) hydroxyl, (ii) a halogen atom, (v') amino group, (vi') C1-2 alkyl, or (ix') C1-2 alkoxy, still more preferably unsubstituted phenyl.
In another preferred embodiment of the present invention, in formula (600), $R^{614}$ represents methyl or ethyl.
Preferably, $R^{607}$ and $R^{609}$ represent a hydrogen atom, a halogen atom, or C1-4 alkyl, more preferably a hydrogen atom, a halogen atom, or methyl, more preferably a hydrogen atom.
Preferably, $R^{608}$ represents
a hydrogen atom,
a halogen atom,
C1-4 alkyl,
a saturated or unsaturated six-membered carbocyclic group, or
a saturated or unsaturated six-membered heterocyclic group,
wherein the carbocyclic and heterocyclic groups are optionally substituted by (i) hydroxyl, (ii) a halogen atom, (v') amino group, (vi') C1-2 alkyl, or (ix') C1-2 alkoxy.
In formula (600), preferably, $R^{607}$ and $R^{609}$ represent a hydrogen atom, a halogen atom, or C1-4 alkyl
$R^{608}$ represents
a hydrogen atom,
a halogen atom,
C1-4 alkyl,
a saturated or unsaturated six-membered carbocyclic group, or
a saturated or unsaturated six-membered heterocyclic group,
wherein the carbocyclic and heterocyclic groups are optionally substituted by (i) hydroxyl, (ii) a halogen atom, (v') amino group, (vi') C1-2 alkyl, or (ix') C1-2 alkoxy.
In a preferred embodiment of the present invention, in formula (600),
$Q^3$ represents a sulfur atom,
$Q^4$ represents a carbon atom,
$R^{608}$ represents
a hydrogen atom,
C1-4 alkyl, or
a saturated or unsaturated six-membered carbocyclic or heterocyclic group,
wherein said carbocyclic or heterocyclic group is optionally substituted by (i) hydroxyl, (ii) a halogen atom, (v') amino group, (vi') C1-2 alkyl, or (ix') C1-2 alkoxy, and
$R^{609}$ represents a hydrogen atom. In this case, more preferably, $R^{608}$ represents a hydrogen atom or phenyl.
In a preferred embodiment of the present invention, in formula (600),
$Q^3$ represents a carbon atom,
$Q^4$ represents a sulfur atom, and
$R^{607}$ and $R^{608}$ are selected from the group consisting of a hydrogen atom and C1-4 alkyl. In this case, more preferably,
both $R^{607}$ and $R^{608}$ represent a hydrogen atom or
both $R^{607}$ and $R^{608}$ represent methyl or
$R^{607}$ represents methyl while $R^{608}$ represents a hydrogen atom.
More preferred compounds of the present invention include compounds described in Examples.
When the compounds of the present invention are compounds of formula (I), the compounds are preferably selected from the group consisting of compounds 1 to 27, 30, 31, 37 to 70, 73, 74, 81 to 179 and 181 to 225, more preferably compounds 1, 4, 6, 13, 16, 18, 27, 30, 37, 49, 50, 51, 56, 66, 103, 110, 117, 126, 133, 140 to 145, 155, 158, 159, 161, 162, 174 to 178, 181, 188, 192, 200, 202 and 205, still more preferably compounds 37, 142, 178, 181, 188, 192, 200, 202 and 205.
When the compounds of the present invention are compounds of formula (II), the compounds are preferably selected from the group consisting of compounds 28, 29, 32 to 35, 71, 72, 75 to 78 and 180, more preferably compounds 28, 32 and 33.

Salts or solvates of compounds

The compounds according to the present invention may form pharmaceutically acceptable salts thereof. Preferred examples of such salts include: alkali metal or alkaline earth metal salts such as sodium salts, potassium salts or calcium salts; hydrohalogenic acid salts such as hydrofluoride salts, hydrochloride salts, hydrobromide salts, or hydroiodide salts; inorganic acid salts such as nitric acid salts, perchloric acid salts, sulfuric acid salts, or phosphoric acid salts; lower alkylsulfonic acid salts such as methanesulfonic acid salts, trifluoromethanesulfonic acid salts, or ethanesulfonic acid salts; arylsulfonic acid salts such as benzenesulfonic acid salts or p-toluenesulfonic acid salts; organic acid salts such as fumaric acid salts, succinic acid salts, citric acid salts, tartaric acid salts, oxalic acid salts, maleic acid salts, acetic acid salts, malic acid salts, lactic acid salts, or ascorbic acid salts; and amino acid salts such as glycine salts, phenylalanine salts, glutamic acid salts, or aspartic acid salts.
The compounds according to the present invention may form solvates. Such solvates include, for example, hydrates, alcoholates, for example, methanolates and ethanolates, and etherates, for example, diethyl etherates.

Production of compounds according to the present invention

The compounds according to the present invention can be produced, for example, according to schemes 1 to 19. Quinoline derivatives or quinazoline derivatives of Reference Examples having structures similar to the compounds according to the present invention can be produced, for example, according to schemes r1 to r34 which will be described later. Further, the compounds according to the present invention may also be synthesized by conventional methods described, for example, in WO 2000/43366 and WO 2004/018430. In the present invention, starting compounds necessary for the synthesis of the compounds according to the present invention are commercially available or alternatively can be easily produced by conventional methods.

Scheme 1

Bipyridine derivatives as a partial structure of the compounds according to the present invention can be produced, for example, accoridng to scheme 1.

[Chemical formula 20]

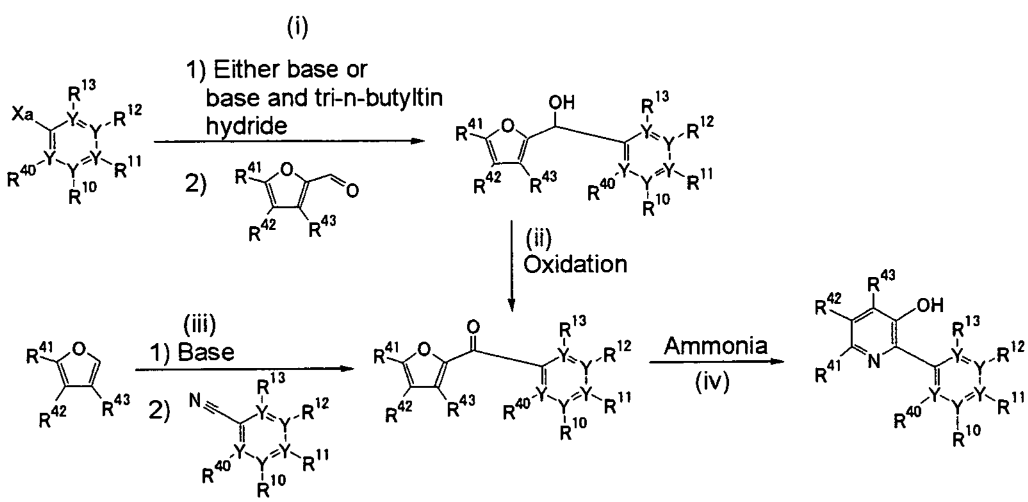


wherein
$R^{10}$ to $R^{13}$ are as defined above,
$R^{40}$ and Xa are selected from groups as defined in $R^{10}$, and
$R^{41}$ ro $R^{43}$ are selected from groups as defined in $R^{15}$, and
Y's each independently represent C or N.

In this scheme, the aimed compounds can be synthesized through the following two routes:

(I) A ketone derivative can be produced by reacting an allyl halogen derivative with a base, for example, n-butyllithium,

either directly or after conversion to a trialkyltin compound, for example, using tri-n-butyltin hydride, reacting the resultant compound with a furfural derivative (step (i)) to give an alcohol derivative, and then oxidizing the alcohol derivative with a suitable oxidizing agent, for example, manganese dioxide (step (ii)).

(II) A ketone derivative can be produced by reacting a furan derivative with an alkyllithium reagent, for example, n-butyllithium, and then reacting the resultant compound with an acylating agent (step (iii)).

The aimed compounds can be produced by reacting the ketone derivative produced in the above step (I) or (II) with ammonia (step (iv)).

Scheme 2

Naphthyridine derivatives as a partial structure of the compounds according to the present invention can be produced, for example, according to scheme 2.

[Chemical formula 21]

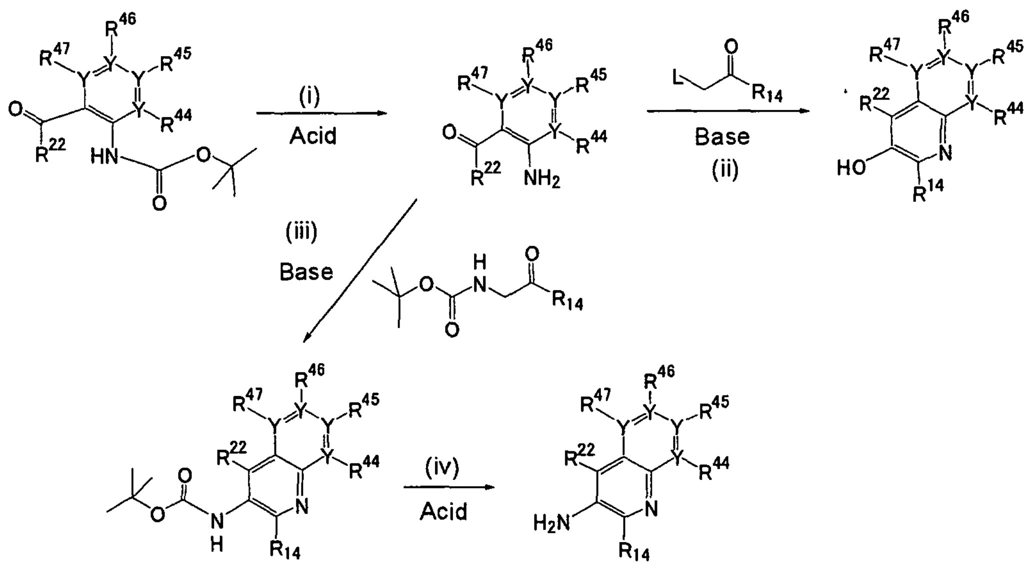


wherein

$R^{14}$ and $R^{22}$ are as defined above,

$R^{44}$ to $R^{47}$ are selected from groups defined in $R^{23}$, and

Y's each independently represent C or N.

The aimed 3-hydroxypyridine derivatives can be produced by deprotecting an aniline derivative in which the amino group has been protected (step (i)), and reacting the resultant o-acylaniline derivative with a methyl ketone derivative (step (ii)).

The aimed 3-aminopyridine derivatives can be produced by reacting the o-acylaniline derivative produced above with an aminomethyl ketone derivative in which the amino group has been protected (step (iii)), and then deprotecting the resultant compound, that is, removing the amino group from the resultant compound (step (iv)).

Schemes 3 and 4

The quinoline derivatives according to the present invention can be produced, for example, according to scheme 3 or 4.

Scheme 3:

[Chemical formula 22]

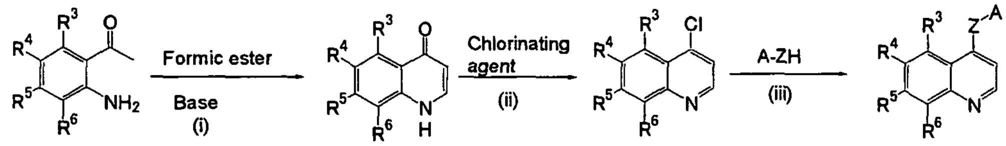


wherein the substituents are as defined above.

In scheme 3, the quinolone derivatives as an intermediate can be synthesized, for example, according to WO 97/17329. The 4- chloroquinoline derivatives can be synthesized by a conventional method described, for example, in Org. Synth. Col. Vol.3, 272 (1955), Acta Chim. Hung., 112, 241 (1983), or WO 98/47873. The aimed compounds can be synthesized by reacting the resultant 4- chloroquinoline derivative with a 3-hydroxypyridine derivative or a corresponding 3-aminopyridine derivative in a suitable solvent, for example, 1,2-dichlorobenzene, or in a system free from a solvent in the presence of a suitable base, for example, 4-dimethylaminopyridine (step (iii)).

Scheme 4:

[Chemical formula 23]

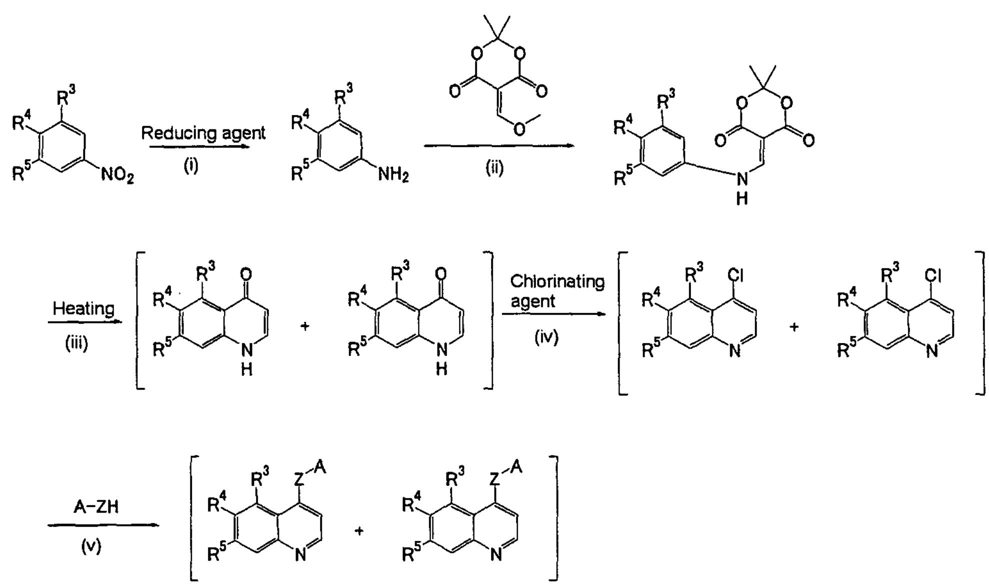


wherein the substituents are as defined above.

In scheme 4, the 4-chloroquinoline derivatives as an intermediate can be synthesized by a conventional method described, for example, in WO 00/50405. The aimed compounds can be synthesized by reacting the resultant 4-chloroquinoline derivative with a 3-hydroxypyridine derivative or a corresponding 3-aminopyridine derivative in a suitable solvent, for example, 1,2-dichlorobenzene, or in a system free from a solvent in the presence of a suitable base, for example, 4-dimethylaminopyridine (step (v)).

Scheme 5

The thiazopyridine derivatives according to the present invention can be produced, for example, according to scheme 5.

[Chemical formula 24]

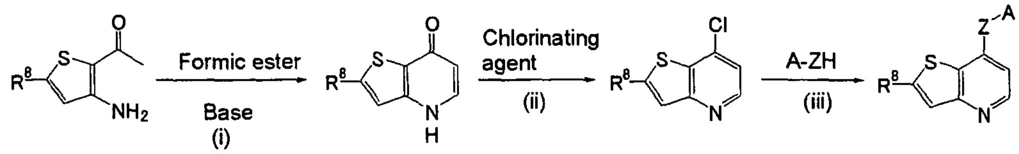


wherein the substituents are as defined above.

The aimed compounds can be synthesized by reacting an acetyl-amino-thiophene derivative with a formic ester in the presence of a suitable base, for example, sodium methoxide (step (i)), reacting the resultant thienopyridin-7-one derivative with a suitable chlorinating agent, for example, phosphorus oxychloride (step (ii)), and reacting the resultant 7-chlorothienopyridine derivative with a 3-hydroxypyridine derivative or a corresponding 3-aminopyridine derivative in a suitable solvent, for example, 1,2-dichlorobenzene, or in a solvent-free system in the presence of a suitable base, for example, 4-dimethylaminopyridine (step (iii)).

Scheme 6

The quinazoline derivatives according to the present invention can be produced, for example, according to scheme 6.

[Chemical formula 25]

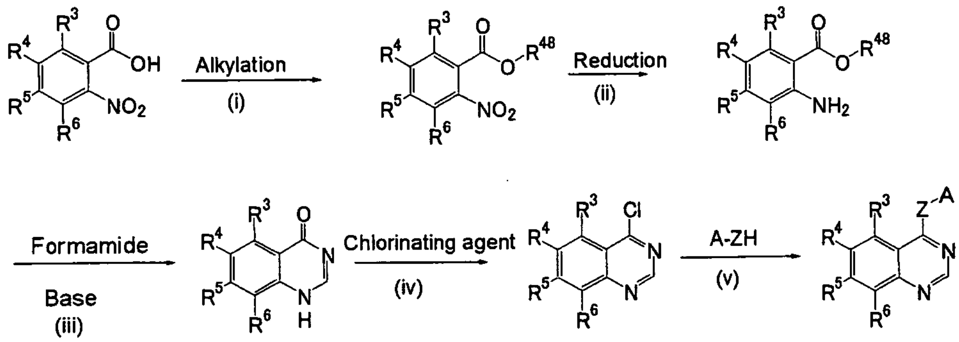


wherein
$R^{48}$ represents a group suitable for the cyclization reaction in step (iii), and
the other substituents are as defined above.

The 4-chloroquinazoline derivatives can be synthesized by a conventional method described, for example, in J. Am. Chem. Soc., 68, 1299 (1946), J. Am. Chem. Soc., 68, 1305 (1946), and Dai Yuki Kagaku (Comprehensive Organic Chemistry), Editor, Kotake, Vol. 17, p. 150, Asakura Publishing Co., Ltd. (published in 1967). The aimed compounds can be synthesized by reacting the resultant 4-chloroquinazoline derivative with a 3-hydroxypyridine derivative or a corresponding 3-aminopyridine derivative in a suitable solvent, for example, 1,2-dichlorobenzene, or in a solvent-free system, in the presence of a suitable base, for example, 4-dimethylaminopyridine (step (v)).

Scheme 7

The heterocondensed ring-type pyrimidine derivatives according to the present invevntion can be produced, for example, according to scheme 7.

[Chemical formula 26]

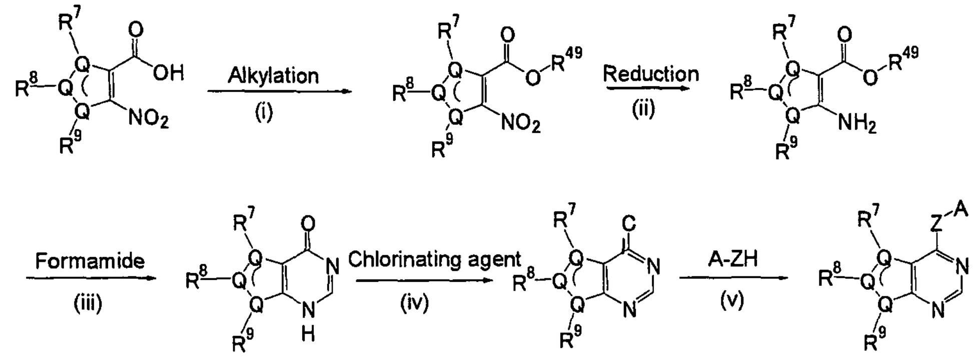


wherein
$R^{49}$ represents a group suitable for the cyclization reaction in step (iii),
Q's each independently represent C, S, or N, and
the other substituents are as defined above.

The aimed compounds can be produced by reacting an o-nitrocarboxylic acid derivative with a suitable alkylating agent, for example, methyl iodide (step (i)), reducing the nitro group in the resultant compound with a suitable reducing agent, for example, palladium hydroxide/a hydrogen gas) (step (ii)) to give an o-aminocarboxylic ester, reacting the o-aminocarboxylic ester with formamide (step (iii)), reacting the resultant compound with a suitable chlorinating agent, for example, phosphorus oxychloride (step (iv)), and reacting the resultant chloropyrimidine derivative with a 3-hydroxypyridine derivative or a corresponding 3-aminopyridine derivative in a suitable solvent, for example, 1,2-dichlorobenzene, or in a solvent-free system in the presence of a suitable base, for example, 4-dimethylaminopyridine (step (v)).

Scheme 8

The 1,5-naphthyridine derivatives according to the present invention can be produced, for example, according to scheme 9.

[Chemical formula 27]

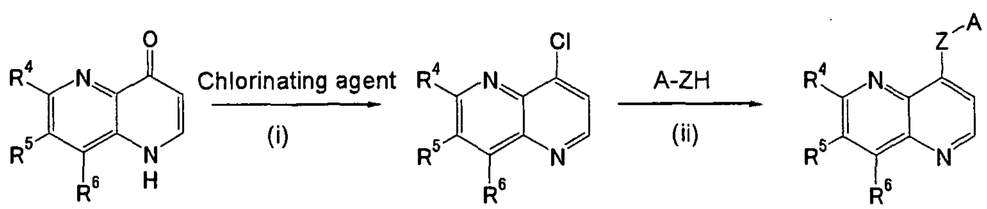


wherein the substituents are as defined above.

The 4-naphthyridinone derivatives can be synthesized in the same manner as shown, for example, in scheme 3 or 4. The aimed compounds can be synthesized by reacting the 4-naphthyridinone derivative with a suitable chlorinating agent, for example, phosphorus oxychloride (step (i)), and reacting the resultant chloronaphthyridine derivative with a 3-hydroxypyridine derivative or a corresponding 3-aminopyridine derivative in a suitable solvent, for example, 1,2-dichlorobenzene, or in a solvent-free system in the presence of a suitable base, for example, 4-dimethylaminopyridine (step (ii)).

Scheme 9

The 1,6-naphthyridine derivatives according to the present invention can be produced, for example, according to scheme 9.

[Chemical formula 28]

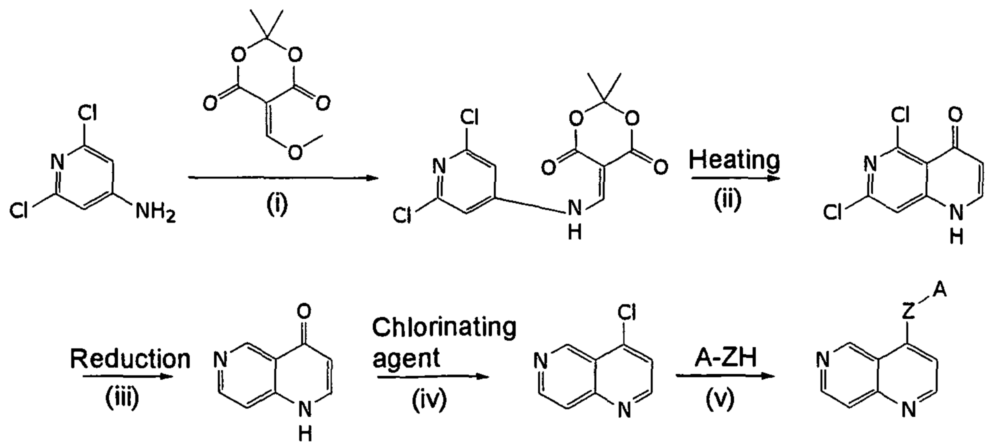


The aimed compounds can be synthesized by reacting an 4-amino-2,6-dichloropyridine with 5-methoxymethylene-2,2-dimethyl-[1,3]dioxan-4,6-dione (step (i)), further heating the mixture, for example, at 220°C for one hr (step (ii)), reducing the resultant compound with a suitable reducing agent, for example, palladium hydroxide/a hydrogen gas (step (iii)) to give a [1,6]naphthyridinone derivative, reacting the [1,6]naphthyridinone derivative with a suitable chlorinating agent, for example, phosphorus oxychloride (step (i)), and reacting the resultant chloronaphthyridine derivative with a 3-hydroxypyridine derivative or a corresponding 3-aminopyridine derivative in a suitable solvent, for example, 1,2-dichlorobenzene, or in a solvent-free system in the presence of a suitable base, for example, 4-dimethylaminopyridine (step (ii)).

Scheme 10

The 6-amidoquinoline derivatives according to the present invention can be produced, for example, according to scheme 10.

[Chemical formula 29]

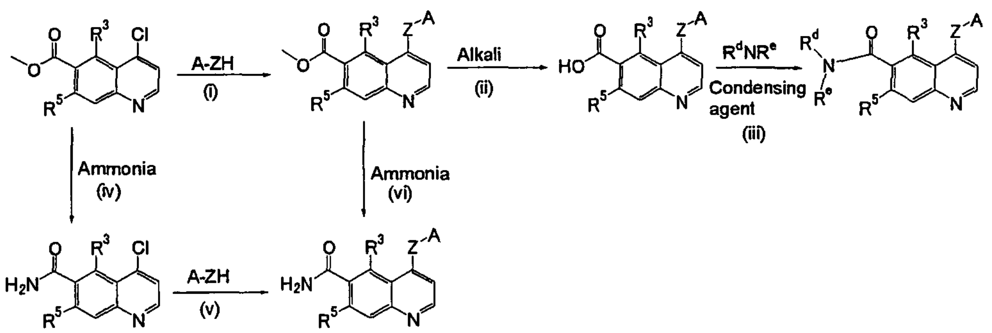


wherein the substituents are as defined above.

The N-monosubstituted or N-disubstituted amide derivatives can be produced by reacting the quinoline-6-carboxylic ester derivative produced according to scheme 3 or 4 with a 3-hydroxypyridine derivative or a corresponding 3-aminopyridine derivative in a suitable solvent, for example, 1,2-dichlorobenzene, or in a solvent-free system in the presence of a suitable base, for example, 4-dimethylaminopyridine (step (i)), subjecting the resultant compound to ester hydrolysis with a suitable alkali reagent, for example, lithium hydroxide (step (ii)), and condensing the resultant carboxylic acid derivative with an amine using a suitable condensing agent, for example, 1-ethyl-3-(3-dimethylaminopropyl)carbodiimide hydrochloride and 1-hydroxybenzotriazole hydrate (step (iii)).

The N-unsubstituted amide derivatives can be synthesized through the following two routes.

(I) The aimed compounds can be synthesized by reacting the compound produced in step (i) with ammonia (step (vi)).
(II) The aimed compounds can be synthesized by reacting the quinoline-6-carboxylic ester derivative with ammonia (step (iv)) and reacting the resultant compound with a 3-hydroxypyridine derivative or a corresponding 3-aminopyridine derivative or a corresponding 3-aminopyridine derivative (step (v)) in a suitable solvent, for example, 1,2-dichlorobenzene, or in a solvent-free system, in the presence of a suitable base, for example, 4-dimethylaminopyridine.

Scheme 11

The 6-phenylquinoline derivatives according to the present invention can be produced, for example, according to scheme 11.

[Chemical formula 30]

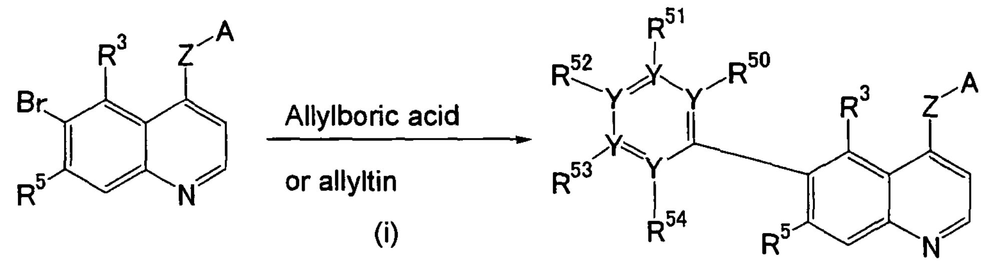


wherein
$R^{50}$ to $R^{53}$ represent (i) hydroxyl, (ii) a halogen atom, (iii) cyano group, (iv) nitro group, (v) amino group wherein one or two hydrogen atoms in the amino group are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-$OR^f$, or (xii) -CO-$NR^gR^h$ wherein $R^f$, $R^g$, and $R^h$, which may be the same or different, represent a hydrogen atom or C1-4 alkyl, and
the other substituents are as defined above.

The aimed compounds can be produced by reacting the 6-bromoquinoline derivative produced according to scheme 3 or 4 with an alkyltin reagent or an alkylboric acid reagent in the presence of a suitable transition metal catalyst, for example, tetrakistriphenylphosphine palladium (step (i)).

Scheme 12

The 7-phenylquinoline derivatives according to the present invention can be produced, for example, according to scheme 12.

[Chemical formula 31]

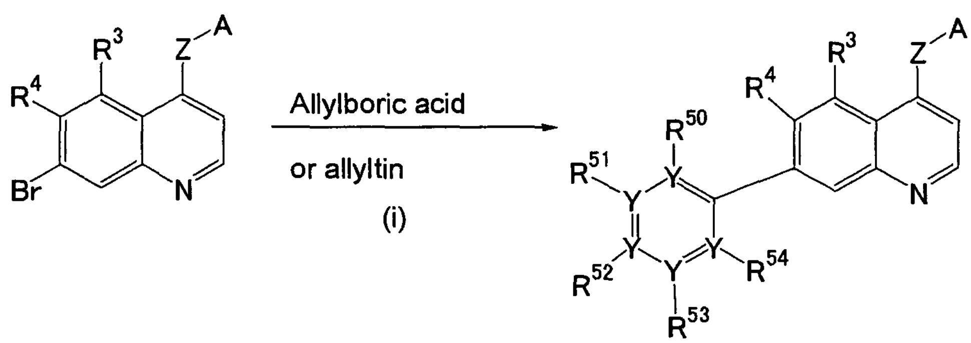


wherein the substituents are as defined above.

The aimed compounds can be produced by reacting the 7-bromoquinoline derivative produced according to scheme 3 or 4 with an alkyltin reagent or an alkylboric acid reagent in the presence of a suitable transition metal catalyst, for example, tetrakistriphenylphosphine palladium (step (i)).

Scheme 13

The 2-heteroring-naphthyridine derivatives according to the present invention can be produced, for example, according to scheme 13.

[Chemical formula 32]

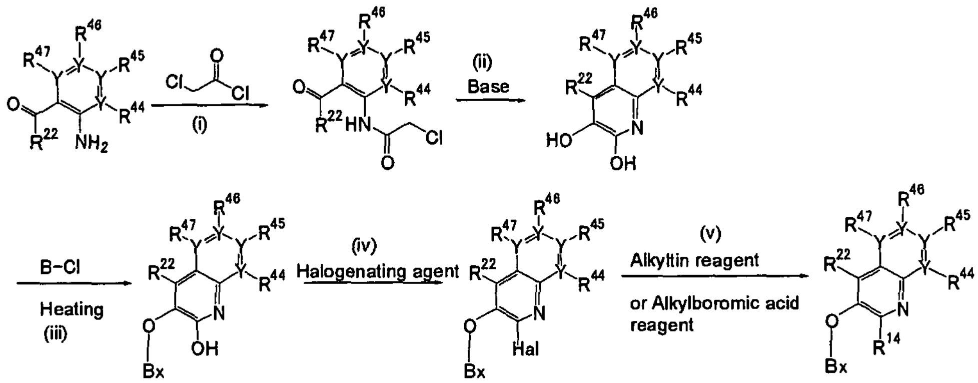


wherein
Hal represents a halogen atom,
Bx represents the compound produced in schemes 3 to 10, and
the other substituents are as defined above.

The aimed compounds can be produced by reacting an aniline derivative with chloroacetyl chloride (step (i)), then reacting the resultant amide derivative with a suitable base, for example, an aqueous potassium hydroxide solution (step (ii)) to give a 2,3-dihydroxypyridine derivative, reacting the 2,3-dihydroxypyridine derivative with the compound produced according to schemes 3 to 10 in a suitable solvent, for example, o-dichlorobenzene, or in a solvent-free system (step (iii)) to give a 2-hydroxyquinoline derivative, reacting the 2-hydroxyquinoline derivative with a suitable halogenating agent, for example, tetrabutylammonium bromide (step (iv)) to give a 2-haloquinoline derivative, and reacting the 2-haloquinoline derivative with an alkyltin reagent or an alkylboric acid reagent in the presence of a suitable transition metal catalyst, for example, tetrakistriphenylphosphine palladium (step (v)).

Scheme 14

The 7-amidoquinoline derivatives according to the present invention can be produced, for example, according to scheme 14.

[Chemical formula 33]

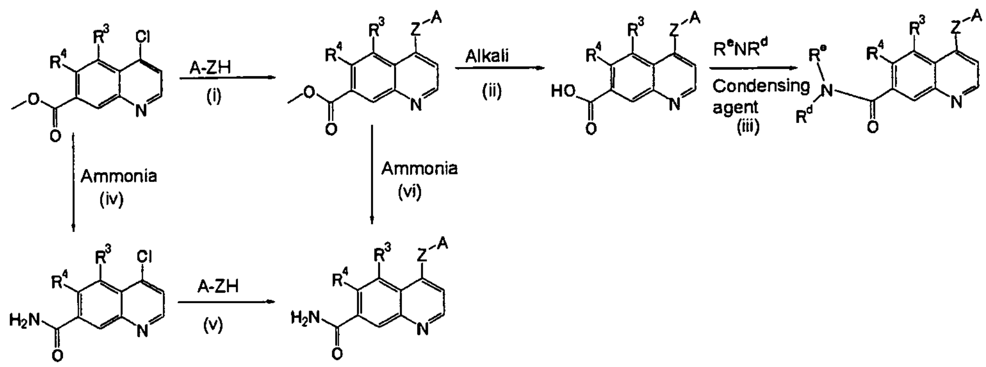


wherein the substituents are as defined above.]

The N-monosubstituted or N-disubstituted amide derivatives can be produced by reacting the quinoline-7-carboxylic ester derivative produced according to scheme 3 or 4 with a 3-hydroxypyridine derivative or a corresponding 3-aminopyridine derivative in a suitable solvent, for example, 1,2-dichlorobenzene, or in a solvent-free system in the presence of a suitable base, for example, 4-dimethylaminopyridine (step (i)), subjecting the resultant compound to ester hydrolysis with a suitable alkali reagent, for example, lithium hydroxide (step (ii)), and condensing the resultant carboxylic acid derivative with an amine using a suitable condensing agent, for example, 1-ethyl-3-(3-dimethylaminopropyl)carbodiimide hydrochloride and 1-hydroxybenzotriazole hydrate (step (iii)).

The N-unsubstituted amide derivatives can be synthesized through the following two routes.

(I) The aimed compounds can be synthesized by reacting the compound produced in step (i) with ammonia (step (vi)).
(II) The aimed compounds can be synthesized by reacting the quinoline-7-carboxylic ester derivative with ammonia (step (iv)) and reacting the resultant compound with a 3-hydroxypyridine derivative or a corresponding 3-aminopyridine derivative (step (v)) in a suitable solvent, for example, 1,2-dichlorobenzene, or in a solvent-free system, in the presence of a suitable base, for example, 4-dimethylaminopyridine.

Scheme 15

The 6-alkoxyquinoline derivatives or 7-alkoxyquinoline derivatives according to the present invention can be produced, for example, according to scheme 15.

[Chemical formula 34]

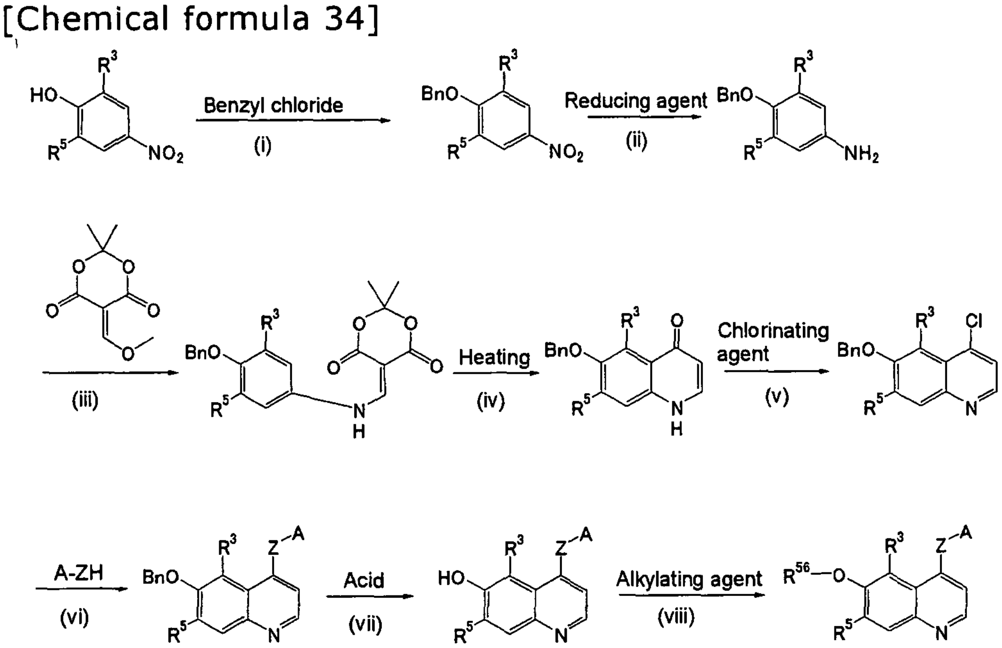

wherein
$R^{56}$ represents a substituent that can be selected when $-OR^{56}$ is provided in the definition of $R^4$, and
the other substituents are as defined above.

The aimed 6-alkoxyquinoline derivatives can be produced by reacting a 4-nitrophenol derivative with benzyl chloride in a suitable solvent, for example, N,N-dimethylformamide, in the presence of a suitable base, for example, potassium carbonate (step (i)), treating the resultant compound in the same manner as shown in scheme 4 (steps (ii) to (vi)) to give a 6-benzyloxyquinoline derivative, treating the resultant 6-benzyloxyquinoline derivative with a suitable acid, for example, methanesulfonic acid/trifluoroacetic acid (step (vii)) to remove the benzyl group for deprotection, and reacting the phenol compound thus obtained with an alkylating agent in a suitable solvent, for example, N,N-dimethylformamide, in the presence of a suitable base, for example, potassium carbonate (step (viii)).
The aimed 7-alkoxyquinoline derivatives can be produced via the same steps as described above, except that a 3-nitrophenol derivative is used as the starting compound.
This scheme was described taking 6-alkoxyquinoline derivatives as an example. However, 7-alkoxyquinoline derivatives can also be produced likewise.

Scheme 16

The 6-alkylamine derivatives according to the present invention can be produced, for example, according to scheme 16.

[Chemical formula 35]

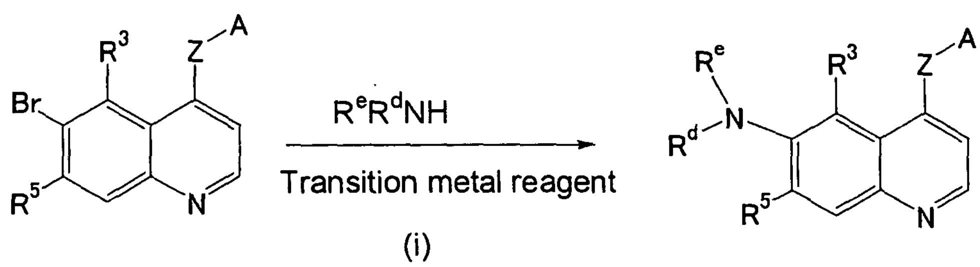


wherein the substituents are as defined above.

The aimed compounds can be produced by reacting the 6-bromoquinoline derivative produced according to scheme 3 or 4 with an alkylamine in the presence of a suitable transition metal catalyst, for example, palladium acetate, and a suitable base, for example, cesium carbonate (step (i)).

Scheme 17

The bipyridine derivatives as a partial structure of the compounds according to the present invention can be produced, for example, according to scheme 17.

[Chemical formula 36]

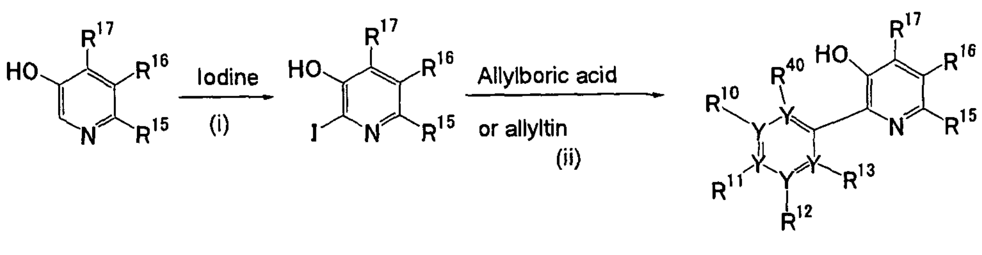


wherein the substituents are as defined above.

The aimed compounds can be produced by reacting a 3-hydroxypyridine derivative with iodine in a suitable solvent, for example, methanol/water (step (i)) and then reacting the resultant compound with an alkyltin reagent or an alkylboric acid reagent in the presence of a suitable transition metal catalyst, for example, tetrakistriphenylphosphine palladium (step (ii)).

Scheme 18

The quinoline derivatives substituted at the 6-position by amide or by an ester derivative according to the present invention can be produced, for example, according to scheme 18.

[Chemical formula 37]

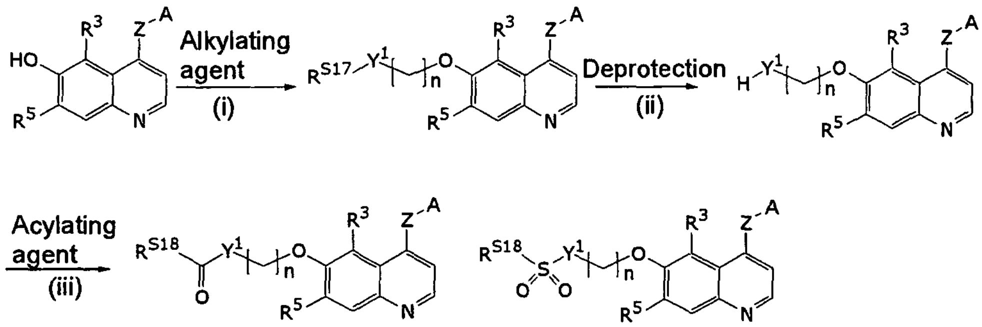


wherein the substituents are as defined above; $Y^1$ represents N or CH; and $R^{s17}$ and $R^{s18}$ represent C1-6 alkyl optionally substituted by amino.
The aimed compounds can be produced by reacting a quinolin-6-ol derivative with an alkylating agent having a protected functional group, for example, n-(2-bromoethyl)phthalimide, in the presence of a base, for example, potassium carbonate (step (i)), deprotecting the resultant compound under suitable conditions, for example, with hydrazine (step (ii)), and reacting the produced functional group with an acylating agent, for example, acetic anhydride, in the presence of a suitable base, for example, triethylamine (step (iii)).

Scheme 19

The derivatives having alkylamine at the 6-position according to the present invention can be produced, for example, according to scheme 19.

[Chemical formula 38]

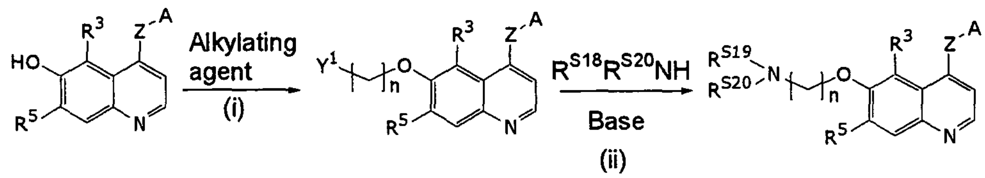


wherein the substituents are as defined above and $R^{s19}$ and $R^{s20}$ represent C1-4 alkyl or phenyl.
The aimed compounds can be produced by reacting a quinolin-6-ol derivative with an alkyl dihalide, for example, 1-bromo-2chloro-ethane, in the presence of a base, for example, potassium carbonate (step (i)) and reacting the resultant compound with an amine in the presence of a suitable base, for example, potassium carbonate (step (ii)).

Use of compounds/pharmaceutical composition

The compounds according to the present invention can inhibit the action of TGFβ on cells in vitro (see Test Example 1A).
Likewise, the quinoline derivatives or quinazoline derivatives of Reference Examples (that is, compounds r1 to r469) having structures close to the compounds according to the present invention can also inhibit the action of TGFβ on cells in vitro (see Test Example 1B). Therefore, a person having ordinary skill in the art could easily understand that naphthyridine derivatives, thienopyridine derivatives, and thienopyrimidine derivatives (falling within the scope of the present invention), which have the same substituents as the quinoline derivatives or quinazoline derivatives of Reference Examples and are different from the quinoline derivatives or quinazoline derivatives of Reference Examples only in the ring structure of the mother nucleus, can also inhibit the action of TGFβ on cells in vitro.
As described in the column of BACKGROUND ART, the inhibition of TGFβ has been regarded as effective for

the prevention or treatment of all of diseases involving the fibrosis including chronic renal diseases. Examples of documents showing the correlation between TGFβ and these diseases are as described in the column of BACKGROUND ART.

The compounds according to the present invention actually exhibited anti-fibrotic activity in vivo (see Test Example 7).
Further, the quinoline derivatives or quinazoline derivatives of the Reference Examples actually exhibited anti-fibrotic activity in vivo (see Test Examples 2 to 4). Accordingly, it would be apparent, from these Test Examples, to a person having ordinary skill in the art that the compounds according to the present invention can be used for the prevention or treatment of diseases for which TGFβ inhibition is effective therapeutically.

Furthermore, the compounds according to the present invention actually suppressed cell growth inhibitory action in a test using A549 cells (Test Example 6). Furthermore, the compounds according to the present invention actually suppressed BMP signal inhibitory action in vitro (Test Example 7).

It was found that some of the compounds according to the present invention are superior to the conventional compounds having TGF-β inhibitory activity in an improvement in pharmacokinetic profile, an improvement in selectivity for various kinases, or a reduction in cell growth inhibitory activity. For example, the selectivity for BMP signals is included in the selectivity for various kinases. BMP signals are deeply involved in the differentiation of osteoblasts, and it has recently been suggested that the genetic mutation of receptors (ALK1 and BMP type II receptors) related to the signals is deeply involved in the onset of hereditary hemorrhagic telangiectasia and primary pulmonary hypertension (J Soc Biol. 2002; 196(1): 53-8). Further, compounds, which strongly inhibit cell growth, are considered to be related directly to the development of toxicity in individuals. For example, compounds 178, 179, 181, 182, 184, 186, 187, 192, 194, 200 to 202, 204 to 206, 217, 222 and 225 may be mentioned as compounds for which the above improvement has been made.

More specifically, compounds 179, 181, 182, 184, 186, 187 and 205 had reduced inhibitory activity against cell growth or BMP4 signals as compared with Example 261 or Example 269 described in WO 03/00660. Likewise, compounds 192, 194, 200, 201, 202, 204, 206, 217, 222 and 225 had reduced inhibitory activity against cell growth of A549 cells and reduced inhibitory activity against BMP4 signals as compared with Example 274 described in WO 03/00660.

Accordingly, the compounds according to the present invention can be used for the prevention or treatment of diseases for which TGFβ inhibition is effective therapeutically. Further, the compounds according to the present invention can be used for ex vivo expansion of cells.

The term "TGFβ inhibition" as used herein means the inhibition of the intracellular or tissue activity of TGFβ, a kind of cytokine.
According to the present invention, there is provided a method for preventing or treating a disease for which TGFβ inhibition is effective therapeutically or prophylactically, said method comprising the step of administering a therapeutically or prophylactically effective amount of a compound according to the present invention to a patient. The term "patient" as used herein means a patient requiring the treatment or prevention of the disease for which TGFβ inhibition is effective therapeutically or prophylactically.
According to the present invention, there is provided use of the compounds according to the present invention, for the manufacture of a medicament used in the treatment or prevention of diseases for which TGFβ inhibition is effective therapeutically or prophylactically.

According to the present invention, there is provided a pharmaceutical composition, comprising a compound according to the present invention or a pharmaceutically acceptable salt or solvate thereof. The pharmaceutical composition according to the present invention can be used for the prevention or treatment of diseases for which TGFβ inhibition is effective therapeutically or prophylactically.
The disease for which TGFβ inhibition is effective therapeutically or prophylactically is preferably a disease involving organ or tissue fibrosis.
Further, diseases for which TGFβ inhibition is effective therapeutically or prophylactically include chronic renal disease, acute renal disease, hepatic fibrosis, cirrhosis, pulmonary fibrosis, scleroderma, wound healing, arthritis, congestive cardiac disease, ulcer, ocular disorder, corneal problem, diabetic nephropathy, peritoneal sclerosis, arteriosclerosis, peritoneal adhesions, and subdermal adhesion.
In another preferred embodiment of the present invention, the disease for which TGFβ inhibition is effective therapeutically or prophylactically is a malignant tumor.

In another preferred embodiment of the present invention, the compounds or pharmaceutical compositions according to the present invention can be used for ex vivo expansion of cells. The cells are preferably hematopoietic stem cells.
Accordingly, in a further preferred embodiment of the present invention, there is provided a method for ex vivo expansion of cells, comprising the step of adding a compound according to the present invention or a pharmaceutically acceptable salt or solvate thereof, in an amount effective for promoting cell growth, to intended cells in vitro to amplify the cells.
In another embodiment of the present invention, there is provided a promoter for ex vivo expansion of cells comprising

a compound according to the present invention or a pharmaceutically acceptable salt or solvate thereof.
In still another embodiment of the present invention, there is provided a TGFβ inhibitor comprising a compound according to the present invention or a pharmaceutically acceptable salt or solvate thereof.

The compounds according to the present invention can be administered to human and non-human animals orally or parenterally by administration routes, for example, intravenous administration, intramuscular administration, subcutaneous administration, rectal administration, or percutaneous administration.
Therefore, the pharmaceutical composition comprising a compound according to the present invention may be formulated into suitable dosage forms according to the administration routes. Specifically, oral preparations include tablets, capsules, powders, granules, and syrups, and parenteral preparations include injections, suppositories, tapes, and ointments.
These various preparations may be prepared by conventional methods, for example, with pharmaceutically acceptable carriers, that is, commonly used excipients, disintegrants, binders, lubricants, colorants, and diluents.
Excipients include, for example, lactose, glucose, corn starch, sorbit, and crystalline cellulose. Disintegrants include, for example, starch, sodium alginate, gelatin powder, calcium carbonate, calcium citrate, and dextrin. Binders include, for example, dimethylcellulose, polyvinyl alcohol, polyvinyl ether, methylcellulose, ethylcellulose, gum arabic, gelatin, hydroxypropylcellulose, and polyvinyl pyrrolidone. Lubricants include, for example, talc, magnesium stearate, polyethylene glycol, and hydrogenated vegetable oils.
In preparing the above injections, if necessary, for example, buffering agents, pH adjustors, stabilizers, tonicity adjusting agents, and preservatives may be added.

The content of a compound according to the present invention in the pharmaceutical composition according to the present invention may vary according to the dosage form. The content, however, is generally 0.5 to 50% by weight, preferably 1 to 20% by weight, based on the whole composition.
The dose may be appropriately determined in consideration of particular conditions, for example, the age, weight, sex, type of disease, and severity of condition of patients. The preparation may be administered, for example, in an amount of 0.1 to 100 mg/kg, preferably 0.1 to 30 mg/kg. This dose may be administered at a time daily or divided doses of several times daily.
Compounds according to the present invention may be administered in combination with other medicaments, for example, carcinostatic agents. The adminstration can be carried out simultaneously or sequentially. The type, administration interval and the like of the carcinostatic agent can be determined by taking into consideration the type of cancer and the conditions of the patient.

When the compounds according to the present invention are used in the ex vivo expansion fo cells, a suitable medium may be selected or prepared depending upon the type of cells and the like. The amount of the compounds according to the present invention added to the medium may be properly determined depending, for example, upon the type and applications of the cells. The 0 amount is preferably 0.01 to 50 μM, more preferably 0.1 to 20 μM.
In another embodiment of the present invention, there is provided a method for inhibiting the action of TGFβ on cells, comprising the step of applying an effective amount of a compound according to the present invention to cells present in vitro or in vivo.

[EXAMPLES]

The present invention is further illustrated by the following Examples. However, it should be noted that embodiments described in the Examples are not intended as a limitation of the invention.

The compounds according to the present invention were produced as follows.
The relationship between the produced compounds and schemes applied to the production of the compounds are shown in the following table.

| Scheme | Compound |
|---|---|
| 1 | : Partial structure of compounds 1 to 51 Synthesis of partial structures of compounds 103 to 108, 110, 111, 117, 119 to 121, 126, 130, 131, 133, 136 to 146, 154 to 159 and 162 to 177 |
| 2 | : Partial structure of compounds 52 to 100 Synthesis of partial structures of compounds 101, 102, 113, 114, 116, 118, 123 to 125, 127, 128, 132, 134, 135, 147 to 153, 160 and 161 |
| 3 | : Compounds 1, 3, 13, 15, 37, 43 to 46, 49, 50, 52 to 54, 81 to 91, 93 to 98, 103, 111, 115, 117, 134, 135, 156, 157 and 188 |
| 4 | : Compounds 2, 4, 14, 16, 55 to 63, 101, 102, 104, 105, 110, 114, 119, 130 to 133, 150, 158 to 162 and 170 |
| 5 | : Compounds 28, 29, 71 and 72 |
| 6 | : Compounds 23 to 27, 38, 39, 41, 48, 51, 68 to 70 and 100 |
| 7 | : Compounds 32 to 35, 75 to 78 and 180 |
| 8 | : Compounds 30, 73, 121 and 128 |

(continued)

| Scheme | Compound |
|---|---|
| 9 | : Compounds 31 and 74 |
| 10 | : Compounds 22, 65, 66, 99, 146, 148, 149, 152 and 153 |
| 11 | : Compounds 5 to 12 |
| 12 | : Compounds 17 to 21, 106 to 108, 155, 163 to 169 and 171 to 177 |
| 13 | : Compound 92 |
| 14 | : Compounds 113, 116, 126, 127, 145, 147, 151, 179, 185, 193, 194 and 217 |
| 15 | : Compounds 118, 120, 123 to 125, 136 to 144, 178, 181, 183, 184, 186, 190 to 192, 200 to 202, 205, 210, 213 to 216, 222 and 225 |
| 16 | : Compound 154 |
| 17 | : Synthesis of partial structure of Compound 115 |
| 18 | : Compounds 182, 187, 203, 204, 206 to 208 and 224 |
| 19 | : Compounds 189, 209, 211, 212, 218 to 221 and 223 |

Example 1: 5,6-Dimethyl-3-(quinolin-4-yloxy)-[2,2']bipyridine (compound 1)

2,3-Dimethylfuran (5.0 g) was dissolved in diethyl ether (75 ml) under an argon atmosphere. A 1.6 M n-butyllithium/hexane solution (35.7 ml) was added dropwise to the solution at 0˚C, and the mixture was stirred under reflux for 2.5 hr. The mixture was then cooled to -78˚C, 2-cyanopyridine (6.0 g) dissolved in diethyl ether (20 ml) was added dropwise thereto, and the mixture was stirred at room temperature for 3 hr. The reaction mixture was poured into ice to stop the reaction and was adjusted to pH 5 by the addition of 2 M hydrochloric acid, followed by extraction with chloroform. The chloroform layer was washed with water and was dried over anhydrous sodium sulfate. The solvent was removed by distillation under the reduced pressure, and the residue was purified by column chromatography with a hexane-ethyl acetate system to give (4,5-dimethylfuran-2-yl)-(2-pyridyl)-methanone (1.8 g, yield 17%). (4,5-Dimethylfuran-2-yl)-(2-pyridyl)-methanone (1.6 g), methanol (15 ml), and a 28% aqueous ammonia solution (15 ml) were placed in a sealed tube, and the mixture was stirred at 160˚C overnight. The reaction mixture was cooled to room temperature, and the solvent was removed by distillation under the reduced pressure. The residue was purified by column chromatography with a hexane-ethyl acetate system to give 5,6-dimethyl-[2,2']bipyridinyl-3-ol (1.2 g, yield 75%).

Dimethyl sulfoxide (1.5 ml) was added to 5,6-dimethyl-[2,2']bipyridinyl-3-ol (30 mg), 4-chloroquinoline (74 mg), and cesium carbonate (147 mg), and the mixture was stirred at 130˚C for 7 hr. The reaction mixture was cooled to room temperature, and water was added to the cooled mixture. The mixture was extracted with ethyl acetate. The ethyl acetate layer was washed with water and was dried over anhydrous sodium sulfate. The solvent was removed by distillation under the reduced pressure, and the residue was purified by thin layer chromatography with an ethyl acetate system to give the title compound (21 mg, yield 43%).

$^1$H-NMR ($CDCl_3$, 400 MHz): δ 2.39 (s, 3H), 2.67 (s, 3H), 6.44 (d, J = 5.2 Hz, 1H), 7.10 (ddd, J = 7.6, 4.9, 1.0 Hz, 1H), 7.37 (s, 1H), 7.53 - 7.60 (m, 2H), 7.73 (ddd, J = 8.3, 6.8, 1.5 Hz, 1H), 7.82 (d, J = 8.0 Hz, 1H), 8.05 (d, J = 8.6 Hz, 1H), 8.35 (dd, J = 8.3, 1.4 Hz, 1H), 8.48 - 8.53 (m, 1H), 7.57 (d, J = 5.2 Hz, 1H)

Mass spectrometric value (ESI-MS, m/z): 328 $(M+1)^+$

Example 2: 3-(6-Bromo-quinolin-4-yloxy)-5,6-dimethyl-[2,2']bipyridine (compound 2)

Diphenyl ether (80 ml) was added to 4-bromoaniline (4.5 g) and 5-(methoxymethylene)-2,2-dimethyl-1,3-dioxan-4,6-dione (5.4 g), and the mixture was stirred at 80˚C for one hr. Biphenyl (24.2 g) was added thereto, and the mixture was stirred at 220˚C for 3 hr. The reaction mixture was cooled to room temperature, and diethyl ether was added to the cooled mixture. The precipitated crystal was collected by filtration and was washed with diethyl ether. The residue was purified by column chromatography with a hexane-acetone system to give 6-bromoquinolone (1.57 g, yield 27%).

Thionyl chloride (5 ml) and a minor amount of dimethylformamide were added to 6-bromoquinolone (1.6 g), and the mixture was stirred under reflux for 3 hr. The reaction mixture was added to a saturated aqueous sodium bicarbonate solution under ice cooling, and the mixture was extracted with ethyl acetate. The ethyl acetate layer was washed with water and was dried over anhydrous sodium sulfate. The solvent was removed by distillation under the reduced pressure, and the residue was purified by column chromatography with a hexane-ethyl acetate system to give 6-bromo-4-chloroquinoline (1.43 g, yield 85%).

Dimethylsulfoxide (25 ml) was added to 5,6-dimethyl-[2,2']bipyridinyl-3-ol (500 mg), 6-bromo-4-chloroquinoline (723 mg), cesium carbonate (2.4 g), and 4-dimethylaminopyridine (916 mg), and the mixture was stirred at 130˚C for 5 hr. The reaction mixture was cooled to room temperature, water was added to the cooled reaction mixture, and the mixture was extracted with ethyl acetate. The ethyl acetate layer was washed with water and was dried over anhydrous

sodium sulfate. The solvent was removed by distillation under the reduced pressure, and the residue was purified by column chromatography with a chloroform-acetone system to give the title compound (886 mg, yield 87%).
$^1$H-NMR ($CDCl_3$, 400 MHz): δ 2.40 (s, 3H), 2.66 (s, 3H), 6.44 (d, J = 5.1 Hz, 1H), 7.11 (ddd, J = 7.6, 4.6, 1.0 Hz, 1H), 7.36 (s, 1H), 7.61 (ddd, J = 7.6, 7.6, 1.7 Hz, 1H), 7.79 (dd, J = 8.8, 2.2 Hz, 1H), 7.84 (d, J = 8.0 Hz, 1H), 7.91 (d, J = 9.0 Hz, 1H), 8.42 - 8.47 (m, 1H), 8.52 (d, J = 2.2 Hz, 1H), 8.55 (d, J = 5.4 Hz, 1H)
Mass spectrometric value (ESI-MS, m/z): 406 $(M+1)^+$

Example 3: 5,6-Dimethyl-3-(6-trifluoromethyl-quinolin-4-yloxy)-[2,2']bipyridine (compound 3)

4-Chloro-6-trifluoromethylquinoline (50 mg), 5,6-dimethyl-[2,2']bipyridinyl-3-ol (43 mg), and 4-dimethylaminopyridine (79 mg) were dissolved in dimethylsulfoxide (1 ml). Cesium carbonate (211 mg) was added to the solution, and the mixture was stirred at 130˚C overnight. The mixture was cooled to room temperature, an aqueous sodium hydrogencarbonate solution was added to the reaction mixture, and the organic layer was extracted with chloroform. The chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (77 mg, yield 89%).
$^1$H-NMR ($CDCl_3$, 400 MHz): δ 2.42 (s, 3H), 2.68 (s, 3H), 6.57 (d, J = 5.6 Hz, 1H), 7.09 (ddd, J = 1.0, 4.9, 7.6 Hz, 1H), 7.41 (s, 1H), 7.63 (m, 1H), 7.95 - 7.97 (m, 2H), 8.28 (m, 2H), 8.67 (d, J = 5.4 Hz, 1H), 8.73 (s, 1H)
Mass spectrometric value (ESI-MS, m/z): 418 $(M+Na)^+$

Example 4: 3-(6-Methoxy-quinolin-4-yloxy)-5,6-dimethyl-[2,2']bipyridine (compound 4)

4-Methoxyaniline (1.27 g) and 5-methoxymethylene-2,2-dimethyl-[1,3]dioxan-4,6-dione (1.82 g) were dissolved in 2-propanol (40 ml), and the solution was stirred at 50˚C for 2 hr. The solvent was removed by distillation under the reduced pressure, and the residue was washed with ether to give 5-[(4-methoxy-phenylamino)-methylene]-2,2-dimethyl-[1,3]dio xan-4,6-dione (1.98 g, yield 73%).
5-[(4-Methoxy-phenylamino)-methylene]-2,2-dimet hyl-[1,3]dioxan-4,6-dione (1.28 g) and biphenyl (5.2 g) were suspended in diphenyl ether (20 ml), and the suspension was stirred at 220˚C for one hr. The reaction mixture as such was purified by column chromatography with a methanol-chloroform system to give 6-methoxy-1H-quinolin-4-one (398 mg, yield 49%).
6-Methoxy-1H-quinolin-4-one (398 mg) was suspended in diisopropylethylamine (3 ml), phosphorus oxychloride (1 ml) was added to the suspension, and the mixture was stirred at 100˚C for one hr. Water was added to the reaction mixture under ice cooling, and the aqueous layer was neutralized with an aqueous sodium hydrogencarbonate solution. The organic layer was extracted with ethyl acetate, and the ethyl acetate layer was washed with water and was dried over anhydrous sodium sulfate. The solvent was removed by distillation under the reduced pressure, and the residue was purified by column chromatography with an acetone-chloroform system to give 4-chloro-6-methoxyquinoline (375 mg, yield 42%).
4-Chloro-6-methoxyquinoline (270 mg), 5,6-dimethyl-[2,2']bipyridinyl-3-ol (279 mg), and 4-dimethylaminopyridine (510 mg) were dissolved in dimethylsulfoxide (4 ml), cesium carbonate (1.36 g) was added to the solution, and the mixture was stirred at 130˚C overnight. The mixture was cooled to room temperature, an aqueous sodium hydrogencarbonate solution was added to the reaction mixture, and the organic layer was extracted with chloroform. The chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (411 mg, yield 83%).
$^1$H-NMR ($CDCl_3$, 400 MHz): δ 2.39 (s, 3H), 2.66 (s, 3H), 3.95 (s, 3H), 6.45 (dd, J = 0.7, 5.1 Hz, 1H), 7.11 (ddd, J = 1.2, 4.9, 7.6 Hz, 1H), 7.37 (s, 1H), 7.39 (m, 1H), 7.57 - 7.61 (m, 2H), 7.82 (dd, J = 1.0, 7.8 Hz, 1H), 7.99 (d, J = 9.3 Hz, 1H), 8.44 (d, J = 5.1 Hz, 1H), 8.50 (d, J = 4.1 Hz, 1H)
Mass spectrometric value (ESI-MS, m/z): 380 $(M+Na)^+$

Example 5: 5,6-Dimethyl-3-(6-phenyl-quinolin-4-yloxy)-[2,2']bipyridine (compound 5)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (30 mg), tetrakistriphenylphosphine palladium (9 mg), and phenylboric acid (27 mg) under an argon atmosphere, and the mixture was stirred at 70˚C for 3 hr. The reaction mixture was cooled to room temperature, and water was added to the cooled mixture. The mixture was extracted with ethyl acetate, the ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent

was removed by distillation under the reduced pressure, and the residue was purified by column chromatography with a chloroform-acetone system to give the title compound (31 mg, yield 100%).
$^1$H-NMR ($CDCl_3$, 400 MHz): δ 2.39 (s, 3H), 2.67 (s, 3H), 6.47 (d, J = 5.1 Hz, 1H), 7.09 (ddd, J = 7.3, 4.9, 1.0 Hz, 1H), 7.37 - 7.43 (m, 2H), 7.50 (dd, J = 7.8, 7.8 Hz, 2H), 7.56 (ddd, J = 7.8, 7.8, 2.0 Hz, 1H), 7.76 (d, J = 7.3 Hz, 2H), 7.83 (d, J = 8.0 Hz, 1H), 8.01 (dd, J = 9.0, 2.2 Hz, 1H), 8.11 (d, J = 8.8 Hz, 1H), 8.48 - 8.51 (m, 1H), 8.54 - 8.59 (m, 2H)
Mass spectrometric value (ESI-MS, m/z): 404 $(M+1)^+$

Example 6: 5,6-Dimethyl-3-(6-pyridin-3-yl-quinolin-4-yloxy)-[2,2']bipyridine (compound 6)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (30 mg), tetrakistriphenylphosphine palladium (9 mg), and 3-pyridylboric acid (18 mg) under an argon atmosphere, and the mixture was stirred at 70°C for 3 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a chloroform-acetone system to give the title compound (30 mg, yield 100%).
$^1$H-NMR ($CDCl_3$, 400 MHz): δ 2.41 (s, 3H), 2.67 (s, 3H), 6.49 (d, J = 5.4 Hz, 1H), 7.09 (ddd, J = 7.6, 4.9, 1.0 Hz, 1H), 7.38 - 7.46 (m, 2H), 7.59 (ddd, J = 7.8, 7.8, 2.0 Hz, 1H), 7.85 (ddd, J = 8.0, 1.2, 1.2 Hz, 1H), 7.98 (dd, J = 8.8, 2.2 Hz, 1H), 8.02 - 8.07 (m, 1H), 8.16 (d, J = 8.8 Hz, 1H), 8.43 - 8.47 (m, 1H), 8.57 (d, J = 2.0 Hz, 1H), 8.60 (d, J = 5.1 Hz, 1H), 8.65 (dd, J = 4.9, 1.7 Hz, 1H), 9.00 (d, J = 1.7 Hz, 1H)
Mass spectrometric value (ESI-MS, m/z): 427 $(M+Na)^+$

Example 7: 5,6-Dimethyl-3-(6-pyridin-4-yl-quinolin-4-yloxy)-[2,2']bipyridine (compound 7)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (30 mg) and tetrakistriphenylphosphine palladium (9 mg) and 4-pyridylboric acid (18 mg) under an argon atmosphere, and the mixture was stirred at 70°C overnight. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a chloroform-acetone system to give the title compound (16 mg, yield 55%).
$^1$H-NMR ($CDCl_3$, 400 MHz): δ 2.41 (s, 3H), 2.67 (s, 3H), 6.49 (d, J = 5.4 Hz, 1H), 7.09 (ddd, J = 7.6, 4.9, 1.2 Hz, 1H), 7.41 (s, 1H), 7.59 (ddd, J = 7.6, 7.6, 1.7 Hz, 1H), 7.68 (dd, J = 4.6, 1.7 Hz, 2H), 7.86 (d, J = 8.0 Hz, 1H), 8.02 (dd, J = 8.8, 2.2 Hz, 1H), 8.16 (d, J = 8.8 Hz, 1H), 8.40 - 8.45 (m, 1H), 8.61 (d, J = 5.1 Hz, 1H), 8.66 (d, J = 2.2 Hz, 1H), 8.73 (dd, J = 6.4, 1.7 Hz, 2H)
Mass spectrometric value (ESI-MS, m/z): 405 $(M+1)^+$

Example 8: 5,6-Dimethyl-3-(6-p-tolyl-quinolin-4-yloxy)-[2,2']bipyridine (compound 8)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (30 mg), tetrakistriphenylphosphine palladium (9 mg), and 4-methylphenylboric acid (30 mg) under an argon atmosphere and the mixture was stirred at 70°C for 3 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a chloroform-acetone system to give the title compound (31 mg, yield 100%).
$^1$H-NMR ($CDCl_3$, 400 MHz): δ 2.39 (s, 3H), 2.43 (s, 3H), 2.67 (s, 3H), 6.46 (d, J = 5.1 Hz, 1H), 7.09 (ddd, J = 7.6, 4.9, 1.2 Hz, 1H), 7.30 (d, J = 7.8 Hz, 2H), 7.39 (s, 1H), 7.56 (ddd, J = 7.8, 7.8, 1.7 Hz, 1H), 7.66 (d, J = 8.0 Hz, 2H), 7.83 (d, J = 7.8 Hz, 1H), 8.00 (dd, J = 8.8, 2.0 Hz, 1H), 8.10 (d, J = 8.8 Hz, 1H), 8.49 - 8.54 (m, 2H), 8.55 (d, J = 5.1 Hz, 1H)
Mass spectrometric value (ESI-MS, m/z): 418 $(M+1)^+$

Example 9: 2-[4-(5,6-Dimethyl-[2,2']bipyridin-3-yloxy)-quinolin-6-yl]-phenylamine (compound 9)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (30 mg), tetrakistriphenylphosphine palladium (9 mg), and 2-aminophenylboric acid (30 mg) under an argon atmosphere, and the mixture was stirred at 70˚C for 3 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a chloroform-acetone system to give the title compound (31 mg, yield 100%).
$^1$H-NMR ($CDCl_3$, 400 MHz): δ 2.39 (s, 3H), 2.66 (s, 3H), 3.83 (brs, 2H), 6.47 (d, J = 5.1 Hz, 1H), 6.82 (dd, J = 8.0, 1.0 Hz, 1H), 6.88 (ddd, J = 8.6, 8.6, 1.2 Hz, 1H), 7.08 - 7.13 (m, 1H), 7.18 - 7.26 (m, 2H), 7.37 (s, 1H), 7.57 (ddd, J = 7.8, 7.8, 1.7 Hz, 1H), 7.82 (ddd, J = 8.1, 8.1, 1.2 Hz, 1H), 7.87 (dd, J = 8.8, 2.0 Hz, 1H), 8.11 (d, J = 8.8 Hz, 1H), 8.43 (d, J = 2.0 Hz, 1H), 8.49 - 8.52 (m, 1H), 8.59 (d, J = 5.2 Hz, 1H)
Mass spectrometric value (ESI-MS, m/z): 441 $(M+Na)^+$

Example 10: 3-[4-(5,6-Dimethyl-[2,2']bipyridin-3-yloxy)-quinolin-6-yl]-phenylamine (compound 10)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (30 mg), tetrakistriphenylphosphine palladium (9 mg), and 3-aminophenylboric acid monohydrate (34 mg) under an argon atmosphere, and the mixture was stirred at 70˚C overnight. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate, and the ethyl acetate layer was washed with water and was then dried over anhydrous sodium sulfate. The solvent was removed by distillation under the reduced pressure, and the residue was purified by column chromatography with a chloroform-acetone system to give the title compound (31 mg, yield 100%).
$^1$H-NMR ($CDCl_3$, 400 MHz): δ 2.40 (s, 3H), 2.67 (s, 3H), 3.80 (brs, 2H), 6.45 (d, J = 5.1 Hz, 1H), 6.70 - 6.76 (m, 1H), 7.02 - 7.17 (m, 3H), 7.26 - 7.31 (m, 1H), 7.38 (s, 1H), 7.57 (ddd, J = 7.8, 7.8, 1.7 Hz, 1H), 7.83 (d, J = 8.0 Hz, 1H), 7.98 (dd, J = 8.8, 2.0 Hz, 1H), 8.09 (d, J = 8.8 Hz, 1H), 8.47 - 8.54 (m, 2H), 8.56 (d, J = 5.4 Hz, 1H)
Mass spectrometric value (ESI-MS, m/z): 441 $(M+Na)^+$

Example 11: 3-[4-(5,6-Dimethyl-[2,2']bipyridin-3-yloxy)-quinolin-6-yl]-phenol (compound 11)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (30 mg), tetrakistriphenylphosphine palladium (9 mg) and 3-hydroxyphenylboric acid (31 mg) under an argon atmosphere, and the mixture was stirred at 70˚C for 3 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a chloroform-acetone system to give the title compound (31 mg, yield 100%).
$^1$H-NMR ($CDCl_3$, 400 MHz): δ 2.33 (s, 3H), 2.61 (s, 3H), 6.40 (d, J = 5.1 Hz, 1H), 6.77 - 6.84 (m, 1H), 7.06 (ddd, J = 7.6, 4.9, 1.0 Hz, 1H), 7.18 - 7.34 (m, 4H), 7.55 (ddd, J = 7.8, 7.8, 2.0 Hz, 1H), 7.79 (ddd, J = 8.0, 1.0, 1.0 Hz, 1H), 7.93 (dd, J = 9.0, 2.2 Hz, 1H), 8.09 (d, J = 8.8 Hz, 1H), 8.42 - 8.47 (m, 1H), 8.53 (d, J = 5.4 Hz, 1H), 8.57 (d, J = 2.2 Hz, 1H)
Mass spectrometric value (ESI-MS, m/z): 420 $(M+1)^+$

Example 12: 4-[4-(5,6-Dimethyl-[2,2']bipyridin-3-yloxy)-quinolin-6-yl]-phenol (compound 12)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (30 mg), tetrakistriphenylphosphine palladium (9 mg), and 4-hydroxyphenylboric acid (31 mg) under an argon atmosphere, and the mixture was stirred at 70˚C for 3 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate.

The solvent was removed by distillation under the reduced pressure, and the residue was purified by column chromatography with a chloroform-acetone system to give the title compound (31 mg, yield 100%).
$^1$H-NMR ($CDCl_3$, 400 MHz): δ 2.38 (s, 3H), 2.65 (s, 3H), 6.46 (d, J = 5.1 Hz, 1H), 6.95 (d, J = 8.5 Hz, 2H), 7.11 (ddd, J = 7.6, 4.9, 1.2 Hz, 1H), 7.39 (s, 1H), 7.54 - 7.64 (m, 3H), 7.85 (d, J = 8.1 Hz, 1H), 7.95 (dd, J = 8.8, 2.0 Hz, 1H), 8.09 (d, J = 8.8 Hz, 1H), 8.48 - 8.51 (m, 2H), 8.54 (d, J = 5.1 Hz, 1H)
Mass spectrometric value (ESI-MS, m/z): 420 $(M+1)^+$

Example 13: 3-(7-Chloro-quinolin-4-yloxy)-5,6-dimethyl-[2,2']bipyridine (compound 13)

Dimethylsulfoxide (2.5 ml) was added to 5,6-dimethyl-[2,2']bipyridinyl-3-ol (50 mg), 4,7-dichloroquinoline (99 mg), and cesium carbonate (244 mg), and the mixture was stirred at 130°C for 3 hr. 4,7-Dichloroquinoline (99 mg) was further added thereto, and the mixture was stirred at 130°C for 3 hr. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a hexane-acetone system to give the title compound (80 mg, yield 88%).
$^1$H-NMR ($CDCl_3$, 400 MHz): δ 2.40 (s, 3H), 2.66 (s, 3H), 6.41 (d, J = 5.1 Hz, 1H), 7.09 (ddd, J = 7.3, 4.6, 1.0 Hz, 1H), 7.38 (s, 1H), 7.51 (dd, J = 9.0, 2.2 Hz, 1H), 7.58 (ddd, J = 8.0, 8.0, 2.0 Hz, 1H), 7.82 (ddd, J = 8.1, 1.0, 1.0 Hz, 1H), 8.03 (d, J = 2.0 Hz, 1H), 8.29 (d, J = 8.8 Hz, 1H), 8.40 - 8.45 (m, 1H), 8.55 (d, J = 5.1 Hz, 1H)
Mass spectrometric value (ESI-MS, m/z): 362 $(M+1)^+$

Example 14: 3-(7-Bromo-quinolin-4-yloxy)-5,6-dimethyl-[2,2']bipyridine (compound 14)

2-Propanol (90 ml) was added to 3-bromoaniline (5.0 g), and 5-(methoxymethylene)-2,2-dimethyl-1,3-dioxan-4,6-dione (6.0 g) was added to the mixture with stirring at 70°C, and the mixture was stirred at 70°C for 3 hr. The reaction mixture was cooled to room temperature, and the precipitated crystal was collected by filtration and was washed with diethyl ether. The crude crystal thus obtained as such was used in the next reaction without purification.
Biphenyl (26.2 g) and diphenyl ether (75 ml) were added to the crude crystal, and the mixture was stirred at 230°C for one hr. The reaction mixture was cooled to room temperature, and ether was added to the cooled mixture. The precipitated crystal was collected by filtration and was washed with diethyl ether, and the crude crystal thus obtained as such was used in the next reaction without purification.
Thionyl chloride (15 ml) and a minor amount of dimethylformamide were added to the crude crystal, and the mixture was stirred under reflux for 3 hr. The reaction mixture was added to a saturated aqueous sodium bicarbonate solution under ice cooling, and the mixture was extracted with ethyl acetate. The ethyl acetate layer was washed with water and was dried over anhydrous sodium sulfate. The solvent was removed by distillation under the reduced pressure, and the residue was purified by column chromatography with a hexane-ethyl acetate system to give 7-bromo-4-chloroquinoline (2.40 g, yield 42%).
Dimethylsulfoxide (25 ml) was added to 5,6-dimethyl-[2,2']bipyridinyl-3-ol (500 mg), 7-bromo-4-chloroquinoline (723 mg), cesium carbonate (2.4 g), and 4-dimethylaminopyridine (916 mg), and the mixture was stirred at 130°C for 5 hr. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by column chromatography with a chloroform-acetone system to give the title compound (705 mg, yield 70%).
$^1$H-NMR ($CDCl_3$, 400 MHz): δ 2.40 (s, 3H), 2.66 (s, 3H), 6.43 (d, J = 5.1 Hz, 1H), 7.09 (dd, J = 7.6, 4.6 Hz, 1H), 7.38 (s, 1H), 7.58 (ddd, J = 7.8, 7.8, 1.7 Hz, 1H), 7.64 (dd, J = 9.0, 1.7 Hz, 1H), 7.82 (d, J = 8.1 Hz, 1H), 8.20 - 8.25 (m, 2H), 8.41 - 8.45 (m, 1H), 8.55 (d, J = 5.1 Hz, 1H)
Mass spectrometric value (ESI-MS, m/z): 406 $(M+1)^+$

Example 15: 5,6-Dimethyl-3-(7-trifluoromethyl-quinolin-4-yloxy)-[2,2']bipyridine (compound 15)

Dimethylsulfoxide (2.5 ml) was added to 5,6-dimethyl-[2,2']bipyridinyl-3-ol (50 mg), 4-chloro-7-trifluoromethylquinoline (116 mg), and cesium carbonate (244 mg), and the mixture was stirred at 130°C for 3 hr. 4-Chloro-7-trifluoromethylquinoline (116 mg) was further added, and the mixture was stirred at 130°C for 3 hr. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a hexane-acetone system to give the title compound (98 mg, yield 99%).
$^1$H-NMR ($CDCl_3$, 400 MHz): δ 2.41 (s, 3H), 2.67 (s, 3H), 6.52 (d, J = 5.4 Hz, 1H), 7.09 (ddd, J = 7.4, 4.7, 1.1 Hz, 1H),

7.40 (s, 1H), 7.60 (ddd, J = 7.8, 7.8, 1.7 Hz, 1H), 7.73 (dd, J = 8.8, 1.7 Hz, 1H), 7.86 (ddd, J = 7.8, 1.0, 1.0 Hz, 1H), 8.35 (s, 1H), 8.37
- 8.41 (m, 1H), 8.49 (d, J = 8.8 Hz, 1H), 8.65 (d, J = 5.1 Hz, 1H)
Mass spectrometric value (ESI-MS, m/z): 396 (M+1)+

Example 16: 3-(7-Methoxy-quinolin-4-yloxy)-5,6-dimethyl-[2,2']bipyridine (compound 16)

3-Methoxyaniline (1.23 g) and 5-methoxymethylene-2,2-dimethyl-[1,3]dioxan-4,6-dione (1.90 g) were dissolved in 2-propanol (40 ml), and the mixture was stirred at 70°C for one hr. The solvent was removed by distillation under the reduced pressure, and the residue was washed with ether to give 5-[(3-methoxy-phenylamino)-methylene]-2,2-dimethyl-[1,3]dio xan-4,6-dione (1.22 g, yield 44%).
5-[(3-Methoxy-phenylamino)-methylene]-2,2-dimethyl-[1,3]dioxan-4,6-dione (1.22 g) and biphenyl (5.1 g) were suspended in diphenyl ether (15 ml), and the mixture was stirred at 220°C for 1.5 hr. The reaction mixture as such was purified by column chromatography with a methanol-chloroform system to give 7-methoxy-1H-quinolin-4-one (394 mg, yield 51%).
7-Methoxy-1H-quinolin-4-one (394 mg) was suspended in diisopropylethylamine (3 ml), phosphorus oxychloride (1 ml) was added to the suspension, and the mixture was stirred at 100°C for one hr. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an acetone-chloroform system to give 4-chloro-7-methoxyquinoline (312 mg, yield 72%).
4-Chloro-7-methoxyquinoline (170 mg), 5,6-dimethyl-[2,2']bipyridinyl-3-ol (176 mg), and 4-dimethylaminopyridine (322 mg) were dissolved in dimethylsulfoxide (2 ml). Cesium carbonate (860 mg) was added to the solution, and the mixture was stirred at 130°C overnight. The mixture was cooled to room temperature. An aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (273 mg, yield 87%). $^1$H-NMR ($CDCl_3$, 400 MHz): δ 2.39 (s, 3H), 2.66 (s, 3H), 3.96 (d, J = 1.2 Hz, 3H), 6.34 (dd, J = 2.2, 5.4 Hz, 1H), 7.10 (m, 1H), 7.21 (m, 1H), 7.36 (s, 1H), 7.40 (m, 1H), 7.57 (m, 1H), 7.81 (dd, J = 1.2, 8.1 Hz, 1H), 8.23 (dd, J = 1.5, 9.3 Hz, 1H), 8.48 - 8.49 (m, 2H)
Mass spectrometric value (ESI-MS, m/z): 380 (M+Na)+

Example 17: 5,6-Dimethyl-3-(7-phenyl-quinolin-4-yloxy)-[2,2']bipyridine (compound 17)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (30 mg), tetrakistriphenylphosphine palladium (9 mg), and phenylboric acid (27 mg) under an argon atmosphere, and the mixture was stirred at 70°C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a chloroform-methanol system to give the title compound (30 mg, yield 100%).
$^1$H-NMR ($CDCl_3$, 400 MHz): δ 2.40 (s, 3H), 2.67 (s, 3H), 6.44 (d, J = 5.2 Hz, 1H), 7.10 (ddd, J = 7.3, 4.6, 1.0 Hz, 1H), 7.39 - 7.45 (m, 2H), 7.51 (dd, J = 7.8, 7.8 Hz, 2H), 7.58 (ddd, J = 7.8, 7.8, 1.7 Hz, 1H), 7.75 - 7.79 (m, 2H), 7.82 - 7.87 (m, 2H), 8.27 (d, J = 1.7 Hz, 1H), 8.41 (d, J = 8.6 Hz, 1H), 8.50 - 8.53 (m, 1H), 8.59 (d, J = 5.4 Hz, 1H)
Mass spectrometric value (ESI-MS, m/z): 426 (M+Na)+

Example 18: 5,6-Dimethyl-3-(7-pyridin-3-yl-quinolin-4-yloxy)-[2,2']bipyridine (compound 18)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (30 mg), tetrakistriphenylphosphine palladium (9 mg), and 3-pyridylboric acid (18 mg) under an argon atmosphere, and the mixture was stirred at 70°C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography

with a chloroform-methanol system to give the title compound (30 mg, yield 100%).
$^1$H-NMR ($CDCl_3$, 400 MHz): δ 2.41 (s, 3H), 2.68 (s, 3H), 6.47 (d, J = 5.1 Hz, 1H), 7.10 (ddd, J = 7.6, 4.9, 1.2 Hz, 1H), 7.40 (s, 1H), 7.44 (ddd, J = 7.8, 4.9, 0.7 Hz, 1H), 7.59 (ddd, J = 8.1, 8.1, 2.0 Hz, 1H), 7.81 (dd, J = 8.8, 2.0 Hz, 1H), 7.86 (d, J = 8.0 Hz, 1H), 8.05 (dd, J = 8.0, 2.4 Hz, 1H), 8.27 (d, J = 1.5 Hz, 1H), 8.45 - 8.50 (m, 2H), 8.61 (d, J = 5.4 Hz, 1H), 8.67 (dd, J = 4.9, 1.7 Hz, 1H), 9.03 (d, J = 1.5 Hz, 1H)
Mass spectrometric value (ESI-MS, m/z): 427 $(M+Na)^+$

Example 19: 5,6-Dimethyl-3-(7-pyridin-4-yl-quinolin-4-yloxy)-[2,2']bipyridine (compound 19)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (30 mg), tetrakistriphenylphosphine palladium (9 mg), and 4-pyridylboric acid (18 mg) under an argon atmosphere, and the mixture was stirred at 70˚C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a chloroform-methanol system to give the title compound (13 mg, yield 43%).
$^1$H-NMR ($CDCl_3$, 400 MHz): δ 2.41 (s, 3H), 2.68 (s, 3H), 6.48 (d, J = 5.1 Hz, 1H), 7.10 (ddd, J = 7.6, 4.9, 1.2 Hz, 1H), 7.41 (s, 1H), 7.60 (ddd, J = 7.8, 7.8, 1.7 Hz, 1H), 7.68 (dd, J = 4.6, 1.7 Hz, 2H), 7.82 - 7.88 (m, 2H), 8.34 (d, J = 2.0 Hz, 1H), 8.45 - 8.50 (m, 2H), 8.62 (d, J = 5.2 Hz, 1H), 8.74 (dd, J = 4.6, 1.7 Hz, 2H)
Mass spectrometric value (ESI-MS, m/z): 427 $(M+Na)^+$

Example 20: 5,6-Dimethyl-3-(7-p-tolyl-quinolin-4-yloxy)-[2,2']bipyridine (compound 20)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (30 mg), tetrakistriphenylphosphine palladium (9 mg), and 4-methylphenylboric acid (30 mg) under an argon atmosphere, and the mixture was stirred at 70˚C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a chloroform-methanol system to give the title compound (31 mg, yield 100%).
$^1$H-NMR ($CDCl_3$, 400 MHz): δ 2.40 (s, 3H), 2.43 (s, 3H), 2.67 (s, 3H), 6.42 (d, J = 5.1 Hz, 1H), 7.10 (ddd, J = 7.6, 4.9, 1.0 Hz, 1H), 7.32 (d, J = 7.8 Hz, 2H), 7.38 (s, 1H), 7.58 (ddd, J = 7.8, 7.8, 1.7 Hz, 1H), 7.67 (d, J = 8.0 Hz, 2H), 7.80 - 7.86 (m, 2H), 8.25 (d, J = 1.5 Hz, 1H), 8.39 (d, J = 8.8 Hz, 1H), 8.50 - 8.54 (m, 1H), 8.58 (d, J = 5.1 Hz, 1H)
Mass spectrometric value (ESI-MS, m/z): 440 $(M+Na)^+$

Example 21: 3-[4-(5,6-Dimethyl-[2,2']bipyridin-3-yloxy)-quinolin-7-yl]-phenylamine (compound 21)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (30 mg), tetrakistriphenylphosphine palladium (9 mg), and 3-aminophenylboric acid monohydrate (34 mg) under an argon atmosphere, and the mixture was stirred at 70˚C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a chloroform-methanol system to give the title compound (31 mg, yield 100%).
$^1$H-NMR ($CDCl_3$, 400 MHz): δ 2.39 (s, 3H), 2.67 (s, 3H), 3.81(brs, 2H), 6.43 (d, J = 5.1 Hz, 1H), 6.74 (ddd, J = 8.0, 2.2, 1.0 Hz, 1H), 7.05 - 7.18 (m, 3H), 7.29 (dd, J = 8.1, 8.1 Hz, 1H), 7.38 (s, 1H), 7.58 (ddd, J = 7.8, 7.8, 2.0 Hz, 1H), 7.80 (dd, J = 8.8, 2.0 Hz, 1H), 7.84 (d, J = 7.8 Hz, 1H), 8.23 (d, J = 1.7 Hz, 1H), 8.37 (d, J = 8.6 Hz, 1H), 8.50 - 8.54 (m, 1H), 8.58 (d, J = 5.1 Hz, 1H)
Mass spectrometric value (ESI-MS, m/z): 441 $(M+Na)^+$

Example 22: 4-(5,6-Dimethyl-[2,2']bipyridin-3-yloxy)-7-methoxy-quinoline-6-carboxylic acid amide (compound 22)

Methyl 4-amino-2-methoxy-benzoate (1.07 g) and 5-methoxymethylene-2,2-dimethyl-[1,3]dioxan-4,6-dione (1.0 g) were dissolved in 2-propanol (20 ml), and the mixture was stirred at 70˚C for one hr. The solvent was removed by distillation under the reduced pressure, and the residue was washed with ether to give methyl 4-[(2,2-dimethyl-4,6-dioxo-[1,3]dioxan-5-ylidenemethyl)-amino]-2-methoxy-benzoate (1.71 g, yield 95%).
Methyl 4-[(2,2-dimethyl-4,6-dioxo-[1,3]dioxan-5-ylidenemethyl)-amino ]-2-methoxy-benzoate (1.70 g) and biphenyl (4.76 g) were suspended in diphenyl ether (15 ml), and the suspension was stirred at 240˚C for one hr. The suspension was cooled to room temperature, and the precipitated crystal was collected by filtration and was washed with ether. The crystal thus obtained as such was used in the next reaction without further purification.
N,N-Dimethylformamide (2 drops) was added to the crystal thus obtained. Further, phosphorus oxychloride (2.5 ml) was added thereto, and the mixture was stirred at 100˚C for 2 hr. The solvent was removed by distillation under the reduced pressure, and water was added to the residue under ice cooling. The aqueous layer was neutralized with an aqueous sodium hydrogencarbonate solution, and the organic layer was extracted with ethyl acetate. The ethyl acetate layer was washed with water and was dried over anhydrous sodium sulfate. The solvent was removed by distillation under the reduced pressure, and the residue was purified by thin layer chromatography with a methanol-chloroform system to give methyl 4-chloro-7-methoxy-quinoline-6-carboxylate (707 mg, yield 55%) (2 steps).

Methyl 4-chloro-7-methoxy-quinoline-6-carboxylate (120 mg) was dissolved in methanol (6 ml), 28% aqueous ammonia (6 ml) was added thereto, and the mixture was stirred at 40˚C overnight. The solvent was removed by distillation under the reduced pressure, and the residue was purified by thin layer chromatography with a methanol-chloroform system to give 4-chloro-7-methoxy-quinoline-6-carboxylic acid amide (91 mg, yield 80%).
4-Chloro-7-methoxy-quinoline-6-carboxylic acid amide (91 mg), 5,6-dimethyl-[2,2']bipyridinyl-3-ol (115 mg), and 4-dimethylaminopyridine (141 mg) were dissolved in dimethylsulfoxide (3 ml), cesium carbonate (375 mg) was added to the solution, and the mixture was stirred overnight at 130˚C. The mixture was cooled to room temperature, and water was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (33 mg, yield 22%).
$^1$H-NMR ($CDCl_3$, 400 MHz): δ 2.40 (s, 3H), 2.67 (s, 3H), 4.13 (s, 3H), 5.92 (m, 1H), 6.39 (d, J = 5.4 Hz, 1H), 7.08 (ddd, J = 1.2, 4.9, 7.6 Hz, 1H), 7.36 (s, 1H), 7.56 - 7.63 (m, 2H), 7.76 (m, 1H), 7.90 (m, 1H), 8.40 (m, 1H), 8.54 (d, J = 5.6 Hz, 1H), 9.27 (d, J = 1.0 Hz, 1H)
Mass spectrometric value (ESI-MS, m/z): 423 $(M+Na)^+$

Example 23: 4-(5,6-Dimethyl-[2,2']bipyridinyl-3-yloxy)-quinazoline (compound 23)

Formamide (12 ml) was added to methyl 2-aminobenzoate (2.0 g), and the mixture was stirred at 160˚C overnight. The reaction mixture was cooled to room temperature, water was added to the cooled mixture, and the mixture was extracted with chloroform/methanol. The solvent was removed from the organic layer by distillation under the reduced pressure, and the residue was used in the next reaction without purification.
Ethyldiisopropylamine (1.6 g) and phosphorus oxychloride (3.8 g) were added to the residue, and the mixture was stirred under reflux for 2 hr. The reaction mixture was cooled to room temperature, and the cooled mixture was poured into an ice cooled saturated aqueous sodium bicarbonate solution. The mixture was stirred for 10 min and was extracted with chloroform. The organic layer was washed with a saturated aqueous sodium bicarbonate solution and water in that order and was dried over anhydrous sodium sulfate. The solvent was removed by distillation under the reduced pressure, and the residue was purified by column chromatography with a hexane-ethyl acetate system to give 4-chloroquinazoline (1.03 g, yield 60%).
Dimethylsulfoxide (2.5 ml) was added to 5,6-dimethyl-[2,2']bipyridinyl-3-ol (50 mg), 4-chloroquinazoline (82 mg), and cesium carbonate (244 mg), and the mixture was stirred at 120˚C overnight. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography using ethyl acetate as developing phase to give the title compound (27 mg, yield 33%).
$^1$H-NMR ($CDCl_3$, 400 MHz): δ 2.41 (s, 3H), 2.63 (s, 3H), 7.04 (ddd, J = 7.6, 4.9, 1.2 Hz, 1H), 7.49 (s, 1H), 7.58 - 7.68 (s, 2H), 7.86 - 8.00 (m, 3H), 8.18 (d, J = 4.6 Hz, 1H), 8.35 (d, J = 8.1 Hz, 1H), 8.63 (s, 1H)
Mass spectrometric value (ESI-MS, m/z): 351 $(M+Na)^+$

Example 24: 6-Chloro-4-(5,6-dimethyl-[2,2']bipyridin-3-yloxy)-quinazoline (compound 24)

Formamide (4 ml) was added to methyl 2-amino-5-chloro-benzoate (400 mg), and the mixture was stirred with a microwave reactor at 220˚C for 20 min. The above reaction was carried out by additional two batches using the same amounts of starting materials. Thereafter, the reaction mixtures were cooled to room temperature and were combined together, and the precipitated crystal was collected by filtration and was washed with ether. The crystal (1.19 g) thus obtained as such was used in the next reaction without further purification.

A part (300 mg) of the crystal obtained above was suspended in diisopropylethylamine (1.45 ml), phosphorus oxychloride (0.77 ml) was added to the suspension, and the mixture was stirred at 100˚C for one hr. The solvent was removed by distillation under the reduced pressure. Water was added to the reaction mixture under ice cooling, the aqueous layer was neutralized with an aqueous sodium hydrogencarbonate solution, and the organic layer was extracted with ethyl acetate. The ethyl acetate layer was washed with water and was dried over anhydrous sodium sulfate. The solvent was removed by distillation under the reduced pressure, and the residue was purified by thin layer chromatography with a methanol-chloroform system to give 4,6-dichloro-quinazoline (151 mg, yield 46%).

4,6-Dichloro-quinazoline (66 mg), 5,6-dimethyl-[2,2']bipyridinyl-3-ol (66 mg), and 4-dimethylaminopyridine (121 mg) were suspended in 1,2-dichlorobenzene (1.5 ml), and the suspension was stirred at 130˚C for one hr. The suspension was cooled to room temperature, and an aqueous sodium hydrogencarbonate solution was then added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (99 mg, yield 83%).

$^1$H-NMR ($CDCl_3$, 400 MHz): δ 2.42 (s, 3H), 2.64 (s, 3H), 7.08 (m, 1H), 7.48 (s, 1H), 7.67 (m, 1H), 7.83 (ddd, J = 0.7, 2.4, 9.0 Hz, 1H), 7.92 (d, J = 9.0 Hz, 1H), 8.02 (dd, J = 1.0, 8.1 Hz, 1H), 8.17 (s, 1H), 8.36 (m, 1H), 8.61 (s, 1H)

Mass spectrometric value (ESI-MS, m/z): 385 $(M+Na)^+$

Example 25: 6-Bromo-4-(5,6-dimethyl-[2,2']bipyridin-3-yloxy)-quinazoline (compound 25)

Formamide (4 ml) was added to methyl 2-amino-5-bromo-benzoate (400 mg), and the mixture was stirred with a microwave reactor at 220˚C for 20 min. The above reaction was carried out using the same amount of starting materials by additional two batches. The reaction mixtures were cooled to room temperature and were combined together. The precipitated crystal was collected by filtration and was washed with ether. The crystal (968 mg) thus obtained as such was used in the next reaction without further purification.

A part (300 mg) of the crystal obtained above was suspended in diisopropylethylamine (1.16 ml), phosphorus oxychloride (0.62 ml) was added to the suspension, and the mixture was stirred at 100˚C for 2 hr. The solvent was removed by distillation under the reduced pressure, and water was added to the residue under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by thin layer chromatography with a methanol-chloroform system to give 6-bromo-4-chloro-quinazoline (58 mg, yield 18%).

6-Bromo-4-chloro-quinazoline (69 mg), 5,6-dimethyl-[2,2']bipyridinyl-3-ol (57 mg), and 4-dimethylaminopyridine (104 mg) were suspended in 1,2-dichlorobenzene (1.5 ml), and the suspension was stirred at 130˚C for one hr. The reaction mixture was then cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (59 mg, yield 52%).

$^1$H-NMR ($CDCl_3$, 400 MHz): δ 2.42 (s, 3H), 2.65 (s, 3H), 7.08 (m, 1H), 7.48 (s, 1H), 7.68 (m, 1H), 7.85 (d, J = 8.8 Hz, 1H), 7.97 (ddd, J = 0.7, 2.2, 8.8 Hz, 1H), 8.02 (d, J = 7.8 Hz, 1H), 8.18 (m, 1H), 8.54 (d, J = 2.0 Hz, 1H), 8.62 (s, 1H)

Mass spectrometric value (ESI-MS, m/z): 429 $(M+Na)^+$

Example 26: 4-(5,6-Dimethyl-[2,2']bipyridin-3-yloxy)-6-methoxy-quinazoline (compound 26)

5-Methoxy-2-nitro-benzoic acid (3.2 g) was dissolved in N,N-dimethylformamide (60 ml). Potassium carbonate (5.61 g) and methyl iodide (5.05 ml) were added to the solution, and the mixture was stirred at room temperature overnight. Water was added to the reaction mixture, and the organic layer was extracted with ethyl acetate. The ethyl acetate layer was then washed with water and saturated brine and was dried over anhydrous sodium sulfate. The solvent was removed by distillation under the reduced pressure, and the residue was purified by column chromatography with an acetone-hexane system to give methyl 5-methoxy-2-nitro-benzoate (3.38 g, yield 99%).

Methyl 5-methoxy-2-nitro-benzoate (3.38 g) was dissolved in N,N-dimethylformamide (34 ml). Triethylamine(7 ml) and 20% palladium hydroxide (340 mg) were added to the solution, and the mixture was stirred under a hydrogen gas atmosphere at room temperature overnight. The reaction mixture was filtered and was washed with chloroform. The solvent was removed by distillation under the reduced pressure, water was added to the residue, and the organic layer was extracted with ethyl acetate. The ethyl acetate layer was then washed with water and saturated brine, was dried over anhydrous sodium sulfate. The solvent was removed by distillation under the reduced pressure, and the residue was purified by column chromatography with an acetone-hexane system to give methyl 2-amino-5-methoxy-benzoate (2.87 g, yield 99%).

Formamide (4 ml) was added to methyl 2-amino-5-methoxy-benzoate (400 mg), and the mixture was stirred with a microwave reactor at 220˚C for 20 min. Further, a reaction was carried out in the same manner as described above, except that methyl 2-amino-5-methoxy-benzoate (100 mg x 2,350 mg x 5) was used. The reaction mixtures were cooled to room temperature and were combined together. Water was added thereto, the organic layer was extracted with methanol/chloroform, and the extract was dried over anhydrous sodium sulfate. The solvent was removed by distillation under the reduced pressure, and the residue (5.76 g) as such was used in the next reaction without further purification. A part (1 g) of the residue obtained above was suspended in diisopropylethylamine (4.94 ml), phosphorus oxychloride (2.65 ml) was added thereto, and the mixture was stirred at 100˚C for one hr. The solvent was removed by distillation under the reduced pressure, and water was added to the residue under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by thin layer chromatography with a methanol-chloroform system to give 4-chloro-6-methoxy-quinazoline (304 mg, yield 69%) (2 steps).

4-Chloro-6-methoxy-quinazoline (40 mg), 5,6-dimethyl-[2,2']bipyridinyl-3-ol (41 mg), and 4-dimethylaminopyridine(75 mg) were dissolved in dimethylsulfoxide (1 ml), cesium carbonate (201 mg) was added thereto, and the mixture was stirred at 130˚C overnight. The mixture was cooled to room temperature, and an aqueous sodium hydrogencarbonate solution was then added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (27 mg, yield 35%).

$^1$H-NMR ($CDCl_3$, 400 MHz): δ 2.42 (s, 3H), 2.64 (s, 3H), 3.96 (s, 3H), 7.07 (m, 1H), 7.50 (s, 1H), 7.52 (dd, J = 2.9, 9.3 Hz, 1H), 7.60 (d, J = 2.7 Hz, 1H), 7.64 (ddd, J = 1.7, 7.8, 7.8 Hz, 1H), 7.88 (d, J = 9.0 Hz, 1H), 7.93 (d, J = 7.8 Hz, 1H), 8.24 (d, J = 4.1 Hz, 1H), 8.51 (s, 1H)

Mass spectrometric value (ESI-MS, m/z): 381 $(M+Na)^+$

Example 27: 4-(5,6-Dimethyl-[2,2']bipyridin-3-yloxy)-6,7-dimethoxy-quinazoline (compound 27)

Dimethylsulfoxide (2.5 ml) was added to 5,6-dimethyl-[2,2']bipyridinyl-3-ol (50 mg), 4-chloro-6,7-dimethoxy-quinazoline (169 mg), and cesium carbonate (244 mg), and the mixture was stirred at 120˚C overnight. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with an ethyl acetate system to give the title compound (79 mg, yield 81%).

$^1$H-NMR ($CDCl_3$, 400 MHz): δ 2.41 (s, 3H), 2.63 (s, 3H), 4.03 (s, 3H), 4.05 (s, 3H), 7.06 (ddd, 7.6, 4.9, 1.2 Hz, 1H), 7.28 (s, 1H), 7.50 (s, 1H), 7.55 (s, 1H), 7.62 (ddd, J = 7.6, 4.9, 1.7 Hz, 1H), 7.89 (d, J = 7.8 Hz, 1H), 8.26 (d, J = 4.1 Hz, 1H), 8.48 (s, 1H)

Mass spectrometric value (ESI-MS, m/z): 411 $(M+Na)^+$

Example 28: 5,6-Dimethyl-3-(thieno[3,2-b]pyridin-7-yloxy)-[2,2']bipyridine (compound 28)

1-(3-Amino-thiophen-2-yl)-ethanone (423 mg) was dissolved in tetrahydrofuran (30 ml). Ethyl formate (3 ml) and sodium methoxide (640 mg) were added to the solution, and the mixture was stirred at room temperature for 3 hr. Water was added to the reaction mixture, and the organic layer was extracted with methanol/chloroform and was dried over anhydrous sodium sulfate. The solvent was removed by distillation under the reduced pressure, and the residue was purified by thin layer chromatography with a methanol-chloroform system to give 4H-thieno[3,2-b]pyridin-7-one (95 mg, yield 21%).

4H-Thieno[3,2-b]pyridin-7-one (94 mg) was suspended in diisopropylethylamine (2 ml), phosphorus oxychloride (0.5 ml) was added to the suspension, and the mixture was stirred at 100˚C for one hr. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was

dried over anhydrous sodium sulfate. The solvent was removed by distillation under the reduced pressure, and the residue was purified by thin layer chromatography with an acetone-chloroform system to give 7-chloro-thieno[3,2-b] pyridine (84 mg, yield 79%).

7-Chloro-thieno[3,2-b]pyridine (43 mg), 5,6-dimethyl-[2,2']bipyridinyl-3-ol (51 mg), and 4-dimethylaminopyridine(93 mg) were dissolved in dimethylsulfoxide (1.5 ml). Cesium carbonate(248 mg) was added to the solution, and the mixture was stirred at 130°C overnight. The mixture was cooled to room temperature, and water was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (67 mg, yield 80%).

$^1$H-NMR ($CDCl_3$, 400 MHz): δ 2.40 (s, 3H), 2.66 (s, 3H), 6.49 (d, J = 5.4 Hz, 1H), 7.12 (dd, J = 4.9, 6.8 Hz, 1H), 7.39 (s, 1H), 7.61 - 7.65 (m, 2H), 7.79 (d, J = 5.6 Hz, 1H), 7.90 (m, 1H), 8.40 (dd, J = 0.7, 5.6 Hz, 1H), 8.44 (m, 1H)

Mass spectrometric value (ESI-MS, m/z): 356 $(M+Na)^+$

Example 29: 5,6-Dimethyl-3-(2-phenyl-thieno[3,2-b]pyridin-7-yloxy)-[2,2']bipyridine (compound 29)

1-(3-Amino-5-phenyl-thiophen-2-yl)-ethanone (640 mg) was dissolved in tetrahydrofuran (30 ml). Ethyl formate (3 ml) and sodium methoxide (640 mg) were added to the solution, and the mixture was stirred at room temperature for 3 hr. Water was added to the reaction mixture, and the organic layer was extracted with methanol/chloroform and was dried over anhydrous sodium sulfate. The solvent was removed by distillation under the reduced pressure, and the residue was washed with chloroform to give 2-phenyl-4H-thieno[3,2-b]pyridin-7-one (462 mg, yield 69%).

2-Phenyl-4H-thieno[3,2-b]pyridin-7-one (493 mg) was suspended in diisopropylethylamine (4 ml). Phosphorus oxychloride (1.5 ml) was added to the suspension, and the mixture was stirred at 100°C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an acetone-chloroform system to give 7-chloro-2-phenyl-thieno [3,2-b]pyridine (390 mg, yield 73%).

7-Chloro-2-phenyl-thieno[3,2-b]pyridine (50 mg), 5,6-dimethyl-[2,2']bipyridinyl-3-ol (41 mg), and 4-dimethylaminopyridine (75 mg) were dissolved in dimethylsulfoxide (1.5 ml). Cesium carbonate (199 mg) was added to the solution, and the mixture was stirred at 130°C overnight. The mixture was cooled to room temperature, and water was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (71 mg, yield 87%).

$^1$H-NMR ($CDCl_3$, 400 MHz): δ 2.41 (s, 3H), 2.67 (s, 3H), 6.46 (d, J = 5.9 Hz, 1H), 7.13 (ddd, J = 1.2, 4.9, 7.6 Hz, 1H), 7.41 (s, 1H), 7.42 - 7.52 (m, 3H), 7.65 (ddd, J = 1.7, 7.8, 7.8 Hz, 1H), 7.76 (m, 2H), 7.83 (s, 1H), 7.93 (d, J = 7.8 Hz, 1H), 8.36 (d, J = 5.9 Hz, 1H), 8.46 (d, J = 4.4 Hz, 1H)

Mass spectrometric value (ESI-MS, m/z): 432 $(M+Na)^+$

Example 30: 4-(5,6-Dimethyl-[2,2']bipyridin-3-yloxy)-[1,5]naphthyridine (compound 30)

[1,5]Naphthyridin-4-ol (200 mg) was suspended in thionyl chloride (3.0 ml), N,N-dimethylformamide (3 drops) was added to the suspension, and the mixture was stirred at 100°C for 4 hr. The solvent was removed by distillation under the reduced pressure. Water was added to the residue, and the solution was rendered weakly basic by the addition of a saturated aqueous sodium hydrogencarbonate solution. Chloroform was added thereto, and the mixture was extracted, and the chloroform layer was dried over magnesium sulfate. The solvent was removed by distillation under the reduced pressure, and the residue was purified by column chromatography with an acetone-chloroform system to give 4-chloro-[1,5]naphthyridine (348 mg, yield 62%).

4-Chloro-[1,5]naphthyridine (41 mg), 5,6-dimethyl-[2,2']bipyridinyl-3-ol (20 mg), and 4-dimethylaminopyridine(37 mg), and cesium carbonate (98 mg) were suspended in dimethylsulfoxide (1.5 ml), and the suspension was stirred at 140°C for 24 hr. The reaction mixture was cooled to room temperature, water was added to the reaction mixture, and the mixture was extracted with ethyl acetate. The ethyl acetate layer was then washed with water and saturated brine and was dried over anhydrous magnesium sulfate. The solvent was removed by distillation under the reduced pressure, and the residue was purified by column chromatography with a chloroform-methanol system to give the title compound (7 mg, yield 21%).

$^1$H-NMR ($CDCl_3$, 400 MHz): δ 2.38 (s, 3H), 2.66 (s, 3H), 6.65 (d, J = 5.2 Hz, 1H), 7.04 (m, 1H), 7.41 (s, 1H), 7.51 (m, 1H), 7.68 (dd, J = 8.4, 4.0 Hz, 1H), 7.96 (d, J = 8.0 Hz, 1H), 8.36 (dd, J = 8.4, 2.0 Hz, 1H), 8.41 (d, J = 4.0 Hz, 1H), 8.60 (d, J = 5.2 Hz, 1H), 9.01 (dd, J = 4.0, 1.6 Hz, 1H)

Mass spectrometric value (ESI-MS, m/z): 351 $(M+Na)^+$

Example 31: 4-(5,6-Dimethyl-[2,2']bipyridin-3-yloxy)-[1,6]naphthyridine (compound 31)

4-Amino-2,6-dichloropyridine (1.76 g) and 5-methoxymethylene-2,2-dimethyl-[1,3]dioxan-4,6-dione (2.05 g) were dissolved in 2-propanol (50 ml), and the solution was stirred at 70˚C for 4 hr. The solvent was removed by distillation under the reduced pressure, and the residue was washed with 2-propanol to give 5-[(2,6-dichloro-pyridin-4-ylamino)-methylene]-2,2-dimethyl-[1,3]dioxan-4,6-dione (3.21 g, yield 94%).
5-[(2,6-Dichloro-pyridin-4-ylamino)-methylene]-2,2 -dimethyl-[1,3]dioxan-4,6-dione (1.03 g) and biphenyl (5.1 g) were suspended in diphenyl ether (17 ml), and the suspension was stirred at 220˚C for 2 hr. The reaction mixture was cooled to room temperature, and the precipitated crystal was collected by filtration and was washed with chloroform. The crystal thus obtained as such was used in the next reaction without further purification.
The crystal (0.58 g) thus obtained was dissolved in N,N-dimethylformamide (12 ml). Triethylamine (1.5 ml) and 20% palladium hydroxide (0.48 g) were added to the solution, and the mixture was stirred under a hydrogen gas atmosphere at room temperature overnight. The reaction mixture was filtered and was washed with chloroform. The solvent was removed by distillation under the reduced pressure, and the residue as such was used in the next reaction without further purification.

The residue was suspended in diisopropylethylamine (3 ml), phosphorus oxychloride (0.7 ml) was added to the suspension, and the mixture was stirred at 100˚C for 4 hr. Water was added to the reaction mixture under ice cooling, the aqueous layer was neutralized with an aqueous sodium hydrogencarbonate solution, and the organic layer was extracted with chloroform and was dried over anhydrous sodium sulfate. The solvent was removed by distillation under the reduced pressure, and the residue was purified by column chromatography with an acetone-chloroform system to give 4-chloro-[1,6]naphthyridine (63 mg, yield 12%) (3 steps).
4-Chloro-[1,6]naphthyridine (39 mg), 5,6-dimethyl-[2,2']bipyridinyl-3-ol (47 mg), and 4-dimethylaminopyridine (86 mg) were dissolved in dimethylsulfoxide (1 ml). Cesium carbonate (229 mg) was added to the solution, and the mixture was stirred at 130˚C overnight. The reaction mixture was cooled to room temperature, and water was then added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (70 mg, yield 92%).
$^1H$-NMR ($CDCl_3$, 400 MHz): δ 2.42 (s, 3H), 2.67 (s, 3H), 6.52 (d, J = 5.4 Hz, 1H), 7.09 (ddd, J = 1.2, 4.9, 7.6 Hz, 1H), 7.43 (s, 1H), 7.62 (ddd, J = 2.0, 7.8, 7.8 Hz, 1H), 7.87 (d, J = 5.9 Hz, 1H), 7.92 (d, J = 8.1 Hz, 1H), 8.35 (m, 1H), 8.71 (d, J = 5.4 Hz, 1H), 8.79 (d, J = 5.9 Hz, 1H), 9.78 (s, 1H)
Mass spectrometric value (ESI-MS, m/z): 351 $(M+Na)^+$

Example 32: 5,6-Dimethyl-3-(thieno[2,3-d]pyrimidin-4-yloxy)-[2,2']bipyridine (compound 32)

Formamide (4 ml) was added to methyl 2-amino-thiophene-3-carboxylate (400 mg), and the mixture was stirred with a microwave reactor at 220˚C for 20 min. The above reaction was carried out using the same amount of starting materials by additional two batches. The reaction mixtures were cooled to room temperature and were combined together. Water was added thereto, and the organic layer was extracted with methanol/chloroform and was dried over anhydrous sodium sulfate. The solvent was removed by distillation under the reduced pressure, and the residue (1.28 g) as such was used in the next reaction without further purification.
A part (300 mg) of the residue was suspended in diisopropylethylamine (1.72 ml). Phosphorus oxychloride (0.92 ml) was added to the suspension, and the mixture was stirred at 100˚C for one hr. The solvent was removed by distillation under the reduced pressure. Water was then added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by thin layer chromatography with a methanol-chloroform system to give 4-chloro-thieno[2,3-d]pyrimidine (151 mg, yield 49%) (2 steps).

4-Chloro-thieno[2,3-d]pyrimidine (40 mg), 5,6-dimethyl-[2,2']bipyridin-3-ol (47 mg), and 4-dimethylaminopyridine (86 mg) were dissolved in dimethylsulfoxide (1 ml). Cesium carbonate (229 mg) was added to the solution, and the mixture was stirred at 130˚C overnight. The reaction mixture was cooled to room temperature, and water was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (62 mg, yield 81%).
$^1H$-NMR ($CDCl_3$, 400 MHz): δ 2.41 (s, 3H), 2.64 (s, 3H), 7.11 (dd, J = 5.1, 6.6 Hz, 1H), 7.47 (m, 1H), 7.49 (s, 1H), 7.55

(d, J = 5.9 Hz, 1H), 7.67 (ddd, J = 1.7, 7.6, 7.8 Hz, 1H), 7.93 (d, J = 8.1 Hz, 1H), 8.32 (d, J = 4.1 Hz, 1H), 8.47 (s, 1H)
Mass spectrometric value (ESI-MS, m/z): 357 $(M+Na)^+$

Example 33: 5,6-Dimethyl-3-(thieno[3,2-d]pyrimidin-4-yloxy)-[2,2']bipyridine (compound 33)

Formamide (4 ml) was added to methyl 3-amino-thiophene-2-carboxylate (400 mg), and the mixture was stirred with a microwave reactor at 220˚C for 20 min. The above reaction was carried out using the same amount of starting materials by additional two batches. The reaction mixtures were cooled to room temperature and were combined together. Water was added thereto, and the organic layer was extracted with methanol/chloroform and was dried over anhydrous sodium sulfate. The solvent was removed by distillation under the reduced pressure, and the residue (1.90 g) as such was used in the next reaction without further purification.
A part (400 mg) of the residue was suspended in diisopropylethylamine (2.29 ml). Phosphorus oxychloride (1.22 ml) was added to the suspension, and the mixture was stirred at 100˚C for one hr. The solvent was removed by distillation under the reduced pressure. Water was then added to the reaction mixture under ice cooling. The aqueous layer was neutralized with an aqueous sodium hydrogencarbonate solution. The organic layer was extracted with ethyl acetate, and the ethyl acetate layer was then washed with water and was dried over anhydrous sodium sulfate. The solvent was removed by distillation under the reduced pressure, and the residue was purified by thin layer chromatography with a methanol-chloroform system to give 4-chloro-thieno[3,2-d]pyrimidine (167 mg, yield 61%) (2 steps).
4-Chloro-thieno[3,2-d]pyrimidine (35 mg), 5,6-dimethyl-[2,2']bipyridinyl-3-ol (41 mg), and 4-dimethylaminopyridine (75 mg) were dissolved in dimethylsulfoxide (1 ml). Cesium carbonate (229 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (64 mg, yield 91%).
$^1$H-NMR ($CDCl_3$, 400 MHz): δ 2.41 (s, 3H), 2.64 (s, 3H), 7.09 (m, 1H), 7.50 (s, 1H), 7.53 (d, J = 5.4 Hz, 1H), 7.66 (ddd, J = 1.7, 7.8, 7.8 Hz, 1H), 7.96 (d, J = 5.6 Hz, 1H), 7.98 (s, 1H), 8.28 (d, J = 4.1 Hz, 1H), 8.58 (s, 1H)
Mass spectrometric value (ESI-MS, m/z): 357 $(M+Na)^+$

Example 34: 5,6-Dimethyl-3-(5-methyl-thieno[2,3-d]pyrimidin-4-yloxy)-[2,2'] bipyridine (compound 34)

Formamide (4 ml) was added to methyl 2-amino-4-methyl-thiophene-3-carboxylate (400 mg), and the mixture was stirred with a microwave reactor at 220˚C for 20 min. The above reaction was carried out using the same amount of starting materials by additional two batches. The reaction mixtures were cooled to room temperature and were combined together. Water was added thereto, and the organic layer was extracted with methanol/chloroform and was dried over anhydrous sodium sulfate. The solvent was removed by distillation under the reduced pressure, and the residue (1.35 g) as such was used in the next reaction without further purification.
A part (300 mg) of the residue was suspended in diisopropylethylamine (1.57 ml). Phosphorus oxychloride (0.84 ml) was added to the suspension, and the mixture was stirred at 100˚C for one hr. The solvent was removed by distillation under the reduced pressure. Water was then added to the reaction mixture under ice cooling. The aqueous layer was neutralized with an aqueous sodium hydrogencarbonate solution. The organic layer was extracted with ethyl acetate, and the ethyl acetate layer was then washed with water and was dried over anhydrous sodium sulfate. The solvent was removed by distillation under the reduced pressure, and the residue was purified by thin layer chromatography with a methanol-chloroform system to give 4-chloro-5-methyl-thieno[2,3-d]pyrimidine (231 mg, yield 87%) (2 steps).
4-Chloro-5-methyl-thieno[2,3-d]pyrimidine (35 mg), 5,6-dimethyl-[2,2']bipyridinyl-3-ol (38 mg), and 4-dimethylaminopyridine(69 mg) were dissolved in dimethylsulfoxide (1 ml). Cesium carbonate (185 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (46 mg, yield 70%).
$^1$H-NMR ($CDCl_3$, 400 MHz): δ 2.41 (s, 3H), 2.56 (d, J = 1.2 Hz, 3H), 2.63 (s, 3H), 7.02 (d, J = 0.7 Hz, 1H), 7.11 (dd, J = 6.1, 6.3 Hz, 1H), 7.46 (s, 1H), 7.66 (dd, J = 7.6, 7.8 Hz, 1H), 7.89 (d, J = 8.1 Hz, 1H), 8.34 (d, J = 4.1 Hz, 1H), 8.44 (s, 1H)
Mass spectrometric value (ESI-MS, m/z): 371 $(M+Na)^+$

Example 35: 3-(5,6-Dimethyl-thieno[2,3-d]pyrimidin-4-yloxy)-5,6-dimethyl-[2,2']bipyridine (compound 35)

Ethyl 2-amino-4,5-dimethyl-thiophene-3-carboxylate (1.00 g) was suspended in formamide (5.0 ml), and the

suspension was stirred at 180˚C for 15 hr. The reaction mixture was cooled to room temperature and was then filtered. The residue was washed with ethyl acetate and diethyl ether to give 5,6-dimethyl-1H-thieno[2,3-d]pyrimidin-4-one (689 mg, yield 71%).

5,6-Dimethyl-1H-thieno[2,3-d]pyrimidin-4-one (200 mg) was suspended in thionyl chloride (2.5 ml). N,N-Dimethylformamide (two drops) was added to the suspension, and the mixture was stirred at 110˚C for 3 hr. The solvent was removed by distillation under the reduced pressure. Water was added to the residue, and the solution was rendered weakly basic by the addition of a saturated aqueous sodium hydrogencarbonate solution. Chloroform was added thereto for extraction, and the chloroform layer was dried over magnesium sulfate. The solvent was removed by distillation under the reduced pressure, and the residue was purified by column chromatography with an acetone-hexane system to give 4-chloro-5,6-dimethyl-thieno[2,3-d]pyrimidine (205 mg, yield 93%).

4-Chloro-5,6-dimethyl-thieno[2,3-d]pyrimidine (40 mg), 5,6-dimethyl-[2,2']bipyridinyl-3-ol (20 mg), and 4-dimethylaminopyridine (37 mg) were suspended in 1,2-dichlorobenzene (1.5 ml), and the mixture was stirred at 140˚C for 3 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give the title compound (20 mg, yield 55%). $^1$H-NMR ($CDCl_3$, 400 MHz): δ 2.39 (s, 3H), 2.43 (s, 3H), 2.48 (s, 3H), 2.61 (s, 3H), 7.09 (m, 1H), 7.43 (s, 1H), 7.64 (ddd, J = 7.6, 7.6, 2.0 Hz, 1H), 7.83 (d, J = 8.0 Hz, 1H), 8.35 (m, 2H)

Mass spectrometric value (ESI-MS, m/z): 385 $(M+Na)^+$

Example 37: 3-(6,7-Dimethoxy-quinolin-4-yloxy)-6-ethyl-[2,2']bipyridine (compound 37)

2-Bromopyridine (500 mg) was dissolved in tetrahydrofuran (15 ml). A 1.58 M n-butyllithium/hexane solution (2 ml) was added to the solution at -78˚C, and the mixture was stirred for 30 min. 5-Ethylfurfural (350 mg) was added to the reaction mixture, and the mixture was stirred at room temperature for additional one hr. Water was added to the reaction mixture, and the organic layer was extracted with ethyl acetate. The ethyl acetate layer was then washed with water and saturated brine and was dried over anhydrous sodium sulfate. The solvent was removed by distillation under the reduced pressure, and the residue was purified by thin layer chromatography with an ethyl acetate-hexane system to give (5-ethyl-furan-2-yl)-pyridin-2-yl-methanol (214 mg, yield 37%).

(5-Ethyl-furan-2-yl)-pyridin-2-yl-methanol (214 mg) was dissolved in chloroform (5 ml). Manganese dioxide (1.37 g) was added to the solution, and the mixture was stirred at room temperature overnight. The reaction mixture was filtered, and the solvent was removed by distillation under the reduced pressure. The residue was purified by thin layer chromatography with an ethyl acetate-hexane system to give (5-ethyl-furan-2-yl)-pyridin-2-yl-methanone (161 mg, yield 76%).

(5-Ethyl-furan-2-yl)-pyridin-2-yl-methanone (160 mg) was dissolved in methanol (3 ml), 28% aqueous ammonia (3 ml) was added to the solution, and the mixture was stirred in a sealed tube at 160˚C overnight. The solvent was removed by distillation under the reduced pressure, and the residue was purified by thin layer chromatography with an acetone-hexane system to give 6-ethyl-[2,2']bipyridinyl-3-ol (104 mg, yield 65%).

4-Chloro-6,7-dimethoxyquinoline (167 mg), 6-ethyl-[2,2']bipyridinyl-3-ol (50 mg), and 4-dimethylaminopyridine (30.5 mg) were dissolved in dimethylsulfoxide (5 ml). Cesium carbonate (81.5 mg) was added to the solution, and the mixture was stirred at 130˚C overnight. The reaction mixture was cooled to room temperature, water was added to the reaction mixture, and the organic layer was extracted with ethyl acetate. The ethyl acetate layer was then washed with water and saturated brine and was dried over anhydrous sodium sulfate. The solvent was removed by distillation under the reduced pressure, and the residue was purified by thin layer chromatography with an acetone-chloroform system to give the title compound (10 mg, yield 10%).

$^1$H-NMR ($CDCl_3$, 400 MHz): δ 1.41 (t, J = 7.6 Hz, 3H), 3.00 (q, J = 7.6 Hz, 2H), 4.02 (s, 3H), 4.03 (s, 3H), 6.37 (d, J = 5.4 Hz, 1H), 7.13 (ddd, J = 1.0, 4.6, 7.6 Hz, 1H), 7.33 (d, J = 8.5 Hz, 1H), 7.37 (s, 1H), 7.54 (s, 1H), 7.55 (d, J = 7.6 Hz, 1H), 7.62 (ddd, J = 1.7, 7.8, 7.8 Hz, 1H), 7.82 (ddd, J = 1.0, 1.0, 8.0 Hz, 1H), 8.40 (d, J = 5.4 Hz, 1H), 8.48 (m, 1H)

Mass spectrometric value (ESI-MS, m/z): 410 $(M+Na)^+$

Example 38: 4-(5,6,5'-Trimethyl-[2,2']bipyridin-3-yloxy)-quinazoline (compound 38)

2-Bromo-5-picoline (1.5 g) was dissolved in anhydrous tetrahydrofuran (40 ml) under an argon atmosphere. A 1.6 M n-butyllithium/hexane solution (5.6 ml) was added dropwise to the solution at -78˚C, and the mixture was stirred at -78˚C for 30 min. 4,5-Dimethylfurfural (1 g) dissolved in anhydrous tetrahydrofuran (20 ml) was added dropwise to the reaction mixture, and the mixture was brought to room temperature with stirring. Water was added to the reaction mixture to stop the reaction. The solvent was removed by distillation under the reduced pressure, water was added to the residue, and the mixture was extracted with ethyl acetate. The ethyl acetate layer was then washed with water and was dried over anhydrous sodium sulfate. The solvent was removed by distillation under the reduced pressure, and the

residue was purified by column chromatography with a hexane-ethyl acetate system to give (4,5-dimethylfuran-2-yl)-(5-methyl-pyridin-2-yl)-methanol (855 mg, yield 44%).

(4,5-Dimethylfuran-2-yl)-(5-methyl-pyridin-2-yl)-m ethanol (850 mg) was dissolved in chloroform (40 ml). Manganese dioxide (3.4 g) was added to the solution, and the mixture was stirred at room temperature overnight. The reaction mixture was filtered through Celite, and the solvent was removed from the filtrate by distillation under the reduced pressure. The residue was purified by column chromatography with a hexane-ethyl acetate system to give (4,5-dimethylfuran-2-yl)-(5-methyl-pyridin-2-yl)-methanone (788 mg, yield 93%).

(4,5-Dimethylfuran-2-yl)-(5-methyl-pyridin-2-yl)-m ethanone (780 mg), methanol (7 ml), and a 28% aqueous ammonia solution (7 ml) were placed in a sealed tube, and the mixture was stirred at 160˚C overnight. The reaction mixture was cooled to room temperature, and the solvent was then removed by distillation under the reduced pressure. The residue was purified by column chromatography with a hexane-ethyl acetate system to give 5,6,5'-trimethyl-[2,2']bipyridinyl-3-ol (529 mg, yield 68%).

Dimethylsulfoxide (1 ml) was added to 5,6,5'-trimethyl-[2,2']bipyridinyl-3-ol (20 mg), 4-chloroquinazoline (31 mg), and cesium carbonate (91 mg), and the mixture was stirred at 120˚C overnight. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a chloroform-methanol system to give the title compound (13 mg, yield 41%).

$^1$H-NMR ($CDCl_3$, 400 MHz): δ 2.19 (s, 3H), 2.40 (s, 3H), 2.63 (s, 3H), 7.42 (d, J = 8.0 Hz, 1H), 7.46 (s, 1H), 7.64 (dd, J = 7.6, 7.6 Hz, 1H), 7.84 (d, J = 8.0 Hz, 1H), 7.90 (dd, J = 8.0, 8.0 Hz, 1H), 7.97 (d, J = 8.6 Hz, 1H), 8.02 (s, 1H), 8.37 (d, J = 8.0 Hz, 1H), 8.63 (s, 1H)

Mass spectrometric value (ESI-MS, m/z): 365 $(M+Na)^+$

Example 39: 6-Methoxy-4-(5,6,5'-trimethyl-[2,2']bipyridin-3-yloxy)-quinazoline (compound 39)

4-Chloro-6-methoxy-quinazoline (40 mg), 5,6,5'-trimethyl-[2,2']bipyridinyl-3-ol (44 mg), and 4-dimethylaminopyridine (75 mg) were dissolved in dimethylsulfoxide (1 ml). Cesium carbonate (201 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (49 mg, yield 63%).

$^1$H-NMR ($CDCl_3$, 400 MHz): δ 2.22 (s, 3H), 2.41 (s, 3H), 2.63 (s, 3H), 3.98 (s, 3H), 7.45 (s, 1H), 7.47 (s, 1H), 7.53 (dd, J = 2.9, 9.3 Hz, 1H), 7.64 (d, J = 2.7 Hz, 1H), 7.84 (d, J = 8.1 Hz, 1H), 7.88 (d, J = 9.0 Hz, 1H), 8.11 (s, 1H), 8.51 (s, 1H)

Mass spectrometric value (ESI-MS, m/z): 395 $(M+Na)^+$

Example 41: 6,7-Dimethoxy-4-(5,6,6'-trimethyl-[2,2']bipyridin-3-yloxy)-quinazoline (compound 41)

2-Bromo-6-picoline (1.5 g) was dissolved in tetrahydrofuran (40 ml) under an argon atmosphere. A 1.6 M solution (5.6 ml) of n-butyllithium in hexane was added dropwise to the solution at -78˚C, and the mixture was then stirred at -78˚C for 30 min. 4,5-Dimethylfurfural (1 g) dissolved in tetrahydrofuran (20 ml) was added dropwise to the solution, and the mixture was brought to room temperature with stirring. Water was added to the reaction mixture to stop the reaction. The solvent was removed by distillation under the reduced pressure, water was added to the residue, and the mixture was extracted with ethyl acetate. The ethyl acetate layer was then washed with water and was dried over anhydrous sodium sulfate. The solvent was removed by distillation under the reduced pressure, and the residue was purified by column chromatography with a hexane-acetone system to give (4,5-dimethylfuran-2-yl)-(6-methyl-pyridin-2-yl)-methanol (1.4 g, yield 74%).

(4,5-Dimethylfuran-2-yl)-(6-methyl-pyridin-2-yl)-m ethanol (1.4 g) was dissolved in chloroform (30 ml). Manganese dioxide (5.7 g) was added to the solution, and the mixture was stirred at room temperature overnight. Further, manganese dioxide (1.8 g) was added thereto, and the mixture was stirred overnight. The reaction mixture was filtered through Celite, and the solvent was removed from the filtrate by distillation under the reduced pressure to give (4,5-dimethylfuran-2-yl)-(6-methyl-pyridin-2-yl)-methanone (815 mg, yield 58%).

(4,5-Dimethylfuran-2-yl)-(6-methyl-pyridin-2-yl)-m ethanone (810 mg), methanol (7 ml), and a 28% aqueous ammonia solution (7 ml) were placed in a sealed tube, and the mixture was stirred at 160˚C overnight. The reaction mixture was cooled to room temperature, and the solvent was then removed by distillation under the reduced pressure. The residue was purified by column chromatography with a hexane-ethyl acetate system to give 5,6,6'-trimethyl-[2,2']bipyridinyl-3-ol (753 mg, yield 93%).

Dimethylsulfoxide (2 ml) was added to 5,6,6'-trimethyl-[2,2']bipyridinyl-3-ol (40 mg), 4-chloro-6,7-dimethoxy-

quinazoline (126 mg), and cesium carbonate (183 mg), and the mixture was stirred at 130˚C overnight. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a chloroform-methanol system to give the title compound (22 mg, yield 29%).
$^1$H-NMR ($CDCl_3$, 400 MHz): δ 1.90 (s, 3H), 2.40 (s, 3H), 2.62 (s, 3H), 4.04 (s, 3H), 4.06 (s, 3H), 6.87 (d, J = 7.8 Hz, 1H), 7.29 (s, 1H), 7.47 (s, 1H), 7.51 (dd, J = 7.8, 7.8 Hz, 1H), 7.58 (s, 1H), 7.79 (d, J = 7.8 Hz, 1H), 8.45 (s, 1H)
Mass spectrometric value (ESI-MS, m/z): 425 $(M+Na)^+$

Example 43: 5,6-Dimethyl-3-(quinolin-4-yloxy)-[2,3']bipyridine (compound 43)

2,3-Dimethylfuran (5 g) was dissolved in diethyl ether (75 ml) under an argon atmosphere. A 1.6 M solution (35.7 ml) of n-butyllithium in hexane was added dropwise to the solution at 0˚C, and the mixture was stirred under reflux for 2.5 hr. The reaction mixture was then cooled to -78˚C, 3-cyanopyridine (6.0 g) dissolved in diethyl ether (20 ml) was added dropwise thereto, and the mixture was stirred at room temperature for 3 hr. The reaction mixture was poured into ice to stop the reaction and was adjusted to pH 5 by the addition of 2 M hydrochloric acid, followed by extraction with chloroform. The chloroform layer was washed with water and was dried over anhydrous sodium sulfate. The solvent was removed by distillation under the reduced pressure, and the residue was purified by column chromatography with a hexane-ethyl acetate system to give (4,5-dimethylfuran-2-yl)-(3-pyridyl)-methanone (1.9 g, yield 18%).
(4,5-Dimethylfuran-2-yl)-(3-pyridyl)-methanone (1.8 g), methanol (30 ml), and a 28% aqueous ammonia solution (30 ml) were placed in a sealed tube, and the mixture was stirred at 160˚C overnight. The reaction mixture was cooled to room temperature, and the solvent was removed by distillation under the reduced pressure. The residue was purified by column chromatography with a hexane-ethyl acetate system to give 5,6-dimethyl-[2,3']-bipyridinyl-3-ol (1.1 g, yield 63%).
1,2-Dichlorobenzene (2.5 ml) was added to 5,6-dimethyl-[2,3']-bipyridinyl-3-ol (50 mg), 4-chloroquinoline (123 mg), and 4-dimethylaminopyridine (92 mg), and the mixture was stirred at 130˚C overnight. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a chloroform-acetone system to give the title compound (71 mg, yield 86%).
$^1$H-NMR ($CDCl_3$, 400 MHz): δ 2.34 (s, 3H), 2.61 (s, 3H), 6.42 (d, J = 5.1 Hz, 1H), 7.19 (dd, J = 8.1, 4.9 Hz, 1H), 7.30 (s, 1H), 7.57 (ddd, J = 8.0, 6.8, 1.2 Hz, 1H), 7.74 (ddd, J = 8.6, 6.8, 1.8 Hz, 1H), 8.05 (d, J = 8.6 Hz, 1H), 8.16 (ddd, 8.1, 2.0, 2.0 Hz, 1H), 8.32 (dd, J = 8.5, 1.0 Hz, 1H), 8.45 (dd, J = 4.9, 1.7 Hz, 1H), 8.60 (d, J = 5.2 Hz, 1H), 9.12 (d, J = 1.7 Hz, 1H)
Mass spectrometric value (ESI-MS, m/z): 328 $(M+1)^+$

Example 44: 3-(6,7-Dimethoxy-quinolin-4-yloxy)-5,6,2'-trimethyl-[2,3']bipyridine (compound 44)

3-Bromo-2-methylpyridine (500 mg) was dissolved in tetrahydrofuran (15 ml). A 1.58 M solution (2 ml) of n-butyllithium/hexane was added to the solution at -78˚C, and the mixture was stirred for 30 min. 4,5-Dimethylfurfural (350 mg) was added to the reaction mixture, and the mixture was stirred at room temperature for additional one hr. Water was added to the reaction mixture, and the organic layer was extracted with ethyl acetate. The ethyl acetate layer was then washed with water and saturated brine and was dried over anhydrous sodium sulfate. The solvent was removed by distillation under the reduced pressure, and the residue was purified by thin layer chromatography with an ethyl acetate-hexane system to give (4,5-dimethyl-furan-2-yl)-(2-methyl-pyridin-3-yl)-methanol (251 mg, yield 40%).
(4,5-Dimethyl-furan-2-yl)-(2-methyl-pyridin-3-yl)-methanol (251 mg) was dissolved in chloroform(5 ml), manganese dioxide (1.51 g) was added to the solutiion, and the mixture was stirred at room temperature overnight. The reaction mixture was filtered, and the solvent was removed by distillation under the reduced pressure. The residue was purified by thin layer chromatography with an ethyl acetate-hexane system to give (4,5-dimethyl-furan-2-yl)-(2-methyl-pyridin-3-yl)-methanone (203 mg, yield 81%).
(4,5-Dimethyl-furan-2-yl)-(2-methyl-pyridin-3-yl)-methanone (203 mg) was dissolved in methanol (2 ml), 28% aqueous ammonia (2 ml) was added to the solution, and the mixture was stirred in a sealed tube at 160˚C overnight. The solvent was removed by distillation under the reduced pressure, and the residue was purified by thin layer chromatography with an acetone-hexane system to give 5,6,2'-trimethoxy-[2,3']bipyridinyl-3-ol (12 mg, yield 6%).
4-Chloro-6,7-dimethoxyquinoline (38 mg), 5,6,2'-trimethoxy-[2,3']bipyridinyl-3-ol (12 mg), and 4-dimethylaminopyridine (21 mg) were suspended in 1,2-dichlorobenzene (1 ml), and the suspension was stirred at 130˚C for 5 hr and further at 140˚C for 2 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was

removed by distillation under the reduced pressure, and the residue was purified by thin layer chromatography with a methanol-chloroform system to give the title compound (13 mg, yield 57%).
$^1$H-NMR ($CDCl_3$, 400 MHz): δ 2.41 (s, 3H), 2.56 (s, 3H), 2.62 (s, 3H), 3.99 (s, 3H), 4.02 (s, 3H), 6.36 (d, J = 5.4 Hz, 1H), 7.00 (dd, J = 4.9, 7.3 Hz, 1H), 7.30 (s, 1H), 7.39 (s, 1H), 7.42 (s, 1H), 7.55 (dd, J = 1.7, 7.6 Hz, 1H), 8.36 (dd, J = 1.7, 4.9 Hz, 1H), 8.42 (d, J = 5.4 Hz, 1H)
Mass spectrometric value (ESI-MS, m/z): 402 $(M+1)^+$

Example 45: 3-(6,7-Dimethoxy-quinolin-4-yloxy)-5'-methoxy-5,6-dimethyl-[2,3']bipyridine (compound 45)

5-Bromo-3-methoxypyridine (500 mg) was dissolved in tetrahydrofuran (15 ml). A 1.58 M n-butyllithium/hexan solution (2 ml) was added to the solution at -78˚C, and the mixture was stirred for 30 min. 4,5-Dimethylfurfural (350 mg) was added to the reaction mixture, and the mixture was stirred at room temperature for additional one hr. Water was added to the reaction mixture, and the organic layer was extracted with ethyl acetate. The ethyl acetate layer was then washed with water and saturated brine and was dried over anhydrous sodium sulfate. The solvent was removed by distillation under the reduced pressure, and the residue was purified by thin layer chromatography with an ethyl acetate-hexane system to give (4,5-dimethyl-furan-2-yl)-(5-methoxy-pyridin-3-yl)-methanol (216 mg, yield 35%).
(4,5-Dimethyl-furan-2-yl)-(5-methoxy-pyridin-3-yl)- methanol (215 mg) was dissolved in chloroform(5 ml). Manganese dioxide (1.20 g) was added to the solution, and the mixture was stirred at room temperature overnight. The reaction mixture was filtered, and the solvent was removed by distillation under the reduced pressure. The residue was purified by thin layer chromatography with an ethyl acetate-hexane system to give (4,5-dimethyl-furan-2-yl)-(5-methoxy-pyridin-3-yl)-methanone (148 mg, yield 69%).
(4,5-Dimethyl-furan-2-yl)-(5-methoxy-pyridin-3-yl) -methanone (148 mg) was dissolved in methanol (1.5 ml), 28% aqueous ammonia (1.5 ml) was added to the solution, and the mixture was stirred in a sealed tube at 180˚C overnight. The solvent was removed by distillation under the reduced pressure, and the residue was purified by thin layer chromatography with an acetone-hexane system to give 2'-methoxy-5,6-dimethyl-[2,3']bipyridinyl-3-ol (32 mg, yield 22%).
4-Chloro-6,7-dimethoxyquinoline (92 mg), 2'-methoxy-5,6-dimethyl-[2,3']bipyridinyl-3-ol (31.5 mg), and 4-dimethylaminopyridine(50 mg) were suspended in 1,2-dichlorobenzene (1 ml), and the suspension was stirred at 140˚C for 4.5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (48 mg, yield 83%).
$^1$H-NMR ($CDCl_3$, 400 MHz): δ 2.38 (s, 3H), 2.64 (s, 3H), 3.75 (s, 3H), 4.04 (s, 3H), 4.06 (s, 3H), 6.37 (d, J = 5.4 Hz, 1H), 7.33 (s, 1H), 7.48 (s, 1H), 7.51 (s, 1H), 7.20 (m, 1H), 8.20 (d, J = 2.7 Hz, 1H), 8.43 (d, J = 5.4 Hz, 1H), 8.74 (d, J = 1.7 Hz, 1H)
Mass spectrometric value (ESI-MS, m/z): 418 $(M+1)^+$

Example 46: 3-(6,7-Dimethoxy-quinolin-4-yloxy)-6-ethyl-[2,3']bipyridine (compound 46)

3-Bromopyridine (500 mg) was dissolved in tetrahydrofuran (15 ml). A 1.58 M n-butyllithium/hexane solution (2 ml) was added to the solution at -78˚C, and the mixture was stirred for 30 min. 5-Ethylfurfural (350 mg) was added to the reaction mixture, and the mixture was stirred at room temperature for additional one hr. Water was added to the reaction mixture, and the organic layer was extracted with ethyl acetate. The ethyl acetate layer was then washed with water and saturated brine and was dried over anhydrous sodium sulfate. The solvent was removed by distillation under the reduced pressure, and the residue was purified by thin layer chromatography with an ethyl acetate-hexane systm to give (5-ethyl-furan-2-yl)-pyridin-3-yl-methanol (209 mg, yield 33%).
(5-Ethyl-furan-2-yl)-pyridin-3-yl-methanol (209 mg) was dissolved in chloroform (5 ml), manganese dioxide (1.34 g) was added to the solution, and the mixture was stirred at room temperature overnight. The reaction mixture was filtered, and the solvent was removed by distillation under the reduced pressure. The residue was purified by thin layer chromatography with an ethyl acetate-hexane system to give (5-ethyl-furan-2-yl)-pyridin-3-yl-methanone (167 mg, yield 81%).
(5-Ethyl-furan-2-yl)-pyridin-3-yl-methanone (160 mg) was dissolved in methanol (2 ml), 28% aqueous ammonia (2 ml) was added to the solution, and the mixture was stirred in a sealed tube at 160˚C for 2 days. The solvent was removed by distillation under the reduced pressure, and the residue was purified by thin layer chromatography with an acetone-hexane system to give 6-ethyl-[2,3']bipyridinyl-3-ol (72 mg, yield 45%).
6-Ethyl-[2,3']bipyridinyl-3-ol (20 mg), 4-chloro-6,7-dimethoxyquinoline (67 mg), and 4-dimethytamino-pyridine (37 mg) were dissolved in 1,2-dichlorobenzene (1 ml), and the solution was stirred at 130˚C overnight. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a hexane-acetone system to give the title compound (16.3 mg, yield 42%).
$^1$H-NMR ($CDCl_3$, 400 MHz): δ 1.42(t, J = 7.6 Hz, 3H), 2.97(q, J = 7.6 Hz, 2H), 4.03 (s, 3H), 4.04 (s, 3H), 6.38 (d, J = 5.4 Hz, 1H), 7.22 - 7.28 (m, 1H), 7.29 (s, 1H), 7.41 (s, 1H), 7.42 - 7.51 (m, 2H), 8.18 - 8.23 (m, 1H), 8.45 (d, J = 5.1 Hz, 1H), 8.52 (dd, J = 4.9, 1.7 Hz, 1H), 9.18 (d, J = 1.7 Hz, 1H)
Mass spectrometric value (ESI-MS, m/z): 388 $(M+1)^+$

Example 48: 4-(5,6-Dimethyl-[2,3']bipyridinyl-3-yloxy)-6,7-dimethoxy-quinazoline (compound 48)

1,2-Dichlorobenzene (2.5 ml) was added to 5,6-dimethyl-[2,3']-bipyridinyl-3-ol (50 mg), 4-chloro-6,7-dimethoxyquinazoline (169 mg), and 4-dimethylaminopyridine(92 mg), and the mixture was stirred at 130˚C overnight. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by column chromatography with a hexane-acetone system to give the title compound (81 mg, yield 84%).
$^1$H-NMR ($CDCl_3$, 400 MHz): δ 2.40 (s, 3H), 2.62 (s, 3H), 4.04 (s, 3H), 4.05 (s, 3H), 7.23 (ddd, J = 8.0, 4.9, 1.0 Hz, 1H), 7.29 (s, 1H), 7.45 (s, 1H), 7.47 (s, 1H), 8.09 (ddd, J = 7.8, 2.2, 2.2 Hz, 1H), 8.47 (dd, J = 4.9, 1.7 Hz, 1H), 8.55 (s, 1H), 9.04 (dd, J = 2.2, 0.8 Hz, 1H)
Mass spectrometric value (ESI-MS, m/z): 411 $(M+Na)^+$

Example 49: 4-(5,6-Dimethyl-2-pyrimidin-2-yl-pyridin-3-yloxy)-quinoline (compound 49)

2,3-Dimethylfuran (1.5 g) was dissolved in diethyl ether (20 ml) under an argon atmosphere. A 1.6 M n-butyllithium/hexane solution (10.9 ml) was added dropwise to the solution at 0˚C, and the mixture was stirred under reflux for 2.5 hr. Thereafter, the reaction mixture was cooled to -78˚C, 2-cyanopyrimidine (1.8 g) dissolved in diethyl ether (8 ml) was added dropwise to the solution, and the mixture was stirred at room temperature overnight. The reaction mixture was poured into ice to stop the reaction, was rendered acidic by the addition of 1 M hydrochloric acid, and was extracted with ethyl acetate. The ethyl acetate layer was then washed with water and was dried over anhydrous sodium sulfate. The solvent was removed by distillation under the reduced pressure, and the residue was purified by column chromatography with a chloroform-acetone system to give (4,5-dimethylfuran-2-yl)-(2-pyrimidyl)-methanone (226 mg, yield 7%).
(4,5-Dimethylfuran-2-yl)-(2-pyrimidyl)-methanone (220 mg), methanol (2 ml), and a 28% aqueous ammonia solution (2 ml) were placed in a sealed tube, and the mixture was stirred at 160˚C overnight. The reaction mixture was cooled to room temperature, and the solvent was then removed by distillation under the reduced pressure. The residue was purified by column chromatography with a hexane-acetone system to give 5,6-dimethyl-2-pyrimidin-2-yl-pyridin-3-ol (129 mg, yield 59%).
Dimethylsulfoxide (1.5 ml) was added to 5,6-dimethyl-2-pyrimidin-2-yl-pyridin-3-ol (30 mg), 4-chloroquinoline (73 mg), and cesium carbonate (146 mg), and the mixture was stirred at 130˚C for 5 hr. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a hexane-acetone system to give the title compound (21 mg, yield 44%).
$^1$H-NMR ($CDCl_3$, 400 MHz): δ 2.42 (s, 3H), 2.69 (s, 3H), 6.50 (d, J = 5.2 Hz, 1H), 7.08 (t, J = 4.9 Hz, 1H), 7.43 (s, 1H), 7.55 (ddd, J = 8.1, 6.8, 1.3 Hz, 1H), 7.73 (ddd, J = 8.3, 6.8, 1.3 Hz, 1H), 8.05 (d, J = 8.3 Hz, 1H), 8.34 (dd, J = 8.5, 1.0 Hz, 1H), 8.59 (d, J = 5.4 Hz, 1H), 8.62 (d, J = 4.9 Hz, 2H)
Mass spectrometric value (ESI-MS, m/z): 329 $(M+1)^+$

Example 50: 4-(6-Ethyl-2-pyrimidin-2-yl-pyridin-3-yloxy)-6,7-dimethoxy-quinoline (compound 50)

A 2.0 M lithium diisopropylamide/heptane, tetrahydrofuran, an ethylbenzene solution (26.2 ml), and tri-n-butyltin hydride (14.1 ml) were added in that order to anhydrous tetrahydrofuran (100 ml) under an argon atmosphere at 0˚C, and the mixture was stirred at 0˚C for 15 min. The reaction mixture was cooled to -78˚C, and 2-chloropyrimidine (5.0 g) dissolved in anhydrous tetrahydrofuran (20 ml) was added dropwise to the cooled mixture. The mixture was slowly brought to room temperature and was stirred overnight. Water was added to the reaction mixture to stop the reaction. The mixture was filtered through Celite, and the filtrate was extracted with ethyl acetate. The ethyl acetate layer was then washed with water and was dried over anhydrous sodium sulfate. The solvent was removed by distillation under the reduced pressure, and the residue was purified by column chromatography with a hexane-ethyl acetate system to give 2-tri-n-butylstannylpyrimidine (9.1 g, yield 56%).
2-Tri-n-butylstannylpyrimidine (5.0 g) was dissolved in tetrahydrofuran (130 ml) under an argon atmosphere. A 1.6 M n-butyllithium/hexane solution (8.6 ml) was added dropwise to the solution at -78˚C, and the mixture was stirred

at -78°C for 30 min. 4,5-Dimethylfurfural (1.85 g) dissolved in tetrahydrofuran (20 ml) was added dropwise thereto, and the mixture was brought to room temperature with stirring. Water was added to the reaction mixture to stop the reaction. The solvent was removed by distillation under the reduced pressure, water was added to the residue, and the mixture was extracted with ethyl acetate. The ethyl acetate layer was then washed with water and was dried over anhydrous sodium sulfate. The solvent was removed by distillation under the reduced pressure, and the residue was purified by column chromatography with a hexane-ethyl acetate system to give (5-ethylfuran-2-yl)-pyrimidin-2-yl-methanol (888 mg, yield 32%).

(5-Ethylfuran-2-yl)-pyrimidin-2-yl-methanol (880 mg) was dissolved in chloroform (15 ml), manganese dioxide (3.8 g) was added to the solution, and the mixture was stirred at room temperature overnight. The reaction mixture was filtered through Celite, and the solvent was removed from the filtrate by distillation under the reduced pressure. The residue was used in the next reaction without purification.

The residue, methanol (7 ml), and a 28% aqueous ammonia solution (8 ml) were placed in a sealed tube, and the mixture was stirred at 160°C overnight. The reaction mixture was cooled to room temperature, and the solvent was then removed by distillation under the reduced pressure. The residue was purified by column chromatography with a hexane-acetone system to give 6-ethyl-2-pyrimidin-2-yl-pyridin-3-ol (305 mg, yield 35%).

Dimethylsulfoxide (1.5 ml) was added to 6-ethyl-2-pyrimidin-2-yl-pyridin-3-ol (30 mg), 4-chloro-6,7-dimethoxyquinoline (101 mg), cesium carbonate (147 mg), and 4-dimethylaminopyridine (55 mg), and the mixture was stirred at 130°C overnight. 6-Ethyl-2-pyrimidin-2-yl-pyridin-3-ol (30 mg) was further added thereto, and the mixture was further stirred overnight. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a chloroform-acetone system. The mixture thus obtained was dissolved in ethyl acetate, and the solution was washed with a 1 N aqueous sodium hydroxide solution and water in that order to give the title compound (20 mg, yield 18%).

$^1$H-NMR ($CDCl_3$, 400 MHz): $\delta$ 1.40(t, J = 7.8 Hz, 3H), 3.04 (q, J = 7.8 Hz, 2H), 4.02 (s, 3H), 4.03 (s, 3H), 6.44 (d, J = 5.4 Hz, 1H), 7.12 (t, J = 4.9 Hz, 1H), 7.38 (s, 1H), 7.42 (d, J = 8.5 Hz, 1H), 7.53 (s, 1H), 7.60 (d, J = 8.3 Hz, 1H), 8.41 (d, J = 5.1 Hz, 1H), 8.66 (d, J = 4.9 Hz, 2H)

Mass spectrometric value (ESI-MS, m/z): 389 $(M+1)^+$

Example 51: 4-(5,6-Dimethyl-2-pyrimidin-2-yl-pyridin-3-yloxy)-6,7-dimethoxy-quinazoline (compound 51)

Dimethylsulfoxide (1.5 ml) was added to 5,6-dimethyl-2-pyrimidin-2-yl-pyridin-3-ol (30 mg), 4-chloro-6,7-dimethoxyquinazoline (73 mg), and cesium carbonate(146 mg), and the mixture was stirred at 130°C for 5 hr. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a hexane-acetone system to give the title compound (35 mg, yield 61%).

$^1$H-NMR ($CDCl_3$, 400 MHz): $\delta$ 2.44 (s, 3H), 2.69 (s, 3H), 4.01 (s, 3H), 4.06 (s, 3H), 7.07(t, J = 4.9 Hz, 1H), 7.30 (s, 1H), 7.49 (s, 1H), 7.55 (s, 1H), 8.49 (s, 1H), 8.58 (d, J = 4.9 Hz, 2H)

Mass spectrometric value (ESI-MS, m/z): 412 $(M+Na)^+$

Example 52: 2-Phenyl-3-(quinolin-4-yloxy)-[1,8]naphthyridine (compound 52)

2-Aminopyridine-3-carbaldehyde (5.00 g) and 2-chloro-1-phenyl-ethanone (7.27 g) were suspended in a 5 N aqueous sodium hydroxide solution (45.0 ml), and the suspension was allowed to stand in an airtightly stoppered state for two days. The reaction mixture was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by thin layer chromatography with a chloroform-methanol system to give 2-phenyl-[1,8]naphthyridin-3-ol (5.8 g, yield 64%).

2-Phenyl-[1,8]naphthyridin-3-ol(10 mg), 4-chloroquinoline (22 mg), and 4-dimethylaminopyridine (17 mg) were dissolved in 1,2-dichlorobenzene (0.5 ml), and the mixture was stirred at 120°C overnight. The reaction mixture was cooled to room temperature, water was added thereto, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a chloroform-methanol system to give the title compound (8.3 mg, yield 53%).

$^1$H-NMR ($CDCl_3$, 400 MHz): $\delta$ 6.54 (d, J = 5.1 Hz, 1H), 7.32 - 7.34 (m, 3H), 7.53 (dd, J = 8.3, 4.4 Hz, 1H), 7.59 - 7.66 (m, 1H), 7.79 (ddd, J = 8.3, 6.8, 1.2 Hz, 1H), 7.96 (s, 1H), 8.11 (d, J = 8.5 Hz, 1H), 8.13 - 8.20 (m, 3H), 8.36 (d, J = 7.6 Hz, 1H), 8.65 (d, J = 5.1 Hz, 1H), 9.18 (dd, J = 4.1, 1.9 Hz, 1H)

Mass spectrometric value (ESI-MS, m/z): 372 $(M+Na)^+$

Example 53: 3-(6-Fluoro-quinolin-4-yloxy)-2-phenyl-[1,8]naphthyridine (compound 53)

2-phenyl-[1,8]naphthyridin-3-ol (61 mg), 4-chloro-6-fluoroquinoline (50 mg), and 4-dimethylaminopyridine (101 mg) were suspended in 1,2-dichlorobenzene (1.5 ml), and the mixture was stirred at 130˚C for 4 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (99 mg, yield 97%).
$^1$H-NMR ($CDCl_3$, 400 MHz): δ 6.53 (d, J = 5.4 Hz, 1H), 7.32 - 7.36 (m, 3H), 7.55 - 7.62 (m, 2H), 8.00 (dd, J = 2.7, 9.0 Hz, 1H), 8.02 (s, 1H), 8.09 (m, 2H), 8.20 - 8.22 (m, 2H), 8.59 (d, J = 5.4 Hz, 1H), 9.21 (dd, J = 2.0, 4.1 Hz, 1H)
Mass spectrometric value (ESI-MS, m/z): 390 $(M+Na)^+$

Example 54: 2-Phenyl-3-(6-trifluoromethyl-quinolin-4-yloxy)-[1,8]naphthyridine (compound 54)

2-Phenyl-[1,8]naphthyridin-3-ol (48 mg), 4-chloro-6-trifluoromethylquinoline (50 mg), and 4-dimethylaminopyridine (79 mg) were suspended in 1,2-dichlorobenzene (1.5 ml), and the suspension was stirred at 130˚C for 4.5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (88 mg, yield 96%).
$^1$H-NMR ($CDCl_3$, 400 MHz): δ 6.52 (d, J = 5.4 Hz, 1H), 7.30 - 7.34 (m, 3H), 7.58 (dd, J = 4.1, 8.1 Hz, 1H), 7.97 (dd, J = 2.2, 9.0 Hz, 1H), 8.06 - 8.09 (m, 2H), 8.08 (s, 1H), 8.24 (m, 2H), 8.69 (d, J = 5.4 Hz, 1H), 8.74 (s, 1H), 9.22 (dd, J = 2.0, 4.4 Hz, 1H)
Mass spectrometric value (ESI-MS, m/z): 440 $(M+Na)^+$

Example 55: 3-(6-Methoxy-quinolin-4-yloxy)-2-phenyl-[1,8]naphthyridine (compound 55)

2-Phenyl-[1,8]naphthyridin-3-ol (72 mg), 4-chloro-6-methoxyquinoline (72 mg), and 4-dimethylaminopyridine (90 mg) were suspended in 1,2-dichlorobenzene (4 ml), and the mixture was stirred at 150˚C for 4 hr. Further, 4-chloro-6,7-dimethoxyquinoline (72 mg) was added thereto, and the mixture was stirred at 150˚C for 5 hr. The reaction mixture was cooled to room temperature, the solvent was removed by distillation under the reduced pressure, and the residue was purified by column chromatography with an acetone-chloroform system to give the title compound (105 mg, yield 85%).
$^1$H-NMR ($CDCl_3$, 400 MHz): δ 3.94 (s, 3H), 6.59 (d, J = 5.2 Hz, 1H), 7.36 - 7.39 (m, 3H), 7.43 (dd, J = 2.9, 9.3 Hz, 1H), 7.51 - 7.54 (m, 2H), 7.93 (s, 1H), 8.02 (d, J = 9.2 Hz, 1H), 8.14 - 8.18 (m, 3H), 8.54 (d, J = 5.1 Hz, 1H), 9.17 (dd, J = 1.9, 4.1 Hz, 1H)
Mass spectrometric value (ESI-MS, m/z): 402 $(M+Na)^+$

Example 56: 3-(7-Methoxy-quinolin-4-yloxy)-2-phenyl-[1,8]naphthyridine (compound 56)

2-Phenyl-[1,8]naphthyridin-3-ol (84 mg), 4-chloro-7-methoxyquinoline (84 mg), and 4-dimethylaminopyridine (158 mg) were suspended in 1,2-dichlorobenzene (4 ml), and the suspension was stirred at 140˚C for 7 hr. The reaction mixture was cooled to room temperature, the solvent was then removed by distillation under the reduced pressure, and the residue was purified by thin layer chromatography with a methanol-chloroform system to give the title compound (105 mg, yield 73%).
$^1$H-NMR ($CDCl_3$, 400 MHz): δ 3.97 (s, 3H), 6.42 (d, J = 5.1 Hz, 1H), 7.24 (d, J = 2.4 Hz, 1H), 7.34 - 7.37 (m, 3H), 7.42 (d, J = 2.4 Hz, 1H), 7.52 (dd, J = 4.4, 8.3 Hz, 1H), 7.93 (s, 1H), 8.12 - 8.17 (m, 3H), 8.22 (d, J = 9.0 Hz, 1H), 8.57 (d, J = 5.4 Hz, 1H), 9.17 (dd, J = 1.9, 4.4 Hz, 1H)
Mass spectrometric value (ESI-MS, m/z): 402 $(M+Na)^+$

Example 57: 3-(5,6-Dichloro-quinolin-4-yloxy)-2-phenyl-[1,8]naphthyridine (compound 57)

3,4-Dichloroaniline (1.65 g) and 5-methoxymethylene-2,2-dimethyl-[1,3]dioxan-4,6-dione (1.95 g) were dissolved in diphenyl ether (20 ml), and the solution was stirred at 70˚C for 30 min. Biphenyl (5.2 g) was added to the

reaction mixture, and the mixture was stirred at 220˚C for additional one hr. The reaction mixture was cooled to room temperature, and the precipitated crystal was collected by filtration and was washed with chloroform. The crystal thus obtained as such was used in the next reaction without further purification.
The residue was suspended in diisopropylethylamine (3 ml), phosphorus oxychloride (1 ml) was added to the solution, and the mixture was stirred at 100˚C for one hr. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by thin layer chromatography with an ethyl acetate-hexane system to give 4,5,6-trichloroquinoline (176 mg, yield 7%) (3 steps) and 4,6,7-trichloroquinoline (113 mg, yield 5%) (3 steps).
4,5,6-Trichloroquinoline (50 mg), 2-phenyl-[1,8]naphthyridin-3-ol (48 mg), and 4-dimethylaminopyridine (79 mg) were suspended in 1,2-dichlorobenzene (1.5 ml), and the mixture was stirred at 130˚C for 2 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (69 mg, yield 75%).
$^1$H-NMR ($CDCl_3$, 400 MHz): δ 6.62 (d, J = 5.1 Hz, 1H), 7.34 - 7.41 (m, 3H), 7.53 (dd, J = 4.1, 8.1 Hz, 1H), 7.83 (s, 1H), 7.83 (d, J = 9.0 Hz, 1H), 8.02 (d, J = 9.0 Hz, 1H), 8.15 (dd, J = 2.0, 8.3 Hz, 1H), 8.22 (m, 2H), 8.61 (d, J = 5.1 Hz, 1H), 9.17 (dd, J = 2.0, 4.1 Hz, 1H)
Mass spectrometric value (ESI-MS, m/z): 440 $(M+Na)^+$

Example 58: 3-(6,7-Dichloro-quinolin-4-yloxy).2-phenyl-[1,8]naphthyridine (compound 58)

4,6,7-Trichloroquinoline (50 mg), 2-phenyl-[1,8]naphthyridin-3-ol (48 mg), and 4-dimethylaminopyridine (79 mg) were suspended in 1,2-dichlorobenzene (1.5 ml), and the mixture was stirred at 130˚C for 5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (86 mg, yield 94%).
$^1$H-NMR ($CDCl_3$, 400 MHz): δ 6.45 (d, J = 5.4 Hz, 1H), 7.33 - 7.37 (m, 3H), 7.57 (dd, J = 4.4, 8.3 Hz, 1H), 8.03 (s, 1H), 8.07 (m, 2H), 8.22 (dd, J = 2.0, 8.2 Hz, 1H), 8.25 (s, 1H), 8.49 (s, 1H), 8.58 (d, J = 5.4 Hz, 1H), 9.21 (dd, J = 2.2, 4.4 Hz, 1H)
Mass spectrometric value (ESI-MS, m/z): 440 $(M+Na)^+$

Example 59: 3-(6-Fluoro-7-methoxy-quinolin-4-yloxy)-2-phenyl-[1,8]naphthyridine (compound 59)

4-Fluoro-3-methoxyaniline (1.0 g) and 5-methoxymethylene-2,2-dimethyl-[1,3]dioxan-4,6-dione (1.62 g) were suspended in 2-propanol (40 ml), and the mixture was stirred at 70˚C for 30 min. The reaction mixture was cooled to room temperature, and the precipitated crystal was collected by filtration and was washed with methanol and then with ether. The crystal thus obtained as such was used in the next reaction without further purification.
The crystal prepared above and biphenyl (6.1 g) were suspended in diphenyl ether (25 ml), and the suspension was stirred at 220˚C for one hr. The reaction mixture was cooled to room temperature, and the precipitated crystal was collected by filtration and was washed with chloroform. The crystal thus obtained as such was used in the next reaction without further purification.
The residue was suspended in diisopropylethylamine (7 ml). Phosphorus oxychloride (2 ml) was added to the suspension, and the mixture was stirred at 100˚C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an ethyl acetate-hexane system to give 4-chloro-6-fluoro-7-methoxy-quinoline (550 mg, yield 37%) (3 steps).
4-Chloro-6-fluoro-7-methoxy-quinoline (50 mg), 2-phenyl-[1,8]naphthyridin-3-ol (53 mg), and 4-dimethylaminopyridine (87 mg) were suspended in 1,2-dichlorobenzene (1.5 ml), and the suspension was stirred at 130˚C for 6 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (78 mg, yield 82%).

$^1$H-NMR ($CDCl_3$, 400 MHz): δ 4.07 (s, 3H), 6.44 (d, J = 5.6 Hz, 1H), 7.33 - 7.36 (m, 3H), 7.56 (dd, J = 4.1, 8.1 Hz, 1H), 7.66 (m, 1H), 7.98 (d, J = 11.2 Hz, 1H), 8.01 (s, 1H), 8.07 (m, 2H), 8.20 (dd, J = 2.0, 8.3 Hz, 1H), 8.51 (d, J = 5.4 Hz, 1H), 9.20 (dd, J = 2.2, 4.4 Hz, 1H)
Mass spectrometric value (ESI-MS, m/z): 420 $(M+Na)^+$

Example 60: 3-(7-Fluoro-6-methoxy-quinolin-4-yloxy)-2-phenyl-[1,8]naphthyridine (compound 60)

3-Fluoro-4-methoxyaniline (1.41 g) and 5-methoxymethylene-2,2-dimethyl-[1,3]dioxan-4,6-dione (2.00 g) were suspended in 2-propanol (40 ml), and the mixture was stirred at 70˚C for 30 min. The reaction mixture was cooled to room temperature, and the precipitated crystal was collected by filtration and was washed with methanol and then with ether. The crystal thus obtained as such was used in the next reaction without further purification.
The crystal prepared above and biphenyl (5.8 g) were suspended in diphenyl ether (20 ml), and the suspension was stirred at 220˚C for one hr. The reaction mixture was cooled to room temperature, and the precipitated crystal was collected by filtration and was washed with chloroform. The crystal thus obtained as such was used in the next reaction without further purification.
The residue was suspended in diisopropylethylamine (8 ml), phosphorus oxychloride (2 ml) was added to the suspension, and the mixture was stirred at 100˚C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an ethyl acetate hexane system to give 4-chloro-7-fluoro-6-methoxy-quinoline (1.10 g, yield 52%) (3 steps).
4-Chloro-7-fluoro-6-methoxy-quinoline (50 mg), 2-phenyl-[1,8]naphthyridin-3-ol (53 mg), and 4-dimethylaminopyridine (87 mg) were suspended in 1,2-dichlorobenzene (1.5 ml), and the suspension was stirred at 130˚C for 8 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (48 mg, yield 50%).
$^1$H-NMR ($CDCl_3$, 400 MHz): δ 4.06 (s, 3H), 6.56 (d, J = 5.4 Hz, 1H), 7.35 - 7.37 (m, 3H), 7.57 (dd, J = 4.1, 8.1 Hz, 1H), 7.65 (d, J = 8.8 Hz, 1H), 7.89 (d, J = 11.0 Hz, 1H), 8.02 (s, 1H), 8.09 (m, 2H), 8.20 (dd, J = 1.7, 8.1 Hz, 1H), 8.52 (d, J = 5.4 Hz, 1H), 9.20 (dd, J = 2.0, 4.1 Hz, 1H)
Mass spectrometric value (ESI-MS, m/z): 420 $(M+Na)^+$

Example 61: 3-(7-Chloro-6-methoxy-quinolin-4-yloxy)-2-phenyl-[1,8]naphthyrridine (compound 61)

3-Chloro-4-methoxyaniline (1.52 g) and 5-methoxymethylene-2,2-dimethyl-[1,3]dioxan-4,6-dione (2.03 g) were suspended in 2-propanol (40 ml), and the suspension was stirred at 70˚C for 30 min. The reaction mixture was cooled to room temperature, and the precipitated crystal was then collected by filtration and was washed with methanol and then with ether. The crystal thus obtained as such was used in the next reaction without further purification.
The crystal prepared above and biphenyl (6.8 g) were suspended in diphenyl ether (20 ml), and the suspension was stirred at 220˚C for 30 min. The reaction mixture was cooled to room temperature, and the precipitated crystal was collected by filtration and was washed with chloroform. The crystal thus obtained as such was used in the next reaction without further purification.
The residue was suspended in diisopropylethylamine (3 ml), phosphorus oxychloride (1 ml) was added to the suspension, and the mixture was stirred at 100˚C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an ethyl acetate-hexane system to give 4,7-dichloro-6-methoxy-quinoline (225 mg, yield 10%) (3 steps).
4,7-Dichloro-6-methoxy-quinoline (50 mg), 2-phenyl-[1,8]naphthyridin-3-ol (49 mg), and 4-dimethylaminopyridine (80 mg) were suspended in 1,2-dichlorobenzene (1.5 ml), and the suspension was stirred at 130˚C for 8 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (25 mg, yield 27%).
$^1$H-NMR ($CDCl_3$, 400 MHz): δ 4.07 (s, 3H), 6.57 (d, J = 5.4 Hz, 1H), 7.32 - 7.38 (m, 3H), 7.57 (dd, J = 4.1, 8.1 Hz, 1H), 7.62 (s, 1H), 8.04 (s, 1H), 8.08 (m, 2H), 8.21 (dd, J = 1.7, 8.1 Hz, 1H), 8.29 (m, 1H), 8.51 (m, 1H), 9.21 (dd, J = 1.7, 4.1

Hz, 1H) Mass spectrometric value (ESI-MS, m/z): 436 (M+Na)+

Example 62: 3-(6-Methoxy-7-methyl-quinolin-4-yloxy)-2-phenyl-[1,8]naphthyridine (compound 62)

2-Bromomethyl-1-methoxy-4-nitrobenzene (2.03 g) was dissolved in N,N-dimethylformamide (30 ml). Triethylamine (5 ml) and 20% palladium hydroxide (1.08 g) were added to the solution, and the mixture was stirred under a hydrogen gas atmosphere at room temperature overnight. The reaction mixture was filtered and was then washed with chloroform. The solvent was removed by distillation under the reduced pressure, water was added to the residue, and the organic layer was extracted with ethyl acetate. The ethyl acetate layer was then washed with water and saturated brine and was dried over anhydrous sodium sulfate. The solvent was removed by distillation under the reduced pressure, and the residue as such was used in the next reaction without purification.
The residue and 5-methoxymethylene-2,2-dimethyl-[1,3]dioxan-4,6-dione (2.00 g) were suspended in 2-propanol (40 ml), and the suspension was stirred at 70˚C for 30 min. The reaction mixture was cooled to room temperature, and the precipitated crystal was then collected by filtration and was washed with methanol and then with ether. The crystal thus obtained as such was used in the next reaction without further purification.
The crystal prepared above and biphenyl (5.9 g) were suspended in diphenyl ether (15 ml), and the suspension was stirred at 220˚C for 3 hr. The reaction mixture was cooled to room temperature, and the cooled reaction mixture as such was purified by column chromatography with an ethyl acetate-hexane system to give 6-methoxy-7-methyl-1H-quinolin-4-one (352 mg, yield 23%) (3 steps).
6-Methoxy-7-methyl-1H-quinolin-4-one (352 mg) was suspended in diisopropylethylamine (3 ml), phosphorus oxychloride (1 ml) was added to the suspension, and the mixture was stirred at 100˚C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an ethyl acetate-hexane system to give 4-chloro-6-methoxy-7-methyl-quinoline (234 mg, yield 61%).
4-Chloro-6-methoxy-7-methyl-quinoline (50 mg), 2-phenyl-[1,8]naphthyridin-3-ol (54 mg), and 4-dimethylaminopyridine (88 mg) were suspended in 1,2-dichlorobenzene (1.5 ml), and the suspension was stirred at 130˚C for 7.5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (71 mg, yield 75%).
$^1$H-NMR ($CDCl_3$, 400 MHz): δ 2.45 (s, 3H), 3.96 (s, 3H), 6.57 (d, J = 5.4 Hz, 1H), 7.35 - 7.39 (m, 3H), 7.44 (s, 1H), 7.53 (dd, J = 4.1, 8.1 Hz, 1H), 7.94 (s, 1H), 7.94 (s, 1H), 8.15 - 8.17 (m, 3H), 8.50 (d, J = 5.4 Hz, 1H), 9.17 (dd, J = 2.0, 4.1 Hz, 1H)
Mass spectrometric value (ESI-MS, m/z): 416 (M+Na)+

Example 63: Methyl 7-methoxy-4-(2-phenyl-[1,8]naphthyridin-3-yloxy)-quinoline-6-carboxylate (compound 63)

Methyl 4-chloro-7-methoxy-quinoline-6-carboxylate (50 mg),2-phenyl-[1,8]naphthyridin-3-ol (44 mg) and 4-dimethylaminopyridine (73 mg) were suspended in 1,2-dichlorobenzene (1 ml), and the suspension was stirred at 130˚C for 4.5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (70 mg, yield 80%).
$^1$H-NMR ($CDCl_3$, 400 MHz): δ 4.00 (s, 3H), 4.05 (s, 3H), 6.35 (a, J = 5.4 Hz, 1H), 7.32 - 7.35 (m, 3H), 7.52 (s, 1H), 7.56 (dd, J = 4.1, 8.1 Hz, 1H), 8.02 (s, 1H), 8.10 (m, 2H), 8.21 (dd, J = 2.0, 8.1 Hz, 1H), 8.57 (d, J = 5.4 Hz, 1H), 8.80 (s, 1H), 9.20 (dd, J = 2.0, 4.1 Hz, 1H)
Mass spectrometric value (ESI-MS, m/z): 460 (M+Na)+

Example 65: 7-Methoxy-4-(2-phenyl-[1,8]naphthyridin-3-yloxy)-quinoline-6-carboxylate benzylamide (compound 65)

Methyl 7-methoxy-4-(2-phenyl-[1,8]naphthyridin-3-yloxy)-quinoline-6-carboxylate (compound 63) (206 mg) was dissolved in ethanol/water (4 ml/0.4 ml). Lithium hydroxide monohydrate (99 mg) was added to the solution, and the mixture was stirred at room temperature overnight. The reaction mixture was neutralized with hydrochloric acid. The solvent was then removed by distillation under the reduced pressure, and the residue (334 mg) as such was used in the next reaction without purification.

A part (271 mg) of the residue, benzylamine (62 μl), and 1-ethyl-3-(3-dimethylaminopropylcarbodiimide hydrochloride (370 mg), and 1-hydroxybenzotriazole hydrate (216 mg) were dissolved in N,N-dimethylformamide (3 ml), and the solution was stirred at room temperature overnight. Water was added to the reaction mixture, and the mixture was extracted with ethyl acetate. The ethyl acetate layer was then washed with water and saturated brine and was dried over anhydrous sodium sulfate. The solvent was removed by distillation under the reduced pressure, and the residue was purified by thin layer chromatography with a methanol/chloroform system to give the title compound (187 mg, yield 96%) (2 steps).
$^1$H-NMR ($CDCl_3$, 400 MHz): δ 4.08 (s, 3H), 4.78 (d, J = 5.6 Hz, 2H), 6.29 (d, J = 5.4 Hz, 1H), 7.33 - 7.44 (m, 8H), 7.53 (s, 1H), 7.55 (dd, J = 4.1, 8.1 Hz, 1H), 8.03 (s, 1H), 8.13 (m, 2H), 8.21 (dd, J = 2.0, 8.3 Hz, 1H), 8.25 (dd, J = 5.4, 5.6 Hz, 1H), 8.53 (d, J = 5.4 Hz, 1H), 9.19 (dd, J = 2.2, 4.4 Hz, 1H), 9.37 (s, 1H)
Mass spectrometric value (ESI-MS, m/z): 535 $(M+Na)^+$

Example 66: 7-Methoxy-4-(2-phenyl-[1,8]naphthyridin-3-yloxy)-quinoline-6-carboxylic acid amide (compound 66)

Methyl 7-methoxy-4-(2-phenyl-[1,8]naphthyridin-3-yloxy)-quinoline-6-carboxylate (compound 63) (46 mg) was dissolved in methanol (2 ml), 28% aqueous ammonia (2 ml) was added to the solution, and the mixture was stirred at 40°C overnight. The solvent was removed by distillation under the reduced pressure, and the residue was purified by thin layer chromatography with a methanol-chloroform system to give the title compound (36 mg, yield 77%).
$^1$H-NMR ($CDCl_3$, 400 MHz): δ 4.14 (s, 3H), 5.97(brs, 1H), 6.30 (d, J = 5.4 Hz, 1H), 7.28 - 7.33 (m, 3H), 7.56 (dd, J = 4.4, 8.3 Hz, 1H), 7.60 (s, 1H), 7.81(brs, 1H), 8.05 (s, 1H), 8.11 (m, 2H), 8.22 (dd, J = 1.7, 8.1 Hz, 1H), 8.54 (d, J = 5.4 Hz, 1H), 9.20 (dd, J = 2.0, 4.1 Hz, 1H), 9.36 (s, 1H)
Mass spectrometric value (ESI-MS, m/z): 445 $(M+Na)^+$

Example 68: 6-Chloro-4-(2-phenyl-[1,8]naphthyridin-3-yloxy)-quinazoline (compound 68)

4,6-Dichloro-quinazoline (50 mg), 2-phenyl-[1,8]naphthyridin-3-ol (56 mg), and 4-dimethylaminopyridine (92 mg) were suspended in 1,2-dichlorobenzene (1 ml), and the suspension was stirred at 130° C for 2 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (83 mg, yield 87%).
$^1$H-NMR ($CDCl_3$, 400 MHz): δ 7.30 - 7.34 (m, 3H), 7.56 (dd, J = 4.4, 8.3 Hz, 1H), 7.86 (dd, J = 2.2, 8.8 Hz, 1H), 7.94 (d, J = 9.0 Hz, 1H), 7.98 (m, 2H), 8.22 (s, 1H), 8.27 (dd, J = 2.0, 8.1 Hz, 1H), 8.33 (d, J = 2.2 Hz, 1H), 8.60 (s, 1H), 9.19 (dd, J = 2.0, 4.4 Hz, 1H)
Mass spectrometric value (ESI-MS, m/z): 407 $(M+Na)^+$

Example 69: 6-Bromo-4-(2-phenyl-[1,8]naphthyridin-3-yloxy)-quinazoline (compound 69)

6-Bromo-4-chloro-quinazoline (100 mg), 2-phenyl-[1,8]naphthyridin-3-ol (91 mg), and 4-dimethylaminopyridine (151 mg) were suspended in 1,2-dichlorobenzene (2 ml), and the suspension was stirred at 130°C for 2 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (165 mg, yield 94%).
$^1$H-NMR ($CDCl_3$, 400 MHz): δ 7.30 - 7.36 (m, 3H), 7.57 (dd, J = 4.4, 8.3 Hz, 1H), 7.86 (d, J = 8.8 Hz, 1H), 7.96 - 8.01 (m, 3H), 8.23 (s, 1H), 8.28 (dd, J = 2.0, 8.3 Hz, 1H), 8.51 (d, J = 2.2 Hz, 1H), 8.62 (s, 1H), 9.20 (dd, J = 2.0, 4.1 Hz, 1H)
Mass spectrometric value (ESI-MS, m/z): 451 $(M+Na)^+$

Example 70: 6-Methoxy-4-(2-phenyl-[1,8]naphthyridin-3-yloxy)-quinazoline (compound 70)

4-Chloro-6-methoxy-quinazoline (50 mg), 2-phenyl-[1,8]naphthyridin-3-ol (57 mg), and 4-dimethylaminopyridine (94 mg) were suspended in 1,2-dichlorobenzene (1 ml), and the suspension was stirred at 130° C for 2 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system

to give the title compound (92 mg, yield 93%).
$^1$H-NMR ($CDCl_3$, 400 MHz): δ 3.97 (s, 3H), 7.30 - 7.34 (m, 3H), 7.52 (d, J = 2.7 Hz, 1H), 7.55 - 7.58 (m, 2H), 7.93 (d, J = 9.0 Hz, 1H), 8.01 (m, 2H), 8.28 (dd, J = 2.0, 6.1 Hz, 1H), 8.32 (s, 1H), 8.56 (s, 1H), 9.19 (dd, J = 2.0, 4.4 Hz, 1H)
Mass spectrometric value (ESI-MS, m/z): 403 $(M+Na)^+$

Example 71: 2-Phenyl-3-(thieno[3,2-b]pyridin-7-yloxy)-[1,8]naphthyridine (compound 71)

7-chloro-thieno[3,2-b]pyridine (29 mg), 2-phenyl-[1,8]naphthyridin-3-ol (38 mg), and 4-dimethylaminopyridine (63 mg) were suspended in 1,2-dichlorobenzene (1 ml), and the suspension was stirred at 130˚C for 16 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (25 mg, yield 41%).
$^1$H-NMR ($CDCl_3$, 400 MHz): δ 6.56 (d, J = 5.6 Hz, 1H), 7.36 - 7.38 (m, 3H), 7.56 (dd, J = 4.4, 8.2 Hz, 1H), 7.70 (d, J = 5.1 Hz, 1H), 7.85 (d, J = 5.1 Hz, 1H), 8.03 (s, 1H), 8.11 (m, 2H), 8.20 (dd, J = 2.0, 8.3 Hz, 1H), 8.46 (d, J = 5.9 Hz, 1H), 9.20 (dd, J = 2.0, 4.1 Hz, 1H)
Mass spectrometric value (ESI-MS, m/z): 378 $(M+Na)^+$

Example 72: 2-Phenyl-3-(2-phenyl-thieno[3,2-b]pyridin-7-yloxy)-[1,8]naphthyridine (compound 72)

7-Chloro-2-phenyl-thieno[3,2-b]pyridine (50 mg), 2-phenyl-[1,8]naphthyridin-3-ol (45 mg), and 4-dimethylaminopyridine (75 mg) were suspended in 1,2-dichlorobenzene (1 ml), and the suspension was stirred at 130˚C for 18 hr. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (46 mg, yield 53%).
$^1$H-NMR ($CDCl_3$, 400 MHz): δ 6.54 (dd, J = 0.5, 5.6 Hz, 1H), 7.36 - 7.50 (m, 6H), 7.55 (ddd, J = 1.0, 4.2, 8.1 Hz, 1H), 7.75 (d, J = 7.3 Hz, 2H), 7.83 (s, 1H), 8.01 (s, 1H), 8.15 (m, 2H), 8.19 (dd, J = 1.7, 8.1 Hz, 1H), 8.43 (d, J = 5.6 Hz, 1H), 9.19 (m, 1H)
Mass spectrometric value (ESI-MS, m/z): 454 $(M+Na)^+$

Example 73: 4-(2-Phenyl-[1,8]naphthyridin-3-yloxy)-[1,5]naphthyridine (compound 73)

4-Chloro-[1,5]naphthyridine (15 mg), 2-phenyl-[1,8]naphthyridin-3-ol (40 mg), and 4-dimethylaminopyridine (33 mg) were suspended in 1,2-dichlorobenzene (1.5 ml), and the suspension was stirred at 140˚C for 3.5 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (11 mg, yield 35%).
$^1$H-NMR ($CDCl_3$, 400 MHz): δ 6.64 (d, J = 5.2 Hz, 1H), 7.26 (m, 3H), 7.52 (dd, J = 8.0, 4.4 Hz, 1H), 7.72 (dd, J = 8.8, 4.0 Hz, 1H), 8.00 (s, 1H), 8.17 (dd, J = 8.0, 2.0 Hz, 1H), 8.24 (m, 2H), 8.39 (dd, J = 8.8, 2.0 Hz, 1H), 8.63 (d, J = 5.2 Hz, 1H), 9.04 (dd, J = 4.0, 2.4 Hz, 1H), 9.16 (dd, J = 4.0, 2.0 Hz, 1H)
Mass spectrometric value (ESI-MS, m/z): 373 $(M+Na)^+$

Example 74: 4-(2-Phenyl-[1,8]naphthyridin-3-yloxy)-[1,6]naphthyridine (compound 74)

4-Chloro-[1,6]naphthyridine (25 mg), 2-phenyl-[1,8]naphthyridin-3-ol (34 mg), and 4-dimethylaminopyridine (56 mg) were suspended in 1,2-dichlorobenzene (1 ml), and the suspension was stirred at 130˚C for 2 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (53 mg, yield 100%).
$^1$H-NMR ($CDCl_3$, 400 MHz): δ 6.48 (d, J = 5.1 Hz, 1H), 7.31 - 7.34 (m, 3H), 7.59 (dd, J = 4.4, 8.3 Hz, 1H), 7.91 (dd, J = 0.7, 5.9 Hz, 1H), 8.09 (m, 2H), 8.12 (s, 1H), 8.25 (dd, J = 2.0, 8.1 Hz, 1H), 8.74 (d, J = 5.4 Hz, 1H), 8.84 (d, J = 6.1 Hz, 1H), 9.23 (dd, J = 2.0, 4.4 Hz, 1H), 9.86 (d, J = 0.7 Hz, 1H)
Mass spectrometric value (ESI-MS, m/z): 373 $(M+Na)^+$

Example 75: 2-Phenyl-3-(thieno[3,2-d]pyrimidin-4-yloxy)-[1,8]naphthyridine (compound 75)

4-Chloro-thieno[3,2-d]pyrimidine (35 mg), 2-phenyl-[1,8]naphthyridin-3-ol (46 mg), and 4-dimethylaminopyridine (75 mg) were suspended in 1,2-dichlorobenzene (1 ml), and the suspension was stirred at 130˚C for 2 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (65 mg, yield 86%).
$^1$H-NMR ($CDCl_3$, 400 MHz): δ 7.29 - 7.36 (m, 3H), 7.55 (d, J = 5.4 Hz, 1H), 7.57 (dd, J = 4.4, 8.1 Hz, 1H), 7.99 (d, J = 5.4 Hz, 1H), 8.04 (m, 2H), 8.26 (s, 1H), 8.28 (dd, J = 2.0, 8.3 Hz, 1H), 8.58 (s, 1H), 9.19 (dd, J = 2.0, 4.1 Hz, 1H)
Mass spectrometric value (ESI-MS, m/z): 379 $(M+Na)^+$

Example 76: 2-Phenyl-3-(thieno[2,3-d]pyrimidin-4-yloxy)-[1,8]naphthyridine (compound 76)

4-Chloro-thieno[2,3-d]pyrimidine (40 mg), 2-phenyl-[1,8]naphthyridin-3-ol (52 mg), and 4-dimethylaminopyridine(86 mg) were suspended in 1,2-dichlorobenzene (1 ml), and the suspension was stirred at 130˚C for 2.5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (79 mg, yield 96%).
$^1$H-NMR ($CDCl_3$, 400 MHz): δ 7.31 - 7.34 (m, 3H), 7.53 - 7.57 (m, 3H), 8.02 (m, 2H), 8.23 (s, 1H), 8.27 (dd, J = 2.0, 8.1 Hz, 1H), 8.49 (s, 1H), 9.18 (dd, J = 2.0, 4.4 Hz, 1H)
Mass spectrometric value (ESI-MS, m/z): 379 $(M+Na)^+$

Example 77: 3-(5-Methyl-thieno[2,3-d]pyrimidin-4-yloxy)-2-phenyl-[1,8]naphthyridine (compound 77)

4-Chloro-5-methyl-thieno[2,3-d]pyrimidine (32 mg), 2-phenyl-[1,8]naphthyridin-3-ol (39 mg), and 4-dimethylaminopyridine (64 mg) were suspended in 1,2-dichlorobenzene (1 ml), and the suspension was stirred at 130˚C for 2.5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (62 mg, yield 99%).
$^1$H-NMR ($CDCl_3$, 400 MHz): δ 2.61 (d, J = 1.2 Hz, 3H), 7.09 (d, J = 1.5 Hz, 1H), 7.31 - 7.35 (m, 3H), 7.54 (dd, J = 4.1, 8.1 Hz, 1H), 7.92 (m, 2H), 8.15 (s, 1H), 8.25 (dd, J = 1.7, 8.1 Hz, 1H), 8.42 (s, 1H), 9.17 (dd, J = 2.0, 4.1 Hz, 1H)
Mass spectrometric value (ESI-MS, m/z): 371 $(M+1)^+$

Example 78: 3-(5,6-Dimethyl-thieno[2,3-d]pyrimidin-4-yloxy)-2-phenyl-[1,8] naphthyridine (compound 78)

4-Chloro-5,6-dimethyl-thieno[2,3-d]pyrimidine (15 mg), 2-phenyl-[1,8]naphthyridin-3-ol (25 mg), and 4-dimethylaminopyridine (28 mg) were suspended in 1,2-dichlorobenzene (2.0 ml), and the suspension was stirred at 140˚C for 1 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (32 mg, yield 100%).
$^1$H-NMR ($CDCl_3$, 400 MHz): δ 2.47 (s, 3H), 2.49 (s, 3H), 7.32 (m, 3H), 7.51 (dd, J = 8.0, 4.4 Hz, 1H), 7.92 (m, 2H), 8.11 (s, 1H), 8.22 (dd, J = 8.0, 1.6 Hz, 1H), 8.35 (s, 1H), 9.14 (dd, J = 8.0, 2.0 Hz, 1H)
Mass spectrometric value (ESI-MS, m/z): 407 $(M+Na)^+$

Example 81: 3-(6,7-Dimethoxy-quinolin-4-yloxy)-2-(3-methoxy-phenyl)-[1,8] naphthyridine compound 81)

2-Aminopyridine-3-carbaldehyde (100 mg) and 2-bromo-1-(3-methoxy-phenyl)-ethanone (188 mg) were suspended in a 5 N aqueous sodium hydroxide solution (0.6 ml), and the suspension was allowed to stand in an airtightly stoppered state for two days. The reaction mixture was neutralized with 10% hydrochloric acid, dichloromethane was then added thereto for extraction. The dichloromethane layer was washed with water and saturated brine and was dried over magnesium sulfate. The solvent was removed by distillation under the reduced pressure, and the residue was

purified by column chromatography with a chloroform-methanol system to give 2-(3-methoxy-phenyl)-[1,8]naphthyridin-3-ol (29 mg, yield 14%).

2-(3-Methoxy-phenyl)-[1,8]naphthyridin-3-ol (29 mg), 4-chloro-6,7-dimethoxyquinoline (64 mg), and 4-dimethylaminopyridine (42 mg) were suspended in 1,2-dichlorobenzene (2.0 ml), and the suspension was stirred at 140˚C for 4 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give the title compound (24 mg, yield 46%).

$^1$H-NMR ($CDCl_3$, 400 MHz): δ 3.72 (s, 3H), 4.00 (s, 3H), 4.03 (s, 3H), 6.47 (d, J = 5.2 Hz, 1H), 6.99 (m, 1H), 7.23 (m, 1H), 7.42 (s, 1H), 7.49 (m, 2H), 7.70 (m, 2H), 7.93 (s, 1H), 8.14 (dd, J = 8.0, 2.0 Hz, 1H), 8.47 (d, J = 5.2 Hz, 1H), 9.15 (dd, J = 4.0, 2.0 Hz, 1H)

Mass spectrometric value (ESI-MS, m/z): 462 $(M+Na)^+$

Example 82: 2-(4-Bromo-phenyl)-3-(quinolin-4-yloxy)-[1,8]naphthyridine (compound 82)

2-Aminopyridine-3-carbaldehyde (100 mg) and 2-bromo-1-(4-bromo-phenyl)-ethanone (228' mg) were suspended in a 5 N aqueous sodium hydroxide solution (0.6 ml), and the suspension was allowed to stand in an airtightly stoppered state for two days. The reaction mixture was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by thin layer chromatography with a chloroform-methanol system to give 2-(4-bromo-phenyl)-[1,8]naphthyridin-3-ol (28 mg, yield 11%).

2-(4-Bromo-phenyl)-[1,8]naphthyridin-3-ol (13 mg), 4-chloroquinoline (21 mg), and 4-dimethylaminopyridine (16 mg) were suspended in 1,2-dichlorobenzene (1.0 ml), and the suspension was stirred at 140˚C for 3.0 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (11 mg, yield 60%).

$^1$H-NMR ($CDCl_3$, 400 MHz): δ 6.54 (d, J = 5.2 Hz, 1H), 7.48 (m, 2H), 7.55 (dd, J = 8.0, 4.0 Hz, 1H), 7.74 (dd, J = 6.8, 6.8 Hz, 1H), 7.82 (dd, J = 6.8, 6.8 Hz, 1H), 7.95 (s, 1H), 8.06 (m, 2H), 8.15 (m, 2H), 8.32 (dd, J = 8, 4, 8.0 Hz, 1H), 8.67 (d, J = 5.2 Hz, 1H), 9.17 (dd, J = 4.4, 2.0 Hz, 1H)

Mass spectrometric value (ESI-MS, m/z): 450 $(M+Na)^+$

Example 83: 3-(6,7-Dimethoxy-quinolin-4-yloxy)-2-(4-fluoro-phenyl)-[1,8]naphthyridine (compound 83)

2-Aminopyridine-3-carbaldehyde (100 mg) and 2-bromo-1-(4-fluoro-phenyl)-ethanone (178 mg) were suspended in a 5 N aqueous sodium hydroxide solution (0.6 ml), and the suspension was allowed to stand in an airtightly stoppered state for two days. The reaction mixture was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by thin layer chromatography with a chloroform-methanol system to give 2-(4-fluoro-phenyl)-[1,8]naphthyridin-3-ol (11 mg, yield 6%).

2-(4-Fluoro-phenyl)-[1,8]naphthyridin-3-ol (14 mg), 4-chloro-6,7-dimethoxyquinoline (39 mg), and 4-dimethylaminopyridine (21 mg) were suspended in 1,2-dichlorobenzene (1.5 ml), and the suspension was stirred at 140˚C for 9 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (14 mg, yield 57%).

$^1$H-NMR ($CDCl_3$, 400 MHz): δ 4.01 (s, 3H), 4.06 (s, 3H), 6.50 (d, J = 5.2 Hz, 1H), 7.04 (m, 2H), 7.46 (s, 1H), 7.48 (s, 1H), 7.53 (dd, J = 8.0, 4.4 Hz, 1H), 7.92 (s, 1H), 8.18 (m, 3H), 8.49 (d, J = 5.2 Hz, 1H), 9.16 (dd, J = 4.0, 2.0 Hz, 1H)

Mass spectrometric value (ESI-MS, m/z): 450 $(M+Na)^+$

Example 84: 2-(4-Chloro-phenyl)-3-(6,7-dimethoxy-quinolin-4-yloxy)-[1,8]naphthyridine (compound 84)

2-Aminopyridine-3-carbaldehyde (100 mg) and 2-chloro-1-(4-chloro-phenyl)-ethanone (155 mg) were suspended in a 5 N aqueous sodium hydroxide solution (0.6 ml), and the suspension was allowed to stand in an airtightly stoppered state for two days. The reaction mixture was neutralized with 10% hydrochloric acid, and dichloromethane was then added thereto for extraction. The dichloromethane layer was washed with water and saturated brine and was

dried over magnesium sulfate. The solvent was removed by distillation under the reduced pressure, and the residue was purified by thin layer chromatography with a chloroform-methanol system to give 2-(4-chloro-phenyl)-[1,8]naphthyridin-3-ol (51 mg, yield 24%).
2-(4-Chloro-phenyl)-[1,8]naphthyridin-3-ol (48 mg), 4-chloro-6,7-dimethoxyquinoline (125 mg), and 4-dimethylaminopyridine (69 mg) were suspended in 1,2-dichlorobenzene (2.0 ml), and the suspension was stirred at 140˚C for 4 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (40 mg, yield 47%).
$^1$H-NMR ($CDCl_3$, 400 MHz): δ 3.99 (s, 3H), 4.04 (s, 3H), 6.48 (d, J = 5.2 Hz, 1H), 7.33 (m, 2H), 7.43 (m, 2H), 7.51 (dd, J = 8.0, 4.0 Hz, 1H), 7.90 (s, 1H), 8.12 (m, 3H), 8.48 (d, J = 5.2 Hz, 1H), 9.14 (dd, J = 4.0, 2.0 Hz, 1H)
Mass spectrometric value (ESI-MS, m/z): 466 $(M+Na)^+$

Example 85: 2-(4-Bromo-phenyl)-3'-(6,7-dimethoxy-quinolin-4-yloxy)-[1,8]naphthyridine (compound 85)

2-(4-Bromo-phenyl)-[1,8]naphthyridin-3-ol (11 mg), 4-chloro-6,7-dimethoxyquinoline (24 mg), and 4-dimethylaminopyridine (13 mg) were suspended in 1,2-dichlorobenzene (1.5 ml), and the suspension was stirred at 140˚C for 5.5 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (9 mg, yield 50%).
$^1$H-NMR ($CDCl_3$, 400 MHz): δ 4.01 (s, 3H), 4.06 (s, 3H), 6.50 (d, J = 5.2 Hz, 1H), 7.45 (m, 2H), 7.49 (m, 3H), 7.92 (s, 1H), 8.05 (m, 2H), 8.14 (dd, J = 8.0, 4.0 Hz, 1H), 8.49 (d, J = 5.2 Hz, 1H), 9.16 (dd, J = 4.0, 2.0 Hz, 1H)
Mass spectrometric value (ESI-MS, m/z): 510 $(M+Na)^+$

Example 86: 4-[3-(6,7-Dimethoxy-quinolin-4-yloxy)-[1,8]naphthyridin-2-yl]-benzonitrile (compound 86)

2-Aminopyridine-3-carbaldehyde (100 mg) and 4-(2-bromo-acetyl)-benzonitrile (184 mg) were suspended in a 5 N aqueous sodium hydroxide solution (0.6 ml), and the suspension was allowed to stand in an airtightly stoppered state for two days. The reaction mixture was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by thin layer chromatography with a chloroform-methanol system to give 4-(3-hydroxy-[1,8]naphthyridin-2-yl)-benzonitrile (7 mg, yield 3%).
4-(3-Hydroxy-[1,8]naphthyridin-2-yl)-benzonitrile (7 mg), 4-chloro-6,7-dimethoxyquinoline (19 mg), and 4-dimethylaminopyridine (10 mg) were suspended in 1,2-dichlorobenzene (1.0 ml), and the suspension was stirred at 140˚C for 3.5 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give the title compound (2 mg, yield 16%).
$^1$H-NMR ($CDCl_3$, 400 MHz): δ 3.98 (s, 3H), 4.04 (s, 3H), 6.47 (d, J = 5.6 Hz, 1H), 7.14 (s, 1H), 7.55 (dd, J = 8.0, 4.0 Hz, 1H), 7.61 (m, 3H), 7.98 (s, 1H), 8.16 (m, 3H), 8.42 (d, J = 5.6 Hz, 1H), 9.16 (dd, J = 4.4, 2.0 Hz, 1H)
Mass spectrometric value (ESI-MS, m/z): 457 $(M+Na)^+$

Example 88: 3-(6,7-Dimethoxy-quinolin-4-yloxy)-2-pyridin-3-yl-[1,8]naphthyridine (compound 88)

2-Pyridin-3-yl-[1,8]naphthyridin-3-ol (17 mg), 4-chloro-6,7-dimethoxyquinoline (51 mg), and 4-dimethylaminopyridine (28 mg) were suspended in 1,2-dichlorobenzene (1.5 ml), and the suspension was stirred at 140˚C for 4 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give the title compound (15 mg, yield 48%).
$^1$H-NMR ($CDCl_3$, 400 MHz): δ 3.99 (s, 3H), 4.04 (s, 3H), 6.53 (d, J = 5.6 Hz, 1H), 7.34 (dd, J = 8.0, 4.4 Hz, 1H), 7.44 (s, 1H), 7.45 (s, 1H), 7.55 (dd, J = 8.0, 4.4 Hz, 1H), 7.95 (s, 1H), 8.15 (dd, J = 8.0, 1.6 Hz, 1H), 8.49 (m, 2H), 8.60 (dd, J = 5.2, 1.6 Hz, 1H), 9.17 (dd, J = 4.0, 2.0 Hz, 1H), 9.39 (d, J = 1.6 Hz, 1H)
Mass spectrometric value (ESI-MS, m/z): 433 $(M+Na)^+$

Example 89: 3-(6,7-Dimethoxy-quinolin-4-yloxy)-2-pyridin-4-yl-[1,8]naphthyridine (compound 89)

2-Amino-pyridine-3-carbaldehyde (100 mg) and 2-bromo-1-pyridin-4-yl-ethanone bromide (230 mg) were suspended in a 5 N aqueous sodium hydroxide solution (0.6 ml), and the suspension was allowed to stand in an airtightly stoppered state for two days. The reaction mixture was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by thin layer chromatography with a chloroform-methanol system to give 2-pyridin-4-yl-[1,8]naphthyridin-3-ol (3 mg, yield 2%).
2-Pyridin-4-yl-[1,8]naphthyridin-3-ol (11 mg), 4-chloro-6,7-dimethoxyquinoline (33 mg), and 4-dimethylaminopyridine (18 mg) were suspended in 1,2-dichlorobenzene (1.0 ml), and the suspension was stirred at 140˚C for 6 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give the title compound (7 mg, yield 35%).
$^1$H-NMR ($CDCl_3$, 400 MHz): δ 4.00 (s, 3H), 4.07 (s, 3H), 6.55 (d, J = 5.2 Hz, 1H), 7.47 (s, 1H), 7.53 (s, 1H), 7.58 (dd, J = 8.0, 4.4 Hz, 1H), 7.97 (s, 1H), 8.03 (m, 2H), 8.19 (dd, J = 8.0, 2.0 Hz, 1H), 8.52 (d, J = 5.2 Hz, 1H), 8.66 (m, 2H), 9.21 (dd, J = 8.0, 1.6 Hz, 1H)
Mass spectrometric value (ESI-MS, m/z): 433 $(M+Na)^+$

Example 90: 2-Benzo[b]thiophen-3-yl-3-(quinolin-4-yloxy)-[1,8]naphthyridine (compound 90)

2-Aminopyridine-3-carbaldehyde (100 mg) and 1-benzo[b]thiophen-3-yl-2-chloro-ethanone (173 mg) were suspended in a 5 N aqueous sodium hydroxide solution (0.6 ml), and the suspension was allowed to stand in an airtightly stoppered state for two days. The reaction mixture was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by thin layer chromatography with a chloroform-methanol system to give 2-benzo[b]thiophen-3-yl-[1,8]naphthyridin-3-ol (88 mg, yield 39%).
2-Benzo[b]thiophen-3-yl-[1,8]naphthyridin-3-ol (15 mg), 4- chloroquinoline (26 mg), and 4-dimethylaminopyridine (20 mg) were suspended in 1,2-dichlorobenzene (1.0 ml), and the suspension was stirred at 120˚C for 7 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (13 mg, yield 59%).
$^1$H-NMR ($CDCl_3$, 400 MHz): δ 6.59 (d, J = 5.2 Hz, 1H), 7.38 (dd, J = 8.0, 8.0 Hz, 1H), 7.47 - 7.62 (m, 3H), 7.80 (m, 2H), 7.97 (s, 1H), 8.13 (m, 2H), 8.22 (s, 1H), 8.28 (d, J = 8.4 Hz, 1H), 8.62 (d, J = 5.2 Hz, 1H), 8.86 (d, J = 8.4 Hz, 1H), 9.19 (dd, J = 4.4, 2.0 Hz, 1H)
Mass spectrometric value (ESI-MS, m/z): 428 $(M+Na)^+$

Example 91: 2-Benzo[b]thiophen-3-yl-3-(6,7-dimethoxy-quinolin-4-yloxy)-[1,8]naphthyridine (compound 91)

2-Benzo[b]thiophen-3-yl-[1,8]naphthyridin-3-ol (24 mg), 4-chloro-6,7-dimethoxyquinoline (58 mg), and 4-dimethylaminopyridine (32 mg) were suspended in 1,2-dichlorobenzene (1.0 ml), and the suspension was stirred at 140˚C for 4 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (29 mg, yield 72%).
$^1$H-NMR ($CDCl_3$, 400 MHz): δ 3.92 (s, 3H), 4.02 (s, 3H), 6.54 (d, J = 5.2 Hz, 1H), 7.36 - 7.54 (m, 5H), 7.83 (d, J = 8.4 Hz, 1H), 7.99 (s, 1H), 8.15 (m, 2H), 8.45 (d, J = 5.2 Hz, 1H), 8.80 (d, J = 8.4 Hz, 1H), 9.18 (dd, J = 4.0, 2.0 Hz, 1H)
Mass spectrometric value (ESI-MS, m/z): 488 $(M+Na)^+$

Example 92: 6,7-Dimethoxy-4-(2-pyrimidin-2-yl-quinolin-3-yloxy)-quinoline (compound 92)

2-Aminobenzaldehyde (650 mg) was dissolved in dichloromethane (20 ml). Chloroacetyl chloride (728 mg) was added to the solution, and the mixture was stirred at room temperature overnight. Water was added thereto, and the mixture was extracted with dichloromethane. The dichloromethane layer was washed with water and saturated brine and was dried over anhydrous sodium sulfate. The solvent was removed by distillation under the reduced pressure, and

the residue was purified by column chromatography with a chloroform system to give 2-chloro-N-(2-formyl-phenyl)-acetamide (1.05 g, yield 99%).
2-Chloro-N-(2-formyl-phenyl)-acetamide (960 mg) was dissolved in a mixed solvent composed of water (60 ml) and methanol (24 ml) at 100˚C. A 10% aqueous potassium hydroxide solution (12 ml) was added dropwise to the solution, and the mixture was heated under reflux for one hr. The reaction mixture was cooled to room temperature, and the methanol was removed under the reduced pressure. The residue was neutralized with a 1 N aqueous hydrochloric acid solution. The resultant precipitate was collected by filtration, was washed with ethyl acetate, and was dried in vacuo to give 2,3-dihydroxyquinoline (450 mg, yield 26%).
2,3-Dihydroxyquinoline (450 mg), 4-chloro-6,7-dimethoxyquinoline (575 mg), and 4-dimethylaminopyridine (472 mg) were suspended in 1,2-dichlorobenzene (12 ml), and the suspension was stirred at 135˚C for 5.5 hr. The reaction mixture was cooled to room temperature, water was added thereto, and the mixture was extracted with ethyl acetate. The ethyl acetate layer was then washed with water and saturated brine and was dried over anhydrous magnesium sulfate. The solvent was removed by distillation under the reduced pressure, and the residue was purified by column chromatography with an acetone-chloroform system to give 3-(6,7-dimethoxy-quinolin-4-yloxy)-quinolin-2-ol (344 mg, yield 77%).

3-(6,7-Dimethoxy-quinolin-4-yloxy)-quinolin-2-ol (50 mg), diphosphorus pentaoxide (51 mg), and tetrabutylammonium bromide (69 mg) were suspended in 1,2-dichlorobenzene (1 ml), and the suspension was stirred at 140˚C for 1.5 hr. The reaction mixture was cooled to room temperature, and a 10% aqueous sodium hydrogencarbonate solution was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give 4-(2-bromo-quinolin-3-yloxy)-6,7-dimethoxy-quinoline (5 mg, yield 8%).
4-(2-Bromo-quinolin-3-yloxy)-6,7-dimethoxy-quinoline (50 mg), tetrakistriphenylphosphine palladium (0) (28 mg), and copper(II) oxide (19 mg) were suspended in N,N-dimethylformamide (1.5 ml). 2-Tributylstannylpyrimidine (90 mg) was added to the suspension, and the mixture was stirred at 100˚C overnight. The reaction mixture was cooled to room temperature and was then filtered. The solvent was removed from the filtrate by distillation under the reduced pressure. Water was added thereto, and the mixture was extracted with dichloromethane. The dichloromethane layer was washed with water and saturated brine and was dried over anhydrous magnesium sulfate. The solvent was removed by distillation under the reduced pressure, and the residue was purified by column chromatography with a chloroform-methanol system to give the title compound (28 mg, yield 56%).
$^1$H-NMP, ($CDCl_3$, 400 MHz): δ 4.05 (s, 6H), 6.44 (d, J = 5.2 Hz, 1H), 7.43 - 7.78 (m, 8H), 7.88 (s, 1H), 8.11 (d, J = 8.4 Hz, 1H), 8.52 (d, J = 5.2 Hz, 1H)
Mass spectrometric value (ESI-MS, m/z): 411 ($M^+$+1)

Example 93: 3-(6,7-Dimethoxy-quinolin-4-yloxy)-2-phenyl-[1,6]naphthyridine (compound 93)

4-Aminopyridine-3-carbaldehyde (100 mg) and 2-bromo-1-phenyl-ethanone (127 mg) were suspended in a 5 N aqueous sodium hydroxide solution (0.6 ml), and the suspension was allowed to stand in an airtightly stoppered state for two days. The reaction mixture was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by thin layer chromatography with a chloroform-methanol system to give 2-phenyl-[1,6]naphthyridin-3-ol (9 mg, yield 5%).
2-Phenyl-[1,6]naphthyridin-3-ol (17 mg), 4-chloro-6,7-dimethoxyquinoline (51 mg), and 4-dimethylaminopyridine (28 mg) were suspended in 1,2-dichlorobenzene (1.5 ml), and the suspension was stirred at 140˚C for 7.5 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (17 mg, yield 54%).
$^1$H-NMR ($CDCl_3$, 400 MHz): δ 4.00 (s, 3H), 4.04 (s, 3H), 6.51 (d, J = 5.2 Hz, 1H), 7.38 (m, 3H), 7.44 (m, 2H), 8.02 (m, 4H), 8.47 (d, J = 9.2 Hz, 1H), 8.79 (d, J = 5.2 Hz, 1H), 9.23 (s, 1H)
Mass spectrometric value (ESI-MS, m/z): 410 $(M+1)^+$

Example 95: 3-(6,7-Dimethoxy-quinolin-4-yloxy)-2-pyridin-3-yl-[1,6]naphthyridine (compound 95)

2-Pyridin-3-yl-[1,6]naphthyridin-3-ol (18 mg), 4-chloro-6,7-dimethoxyquinoline (54 mg), and 4-dimethylaminopyridine (30 mg) were suspended in 1,2-dichlorobenzene (1.5 ml), and the suspension was stirred at 140˚C for 5.5 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by

thin layer chromatography with a chloroform-methanol system to give the title compound (7 mg, yield 21%).
$^1$H-NMR ($CDCl_3$, 400 MHz): δ 4.01 (s, 3H), 4.05 (s, 3H), 6.53 (d, J = 5.2 Hz, 1H), 7.35 (dd, J = 8.4, 5.2 Hz, 1H), 7.43 (s, 1H), 7.50 (s, 1H), 8.04 (m, 2H), 8.34 (m, 1H), 8.50 (d, J = 5.2 Hz, 1H), 8.63 (dd, J = 4.8, 1.6 Hz, 1H), 8.83 (d, J = 5.2 Hz, 1H), 9.26 (s, 1H), 9.30 (d, J = 1.6 Hz, 1H)
Mass spectrometric value (ESI-MS, m/z): 433 $(M+Na)^+$

Example 97: 3-(6,7-Dimethoxy-quinolin-4-yloxy)-2-methyl-[1,7]naphthyridine (compound 97)

(4-Formyl-pyridin-3-yl)-carbamic acid tert-butyl ester (100 mg) was dissolved in a hydrochloric acid-methanol solution (2.0 ml), and the solution was stirred under reflux for 30 min. The solvent was removed by distillation under the reduced pressure. 1-Chloro-propan-2-one (42 mg) dissolved in a 5 N aqueous sodium hydroxide solution (0.5 ml) was added to the residue, and the mixture was allowed to stand in an airtightly stoppered state for two days. The reaction mixture was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by column chromatography with a chloroform-acetone system to give 2-methyl-[1,7]naphthyridin-3-ol (22 mg, yield 31%).
2-Methyl-[1,7]naphthyridin-3-ol (22 mg), 4-chloro-6,7-dimethoxyquinoline (92 mg), and 4-dimethylaminopyridine (50 mg) were suspended in 1,2-dichlorobenzene (1.5 ml), and the suspension was stirred at 140°C for 8.5 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by column chromatography with a chloroform-methanol system to give the title compound (25 mg, yield 53%). $^1$H-NMR ($CDCl_3$, 400 MHz): δ 2.76 (s, 3H), 4.01 (s, 3H), 4.06 (s, 3H), 6.54 (d, J = 5.6 Hz, 1H), 7.44 (s, 1H), 7.49 (s, 1H), 7.52 (d, J = 6.0 Hz, 1H), 7.61 (s, 1H), 8.57 (m, 2H), 9.45 (s, 1H)
Mass spectrometric value (ESI-MS, m/z): 348 $(M+1)^+$

Example 98: 3-(6,7-Dimethoxy-quinolin-4-yloxy)-2-phenyl-[1,7]naphthyridine (compound 98)

(4-Formyl-pyridin-3-yl)-carbamic acid tert-butyl ester (100 mg) was dissolved in a hydrochloric acid-methanol solution (2.0 ml), and the solution was stirred under reflux for 30 min. The solvent was removed by distillation under the reduced pressure. 2-Chloro-1-phenyl-ethanone (70 mg) dissolved in a 5 N aqueous sodium hydroxide solution (0.6 ml) was then added to the residue, and the mixture was allowed to stand in an airtightly stoppered state for two days. The reaction solution was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by thin layer chromatography with a chloroform-acetone system to give 2-phenyl-[1,7]naphthyridin-3-ol (3 mg, yield 3%).
2-Phenyl-[1,7]naphthyridin-3-ol (3 mg), 4-chloro-6,7-dimethoxyquinoline (9 mg), and 4-dimethylaminopyridine (5 mg) were suspended in 1,2-dichlorobenzene (1.0 ml), and the suspension was stirred at 140°C for 9 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give the title compound (3 mg, yield 54%).
$^1$H-NMR ($CDCl_3$, 400 MHz): δ 4.00 (s, 3H), 4.07 (s, 3H), 6.59 (d, J = 5.6 Hz, 1H), 7.39 (m, 4H), 7.61 (m, 2H), 7.86 (s, 1H), 7.98 (m, 2H), 8.50 (d, J = 5.6 Hz, 1H), 8.64 (d, J = 5.6 Hz, 1H) 9.62 (s, 1H)
Mass spectrometric value (ESI-MS, m/z): 432 $(M+Na)^+$

Example 99: 7-Methoxy-4-(2-phenyl-5,6,7,8-tetrahydro-[1,8]naphthyridin-3-yloxy)-quinoline-6-carboxylate benzylamide (compound 99)

7-Methoxy-4-(2-phenyl-[1,8]naphthyridin-3-yloxy)-quinoline-6-carboxylate benzylamide (compound 65) (164 mg) was dissolved in N,N-dimethylformamide (4 ml). Triethylamine (0.4 ml) and 20% palladium hydroxide (16 mg) were added to the solution, and the mixture was stirred under a hydrogen gas atmosphere at room temperature overnight. The reaction mixture was filtered and was then washed with chloroform. The solvent was removed by distillation under the reduced pressure, water was added to the residue, and the organic layer was extracted with ethyl acetate. The ethyl acetate layer was then washed with water and saturated brine and was dried over anhydrous sodium sulfate. The solvent was removed by distillation under the reduced pressure, and the residue was purified by thin layer chromatography with a methanol-chloroform system to give the title compound (138 mg, yield 84%).
$^1$H-NMR ($CDCl_3$, 400 MHz): δ 2.00 (m, 2H), 2.81(t, J = 6.1 Hz, 2H), 3.50 (m, 2H), 4.04 (s, 3H), 4.76 (d, J = 5.6 Hz, 2H),

6.35 (d, J = 5.1 Hz, 1H), 7.07 (s, 1H), 7.15 - 7.45 (m, 8H), 7.75 - 7.78 (m, 2H), 8.22 (m, 1H), 8.51 (d, J = 5.4 Hz, 1H), 9.29 (s, 1H)
Mass spectrometric value (ESI-MS, m/z): 539 $(M+Na)^+$

Example 100: 1-[2-(1H-[1,8]naphthyridin-4-ylideneamino)-pyridin-3-yl]-ethanone (compound 100)

3-Acetyl-2-aminopyridine (25 mg) was dissolved in anhydrous tetrahydrofuran (1 ml). Sodium methoxide (50 mg) was added to the solution, and the mixture was stirred at room temperature. Ethyl formate (68 mg) was added thereto, and the mixture was stirred at room temperature overnight. Anhydrous N,N-dimethylformamide (1 ml) was added to the reaction mixture, and the mixture was stirred at 90˚C for another overnight period. The reaction mixture was cooled to room temperature, water was added to the cooled mixture, and the mixture was extracted with ethyl acetate. The ethyl acetate layer was washed with water and was then dried over anhydrous sodium sulfate. The solvent was removed by distillation under the reduced pressure, and the residue was purified by thin layer chromatography with a hexane-acetone system to give the title compound (4.6 mg, yield 9%).
$^1$H-NMR ($CDCl_3$, 400 MHz): δ 2.82 (s, 3H), 7.19 (dd, J = 7.8, 4.6 Hz, 1H), 7.56 (dd, J = 8.3, 4.1 Hz, 1H), 7.80 (s, 1H), 8.22 (dd, J = 7.8, 1.5 Hz, 1H), 8.37 (dd, J = 4.6, 1.4 Hz, 1H), 8.41 (dd, J = 8.5, 2.0 Hz, 1H), 9.16 (dd, J = 4.1, 1.9 Hz, 1H), 9.73 (d, J = 10.0 Hz, 1H), 12.65 - 12.73 (m, 1H)
Mass spectrometric value (ESI-MS, m/z): 287 $(M+Na)^+$

Example 101: (6,7-Dimethoxy-quinazolin-4-yl)-(2-phenyl-[1,8]naphthyridin-3-yl)-amine (compound 101)

2-Amino-1-phenyl-ethanone hydrochloride (1.00 g) was dissolved in acetonitrile (15 ml). Di-tert-butyl dicarbonate (1.58 g), triethylamine(1.30 g), and 4-dimethylaminopyridine (71 mg) were added to the solution, and the mixture was stirred at room temperature for one hr. Water and dichloromethane were added to the reaction mixture for extraction. The dichloromethane layer was washed with water and saturated brine and was dried over magnesium sulfate. The solvent was removed by distillation under the reduced pressure, and the residue was purified by column chromatography with a hexane-ethyl acetate system to give (2-oxo-2-phenyl-ethyl)-carbamic acid tert-butyl ester (950 mg, yield 70%).
2-Aminopyridine-3-carbaldehyde (150 mg) and (2-oxo-2-phenyl-ethyl)-carbamic acid tert-butyl ester (289 mg) were suspended in a 5 N aqueous sodium hydroxide solution (1.0 ml), and the suspension was allowed to stand in an airtightly stoppered state for two days. The reaction mixture was neutralized with 10% hydrochloric acid, and dichloromethane was then added thereto for extraction. The dichloromethane layer was washed with water and saturated brine and was dried over magnesium sulfate. The solvent was removed by distillation under the reduced pressure, and the residue was purified by column chromatography with a chloroform-acetone system to give (2-phenyl-[1,8]naphthyridin-3-yl)-carbamic acid tert-butyl ester (251 mg, yield 64%).
(2-Phenyl-[1,8]naphthyridin-3-yl)-carbamic acid tert-butyl ester (247 mg) was dissolved in a hydrochloric acid-methanol solution (10 ml), and the solution was heated under reflux for 45 min. The reaction mixture was neutralized with 10% sodium hydroxide, and dichloromethane was then added thereto for extraction. The dichloromethane layer was washed with water and saturated brine and was dried over magnesium sulfate. The solvent was removed by distillation under the reduced pressure, and the residue was purified by column chromatography with a chloroform-acetone system to give 2-phenyl-[1,8]naphthyridin-3-ylamine (155 mg, yield 91%).
2-Phenyl-[1,8]naphthyridin-3-ylamine (20 mg) and 4-chloro-6,7-dimethoxyquinazoline (51 mg) were suspended in 2-propanol (3.0 ml). A hydrochloric acid-methanol solution (0.2 ml) was added to the suspension, and the mixture was stirred under reflux overnight. The solvent was removed by distillation under the reduced pressure. A saturated aqueous sodium hydrogencarbonate solution was added to the residue, and the mixture was extracted with chloroform. The chloroform layer was washed with water and saturated brine and was dried over anhydrous magnesium sulfate. The solvent was then removed by distillation under the reduced pressure, and the residue was purified by column chromatography with a chloroform-acetone system to give the title compound (4 mg, yield 11%).
$^1$H-NMR ($CDCl_3$, 400 MHz): δ 3.78 (s, 3H), 4.01 (s, 3H), 6.55 (brs, 1H), 7.30 (s, 1H), 7.55 (m, 4H), 7.85 (m, 2H), 8.31 (dd, J = 8.4, 2.0 Hz, 1H), 8.75 (s, 1H), 9.03 (dd, J = 4.0, 2.0 Hz, 1H), 9.68 (brs, 1 H),
Mass spectrometric value (ESI-MS, m/z): 432 $(M+Na)^+$

Example 101: 3-(6,7-Difluoro-quinolin-4-yloxy)-2-phenyl-[1,8]naphthyridine (compound 101)

3,4-Difluoroaniline (1.32 g) and 5-methoxymethylene-2,2-dimethyl-[1,3]dioxa,n-4,6-dione (2.0 g) were dissolved in 2-propanol (40 ml), and the solution was stirred at 70˚C for 30 min. The reaction mixture was filtered and was washed with methanol and then with ether. The residue as such was used in the next reaction without purification.
The residue and biphenyl (5.8 g) were suspended in diphenyl ether (20 ml), and the suspension was stirred at 220˚C for one hr. The reaction mixture was filtered and was washed with chloroform, and the residue as such was used in the

next reaction without purification.
The residue was suspended in diisopropylethylamine (7 ml), phosphorus oxychloride (2 ml) was added to the suspension, and the mixture was stirred at 100˚C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an ethyl acetate-hexane system to give 4-chloro-6,7-difluoro-quinoline (0.94 g, yield 46%) (3 steps).

2-Phenyl-[1,8]naphthyridin-3-ol (56 mg), 4-chloro-6,7-difluoro-quinoline (50 mg), and 4-dimethylaminopyridine (92 mg) were suspended in 1,2-dichlorobenzene (1.5 ml), and the suspension was stirred at 130˚C for 6 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (24 mg, yield 25%).
$^1$H-NMR ($CDCl_3$, 400 MHz): δ 6.49 (d, J = 5.4 Hz, 1H), 7.32 - 7.36 (m, 3H), 7.58 (dd, J = 4.1, 8.1 Hz, 1H), 7.96 (m, 1H), 8.04 - 8.07 (m, 3H), 8.13 (m, 1H), 8.22 (dd, J = 2.0, 8.3 Hz, 1H), 8.58 (d, J = 5.4 Hz, 1H), 9.22 (dd, J = 2.0, 4.4 Hz, 1H)
Mass spectrometric value (ESI-MS, m/z): 408 $(M+Na)^+$

Example 102: 2-Phenyl-3-(5,6,7-trifluoro-quinolin-4-yloxy)-[1,8]naphthyridine (compound 102)

3,4,5-Trifluoroaniline (1.20 g) and 5-methoxymethylene-2,2-dimethyl-[1,3]dioxan-4,6-dione (1.93 g) were dissolved in 2-propanol (40 ml), and the solution was stirred at 70˚C for 30 min. The reaction mixture was filtered and was washed with methanol and then with ether, and the residue was used in the next reaction without purification.
The residue and biphenyl (6.3 g) were suspended in diphenyl ether (25 ml), and the suspension was stirred at 220˚C for one hr. The reaction mixture was filtered and was washed with chloroform, and the residue as such was used in the next reaction without purification.
The residue was suspended in diisopropylethylamine (5 ml), phosphorus oxychloride (1 ml) was added to the suspension, and the mixture was stirred at 100˚C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an ethyl acetate-hexane system to give 4-chloro-5,6,7-trifluoro-quinoline (176 mg, yield 10%) (3 steps).

2-Phenyl-[1,8]naphthyridin-3-ol (51 mg), 4-chloro-5,6,7-trifluoro-quinoline (50 mg), and 4-dimethylaminopyridine (84 mg) were suspended in 1,2-dichlorobenzene (1.5 ml), and the suspension was stirred at 130˚C for 5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (6 mg, yield 6%).
$^1$H-NMR ($CDCl_3$, 400 MHz): δ 6.45 (d, J = 5.1 Hz, 1H), 7.34 - 7.39 (m, 3H), 7.57 (dd, J = 4.1, 8.1 Hz, 1H), 7.72 (m, 1H), 8.00 (s, 1H), 8.13 - 8.15 (m, 2H), 8.21 (dd, J = 2.0, 8.1 Hz, 1H), 8.53 (d, J = 5.1 Hz, 1H), 9.20 (dd, J = 2.0, 4.1 Hz, 1H)
Mass spectrometric value (ESI-MS, m/z): 426 $(M+Na)^+$

Example 103: 3-(6-Fluoro-quinolin-4-yloxy)-5,6-dimethyl-[2,2']bipyridine (compound 103)

4-Chloro-6-fluoro-quinoline (50 mg), 5,6-dimethyl-[2,2']bipyridinyl-3-ol (55 mg), and 4-dimethylaminopyridine (101 mg) were dissolved in dimethylsulfoxide (1.5 ml), cesium carbonate (269 mg) was added to the suspension, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (87 mg, yield 90%).
$^1$H-NMR ($CDCl_3$, 400 MHz): δ 2.41 (s, 3H), 2.67 (s, 3H), 6.51 (d, J = 3.7 Hz, 1H), 7.10 (ddd, J = 1.0, 4.9, 7.6 Hz, 1H), 7.38 (s, 1H), 7.54 - 7.64 (m, 2H), 7.90 (d, J = 7.6 Hz, 1H), 7.98 (dd, J = 2.7, 9.0 Hz, 1H), 8.21 (m, 1H), 8.36 (m, 1H), 8.54 (d, J = 5.4 Hz, 1H)
Mass spectrometric value (ESI-MS, m/z): 368 $(M+Na)^+$

Example 104: 3-(7-Fluoro-6-methoxy-quinolin-4-yloxy)-5,6-dimethyl-[2,2']bipyridine (compound 104)

4-Chloro-7-fluoro-6-methoxy-quinoline (50 mg), 5,6-dimethyl-[2,2']bipyridinyl-3-ol (47 mg), and 4-dimethylaminopyridine (87 mg) were dissolved in dimethylsulfoxide (1.5 ml), cesium carbonate (231 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (47 mg, yield 52%).
$^1$H-NMR ($CDCl_3$, 400 MHz): δ 2.41 (s, 3H), 2.67 (s, 3H), 4.04 (s, 3H), 6.47 (d, J = 5.6 Hz, 1H), 7.11 (ddd, J = 1.0, 4.9, 7.6 Hz, 1H), 7.39 (s, 1H), 7.63 (m, 1H), 7.70 (d, J = 9.0 Hz, 1H), 7.88 - 7.90 (m, 2H), 8.35 (m, 1H), 8.45 (d, J = 5.6 Hz, 1H)
Mass spectrometric value (ESI-MS, m/z): 398 $(M+Na)^+$

Example 105: 3-(7-Chloro-6-methoxy-quinolin-4-yloxy)-5,6-dimethyl-[2,2']bipyridine (compound 105)

4,7-Dichloro-6-methoxy-quinoline (50 mg), 5,6-dimethyl-[2,2']bipyridinyl-3-ol (44 mg), and 4-dimethylaminopyridine (80 mg) were dissolved in dimethylsulfoxide (1.5 ml), cesium carbonate (214 mg) was added to the solution, and the mixture was stirred at 130˚C for overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (80 mg, yield 93%).
$^1$H-NMR ($CDCl_3$, 400 MHz): δ 2.41 (s, 3H), 2.67 (s, 3H), 4.05 (s, 3H), 6.47 (d, J = 5.4 Hz, 1H), 7.11 (ddd, J = 1.2, 5.1, 7.6 Hz, 1M), 7.39 (s, 1H), 7.63 (m, 1H), 7.66 (s, 1H), 7.88 (d, J = 7.8 Hz, 1H), 8.22 (m, 1H), 8.36 (m, 1H), 8.44 (d, J = 5.4 Hz, 1H)
Mass spectrometric value (ESI-MS, m/z): 414 $(M+Na)^+$

Example 106: 3-[4-(5,6-Dimethyl-[2,2']bipyridinyl-3-yloxy)-quinolin-7-yl]-phenol (compound 106)

N,N-Dimethylformamide (1 ml) and a 2 M aqueous potassium carbonate solution (0.5 ml) were added to 3-(7-Bromo-quinolin-4-yloxy)-5,6-dimethyl-[2,2']bipyridine (compound 14) (30 mg), tetrakistriphenylphosphine palladium (9 mg), and 3-hydroxyphenylboric acid (31 mg) under an argon atmosphere, and the mixture was stirred at 70˚C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (31 mg, yield 100%).
$^1$H-NMR (DMSO-$d_6$, 400 MHz): δ 2.35 (s, 3H), 2.54 (s, 3H), 6.49 (d, J = 5.1 Hz, 1H), 6.83 (ddd, J = 8.0, 2.4, 1.0 Hz, 1H), 7.16 - 7.27 (m, 3H), 7.33 (dd, J = 7.8, 7.8 Hz, 1H), 7.66 (s, 1H), 7.76 (ddd, J = 7.8, 7.8, 2.0 Hz, 1H), 7.83 - 7.89 (m, 2H), 8.11 (d, J = 1.7 Hz, 1H), 8.20 - 8.23 (m, 2H), 8.31 (d, J = 1.8 Hz, 1H), 8.57 (d, J = 5.1 Hz, 1H)
Mass spectrometric value (ESI-MS, m/z): 418 $(M-1)^-$

Example 107: 4-[4-(5,6-Dimethyl-[2,2']bipyridinyl-3-yloxy)-quinolin-7-yl]-phenol (compound 107)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (compound 14) (30 mg), tetrakistriphenylphosphine palladium (9 mg), and 4-hydroxyphenylboric acid (31 mg) under an argon atmosphere, and the mixture was stirred at 70˚C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (31 mg, yield 100%).
$^1$H-NMR (DMSO-$d_6$, 400 MHz): δ 2.35 (s, 3H), 2.54 (s, 3H), 6.45 (d, J = 5.1 Hz, 1H), 6.90 (d, J = 8.6 Hz, 2H), 7.19 (ddd, J = 7.3, 4.9, 1.2 Hz, 1H), 7.64 - 7.70 (m, 3H), 7.75 (ddd, J = 7.8, 7.8, 1.7 Hz, 1H), 7.83 - 7.90 (m, 2H), 8.08 (d, J = 1.7 Hz, 1H), 8.20 - 8.24 (m, 1H), 8.27 (d, J = 8.8 Hz, 1H), 8.54 (d, J = 5.2 Hz, 1H)
Mass spectrometric value (ESI-MS, m/z): 418 $(M-1)^-$

Example 108: 2-[4-(5,6-Dimethyl-[2,2']bipyridinyl-3-yloxy)-quinolin-7-yl]-phenylamine (compound 108)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (compound 14) (30 mg), tetrakistriphenylphosphine palladium (9 mg), and 2-aminophenylboric acid (30 mg) under an argon atmosphere and the mixture was stirred at 70°C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by thin layer chromatography with chloroform-methanol to give the title compound (31 mg, yield 100%).
$^1$H-NMR ($CDCl_3$, 400 MHz): δ 2.40 (s, 3H), 2.67 (s, 3H), 3.86 (bs, 2H), 6.46 (d, J = 5.1 Hz, 1H), 6.81 (d, J = 8.0 Hz, 1H), 6.88 (dd, J = 7.6, 7.6 Hz, 1H), 7.09 - 7.15 (m, 1H), 7.17 - 7.28 (m, 2H), 7.38 (s, 1H), 7.60 (ddd, J = 7.8, 7.8, 1.7 Hz, 1H), 7.69 (dd, J = 8.5, 1.4 Hz, 1H), 7.85 (d, J = 8.0 Hz, 1H), 8.14 (s, 1H), 8.41 (d, J = 8.6 Hz, 1H), 8.53 (d, J = 4.6 Hz, 1H), 8.60 (d, J = 5.4 Hz, 1H)
Mass spectrometric value (ESI-MS, m/z): 419 $(M+1)^+$

Example 110: 3-(6-Fluoro-7-methoxy-quinolin-4-yloxy)-5,6-dimethyl-[2,2']bipyridine (compound 110)

4-Chloro-6-fluoro-7-methoxy-quinoline (50 mg), 5,6-dimethyl-[2,2']bipyridinyl-3-ol (47 mg), and 4-dimethylaminopyridine (87 mg) were dissolved in dimethylsulfoxide (1.5 ml), cesium carbonate (231 mg) was added to the solution, and the mixture was stirred at 130°C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (50 mg, yield 55%).
$^1$H-NMR ($CDCl_3$, 400 MHz): δ 2.41 (s, 3H), 2.66 (s, 3H), 4.07 (s, 3H), 6.43 (d, J = 5.6 Hz, 1H), 7.10 (ddd, J = 1.2, 4.9, 7.6 Hz, 1H), 7.38 (s, 1H), 7.60 - 7.68 (m, 2H), 7.90 (d, J = 8.1 Hz, 1H), 7.97 (d, J = 11.2 Hz, 1H), 8.34 (m, 1H), 8.46 (d, J = 5.6 Hz, 1H)
Mass spectrometric value (ESI-MS, m/z): 398 $(M+Na)^+$

Example 111: 3-(6,8-Difluoro-quinolin-4-yloxy)-5,6-dimethyl-[2,2']bipyridine (compound 111)

4-Chloro-6,8-difluoro-quinoline (50 mg), 5,6-dimethyl-[2,2']bipyridinyl-3-ol (50 mg), and 4-dimethylaminopyridine (92 mg) were dissolved in dimethylsulfoxide (1.5 ml), cesium carbonate (245 mg) was added to the solution, and the mixture was then stirred at 130°C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (18 mg, yield 20%).
$^1$H-NMR ($CDCl_3$, 400 MHz): δ 2.41 (s, 3H), 2.68 (s, 3H), 6.54 (d, J = 5.4 Hz, 1H), 7.17 (m, 1H), 7.28 (m, 1H), 7.37 (s, 1H), 7.68 (m, 1H), 7.79 (ddd, J = 1.5, 4.1, 9.0 Hz, 1H), 7.91 (d, J = 7.8 Hz, 1H), 8.48 (m, 1H), 8.59 (d, J = 5.1 Hz, 1H)
Mass spectrometric value (ESI-MS, m/z): 386 $(M+Na)^+$

Example 113: Methyl 4-(2-Phenyl-[1,8]naphthyridin-3-yloxy)-quinoline-7-carboxarlate (compound 113)

3-Amino-2,5-dichlorobenzoic acid (1.74 g), 1-ethyl-3-(3-dimethylaminopropyl)carbodiimide hydrochloride (3.70 g), and 1-hydroxybenzotriazole hydrate (2.66 g) were dissolved in N,N-dimethylformamide (40 ml). Methanol (5 ml) was added to the solution, and the mixture was stirred at room temperature for 5 hr. Water was added to the reaction mixture, and the mixture was extracted with ethyl acetate. The ethyl acetate layer was then washed with water and saturated brine and was dried over anhydrous sodium sulfate. The solvent was removed by distillation under the reduced pressure, and the residue was purified by column chromatography with an ethyl acetate-hexane system to give methyl 3-amino-2,5-dichlorobenzoate (1.45 g, yield 78%).
Methyl 3-amino-2,5-dichlorobenzoate (1.45 g) and 5-methoxymethylene-2,2-dimethyl-[1,3]dioxan-4,6-dione (1.50 g) were suspended in 2-propanol (40 ml), and the suspension was stirred at 70°C for 30 min. The reaction mixture was cooled to room temperature, and the precipitated crystal was then collected by filtration and was washed with methanol and then with ether. The crystal thus obtained as such was used in the next reaction without further purification.
The crystal prepared above and biphenyl (5.8 g) were suspended in diphenyl ether (25 ml), and the suspension was

stirred at 220˚C for 30 min. The reaction mixture was cooled to room temperature, and the precipitated crystal was collected by filtration and was washed with chloroform. The crystal thus obtained (1.19 g) as such was used in the next reaction without further purification.

A part of the residue (0.59 g) was dissolved in N,N-dimethylformamide (20 ml). Triethylamine (4 ml) and 10% palladium/carbon (0.59 g) were added to the solution, and the mixture was stirred under a hydrogen gas atmosphere at room temperature overnight. The reaction mixture was filtered and was then washed with chloroform. The solvent was removed by distillation under the reduced pressure, and the residue was washed with methanol to give methyl 4-oxo-1,4-dihydro-quinoline-7-carboxylate (187 mg, yield 28%) (3 steps).

Methyl 4-oxo-1,4-dihydro-quinoline-7-carboxylate (650 mg) was suspended in diisopropylethylamine (7 ml), phosphorus oxychloride (1.5 ml) was added to the suspension, and the mixture was stirred at 120˚C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an acetone-chloroform system to give methyl 4-chloro-quinoline-7-carboxylate (609 mg, yield 86%).

2-Phenyl-[1,8]naphthyridin-3-ol (100 mg), methyl 4-chloro-quinoline-7-carboxylate (100 mg), and 4-dimethylaminopyridine (165 mg) were suspended in 1,2-dichlorobenzene (3 ml), and the suspension was stirred at 130˚C for 5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (143 mg, yield 78%).

$^1$H-NMR ($CDCl_3$, 400 MHz): δ 4.03 (s, 3H), 6.56 (d, J = 5.1 Hz, 1H), 7.29 - 7.35 (m, 3H), 7.57 (dd, J = 4.4, 8.3 Hz, 1H), 8.04 (s, 1H), 8.10 (m, 1H), 8.21 (m, 2H), 8.25 (dd, J = 1.7, 8.8 Hz, 1H), 8.46 (d, J = 8.8 Hz, 1H), 8.69 (d, J = 5.4 Hz, 1H), 8.85 (s, 1H), 9.21 (dd, J = 2.0, 4.1 Hz, 1H)

Mass spectrometric value (ESI-MS, m/z): 430 $(M+Na)^+$

Example 115: 3-(6,7-Dimethoxy-quinolin-4-yloxy)-6-fluoro-[2,2']bipyridine (compound 115)

2-Fluoro-5-hydroxypyridine (2.00 g) and iodine (44.9 g) were dissolved in a mixed solvent composed of methanol (40 ml) and water (20 ml), and the mixture was stirred at room temperature for 167 hr. Sodium sulfite was added to the reaction mixture until the solution became transparent. Methanol was then removed from the reaction mixture under the reduced pressure. Water was added to the residue, and the mixture was extracted with chloroform. The chloroform layer was washed with water and saturated brine and was dried over anhydrous magnesium sulfate. The solvent was removed by distillation under the reduced pressure, and the residue was purified by column chromatography with an acetone-chloroform system to give 6-fluoro-2-iodo-pyridin-3-ol (761 mg, yield 18%).

6-Fluoro-2-iodo-pyridin-3-ol (135 mg), 4-chloro-6,7-dimethoxyquinoline (378 mg), and 4-dimethylaminopyridine (207 mg) were suspended in 1,2-dichlorobenzene (8 ml), and the suspension was stirred at 130˚C for 3.5 hr. The reaction mixture was cooled to room temperature, and water was added thereto. The mixture was extracted with ethyl acetate, and the ethyl acetate layer was then washed with water and saturated brine and was dried over anhydrous magnesium sulfate. The solvent was removed by distillation under the reduced pressure, and the residue was purified by column chromatography with an acetone-hexane system to give 4-(6-fluoro-2-iodo-pyridin-3-yloxy)-6,7-dimethoxy-quinoline (89 mg, yield 37%).

4-(6-Fluoro-2-iodo-pyridin-3-yloxy)-6,7-dimethoxy-quinoline (89 mg), tetrakistriphenylphosphine palladium (48 mg), and copper(II) oxide (33 mg) were suspended in N,N-dimethylformamide (2 ml). 2-Tributylstannylpyridine (154 mg) was added to the suspension, and the mixture was stirred at 100˚C overnight. The reaction mixture was cooled to room temperature, and the reaction mixture was filtered. The solvent was removed from the filtrate by distillation under the reduced pressure. Water was then added thereto, and the mixture was extracted with dichloromethane. The dichloromethane layer was washed with water and saturated brine and was dried over anhydrous magnesium sulfate. The solvent was removed by distillation under the reduced pressure, and the residue was purified by thin layer chromatography with a chloroform-methanol system to give the title compound (31 mg, yield 39%).

$^1$H-NMR ($CDCl_3$, 400 MHz): δ 4.03 (s, 3H), 4.04 (s, 3H), 6.36 (d, J = 5.2 Hz, 1H), 7.10 (dd, J = 8.8, 4.0 Hz, 1H), 7.26 (ddd, J = 7.6, 4.4, 0.8 Hz, 1H), 7.39 (s, 1H), 7.56 (s, 1H), 7.66 (ddd, J = 9.2, 7.2, 1.6 Hz, 1H), 7.74 (dd, J = 8.4, 6.4 Hz, 1H), 7.90 (d, J = 5.2 Hz, 1H), 8.34 (m, 2H)

Mass spectrometric value (ESI-MS, m/z): 400 $(M+Na)^+$

Example 116: Methyl 6-methoxy-4-(2-phenyl-[1,8]naphthyridin-3-yloxy)-quinoline-7-carboxylate (compound 116)

Methyl 2-methoxy-5-nitro-benzoate (300 mg), ammonium chloride (228 mg), and zinc (929 mg) were suspended in ethanol (10 ml) and water (0.5 ml), and the suspension was stirred under reflux for 3 hr. The reaction mixture was filtered, and the solvent was removed from the filtrate by distillation under the reduced pressure. A saturated aqueous sodium hydrogencarbonate solution was added to the residue, and the mixture was extracted with chloroform. The chloroform layer was washed with water and saturated brine and was dried over anhydrous magnesium sulfate. The solvent was removed by distillation under the reduced pressure, and the residue was purified by column chromatography with an acetone-chloroform system to give methyl 5-amino-2-methoxy-benzoate (244 mg, yield 95%).
Methyl 5-amino-2-methoxy-benzoate (244 mg) and 5-methoxymethylene-2,2-dimethyl-[1,3]dioxan-4,6-dione (228 mg) were dissolved in 2-propanol (3 ml), and the solution was stirred at 100˚C for 15 hr. The solvent was removed by distillation under the reduced pressure, and the residue was washed with diethyl ether to give methyl 5-[(2,2-dimethyl-4,6-dioxo-[1,3]dioxan-5-ylidenemethyl)-amino]-2-methoxy-benzoate (248 mg, yield 55%).
Methyl 5-[(2,2-dimethyl-4,6-dioxo-[1,3]dioxan-5-ylidenemethyl)-amino]-2-methoxy-benzoate (245 mg) and biphenyl (676 mg) were suspended in diphenyl ether (2 ml), and the suspension was stirred at 260˚C for 45 min. The reaction mixture was cooled to room temperature, and the precipitated crystal was collected by filtration and was washed with diethyl ether. The crystal thus obtained as such was used in the next reaction without further purification.
The crystal (45 mg) was suspended in thionyl chloride (1 ml), a minor amount of N,N-dimethylformamide was added to the suspension, and the mixture was stirred at 100˚C for 3.5 hr. The reaction mixture was added to a saturated aqueous sodium hydrogencarbonate solution under ice cooling. The mixture was extracted with chloroform, and the chloroform layer was washed with water and saturated brine and was dried over anhydrous magnesium sulfate. The solvent was removed by distillation under the reduced pressure, and the residue was purified by column chromatography with an acetone-chloroform system to give methyl 4-chloro-6-methoxy-quinoline-7-carboxylate (4 mg, yield 2%) (2 steps).
2-Phenyl-[1,8]naphthyridin-3-ol (11 mg), methyl 4-chloro-6-methoxy-quinoline-7-carboxylate (4 mg), and 4-dimethylaminopyridine (6 mg) were suspended in 1,2-dichlorobenzene (1 ml), and the suspension was stirred at 120˚C for 36 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give the title compound (5 mg, yield 71%).
$^1$H-NMR ($CDCl_3$, 400 MHz): δ 3.98 (s, 3H), 4.02 (s, 3H), 6.57 (d, J = 4.8 Hz, 1H), 7.35 (m, 3H), 7.55 (dd, J = 8.8, 4.4 Hz, 1H), 7.61 (s, 1H), 7.99 (s, 1H), 8.11 (m, 2H), 8.19 (dd, J = 8.0, 1.6 Hz, 1H), 8.44 (s, 1H), 8.56 (d, J = 4.8 Hz, 1H), 9.19 (dd, J = 4.4, 1.6 Hz, 1H)
Mass spectrometric value (ESI-MS, m/z): 460 $(M+Na)^+$

Example 117: 4-(6-Ethyl-2-pyrimidin-2-yl-pyridin-3-yloxy)-quinoline (compound 117)

A 2.0 M lithium diisopropylamide/heptane, tetrahydrofuran, an ethylbenzene solution (26.2 ml), and tri-n-butyltin hydride (14.1 ml) were added in that order to anhydrous tetrahydrofuran (100 ml) under an argon atmosphere at 0˚C, and the mixture was stirred at 0˚C for 15 min. The reaction mixture was cooled to -78˚C. 2-Chloropyrimidine (5.0 g) dissolved in anhydrous tetrahydrofuran (20 ml) was added dropwise thereto. The mixture was slowly brought to room temperature and was stirred overnight. Water was added to the reaction mixture to stop the reaction. The mixture was filtered through Celite, and the filtrate was extracted with ethyl acetate. The ethyl acetate layer was washed with water and was then dried over anhydrous sodium sulfate. The solvent was removed by distillation under the reduced pressure, and the residue was purified by column chromatography with a hexane-ethyl acetate system to give 2-tri-n-butylstannylpyrimidine (9.1 g, yield 56%).
2-Tri-n-butylstannylpyrimidine (5.0 g) was dissolved in anhydrous tetrahydrofuran (130 ml) under an argon atmosphere. A 1.6 M n-butyllithium/hexane solution (8.6 ml) was added dropwise at -78˚C, and the mixture was stirred at -78˚C for 30 min. 5-Ethylfurfural (1.85 g) dissolved in anhydrous tetrahydrofuran (20 ml) was added dropwise thereto, and the mixture was brought to room temperature with stirring. Water was added to the reaction mixture to stop the reaction. The solvent was removed by distillation under the reduced pressure, and water was added to the residue. The mixture was extracted with ethyl acetate, and the ethyl acetate layer was then washed with water and was dried over anhydrous sodium sulfate. The solvent was removed by distillation under the reduced pressure, and the residue was purified by column chromatography with a hexane-ethyl acetate system to give (5-ethylfuran-2-yl)-pyrimidin-2-yl-methanol (888 mg, yield 32%).
(5-Ethylfuran-2-yl)-pyrimidin-2-yl-methanol (880 mg) was dissolved in chloroform (15 ml), manganese dioxide (3.8 g) was added to the solution, and the mixture was stirred at room temperature overnight. The reaction mixture was filtered through Celite, and the solvent was removed from the filtrate by distillation under the reduced pressure. The residue was used in the next reaction without purification.

The residue, methanol (7 ml), and a 28% aqueous ammonia solution (8 ml) were placed in a sealed tube, and the mixture was stirred at 160˚C overnight. The reaction mixture was cooled to room temperature, and the solvent was then removed by distillation under the reduced pressure. The residue was purified by column chromatography with a hexane-acetone system to give 6-ethyl-2-pyrimidin-2-yl-pyridin-3-ol (305 mg, yield 35%).
Dimethylsulfoxide (2 ml) was added to 6-ethyl-2-pyrimidin-2-yl-pyridin-3-ol (40 mg), 4-chloroquinoline (164 mg), cesium carbonate (196 mg), and 4-dimethylaminopyridine (73 mg), and the mixture was stirred at 130˚C for 6 hr. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by thin layer chromatography with a hexane-acetone system to give the title compound (47 mg, yield 72%).
$^1$H-NMR ($CDCl_3$, 400 MHz): δ 1.41 (t, J = 7.6 Hz, 3H), 3.05 (q, J = 7.6 Hz, 2H), 6.53 (d, J = 5.2 Hz, 1H), 7.10 (t, J = 4.9 Hz, 1H), 7.43 (d, J = 8.3 Hz, 1H), 7.53 (dd, J = 7.1, 7.1 Hz, 1H), 7.61 (d, J = 8.3 Hz, 1H), 7.73 (ddd, J = 8.6, 8.6, 1.2 Hz, 1H), 8.05 (d, J = 8.5 Hz, 1H), 8.31 (d, J = 8.3 Hz, 1H), 8.60 (d, J = 5.1 Hz, 1H), 8.64 (d, J = 4.9 Hz, 2H)
Mass spectrometric value (ESI-MS, m/z): 329 $(M+1)^+$

Example 118: 3-(6-Isopropoxy-7-methoxy-quinolin-4-yloxy)-2-phenyl-[1,8]naphthyridine (compound 118)

2-Methoxy-4-nitrophenol (2.46 g) was dissolved in N,N-dimethylformamide (40 ml). Potassium carbonate (3.84 g) and 2-bromopropane (2.0 ml) were added to the solution, and the mixture was stirred at 100˚C for 3 hr. Water was added to the reaction mixture, and the mixture was extracted with ethyl acetate. The ethyl acetate layer was washed with water and saturated brine and was dried over anhydrous sodium sulfate. The solvent was removed by distillation under the reduced pressure, and the residue as such was used in the next reaction without purification.
The residue was dissolved in N,N-dimethylformamide (40 ml). Triethylamine (5 ml) and 20% palladium hydroxide/carbon (1.48 g) were added to the solution under a hydrogen gas atmosphere, and the mixture was stirred at room temperature overnight. The reaction mixture was filtered and was then washed with chloroform. The solvent was removed by distillation under the reduced pressure, and ethyl acetate was added to the residue. The ethyl acetate layer was washed with water and saturated brine and was dried over anhydrous sodium sulfate. The solvent was removed by distillation under the reduced pressure, and the residue as such was used in the next reaction without purification.
The residue and 5-methoxymethylene-2,2-dimethyl-[1,3]dioxan-4,6-dione (2.08 g) were dissolved in 2-propanol (40 ml), and the solution was stirred at 70˚C for 30 min. The reaction mixture was cooled to room temperature. The solvent was then removed by distillation under the reduced pressure, and the residue as such was used in the next reaction without purification.
The residue and biphenyl (6.0 g) was suspended in diphenyl ether (25 ml), and the suspension was stirred at 220˚C overnight. The reaction mixture was cooled to room temperature, and the reaction mixture as such was purified by column chromatography with a methanol-chloroform system to give 6-isopropoxy-7-methoxy-1H-quinolin-4-one (2.10 g, yield 62%) (4 steps).
6-Isopropoxy-7-methoxy-1H-quinolin-4-one (2.10 g) was suspended in diisopropylethylamine (5 ml), phosphorus oxychloride (1.5 ml) was added to the suspension, and the mixture was stirred at 100˚C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an ethyl acetate-hexane system to give 4-chloro-6-isopropoxy-7-methoxy-quinoline (2.05 g, yield 90%).
2-Phenyl-[1,8]naphthyridin-3-ol (44 mg), 4-chloro-6-isopropoxy-7-methoxy-quinoline (50 mg), and 4-dimethylaminopyridine (73 mg) were suspended in 1,2-dichlorobenzene (1.5 ml), and the suspension was stirred at 130˚C for 8 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (86 mg, yield 98%).
$^1$H-NMR ($CDCl_3$, 400 MHz): δ 1.48 (d, J = 6.1 Hz, 6H), 4.06 (s, 3H), 4.77 (m, 1H), 6.51 (d, J = 5.6 Hz, 1H), 7.34 - 7.36 (m, 3H), 7.53 (s, 1H), 7.56 (dd, J = 4.4, 8.3 Hz, 1H), 7.62 (s, 1H), 8.00 (s, 1H), 8.09 - 8.11 (m, 2H), 8.19 (dd, J = 1.7, 8.1 Hz, 1H), 8.43 (d, J = 5.6 Hz, 1H), 9.19 (dd, J = 1.7, 4.1 Hz, 1H)
Mass spectrometric value (ESI-MS, m/z): 460 $(M+Na)^+$

Example 119: 3-(6-Methoxy-7-methyl-quinolin-4-yloxy)-5,6-dimethyl-[2,2']bipyridine (compound 119)

4-Chloro-6-methoxy-7-methyl-quinoline (50 mg), 5,6-dimethyl-[2,2']bipyridinyl-3-ol (48 mg), and 4-dimethylaminopyridine (88 mg) were dissolved in dimethylsulfoxide (1.5 ml), cesium carbonate (235 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (45 mg, yield 51%).
$^1$H-NMR ($CDCl_3$, 400 MHz): δ 2.41 (s, 3H), 2.45 (s, 3H), 2.67 (s, 3H), 3.99 (s, 3H), 6.47 (d, J = 5.1 Hz, 1H), 7.10 (ddd, J = 1.0, 4.9, 7.3 Hz, 1H), 7.38 (s, 1H), 7.52 (s, 1H), 7.62 (m, 1H), 7.89 (d, J = 8.1 Hz, 1H), 8.02 (m, 1H), 8.36 (m, 1H), 8.40 (d, J = 5.6 Hz, 1H)
Mass spectrometric value (ESI-MS, m/z): 394 $(M+Na)^+$

Example 120: 3-(6-Isopropoxy-7-methoxy-quinolin-4-yloxy)-5,6-dimethyl-[2,2']bipyridine (compound 120)

4-Chloro-6-isopropoxy-7-methoxy-quinoline (50 mg), 5,6-dimethyl-[2,2']bipyridinyl-3-ol (40 mg), and 4-dimethylaminopyridine (73 mg) were dissolved in dimethylsulfoxide (1.5 ml), cesium carbonate (194 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (45 mg, yield 55%).
$^1$H-NMR ($CDCl_3$, 400 MHz): δ 1.46 (d, J = 6.1 Hz, 6H), 2.40 (s, 3H), 2.66 (s, 3H), 4.04 (s, 3H), 4.78 (m, 1H), 6.41 (d, J = 5.6 Hz, 1H), 7.10 (dd, J = 4.9, 7.3 Hz, 1H), 7.38 (s, 1H), 7.56 - 7.62 (m, 3H), 7.86 (d, J = 7.8 Hz, 1H), 8.36 (d, J = 5.9 Hz, 1H), 8.39 (m, 1H)
Mass spectrometric value (ESI-MS, m/z): 438 $(M+Na)^+$

Example 121: 8-(5,6-Dimethyl-[2,2']bipyridinyl-3-yloxy)-2-methoxy-[1,5]naphthyridine (compound 121)

5-Amino-2-methoxy-pyridine (1.26 g) and 5-methoxymethylene-2,2-dimethyl-[1,3]dioxan-4,6-dione (2.06 g) were suspended in 2-propanol (40 ml), and the suspension was stirred at 70˚C for 30 min. The reaction mixture was cooled to room temperature, and the precipitated crystal was then collected by filtration and was washed with methanol and then with ether. The crystal thus obtained as such was used in the next reaction without further purification.
The crystal prepared above and biphenyl (5.1 g) were suspended in diphenyl ether (20 ml), and the suspension was stirred at 220˚C for one hr. The reaction mixture was cooled to room temperature, and the reaction mixture was then filtered and was washed with chloroform. The residue as such was used in the next reaction without further purification.
The residue was suspended in diisopropylethylamine (7 ml), phosphorus oxychloride (1.5 ml) was added to the suspension, and the mixture was stirred at 100˚C for one hr. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an acetone-chloroform system to give 8-chloro-2-methoxy-[1,5]naphthyridine (572 mg, yield 29%) (3 steps).
8-Chloro-2-methoxy-[1,5]naphthyridine (50 mg), 5,6-dimethyl-[2,2']bipyridinyl-3-ol (51 mg), and 4-dimethylaminopyridine (94 mg) were dissolved in dimethylsulfoxide (1.5 ml), cesium carbonate (251 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (70 mg, yield 75%).
$^1$H-NMR ($CDCl_3$, 400 MHz): δ 2.34 (s, 3H), 2.66 (s, 3H), 3.92 (s, 3H), 6.92 (d, J = 5.4 Hz, 1H), 7.17 - 7.26 (m, 3H), 7.65 (dd, J = 7.6, 7.6 Hz, 1H), 8.16 (d, J = 8.1 Hz, 1H), 8.36 (s, 1H), 8.53 (d, J = 5.9 Hz, 1H), 8.58 (m, 1H)
Mass spectrometric value (ESI-MS, m/z): 381 $(M+Na)^+$

Example 123: 3-(6-Benzyloxy-7-methoxy-quinolin-4-yloxy)-2-phenyl-[1,8]naphthyridine (compound 123),

2-Methoxy-4-nitrophenol (2.07 g) was dissolved in N,N-dimethylformamide (40 ml). Potassium carbonate (5.9 g) and benzyl chloride (1.8 ml) were added to the solution, and the mixture was stirred at room temperature overnight. Benzyl chloride (1 ml) was further added to the reaction mixture, and the mixture was stirred at room temperature for 4 days. Water was added to the reaction mixture, and the mixture was extracted with ethyl acetate. The ethyl acetate

layer was washed with water and saturated brine and was dried over anhydrous sodium sulfate. The solvent was removed by distillation under the reduced pressure, and the residue was purified by column chromatography with an ethyl acetate-hexane system to give 1-benzyloxy-2-methoxy-4-nitro-benzene (2.94 g, yield 93%).
1-Benzyloxy-2-methoxy-4-nitro-benzene was suspended in ethanol/water (70 ml/7 ml). Ammonium chloride (2.49 g) and zinc (15.3 g) were added to the suspension, and the mixture was stirred at 120˚C for 4 hr. The reaction mixture was filtered and was washed with chloroform. The solvent was removed by distillation under the reduced pressure, and the residue as such was used in the next reaction without purification.

The residue and 5-methoxymethylene-2,2-dimethyl-[1,3]dioxan-4,6-dione (2.42 g) were suspended in 2-propanol (50 ml), and the suspension was stirred at 70˚C for 30 min. The reaction mixture was filtered, and the residue was used in the next reaction without purification.
The residue and biphenyl (6.0 g) were suspended in diphenyl ether (25 ml), and the suspension was stirred at 220˚C for one hr. The reaction mixture as such was purified by column chromatography with a methanol-chloroform system to give 6-benzyloxy-7-methoxy-1H-quinolin-4-one (1.0 g, yield 32%) (3 steps).
6-Benzyloxy-7-methoxy-1H-quinolin-4-one (1.0 g) was suspended in diisopropylethylamine (15 ml), phosphorus oxychloride (1.5 ml) was added to the suspension, and the mixture was stirred at 120˚C for one hr. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an acetone-chloroform system to give 6-benzyloxy-4-chloro-7-methoxyquinoline (971 mg, yield 90%).

2-Phenyl-[1,8]naphthyridin-3-ol (231 mg), 6-benzyloxy-4-chloro-7-methoxyquinoline (306 mg), and 4-dimethylaminopyridine (369 mg) were suspended in 1,2-dichlorobenzene (10 ml), and the suspension was stirred at 130˚C for 7 hr and was further stirred at 110˚C overnight. Further, 6-benzyloxy-4-chloro-7-methoxyquinoline (30 mg) was added to the reaction mixture, and the mixture was stirred at 130˚C for 6 hr and further at 110˚C overnight. The reaction mixture was cooled to room temperature, and the solvent was then removed by distillation under the reduced pressure, and the residue was purified by column chromatography with a methanol-chloroform system to give the title compound (393 mg, yield 78%).
$^1$H-NMR ($CDCl_3$, 400 MHz): δ 4.06 (s, 3H), 5.27 (s, 2H), 6.50 (d, J = 5.1 Hz, 1H), 7.20 (m, 1H), 7.27 - 7.48 (m, 9H), 7.50 (dd, J = 4.1, 8.0 Hz, 1H), 7.76 (d, J = 1.0 Hz, 1H), 8.08 - 8.12 (m, 3H), 8.48 (d, J = 5.1 Hz, 1H), 9.15 (dd, J = 2.0, 4.2 Hz, 1H)
Mass spectrometric value (ESI-MS, m/z): 508 $(M+Na)^+$

Example 124: 7-Methoxy-4-(2-phenyl-[1,8]naphthyridin-3-yloxy)-quinolin-6-ol (compound 124)

3-(6-Benzyloxy-7-methoxy-quinolin-4-yloxy)-2-phe nyl-[1,8]naphthyridine (compound 123) (380 mg) was dissolved in trifluoroacetic acid (3 ml). Methanesulfonic acid (0.3 ml) was added to the solution, and the mixture was stirred at room temperature for 3 hr. Water was added to the reaction mixture. The mixture was neutralized with an aqueous sodium hydrogencarbonate solution, and the organic layer was extracted with chloroform. The chloroform layer was washed with water and was dried over anhydrous sodium sulfate. The solvent was removed by distillation under the reduced pressure, and the residue was purified by thin layer chromatography with an acetone-chloroform system to give the title compound (275 mg, yield 89%).
$^1$H-NMR ($CDCl_3$, 400 MHz): δ 4.08 (s, 3H), 6.48 (d, J = 5.4 Hz, 1H), 7.27 - 7.34 (m, 3H), 7.44 (s, 1H), 7.50 (dd, J = 4.2, 8.0 Hz, 1H), 7.69 (s, 1H), 7.81 (s, 1H), 8.10 - 8.14 (m, 3H), 8.46 (d, J = 5.1 Hz, 1H), 9.14 (dd, J = 2.0, 4.1 Hz, 1H)
Mass spectrometric value (ESI-MS, m/z): 418 $(M+Na)^+$

Example 125: 2-[7-Methoxy-4-(2-phenyl-[1,8]naphthyridin-3-yloxy)-quinolin-6-yloxy]-ethanol compound 125)

7-Methoxy-4-(2-phenyl-[1,8]naphthyridin-3-yloxy)-quinolin-6-ol (compound 124) (70 mg) was dissolved in N, N-dimethylformamide (3 ml). Potassium carbonate (180 mg) and 2-bromoethanol (0.1 ml) were added to the solution, and the mixture was stirred at 70˚C overnight. Water was added to the reaction mixture, and the mixture was extracted with ethyl acetate. The ethyl acetate layer was washed with water and saturated brine and was dried over anhydrous sodium sulfate. The solvent was removed by distillation under the reduced pressure, and the residue was purified by thin layer chromatography with an acetone-chloroform system to give the title compound (34 mg, yield 44%).
$^1$H-NMR ($CDCl_3$, 400 MHz): δ 4.02 (s, 3H), 4.07 (t, J = 4.1 Hz, 2H), 4.24 (t, J = 4.4 Hz, 2H), 6.49 (d, J = 5.1 Hz, 1H), 7.34 - 7.37 (m, 3H), 7.43 (s, 1H), 7.51 - 7.54 (m, 2H), 7.92 (s, 1H), 8.13 - 8.16 (m, 3H), 8.49 (d, J = 5.4 Hz, 1H), 9.16 (dd, J = 2.0, 4.1 Hz, 1H)
Mass spectrometric value (ESI-MS, m/z): 462 $(M+Na)^+$

Example 126: 4-(5,6-Dimethyl-[2,2']bipyridinyl-3-yloxy)-quinoline-7-carboxylic acid amide (compound 126)

Methyl 4-chloro-quinoline-7-carboxylate (100 mg) was dissolved in methanol (5 ml), 28% aqueous ammonia (5 ml) was added to the solution, and the mixture was stirred at 40˚C overnight. The solvent was removed by distillation under the reduced pressure. Water was added to the residue, and the mixture was extracted with chloroform. The organic layer was then washed with saturated brine and was dried over anhydrous sodium sulfate. The solvent was removed by distillation under the reduced pressure, and the residue (58.5 mg) as such was used in the next reaction without purification.
A part of the residue (55 mg), 5,6-dimethyl-[2,2']bipyridinyl-3-ol (53 mg), and 4-dimethylaminopyridine (98 mg) was dissolved in dimethylsulfoxide (1.5 ml), cesium carbonate (260 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (5 mg, yield 3%) (2 steps).
$^1$H-NMR ($CDCl_3$, 400 MHz): δ 2.42 (s, 3H), 2.67 (s, 3H), 6.59 (d, J = 5.4 Hz, 1H), 7.08 (m, 1H), 7.41 (s, 1H), 7.61 (m, 1H), 7.92 (d, J = 8.1 Hz, 1H), 8.13 (d, J = 8.8 Hz, 1H), 8.28 (d, J = 4.1 Hz, 1H), 8.53 (d, J = 8.8 Hz, 1H), 8.64 - 8.68 (m, 2H)
Mass spectrometric value (ESI-MS, m/z): 393 $(M+Na)^+$

Example 128: 2-Methoxy-8-(2-phenyl-[1,8]naphthyridin-3-yloxy)-[1,5]naphthyridine (compound 128)

2-Phenyl-[1,8]naphthyridin-3-ol (57 mg), 8-chloro-2-methoxy-[1,5]naphthyridine (50 mg), and 4-dimethylaminopyridine (94 mg) were suspended in 1,2-dichlorobenzene (1.5 ml), and the suspension was stirred at 130˚C for 8 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (27 mg, yield 28%).
$^1$H-NMR ($CDCl_3$, 400 MHz): δ 3.68 (s, 3H), 7.15 (d, J = 9.0 Hz, 1H), 7.18 (d, J = 5.1 Hz, 1H), 7.40 - 7.42 (m, 3H), 7.46 (dd, J = 4.4, 8.3 Hz, 1H), 7.58 (s, 1H), 8.03 (dd, J = 2.0, 8.3 Hz, 1H), 8.32 (d, J = 9.3 Hz, 1H), 8.38 - 8.41 (m, 2H), 8.69 (d, J = 5.1 Hz, 1H), 9.10 (dd, J = 2.0, 4.1 Hz, 1H)
Mass spectrometric value (ESI-MS, m/z): 403 $(M+Na)^+$

Example 130: 3-(5,6-Dichloro-quinolin-4-yloxy)-5,6-dimethyl-[2,2']bipyridine (compound 130)

4,5,6-Trichloroquinoline (50 mg), 5,6-dimethyl-[2,2']bipyridinyl-3-ol (43 mg), and 4-dimethylaminopyridine (79 mg) were dissolved in dimethylsulfoxide (1.5 ml). Cesium carbonate (210 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (57 mg, yield 66%).
$^1$H-NMR ($CDCl_3$, 400 MHz): δ 2.40 (s, 3H), 2.68 (s, 3H), 6.67 (d, J = 5.4 Hz, 1H), 7.17 (m, 1H), 7.32 (s, 1H), 7.68 (dd, J = 7.3, 7.6 Hz, 1H), 7.81 (d, J = 9.0 Hz, 1H), 8.03 (d, J = 7.8 Hz, 1H), 8.07 (m, 1H), 8.47 (m, 1H), 8.56 (d, J = 5.4 Hz, 1H)
Mass spectrometric value (ESI-MS, m/z): 418 $(M+Na)^+$

Example 131: 3-(6,7-Dichloro-quinolin-4-yloxy)-5,6-dimethyl-[2,2']bipyridine (compound 131)

4,6,7-Trichloroquinoline (62 mg), 5,6-dimethyl-[2,2']bipyridinyl-3-ol (54 mg), and 4-dimethylaminopyridine (98 mg) were dissolved in dimethylsulfoxide (1.5 ml), cesium carbonate (262 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (70 mg, yield 65%).
$^1$H-NMR ($CDCl_3$, 400 MHz): δ 2.41 (s, 3H), 2.67 (s, 3H), 6.48 (d, J = 5.4 Hz, 1H), 7.12 (m, 1H), 7.38 (s, 1H), 7.65 (m, 1H), 7.92 (d, J = 7.8 Hz, 1H), 8.27 - 8.35 (m, 2H), 8.50 (d, J = 2.2 Hz, 1H), 8.55 (dd, J = 1.7, 5.4 Hz, 1H).
Mass spectrometric value (ESI-MS, m/z): 418 $(M+Na)^+$

Example 132: 3-(6-Bromo-7-methoxy-quinolin-4-yloxy)-2-phenyl-[1,8]naphthyridine (compound 132)

1-Bromo-2-methoxy-4-nitrobenzene (2.32 g) was suspended in ethanol/water (60 ml/5 ml). Ammonium chloride (1.94 g) and zinc (10.1 g) were added to the suspension, and the mixture was stirred at 120˚C for 3 hr. The reaction mixture was filtered and was washed with methanol. The solvent was removed by distillation under the reduced pressure, and the residue as such was used in the next reaction without purification.
The residue and 5-methoxymethylene-2,2-dimethyl-[1,3]dioxan-4,6-dione (2.3 g) were dissolved in 2-propanol (60 ml), and the solution was stirred at 70˚C for one hr. The reaction mixture was filtered and was washed with methanol, and the residue as such was used in the next reaction without purification.
The residue and biphenyl (6.86 g) were suspended in diphenyl ether (25 ml), and the suspension was stirred at 220˚C for 3 hr. The reaction mixture was cooled to room temperature, was filtered and was washed with chloroform, and the residue as such was used in the next reaction without purification.
The residue was suspended in diisopropylethylamine (7 ml), phosphorus oxychloride (2 ml) was added to the suspension, and the mixture was stirred at 100˚C for one hr. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an acetone-chloroform system to give 6-bromo-4-chloro-7-methoxy-quinoline (1.25 g, yield 46%) (4 steps).
2-Phenyl-[1,8]naphthyridin-3-ol (163 mg), 6-bromo-4-chloro-7-methoxy-quinoline (200 mg), and 4-dimethylaminopyridine (269 mg) were suspended in 1,2-dichlorobenzene (3 ml), and the suspension was stirred at 130˚C for 5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (145 mg, yield 43%).
$^1$H-NMR ($CDCl_3$, 400 MHz): δ 4.09 (s, 3H), 6.40 (d, J = 5.6 Hz, 1H), 7.33 - 7.37 (m, 3H), 7.57 (dd, J = 4.1, 8.1 Hz, 1H), 7.61 (s, 1H), 8.03 (s, 1H), 8.06 - 8.08 (m, 2H), 8.22 (dd, J = 2.0, 8.3 Hz, 1H), 8.52 (d, J = 5.6 Hz, 1H), 8.62 (s, 1H), 9.21 (dd, J = 2.0, 4.1 Hz, 1H)
Mass spectrometric value (ESI-MS, m/z): 480 $(M+Na)^+$

Example 133: 3-(6-Bromo-7-methoxy-quinolin-4-yloxy)-5,6-dimethyl-[2,2']bipyridine (compound 133)

6-Bromo-4-chloro-7-methoxy-quinoline (200 mg), 5,6-dimethyl-[2,2']bipyridinyl-3-ol (147 mg), and 4-dimethylaminopyridine (269 mg) were dissolved in dimethylsulfoxide (3 ml), cesium carbonate (717 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (308 mg, yield 97%). $^1$H-NMR ($CDCl_3$, 400 MHz): δ 2.41 (s, 3H), 2.66 (s, 3H), 4.07 (s, 3H), 6.39 (d, J = 5.6 Hz, 1H), 7.11 (ddd, J = 1.2, 4.9, 7.6 Hz, 1H), 7.37 (s, 1H), 7.52 (s, 1H), 7.63 (m, 1H), 7.91 (d, J = 8.1 Hz, 1H), 8.35 (s, 1H), 8.48 (d, J = 5.6 Hz, 1H), 8.48 (s, 1H)
Mass spectrometric value (ESI-MS, m/z): 458 $(M+Na)^+$

Example 136: 3-(6-Benzyloxy-quinolin-4-yloxy)-5,6-dimethyl-[2,2']bipyridine (compound 136)

4-Benzyloxy-aniline hydrochloride (1.18 g) and 5-methoxymethylene-2,2-dimethyl-[1,3]dioxan-4,6-dione (1.03 g) were suspended in 2-propanol (15 ml). Triethylamine (0.78 ml) was added to the suspension, and the mixture was stirred at 70˚C for 3 hr. The reaction mixture was cooled to room temperature, was filtered, and was washed with diethyl ether. The residue as such was used in the next reaction without purification.
The residue and biphenyl (4.34 g) were suspended in diphenyl ether (12 ml), and the suspension was stirred at 240˚C overnight. The reaction mixture was cooled to room temperature, and the cooled reaction mixture as such was applied to column chromatography with a methanol-chloroform system. The crude product as such was used in the next reaction without further purification.
The crude product was suspended in diisopropylethylamine (3 ml), phosphorus oxychloride (0.5 ml) was added to the suspension, and the mixture was stirred at 120˚C for 3 hr. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium

sulfate. The solvent was removed by distillation under the reduced pressure, and the residue was purified by column chromatography with an ethyl acetate-hexane system to give 6-benzyloxy-4-chloro-quinoline (147 mg, yield 10%) (3 steps).

6-Benzyloxy-4-chloro-quinoline (147 mg), 5,6-dimethyl-[2,2']bipyridinyl-3-ol (140 mg), and 4-dimethylaminopyridine (200 mg) were dissolved in dimethylsulfoxide (3 ml), cesium carbonate (540 mg) was added to the solution, and the mixture was stirred at 130°C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by column chromatography with an acetone-chloroform system to give the title compound (158 mg, yield 66%).

$^1$H-NMR ($CDCl_3$, 400 MHz): δ 2.39 (s, 3H), 2.66 (s, 3H), 5.21 (s, 2H), 6.43 (d, J = 5.1 Hz, 1H), 7.10 (ddd, J = 1.2, 4.9, 7.6 Hz, 1H), 7.35 - 7.55 (m, 8H), 7.67 (d, J = 2.7 Hz, 1H), 7.76 (m, 1H), 7.96 (d, J = 9.3 Hz, 1H), 8.44 (d, J = 5.1 Hz, 1H), 8.51 (m, 1H)

Mass spectrometric value (ESI-MS, m/z): 456 $(M+Na)^+$

Example 137: 3-(7-Benzyloxy-quinolin-4-yloxy)-5,6-dimethyl-[2,2']bipyridine (compound 137)

3-Benzyloxy-aniline (1.0 g) and 5-methoxymethylene-2,2-dimethyl-[1,3]dioxan-4,6-dione (1.03 g) were suspended in 2-propanol (15 ml) and the suspension was stirred at 70°C for 3 hr. The reaction mixture was cooled to room temperature, was filtered, and was washed with ether. The residue as such was used in the next reaction without purification.

The residue and biphenyl (4.34 g) were suspended in diphenyl ether (12 ml), and the suspension was stirred at 240°C overnight. The reaction mixture was cooled to room temperature, and the cooled reaction mixture as such was applied to column chromatography with a methanol-chloroform system. The crude product as such was used in the next reaction without further purification.

The crude product was suspended in diisopropylethylamine (7 ml), phosphorus oxychloride (1.5 ml) was added to the suspension, and the mixture was stirred at 120°C for 30 min. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an ethyl acetate-hexane system to give 7-benzyloxy-4-chloro-quinoline (222 mg, yield 16%) (3 steps).

7-Benzyloxy-4-chloro-quinoline (222 mg), 5,6-dimethyl-[2,2']bipyridinyl-3-ol (220 mg), and 4-dimethylaminopyridine (302 mg) were dissolved in dimethylsulfoxide (3 ml), cesium carbonate (820 mg) was added to the solution, and the mixture was stirred at 130°C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by column chromatography with an acetone-chloroform system to give the title compound (239 mg, yield 67%).

$^1$H-NMR ($CDCl_3$, 400 MHz): δ 2.38 (s, 3H), 2.66 (s, 3H), 5.21 (s, 2H), 6.32 (d, J = 5.1 Hz, 1H), 7.10 (ddd, J = 1.2, 4.9, 7.6 Hz, 1H), 7.26 - 7.59 (m, 9H), 7.80 (d, J = 7.9 Hz, 1H), 8.23 (d, J = 9.0 Hz, 1H), 8.48 (d, J = 5.1 Hz, 1H), 8.52 (m, 1H)

Mass spectrometric value (ESI-MS, m/z): 456 $(M+Na)^+$

Example 138: 3-(6-Benzyloxy-7-methoxy-quinolin-4-yloxy)-5,6-dimethyl-[2,2'] bipyridine (compound 138)

6-Benzyloxy-4-chloro-7-methoxyquinoline (200 mg), 5,6-dimethyl-[2,2']bipyridinyl-3-ol (195 mg), and 4-dimethylaminopyridine (244 mg) were dissolved in dimethylsulfoxide (3 ml), cesium carbonate (655 mg) was added to the solution, and the mixture was stirred at 130°C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by column chromatography with an acetone-chloroform system to give the title compound (239 mg, yield 77%).

$^1$H-NMR ($CDCl_3$, 400 MHz): δ 2.37 (s, 3H), 2.66 (s, 3H), 4.03 (s, 3H), 5.29 (s, 2H), 6.33 (d, J = 5.4 Hz, 1H), 7.08 (m, 1H), 7.30 - 7.39 (m, 5H), 7.47 (ddd, J = 2.0, 7.8, 7.8 Hz, 1H), 7.50 (m, 2H), 7.60 (s, 1H), 7.68 (d, J = 7.8 Hz, 1H), 8.38 (d, J = 5.4 Hz, 1H), 8.51 (m, 1H)

Mass spectrometric value (ESI-MS, m/z): 486 $(M+Na)^+$

Example 139: 4-(5,6-Dimethyl-[2,2']bipyridinyl-3-yloxy)-quinolin-6-ol (compound 139)

3-(6-Benzyloxy-quinolin-4-yloxy)-5,6-dimethyl-[2,2 ']-bipyridine (compound 136) (150 mg) was dissolved in trifluoroacetic acid (3 ml). Methanesulfonic acid (0.3 ml) was added to the solution, and the mixture was stirred at room temperature for one hr. Water was added to the reaction mixture, the mixture was neutralized with an aqueous sodium hydrogencarbonate solution, and the organic layer was extracted with chloroform. The chloroform layer was washed with water and was dried over anhydrous sodium sulfate. The solvent was removed by distillation under the reduced pressure, and the residue was purified by column chromatography with a methanol-chloroform system to give the title compound (117 mg, yield 99%).
$^1$H-NMR ($CDCl_3$, 400 MHz): δ 2.24 (s, 3H), 2.62 (s, 3H), 6.50 (d, J = 5.2 Hz, 1H), 7.13 (ddd, J = 1.2, 4.9, 7.9 Hz, 1H), 7.15 (s, 1H), 7.32 (m, 1H), 7.36 (dd, J = 2.8, 9.0 Hz, 1H), 7.51 (m, 1H), 7.66 (dd, J = 1.2, 8.0 Hz, 1H), 7.94 (d, J = 9.0 Hz, 1H), 8.45 (d, J = 5.2 Hz, 1H), 8.56 (m, 1H)
Mass spectrometric value (ESI-MS, m/z): 366 $(M+Na)^+$

Example 140: 4-(5,6-Dimethyl-[2,2']bipyridinyl-3-yloxy)-quinolin-7-ol (compound 140)

3-(7-Benzyloxy-quinolin-4-yloxy)-5,6-dimethyl-[2,2'] bipyridine (compound 137) (230 mg) was dissolved in trifluoroacetic acid (3 ml). Methanesulfonic acid (0.3 ml) was added to the solution, and the mixture was stirred at room temperature for one hr. Water was added to the reaction mixture, the mixture was neutralized with an aqueous sodium hydrogencarbonate solution, and the organic layer was extracted with chloroform. The chloroform layer was washed with water and was dried over anhydrous sodium sulfate. The solvent was removed by distillation under the reduced pressure, and the residue was purified by thin layer chromatography with a methanol-chloroform system to give the title compound (182 mg, yield 100%).
$^1$H-NMR ($CDCl_3$, 400 MHz): δ 2.39 (s, 3H), 2.64 (s, 3H), 6.27 (d, J = 5.4 Hz, 1H), 6.87 (dd, J = 2.2, 9.0 Hz, 1H), 7.08 (dd, J = 4.9, 7.6 Hz, 1H), 7.23 (m, 1H), 7.40 (s, 1H), 7.58 (dd, J = 7.6, 7.8 Hz, 1H), 7.81 (d, J = 7.8 Hz, 1H), 7.99 (d, J = 9.0 Hz, 1H), 8.35 (d, J = 5.4 Hz, 1H), 8.50 (m, 1H)
Mass spectrometric value (ESI-MS, m/z): 344 $(M+1)^+$

Example 141: 4-(5,6-Dimethyl-[2,2']bipyridinyl-3-yloxy)-7-methoxy-quinolin-6 -ol (compound 141)

3-(6-Benzyloxy-7-methoxy-quinolin-4-yloxy)-5,6-di methyl-[2,2']bipyridine (compound 138) (230 mg) was dissolved in trifluoroacetic acid (3 ml). Methanesulfonic acid (0.3 ml) was added to the solution, and the mixture was stirred at room temperature for one hr. Water was added to the reaction mixture, the mixture was neutralized with an aqueous sodium hydrogencarbonate solution, and the organic layer was extracted with chloroform. The chloroform layer was washed with water and was dried over anhydrous sodium sulfate. The solvent was removed by distillation under the reduced pressure, and the residue was purified by column chromatography with a methanol-chloroform system to give the title compound (106 mg, yield 55%).
$^1$H-NMR ($CDCl_3$, 400 MHz): δ 2.37 (s, 3H), 2.66 (s, 3H), 4.05 (s, 3H), 6.34 (d, J = 5.4 Hz, 1H), 7.11 (ddd, J = 1.2, 4.9, 7.6 Hz, 1H), 7.32 (s, 1H), 7.38 (s, 1H), 7.56 (ddd, J = 1.7, 7.8, 7.8 Hz, 1H), 7.68 (s, 1H), 7.79 (ddd, J = 1.0, 1.2, 7.8 Hz, 1H), 8.37 (d, J = 5.1 Hz, 1H), 8.57 (m, 1H)
Mass spectrometric value (ESI-MS, m/z): 374 $(M+1)^+$

Example 142: 2-[4-(5,6-Dimethyl-[2,2']bipyridinyl-3-yloxy)-quinolin-6-yloxy]-ethanol (compound 142)

4-(5,6-Dimethyl-[2,2']bipyridinyl-3-yloxy)-quinolin-6-ol (compound 139) (88 mg) was dissolved in N,N-dimethylformamide (3 ml). Potassium carbonate (200 mg) and 2-bromoethanol (0.2 ml) were added to the solution, and the mixture was stirred at 70°C overnight. Water was added to the reaction mixture, and the mixture was extracted with ethyl acetate. The ethyl acetate layer was washed with water and saturated brine and was dried over anhydrous sodium sulfate. The solvent was removed by distillation under the reduced pressure, and the residue was purified by column chromatography with a methanol-chloroform system to give the title compound (41 mg, yield 41%).
$^1$H-NMR ($CDCl_3$, 400 MHz): δ 2.38 (s, 3H), 2.65 (s, 3H), 4.30 (t, J = 4.2 Hz, 2H), 4.23 (t, J = 4.4 Hz, 2H), 6.44 (d, J = 5.2 Hz, 1H), 7.11 (dd, J = 4.9, 7.6 Hz, 1H), 7.34 (s, 1H), 7.37 (m, 1H), 7.57 - 7.61 (m, 2H), 7.81 (d, J = 8.0 Hz, 1H), 7.95 (d, J = 9.0 Hz, 1H), 8.45 (d, J = 5.1 Hz, 1H), 8.49 (m, 1H)
Mass spectrometric value (ESI-MS, m/z): 410 $(M+Na)^+$

Example 143: 2-[4-(5,6-Dimethyl-[2,2']bipyridinyl-3-ylox)-quinolin-7-yloxy]-ethanol (compound 143)

4-(5,6-Dimethyl-[2,2']bipyridinyl-3-yloxy)-quinolin-7-ol (compound 140) (152 mg) was dissolved in N,N-dimethylformamide (3 ml). Potassium carbonate (200 mg) and 2-bromoethanol (0.2 ml) were added to the solution, and the mixture was stirred at 70˚C overnight. Water was added to the reaction mixture, and the mixture was extracted with ethyl acetate. The ethyl acetate layer was washed with water and saturated brine and was dried over anhydrous sodium sulfate. The solvent was removed by distillation under the reduced pressure, and the residue was purified by thin layer chromatography with a methanol-chloroform system to give the title compound (93 mg, yield 54%).
$^1$H-NMR ($CDCl_3$, 400 MHz): δ 2.39 (s, 3H), 2.66 (s, 3H), 4.05 (t, J = 4.2 Hz, 2H), 4.24 (t, J = 4.4 Hz, 2H), 6.33 (d, J = 5.4 Hz, 1H), 7.10 (dd, J = 4.9, 6.6 Hz, 1H), 7.22 (ddd, J = 1.2, 2.4, 9.3 Hz, 1H), 7.36 - 7.37 (m, 2H), 7.57 (ddd, J = 1.7, 7.8, 7.8 Hz, 1H), 7.80 (d, J = 7.8 Hz, 1H), 8.23 (d, J = 9.3 Hz, 1H), 8.48 - 8.49 (m, 2H)
Mass spectrometric value (ESI-MS, m/z): 410 $(M+Na)^+$

Example 144: 2-[4-(5,6-Dimethyl-[2,2']bipyridinyl-3-yloxy)-7-methoxy-quinolin-6-yloxy]-ethanol (compound 144)

4-(5,6-Dimethyl-[2,2']bipyridinyl-3-yloxy)-7-metho xy-quinolin-6-ol (compound 141) (88 mg) was dissolved in N,N-dimethylformamide (3 ml). Potassium carbonate (200 mg) and 2-bromoethanol (0.2 ml) were added to the solution, and the mixture was stirred at 70˚C overnight. Water was added to the reaction mixture, and the mixture was extracted with ethyl acetate. The ethyl acetate layer was washed with water and saturated brine and was dried over anhydrous sodium sulfate. The solvent was removed by distillation under the reduced pressure, and the residue was purified by column chromatography with a methanol-chloroform system to give the title compound (59 mg, yield 67%).
$^1$H-NMR ($CDCl_3$, 400 MHz): δ 2.38 (s, 3H), 2.65 (s, 3H), 4.00 (s, 3H), 4.04 (t, J = 4.4 Hz, 2H), 4.27 (t, J = 4.6 Hz, 2H), 6.37 (d, J = 5.4 Hz, 1H), 7.12 (dd, J = 4.9, 6.6 Hz, 1H), 7.33 (s, 1H), 7.37 (s, 1H), 7.58 (s, 1H), 7.59 (ddd, J = 2.0, 7.8, 7.8 Hz, 1H), 7.79 (d, J = 8.0 Hz, 1H), 8.40 (d, J = 5.4 Hz, 1H), 8.50 (m, 1H)
Mass spectrometric value (ESI-MS, m/z): 440 $(M+Na)^+$

Example 145: 4-(5,6-Dimethyl-[2,2']bipyridinyl-3-yloxy)-6-methgxy-quinoline-7-carboxylic acid amide (compound 145)

Methyl 2-methoxy-5-nitro-benzoate (300 mg), ammonium chloride (228 mg), and zinc (929 mg) were suspended in ethanol (10 ml) and water (0.5 ml), and the suspension was stirred under reflux for 3 hr. The reaction mixture was filtered, and the solvent was removed from the filtrate by distillation under the reduced pressure. A saturated aqueous sodium hydrogencarbonate solution was added to the residue, and the mixture was extracted with chloroform, and the chloroform layer was washed with water and saturated brine and was dried over anhydrous magnesium sulfate. The solvent was removed by distillation under the reduced pressure, and the residue was purified by column chromatography with an acetone-chloroform system to give methyl 5-amino-2-methoxy-benzoate (244 mg, yield 95%).
Methyl 5-amino-2-methoxy-benzoate (244 mg) and 5-methoxymethylene-2,2-dimethyl-[1,3]dioxan-4,6-dione (228 mg) were dissolved in 2-propanol (3 ml), and the mixture was stirred at 100˚C for 15 hr. The solvent was removed by distillation under the reduced pressure, and the residue was washed with diethyl ether to give methyl 5-[(2,2-dimethyl-4,6-dioxo-[1,3] dioxan-5-ylidenemethyl)-amino]-2-methoxy-benzoate (248 mg, yield 55%).
Methyl 5-[(2,2-dimethyl-4,6-dioxo-[1,3]dioxan-5-ylidenemethyl)-amino]-2-methoxy-benzoate (245 mg) and biphenyl (676 mg) were suspended in diphenyl ether (2 ml), and the suspension was stirred at 260˚C for 45 min. The reaction mixture was cooled to room temperature, and the precipitated crystal was collected by filtration and was washed with diethyl ether. The crystal thus obtained as such was used in the next reaction without further purification.
The crystal (45 mg) was suspended in thionyl chloride (1 ml), a minor amount of dimethylformamide was added to the suspension, and the mixture was stirred at 100˚C for 3.5 hr. The reaction mixture was added to a saturated aqueous sodium hydrogencarbonate solution under ice cooling. The mixture was extracted with chloroform, and the chloroform layer was washed with water and saturated brine and was dried over anhydrous magnesium sulfate. The solvent was removed by distillation under the reduced pressure, and the residue was purified by column chromatography with an acetone-chloroform system to give methyl 4-chloro-6-methoxy-quinoline-7-carboxylate (4 mg, yield 2%) (2 steps).
Methyl 4-chloro-6-methoxy-quinoline-7-carboxylate (99 mg) was dissolved in methanol (5 ml). A 28% aqueous ammonia solution (5 ml) was added to the solution, and the mixture was stirred at 40˚C overnight. Methanol was removed by distillation under the reduced pressure, and ethyl acetate was added to the residue for extraction. The ethyl acetate layer was washed with water and saturated brine and was dried over magnesium sulfate. The solvent was removed by distillation under the reduced pressure, and the residue was purified by column chromatography with a methanol-chloroform system to give 4-chloro-6-methoxy-quinoline-carboxylic acid amide (85 mg, yield 91%).
4-Chloro-6-methoxy-quinoline-carboxylic acid amide (41 mg), 5,6-dimethyl-[2,2']bipyridinyl-3-ol (87 mg), 4-dimethylaminopyridine (63 mg), and cesium carbonate (169 mg) were suspended in dimethylsulfoxide (2 ml), and the suspension was stirred at 120˚C for 22 hr. The reaction mixture was cooled to room temperature, and water was added thereto. The mixture was extracted with ethyl acetate, and the ethyl acetate layer was then washed with water and saturated brine and was dried over anhydrous magnesium sulfate. The solvent was removed by distillation under the reduced pressure, and the residue was purified by thin layer chromatography with a chloroform-methanol system to

give the title compound (13 mg, yield 19%).
$^1$H-NMR ($CDCl_3$, 400 MHz): δ 2.40 (s, 3H), 2.66 (s, 3H), 4.10 (s, 3H), 6.03 (brs, 1H), 6.46 (d, J = 5.2 Hz, 1H), 7.11 (m, 1H), 7.38 (s, 1H), 7.60 (m, 1H), 7.71 (m, 2H), 7.82 (d, J = 7.6 Hz, 1H), 8.44 (dd, J = 4.8, 0.8 Hz, 1H), 8.53 (d, J = 5.2 Hz, 1H), 8.89 (s, 1H)
Mass spectrometric value (ESI-MS, m/z): 423 $(M+Na)^+$

Example 146: 7-Chloro-4-(5,6-dimethyl-[2,2']bipyridinyl-3-yloxy)-quinoline-6-carboxylic acid amide (compound 146)

4-Amino-2-chloro-benzoic acid (2.50 g) and lithium hydroxide monohydrate (611 mg) were suspended in tetrahydrofuran (20 ml), and the suspension was stirred at room temperature for 20 min. Thereafter, dimethylsulfuric acid (1.38 ml) was added to the reaction mixture, and the mixture was stirred under reflux for 2 hr. The solvent was removed by distillation under the reduced pressure. Water was added to the residue, and the mixture was neutralized with a saturated aqueous sodium hydrogencarbonate solution and was extracted with diethyl ether. The diethyl ether layer was then washed with water and saturated brine and was dried over anhydrous magnesium sulfate. The solvent was removed by distillation under the reduced pressure to give methyl 4-amino-2-chloro-benzoate (1.68 g, yield 62%).
Methyl 4-amino-2-chloro-benzoate (1.68 g) and 5-methoxymethylene-2,2-dimethyl-[1,3]dioxan-4,6-dione (1.53 g) were dissolved in 2-propanol (25 ml), and the mixture was stirred at 100˚C for 15.5 hr. The solvent was removed by distillation under the reduced pressure, and the residue was washed with diethyl ether to give methyl 2-chloro-4-[(2,2-dimethyl-4,6-dioxo-[1,3]dioxan-5-ylidenemeth yl)-amino]-benzoate (1.92 g, yield 63%).

Methyl 2-chloro-4-[(2,2-dimethyl-4,6-dioxo-[1,3]dioxan-5-ylidenemeth yl)-amino]-benzoate (1.81 g) and biphenyl (4.93 g) were suspended in diphenyl ether (12 ml), and the suspension was stirred at 260˚C for 45 min. The reaction mixture was cooled to room temperature, and the precipitated crystal was collected by filtration and was washed with diethyl ether. The crystal thus obtained as such was used in the next reaction without further purification.
The crystal (1.21 g) was suspended in thionyl chloride (12 ml), a minor amount of N,N-dimethylformamide was added to the suspension, and the mixture was stirred at 100˚C for 4 hr. The reaction mixture was added to a saturated aqueous sodium hydrogencarbonate solution under ice cooling. The mixture was extracted with chloroform, and the chloroform layer was washed with water and saturated brine and was dried over anhydrous magnesium sulfate. The solvent was removed by distillation under the reduced pressure, and the residue was purified by column chromatography with an acetone-hexane system to give methyl 4,7-dichloro-quinoline-6-carboxylate (350 mg, yield 26%) (2 steps).
Methyl 4,7-dichloro-quinoline-6-carboxylate (197 mg) was dissolved in methanol (7 ml). A 28% aqueous ammonia solution (7 ml) was added to the solution, and the mixture was stirred at 40˚C overnight. Methanol was removed by distillation under the reduced pressure, and ethyl acetate was added to the residue for extraction. The ethyl acetate layer was washed with water and saturated brine and was dried over magnesium sulfate. The solvent was removed by distillation under the reduced pressure, and the residue was purified by column chromatography with a methanol-chloroform system to give 4,7-dichloro-quinoline-6-carboxylic acid amide (145 mg, yield 78%).

4,7-Dichloro-quinoline-6-carboxylic acid amide (50 mg), 5,6-dimethyl-[2,2']bipyridinyl-3-ol (83 mg), 4-dimethylaminopyridine (76 mg), and cesium carbonate (203 mg) were suspended in dimethylsulfoxide (2 ml), and the suspension was stirred at 120˚C for 22 hr. The reaction mixture was cooled to room temperature, and water was added thereto. The mixture was extracted with ethyl acetate, and the ethyl acetate layer was then washed with water and saturated brine and was dried over anhydrous magnesium sulfate. The solvent was removed by distillation under the reduced pressure, and the residue was purified by thin layer chromatography with a chloroform-methanol system to give the title compound (16 mg, yield 19%).
$^1$H-NMR ($CDCl_3$, 400 MHz): δ 2.39 (s, 3H), 2.65 (s, 3H), 6.36 (brs, 1H), 6.44 (d, J = 5.2 Hz, 1H), 6.62 (brs, 1H), 7.08 (ddd, J = 7.2, 4.8, 1.2 Hz, 1H), 7.35 (s, 1H), 7.60 (m, 1H), 7.86 (m, 1H), 8.09 (s, 1H), 8.36 (m, 1H), 8.59 (d, J = 5.2 Hz, 1H), 8.76 (s, 1 H)
Mass spectrometric value (ESI-MS, m/z): 427 $(M+Na)^+$

Example 147: 6-Methoxy-4-(2-phenyl-[1,8]naphthyridin-3-yloxy)-quinoline-7-carboxylic acid amide (compound 147)

4-Chloro-6-methoxy-quinoline-7-carboxylic acid amide (21 mg), 2-phenyl-[1,8]naphthyridin-3-ol (49 mg), and 4-dimethylaminopyridine (33 mg) were suspended in 1,2-dichlorobenzene (1.5 ml), and the suspension was stirred at 140˚C for 3.5 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give the title compound (26 mg, yield 69%). $^1$H-NMR ($CD_3OD$, 400 MHz): δ 4.11 (s, 3H), 6.66 (d, J = 5.6 Hz, 1H), 7.32 (m, 3H), 7.70 (dd, J = 8.4, 4.4 Hz, 1H), 7.89 (s, 1H), 8.01 (m; 2H), 8.39 - 8.52 (m, 4H), 9.11 (d, J = 2.4 Hz, 1H)
Mass spectrometric value (ESI-MS, m/z): 445 $(M+Na)^+$

Example 148: Methyl 7-chloro-4-(2-phenyl-[1,8]naphthyridin-3-yloxy)-quinoline-6-carboxylate (compound 148)

Methyl 4,7-dichloro-quinoline-6-carboxylate (46 mg), 2-phenyl-[1,8]naphthyridin-3-ol (100 mg), and 4-dimethylaminopyridine (66 mg) were suspended in 1,2-dichlorobenzene (1.5 ml), and the suspension was stirred at 140˚C for 3.5 hr. The reaction mixture was cooled to room temperature, and water was added thereto. The mixture was extracted with chloroform, and the chloroform layer was washed with water and saturated brine and was dried over anhydrous magnesium sulfate. The solvent was removed by distillation under the reduced pressure, and the residue was purified by thin layer chromatography with a chloroform-methanol system to give the title compound (33 mg, yield 42%).
$^1$H-NMR ($CDCl_3$, 400 MHz): δ 4.03 (s, 3H), 6.44 (d, J = 5.2 Hz, 1H), 7.33 (m, 3H), 7.56 (dd, J = 8.0, 4.4 Hz, 1H), 8.07 (m, 3H), 8.15 (s, 1H), 8.21 (dd, J = 8.4, 2.0 Hz, 1H), 8.63 (d, J = 5.2 Hz, 1H), 8.89 (s, 1H), 9.19 (dd, J = 4.4, 2.0 Hz, 1H)
Mass spectrometric value (ESI-MS, m/z): 464 $(M+Na)^+$

Example 154: 2-[4-(5,6-Dimethyl-[2,2']bipyridinyl-3-yloxy)-7-methoxy-quinolin-6-ylamino]-ethanol (compound 154)

Palladium acetate (30 mg) and 2,2'-bis(diphenylphosphino)-1,1'-binaphthyl (80 mg) were dissolved in toluene (4 ml), and the solution was stirred at room temperature for 5 min. 3-(6-Bromo-7-methoxy-quinolin-4-yloxy)-5,6-dimethyl-[2,2']bip yridine (compound 133) (100 mg) and 2-aminoethanol (0.2 ml) were added thereto, and the mixture was stirred at room temperature for additional 5 min. Cesium carbonate (250 mg) was added to the reaction mixture, and the mixture was stirred at 80˚C for two days. Water was added to the reaction mixture, the mixture was extracted with chloroform, and the chloroform layer was washed with water and was dried over anhydrous sodium sulfate. The solvent was removed by distillation under the reduced pressure, and the residue was purified by thin layer chromatography with a methanol-chloroform system to give the title compound (9 mg, yield 9%).
$^1$H-NMR ($CDCl_3$, 400 MHz): δ 2.27 (s, 3H), 2.56 (s, 3H), 3.35 (t, J = 5.4 Hz, 2H), 3.82 (t, J = 5.4 Hz, 2H), 3.88 (s, 3H), 6.32 (d, J = 5.1 Hz, 1H), 7.00 (m, 1H), 7.07 (ddd, J = 1.0, 4.9, 7.6 Hz, 1H), 7.19 - 7.20 (m, 2H), 7.52 (ddd, J = 1.7, 7.8, 8.1 Hz, 1H), 7.73 (d, J = 7.8 Hz, 1H), 8.21 (d, J = 5.1 Hz, 1H), 8.51 (m, 1H)
Mass spectrometric value (ESI-MS, m/z): 439 $(M+Na)^+$

Example 155: 3-(7-Methoxy-6-pyridin-3-yl-quinotin-4-yloxy)-5,6-dimethyl-[2,2']bipyridine (compound 155)

N,N-Dimethylformamide (3 ml) and a 2 M aqueous potassium carbonate solution (1.5 ml) were added to 3-(6-bromo-7-methoxy-quinolin-4-yloxy)-5,6-dimethyl-[2,2']bip yridine (compound 133) (70 mg), tetrakistriphenylphosphine palladium (29 mg), and 3-pyridylboric acid (57 mg) under an argon atmosphere, and the mixture was stirred at 70˚C for 2 hr. The reaction mixture was cooled to room temperature, water was added to the cooled mixture, and the mixture was extracted with ethyl acetate. The ethyl acetate layer was then washed with water and was dried over anhydrous sodium sulfate. The solvent was removed by distillation under the reduced pressure, and the residue was purified by column chromatography with a chloroform-methanol system to give the title compound (56 mg, yield 80%).
$^1$H-NMR ($CDCl_3$, 400 MHz): δ 2.40 (s, 3H), 2.66 (s, 3H), 3.98 (s, 3H), 6.35 (d, J = 5.4 Hz, 1H), 7.10 (dd, J = 5.8, 7.6 Hz, 1H), 7.36 - 7.39 (m, 2H), 7.48 (s, 1H), 7.59 (ddd, J = 1.7, 7.6, 7.8 Hz, 1H), 7.82 (d, J = 8.0 Hz, 1H), 7.95 (d, J = 7.8 Hz, 1H), 8.25 (s, 1H), 8.47 (m, 1H), 8.51 (d, J = 5.4 Hz, 1H), 8.61 (m, 1H), 8.85 (m, 1H)
Mass spectrometric value (ESI-MS, m/z): 457 $(M+Na)^+$

Example 156: 3-(6-Chloro-quinolin-4-yloxy)-5,6-dimethyl-[2,2']bipyridine (compound 156)

4,6-Dichloro-quinoline (50 mg), 5,6-dimethyl-[2,2']bipyridinyl-3-ol (51 mg), and 4-dimethylaminopyridine (93 mg) were dissolved in dimethylsulfoxide (1.5 ml), cesium carbonate (247 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (90 mg, yield 100%).
$^1$H-NMR ($CDCl_3$, 400 MHz): δ 2.41 (s, 3H), 2.67 (s, 3H), 6.51 (d, J = 5.4 Hz, 1H), 7.11 (ddd, J = 1.0, 4.9, 7.6 Hz, 1H), 7.38 (s, 1H), 7.63 (ddd, J = 1.7, 7.8, 7.8 Hz, 1H), 7.73 (dd, J = 2.2, 8.8 Hz, 1H), 7.91 (d, J = 8.1 Hz, 1H), 8.15 (d, J = 8.8 Hz, 1H), 8.34 (m, 1H), 8.37 (d, J = 2.2 Hz, 1H), 8.56 (d, J = 5.4 Hz, 1H)
Mass spectrometric value (ESI-MS, m/z): 384 $(M+Na)^+$

Example 157: 5,6-Dimethyl-3-(6-methyl-quinolin-4-yloxy)-[2,2']bipyridine (compound 157)

4-Chloro-6-methyl-quinoline (50 mg), 5,6-dimethyl-[2,2']bipyridinyl-3-ol (56 mg), and 4-dimethylaminopyridine

(103 mg) were dissolved in dimethylsulfoxide (1.5 ml), cesium carbonate (275 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (82 mg, yield 85%).
$^1$H-NMR ($CDCl_3$, 400 MHz): δ 2.40 (s, 3H), 2.58 (s, 3H), 2.67 (s, 3H), 6.46 (d, J = 5.4 Hz, 1H), 7.10 (ddd, J = 1.2, 4.9, 7.6 Hz, 1H), 7.36 (s, 1H), 7.60 (m, 1H), 7.64 (s, 1H), 7.89 (d, J = 7.8 Hz, 1H), 8.09 (d, J = 8.5 Hz, 1H), 8.15 (m, 1H), 8.40 (m, 1H), 8.51 (d, J = 5.4 Hz, 1H)
Mass spectrometric value (ESI-MS, m/z): 364 $(M+Na)^+$

Example 158: 4-(5,6-Dimethyl-2-pyrimidin-2-yl-pyridin-3-yloxy)-6-fluoro-7-methoxy-quinoline (compound 158)

4-Chloro-6-fluoro-7-methoxy-quinoline (13 mg), 5,6-dimethyl-2-pyrimidin-2-yl-pyridin-3-ol (12 mg), and 4-dimethylaminopyridine (22 mg) were dissolved in dimethylsulfoxide (1 ml), cesium carbonate (58 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (15 mg, yield 66%).
$^1$H-NMR ($CDCl_3$, 400 MHz): δ 2.44 (s, 3H), 2.70 (s, 3H), 4.07 (s, 3H), 6.48 (d, J = 5.4 Hz, 1H), 7.12 (t, J = 4.9 Hz, 1H), 7.43 (s, 1H), 7.65 (m, 1H), 7.95 (d, J = 11.5 Hz, 1H), 8.48 (d, J = 5.6 Hz, 1H), 8.62 (d, J = 4.9 Hz, 2H)
Mass spectrometric value (ESI-MS, m/z): 399 $(M+Na)^+$

Example 159: 4-(6-Ethyl-2-pyrimidin-2-yl-pyridin-3-yloxy)-6-fluoro-7-methoxy-quinoline (compound 159)

4-Chloro-6-fluoro-7-methoxy-quinoline (50 mg), 6-ethyl-2-pyrimidin-2-yl-pyridin-3-ol(48 mg), and 4-dimethylaminopyridine (84 mg) were dissolved in dimethylsulfoxide (1.5 ml), cesium carbonate (231 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (54 mg, yield 60%).
$^1$H-NMR ($CDCl_3$, 400 MHz): δ 1.42 (t, J = 7.8 Hz, 3H), 3.06 (q, J = 7.6 Hz, 2H), 4.06 (s, 3H), 6.51 (d, J = 5.4 Hz, 1H), 7.14 (t, J = 4.9 Hz, 1H), 7.45 (d, J = 8.1 Hz, 1H), 7.61 (m, 2H), 7.92 (d, J = 10.7 Hz, 1H), 8.50 (d, J = 5.4 Hz, 1H), 8.65 (d, J = 4.9 Hz, 2H)
Mass spectrometric value (ESI-MS, m/z): 399 $(M+Na)^+$

Example 160: 3-(6-Fluoro-7-methoxy-quinolin-4-yloxy)-2-methyl-[1,8]naphthyridine (compound 160)

2-Methyl-[1,8]naphthyridin-3-ol (42 mg), 4-chloro-6-fluoro-7-methoxy-quinoline (50 mg), and 4-dimethylaminopyridine (87 mg) were suspended in 1,2-dichlorobenzene (1.5 ml), and the suspension was stirred at 130˚C for 5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (63 mg, yield 78%).
$^1$H-NMR ($CDCl_3$, 400 MHz): δ 2.73 (s, 3H), 4.11 (s, 3H), 6.49 (d, J = 5.4 Hz, 1H), 7.52 (dd, J = 4.4, 8.3 Hz, 1H), 7.74 (d, J = 7.8 Hz, 1H), 7.84 (s, 1H), 8.01 (d, J = 11.2 Hz, 1H), 8.15 (dd, J = 2.0, 8.1 Hz, 1H), 8.63 (d, J = 5.4 Hz, 1H), 9.14 (dd, J = 2.0, 4.4 Hz, 1H)
Mass spectrometric value (ESI-MS, m/z): 358 $(M+Na)^+$

Example 161: 2-Ethyl-3-(6-fluoro-7-methoxy-qulnolin-4-yloxy)-[1,8]naphthyridine (compound 161)

2-Aminopyridine-3-carbaldehyde (100 mg) and 1-bromo-butan-2-one (124 mg) were suspended in a 5 N aqueous sodium hydroxide solution (0.6 ml), and the suspension was allowed to stand in a hermetically stoppered state for three days. The reaction mixture was neutralized with 10% hydrochloric acid, and the resultant precipitate was filtered, and the residue was washed with water and chloroform. The powder was dried in vacuo to give 2-ethyl-[1,8]naphthyridin-3-ol (106 mg, yield 74%).
2-Ethyl-[1,8]naphthyridin-3-ol (41 mg), 4-chloro-6-fluoro-7-methoxy-quinoline (50 mg), and 4-dimethylaminopyridine (87 mg) were suspended in 1,2-dichlorobenzene (1.5 ml), and the suspension was stirred at 130˚C for 6.5 hr. The reaction mixture was cooled to room temperature, and an aqueous sodium hydrogencarbonate solution was added to the reaction mixture. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (38 mg, yield 46%).
$^1$H-NMR ($CDCl_3$, 400 MHz): δ 1.45 (t, J = 7.6 Hz, 3H), 3.04 (q, J = 7.3 Hz, 2H), 4.10 (s, 3H), 6.50 (d, J = 5.4 Hz, 1H), 7.51 (dd, J = 4.4, 7.6 Hz, 1H), 7.69 (m, 1H), 7.82 (s, 1H), 7.99 (dd, J = 1.2, 11.2 Hz, 1H), 8.14 (dd, J = 1.5, 8.3 Hz, 1H), 8.63 (d, J = 5.4 Hz, 1H), 9.14 (ddd, J = 1.0, 2.0, 4.4 Hz, 1H)
Mass spectrometric value (ESI-MS, m/z): 372 $(M+Na)^+$

Example 162: 6-Ethyl-3-(6-fluoro-7-methoxy-quinolin-4-yloxy)-[2,2']bipyridine (compound 162)

4-Chloro-6-fluoro-7-methoxy-quinoline (50 mg), 6-ethyl-[2,2']bipyridinyl-3-ol (52 mg), and 4-dimethylaminopyridine (87 mg) were dissolved in dimethylsulfoxide (1.5 ml), cesium carbonate (231 mg) was added to the solution, and the mixture was stirred at 130˚C overnight. The reaction mixture was cooled to room temperature, and water was added thereto. The organic layer was extracted with chloroform, and the chloroform layer was then washed with water and saturated brine and was dried over anhydrous sodium sulfate. The solvent was removed by distillation under the reduced pressure, and the residue was purified by thin layer chromatography with a methanol-chloroform system to give the title compound (84 mg, yield 93%).
$^1$H-NMR ($CDCl_3$, 400 MHz): δ 1.42 (t, J = 7.8 Hz, 3H), 3.01 (q, J = 7.6 Hz, 2H), 4.06 (s, 3H), 6.43 (d, J = 5.6 Hz, 1H), 7.12 (ddd, J = 1.2, 4.9, 7.6 Hz, 1H), 7.36 (d, J = 8.3 Hz, 1H), 7.56 (d, J = 8.3 Hz, 1H), 7.63 - 7.67 (m, 2H), 7.92 (d, J = 7.8 Hz, 1H), 7.96 (d, J = 11.5 Hz, 1H), 8.33 (d, J = 3.9 Hz, 1H), 8.47 (d, J = 5.6 Hz, 1H)
Mass spectrometric value (ESI-MS, m/z): 398 $(M+Na)^+$

Example 163: 5,6-Dimethyl-3-(6-thiophen-3-yl-quinolin-4-yloxy)-[2,2']bipyridine (compound 163)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (compound 2) (30 mg), tetrakistriphenylphosphine palladium (9 mg), and thiophene-3-boric acid (28 mg) under an argon atmosphere, and the mixture was stirred at 70˚C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (30 mg, yield 100%).
$^1$H-NMR ($CDCl_3$, 400 MHz): δ 2.39 (s, 3H), 2.67 (s, 3H), 6.45 (d, J = 5.1 Hz, 1H), 7.09 (ddd, J = 7.6, 4.9, 1.2 Hz, 1H), 7.39 (s, 1H), 7.44 (dd, J = 5.1, 2.9 Hz, 1H), 7.54 - 7.60 (m, 2H), 7.64 (dd, J = 2.9, 1.5 Hz, 1H), 7.83 (ddd, J = 7.1, 1.0, 1.0 Hz, 1H), 8.00 (dd, J = 8.8, 2.0 Hz, 1H), 8.06 (d, J = 8.8 Hz, 1H), 8.47 - 8.51 (m, 1H), 8.52 - 8.56 (m, 2H)
Mass spectrometric value (ESI-MS, m/z): 410 $(M+1)^+$

Example 164: 3-(6-Benzo[b]thiophen-3-yl-quinolin-4-yloxy)-5,6-dimethyl-[2,2']bipyridine (compound 164)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (compound 2) (30 mg), tetrakistriphenylphosphine palladium (9 mg), and thianaphthene-3-boric acid (39 mg) under an argon atmosphere, and the mixture was stirred at 70˚C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (34 mg, yield 100%).
$^1$H-NMR ($CDCl_3$, 400 MHz): δ 2.40 (s, 3H), 2.66 (s, 3H), 6.50 (d, J = 5.1 Hz, 1H), 7.11 (ddd, J = 7.6, 4.9, 1.2 Hz, 1H), 7.36 - 7.44 (m, 3H), 7.54 - 7.62 (m, 2H), 7.83 (ddd, J = 8.0, 1.0, 1.0 Hz, 1H), 7.92 - 8.01 (m, 3H), 8.16 (d, J = 8.6 Hz, 1H), 8.48 - 8.53 (m, 1H), 8.55 (d, J = 2.0 Hz, 1H), 8.61 (d, J = 5.1 Hz, 1H) Mass spectrometric value (ESI-MS, m/z): 482 $(M+Na)^+$

Example 165: 5,6-Dimethyl-3-[6-(5-methyl-thiophen-2-yl)-quinolin-4-yloxy]-[2,2']bipyridine (compound 165)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (compound 2) (30 mg), tetrakistriphenylphosphine palladium (9 mg), and 5-methylthiophene-2-boric acid (31 mg) under an argon atmosphere, and the mixture was stirred at 70°C overnight. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by thin layer chromatography with a hexane-acetone system to give the title compound (4 mg, yield 13%).
$^1$H-NMR ($CDCl_3$, 400 MHz): δ 2.40 (s, 3H), 2.55 (s, 3H), 2.67 (s, 3H), 6.43 (d, J = 4.9 Hz, 1H), 6.79 (d, J = 2.4 Hz, 1H), 7.06 - 7.14 (m, 1H), 7.29 (d, J = 3.2 Hz, 1H), 7.39 (s, 1H), 7.54 - 7.62 (m, 1H), 7.84 (d, J = 7.8 Hz, 1H), 7.95 (dd, J = 8.8, 2.0 Hz, 1H), 8.01 (d, J = 8.8 Hz, 1H), 8.45 (s, 1H), 8.47 - 8.55 (m, 2H)
Mass spectrometric value (ESI-MS, m/z): 424 $(M+1)^+$

Example 166: 3-(6-Benzofuran-2-yl-quinolin-4-yloxy)-5,6-dimethyl-[2,2']bipyridine (compound 166)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (compound 2) (30 mg), tetrakistriphenylphosphine palladium (9 mg), and benzo[b]furan-2-boric acid (36 mg) under an argon atmosphere, and the mixture was stirred at 70°C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (4 mg, yield 13%).
$^1$H-NMR ($CDCl_3$, 400 MHz): δ 2.40 (s, 3H), 2.68 (s, 3H), 6.46 (d, J = 5.1 Hz, 1H), 7.08 (ddd, J = 8.8, 4.9, 1.2 Hz, 1H), 7.20 (d, J = 0.8 Hz, 1H), 7.23 - 7.34 (m, 2H), 7.41 (s, 1H), 7.51 - 7.65 (m, 3H), 7.89 (ddd, J = 7.8, 7.8, 1.0 Hz, 1H), 8.08 (d, J = 8.8 Hz, 1H), 8.17 (dd, J = 8.8, 2.0 Hz, 1H), 8.47 - 8.51 (m, 1H), 8.55 (d, J = 5.1 Hz, 1H), 8.87 (d, J = 1.8 Hz, 1H)
Mass spectrometric value (ESI-MS, m/z): 466 $(M+Na)^+$

Example 167: 2-[4-(5,6-Dimethyl-[2,2']bipyridinyl-3-yloxy)-quinolin-6-yl]-pyrrole-1-carboxylate tert-butyl ester (compound 167)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (compound 2) (30 mg), tetrakistriphenylphosphine palladium (9 mg), and 1-(tert-butoxycarbonyl)pyrrole-2-boric acid (47 mg) under an argon atmosphere, and the mixture was stirred at 70°C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (31 mg, yield 85%).
$^1$H-NMR ($CDCl_3$, 400 MHz): δ 1.37 (s, 9H), 2.38 (s, 3H), 2.66 (s, 3H), 6.27 (dd, J = 3.2, 3.2 Hz, 1H), 6.31 - 6.35 (m, 1H), 6.45 (d, J = 5.1 Hz, 1H), 7.10 (dd, J = 7.1, 5.1 Hz, 1H), 7.34 (s, 1H), 7.38 - 7.43 (m, 1H), 7.57 (ddd, J = 7.8, 7.8, 1.2 Hz, 1H), 7.73 (dd, J = 8.8, 1.7 Hz, 1H), 7.82 (d, J = 7.8 Hz, 1H), 8.00 (d, J = 8.8 Hz, 1H), 8.31 (d, J = 1.7 Hz, 1H), 8.52 (d, J = 4.4 Hz, 1H), 8.56 (d, J = 5.2 Hz, 1H)
Mass spectrometric value (ESI-MS, m/z): 515 $(M+Na)^+$

Example 168: 5,6-Dimethyl-3-[6-(1H-pyrrol-2-yl)-quinolin-4-yloxy]-[2,2']bipyridine (compound 168)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (compound 2) (30 mg), tetrakistriphenylphosphine palladium (9 mg), and 1-(tert-butoxycarbonyl)pyrrole-2-boric acid (47 mg) under an argon atmosphere, and the mixture was stirred at 70°C for 5 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium

carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (4 mg, yield 15%).
$^1$H-NMR ($CDCl_3$, 400 MHz): δ 2.36 (s, 3H), 2.63 (s, 3H), 6.32 - 6.37 (m, 1H), 6.47 (d, J = 5.1 Hz, 1H), 6.70 - 6.74 (m, 1H), 6.94 - 6.98 (m, 1H), 7.08 - 7.15 (m, 1H), 7.29 (s, 1H), 7.60 (ddd, J = 7.8, 7.8, 1.2 Hz, 1H), 7.79 (d, J = 8.1 Hz, 1H), 7.95 (dd, J = 9.0, 2.2 Hz, 1H), 8.02 (d, J = 8.6 Hz, 1H), 8.36 (d, J = 1.7 Hz, 1H), 8.47 - 8.52 (m, 2H)
Mass spectrometric value (ESI-MS, m/z): 393 $(M+1)^+$

Example 169: 5,6-Dimethyl-3-[6-(1H-pyrazol-4-yl)-quinolin-4-yloxy]-[2,2']bipyridine (compound 169)

N,N-Dimethylformamide (1 ml) and a 2 M aqueous potassium carbonate solution (0.5 ml) were added to 3-(6-bromo-quinolin-4-yloxy)-5,6-dimethyl-[2,2']bipyridine (compound 2) (30 mg), tetrakistriphenylphosphine palladium (9 mg), and 1H-pyrazole-4-boric acid pinacol ester (43 mg) under an argon atmosphere, and the mixture was stirred at 70˚C overnight. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (10 mg, yield 33%). $^1$H-NMR ($CDCl_3$, 400 MHz): δ 2.40 (s, 3H), 2.67 (s, 3H), 6.45 (d, J = 5.1 Hz, 1H), 7.07 - 7.13 (m, 1H), 7.39 (s, 1H), 7.59 (ddd, J = 7.6, 7.6, 1.7 Hz, 1H), 7.84 (d, J = 7.8 Hz, 1H), 7.91 (dd, J = 8.8, 2.2 Hz, 1H), 8.03 (s, 2H), 8.06 (d, J = 8.8 Hz, 1H), 8.45 (d, J = 2.0 Hz, 1H), 8.48 - 8.51 (m, 1H), 8.53 (d, J = 5.1 Hz, 1H)
Mass spectrometric value (ESI-MS, m/z): 394 $(M+1)^+$

Example 170: 3-(6,7-Dimethyl-quinolin-4-yloxy)-5,6-dimethyl-[2,2']bipyridine (compound 170)

3,4-Dimethylaniline (3.0 g) was dissolved in 2-propanol (70 ml), and the mixture was stirred at 70˚C for 10 min. 5-(Methoxymethylene)-2,2-dimethyl-[1,3]dioxan-4,6-dione (5.1 g) was added thereto, and the mixture was stirred at 70˚C for one hr. The reaction mixture was cooled to room temperature, and the solvent was removed by distillation under the reduced pressure. The residue was suspended in diethyl ether, was collected by filtration, and was washed with diethyl ether. The crude crystal thus obtained as such was used in the next reaction without purification.
Diphenyl ether (50 ml) was added to the crude crystal obtained above and biphenyl (22.2 g), and the mixture was stirred at 240˚C for 3 hr. The reaction mixture was cooled to room temperature. Diethyl ether and hexane were added to the cooled reaction mixture, and the resultant precipitate was collected by filtration and was washed with diethyl ether. The crude crystal thus obtained as such was used in the next reaction without purification.
Thionyl chloride (10 ml) and a minor amount of N,N-dimethylformamide were added to the crude crystal, and the mixture was stirred under reflux for 3 hr. The reaction mixture was added to a saturated aqueous sodium bicarbonate solution under ice cooling, and the mixture was extracted with ethyl acetate. The ethyl acetate layer was washed with water and was dried over anhydrous sodium sulfate. The solvent was removed by distillation under the reduced pressure, and the residue was purified by column chromatography with a hexane-ethyl acetate system to give 4-chloro-6,7-dimethylquinoline (1.07 g, yield 31%).
Dimethylsulfoxide (2 ml) was added to 5,6-dimethyl-[2,2']bipyridinyl-3-ol (40 mg), 4-chloro-6,7-dimethylquinoline (115 mg), cesium carbonate(196 mg), and 4-dimethylaminopyridine (73 mg), and the mixture was stirred at 130˚C overnight. The reaction mixture was cooled to room temperature, water was added to the cooled mixture, and the mixture was extracted with ethyl acetate. The ethyl acetate layer was washed with water and was dried over anhydrous sodium sulfate. The solvent was removed by distillation under the reduced pressure, and the residue was purified by column chromatography with a hexane-acetone system to give the title compound (27 mg, yield 38%).
$^1$H-NMR ($CDCl_3$, 400 MHz): δ 2.36 (s, 3H), 2.48 (s, 3H), 2.65 (s, 3H), 2.79 (s, 3H), 6.48 (d, J = 4.9 Hz, 1H), 7.14 (ddd, J = 7.6, 4.9, 1.2 Hz, 1H), 7.26 (s, 1H), 7.52 (d, J = 8.6 Hz, 1H), 7.61 (ddd, J = 7.6, 7.6, 1.9 Hz, 1H), 7.76 - 7.83 (m, 2H), 8.48 (d, J = 5.1 Hz, 1H), 8.53 - 8.58 (m, 1H)
Mass spectrometric value (ESI-MS, m/z): 378 $(M+Na)^+$

Example 171: 5,6-Dimethyl-3-(7-thioahen-3-yl-quinolin-4-yloxy)-[2,2']bipyridine (compound 171)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (compound 14) (30 mg), tetrakistriphenylphosphine palladium (9 mg), and thiophene-3-boric acid (28 mg) under an argon atmosphere, and the mixture was stirred at 70˚C for 7 hr. The reaction mixture was cooled to room temperature, water was added to the cooled mixture, and the mixture was extracted

with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (18 mg, yield 59%).
$^1$H-NMR ($CDCl_3$, 400 MHz): δ 2.40 (s, 3H), 2.67 (s, 3H), 6.41 (d, J = 5.4 Hz, 1H), 7.10 (ddd, J = 7.6, 4.6, 1.0 Hz, 1H), 7.39 (s, 1H), 7.46 (dd, J = 4.9, 3.0 Hz, 1H), 7.55 - 7.61 (m, 2H), 7.68 (dd, J = 2.9, 1.2 Hz, 1H), 7.81 - 7.86 (m, 2H), 8.26 (d, J = 1.7 Hz, 1H), 8.36 (d, J = 8.6 Hz, 1H), 8.48 - 8.52 (m, 1H), 8.57 (d, J = 5.4 Hz, 1H)
Mass spectrometric value (ESI-MS, m/z): 410 $(M+1)^+$

Example 172: 3-(7-Benzo[b]thiophen-3-yl-quinolin-4-yloxy)-5,6-dimethyl-[2,2']bipyridine (compound 172)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (compound 14) (30 mg), tetrakistriphenylphosphine palladium (9 mg), and thianaphthene-3-boric acid (39 mg) under an argon atmosphere, and the mixture was stirred at 70˚C for 7 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (33 mg, yield 96%).
$^1$H-NMR ($CDCl_3$, 400 MHz): δ 2.41 (s, 3H), 2.68 (s, 3H), 6.47 (d, J = 5.1 Hz, 1H), 7.12 (ddd, J = 7.6, 4.9, 1.2 Hz, 1H), 7.39 - 7.46 (m, 3H), 7.58 - 7.64 (m, 2H), 7.82 (dd, J = 8.6, 1.7 Hz, 1H), 7.87 (ddd, J = 7.8, 1.0, 1.0 Hz, 1H), 7.92 - 7.97 (m, 1H), 8.02 - 8.09 (m, 1H), 8.30 (d, J = 1.7 Hz, 1H), 8.45 (d, J = 8.8 Hz, 1H), 8.51 - 8.55 (m, 1H), 8.62 (d, J = 5.1 Hz, 1H)
Mass spectrometric value (ESI-MS, m/z): 460 $(M+1)^+$

Example 173: 3-(7-Benzofuran-2-yl-quinolin-4-yloxy)-5,6-dimethyl-[2,2']bipyridine (compound 173)

N,N-Dimethylformamide (1 ml) and a 2 M aqueous potassium carbonate solution (0.5 ml) were added to 3-(7-bromo-quinolin-4-yloxy)-5,6-dimethyl-[2,2']bipyridine (compound 14) (30 mg), tetrakistriphenylphosphine palladium (9 mg), and benzo[b]furan-2-boric acid (36 mg) under an argon atmosphere, and the mixture was stirred at 70˚C for 7 hr. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by column chromatography with a hexane-acetone system to give the title compound (26 mg, yield 78%).
$^1$H-NMR ($CDCl_3$, 400 MHz): δ 2.40 (s, 3H), 2.67 (s, 3H), 6.43 (d, J = 5.1 Hz, 1H), 7.09 (ddd, J = 7.6, 4.9, 1.2 Hz, 1H), 7.23 - 7.29 (m, 2H), 7.33 (ddd, J = 8.3, 8.3, 1.4 Hz, 1H), 7.40 (s, 1H), 7.55 - 7.61 (m, 2H), 7.64 (d, J = 7.6 Hz, 1H), 7.84 (ddd, J = 7.8, 1.2, 1.2 Hz, 1H), 8.03 (dd, J = 8.8, 1.7 Hz, 1H), 8.41 (d, J = 8.8 Hz, 1H), 8.46 - 8.51 (m, 1H), 8.54 (d, J = 1.4 Hz, 1H), 8.59 (d, J = 5.1 Hz, 1H)
Mass spectrometric value (ESI-MS, m/z): 444 $(M+1)^+$

Example 174: 5,6-Dimethyl-3-(6-pyridin-2-yl-quinolin-4-yloxy)-[2,2']bipyridine (compound 174)

N,N-Dimethylformamide (1 ml) was added to 3-(6-bromo-quinolin-4-yloxy)-5,6-dimethyl-[2,2']bipyridine (compound 2) (25 mg), tetrakistriphenylphosphine palladium (7 mg), 2-tributylstannylpyridine (45 mg), and copper(II) oxide (1 mg) under an argon atmosphere, and the mixture was stirred at 100˚C overnight. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by thin layer chromatography with a hexane-acetone system to give the title compound (12 mg, yield 46%).
$^1$H-NMR ($CDCl_3$, 400 MHz): δ 2.40 (s, 3H), 2.67 (s, 3H), 6.46 (d, J = 5.1 Hz, 1H), 7.08 (ddd, J = 7.3, 4.6, 1.0 Hz, 1H), 7.27 - 7.32 (m, 1H), 7.39 (s, 1H), 7.56 (ddd, J = 7.8, 7.8, 1.9 Hz, 1H), 7.81 (ddd, J = 7.8, 7.8, 1.7 Hz, 1H), 7.85 (d, J = 7.8 Hz, 1H), 7.94 (d, J = 8.1 Hz, 1H), 8.14 (d, J = 8.8 Hz, 1H), 8.44 (dd, J = 8.8, 2.0 Hz, 1H), 8.47 - 8.51 (m, 1H), 8.58 (d, J = 5.1 Hz, 1H), 8.74 - 8.79 (m, 1H), 8.96 (d, J = 2.0 Hz, 1H)

Mass spectrometric value (ESI-MS, m/z): 405 $(M+1)^+$

Example 175: 5,6-Dimethyl-3-(6-pyrimidin-2-yl-quinolin-4-yloxy)-[2,2']bipyridine (compound 175)

N,N-Dimethylformamide (1 ml) was added to 3-(6-bromo-quinolin-4-yloxy)-5,6-dimethyl-[2,2']bipyridine (compound 2) (25 mg), tetrakistriphenylphosphine palladium (7 mg), 2-tributylstannylpyrimidine (45 mg), and copper(II) oxide (1 mg) under an argon atmosphere, and the mixture was stirred at 100˚C overnight. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by thin layer chromatography with a hexane-acetone system to give the title compound (6 mg, yield 22%).
$^1$H-NMR ($CDCl_3$, 400 MHz): $\delta$ 2.40 (s, 3H), 2.68 (s, 3H), 6.46 (d, J = 5.2 Hz, 1H), 7.07 (ddd, J = 7.6, 4.9, 1.0 Hz, 1H), 7.24 - 7.28 (m, 1H), 7.39 (s, 1H), 7.53 (ddd, J = 7.8, 7.8, 2.0 Hz, 1H), 7.88 (d, J = 8.1 Hz, 1H), 8.14 (d, J = 8.8 Hz, 1H), 8.48 - 8.53 (m, 1H), 8.60 (d, J = 5.1 Hz, 1H), 8.81 (dd, J = 8.8, 2.0 Hz, 1H), 8.88 (d, J = 4.9 Hz, 2H), 9.48 (d, J = 1.7 Hz, 1H)
Mass spectrometric value (ESI-MS, m/z): 428 $(M+Na)^+$

Example 176: 5,6-Dimethyl-3-(7-pyridin-2-yl-quinolin-4-yloxy)-[2,2']bipyridine (compound 176)

N,N-Dimethylformamide (1 ml) was added to 3-(7-bromo-quinolin-4-yloxy)-5,6-dimethyl-[2,2']bipyridine (compound 14) (25 mg), tetrakistriphenylphosphine palladium (7 mg), 2-tributylstannylpyridine (45 mg), and copper(II) oxide (1 mg) under an argon atmosphere, and the mixture was stirred at 100˚C overnight. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by thin layer chromatography with a hexane-acetone system to give the title compound (18 mg, yield 72%).
$^1$H-NMR ($CDCl_3$, 400 MHz): $\delta$ 2.40 (s, 3H), 2.67 (s, 3H), 6.45 (d, J = 5.1 Hz, 1H), 7.08 (ddd, J = 7.6, 4.9, 1.2 Hz, 1H), 7.28 - 7.34 (m, 1H), 7.42 (s, 1H), 7.56 (ddd, J = 7.8, 7.8, 1.7 Hz, 1H), 7.80 - 7.87 (m, 2H), 7.94 (d, J = 8.0 Hz, 1H), 8.35 (dd, J = 8.8, 1.7 Hz, 1H), 8.46 (d, J = 8.8 Hz, 1H), 8.47 - 8.51 (m, 1H), 8.58 (d, J = 1.5 Hz, 1H), 8.60 (d, J = 5.1 Hz, 1H), 8.75 - 8.80 (m, 1H)
Mass spectrometric value (ESI-MS, m/z): 427 $(M+Na)^+$

Example 177: 5,6-Dimethyl-3-(7-pyrimidin-2-yl-quinolin-4-yloxy)-[2,2']blpyridine (compound 177)

N,N-Dimethylformamide (1 ml) was added to 3-(7-bromo-quinolin-4-yloxy)-5,6-dimethyl-[2,2']bipyridine (compound 14) (25 mg), tetrakistriphenylphosphine palladium (7 mg), 2-tributylstannylpyrimidine (45 mg), and copper(II) oxide (1 mg) under an argon atmosphere, and the mixture was stirred at 100˚C overnight. The reaction mixture was cooled to room temperature, water was added to the cooled mixture, and the mixture was extracted with ethyl acetate. The ethyl acetate layer was then extracted with 1 N hydrochloric acid, and the aqueous layer was washed with ethyl acetate. The aqueous layer was rendered alkaline by the addition of potassium carbonate and was extracted with ethyl acetate. The ethyl acetate layer was washed with water and was then dried over anhydrous sodium sulfate. The solvent was removed by distillation under the reduced pressure, and the residue was purified by thin layer chromatography with a hexane-acetone system to give the title compound (6 mg, yield 24%).
$^1$H-NMR ($CDCl_3$, 400 MHz): $\delta$ 2.41 (s, 3H), 2.67 (s, 3H), 6.46 (d, J = 5.2 Hz, 1H), 7.08 (ddd, J = 7.6, 4.9, 1.2 Hz, 1H), 7.25 - 7.29 (m, 1H), 7.42 (s, 1H), 7.56 (ddd, J = 7.6, 7.6, 1.7 Hz, 1H), 7.84 (d, J = 8.0 Hz, 1H), 8.44 - 8.51 (m, 2H), 8.61 (d, J = 2.7 Hz, 1H), 8.63 (d, J = 2.4 Hz, 1H), 8.90 (d, J = 4.9 Hz, 2H), 9.16 (d, J = 1.4 Hz, 1H)
Mass spectrometric value (ESI-MS, m/z): 428 $(M+Na)^+$

Examples 178 to 225

Compounds of Examples 178 to 225 were produced according to the above schemes. The names of compounds produced in these Examples and the measured values of the molecular weight of the actually produced compounds were as shown in Table A which will be described later.
For typical examples thereof, specific production processes will be described.

Example 178: 2-[6-Methoxy-4-(2-phenyl-[1,8]naphthyridin-3-yloxy)-quinolin-7-yloxy]-ethanol (compound 178)

2-Phenyl-[1,8]naphthyridin-3-ol (6.61 g), 7-benzyloxy-4-chloro-6-methoxy-quinoline (16.6 g), and 4-dimethylaminopyridine (10.9 g) were suspended in 1,2-dichlorobenzene (80 ml), and the suspension was stirred at 140˚C for 20 hr. The solvent was removed by distillation under the reduced pressure. Water was added to the residue, and the mixture was extracted with chloroform. The chloroform layer was dried over anhydrous magnesium sulfate. The solvent was removed by distillation under the reduced pressure, and the residue was purified by column chromatography with a methanol-chloroform system to give 3-(7-benzyloxy-6-methoxy-quinolin-4-yloxy)-2-phenyl-[1,8]naphthyridine (6.00 g, yield 42%).
3-(7-Benzyloxy-6-methoxy-quinolin-4-yloxy)-2-phe nyl-[1,8]naphthyridine (4.36 g) was dissolved in trifluoroacetic acid (40 ml), methanesulfonic acid (3 ml) was added to the solution, and the mixture was stirred at 80˚C for one hr. Water was added to the reaction mixture, the mixture was neutralized with an aqueous sodium hydrogencarbonate solution, and the organic layer was extracted with chloroform. The chloroform layer was washed with water and was dried over anhydrous sodium sulfate. The solvent was removed by distillation under the reduced pressure to give 6-methoxy-4-(2-phenyl-[1,8]naphthyridin-3-yloxy)-quinolin-7-ol (3.55 g, yield 100%).
6-Methoxy-4-(2-phenyl-[1,8]naphthyridin-3-yloxy)-quinolin-7-ol (3.55 g) was dissolved in N,N-dimethylformamide (30 ml). Potassium carbonate (6.20 g) and 2-bromoethanol (5.61 g) were added to the solution, and the mixture was stirred at 60˚C overnight. The solvent was removed by distillation under the reduced pressure. Water was added to the residue, and the mixture was extracted with chloroform. The chloroform layer was dried over anhydrous magnesium sulfate. The solvent was removed by distillation under the reduced pressure, and the residue was purified by column chromatography with a methanol-chloroform system to give the title compound (3.20 g, yield 81%).
$^1$H-NMR ($CDCl_3$, 400 MHz): $\delta$ 2.33 (m, 1H), 3.98 (s, 3H), 4.10 (m, 2H), 4.30(t, J = 4.8 Hz, 2H), 6.52 (d, J = 5.2 Hz, 1H), 7.37 (m, 3H), 7.44 (s, 1H), 7.48 (s, 1H), 7.92 (dd, J = 8.0, 4.0 Hz, 1H), 7.93 (s, 1H), 8.16 (m, 3H), 8.49 (d, J = 5.2 Hz, 1H), 9.16 (dd, J = 4.0, 2.0 Hz, 1H)
Mass spectrometric value (ESI-MS, m/z): 462 $(M+Na)^+$

Example 181: 2-[4-(6-Ethyl-[2,2']bipyridinyl-3-yloxy)-quinolin-6-yloxy]-ethanol (compound 181)

2-Bromopyridine (500 mg) was dissolved in tetrahydrofuran (15 ml). A 1.58 M n-butyllithium/hexan solution (2 ml) was added to the solution, and the mixture was stirred at -78˚C for 30 min. 5-Ethylfurfural (350 mg) was added to the reaction mixture, and the mixture was stirred at room temperature for additional one hr. Water was added to the reaction mixture, and the organic layer was extracted with ethyl acetate. The ethyl acetate layer was then washed with water and saturated brine and was dried over anhydrous sodium sulfate. The solvent was removed by distillation under the reduced pressure, and the residue was purified by thin layer chromatography with an ethyl acetate-hexane system to give (5-ethyl-furan-2-yl)-pyridin-2-yl-methanol (214 mg, yield 37%).
(5-Ethyl-furan-2-yl)-pyridin-2-yl-methanol (214 mg) was dissolved in chloroform (5 ml), manganese dioxide (1.37 g) was added to the solution, and the mixture was stirred at room temperature overnight. The reaction mixture was filtered, and the solvent was removed by distillation under the reduced pressure. The residue was purified by thin layer chromatography with an ethyl acetate-hexane system to give (5-ethyl-furan-2-yl)-pyridin-2-yl-methanone (161 mg, yield 76%).
(5-Ethyl-furan-2-yl)-pyridin-2-yl-methanone (160 mg) was dissolved in methanol (3 ml), 28% aqueous ammonia (3 ml) was added to the solution, and the mixture was stirred in a sealed tube at 160˚C overnight. The solvent was removed by distillation under the reduced pressure, and the residue was purified by thin layer chromatography with an acetone-hexane system to give 6-ethyl-[2,2']bipyridin-3-ol (104 mg, yield 65%).
6-Benzyloxy-4-chloro-quinoline (3.47 g) and 6-ethyl-[2,2']bipyridin-3-ol (5.03 g) were dissolved in dimethylsulfoxide (25 ml), cesium carbonate (16.0 g) was added to the solution, and the mixture was stirred at 140˚C overnight. The reaction mixture was cooled to room temperature. Water was added to the reaction mixture, and the organic layer was extracted with ethyl acetate. The ethyl acetate layer was then washed with water and saturated brine and was dried over anhydrous sodium sulfate. The solvent was removed by distillation under the reduced pressure, and the residue as such was used in the next reaction without purification.
The crude product prepared above was dissolved in trifluoroacetic acid (10 ml). Methanesulfonic acid (2 ml) was added to the solution, and the mixture was stirred at room temperature for 2 hr and then at 80˚C for 7 hr. The reaction mixture was concentrated under the reduced pressure. Water was added to the reaction mixture, and the mixture was neutralized with an aqueous sodium hydrogencarbonate solution. The purified insolubles were collected by filtration and were washed with ethyl acetate to give 4-(6-ethyl-[2,2']bipyridinyl-3-yloxy)-quinolin-6-ol (2.57 g, yield 58%).
4-(6-Ethyl-[2,2']bipyridinyl-3-yloxy)-quinolin-6-ol (2.57 g) was dissolved in N,N-dimethylformamide (25 ml). Potassium carbonate (9.08 g) and 2-bromoethanol (7.0 ml) were added to the solution, and the mixture was stirred at 80˚C overnight. Water was added to the reaction mixture, and the mixture was extracted with ethyl acetate. The ethyl acetate layer was washed with water and saturated brine and was dried over anhydrous sodium sulfate. The solvent was removed

by distillation under the reduced pressure, and the residue was washed with diethyl ether to give the title compound (1.29 g, yield 44%).
$^1$H-NMR ($CDCl_3$, 400 MHz): δ 1.41(t, J = 7.8 Hz, 3H), 2.17 (m, 1H), 3.01(q, J = 7.8 Hz, 2H), 4.04 (m, 2H), 4.24 (t, J = 4.9 Hz, 2H), 6.45 (d, J = 5.1 Hz, 1H), 7.13 (ddd, J = 1.2, 4.9, 7.6 Hz, 1H), 7.34 (d, J = 8.3 Hz, 1H), 7.40 (dd, J = 2.9, 9.3 Hz, 1H), 7.54 (d, J = 8.3 Hz, 1H), 7.60 (d, J = 2.9 Hz, 1H), 7.62 (ddd, J = 1.9, 7.8, 7.8 Hz, 1H), 7.84 (d, J = 8.0 Hz, 1H), 7.96 (d, J = 9.2 Hz, 1H), 8.45 - 8.48 (m, 2H)
Mass spectrometric value (ESI-MS, m/z): 410 $(M+Na)^+$

Example 188: 4-(6-Methyl-2-pyrimidin-2-yl-pyridin-3-yloxy)-6,7-dimethoxy-quinoline (compound 188)

2-Methylfuran (4.92 g) was dissolved in anhydrous ether (80 ml). A 1.58 M n-butyllithium/hexan solution (31.6 ml) was added to the solution at 0˚C, and the mixture was stirred for 3 hr. The reaction mixture was cooled to -78˚C. An ether solution (80 ml) of 2-cyanopyrimidine (5.78 g) was added to the reaction mixture, and the mixture was stirred at -78˚C for additional 4 hr. The reaction mixture was rendered weakly acidic by the addition of 10% hydrochloric acid, and the mixture was then stirred at room temperature for 30 min. The reaction mixture was neutralized with potassium carbonate. The organic layer was extracted with ethyl acetate, and the ethyl acetate layer was then washed with water and saturated brine and was dried over anhydrous sodium sulfate. The solvent was removed by distillation under the reduced pressure, and the residue was purified by column chromatography with an acetone-hexane system to give (5-methylfuran-2-yl)-(2-pyrimidyl)-methanone (3.2 g, yield 34%).
(5-Methylfuran-2-yl)-(2-pyrimidyl)-methanone (3.2 g), methanol (10 ml), and a 28% aqueous ammonia solution (20 ml) were placed in a sealed tube, and the mixture was stirred at 160˚C overnight. The reaction mixture was cooled to room temperature, and the solvent was then removed by distillation under the reduced pressure. The residue was purified by column chromatography with a hexane-acetone system to give 6-methyl-2-pyrimidin-2-yl-pyridin-3-ol (1.32 g, yield 50%).
4-Chloro-6,7-dimethoxy-quinoline (790 mg) and 6-methyl-2-pyrimidin-2-yl-pyridin-3-ol (660 mg) were dissolved in dimethylsulfoxide (4 ml), cesium carbonate (3.45 g) was added to the solution, and the mixture was stirred at 130˚C overnight. The reaction mixture was cooled to room temperature, and water was added thereto. The organic layer was extracted with chloroform, and the organic layer was then washed with saturated brine and was dried over anhydrous sodium sulfate. The solvent was removed by distillation under the reduced pressure, and the residue was purified by column chromatography with a methanol-chloroform system and was further purified by thin layer chromatography with a methanol-chloroform system to give the title compound (438 mg, yield 33%).
$^1$H-NMR ($CDCl_3$, 400 MHz): δ 2.76 (s, 3H), 4.02 (s, 3H), 4.05 (s, 3H), 6.46 (d, J = 5.4 Hz, 1H), 7.14 (t, J = 4.9 Hz, 1H), 7.42 (d, J = 8.3 Hz, 1H), 7.48 (s, 1H), 7.54 (s, 1H), 7.58 (d, J = 8.3 Hz, 1H), 8.41 (d, J = 5.4 Hz, 1H), 8.66 (d, J = 4.9 Hz, 2H)
Mass spectrometric value (ESI-MS, m/z): 397 $(M+Na)^+$

Example 192: 2-[4-(6-Ethyl-2-pyrimidin-2-yl-pyridin-3-yloxy)-quinolin-6-yloxy]-ethanol (compound 192),

To anhydrous tetrahydrofuran (100 ml) were added a 2.0 M lithium diisopropylamide/heptane, tetrahydrofuran, an ethylbenzene solution (26.2 ml), and tri-n-butyltin hydride (14.1 ml) in that order under an argon atmosphere at 0˚C. The mixture was stirred at 0˚C for 15 min. The reaction mixture was cooled to -78˚C. 2-Chloropyrimidine (5.0 g) dissolved in anhydrous tetrahydrofuran (20 ml) was added dropwise to the cooled reaction mixture. The mixture was slowy brought to room temperature and was stirred overnight. Water was added to the reaction mixture to stop the reaction. The mixture was filtered through Celite, and the filtrate was extracted with ethyl acetate. The ethyl acetate layer was then washed with water and was dried over anhydrous sodium sulfate. The solvent was removed by distillation under the reduced pressure, and the residue was purified by column chromatography with a hexane-ethyl acetate system to give 2-tri-n-butylstannylpyrimidine (9.1 g, yield 56%).
2-Tri-n-butylstannylpyrimidine (5.0 g) was dissolved in tetrahydrofuran (130 ml) under an argon atmosphere. A 1.6 M n-butyllithium/hexane solution (8.6 ml) was added dropwise to the solution at -78˚C, and the mixture was stirred at -78˚C for 30 min. 4,5-Dimethylfurfural (1.85 g) dissolved in tetrahydrofuran (20 ml) was added dropwise to the solution, and the mixture was brourgh to room temperature with stirring. Water was added to the reaction mixture to stop the reaction. The solvent was removed by distillation under the reduced pressure, and water was added to the residue. The mixture was extracted with ethyl acetate, and the ethyl acetate layer was then washed with water and was dried over anhydrous sodium sulfate. The solvent was removed by distillation under the reduced pressure, and the residue was purified by column chromatography with a hexane-ethyl acetate system to give (5-ethylfuran-2-yl)-pyrimidin-2-yl-methanol (888 mg, yield 32%).
(5-Ethylfuran-2-yl)-pyrimidin-2-yl-methanol (880 mg) was dissolved in chloroform (15 ml), manganese dioxide (3.8 g) was added to the solution, and the mixture was stirred at room temperature overnight. The reaction mixture was filtered through Celite, and the solvent was removed from the filtrate by distillation under the reduced pressure. The residue was used in the next reaction without purification.

The residue, methanol (7 ml), and a 28% aqueous ammonia solution (8 ml) were placed in a sealed tube, and the mixture was stirred at 160˚C overnight. The reaction mixture was cooled to room temperature, and the solvent was then removed by distillation under the reduced pressure. The residue was purified by column chromatography with a hexane-acetone system to give 6-ethyl-2-pyrimidin-2-yl-pyridin-3-ol (305 mg, yield 35%).

6-Benzyloxy-4-chloro-quinoline (1.00 g), 6-ethyl-2-pyrimidin-2-yl-pyridin-3-ol(746 mg), and 4-dimethylaminopyridine (1.36 g) were dissolved in dimethylsulfoxide (5 ml), cesium carbonate (3.62 g) was added to the solution, and the mixture was stirred at 130˚C overnight. The reaction mixture was cooled to room temperature, and water was added thereto. The organic layer was extracted with chloroform, and the organic layer was then washed with saturated brine and was dried over anhydrous sodium sulfate. The solvent was removed by distillation under the reduced pressure, and the residue was purified by column chromatography with a methanol-chloroform system and further purified by thin layer chromatography with a methanol-chloroform system to give 6-benzyloxy-4-(6-ethyl-2-pyrimidin-2-yl-pyridin-3-yloxy)-quinoline (1.25 g, yield 77%).

6-Benzyloxy-4-(6-ethyl-2-pyrimidin-2-yl-pyridin-3-yloxy)-quinoline (1.25 g) was dissolved in trifluoroacetic acid (7 ml). Methanesulfonic acid (0.7 ml) was added to the solution, and the mixture was stirred at 70˚C for 2 hr. The reaction mixture was concentrated under the reduced pressure. Water was added to the reaction mixture, and the mixture was neutralized with an aqueous sodium hydrogencarbonate solution. The organic layer was extracted with chloroform, and the organic layer was then washed with saturated brine and was dried over anhydrous sodium sulfate. The solvent was removed by distillation under the reduced pressure, and the residue was purified by thin layer chromatography with a methanol-chloroform system to give 4-(6-ethyl-2-pyrimidin-2-yl-pyridin-3-yloxy)-quinolin-6-ol (990 mg, yield 100%).

4-(6-Ethyl-2-pyrimidin-2-yl-pyridin-3-yloxy)-quinoli n-6-ol (990 mg) was dissolved in N,N-dimethylformamide (6 ml). Potassium carbonate (1.35 g) and 2-bromoethanol (0.7 ml) were added to the solution, and the mixture was stirred at 70˚C overnight. The reaction mixture was concentrated under the reduced pressure. Water was added to the residue, and the orgnaic layer was extracted with chloroform. The organic layer was then washed with saturated brine and was dried over anhydrous sodium sulfate. The solvent was removed by distillation under the reduced pressure, and the residue was purified by thin layer chromatography with an acetone-chloroform system to give the title compound (789 mg, yield 71%).

$^1$H-NMP, ($CDCl_3$, 400 MHz): δ 1.41 (t, J = 7.8 Hz, 3H), 2.19 (m, 1H), 3.05 (q, J = 7.8 Hz, 2H), 4.04 (m, 2H), 4.22 (t, J = 4.2 Hz, 2H), 6.56 (d, J = 5.4 Hz, 1H), 7.14 (t, J = 4.9 Hz, 1H), 7.41 - 7.45 (m, 2H), 7.58 - 7.61 (m, 2H), 8.05 (d, J = 9.0 Hz, 1H), 8.48 (d, J = 5.4 Hz, 1H), 8.66 (d, J = 4.9 Hz, 2H)

Mass spectrometric value (ESI-MS, m/z): 411 $(M+Na)^+$

Example 200: 2-[4-(5,6-Dimethyl-2-pyrimidin-2-yl-pyridin-3-yloxy)-quinolin-6-yloxy]-ethanol (compound 200)

2,3-Dimethylfuran (1.5 g) was dissolved in diethyl ether (20 ml) under an argon atmosphere. A 1.6 M n-butyllithium/hexane solution (10.9 ml) was added dropwise to the solution at 0˚C, and the mixture was stirred under reflux for 2.5 hr. Thereafter, the reaction mixture was cooled to -78˚C. 2-Cyanopyrimidine (1.8 g) dissolved in diethyl ether (8 ml) was added dropwise to the cooled reaction mixture, and the mixture was stirred at room temperature overnight. The reaction mixture was poured into ice to stop the reaction, was acidified with 1 M hydrochloric acid, and was extracted with ethyl acetate. The ethyl acetate layer was then washed with water and was dried over anhydrous sodium sulfate. The solvent was removed by distillation under the reduced pressure, and the residue was purified by column chromatography with a chloroform-acetone system to give (4,5-dimethylfuran-2-yl)-(2-pyrimidyl)-methanone (226 mg, yield 7%).

(4,5-Dimethylfuran-2-yl)-(2-pyrimidyl)-methanone (220 mg), methanol (2 ml), and a 28% aqueous ammonia solution (2 ml) were placed in a sealed tube, and the mixture was stirred at 160˚C overnight. The reaction mixture was cooled to room temperature, and the solvent was then removed by distillation under the reduced pressure. The residue was purified by column chromatography with a hexane-acetone system to give 5,6-dimethyl-2-pyrimidin-2-yl-pyridin-3-ol (129 mg, yield 59%).

6-Benzyloxy-4-chloro-quinoline (1.30 g) and 5,6-dimethyl-2-pyrimidin-2-yl-pyridin-3-ol (1.46 g) were dissolved in dimethylsulfoxide (15 ml), cesium carbonate (4.71 g) was added thereto, and the mixture was stirred at 130˚C overnight. The reaction mixture was cooled to room temperature. Water was then added to the reaction mixture, and the organic layer was extracted with ethyl acetate. The ethyl acetate layer was then washed with water and saturated brine and was dried over anhydrous sodium sulfate. The solvent was removed by distillation under the reduced pressure, and the residue was purified by column chromatography with an acetone-hexane system to give 6-benzyloxy-4-(5,6-dimethyl-2-pyrimidin-2-yl-pyridin-3-yloxy)-quinoline (1.52 g, yield 73%).

6-Benzyloxy-4-(5,6-dimethyl-2-pyrimidin-2-yl-pyrid in-3-yloxy)-quinoline (773 mg) was dissolved in trifluoroacetic acid (3 ml). Methanesulfonic acid (0.3 ml) was added to the solution, and the mixture was stirred at room temperature for 4 hr. Water was added to the reaction mixture, and the mixture was neutralized with an aqueous sodium hydrogencarbonate solution. The organic layer was extracted with ethyl acetate, and the ethyl acetate layer was then washed with water and saturated brine and was dried over anhydrous sodium sulfate. The solvent was removed by distillation under the

reduced pressure, and the residue was washed with diethyl ether to give 4-(5,6-dimethyl-2-pyrimidin-2-yl-pyridin-3-yloxy)-quinolin-6-ol (467 mg, yield 76%).

4-(5,6-Dimethyl-2-pyrimidin-2-yl-pyridin-3-yloxy)-quinolin-6-ol (439 mg) was dissolved in N,N-dimethylformamide (5 ml). Potassium carbonate (0.89 g) and 2-bromoethanol (0.27 ml) were added to the solution, and the mixture was stirred at 80˚C for 5.5 hr. Water was added to the reaction mixture, and the mixture was extracted with ethyl acetate. The ethyl acetate layer was washed with water and saturated brine and was dried over anhydrous sodium sulfate. The solvent was removed by distillation under the reduced pressure, and the residue was purified by thin layer chromatography with a methanol-chloroform system to give the title compound (159 mg, yield 32%).

$^{1}$H-NMR ($CDCl_3$, 400 MHz): δ 2.41 (s, 3H), 2.69 (s, 3H), 4.03 (t, J = 4.4 Hz, 2H), 4.23 (t, J = 4.9 Hz, 2H), 6.49 (d, J = 5.2 Hz, 1H), 7.10(t, J = 4.9 Hz, 1H), 7.38 - 7.41 (m, 2H), 7.60 (d, J = 3.0 Hz, 1H), 7.96 (d, J = 9.3 Hz, 1H), 8.46 (d, J = 5.2 Hz, 1H), 8.65 (d, J = 4.9 Hz, 2H)

Mass spectrometric value (ESI-MS, m/z): 411 $(M+Na)^+$

Example 202: 2-[4-(6-Methyl-2-pyrimidin-2-yl-pyridin-3-yloxy)-quinolin-6-yloxy]-ethanol (compound 202)

6-Benzyloxy-4-chloro-quinoline (2.73 g), 6-methyl-2-pyrimidin-2-yl-pyridin-3-ol(949 mg), and cesium carbonate (4.96 g) were suspended in dimethylsulfoxide (25 ml), and the suspension was stirred at 130˚C overnight. The reaction mixture was cooled to room temperature, and water was added thereto. The mixture was extracted with chloroform, and the chloroform layer was washed with saturated brine and was dried over anhydrous magnesium sulfate. The solvent was removed by distillation under the reduced pressure, and the residue was purified by column chromatography with a chloroform-methanol system to give 6-benzyloxy-4-(6-methyl-2-pyrimidin-2-yl-pyridin-3-yloxy)-quinoline (1.64 g, yield 77%).

6-Benzyloxy-4-(6-methyl-2-pyrimidin-2-yl-pyridin-3- yloxy)-quinoline (1.64 g) was dissolved in trifluoroacetic acid (30 ml). Methanesulfonic acid (2.5 ml) was added to the solution, and the solution was stirred at 80˚C for one hr. Water was added to the reaction mixture, the mixture was neutralized with an aqueous sodium hydrogencarbonate solution, and the organic layer was extracted with chloroform. The chloroform layer was washed with water and was dried over anhydrous sodium sulfate. The solvent was removed by distillation under the reduced pressure to give 4-(6-methyl-2-pyrimidin-2-yl-pyridin-3-yloxy)-quinolin-6-ol (1.28 g, yield 99%).

4-(6-Methyl-2-pyrimidin-2-yl-pyridin-3-yloxy)-quino lin-6-ol (1.10 g) was dissolved in N,N-dimethylformamide (10 ml). Potassium carbonate (1.85 g), N,N,N,N-tetrabutylammonium iodide (124 mg), and 2-bromoethanol (1.47 g) were added to the solution, and the mixture was stirred at 40˚C for 20 hr. The solvent was removed by distillation under the reduced pressure. Water was added to the residue, and the mixture was extracted with chloroform. The chloroform layer was dried over anhydrous magnesium sulfate. The solvent was removed by distillation under the reduced pressure, and the residue was purified by column chromatography with a methanol-chloroform system to give the title compound (745 mg, yield 59%).

$^{1}$H-NMR ($CDCl_3$, 400 MHz): δ 2.74 (s, 3H), 4.03(t, J = 4.4 Hz, 2H), 4.21(t, J = 4.4 Hz, 2H), 6.50 (d, J = 5.2 Hz, 1H), 7.12 (t, J = 4.8 Hz, 1H), 7.36 (dd, J = 9.6, 2.8 Hz, 1H), 7.39 (d, J = 8.4 Hz, 1H), 7.55 (d, J = 8.4 Hz, 1H), 7.57 (d, J = 2.8 Hz, 1H), 7.94 (d, J = 9.6 Hz, 1H), 8.46 (d, J = 5.2 Hz, 1H), 8.66 (d, J = 4.8 Hz, 2H)

Mass spectrometric value (ESI-MS, m/z): 397 $(M+Na)^+$

Example 205; 2-[4-(6-Methyl-[2,2']bipyridyl-3-yloxy)-quinolin-6-yloxy]-ethanol (compound 205)

6-Iodo-2-picolin-5-ol (10.0 g) was dissolved in N,N-dimethylformamide (100 ml). Benzyl bromide (7.50 g) and potassium carbonate (17.6 g) were added to the solution, and the mixture was stirred at room temperature for 6 hr. The reaction mixture was filtered, water was added to the filtrate, and the mixture was extracted with ethyl acetate. The ethyl acetate layer was washed with water and was dried over sodium sulfate, and the solvent was removed by distillation under the reduced pressure. The residue was used in the next reaction without purification.

The residue and dichlorobis(triphenylphosphine)-palladium(II) (1.50 g) were dissolved in anhydrous tetrahydrofuran (200 ml) under an argon atmosphere. A 0.5 M 2-pyridylzinc bromide/tetrahydrofuran solution (100 ml) was added to the solution, and the mixture was stirred under reflux overnight. The reaction mixture was cooled to room temperature, and the solvent was removed by distillation under the reduced pressure. A 1 N sodium hydroxide solution was added to the residue, and the mixture was extracted with chloroform. The chloroform layer was washed with water and was then dried over sodium sulfate, and the solvent was removed by distillation under the reduced pressure. The residue was used in the next reaction without purification.

Trifluoroacetic acid (40 ml) and methanesulfonic acid (2 ml) were added to the residue, and the mixture was stirred at 100˚C overnight. The reaction mixture was cooled to room temperature, and the solvent was removed by distillation under the reduced pressure. The residue was neutralized with a saturated aqueous sodium bicarbonate solution and was then filtered. The filtrate was extracted with ethyl acetate, and the ethyl acetate layer was washed with water and

was extracted with 1 N hydrochloric acid. The aqueous layer was washed with ethyl acetate and was then neutralized by the addition of a saturated aqueous sodium bicarbonate solution. The aqueous layer was extracted with ethyl acetate, was washed with water and was dried over sodium sulfate, and the solvent was removed by distillation under the reduced pressure. The residue was purified by column chromatography with a hexane-ethyl acetate system to give 6-methyl-[2,2'] bipyridyl-3-ol (5.09 g, yield 64%) (3 steps).

4-Benzyloxy-phenylamine hydrochloride (1.18 g) and 5-methoxymethylene-2,2-dimethyl-[1,3]dioxan-4,6-dione (1.03 g) were suspended in 2-propanol (15 ml). Triethylamine (0.78 ml) was added to the suspension, and the mixture was stirred at 70˚C for 3 hr. The reaction mixture was cooled to room temperature, was filtered and was washed with ether. The residue as such was used in the next reaction without purification.

The residue and biphenyl (4.34 g) were suspended in diphenyl ether (12 ml), and the suspension was stirred at 240˚C overnight. The reaction mixture was cooled to room temperature, and the cooled reaction mixture as such was applied to column chromatography with a methanol-chloroform system. The crude product as such was used in the next reaction without further purification.

The crude product was suspended in diisopropylethylamine (3 ml), phosphorus oxychloride (0.5 ml) was added to the suspension, and the mixture was stirred at 120˚C for 3hr. Water was added to the reaction mixture under ice cooling. The aqueous layer was neutralized with an aqueous sodium hydrogencarbonate solution, and the organic layer was extracted with ethyl acetate. The ethyl acetate layer was then washed with water and was dried over anhydrous sodium sulfate. The solvent was removed by distillation under the reduced pressure, and the residue was purified by column chromatography with an ethyl acetate-hexane system to give 6-benzyloxy-4-chloro-quinoline (147 mg, yield 10%) (3 steps).

Anhydrous dimethylsulfoxide (25 ml) was added to 6-methyl-[2,2']bipyridyl-3-ol (2.00 g), 6-benzyloxy-4-chloroquinoline (5.79 g), and cesium carbonate (7.00 g), and the mixture was stirred at 130˚C overnight. The reaction mixture was cooled to room temperature. Water was added to the cooled reaction mixture, and the mixture was extracted with chloroform. The chloroform layer was washed with water and was dried over sodium sulfate, and the solvent was removed by distillation under the reduced pressure. The residue was purified by column chromatography with a hexane-acetone system to give 3-(6-benzyloxy-quinolin-4-yloxy)-6-methyl-[2,2']bipyridyl (3.38 g, yield 75%).

Trifluoroacetic acid (10 ml) and methanesulfonic acid (0.5 ml were added to 3-(6-benzyloxy-quinolin-4-yloxy)-6-methyl-[2,2']bipyridyl (3.36 g), and the mixture was stirred under reflux for 6 hr. The solvent was removed by distillation under the reduced pressure. The residue was neturatlized by the addition of a saturated aqueous sodium bicarbonate solution. Chloroform was added thereto, and the resultant precipitate was collected by filtration and was washed with water and chloroform to give crystals. The filtrate was subjected to separation, and the chloroform layer was washed with water and was then dried over sodium sulfate. The solvent was removed by distillation under the reduced pressure. The residue was purified by column chromatography with a chloroform-methanol system. The purified product was combined with the above crystals to give 4-(6-methyl-[2,2']bipyridyl-3-yloxy)-quinolin-6-ol (2.42 g, yield 92%).

N,N-Dimethylformamide (40 ml) was added to 4-(6-methyl-[2,2']bipyridyl-3-yloxy)-quinolin-6-ol (2.42 g), potassium carbonate (3.05 g), and 2-bromoethanol (2.76 g), and the mixture was stirred at 60˚C overnight. The reaction mixture was cooled to room temperature, water was added to the cooled mixture, and the mixture was extracted with ethyl acetate. The ethyl acetate layer was dried over sodium sulfate, and the solvent was removed by distillation under the reduced pressure. The residue was purified by column chromatography with a chloroform-methanol system to give the title compound (1.19 g, yield 43%).

$^1$H-NMR ($CDCl_3$, 400 MHz): $\delta$ 2.33(bs, 1H), 2.73 (s, 3H); 4.04 (m, 2H), 4.23 (t, J = 4.2 Hz, 2H), 6.45 (d, J = 5.1 Hz, 1H), 7.14 (dd, J = 7.6, 4.9 Hz, 1H), 7.32 (d, J = 8.3 Hz, 1H), 7.40 (dd, J = 2.7, 9.3 Hz, 1H), 7.51 (d, J = 8.3 Hz, 1H), 7.57 - 7.65 (m, 2H), 7.80 (d, J = 7.3 Hz, 1H), 7.96 (d, J = 9.3 Hz, 1H), 8.46 (d, J = 5.1 Hz, 1H), 8.52 (m, J = 4.6 Hz, 1H)

Mass spectrometric value (ESI-MS, m/z): 374 $(M+1)^+$

Example 206: 2-[4-(6-Ethyl-2-pyrimidin-2-yl-pyridin-3-yloxy)-quinolin-6-yloxy]-ethyl acetate (compound 206)

2-[4-(6-Ethyl-2-pyrimidin-2-yl-pyridin-3-yloxy)-qui nolin-6-yloxy]-ethanol (compound 192) (210 mg) was dissolved in dichloromethane (5 ml). Triethylamine (0.75 ml) and acetic anhydride (0.15 ml) were added to the solution under ice cooling, and the mixture was stirred at room temperature for 3 hr. Water was added to the reaction mixture, and the mixture was neutralized with an aqueous sodium hydrogencarbonate solution. The organic layer was extracted with ethyl acetate, and the organic layer was then washed with saturated brine and was dried over anhydrous sodium sulfate. The solvent was removed by distillation under the reduced pressure, and the residue was purified by thin layer chromatography with a methanol-chloroform system to give the title compound (202 mg, yield 87%).

$^1$H-NMR ($CDCl_3$, 400 MHz): $\delta$ 1.41 (t, J = 7.8 Hz, 3H), 2.12 (s, 3H), 3.04 (q, J = 7.6 Hz, 2H), 4.31 (t, J = 4.9 Hz, 2H), 4.49 (t, J = 4.9 Hz, 2H), 6.52 (d, J = 5.1 Hz, 1H), 7.13 (t, J = 4.9 Hz, 1H), 7.38 - 7.44 (m, 2H), 7.56 - 7.59 (m, 2H), 7.96 (d, J = 9.3 Hz, 1H), 8.48 (d, J = 5.1 Hz, 1H), 8.67 (d, J = 4.9 Hz, 2H)

Mass spectrometric value (ESI-MS, m/z): 453 $(M+Na)^+$

Example 209: 4-(6-Methyl-2-pyrimidin-2-yl-pyridin-3-yloxy)-6-(2-morpholin-4--yl-ethoxy)-quinoline (compound 209)

4-(6-Methyl-2-pyrimidin-2-yl-pyridin-3-yloxy)-quino lin-6-ol (1.03 g) was dissolved in N,N-dimethylformamide (15 ml). Potassium carbonate (1.60 g) and 2-bromo-1-chloroethane (1.5 ml) were added to the solution, and the mixture was stirred at 65˚C overnight. Water was added to the reaction mixture, and the mixture was extracted with ethyl acetate. The organic layer was washed with water and saturated brine and was dried over anhydrous sodium sulfate. The solvent was removed by distillation under the reduced pressure, and the residue was purified by column chromatography on silica gel with an ethyl acetate-hexane system to give 6-(2-chloro-ethoxy)-4-(6-methyl-2-pyrimidin-2-yl-pyridin-3-ylox y)-quinoline as a crude product (741 mg).
A part (103 mg) of the crude product was dissolved in N,N-dimethylformamide (2 ml). Potassium carbonate (240 mg) and morpholine (0.2 ml) were added to the solution, and the mixture was stirred at 80˚C overnight. Water was added to the reaction mixture, and the mixture was extracted with ethyl acetate. The organic layer was then washed with water and saturated brine and was dried over anhydrous sodium sulfate. The solvent was removed by distillation under the reduced pressure, and the residue was purified by column chromatography on silica gel with an acetone-chloroform system to give the title compound (24 mg, yield 12%) (2 steps).
$^1$H-NMR ($CDCl_3$, 400 MHz): δ 2.60 - 2.6.2 (m, 4H), 2.76 (s, 3H), 2.87 (t, J = 5.6 Hz, 2H), 3.73 - 3.75 (m, 4H), 4.24 (t, J = 5.6 Hz, 2H), 6.50 (d, J = 5.4 Hz, 1H), 7.13 (t, J = 4.9 Hz, 1H), 7.37 - 7.41 (m, 2H), 7.54 - 7.57 (m, 2H), 7.94 (d, J = 9.3 Hz, 1H), 8.46 (d, J = 5.2 Hz, 1H), 8.67 (d, J = 4.9 Hz, 2H)
Mass spectrometric value (ESI-MS, m/z): 466 $(M+Na)^+$

Reference Examples

Synthesis schemes and Synthesis Examples of quinoline derivatives or quinazoline derivatives, that is, compounds r1 to r469, having structures close to those of the compounds according to the present invention will be described. Typical Synthesis Examples in each of the following shemes will be described. Other compounds in each of the schemes could easily be produced by a person having ordinary skill in the art according to the procedure of each scheme and the description of typical Synthesis Examples in each of the schemes.
Reference Production Example 1 shows a production example of intermediate r1 in scheme r3. Reference Production Example 2 shows a production examples of intermediate r2 in scheme r4. Reference Production Examples 3 and 4 show production examples of intermediates r3 and r4 in scheme r5.

Reference Production Example 1: 2-[(6,7-Dimethoxy-4-quinolyl)oxy]-5-methylbenzaldehyde (intermediate r1)

4-Chloro-6,7-dimethoxyquinoline (113 mg), 2-hydroxy-5-methylbenzaldehyde (344 mg), and 4-dimethylaminopyridine (313 mg) were suspended in o-dichlorobenzene (5 ml), and the suspension was stirred at 160˚C for 2 hr. The reaction mixture was cooled to room temperature, and the solvent was then removed from the reaction mixture by distillation under the reduced pressure. Chloroform was added to the residue. The organic layer was washed with a 1 N aqueous sodium hydroxide solution and saturated brine and was dried over anhydrous magnesium sulfate. The solvent was removed from the reaction mixture by distillation under the reduced pressure. The residue was purified by column chromatography with a chloroform system to give the title compound (157 mg, yield 96%).
$^1$H-NMR ($CDCl_3$, 400 MHz): δ 2.46 (s, 3H), 4.06 (s, 3H), 4.06 (s, 3H), 6.44 (d, J = 5.1 Hz, 1H), 7.10 (d, J = 8.3 Hz, 1H), 7.45 (s, 1H), 7.49 (m, 1H), 7.57 (s, 1H), 7.83 (d, J = 1.9 Hz, 1H), 8.51 (d, J = 1.5 Hz, 1H), 10.28 (s, 1H)
Mass spectrometric value (ESI-MS, m/z): 324 $(M+1)^+$

Reference Production Example 2: 2-(6,7-Dimethoxyquinolin-4-yloxy)-5-methoxybenzoic acid (intermediate r2)

Ethyl 2-(6,7-dimethoxyquinolin-4-yloxy)-5-methoxy-benzoate (143 mg) and lithium hydroxide (78 mg) were suspended in a mixed solvent composed of ethanol (10 ml) and water (1 ml), and the suspension was stirred at room temperature overnight. Next, the solvent was removed from the reaction mixture by distillation under the reduced pressure. Water was added to the residue, and the mixture was neutralized with 12 N hydrochloric acid. The mixture was then extracted with chloroform. The chloroform layer was washed with water and saturated brine and was dried over anhydrous sodium sulfate. The solvent was removed from the extract by distillation under the reduced pressure to give the title compound (140 mg, yield 100%).
$^1$H-NMR ($CDCl_3$-$d_1$, 400 MHz): δ 3.73 (s, 3H), 3.80 (s, 3H), 3.95 (s, 3H), 6.44 (d, J = 5.6 Hz, 1H), 6.91 - 7.19 (m, 3H), 7.34 (s, 1H), 7.46 (s, 1H), 7.60 (s, 1H), 8.10 (d, J = 5.6 Hz, 1H), 13.26 (brs, 1H),
Mass spectrometric value (ESI-MS, m/z): 356 $(M^++1)$

Reference Production Example 3: [2-(7-Benzyloxy-6-methoxyquinolin-4-yloxy)-5-methoxyphenyl] ethanone (intermediate r3)

7-Benzyloxy-4-chloro-6-methoxyquinoline (3.00 mg), 5-methoxy-2-acetophenone (6.7 g), and 4-dimethylaminopyridine (4.9 g) were suspended in o-dichlorobenzene (30 ml), and the suspension was stirred at 180°C for 2 hr. The reaction mixture was cooled to room temperature. Water was added to the cooled reaction mixture, and the mixture was extracted with chloroform. The chloroform layer was washed with water and saturated brine and was dried over anhydrous sodium sulfate. The solvent was removed from the reaction mixture by distillation under the reduced pressure. The residue was purified by thin layer chromatography with an acetone-hexane system to give the title compound (2.53 g, yield 59%).
$^1$H-NMR ($CDCl_3$-$d_1$, 400 MHz): δ 2.42 (s, 3H), 3.82 (s, 3H), 3.98 (s, 3H), 5.26 (s, 2H), 6.28 (d, J = 5.6 Hz, 1H), 7.03 - 7.08 (m, 1H), 7.23 - 7.51 (m, 9H), 8.39 (d, J = 5.6 Hz, 1H)
Mass spectrometric value (ESI-MS, m/z): 430 ($M^+$+1)

Reference Production Example 4: [2-(7-Hydroxy-6-methoxyquinolin-4-yloxy)-5-methoxyphenyl]ethanone (intermediate r4)

[2-(7-Benzyloxy-6-methoxyquinolin-4-yloxy)-5-met hoxy-phenyl]ethanone (intermediate r3) (2.52 g) was suspended in a mixed solvent composed of methanesulfonic acid (3.0 ml) and trifluoroacetic acid (50 ml), and the suspension was stirred at 70°C for 0.5 hr. The solvent was removed from the reaction mixture by distillation under the reduced pressure. Water was added to the residue, and the mixture was neutralized with a sodium hydrogencarbonate powder. The mixture was then extracted with chloroform. The chloroform layer was then washed with water and saturated brine and was dried over anhydrous sodium sulfate. The solvent was removed from the reaction mixture by distillation under the reduced pressure. The residue was purified by column chromatography with an acetone-hexane system to give the title compound (1.23 g, yield 62%).
$^1$H-NMR (DMSO-$d_6$, 400 MHz): δ 2.50 (s, 3H), 3.91 (s, 3H), 4.00 (s, 3H), 6.35 (d, J = 5.2 Hz, 1H), 7.34 (m, 3H), 7.42 (s, 1H), 7.61 (s, 1H), 8.44 (d, J = 5.2 Hz, 1H), 10.24 (brs, 1H),
Mass spectrometric value (ESI-MS, m/z): 338 ($M^+$-1)

Production of compounds of Reference Examples

Compounds according to the present invention and compounds close thereto were produced.
The relationship between the produced compounds and the schemes applied to the production of the compounds is as shown in the following table.
Table r-1:

| Scheme | Compound |
|---|---|
| r2: | r1 to r5, r12, r13, r15 to r17, r19, r22, r24, r26 to r42, r57, r62 to r72, r74, r75, r77, r78, r161, r162 and r425 |
| r3: | r9 to r11, r18, r43 to 56, r58 to 61, r73, r166, r172, r181, r188 to r192, r293, r306 to r311 and r418 to r420 |
| r4: | r79 to r114 |
| r5: | r118 to r160, r312 to r409 and r440 to r469 |
| r6: | r163 |
| r7: | r7 and r8 |
| r8: | r6 |
| r9: | r14 |
| r10: | r20 and r21 |
| r11: | r23 |
| r12: | r76, r165, r173, r174 and r182 to r187 |
| r13: | r115 to r117, r202, r203, r205, r206, r208 to r210, r214, r215, r217 to r221, r275 and r276 |

Table r-2:

| Scheme | Compound |
|---|---|
| r14: | r164, r167 to r171, r294 to r296, r298 and r302 |
| r15: | r175 to r180 |
| r16: | r200 and r201 |
| r17: | r204, r222 and r280 |
| r18: | r207 |
| r19: | r211, r212, r216, r223 to r255, r259, r261 to r267, r291, r434 and r438 |
| r20: | r213 |
| r21: | r256 to r258 |
| r22: | r277 |
| r23: | r278 and r279 |
| r24: | r281, r282, r284 and r287 to r289 |
| r25: | r283, r285, r286, r290, r292, r421 to r424, r426 to r428, r436, and r439 |
| r26: | r297 |
| r27: | r299 |
| r28: | r300 |
| r29: | r301, r303 and r304 |
| r30: | r305 |
| r31: | r410 to r414 |
| r32: | r415 to r417 |
| r33: | r193 to r199 |
| r34: | r260, r268 to r274, r429 to r433, r435 and r437 |

Production schemes

A certain group of compounds related to the compounds according to the present invention can be produced according to the following schemes r1 to r34.
In the description of production schemes r1 to r34 in the following Reference Examples, symbols of substituents such as R1 and R2 are used. Despite the definition in the description other than Reference Examples in the present specification, the symbols of the substituents means as defined in the Reference Examples.

Scheme r1:

[Chemical formula 39]

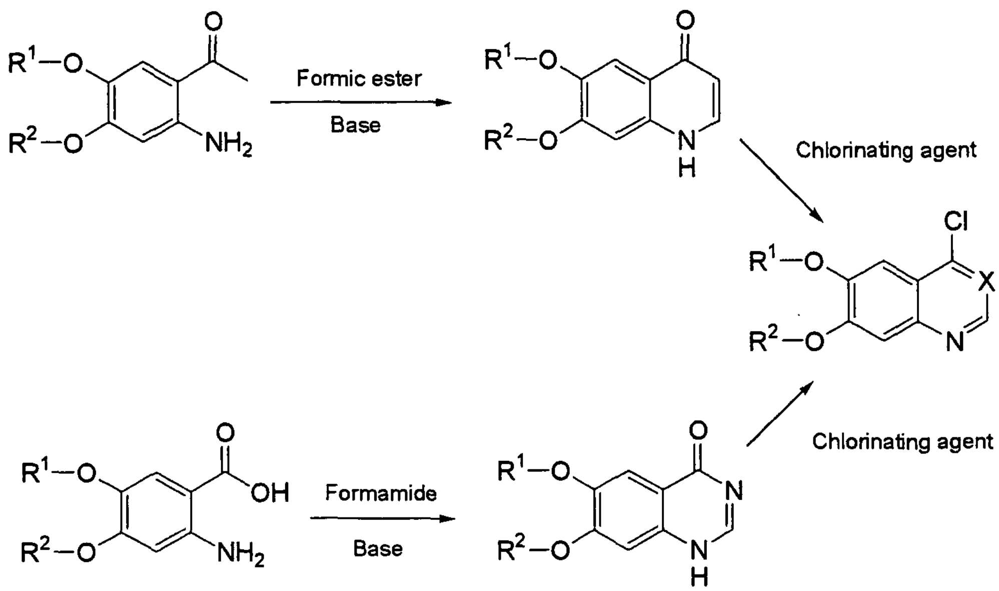


wherein X represents CH or N, and
$R^1$ and $R^2$ independently may represent group $-OR^X$
wherein $R^X$ represents a hydrogen atom or $-(CH_2)m-R^{aX}$. $R^{aX}$ represents a hydrogen atom, a halogen atom, hydroxyl, a saturated or unsaturated three- to six-membered carbocyclic or heterocyclic group, C1-4 alkoxy, C1-4 alkoxycarbonyl, or $-NR^{bX}R^{cX}$. $R^{bx}$ and $R^{cx}$, which may be the same or different, represent a hydrogen atom or C1-6 alkyl wherein the C1-6 alkyl group is optionally substituted by hydroxyl, an oxygen atom, amino, a nitrogen atom, or C1-4 alkyl. $R^{bx}$ and $R^{cX}$ together may combine with the nitrogen atom to which they are attached to form a saturated or unsaturated five- or six-membered heterocyclic group. This heterocyclic group may further contain one or more heteroatoms. The heterocyclic group is optionally substituted by C1-4 alkyl optionally substituted by hydroxyl; hydroxyl; an oxygen atom; aminocarbonyl; C1-4 alkoxy; C1-4 alkoxycarbonyl; or a saturated or unsaturated five- or six-membered heterocyclic group. Further, the heterocyclic group may condense with another saturated or unsaturated five- or six-membered carbocyclic or heterocyclic group to form a bicyclic group. m is an integer of 1 to 6. The alkyl chain part $-(CH_2)m-$ in the group $-(CH_2)m-R^{aX}$ is optionally substituted by hydroxy; an oxygen atom; $-OR^{dX}$ wherein $R^{dX}$ represents C1-4 alkyl or C1-4 alkylcarbonyl; or C1-4 alkyl optionally substituted by hydroxyl or a halogen atom.

Scheme r2:

[Chemical formula 40] .

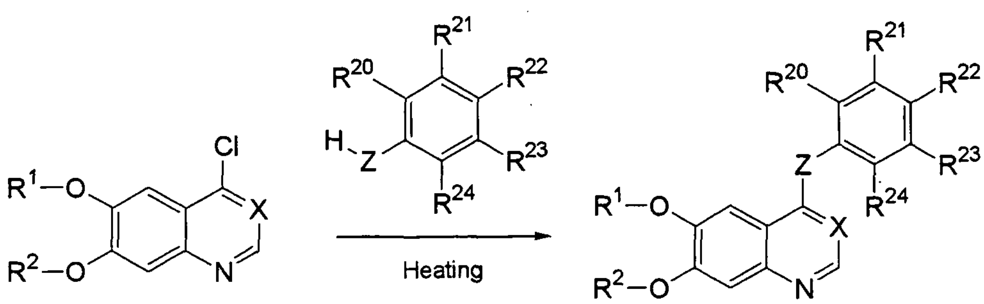


wherein Z represents -O-, -NH-, -S-, or -C(=O)-, and
$R^{20}$ to $R^{24}$, which may be the same or different, represent a hydrogen atom, a halogen atom, C1-10 alkyl, C1-8 alkoxy, C2-6 alkenyl, phenylcarbonyl, amino, nitro, or a saturated or unsaturated five- or six-membered carbocyclic or heterocyclic group. $R^{20}$ and $R^{21}$, or $R^{23}$ and $R^{24}$ together may combine with the carbon atoms to which they are attached represent

a saturated or unsaturated five- or six-membered carbocyclic or heterocyclic group. The other substituents are as defined in other production schemes in other Reference Examples.

The aimed compounds, i.e., 4-phenoxyquinoline derivatives, 4-anilinoquinoline derivatives, or corresponding quinazoline derivatives can be synthesized by reacting a phenol derivative or a corresponding aniline derivative with a 4-chloroquinoline derivative or a corresponding quinazoline derivative in a suitable solvent, for example, o-dichlorobenzene, or in the absence of a solvent, for example, at 120 to 180°C. In scheme r1, phosphoryl chloride may be mentioned as the chlorinating agent.

Scheme r3:

[Chemical formula 41]

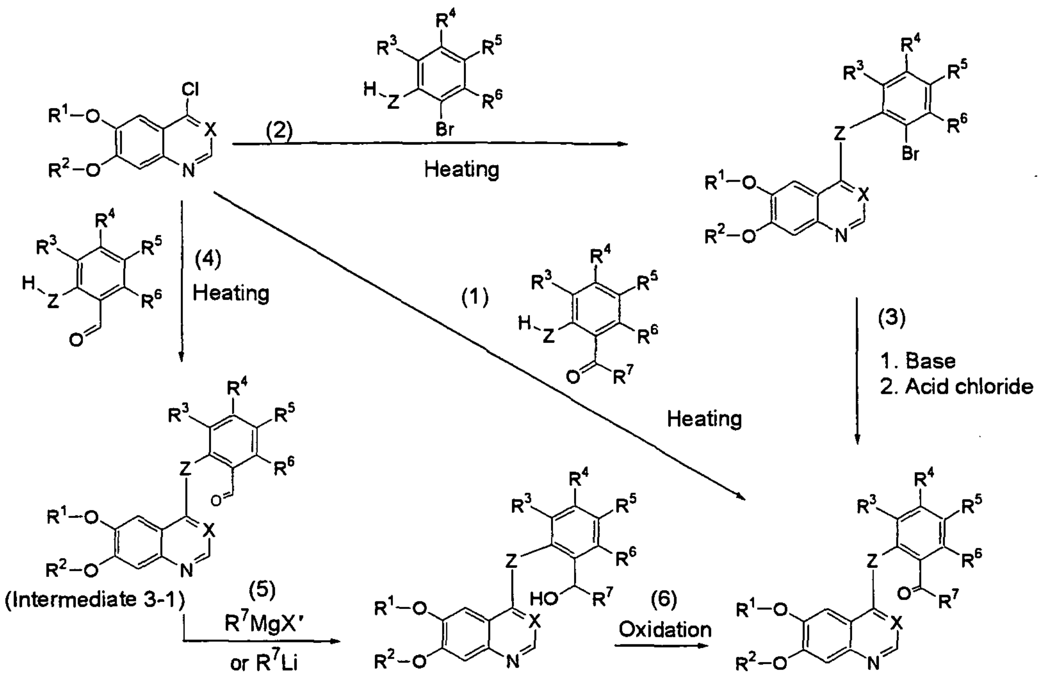


wherein X' represents a halogen atom,
$R^3$ to $R^6$, which may be the same or different, represent a hydrogen atom, hydroxyl, a halogen atom, C1-6 alkyl, C1-10 alkoxy, C2-6 alkenylcarbonyloxy, C1-4 alkylcarbonyl, C1-4 alkylthio, or phenyl, and $R^3$ and $R^4$, $R^4$ and $R^5$, and $R^5$ and $R^6$ together may combine with the carbon atoms to which they are attached represent a saturated or unsaturated five- or six-membered carbocyclic or heterocyclic group,
$R^7$ represents a hydrogen atom, C1-8 alkyl, C2-6 alkenyl, a saturated or an unsaturated five- or six-membered carbocyclic or heterocyclic group, group -O-$R^8$, or group -N(-$R^9$)$R^{10}$ wherein $R^8$, $R^9$ and $R^{10}$, and $R^{11}$ and $R^{12}$ represent C1-10 alkyl, C2-8 alkenyl, or a saturated or unsaturated five- or six-membered carbocyclic group or the like, and
the other substituents are as defined in other production schemes in other Reference Examples.

In this scheme, the aimed compounds can be synthesized through any of the following three routes (i) to (iii).

(i) The aimed compounds can be synthesized by reacting a 4-chloroquinoline derivative or a corresponding quinazoline derivative with a phenol derivative or a corresponding aniline derivative in a suitable solvent, for example, o-dichlorobenzene, or in the absence of a solvent, for example, at 120 to 180°C (step (1)).

(ii) The aimed compounds can be synthesized by reacting a 4-chloroquinoline derivative or a corresponding quinazoline derivative with an o-bromophenol derivative or a corresponding o-bromoaniline derivative in a suitable solvent, for example, o-dichlorobenzene, or in the absence of a solvent, for example, at 120 to 180˚C (step (2)), subjecting the bromo site to dipole inversion with a metal base, for example, n-butyllithium, and reacting the resultant anion with an acid chloride (step (3)).

(iii) The aimed compounds can be produced by reacting a 4-chloroquinoline derivative or a corresponding quinazoline derivative with an o-hydroxybenzaldehyde derivative or a corresponding o-aminobenzaldehyde derivartive in a suitable solvent, for example, o-dichlorobenzene, or in the absence of a solvent, for example, at 120 to 180˚C (step (4)), then reacting the resultant compound with an alkylating agent, for example, methylmagnesium bromide (step (5)), and oxidizing the resultant alcohol (step (6)).

Scheme r4:

[Chemical formula 42]

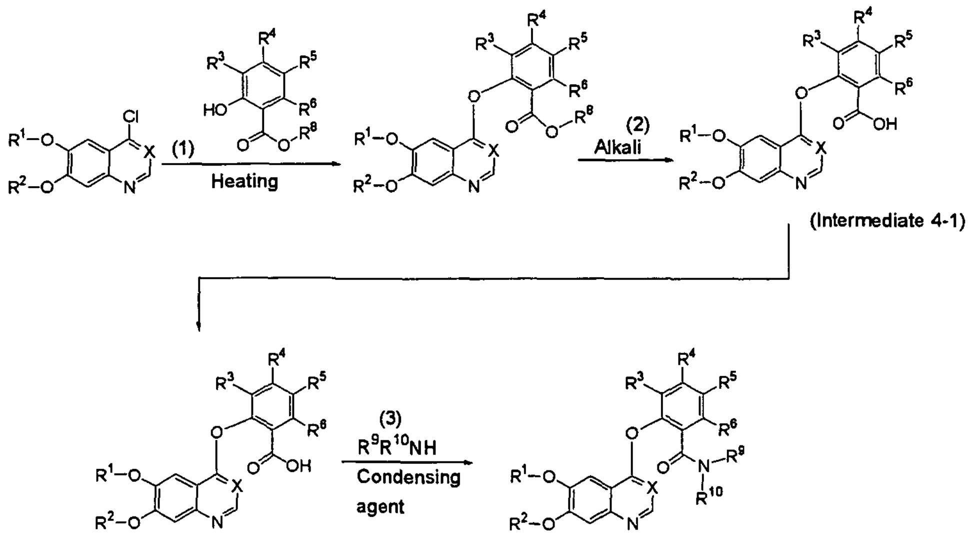


wherein the substituents are as defined above.

The aimed ester-type compounds can be produced by reacting a 4-chloroquinoline derivative or a corresponding quinazoline derivative with an o-hydroxybezoic ester derivative or a corresponding o-aminobenzoic ester derivative in a suitable solvent, for example, o-dichlorobenzene, or in the absence of a solvent, for example, at 120 to 180˚C (step (1)). Next, the aimed compounds can be synthesized by hydrolyzing the ester-type compound with an alkali (step (2)) and reacting the amine with a condensing agent, for example, 1-ethyl-3-(3-dimethylaminopropyl)carbodiimide hydrochloride (step (3)).

Scheme r5:

[Chemical formula 43]

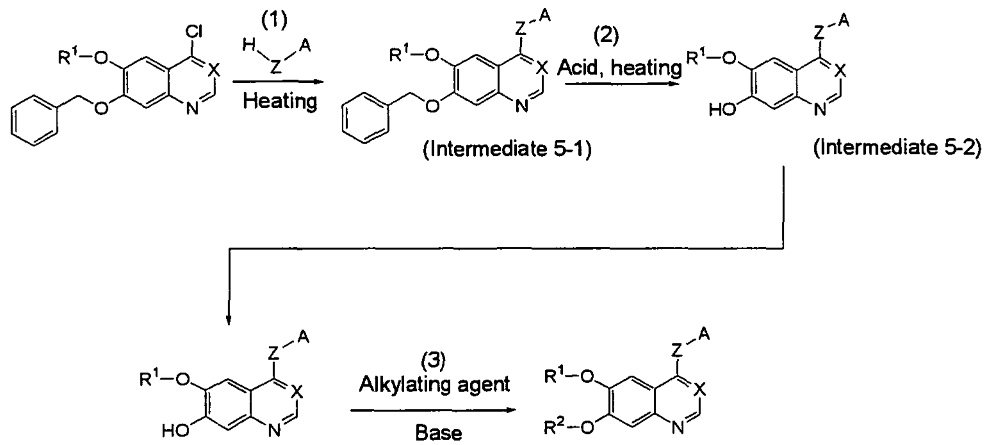


wherein A is as defined in the portions other than the Reference Examples in the present specification, and the other substituents are as defined above.

A 7-benzyloxy-4-chloroquinoline derivative or a corresponding quinazoline derivative is provided and is reacted with a phenol derivative or a corresponding aniline derivative in a suitable solvent, for example, o-dichlorobenzene, or in the absence of a solvent, for example, at 120 to 180°C (step (1)). The aimed compounds can be synthesized by deprotecting the resultant intermediate 5-1, that is, removing the benzyl group, with an acid (step (2)) and reacting the resultant intermediate 5-2 with an alkylating agent, for example, 1-bromo-2-chloroethane, in the presence of a base (step (3)).

Scheme r6:

[Chemical formula 44]

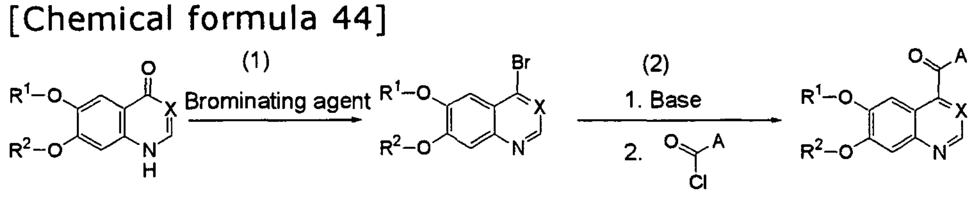


wherein the substituents are as defined above.

The aimed compounds can be produced by providing a 4-quinolone derivative or a corresponding quinazolone derivative, reacting this compound with a brominating agent, for example, phosphoryl bromide (step (1)), then subjecting the bromo site to dipole inversion with a metal base, for example, n-butyllithium, and reacting the resultant anion with an acid chloride (step (2)).

Scheme r7:

[Chemical formula 45]

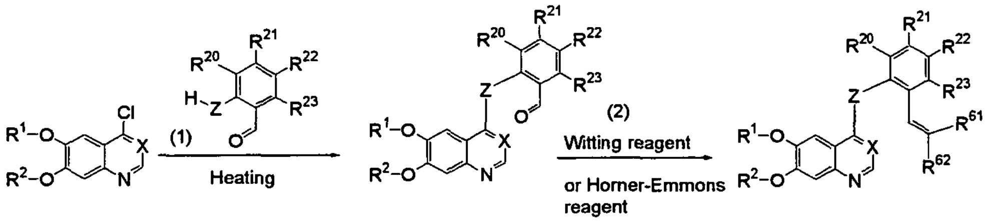


wherein $R^{61}$ and $R^{62}$, which may be the same or different, represent a hydrogen atom, C1-4 alkyl, C1-4 alkoxy, C1-4 alkoxycarbonyl, or phenyl, and the substituents are as defined above.

The aimed compounds can be synthesized by reacting a 4-chloroquinoline derivative or a corresponding quinazoline derivative with an o-hydroxybenzaldehyde derivative or a corresponding o-aminobenzaldehyde derivative in a suitable solvent, for example, o-dichlorobenzene, or in the absence of a solvent, for example, at 120 to 180˚C (step (1)) and then reacting the resultant compound with a Witting reagent or a Horner-Emmons reagent (step (2)). Phosphorus ylide may be mentioned as these reagents.

Scheme r8:

[Chemical formula 46]

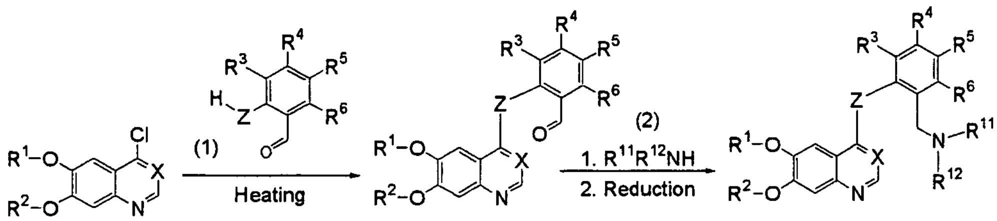


wherein the substituents are as defined above.

The aimed compounds can be produced by reacting a 4-chloroquinoline derivative or a corresponding quinazoline derivative with an o-hydroxybenzaldehyde derivative or a corresponding o-aminobenzaldehyde derivative in a suitable solvent, for example, o-dichlorobenzene, or in the absence of a solvent, for example, at 120 to 180˚C (step (1)), then reacting the resultant compound with an amine ($R^{11}R^{12}NH$) to give an imine, and then reducing the imine (step (2)).

Scheme r9:

[Chemical formula 47]

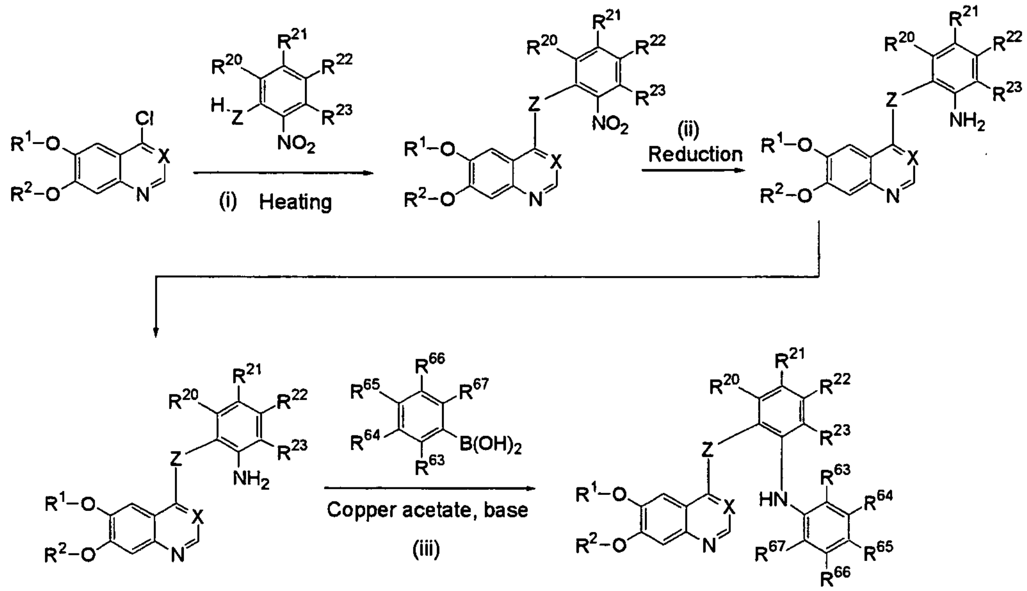


wherein $R^{63}$ to $R^{67}$, which may be the same or different, represent a hydrogen atom, hydroxyl, a halogen atom, or $C_{1-4}$ alkyl, and the other substituents are as defined above.

The aimed compounds can be synthesized by reacting a 4-chloroquinoline derivative or a corresponding quinazoline derivative with an o-nitrophenol derivative or a corresponding o-nitroaniline derivative in a suitable solvent, for example, o-dichlorobenzene, or in the absence of a solvent, for example, at 120 to 180°C (step (i)), then reducing the nitro group (step (ii)), and reacting the resultant compound with a phenylboronic acid derivative (step (iii)).

Scheme r10:

[Chemical formula 48]

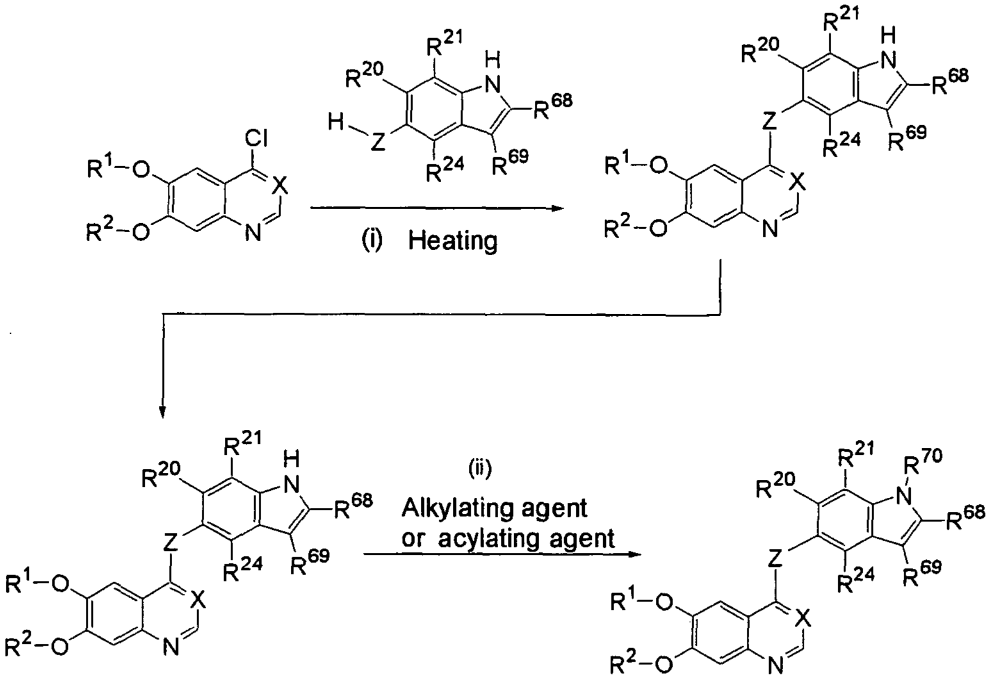


wherein $R^{68}$ to $R^{70}$, which may be the same or different, represent a hydrogen atom, a halogen atom, $C_{1-2}$ alkylcarbonyl, $C_{1-2}$ alkoxycarbonyl, or $C_{1-4}$ alkyl, and the other substituents are as defined above.

The aimed compounds can be synthesized by reacting a 4-chloroquinoline derivative or a corresponding quinazoline derivative with a 5-hydroxyindole derivative or a corresponding 5-aminoindole derivative in a suitable solvent, for example, o-dichlorobenzene, or in the absence of a solvent, for example, at 120 to 180˚C (step (i)) and then alkylating the amino group with an alkylating agent, for example, methyl iodide, or acylating the amino group with an acylating agent, for example, acetyl chloride (step (ii)).

Scheme r11:

[Chemical formula 49]

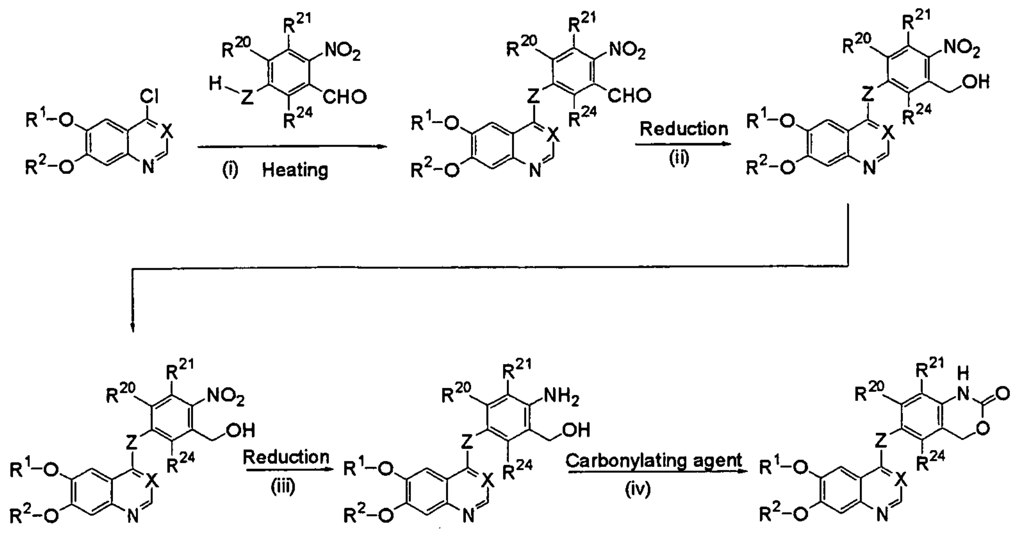


wherein the substituents are as defined above.

The aimed compounds can be synthesized by reacting a 4-chloroquinoline derivative or a corresponding quinazoline derivative with a 3-hydroxy-6-nitrobenzaldehyde derivative or a corresponding 5-amino-2-nitrobenzaldehyde derivative in a suitable solvent, for example, o-dichlorobenzene, or in the absence of a solvent (step (i)), reducing the formyl group (step (ii)), then reducing the nitro group in the resultant compound (step (iii)), and reacting the resultant compound with a carbonylation agent, for example, triphosgene (step (iv)).

Scheme r12:

[Chemical formula 50]

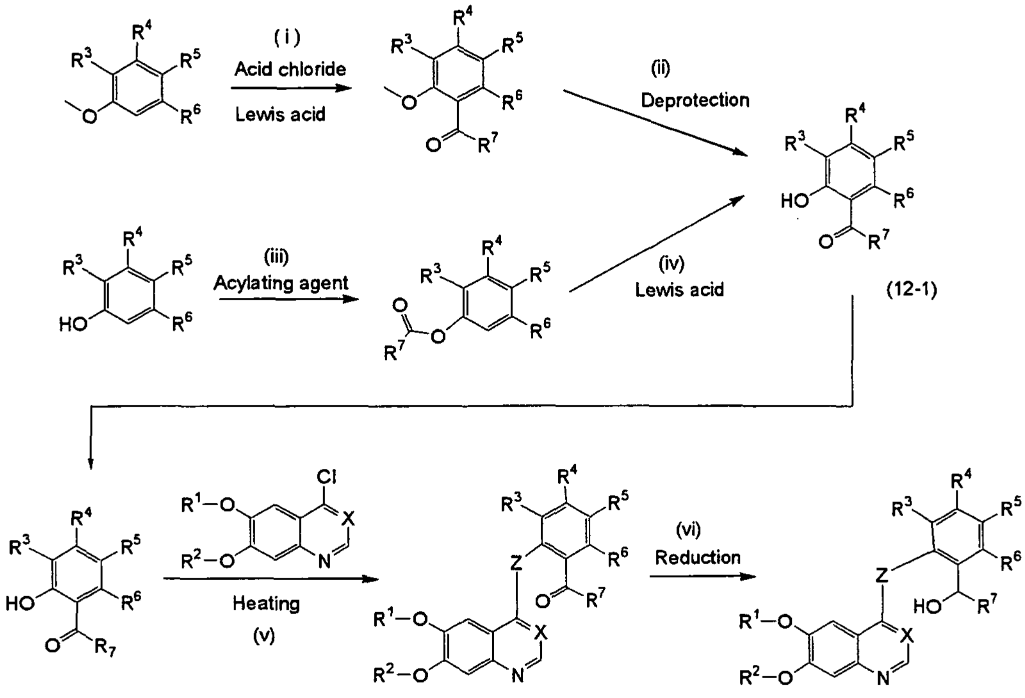


wherein the substituents are as defined above.

The compounds of formula (12-1) can be produced by reacting an anisole derivative with an acid chloride in the presence of a Lewis acid (step (i)) and deprotecting the resultant compound, that is, removing the methoxy group (step (ii)).

Alternatively, the compounds of formula (12-1) can be produced by acylating a phenol derivative with an acylating agent, for example, acetyl chloride or acetic anhydride (step (iii)) and then reacting the resultant compound with a Lewis acid, for example, scandium trifluoromethanesulfonate (step (iv)).

A aimed compound, for example, compound r76, can be synthesized by reacting the compound of formula (12-1) with a 4-chloroquinoline derivative or a corresponding quinazoline derivative in a suitable solvent, for example, o-dichlorobenzene, or in the absence of a solvent, for example, at 120 to 180°C (step (v)).

Further, a aimed compound, for example, compound r165, can be synthesized by reducing the acyl group in the resultant compound (step (vi)).

Scheme r13:

[Chemical formula 51]

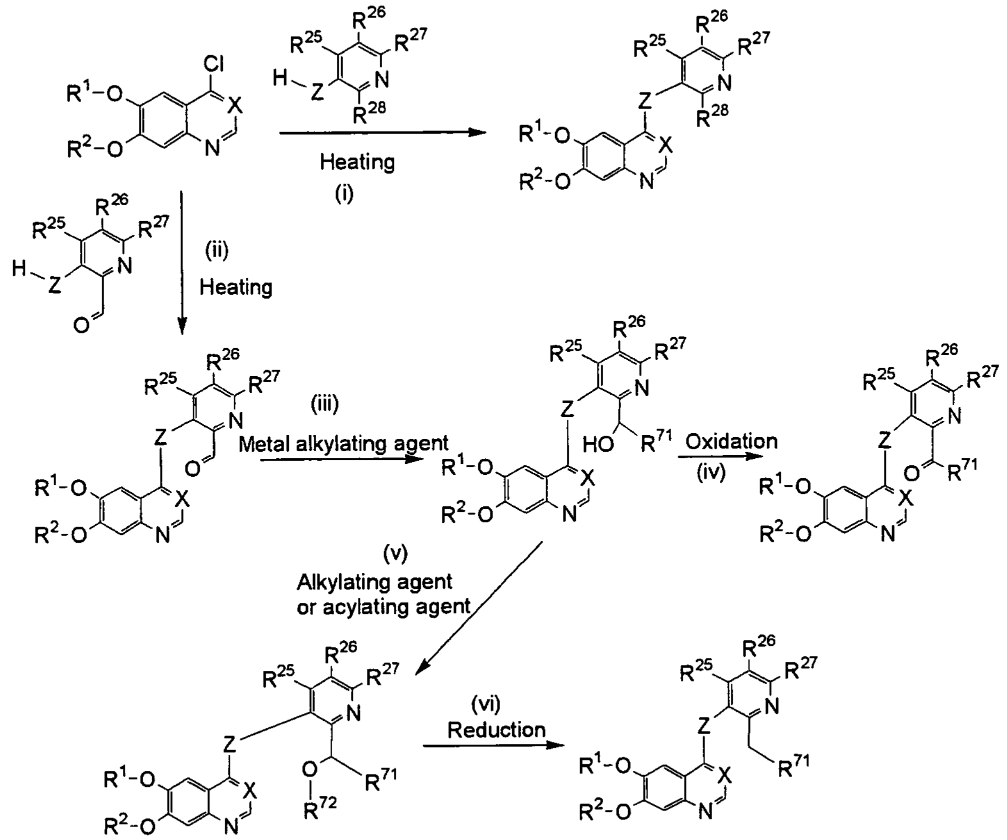


wherein $R^{25}$ to $R^{27}$, which may be the same or different, represent a hydrogen atom, a halogen atom, C1-6 alkyl, C1-8 alkoxy, C1-4 alkylcarbonyl, C1-4 alkylthio, or phenylcarbonyl, and $R^{25}$ and $R^{26}$, and $R^{26}$ and $R^{27}$ together may combine with the carbon atoms to which they are attached represent a saturated or unsaturated five- or six-membered carbocyclic or heterocyclic group,
$R^{28}$ represents a hydrogen atom, a halogen atom, nitro group, cyano group, C1-6 alkyl, C1-8 alkoxy, C1-4 alkylcarbonyl, C2-6 alkenyl, C2-6 alkynyl, a saturated or unsaturated three- to eight-membered carbocyclic oxy, a saturated or unsaturated six-membered carbocyclic carbonyl or heterocyclic carbonyl, or a saturated or unsaturated three- to eight-membered carbocyclic or heterocyclic group,
$R^{71}$ represents methyl, ethyl, phenyl, or 2-pyridyl,
$R^{72}$ represents a hydrogen atom or methylcarbonyl, and
the other substituents are as defined above.

The aimed compounds can be synthesized by reacting a 4-chloroquinoline derivative or a corresponding quinazoline derivative with a 3-hydroxypyridine derivative or a corresponding 3-aminopyridine derivative in a suitable solvent, for example, o-dichlorobenzene, or in the absence of a solvent, for example, at 120 to 180°C (step (i)).
Alternatively, a 4-chloroquinoline derivative or a corresponding quinazoline derivative is reacted with a 3-hydroxy-2-pyridine carbaldehyde derivative or a corresponding 3-amino-2-pyridine carbaldehyde derivative in a suitable solvent, for example, o-dichlorobenzene, or in the absence of a solvent, for example, at 120 to 180°C (step (ii)), and the resultant compound is then reacted with an alkylating agent, for example, methylmagnesium bromide (step (iii)).
A aimed compound, for example, compound r117, can be then synthesized by oxidizing the resultant alcoholic compound, for example, with manganese dioxide as an oxidizing agent (step (iv)).
Alternatively, another aimed compound, for example, compound r218, can be synthesized by alkylating the hydroxyl group in the resultant alcoholic compound with an alkylating agent, for example, methyl iodide or ethyl iodide, or by acylating the hydroxyl group in the resultant alcoholic compound with an acylating agent, for example, acetyl chloride or acetic anhydride (step (v)). A further aimed compound, for example, compound r214, can be synthesized by reducing this compound, for example, with a hydrogen gas/palladium hydroxide as a reducing agent (step (vi)).

Scheme r14:

[Chemical formula 52]

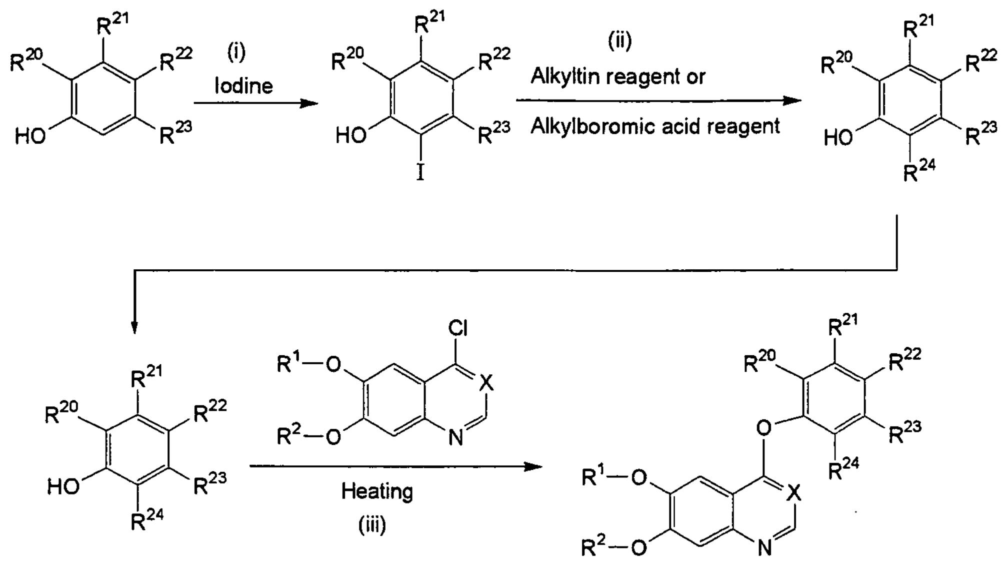


wherein the substituents are as defined above.

The aimed compounds can be produced by reacting a phenol derivative with iodine (step (i)), reacting the allyl iodide with an alkyltin reagent, for example, tri-n-butyl-(2-pyridyl)-tin, or an alkylboronic acid reagent, for example, 3-pyridylboronic acid, in the presence of a suitable transition metal catalyst, for example, tetrakistriphenylphosphine palladium (step (ii)), and reacting the phenol derivative thus obtained with a 4-chloroquinoline derivative or a corresponding quinazolone derivative in a suitable solvent, for example, o-dichlorobenzene, or in the absence of a solvent, for example, at 120 to 180°C (step (iii)).

Scheme r15:

[Chemical formula 53]

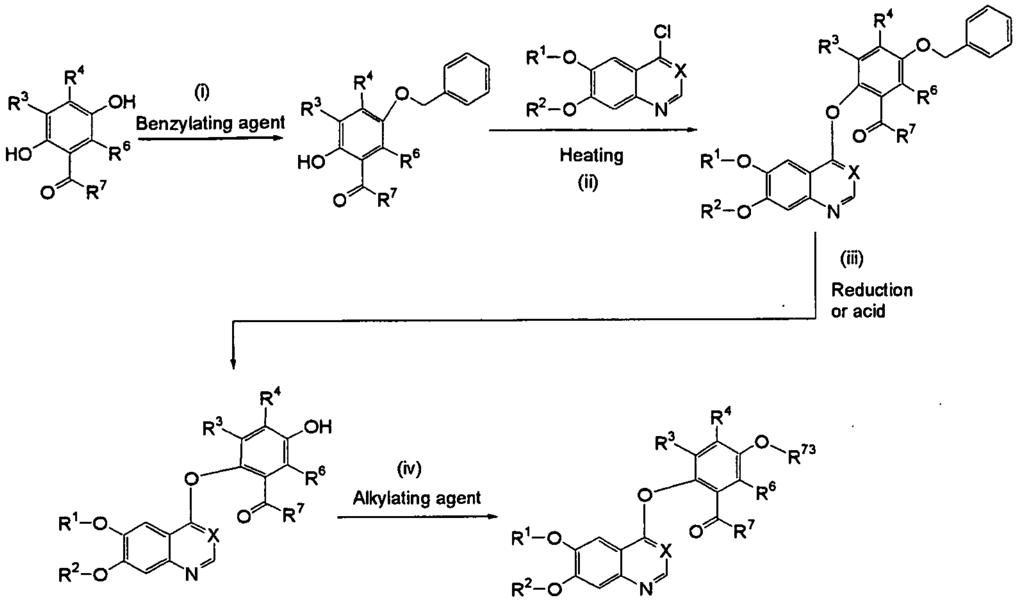


wherein $R^{73}$ represents a hydrogen atom, or $C_{1-4}$ alkyl optionally substituted by phenyl, and the other substituents are as defined above.

The aimed compounds can be produced by reacting a 2,5-dihydroxyphenyl ketone derivative with a benzylating agent, for example, benzyl bromide (step (i)), reacting the monophenol derivative thus obtained with a 4-chloroquinoline derivative or a corresponding quinazolone derivative in a suitable solvent, for example, o-dichlorobenzene, or in the absence of a solvent, for example, at 120 to 180˚C (step (ii), deprotecting the resultant compound, that is, removing the benzyl group in the resultant compound, with a suitable acid, for example, methanesulfonic acid/trifluoroacetic acid, or by reductiing agent (step (iii)), and then alkylating the phenolic hydroxyl in the resultant compound with an alkylating agent, for example, ethyl iodide (step (iv)).

Scheme r16:

[Chemical formula 54]

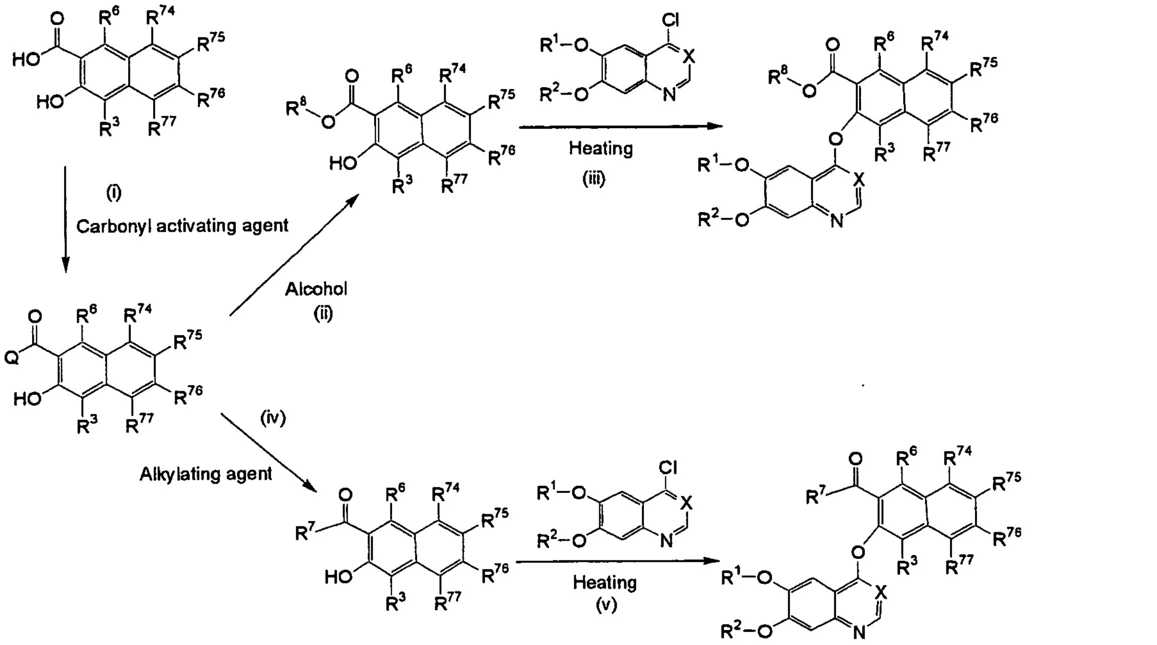


wherein $R^{74}$ to $R^{77}$ represent a hydrogen atom, a halogen atom, hydroxyl, or $C_{1-4}$ alkyl, Q represents, for example, chlorine or N,O-dimethylhydroxyamine, and the other substituents are as defined above.

In this scheme, the aimed compounds can be synthesized through the following two routes.

(I) The comtemplated compound can be produced by reacting a hydroxynaphthalenecarboxylic acid derivative with a suitable carbonyl activating agent, for example, thionyl chloride (step (i)), then reacting the active carboxylic acid derivative thus obtained with an alcohol (step (ii)) to give an ester derivative, then reacting the ester derivative with a 4-chloroquinoline derivative or a corresponding quinazolone derivative in a suitable solvent, for example, o-dichlorobenzene, or in the absence of a solvent, for example, at 120 to 180°C (step (iii)).

(II) The aimed compounds can be produced by reacting the active carboxylic acid derivative produced in step (i) with an alkylating agent, for example, methylmagnesium bromide (step (iv)) to give a ketone derivative and then reacting the ketone derivative with a 4- chloroquinoline derivative or a corresponding quinazolone derivative in a suitable solvent, for example, o-dichlorobenzene, or in the absence of a solvent, for example, at 120 to 180°C (step (v)).

Scheme r17:

[Chemical formula 55]

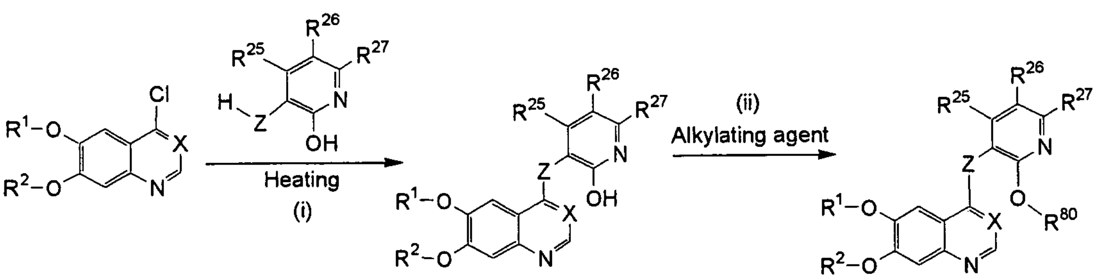


wherein $R^{80}$ represents cyclopentyl, cyclohexyl, or phenyl, and the other substituents are as defined above.

The aimed compounds can be produced by reacting a 2-hydroxypyridine derivative with a 4-chloroquinoline derivative or a corresponding quinazolone derivative in a suitable solvent, for example, o-dichlorobenzene, or in the absence of a solvent, for example, at 120 to 180°C (step (i)) and then alkylating the resultant compound with an alkylating agent, for example, ethyl iodide (step (ii)).

Scheme r18:

[Chemical formula 56]

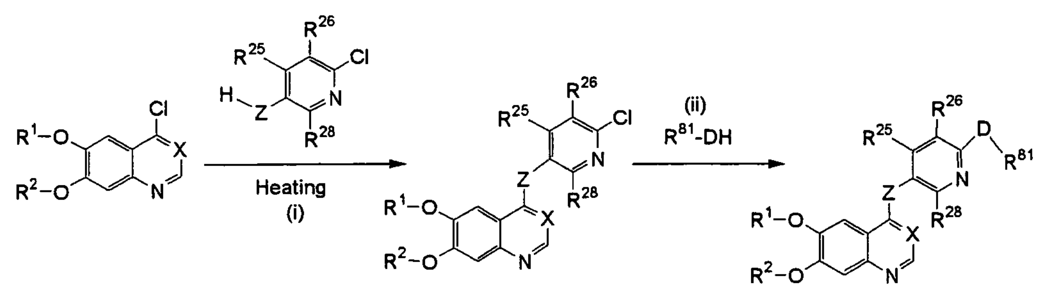


wherein $R^{81}$ represents a hydrogen atom or $C_{1-4}$ alkyl, D represents an oxygen atom, a nitrogen atom, or a sulfur atom, and the other substituents are as defined above.

The aimed compounds can be produced by reacting a 2-chloropyridine derivative with a 4-chloroquinoline derivative or a corresponding quinazolone derivative in a suitable solvent, for example, o-dichlorobenzene, or in the absence of a solvent, for example, at 120 to 180°C (step (i)) and reacting the resultant compound with a nucleophilic reagent, for example, methanol (step (ii)).

Scheme r19:

[Chemical formula 57]

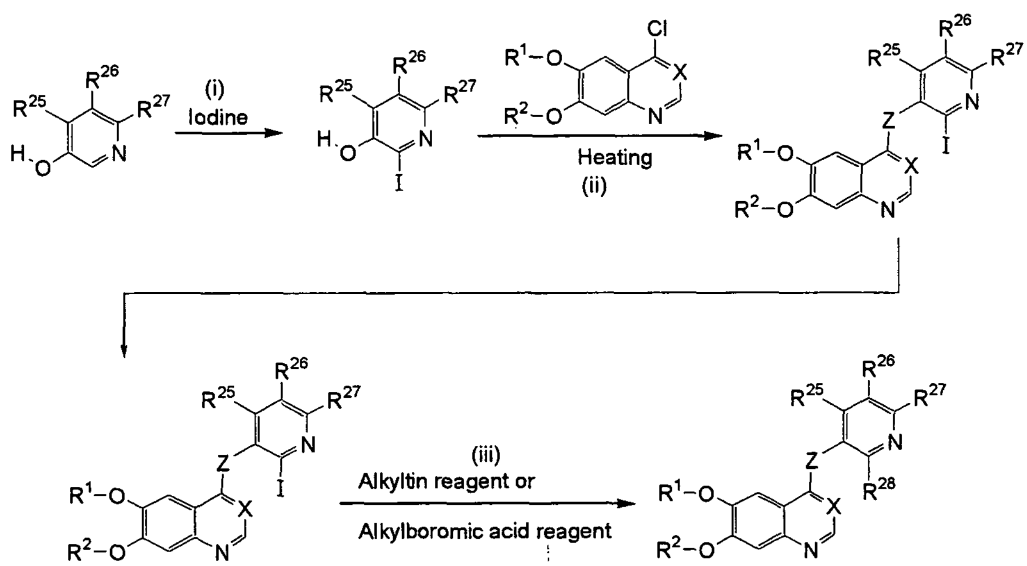


wherein the substituents are as defined above.

The aimed compounds can be produced by reacting a 3-hydroxypyridine derivative with iodine in a suitable solvent, for example, methanol (step (i)), reacting the 2-iodopyridine derivative thus obtained with a 4-chloroquinoline derivative or a corresponding quinazolone derivative in a suitable solvent, for example, o-dichlorobenzene, or in the absence of a solvent, for example, at 120 to 180˚C (step (ii)), then reacting the resultant compound with an alkyltin reagent, for example, tri-n-butyl-(2-pyridyl)-tin, or an alkylboronic acid reagent, for example, 3-pyridylboronic acid, in the presence of a suitable transition metal catalyst, for example, tetrakistriphenylphosphine palladium (step (iii)).

Scheme r20:

[Chemical formula 58]

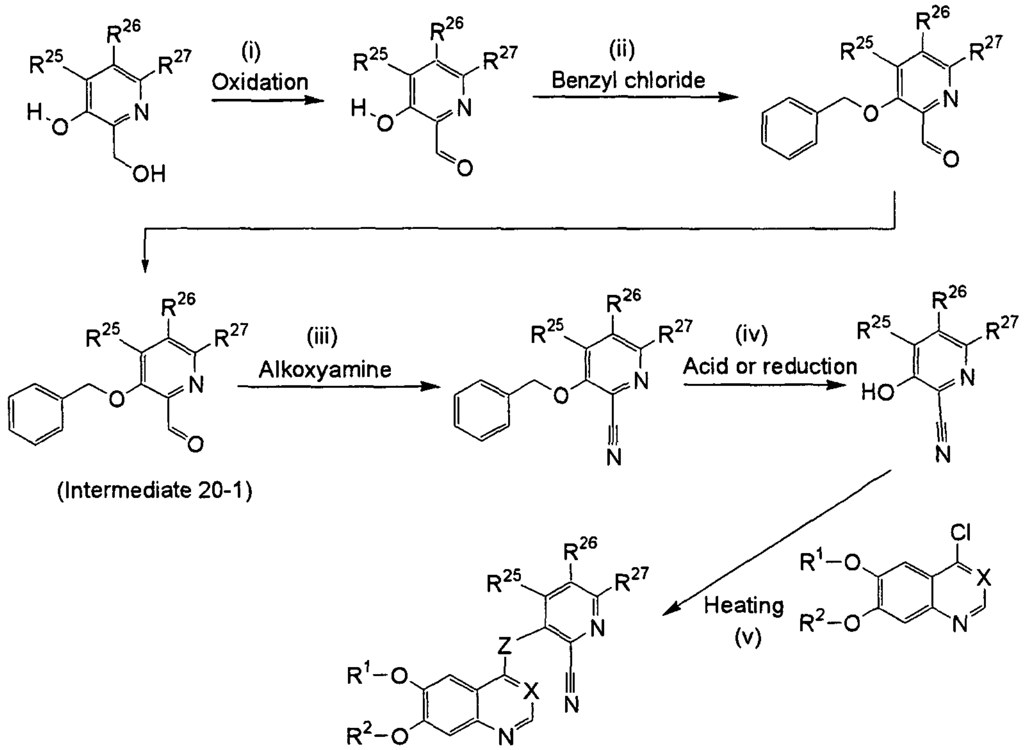


wherein the substituents are as defined above.

Intermediate 20-1 can be produced by reacting the above starting compound with a suitable oxidizing agent, for example, manganese dioxide (step (i)) and then reacting the remaining hydroxy group in the resultant compound with a suitable benzylating agent, for example, benzyl chloride (step (ii)). Next, a corresponding cyanopyridine derivative can be produced by reacting intermediate 20-1 with an alkoxyamine (step (iii)). The aimed compounds can be produced by deprotecting the cyanopyridine derivative thus obtained with a suitable acid, for example, methanesulfonic acid/ trifluoroacetic acid, or a suitable reducing agent, for example, a hydrogen gas/palladium hydroxide (step (iv)), and then reacting the resultant hydroxypyridine derivative with a 4-chloroquinoline derivative or a corresponding quinazolone derivative in a suitable solvent, for example, o-dichlorobenzene, or in the absence of a solvent, for example, at 120 to 180˚C (step (v)).

Scheme r21:

[Chemical formula 59]

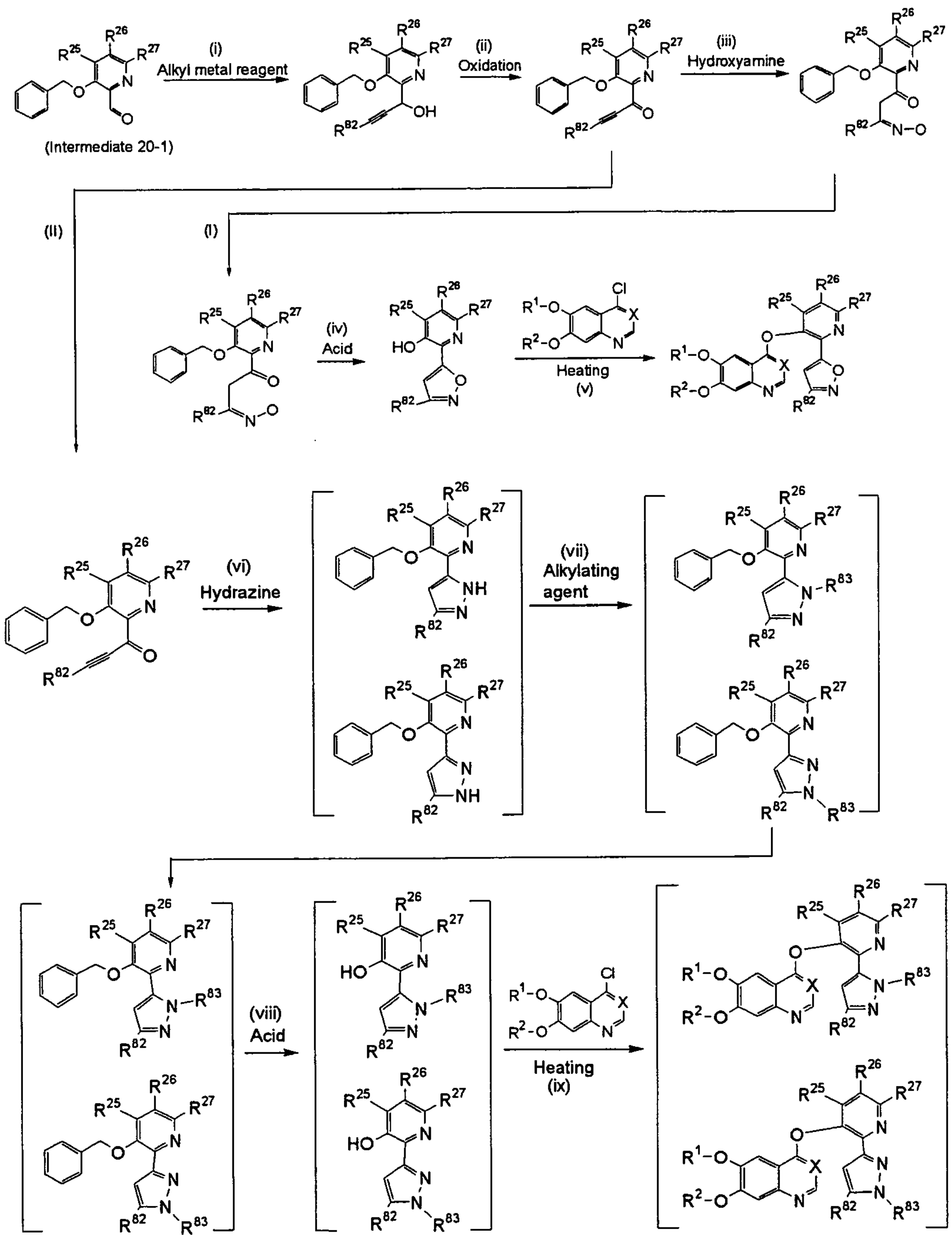


wherein $R^{82}$ and $R^{83}$ represent hydroxyl; cyano; a halogen atom; C1-4 alkoxy; phenyloxy; C1-4 alkylcarbonyl; amino optionally substituted by C1-4 alkyl or C1-4 alkylcarbonyl; aminocarbonyl optionally substituted by C1-4 alkyl; or C1-4 alkyl optionally substituted by hydroxyl or a halogen atom, and
the other substituents are as defined above.

In this scheme, the aimed compounds can be synthesized through the following two routes.

(I) The aimed compounds can be produced by reacting intermediate 20-1 produced according to scheme r20 with an alkyne metal reagent, for example, 1-propynylmagnesium bromide (step (i)), then oxidizing the hydroxyl group in the resultant compound with a suitable oxidizing agent, for example, a benzyl group (step (ii)) to give a ketone

derivative, reacting the ketone derivative with hydroxyamine (step (iii)), reacting the resultant compound with a suitable acid, for example, methanesulfonic acid/trifluoroacetic acid (step (iv)) to give a 3-hydroxy-2-isoxazoylpyridine derivative, and then reacting the 3-hydroxy-2-isoxazoylpyridine derivative thus obtained with a 4-chloroquinoline derivative or a corresponding quinazolone derivative in a suitable solvent, for example, o-dichlorobenzene, or in the absence of a solvent, for example, at 120 to 180˚C (step (v)).

(II) The aimed compounds can be produced by reacting intermediate 20-1 produced according to scheme r20 with an alkyne metal reagent, for example, 1-propynylmagnesium bromide (step (i)), then oxidizing the hydroxyl group in the resultant compound with a suitable oxidizing agent, for example, a benzyl group (step (ii)) to give a ketone derivative, reacting the ketone derivative with hydrazine (step (vi)), reacting the resultant compound with an alkylating agent, for example, methyl iodide (step (vii)) to give an N-alkylpyrazole derivative, reacting the N-alkylpyrazole derivative with a suitable acid, for example, methanesulfonic acid/trifluoroacetic acid (step (viii)) to give a 3-hydroxypyridine derivative, and then reacting the 3-hydroxypyridine derivative with a 4-chloroquinoline derivative or a corresponding quinazolone derivative in a suitable solvent, for example, o-dichlorobenzene, or in the absence of a solvent, for example, at 120 to 180˚C (step (ix)).

Scheme r22:

[Chemical formula 60]

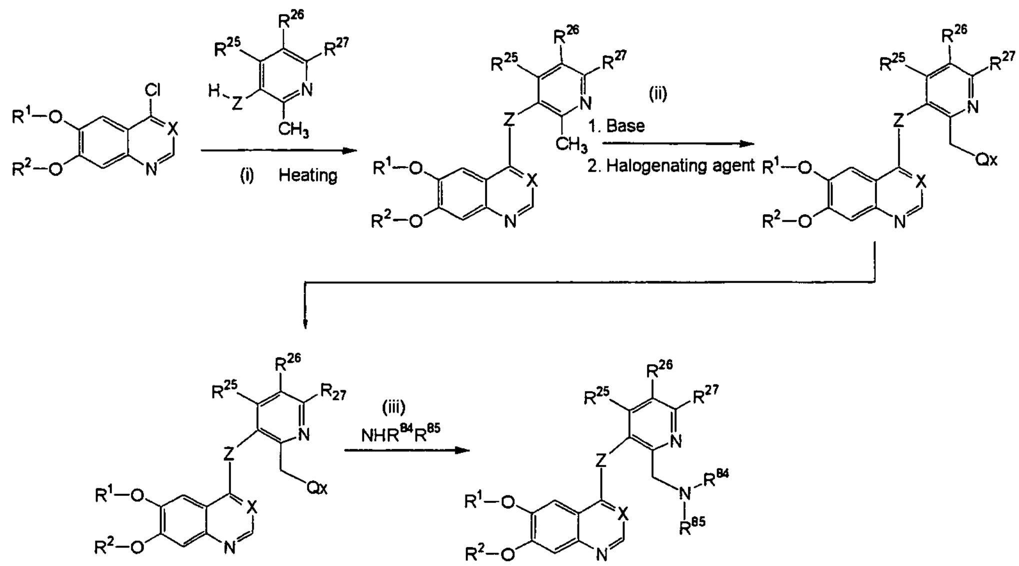


wherein Qx represents a halogen atom, preferably a chlorine atom or a bromine atom, and the other substituents are as defined above.

The aimed compounds can be produced by reacting a 3-hydroxy-2-methylpyridine derivative with a 4-chloroquinoline derivative or a corresponding quinazolone derivative in a suitable solvent, for example, o-dichlorobenzene, or in the absence of a solvent, for example, at 120 to 180˚C (step (i)) to give a quinoline derivative, allowing a suitable base, for example, lithium diisopropylamide, to act on the resultant compound to produce carbanion, reacting the carbanion with a halogenating agent, for example, N-bromosucciimide (step (ii)), and then reacting the resultant compound with an amine (step (iii)).

Scheme r23:

[Chemical formula 61]

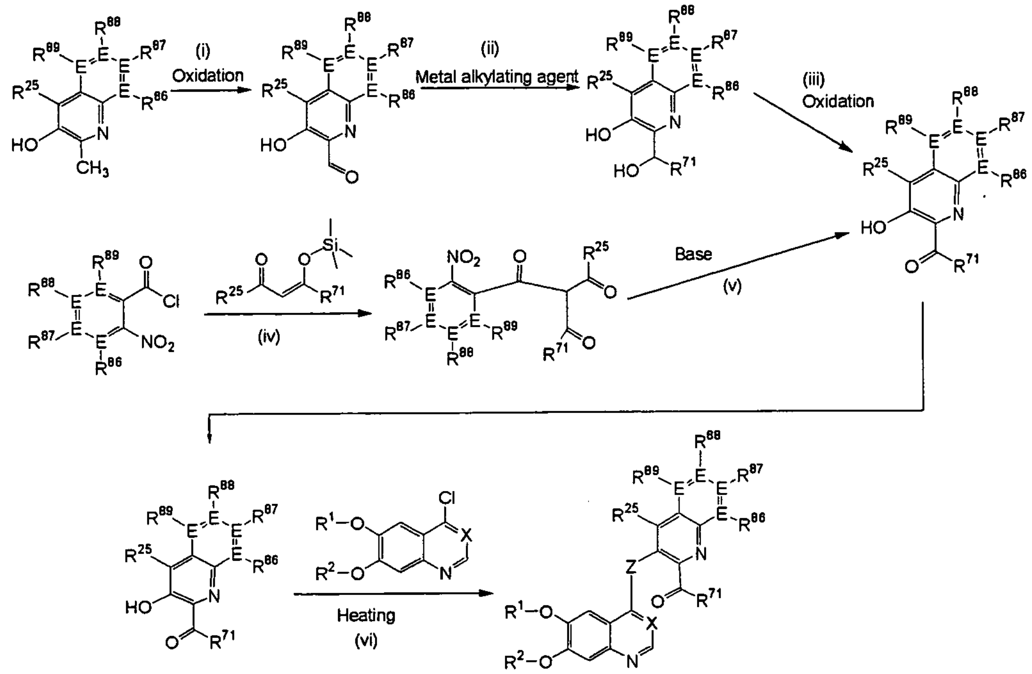


wherein at least one of E's represents a heteroatom such as a nitrogen atom while the other E's represent a carbon atom, or all of E's represent a carbon atom,
$R^{86}$ to $R^{89}$ represent a hydrogen atom, a halogen atom, or $C_{1-4}$ alkyl, and
the other substituents are as defined above.

In this scheme, the aimed compounds can be synthesized through the following two routes.

(I) The aimed compounds can be produced by reacting a 3-hydroxy-2-methylquinoline derivative with a suitable oxidizing agent, for example, selenium dioxide (step (i)), reacting the aldehyde derivative with a metal alkylating agent, for example, methylmagnesium bromide (step (ii)), then oxidizing the resultant alcoholic compound with a suitable oxidizing agent, for example, manganese dioxide (step (iii)) to give a ketone derivative, and reacting the ketone derivative with a 4-chloroquinoline derivative or a corresponding quinazolone derivative in a suitable solvent, for example, o-dichlorobenzene, or in the absence of a solvent, for example, at 120 to 180°C (step (vi)).

(II) The aimed compounds can be produced by reacting an acid chloride derivative with silyl enol ether, for example, 4-trimethylsilanyloxy-penta-3-en-2-one) (step (iv)), reacting the ketone derivative thus obtained with a suitable base, for example, an aqueous potassium hydroxide solution (step (v)) to give a 3-hydroxyquinoline derivative, and reacting the resultant 3-hydroxyquinoline derivative with a 4-chloroquinoline derivative or a corresponding quinazolone derivative in a suitable solvent, for example, o-dichlorobenzene, or in the absence of a solvent, for example, at 120 to 180°C (step (vi)).

Scheme r24:

[Chemical formula 62]

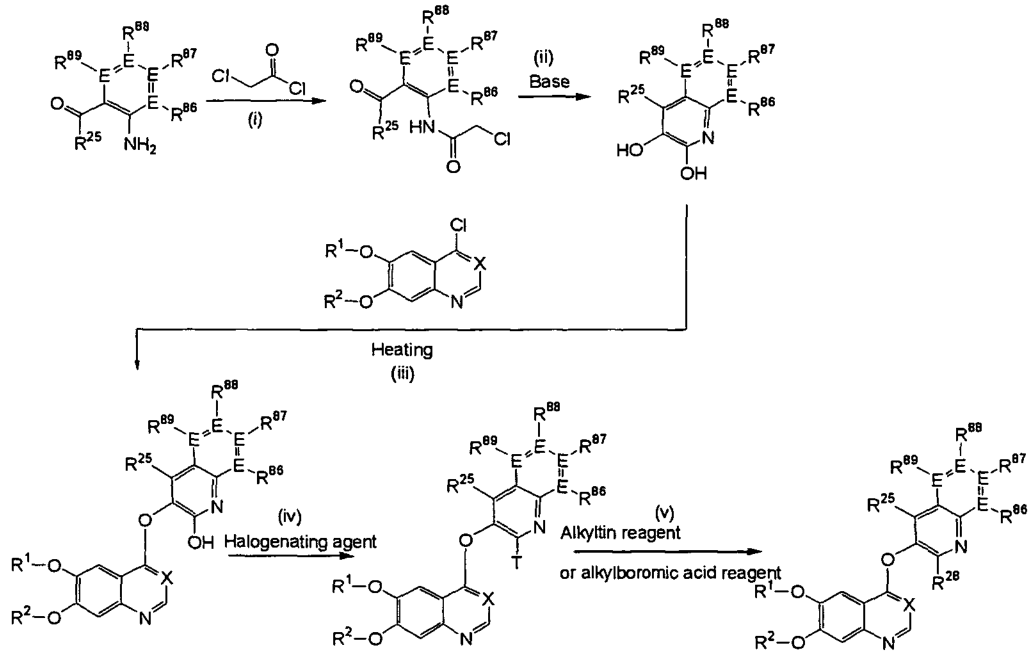


wherein at least one of E's represents a heteroatom such as a nitrogen atom while the other E's represent a carbon atom, or all of E's represent a carbon atom,
T represents a halogen atom, preferably a bromine atom, and
the other substituents are as defined above.

The aimed compounds can be produced by reacting an aniline derivative with chloroacetyl chloride (step (i)), then reacting the resultant amide derivative with a suitable base, for example, an aqueous potassium hydroxide solution (step (ii)) to give a 2,3-dihydroxyquinoline derivative, reacting the 2,3-dihydroxyquinoline derivative thus obtained with a 4-chloroquinoline derivative or a corresponding quinazolone derivative in a suitable solvent, for example, o-dichlorobenzene, or in the absence of a solvent, for example, at 120 to 180°C (step (iii)) to give a 2-hydroxyquinoline derivative, reacting the 2-hydroxyquinoline derivative with a suitable halogenating agent, for example, tetrabutylammonium bromide (step (iv)) to give a 2-haloquinoline derivative, and reacting the 2-haloquinoline derivative with an alkyltin reagent , for example, tri-n-butyl-(2-pyridyl)-tin, or an alkylboronic acid reagent, for example, 3-pyridylboronic acid in the presence of a suitable transition metal catalyst, for example, tetrakistriphenylphosphine palladium (step (v)).

<u>Scheme r25:</u>

[Chemical formula 63]

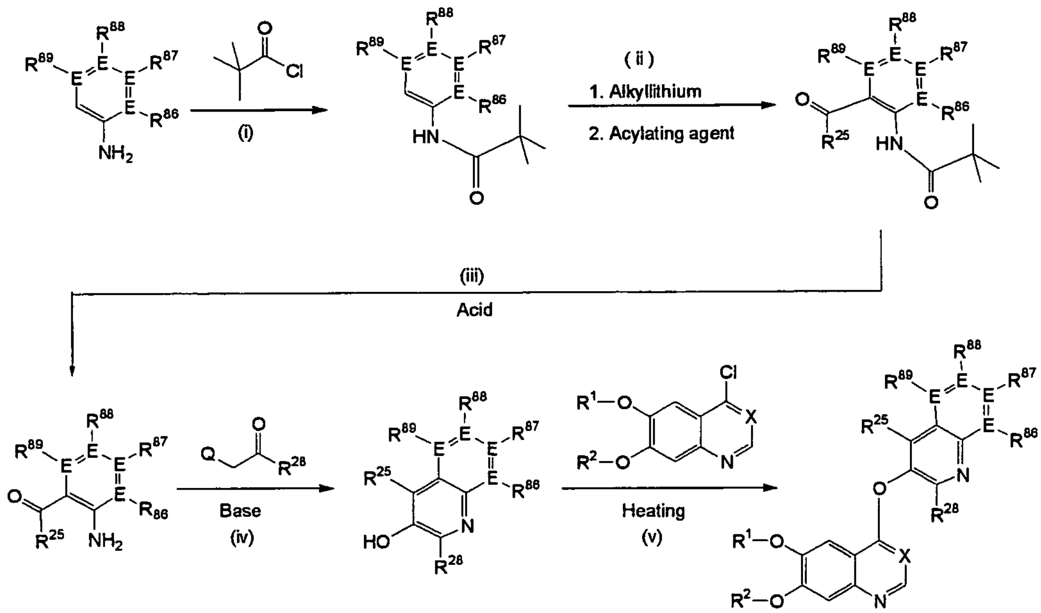


wherein at least one of E's represents a heteroatom such as a nitrogen atom while the other E's represent a carbon atom, or all of E's represent a carbon atom, and the other substituents in the scheme are as defined above.

The aimed compounds can be produced by reacting an aniline derivative with pivaloyl chloride (step (i)) to give an amide derivative, then reacting the amide derivative thus obtained with a suitable alkyllithium, for example, n-butyllithium, reacting the resultant anion with an acylating agent, for example, N,N-dimethylformamide (step (ii)), removing the pivaloyl group with a suitable acid, for example, hydrochloric acid (step (iii)) to give an o-acylaniline derivative, reacting the resultant o-acylaniline derivative with a methyl ketone derivative (step (iv)), and then reacting the resultant 3-hydroxyquinoline derivative with a 4-chloroquinoline derivative or a corresponding quinazolone derivative in a suitable solvent, for example, o-dichlorobenzene, or in the absence of a solvent, for example, at 120 to 180°C (step (v)).

Scheme r26:

[Chemical formula 64]

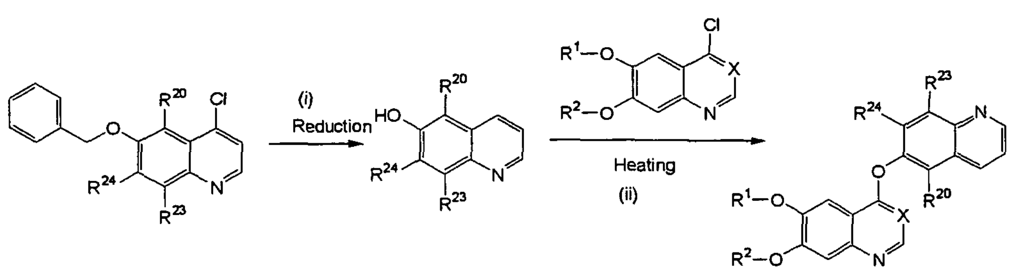


wherein the substituents are as defined above.

The aimed compounds can be produced by reacting a 6-benzyloxy-4-chloroquinoline derivative with a suitable reducing agent, for example, a hydrogen gas/palladium hydroxide) (step (i)), reacting the 6-hydroxyquinoline derivative with a 4-chloroquinoline derivative or a corresponding quinazolone derivative in a suitable solvent, for example, o-dichlorobenzene, or in the absence of a solvent, for example, at 120 to 180°C (step (ii)).

Scheme r27:

[Chemical formula 65]

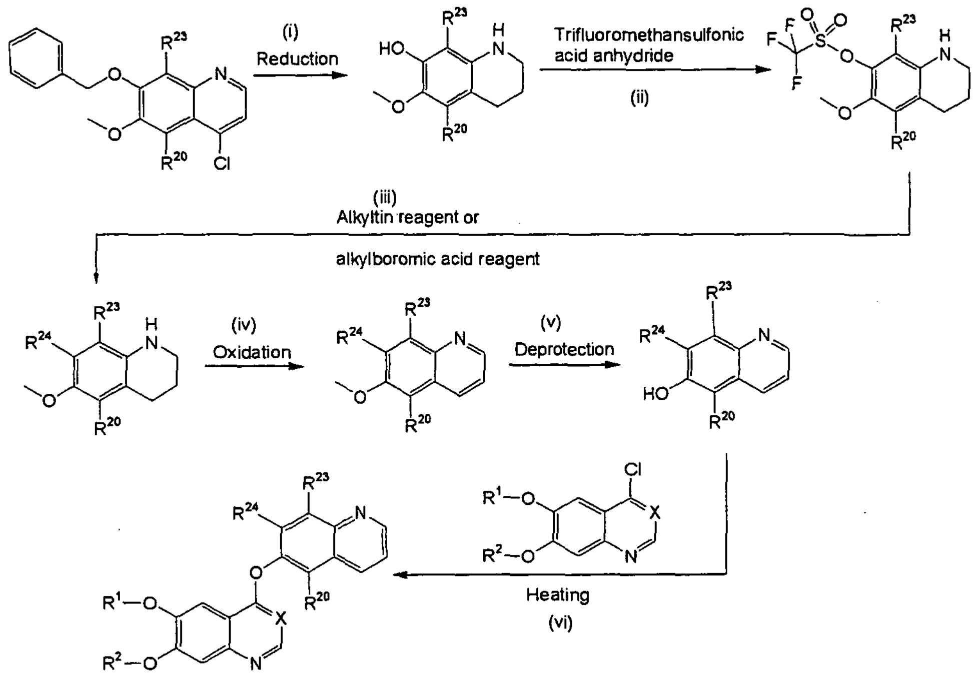


wherein the substituents are as defined above.

The aimed compounds are produced by reacting a 7-benzyloxy-4-chloroquinoline derivative with a suitable reducing agent, for example, a hydrogen gas/palladium hydroxide (step (i)), then reacting the resultant alcohol with trifluoromethanesulfonic acid anhydride (step (ii)) to give a trifluoromethanesulfonate derivative, reacting the trifluoromethanesulfonate derivative with an alkyltin reagent, for example, tri-n-butyl-(2-pyridyl)-tin, or an alkylboronic acid reagent, for example, 3-pyridylboronic acid in the presence of a suitable transition metal catalyst, for example, tetrakistriphenylphosphine palladium (step (iii)), and reacting the resultant compound with a suitable oxidizing agent, for example, 2,3-dichloro-5,6-dicyano-1,4-benzoquinone (step (iv)) to give a quinoline derivative, then reacting the quinoline derivative thus obtained with a suitable reagent, for example, boron tribromide (step (v)) to give 6-hydroxyquinoline derivative, reacting the 6-hydroxyquinoline derivative with a 4-chloroquinoline derivative or a corresponding quinazolone derivative in a suitable solvent, for example, o-dichlorobenzene, or in the absence of a solvent, for example, at 120 to 180˚C (step (vi)).

Scheme r28:

[Chemical formula 66]

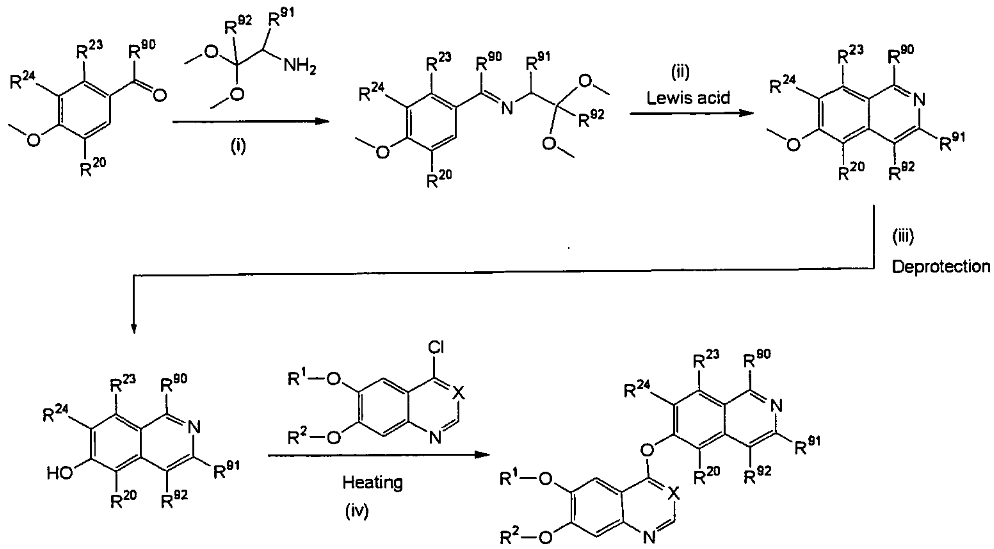


wherein $R^{90}$ to $R^{92}$, which may be the same or different, represent a hydrogen atom, a halogen atom, C1-2 alkylcarbonyl, C1-2 alkoxycarbonyl, or C1-4 alkyl, and the other substituents are as defined above.

The aimed compounds can be produced by reacting a 4-methoxybenzaldehyde derivative with a dimethoxyalkylamine derivative (step (i)), reacting the resultant imine derivative with a suitable Lewis acid, for example, titanium tetrachloride (step (ii)) to give a 6-methoxyisoquinoline derivative, reacting the 6-methoxyisoquinoline derivative with a suitable reagent, for example, boron tribromide (step (iii)) to give a 6-hydroxyisoquinoline derivative, then reacting the 6-hydroxyquinoline derivative with a 4-chloroquinoline derivative or a corresponding quinazolone derivative in a suitable solvent, for example, o-dichlorobenzene, or in the absence of a solvent, for example, at 120 to 180˚C (step (iv)).

Scheme r29:

[Chemical formula 67]

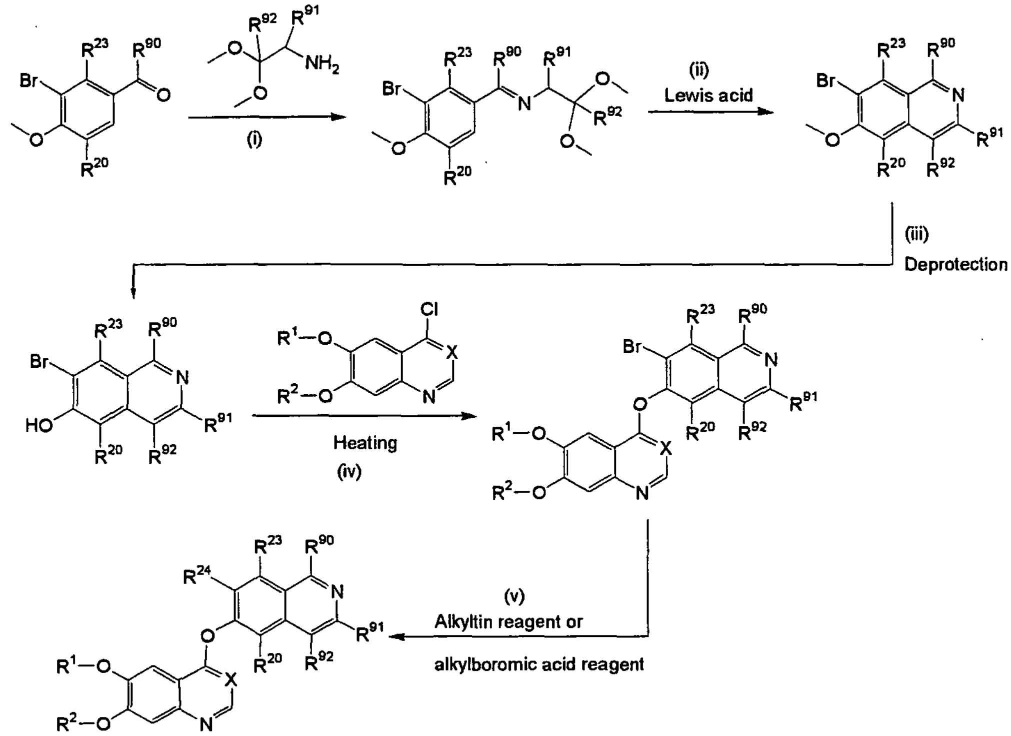


wherein the substituents are as defined above.

The aimed compounds can be produced by reacting a 3-bromo-4-methoxybenzaldehyde derivative with a dimethoxyalkylamine derivative (step (i)), then reacting the resultant imine derivative with a suitable Lewis acid, for example, titanium tetrachloride (step (ii)) to give a 6-methoxyisoquinoline derivative, reacting the 6-methoxyisoquinoline derivative with a suitable reagent, for example, boron tribromide (step (iii)) to give a 6-hydroxyisoquinoline derivative, then reacting the 6-hydroxyquinoline derivative with a 4-chloroquinoline derivative or a corresponding quinazolone derivative in a suitable solvent, for example, o-dichlorobenzene, or in the absence of a solvent, for example, at 120 to 180°C (step (iv)) and reacting the resultant compound with an alkyltin reagent, for example, tri-n-butyl-(2-pyridyl)-tin, or an alkylboronic acid reagent, for example, 3-pyridylboronic acid, in the presence of a suitable transition metal catalyst, for example, tetrakistriphenylphosphine palladium) (step (v)).

<u>Scheme r30:</u>

[Chemical formula 68]

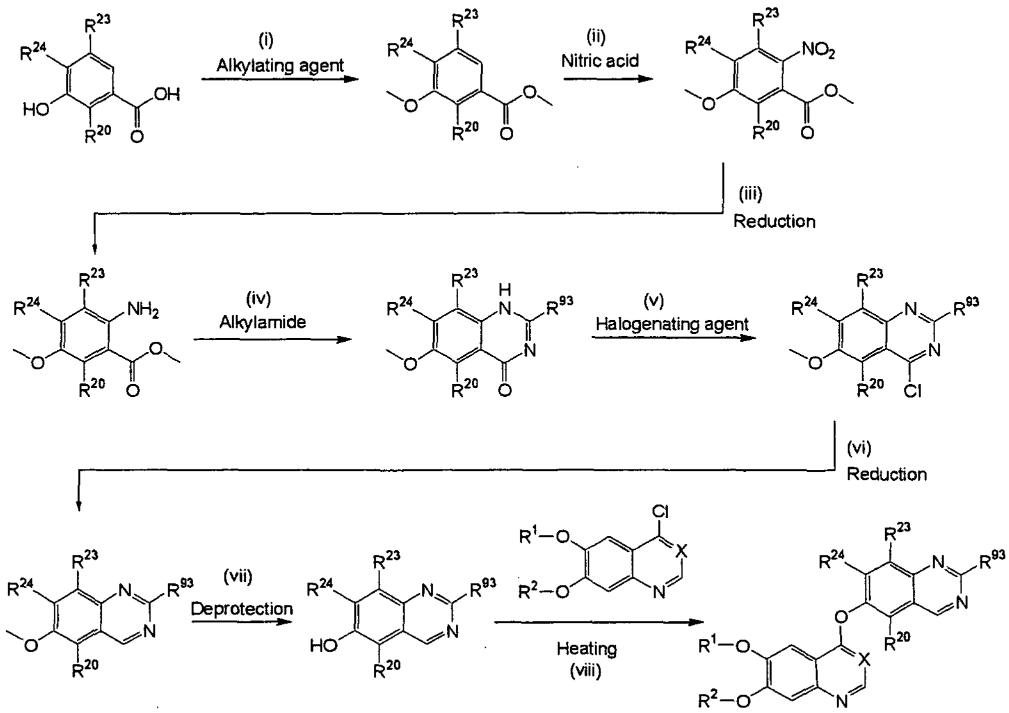


wherein $R^{93}$ represents a hydrogen atom, a halogen atom, C1-2 alkylcarbonyl, C1-2 alkoxycarbonyl, or C1-4 alkyl, and the other substituents are as defined above.

The aimed compounds can be produced by reacting a 3-hydroxybenzoic acid derivative with an alkylating agent, for example, methyl iodide (step (i)) to give an alkyl 3-alkoxybenzoate derivative, reacting the alkyl 3-alkoxybenzoate derivative with nitric acid in the presence of a suitable acid, for example, acetic acid (step (ii)), then reducing the resultant nitro group with a suitable reducing agent, for example, a hydrogen gas/palladium hydroxide (step (iii)) to give an aniline derivative, reacting the amino group in the aniline derivative with an alkyl amide (step (iv)), then reacting the resultant compound with a suitable halogenating agent, for example, phosphorus oxychloride (step (v)) to give a 4-chloroquinazoline derivative, then reducing the 4-chloroquinazoline derivative with a suitable reducing agent, for example, a hydrogen gas/palladium hydroxide (step (vi)), then reacting the resultant compound with a suitable reagent, for example, boron tribromide (step (vii)) to give a 6-hydroxyquinazoline derivative, and reacting the 6-hydroxyquinazoline derivative with a 4-chloroquinoline derivative or a corresponding quinazolone derivative in a suitable solvent, for example, o-dichlorobenzene, or in the absence of a solvent, for example, at 120 to 180°C (step (viii)).

Scheme r31:

[Chemical formula 69]

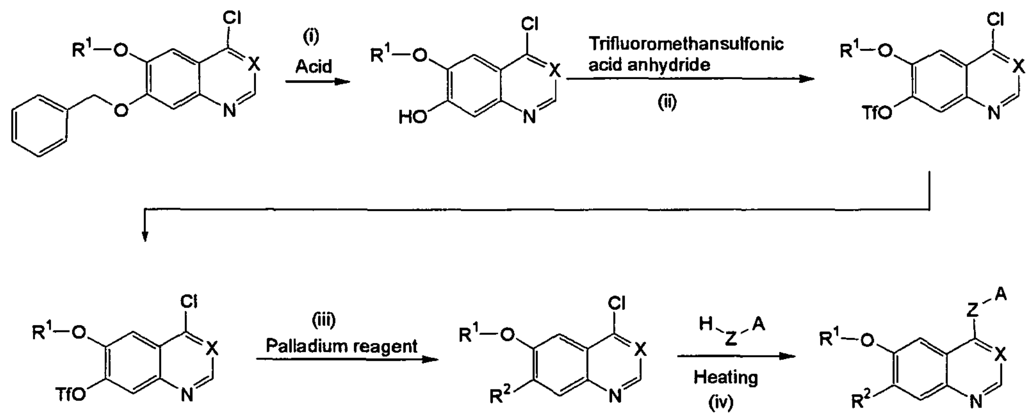


wherein the substituents are as defined above.

The aimed compounds can be produced by reacting a 7-benzyloxy-4-chloroquinoline derivative or a corresponding quinazolone derivative with a suitable acid, for example, methanesulfonic acid/trifluoroacetic acid (step (i)), then reacting the resultant alcohol with trifluoromethanesulfonic acid anhydride (step (ii)) to give a trifluoromethanesulfonate derivative, reacting the trifluoromethanesulfonate derivative with an amine or an alkene in the presence of a suitable transition metal catalyst, for example, tetrakistriphenylphosphine palladium (step (iii)), and reacting the resultant compound with a phenol derivative or a corresponding aniline derivative in a suitable solvent, for example, o-dichlorobenzene, or in the absence of a solvent, for example, at 120 to 180°C (step (iv)).

Scheme r32:

[Chemical formula 70]

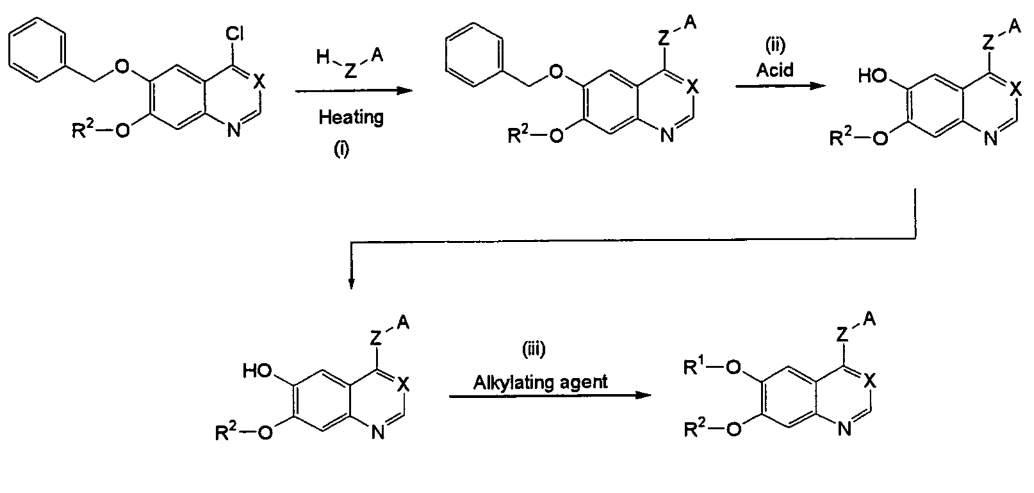


wherein the substituents are as defined above.

The aimed compounds can be produced by reacting a 6-benzyloxy-4-chloroquinoline derivative or a corresponding quinazolone derivative with a phenol derivative or a corresponding aniline derivative in a suitable solvent, for example, o-dichlorobenzene, or in the absence of a solvent, for example, at 120 to 180°C (step (i)), then reacting the resultant compound with a suitable acid, for example, methanesulfonic acid/trifluoroacetic acid (step (ii)) to give 6-hydroxyquinoline

derivative, and reacting the 6-hydroxyquinoline derivative with an alkylating agent, for example, 1-bromo-2-chloroethane (step (iii)).

Scheme r33:

[Chemical formula 71]

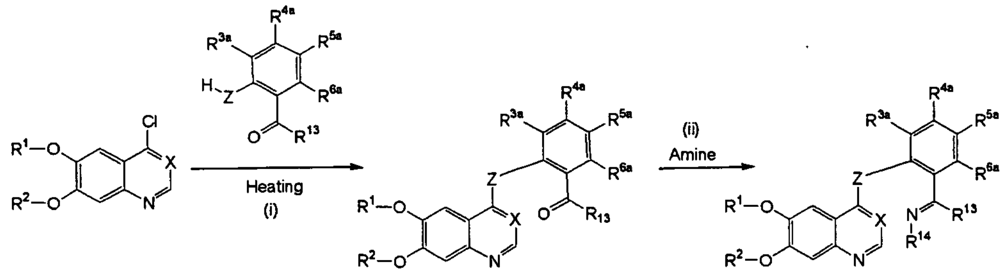


wherein $R^{3a}$ to $R^{6a}$, which may be the same or different, represent a hydrogen atom, a halogen atom, or C1-6 alkyl, and the other substituents are as defined above.

The aimed compounds can be produced by reacting a 4-chloroquinoline derivative or a corresponding quinazolone derivative with a phenol derivative or a corresponding aniline derivative in a suitable solvent, for example, o-dichlorobenzene, or in the absence of a solvent, for example, at 120 to 180°C (step (i)) and then reacting the resultant compound with an amine derivative (step (ii)).

Scheme r34:

[Chemical formula 72]

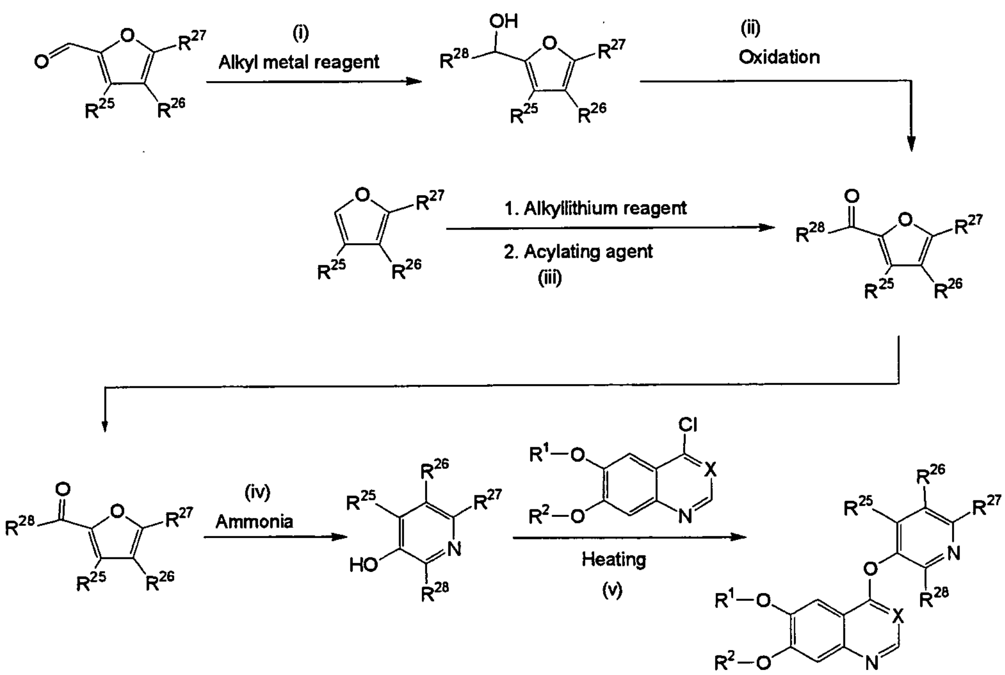

wherein the substituents are as defined above.

In this scheme, the aimed compounds can be synthesized through the following two routes.

(I) The aimed compounds can be produced by reacting a furfural derivative with an alkyl metal reagent, for example, phenylmagnesium bromide (step (i)) to give an alcohol derivative, oxidizing the alcohol derivative with a suitable oxidizing agent, for example, manganese dioxide (step (ii)) to give a ketone derivative, then reacting the ketone derivative with ammonia (step (iv)) to give a 3-hydroxypyridine derivative, reacting the 3-hydroxypyridine derivative with a 4-chloroquinoline derivative or a corresponding quinazolone derivative in a suitable solvent, for example, o-dichlorobenzene, or in the absence of a solvent, for example, at 120 to 180˚C (step (v)).

(II) The aimed compounds can be produced by reacting a furan derivative with an alkyllithium reagent, for example, n-butyllithium, then reacting the resultant compound with an acylating agent, for example, benzoyl chloride (step (iii)) to give a ketone derivative, reacting the ketone derivative with ammonia (step (iv)) to give a 3-hydroxypyridine derivative, then reacting the 3-hydroxypyridine derivative with a 4-chloroquinoline derivative or a corresponding quinazolone derivative in a suitable solvent, for example, o-dichlorobenzene, or in the absence of a solvent, for example, at 120 to 180˚C (step (v)).

Compound r1 to r469

Compounds r1 to r469 as Reference Examples can be produced according to schemes r1 to r34 as described above. These compounds can also be produced with reference to WO 2004/018430. For typical examples of these compounds, the names of the compounds and the measured data on the actually produced compounds will be summarized below.

Compound r1: 4-(2-Benzylphenoxy)-6,7-dimethoxyquinoline

$^1$H-NMR ($CDCl_3$, 400 MHz): δ 3.90 (s, 2H), 3.96 (s, 3H), 4.05 (s, 3H), 6.31 (d, J = 5.4 Hz, 1H), 7.07 - 7.37 (m, 9H), 7.40 (s, 1H), 7.44 (s, 1H), 8.42 (d, J = 5.1 Hz, 1H)
Mass spectrometric value (ESI-MS, m/z): 372 $(M+1)^+$

Compound r6: 6,7-Dimethoxy-4-[4-methyl-2-(piperidinomethyl)phenoxy]quinoline

$^1$H-NMR ($CDCl_3$, 400 MHz): δ 1.25-1.49 (m, 6H), 2.30-2.38 (m, 4H), 2.40 (s, 3H), 3.39 (s, 2H), 4.06 (s, 6H), 6.30 (d, J = 5.4 Hz, 1H), 7.01 (d, J = 8.1 Hz, 1H), 7.14 (dd, J = 1.7 Hz, 8.0 Hz, 1H), 7.39 (d, J = 1.4 Hz, 1H), 7.44 (s, 1H), 7.61 (s, 1H), 8.44 (d, J = 5.4 Hz, 1 H)
Mass spectrometric value (ESI-MS, m/z): 393 $(M+1)^+$

Compound r7: 6,7-Dimethoxy-4-{4-methoxy-2-[(E)-2-phenyl-1-ethenyl]phenoxy}quinoline

$^1$H-NMR ($CDCl_3$, 400 MHz): δ 3.85 (s, 3H), 4.00 (s, 3H), 4.01 (s, 3H), 6.29 (d, J = 5.4 Hz, 1H), 6.84 (dd, J = 3.2 Hz, 9.0 Hz, 1H), 7.01 (d, J = 8.8 Hz, 1H), 7.06 (d, J = 7.4 Hz, 1H), 7.11 - 7.28 (m, 7H), 7.43 (s, 1H), 7.61 (s, 1H), 8.38 (d, J = 5.4 Hz, 1H)
Mass spectrometric value (ESI-MS, m/z): 414 $(M+1)^+$

Compound r9: 1-{2-[(6,7-Dimethoxy-4-quinolyl)oxy]-5-methylphenyl}-1-propanol

$^1$H-NMR ($CDCl_3$, 400 MHz): δ 0.92 (t, J = 7.3 Hz, 3H), 1.80 (m, 2H), 2.42 (s, 3H), 3.99 (s, 3H), 4.02 (s, 3H), 4.75 (t, J = 6.3 Hz, 1H), 6.24 (d, J = 5.4 Hz, 1H), 6.95 (d, J = 8.3 Hz, 1H), 7.14 (dd, J = 2.2 Hz, 8.0 Hz, 1H), 7.32 (s, 1H), 7.53 (s, 1H), 7.54 (d, J = 1.9 Hz, 1H), 8.13 (d, J = 5.4 Hz, 1H)
Mass spectrometric value (ESI-MS, m/z): 354 $(M+1)^+$

Compound r14: N-[2-[(6,7-Dimethoxy-4-quinolyl)oxy]-5-methylphenyl]-N-phenylamine

$^1$H-NMR ($CDCl_3$, 400 MHz): δ 2.35 (s, 3H), 4.03 (s, 3H), 4.04 (s, 3H), 5.77 (s, 1H), 6.51 (d, J = 5.4 Hz, 1H), 6,76 (m, 1H), 6.96 (m, 1H), 7.01 (d, J = 8.1 Hz, 1H), 7.05 (m, 2H), 7.23 - 7.27 (m, 3H), 7.43 (s, 1H), 7.54 (s, 1H), 8.46 (d, J = 5.1 Hz, 1H)

Mass spectrometric value (ESI-MS, m/z): 387 $(M+1)^+$

Compound r20: 6,7-Dimethoxy-4-[(1-methyl-5-indolyl)oxy]quinoline

$^1$H-NMR ($CDCl_3$, 500 MHz): δ 3.86 (s, 3H), 4.06 (s, 3H), 4.08 (s, 3H), 6.39 (d, J = 5.5 Hz, 1H), 6.50 (d, J = 3.1 Hz, 1H), 7.06 (dd, J = 2.4 Hz, 8.6 Hz, 1H), 7.15 (d, J = 3.1 Hz, 1H), 7.39 (d, J = 8.5 Hz, 1H), 7.43 (s, 1H), 7.43 (d, J = 2.4 Hz, 1H), 7.6,7 (s, 1H), 8.43 (d, J = 5.5 Hz, 1H)
Mass spectrometric value (FD-MS, m/z): 334 $(M^+)$

Compound r23: 6-[(6,7-Dimethoxy-4-quinolyl)oxy]-1,4-dihydro-2H-3,1-benzoxazin-2-one

$^1$H-NMR ($CDCl_3$, 500 MHz): δ 4.06 (s, 3H), 4.09 (s, 3H), 5.36 (s, 2H), 6.47 (d, J = 5.1 Hz, 1H), 7.00 (s, 1H), 7.04 (d, J = 8.5 Hz, 1H), 7.14 (d, J = 8.3 Hz, 1H), 7.52 (s, 1H), 7.53 (s, 1H), 8.53 (m, 1H), 9.13 (s, 1H)
Mass spectrometric value (FD-MS, m/z): 352 $(M^+)$

Compound r76: {2-[(6,7-Dimethoxy-4-quinolyl)oxy]-4,5-dimethylphenyl}(phenyl)methanone hydrochloride

$^1$H-NMR ($CDCl_3$, 400 MHz): δ 2.40 (s, 3H), 2.43 (s, 3H), 3.93 (s, 3H), 4.12 (s, 3H), 6.65 (d, J = 6.6 Hz, 1H), 7.11 (s, 1H), 7.13 (s, 1H), 7.36 (dd, J = 7.6 Hz, 7.6 Hz, 2H), 7.49 (s, 1H), 7.51 (dd, J = 7.3 Hz, 7.3 Hz, 1H), 7.66 (d, J = 7.1 Hz, 2H), 8.06 (s, 1H), 8.42 (dd, J = 6.6 Hz, 6.6 Hz, 1H)
Mass spectrometric value (ESI-MS, m/z): 414 $(M-HCl+1)^+$

Compound r79: Methyl 3-[(6,7-Dimethoxy-4-quinolyl)oxy]naphthalene-2-carboxylate

$^1$H-NMR ($CDCl_3$, 400 MHz): δ 3.57 (s, 3H), 4.01 (s, 6H), 6.28 (d, J = 5.4 Hz, 1H), 7.46 - 7.61 (m, 5H), 7.77 (d, J = 8.0 Hz, 1H), 7.93 (d, J = 8.0 Hz, 1H), 8.37 (d, J = 5.4 Hz, 1H), 8.59 (s, 1H)
Mass spectrometric value (ESI-MS, m/z): 390 $(M+1)^+$

Compound r115: 4-[(6-Methyl-3-pyridyl)oxy]-6,7-dimethoxyquinoline

$^1$H-NMR ($CDCl_3$, 400 MHz): δ 2.63 (s, 3H), 4.06 (s, 3H), 4.06 (s, 3H), 6.44 (d, J = 5.4 Hz, 1H), 7.26 (d, J = 8.3 Hz, 1H), 7.42 (dd, J = 2.7 Hz, 8.6 Hz, 1H), 7.44 (s, 1H), 7.54 (s, 1H), 8.46 (d, J = 2.7 Hz, 1H), 8.51 (d, J = 5.1 Hz, 1H)
Mass spectrometric value (ESI-MS, m/z): 297 $(M+1)^+$

Compound r117: 1-{[3-(6, 7-Dimethoxy-4-quinolyl)oxy]-6-methyl-2-pyridyl}-1-ethanone

$^1$H-NMR ($CDCl_3$, 400 MHz): δ 2.63 (s, 3H), 2.67 (s, 3H), 4.05 (s, 3H), 4.05 (s, 3H), 6.28 (d, J = 5.1 Hz, 1H), 7.40 (d, J = 8.6 Hz, 1H), 7.44 (s, 1H), 7.46 (d, J = 8.3 Hz, 1H), 7.57 (s, 1H), 8.47 (d, J = 5.2 Hz, 1H)
Mass spectrometric value (ESI-MS, m/z): 339 $(M+1)^+$

Compound r118: 4-(2-Acetyl-4-methoxyphenoxy)-6-methoxy-7-quinolyl 4-morpholine carboxylate

$^1$H-NMR ($CDCl_3$, 400 MHz): δ 2.42 (s, 3H), 3.29 (m, 4H), 3.60 (m, 4H), 3.82 (s, 3H), 3.95 (s, 3H), 6.32 (d, J = 5.2 Hz, 1H), 7.02 - 7.09 (m, 2H), 7.36 (d, J = 2.8 Hz, 1H), 7.54 (s, 1H), 7.87 (s, 1H), 8.43 (d, J = 5.2 Hz, 1H)
Mass spectrometric value (ESI-MS, m/z): 453 $(M+1)^+$

Compound r163: Ethyl 2-[(6,7-dimethoxy-4-quinolyl)carbonyl]benzoate

$^1$H-NMR ($CDCl_3$, 400 MHz): δ 1.01 (t, J = 7.1 Hz, 3H), 3.98 (q, J = 7.1 Hz, 2H), 4.07 (s, 3H), 4.07 (s, 3H), 7.07 (d, J = 4.6 Hz, 1H), 7.51 (s, 1H), 7.53 (dd, J = 1.4 Hz, 7.3 Hz, 1H), 7.64 (m, 1H), 7.66 (m, 1H), 8.02 (m, 1H), 8.33 (s, 1H), 8.69 (d, J = 4.6 Hz, 1H)
Mass spectrometric value (ESI-MS, m/z): 366 $(M+1)^+$

Compound r164: 4-(2-Iodo-4,5-dimethyl-phenoxy)-6,7-dimethoxy-quinoline

$^1$H-NMR ($CDCl_3$, 400 MHz): δ 2.25 (s, 3H), 2.28 (s, 3H), 4.05 (s, 3H), 4.07 (s, 3H), 6.32 (d, J = 5.4 Hz, 1H), 6.97 (s, 1H), 7.44 (s, 1H), 7.61 (s, 1H), 7.68 (s, 1H), 8.48 (d, J = 5.1 Hz, 1H)
Mass spectrometric value (ESI-MS, m/z): 436 $(M+1)^+$

Compound r175: 1-[5-Benzyloxy-2-(6,7-dimethoxy-quinotin-4-yloxy)-phenyl]-ethanone

$^1$H-NMR ($CDCl_3$, 400 MHz): δ 2.41 (s, 3H), 3.97 (s, 6H), 5.06 (s, 2H), 6.31 (d, J = 5.2 Hz, 1H), 7.02 (d, J = 8.8 Hz, 1H), 7.13 (dd, J = 8.8 Hz, J = 3.2 Hz, 1H), 7.25 - 7.41 (m, 6H), 7.45 (d, J = 3.2 Hz, 1H), 7.49 (s, 1H), 8.41 (d, J = 5.2 Hz, 1H)
Mass spectrometric value (ESI-MS, m/z): 430 $(M+1)^+$

Compound r178: 1-[2-(6,7-Dimethoxy-quinolin-4-yloxy)-5-ethoxy-phenyl]-ethanone

$^1$H-NMR ($CDCl_3$, 400 MHz): δ 1.38 (t, J = 6.8 Hz, 3H), 2.41 (s, 3H), 3.98 (s, 6H), 4.04 (q, J = 6.8 Hz, 2H), 6.30 (d, J = 5.2 Hz, 1H), 7.00 - 7.07 (m, 2H), 7.34 (d, J = 2.8 Hz, 1H), 7.36 (s, 1H), 7.49 (s, 1H), 8.41 (d, J = 5.2 Hz, 1H)
Mass spectrometric value (ESI-MS, m/z): 368 $(M+1)^+$

Compound r193: {1-[2-(6,7-Dimethoxy-quinolin-4-yloxy)-4,5-dimethylphenyl]-ethylidene}-hydrazine

$^1$H-NMR ($CDCl_3$, 400 MHz): δ 1.96 (s, 3H), 2.27 (s, 3H), 2.31 (s, 3H), 4.05 (s, 3H), 4.06 (s, 3H), 5.17 (brs, 2H), 6.38 (d, J = 5.4 Hz, 1H), 6.91 (s, 1H), 7.37 (s, 1H), 7.45 (s, 1H), 7.56 (s, 1H), 8.44 (d, J = 5.4 Hz, 1H)
Mass spectrometric value (ESI-MS, m/z): 388 $(M+Na)^+$

Compound r200: 1-[3-(6,7-Dimethoxy-quinolin-4-yloxy)-naphthalene-2-yl]-ethanone

$^1$H-NMR ($CDCl_3$, 400 MHz): δ 2.60 (s, 3H), 4.05 (s, 3H), 4.06 (s, 3H), 6.46 (d, J = 5.2 Hz, 1H), 7.46 (s, 1H), 7.51 - 7.62 (m, 4H), 7.77 (d, J = 8 Hz, 1H), 7.99 (d, 7.6 Hz, 1H), 8.44 (s, 1H), 8.49 (d, J = 5.6 Hz, 1H)
Mass spectrometric value (ESI-MS, m/z): 374 $(M+1)^+$

Compound r204: 4-(2-Cyclopentyloxy-pyridin-3-yloxy)-6,7-dimethoxy-quinoline

$^1$H-NMR ($CDCl_3$, 400 MHz): δ 1.23 - 1.81 (m, 8H), 4.06 (s, 3H), 4.06 (s, 3H), 5.43 (m, 1H), 6.31 (d, J = 5.1 Hz, 1H), 6.95 (dd, J = 1.1, 7.6 Hz, 1H), 7.43 (s, 1H), 7.49 (m, 1H), 7.58 (s, 1H), 8.10 (m, 1H), 8.46 (d, J = 5.1 Hz, 1H)
Mass spectrometric value (ESI-MS, m/z): 367 $(M+1)^+$

Compound r207: 6,7-Dimethoxy-4-(6-methoxy-pyridin-3-yloxy)-quinoline

$^1$H-NMR ($CDCl_3$, 400 MHz): δ 3.89 (s, 3H), 3.96 (s, 3H), 3.97 (s, 3H), 6.31 (d, J = 5.2 Hz, 1H), 6.76 (d, J = 9.2 Hz, 1H), 7.31 - 7.39 (m, 2H), 7.47 (s, 1H), 8.02 (d, J = 2.8 Hz, 1H), 8.41 (d, J = 5.2 Hz, 1H)
Mass spectrometric value (ESI-MS, m/z): 313 $(M+1)^+$

Compound r211: 4-(6-Fluoro-2-iodo-pyridin-3-yloxy)-6,7-dimethoxy-quinoline

$^1$H-NMR ($CDCl_3$, 400 MHz): δ 3.99 (s, 3H), 4.00 (s, 3H), 6.27 (d, J = 5.6 Hz, 1H), 6.95 (dd, J = 8.4 Hz, 3.6 Hz, 1H), 7.37 - 7.50 (m, 3H), 8.47 (d, J = 5.2 Hz, 1H)
Mass spectrometric value (ESI-MS, m/z): 875 $(2M+Na)^+$

Compound r213: 3-(6,7-Dimethoxy-quinolin-4-yloxy)-6-methyl-pyridin-2-carbonitrile

$^1$H-NMR ($CDCl_3$, 400 MHz): δ 2.65 (s, 3H), 4.05 (s, 3H), 4.06 (s, 3H), 6.54 (d, J = 5.1 Hz, 1H), 7.41 (d, J = 8.8 Hz, 1H), 7.44 (d, J = 8.8 Hz, 1H), 7.46 (s, 1H), 7.46 (s, 1H), 8.59 (d, J = 5.1 Hz, 1H)
Mass spectrometric value (ESI-MS, m/z): 344 $(M+Na)^+$

Compound r223: 6,7-Dimethoxy-4-(6-methyl-2-phenyl-pyridin-3-yloxy)-quinoline

$^1$H-NMR ($CDCl_3$, 400 MHz): δ 2.69 (s, 3H), 4.03 (s, 3H), 4.05 (s, 3H), 6.39 (d, J = 5.4 Hz, 1H), 7.22 - 7.59 (m, 5H), 7.65 - 7.70 (m, 2H), 7.81 - 7.84 (m, 2H), 8.42 (dd, J = 2.0, 5.6 Hz, 1H)
Mass spectrometric value (ESI-MS, m/z): 373 $(M+1)^+$

Compound r256: 4-[2-(1,5-Dimethyl-1H-pyrazol-3-yl)-6-methyl-pyridin-3-yloxy]-6,7-dimethoxy-quinoline

$^1$H-NMR ($CDCl_3$, 400 MHz): δ 2.13 (s, 3H), 2.71 (s, 3H), 3.75 (s, 3H), 4.06 (s, 3H), 4.07 (s, 3H), 6.33 (d, J = 5.4 Hz, 1H), 6.41 (s, 1H), 7.17 (d, J = 8.3 Hz, 1H), 7.39 (d, J = 8.0 Hz, 1H), 7.44 (s, 1H), 7.65 (s, 1H), 8.42 (d, J = 5.1 Hz, 1H)

Mass spectrometric value (ESI-MS, m/z): 413 $(M+Na)^+$

Compound r260: 4-[2-(4,5-Dimethylthiazol-2-yl)-5,6-dimethyl-pyridin-3-yloxy]-6,7-dimethoxy-quinoline

$^1$H-NMR ($CDCl_3$, 400 MHz): δ 2.13 (s, 3H), 2.27 (s, 3H), 2.35 (s, 3H), 2.64 (s, 3H), 4.07 (s, 3H), 4.07 (s, 3H), 6.33 (d, J = 5.1 Hz, 1H), 7.33 (s, 1H), 7.45 (s, 1H), 7.71 (s, 1H), 8.42 (d, J = 5.4 Hz, 1H)
Mass spectrometric value (ESI-MS, m/z): 444 $(M+Na)^+$

Compound r277: [3-(6,7-Dimethoxy-quinolin-4-yloxy)-quinolin-2-ylmethyl]-diisopropyl-amine

$^1$H-NMR ($CDCl_3$, 400 MHz): δ 0.91 - 0.95 (m, 12H), 3.06 (m, 2H), 4.05 (s, 3H), 4.06 (s, 3H), 4.10 (s, 2H), 6.48 (d, J = 5.4 Hz, 1H), 7.46 (s, 1H), 7.54 (m, 1H), 7.61 (s, 1H), 7.70 - 7.73 (m, 2H), 7.73 (s, 1H), 8.17 (d, J = 8.5 Hz, 1H), 8.51 (d, J = 5.4 Hz, 1H)
Mass spectrometric value (ESI-MS, m/z): 446 $(M+1)^+$

Compound r278: 1-[3-(6,7-Dimethoxy-quinolin-4-yloxy)-quinolin-2-yl]-ethanone

$^1$H-NMR ($CDCl_3$, 400 MHz): δ 2.70 (s, 3H), 3.99 (s, 6H), 6.30 (d, J = 5.2 Hz, 1H), 7.42 (s, 1H), 7.53 - 7.66 (m, 2H), 7.76 (m, 2H), 7.90 (s, 1H), 8.16 (d, J = 9.2 Hz, 1H), 8.41 (d, J = 5.2 Hz, 1H)
Mass spectrometric value (ESI-MS, m/z): 375 $(M+1)^+$

Compound r281: 4-(2-Bromo-quinolin-3-yloxy)-6,7-dimethoxy-quinoline

$^1$H-NMR ($CDCl_3$, 400 MHz): δ 4.05 (s, 6H), 6.44 (d, J = 5.2 Hz, 1H), 7.50 (s, 1H), 7.54 - 7.65 (m, 2H), 7.70 - 7.79 (m, 2H), 7.89 (s, 1H), 8.11 (d, J = 8.4 Hz, 1H), 8.51 (d, J = 5.2 Hz, 1H)
Mass spectrometric value (ESI-MS, m/z): 411 $(M+1)^+$

Compound r283: 6,7-Dimethoxy-4-(2-phenyl-quinolin-3-yloxy)-quinoline

$^1$H-NMR ($CDCl_3$, 400 MHz): δ 4.02 (s, 3H), 4.05 (s, 3H), 6.47 (d, J = 5.1 Hz, 1H), 7.32 - 7.36 (m, 3H), 7.41 (s, 1H), 7.50 (s, 1H), 7.59 (m, 1H), 7.76 (m, 1H), 7.80 (d, J = 8.1 Hz, 1H), 7.95 - 7.98 (m, 3H), 8.24 (d, J = 8.6 Hz, 1H), 8.45 (d, J = 5.1 Hz, 1H)
Mass spectrometric value (ESI-MS, m/z): 409 $(M+1)^+$

Compound r284: {3-[3-(6,7-Dimethoxy-quinolin-4-yloxy)-quinolin-2-yl]-phenyl}-methanol

$^1$H-NMR ($CDCl_3$, 400 MHz): δ 3.86 (s, 3H), 3.95 (s, 3H), 4.56 (s, 2H), 6.24 (d, J = 5.2 Hz, 1H), 7.16 - 7.24 (m, 3H), 7.42 (s, 1H), 7.49 (m, 1H), 7.68 (m, 2H), 7.80 (d, J = 5.2 Hz, 1H), 7.84 (s, 1H), 8.01 (s, 1H), 8.12 (m, 2H)
Mass spectrometric value (ESI-MS, m/z): 437 $(M-1)^-$

Compound r297: 6,7-Dimethoxy-4-(7-methoxy-quinolin-6-yloxy)-quinoline

$^1$H-NMR ($CDCl_3$, 400 MHz): δ 3.93 (s, 3H), 4.06 (s, 3H), 4.07 (s, 3H), 6.38 (d, J = 5.1 Hz, 1H), 7.34 (dd, J = 4.4, 8.3 Hz, 1H), 7.45 (s, 1H), 7.56 (s, 1H), 7.63 (s, 1H), 7.63 (s, 1H), 8.05 (d, J = 8.1 Hz, 1H), 8.48 (d, J = 5.1 Hz, 1H), 8.88 (dd, J = 1.4, 4.4 Hz, 1H)
Mass spectrometric value (ESI-MS, m/z): 363 $(M+1)^+$

Compound r299: 6,7-Dimethoxy-4-(7-phenyl-quinolin-6-yloxy)-quinoline

$^1$H-NMR ($CDCl_3$, 400 MHz): δ 4.01 (s, 3H), 4.03 (s, 3H), 6.47 (d, J = 5.4 Hz, 1H), 7.23 - 7.32 (m, 3H), 7.38 (s, 1H), 7.44 - 7.47 (m, 2H), 7.61 - 7.63 (m, 3H), 8.12 (d, J = 7.8 Hz, 1H), 8.30 (s, 1H), 8.43 (d, J = 5.1 Hz, 1H), 8.97 (dd, J = 1.7, 4.2 Hz, 1H)
Mass spectrometric value (ESI-MS, m/z): 409 $(M+1)^+$

Compound r300: 6,7-Dimethoxy-4-(7-methyl-isoquinolin-6-yloxy)-quinoline

$^1$H-NMR ($CDCl_3$, 400 MHz): δ 2.37 (s, 3H), 3.96 (s, 3H), 3.99 (s, 3H), 6.35 (d, J = 5.2 Hz, 1H), 7.36 (s, 1H), 7.39 (s, 1H), 7.47 (m, 2H), 7.86 (s, 1H), 8.41 (d, J = 5.6 Hz, 1H), 8.44 (d, J = 5.2 Hz, 1H), 9.15 (s, 1H)

Mass spectrometric value (ESI-MS, m/z): 347 $(M+1)^+$

Compound r301: 4-(7-Bromo-isoquinolin-6-yloxy)-6,7-dimethoxy-quinoline

$^1$H-NMR ($CDCl_3$, 400 MHz): δ 3.97 (s, 3H), 3.99 (s, 3H), 6.44 (d, J = 5.2 Hz, 1H), 7.40 (s, 1H), 7.49 (m, 3H), 8.32 (s, 1H), 8.50 (m, 2H), 9.17 (s, 1H)
Mass spectrometric value (ESI-MS, m/z): 412 $(M+1)^+$

Compound r303: 6,7-Dimethoxy-4-(7-pyridin-2-yl-isoquinolin-6-yloxy)-quinoline

$^1$H-NMR ($CDCl_3$, 400 MHz): δ 3.93 (s, 3H), 3.97 (s, 3H), 6.27 (d, J = 5.2 Hz, 1H), 7.12 (m, 1H), 7.34 (s, 1H), 7.41 (s, 1H), 7.51 (m, 3H), 7.71 (d, J = 7.6 Hz, 1H), 8.40 (d, J = 5.2 Hz, 1H), 8.49 (d, J = 6.0 Hz, 1H), 8.52 (s, 1H), 8.60 (d, J = 4.4 Hz, 1H), 9.31 (s, 1H)
Mass spectrometric value (ESI-MS, m/z): 432 $(M+Na)^+$

Compound r305: 6-(6,7-Dimethoxy-quinolin-4-yloxy)-7-methyl-quinazoline

$^1$H-NMR ($CDCl_3$, 400 MHz): δ 2.51 (s, 3H), 4.07 (s, 3H), 4.10 (s, 3H), 6.45 (d, J = 5.4 Hz, 1H), 7.57-7.59 (m, 3H), 8.06 (s, 1H), 8.53 (d, J = 5.4 Hz, 1H), 9.31 (s, 1H), 9.34 (s, 1H)
Mass spectrometric value (ESI-MS, m/z): 348 $(M+1)^+$

Compound r320: 1-{2-[6-Methoxy-7-(3-morpholin-4-yl-propoxy)-quinolin-4-yloxy]-4,5-dimethyl-phenyl}-ethanone hydrochloride

$^1$H-NMR ($CD_3OD$, 400 MHz): δ 2.25 - 2.55 (m, 11H), 3.22 (m, 2H), 3.49 (m, 2H), 3.67 (d, J = 12.0 Hz, 2H), 3.86 (t, J = 12.0 Hz, 2H), 4.02 - 4.18 (m, 5H), 4.47 (m, 2H), 6.76 (d, J = 5.2 Hz, 1H), 7.22 (s, 1H), 7.52 (s, 1H), 7.84 (s, 1H), 7.92 (s, 1H), 8.60 (d, J = 5.2 Hz, 1H)
Mass spectrometric value (ESI-MS, m/z): 465 $(M+1-2HCl)^+$

Compound r351: 7-Benzyloxy-6-methoxy-4-(2-methyl-quinolin-3-yloxy)-quinoline

$^1$H-NMR ($CDCl_3$, 400 MHz): δ 2.66 (s, 3H), 4.07 (s, 3H), 5.35 (s, 2H), 6.37 (d, J = 5.1 Hz, 1H), 7.29 - 7.37 (m, 1H), 7.37 - 7.44 (m, 2H), 7.49 - 7.57 (m, 4H), 7.61 (s, 1H), 7.72 (ddd, J = 1.5, 6.8, 8.3 Hz, 1H), 7.75 (d, J = 8.1 Hz, 1H), 7.80 (s, 1H), 8.10 (d, J = 8.6 Hz, 1H), 8.48 (d, J = 5.1 Hz, 1H)
Mass spectrometric value (ESI-MS, m/z): 423 $(M+1)^+$

Compound r352: 6-Methoxy-4-(2-methyl-quinolin-3-yloxy)-quinolin-7-ol

$^1$H-NMR (DMSO-$d_6$, 400 MHz): δ 2.54 (s, 3H), 3.93 (s, 3H), 6.43 (d, J = 5.1 Hz, 1H), 7.31 (s, 1H), 7.52 - 7.62 (m, 2H), 7.73 (dd, J = 7.1, 7.1 Hz, 1H), 7.92 (d, J = 8.1 Hz, 1H), 8.00 (d, J = 8.3 Hz, 1H), 8.13 (s, 1H), 8.40 (d, J = 5.1 Hz, 1H)
Mass spectrometric value (ESI-MS, m/z): 333 $(M+1)^+$

Compound r383: 6-Methoxy-4-(2-methyl-quinolin-3-yloxy)-7-oxilanyl methoxy-quinoline

$^1$H-NMR ($CDCl_3$, 400 MHz): δ 2.66 (s, 3H), 2.83 - 2.89 (m, 1H), 2.94 - 3.01 (m, 1H), 3.49 - 3.57 (m, 1H), 4.06 (s, 3H), 4.21 (dd, J = 5.6, 11.2 Hz, 1H), 4.46 (dd, J = 3.4, 11.5 Hz, 1H), 6.38 (d, J = 5.4 Hz, 1H), 7.48 (s, 1H), 7.55 (dd, J = 7.8, 7.8 Hz, 1H), 7.60 (s, 1H), 7.69 - 7.80 (m, 2H), 7.82 (s, 1H), 8.10 (d, J = 8.6 Hz, 1H), 8.50 (d, J = 5.2 Hz, 1H)
Mass spectrometric value (ESI-MS, m/z): 389 $(M+1)^+$

Compound r384: 3-[6-Methoxy-4-(2-metharl-quinolin-3-yloxy)-quinolin-7-yloxy]-propane-1,2-diol

$^1$H-NMR ($CDCl_3$, 400 MHz): δ 2.65 (s, 3H), 3.86 - 3.97 (m, 2H), 4.04 (s, 3H), 4.22-4.35 (m, 2H), 4.39 (dd, J = 3.9, 9.8 Hz, 1H), 6.41 (d, J = 5.4 Hz, 1H), 7.55 (dd, J = 7.6, 7.6 Hz, 1H), 7.57 (s, 1H), 7.60 (s, 1H), 7.72 (d, J = 7.1 Hz, 1H), 7.76 (d, J = 8.5 Hz, 1H), 7.83 (s, 1H), 8.11 (d, J = 8.6 Hz, 1H), 8.50 (d, J = 5.4 Hz, 1H)
Mass spectrometric value (ESI-MS, m/z): 407 $(M+1)^+$

Compound r410: [6-Methoxy-4-(2-methyl-quinolin-3-yloxy)-quinolin-7-yl]-(2-morpholin-4-yl-ethyl)-amine

$^1$H-NMR ($CDCl_3$, 400 MHz): δ 2.53 (s, 3H), 2.68 (s, 3H), 2.76 (m, 2H), 3.38 (m, 2H), 3.76 (m, 4H), 4.04 (s, 3H), 5.41 (m, 1H), 6.28 (d, J = 5.4 Hz, 1H), 7.08 (s, 1H), 7.44 (s, 1H), 7.53 (m, 1H), 7.68 - 7.74 (m, 2H), 7.76 (s, 1H), 8.09 (d, J = 8.6 Hz, 1H), 8.42 (d, J = 5.4 Hz, 1H)
Mass spectrometric value (ESI-MS, m/z): 445 $(M+1)^+$

Compound r415: 6-Benzyloxy-7-methoxy-4-(2-methyl-quinolin-3-yloxy)-quinoline

$^1$H-NMR ($CDCl_3$, 400 MHz): δ 2.61 (s, 3H), 4.06 (s, 3H), 5.32 (s, 2H), 6.41 (d, J = 5.1 Hz, 1H), 7.22 - 7.40 (m, 3H), 7.44 - 7.58 (m, 4H), 7.61 (s, 1H), 7.67 - 7.76 (m, 3H), 8.09 (d, J = 8.3 Hz, 1H), 8.51 (d, J = 5.1 Hz, 1H)
Mass spectrometric value (ESI-MS, m/z): 423 $(M+1)^+$

Compound r416: 6-(2-Imidazol-1-yl-ethoxy)-7-methoxy-4-(2-methyl-quinolin-3-yloxy)-quinoline

$^1$H-NMR ($CDCl_3$, 400 MHz): δ 2.62 (s, 3H), 4.06 (s, 3H), 4.39 - 4.54 (m, 4H), 6.36 (d, J = 5.2 Hz, 1H), 7.08 (s, 1H), 7.15 (s, 1H), 7.48 (s, 1H), 7.50 - 7.59 (m, 2H), 7.68 - 7.78 (m, 3H), 7.79 (s, 1H), 8.10 (d, J = 8.1 Hz, 1H), 8.51 (d, J = 5.4 Hz, 1H)
Mass spectrometric value (ESI-MS, m/z): 449 $(M+Na)^+$

Evaluation test

Test Example 1A: TGFβ signal inhibitory activity (in vitro test)
The compounds according to the present invention was evaluated for TGFβ signal inhibitory activity according to the method described in J. Boil .Chem., 273, 21145-21152 (1998).
Specifically, a plasmid having four tandem binding sequences of Smad3, a TGF-beta signal transducer, to the upstream of a luciferases gene were used as a reporter plasmid ((SBE)4-Luc) to detect TGF-beta signal,
This reporter gene was introduced into human pulmonary carcinoma epithelial cells (A549) (available from ATCC) to establish a stable cell line.
A test compound and TGFβ-1 (2 ng/ ml) were added to the cells, and the mixture was cultured for 4 hr. In this case, the compounds according to the present invention synthesized in the Examples were used as the test compound. After the culture, the luciferase activity of the cells were measured by chemiluminescence (Steady Glo (trademark) luciferase assay system, available from Promega).
Likewise, the luciferase activity was measured as a control against the culture of cells with the addition of TGFβ only and the culture of cells without the addition of both TGFβ and the test compound.
The TGFβ inhibition (%) was calculated by the following equation, based on the measurement results.

$$\text{TGF}\beta \text{ inhibition (\%)} = (A - B)/(A - C) \times 100$$

wherein

A: luciferase activity with the addition of TGFβ1 and without the addition of test compound (relative luciferase unit),
B: luciferase activity with the addition of both TGFβ1 and test compound (relative luciferase unit), and
C: luciferase activity without the addition of both TGFβ1 and test compound (relative luciferase unit).

The test was carried out for each of test compound at the concentrations of 3 μM and 10 μM. For certain test compounds, the test was further carried out for test compound at the concentration of 1 μM.
The results were as shown in Table 1A.
The results show that the compounds according to the present invention have antagonistic activities against the action of TGFβ.

Test Example 1B: TGFβ signal inhibitory activity (in vitro test)

For the compounds of Reference Examples, the TGFβ signal inhibitory activity was evaluated in the same manner as in Test Example 1A.
The results were as shown in Table 1B

Table A:

[Table 1]

| Compound | Name of compound | Measured molecular weight |
|---|---|---|
| 178 | 2-[6-Methoxy-4-(2-phenyl-[1,8]naphthyridin-3-yl oxy)-quinolin-7-yloxy]-ethanol | 462(M+ Na)+ |
| 179 | 4-(6-Ethyl-[2,2']bipyridinyl-3-yloxy)-6-methoxy-quinoline-7-carboxylic acid amide | 423(M+ Na)+ |
| 180 | 3-(2-Tert-butyl-thieno[3,2-b]pyridin-7-yloxy)-6-ethyl-[2,2']bipyridine | 412(M+ Na)+ |
| 181 | 2-[4-(6-Ethyl-[2,2']bipyridinyl-3-yloxy)-quinolin-6-yloxy]-ethanol | 410(M+ Na)+ |
| 182 | 2-[4-(6-Ethyl-[2,2']bipyridinyl-3-yloxy)-quinolin-6-yloxy]-ethyl acetate | 452(M+ Na)+ |
| 183 | 3-(6-Benzyloxy-quinolin-4-yloxy)-6-ethyl-[2,2']bipyridine | 456(M+ Na)+ |
| 184 | 4-(6-Ethyl-[2,2']bipyridinyl-3-yloxy)-quinolin-6-ol | 366(M+ Na)+ |
| 185 | 6-Chloro-4-(6-ethyl-[2,2']bipyridinyl-3-yloxy)-quinoline-7-carboxylic acid amide | 427(M+ Na)+ |
| 186 | 4-(6-Ethyl-[2,2']bipyridinyl-3-yloxy)-quinolin-6-ylacetate | 408(M+ Na)+ |
| 187 | 4-(6-Ethyl-[2,2']bipyridinyl-3-yloxy)-quinolin-6-yl methanesulfonate | 444(M+ Na)+ |
| 188 | 6,7-Dimethoxy-4-(6-methyl-2-pyrimidin-2-yl-pyridin-3-yloxy)-quinoline | 397(M+ Na)+ |
| 189 | 1-{2-[4-(6-Ethyl-[2,2']bipyridinyl-3-yloxy)-quinolin-6-yloxy]-ethyl}-3-propyl-urea | 494(M+ Na)+ |
| 190 | 6-Benzyloxy-4-(6-ethyl-2-pyrimidin-2-yl-pyridin-3-yloxy)-quinoline | 457(M+ Na)+ |
| 191 | 4-(6-Ethyl-2-pyrimidin-2-yl-pyridin-3-yloxy)-quinolin-6-ol | 343(M-1)- |
| 192 | 2-[4-(6-Ethyl-2-pyrimidin-2-yl-pyridin-3-yloxy)-quinolin-6-yloxy]-ethanol | 411(M+ Na)+ |
| 193 | 6-Benzyloxy-4-(6-ethyl-2-pyrimidin-2-yl-pyridin-3-yloxy)-quinoline-7-carboxylic acid amide | 500(M+ Na)+ |
| 194 | 4-(6-Ethyl-2-pyrimidin-2-yl-pyridin-3-yloxy)-6-hydroxy-quinoline-7-carboxylic acid amide | 386(M-1)- |
| 200 | 2-[4-(5,6-Dimethyl-2-pyrimidin-2-yl-pyridin-3-yloxy)-quinolin-6-yloxy]-ethanol | 411(M +Na)+ |
| 201 | 4-(6-Methyl-2-pyrimidin-2-yl-pyridin-3-yloxy)-quinolin -6-ol | 329(M -1)- |
| 202 | 2-[4-(6-Methyl-2-pyrimidin-2-yl-pyridin-3-yloxy)-quinolin-6-yloxy]-ethanol | 397(M +Na)+ |
| 203 | N-{2-[4-(6-Ethyl-2-pyrimidin-2-yl-pyridin-3-yloxy)-quinolin-6-yloxy]-ethyl}-methanesulfonic acid amide | 488(M +Na)+ |
| 204 | 2-[4-(6-Ethyl-2-pyrimidin-2-yl-pyridin-3-yloxy)-quinolin-6-yloxy]-ethylamine | 410(M +Na)+ |
| 205 | 2-[4-(6-Methyl-[2,2']bipyridinyl-3-yloxy)-quinolin-6-yloxy]-ethanol | 374(M +1)+ |
| 206 | 2-[4-(6-Ethyl-2-pyrimidin-2-yl-pyridin-3-yloxy)-quinolin-6-yloxy]-ethyl acetate | 453(M +Na)+ |
| 207 | 2-[4-(6-Ethyl-2-pyrimidin-2-yl-pyridin-3-yloxy)-quinolin-6-yloxy]-ethyl 2,2-dimethyl-propanate | 495(M +Na)+ |
| 208 | 2-[4-(6-Ethyl-2-pyrimidin-2-yl-pyridin-3-yloxy)-quinolin-6-yloxy]-ethyl (S)-2-amino-3-methyl-butanoate | 510(M +Na)+ |

(continued)

| Compound | Name of compound | Measured molecular weight |
|---|---|---|
| 209 | 4-(6-Methyl-2-pyrimidin-2-yl-pyridin-3-yloxy)-6-(2-morpholin-4-yl-ethoxy)-quinoline | 466(M +Na)+ |
| 210 | 6-(3-Chloro-propoxy)-4-(6-methyl-2-pyrimidin-2-yl-pyridin-3-yloxy)-quinoline | 429(M +Na)+ |
| 211 | 2-(Methyl-{3-[4-(6-methyl-2-pyrimidin-2-yl-pyridin-3-yloxy)-quinolin-6-yloxy]-propyl}-amino)-ethanol | 468(M +Na)+ |
| 212 | 4-(6-Methyl-2-pyrimidin-2-yl-pyridin-3-yloxy)-6-(3-pip eridin-1-イル-propoxy)-quinoline | 478(M +Na)+ |
| 213 | 6-Cyclobutylmethoxy-4-(6-methyl-2-pyrimidin-2-yl-py ridin-3-yloxy)-quinoline | 421(M +Na)+ |
| 214 | 6-(2,2-Dimethyl-propoxy)-4-(6-methyl-2-pyrimidin-2-yl-pyridin-3-yloxy)-quinoline | 423(M +Na)+ |
| 215 | 2,2-Dimethyl-3-[4-(6-methyl-2-pyrimidin-2-yl-pyridin-3-yloxy)-quinolin-6-yloxy]-propan-1-ol | 439(M +Na)+ |
| 216 | 6-(2-Cyclohexylethoxy)-4-(6-methyl-2-pyrimidin-2-yl-pyridin-3-yloxy)-quinoline | 463(M +Na)+ |
| 217 | 4-(6-Methyl-2-pyrimidin-2-yl-pyridin-3-yloxy)-quinoline-7-carboxylic acid amide | 380(M +Na)+ |
| 218 | 4-(6-Methyl-2-pyrimidin-2-yl-pyridin-3-yloxy)-6-[3-(4-pyridin-2-yl-piperazin-1-yl)-propoxy]-quinoline | 556(M +Na)+ |
| 219 | 2-({3-[4-(6-Methyl-2-pyrimidin-2-yl-pyridin-3-yloxy)-quinolin-6-yloxy]-propyl}-phenylamino)-ethanol | 530(M +Na)+ |
| 220 | 6-[3-(4-Benzyl-piperidin-1-yl)-propoxy]-4-(6-methyl-2-pyrimidin-2-yl-pyridin-3-yloxy)-quinoline | 568(M+Na) + |
| 221 | 2-(4-{3-[4-(6-Methyl-2-pyrimidin-2-yl-pyridin-3-yloxy)-quinolin-6-yloxy]-propyl}-piperazin-1-yl)-ethano l | 523(M +Na) + |
| 222 | 3-[4-(6-Methyl-2-pyrimidin-2-yl-pyridin-3-yloxy)-quinolin-6-yloxy]-propan-1-ol | 411(M+Na) + |
| 223 | ethyl-methyl-{2-[4-(6-methyl-2-pyrimidin-2-yl-pyridin-3-yloxy)-quinolin-6-yloxy]-ethyl}-amine | 438(M+Na) + |
| 224 | 2-[4-(6-Ethyl-2-pyrimidin-2-yl-pyridin-3-yloxy)-quinolin-6-yloxy]-ethyl 2,2-dimethyl-butanoate | 509(M +Na)+ |
| 225 | 2-[4-(6-Ethyl-2-pyrimidin-2-yl-pyridin-3-yloxy)-7-fluoro-quinolin-6-yloxy]-ethanol | 429(M +Na)+ |

Table 1A:

[Table 1A]

| Compound | Molecular structure | TGFβ inhibition rate, % | | |
|---|---|---|---|---|
| | | 10 μM | 3 μM | 1 μM |
| 1 | 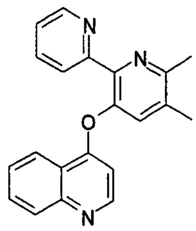 | 100 | 100 | |
| 2 | 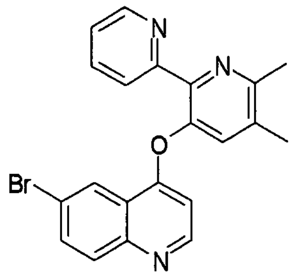 | 100 | 100 | |
| 3 | 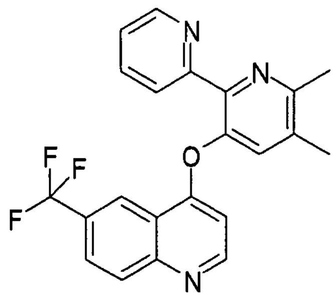 | | 99 | 79 |
| 4 | 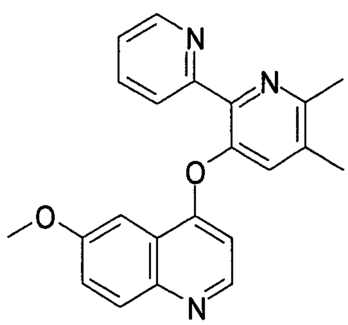 | | 100 | 94 |
| 5 | 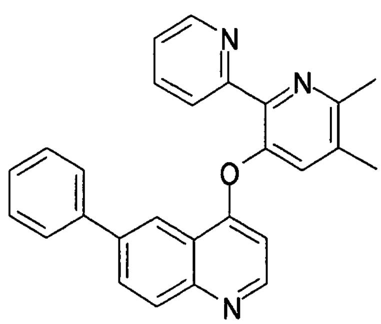 | 100 | 79 | |

(continued)

| Compound | Molecular structure | TGFβ inhibition rate, % | | |
|---|---|---|---|---|
| | | 10 μM | 3 μM | 1 μM |
| 6 | 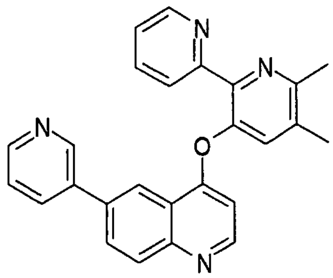 | 100 | 100 | |
| 7 | 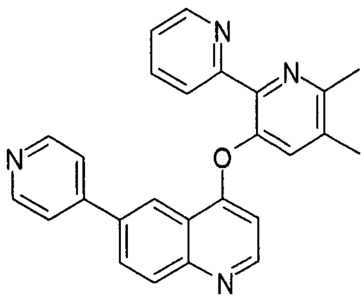 | 100 | 100 | |
| 8 | 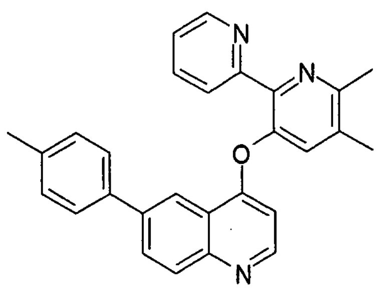 | 100 | 60 | |
| 9 | 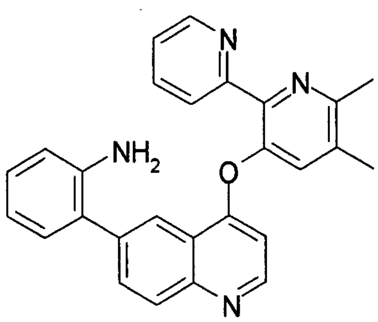 | 100 | 87 | |
| 10 | 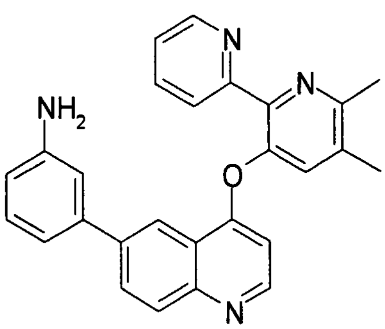 | 100 | 100 | |

(continued)

| Compound | Molecular structure | TGFβ inhibition rate, % | | |
|---|---|---|---|---|
| | | 10 μM | 3 μM | 1 μM |
| 11 | 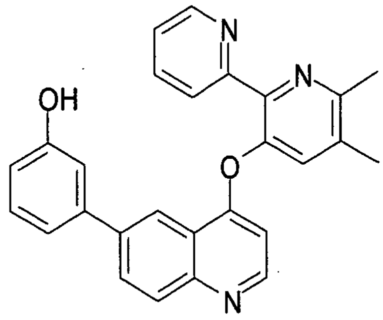 | 100 | 100 | |
| 12 | 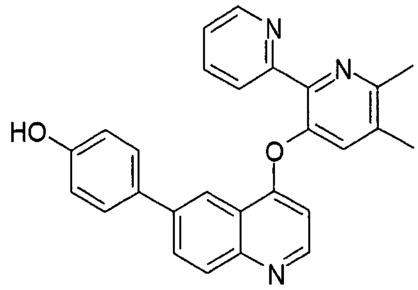 | 100 | 100 | |
| 13 | 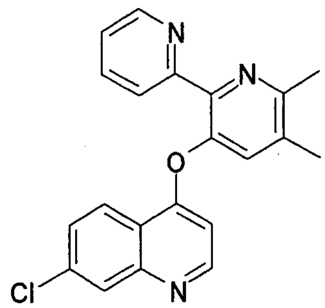 | 100 | 99 | |
| 14 | 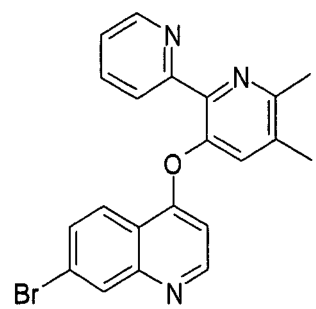 | 100 | 100 | |

(continued)

| Compound | Molecular structure | TGFβ inhibition rate, % | | |
|---|---|---|---|---|
| | | 10 μM | 3 μM | 1 μM |
| 15 | 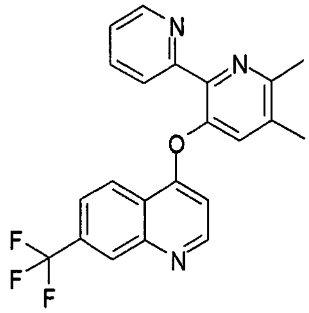 | 100 | 99 | |
| 16 | 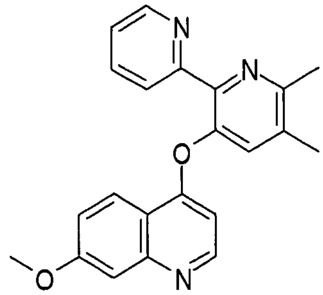 | | 100 | 97 |
| 17 | 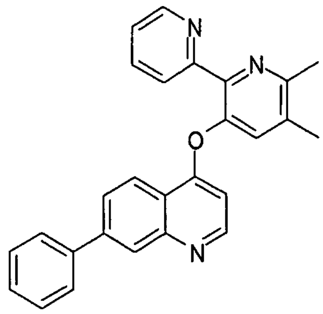 | 100 | 98 | |
| 18 | 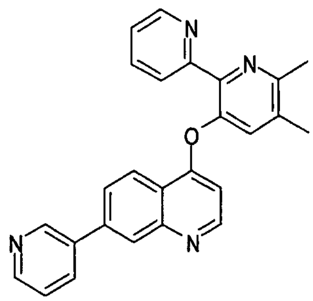 | 100 | 95 | |
| 19 | 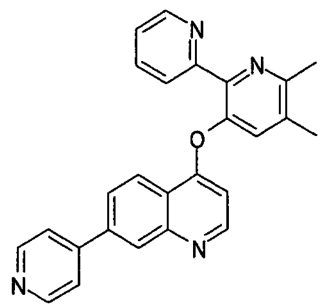 | 100 | 100 | |

(continued)

| Compound | Molecular structure | TGFβ inhibition rate, % | | |
|---|---|---|---|---|
| | | 10 μM | 3 μM | 1 μM |
| 20 | 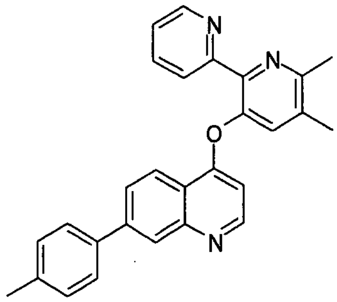 | 100 | 90 | |
| 21 | 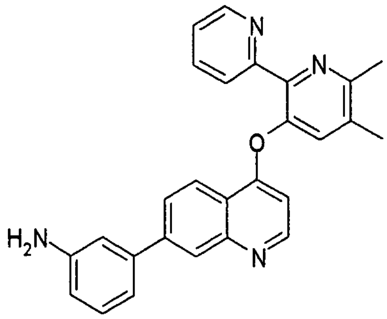 | 100 | 99 | |
| 22 | 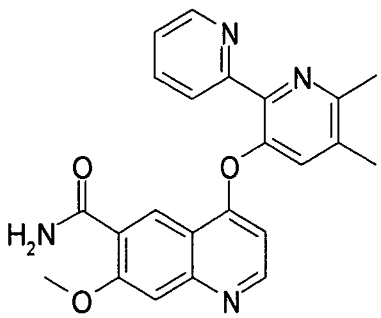 | | 99 | 81 |
| 23 | 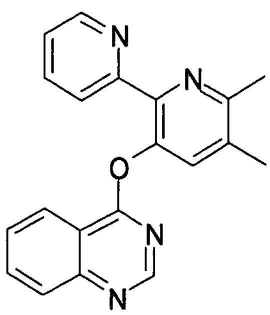 | 98 | 78 | |

(continued)

| Compound | Molecular structure | TGFβ inhibition rate, % | | |
|---|---|---|---|---|
| | | 10 μM | 3 μM | 1 μM |
| 24 | 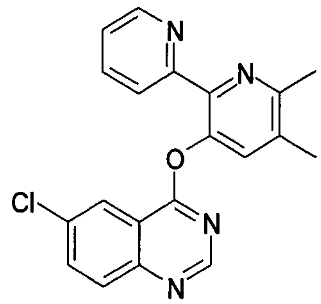 | 99 | 90 | |
| 25 | 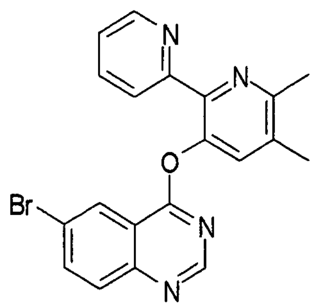 | 99 | 89 | |
| 26 | 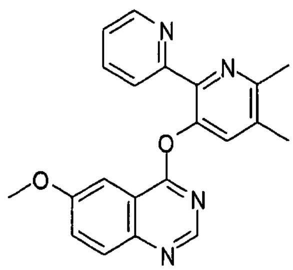 | 99 | 83 | |
| 27 | 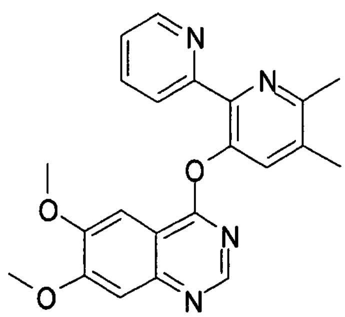 | | 100 | 89 |
| 28 | 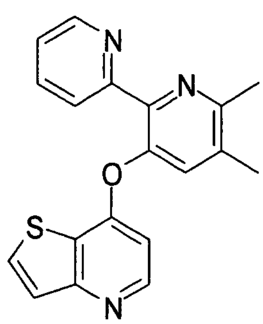 | | 100 | 96 |

(continued)

| Compound | Molecular structure | TGFβ inhibition rate, % | | |
|---|---|---|---|---|
| | | 10 μM | 3 μM | 1 μM |
| 29 | 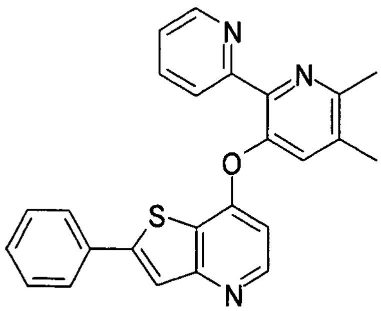 | | 100 | 89 |
| 30 | 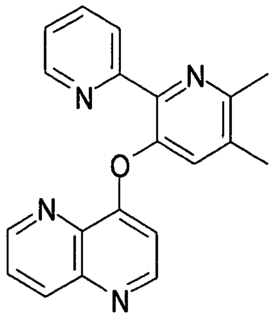 | 99 | 84 | |
| 31 | 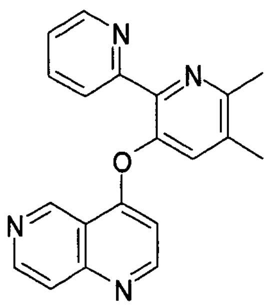 | 99 | 85 | |
| 32 | 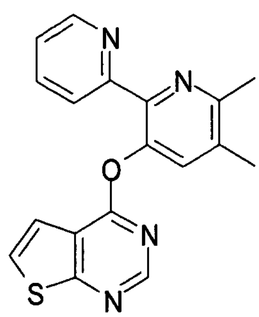 | 94 | 64 | |

(continued)

| Compound | Molecular structure | TGFβ inhibition rate, % | | |
|---|---|---|---|---|
| | | 10 μM | 3 μM | 1 μM |
| 33 | 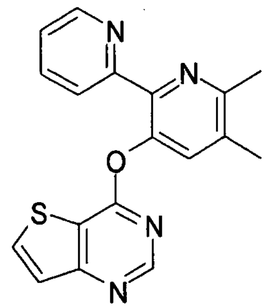 | 99 | 86 | |
| 34 | 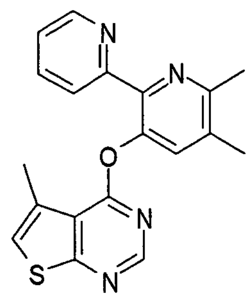 | 100 | 94 | |
| 35 | 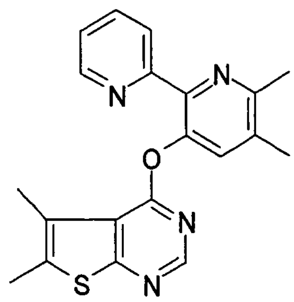 | 100 | 95 | |
| 37 | 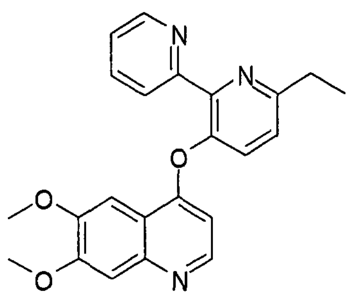 | | 100 | 98 |
| 38 | 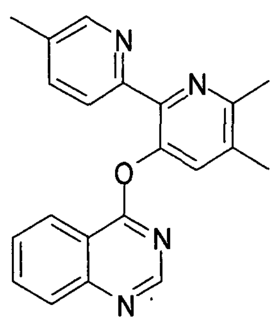 | 89 | 45 | |

(continued)

| Compound | Molecular structure | TGFβ inhibition rate, % | | |
|---|---|---|---|---|
| | | 10 μM | 3 μM | 1 μM |
| 39 | 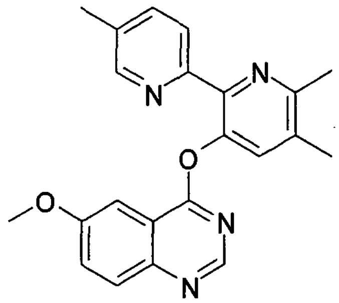 | 99 | 88 | |
| 41 | 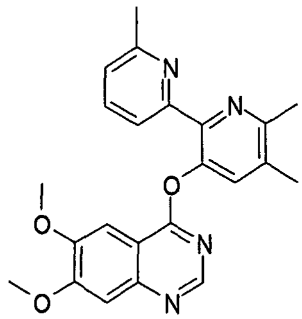 | 98 | 66 | |
| 43 | 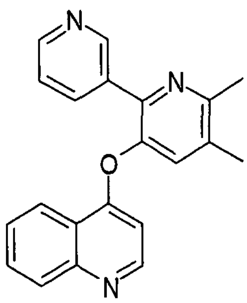 | | 84 | 45 |
| 44 | 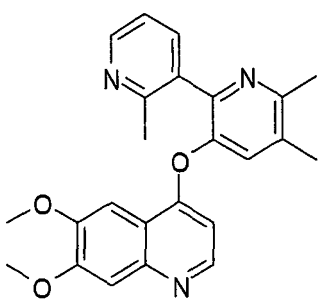 | 99 | 82 | |

(continued)

| Compound | Molecular structure | TGFβ inhibition rate, % | | |
|---|---|---|---|---|
| | | 10 μM | 3 μM | 1 μM |
| 45 | 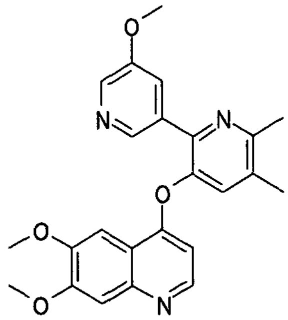 | 100 | 89 | |
| 46 | 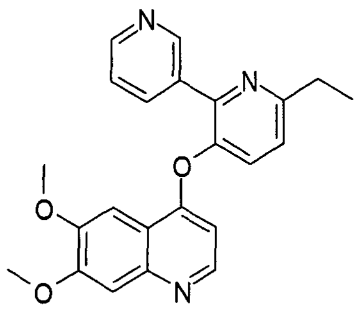 | 100 | 100 | |
| 48 | 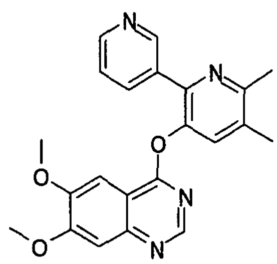 | | 64 | 28 |
| 49 | 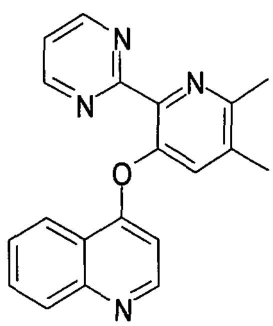 | 100 | 97 | |
| 50 | 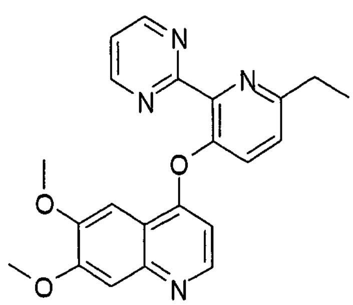 | 100 | 98 | |

(continued)

| Compound | Molecular structure | TGFβ inhibition rate, % | | |
|---|---|---|---|---|
| | | 10 μM | 3 μM | 1 μM |
| 51 | 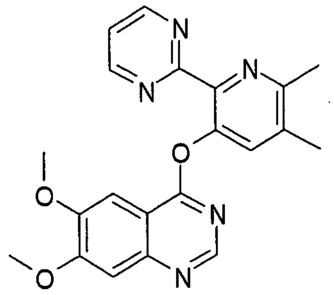 | 100 | 93 | |
| 52 | 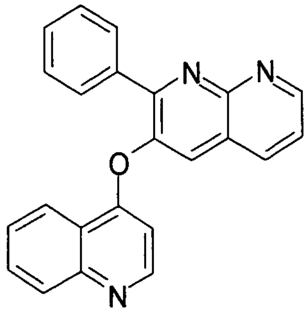 | 100 | 96 | |
| 53 | 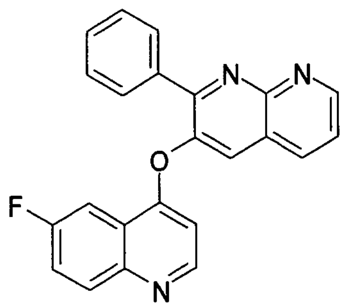 | | 83 | 43 |
| 54 | 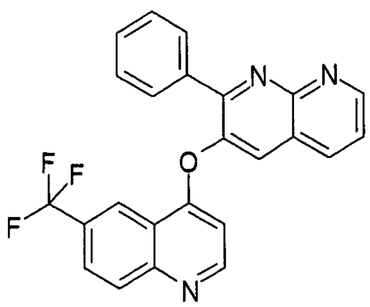 | 99 | 76 | |
| 55 | 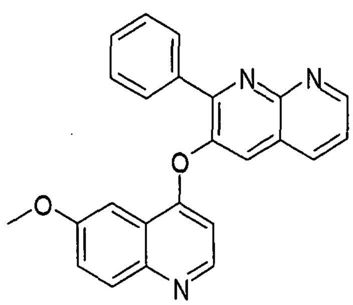 | 100 | 94 | |

(continued)

| Compound | Molecular structure | TGFβ inhibition rate, % | | |
|---|---|---|---|---|
| | | 10 μM | 3 μM | 1 μM |
| 56 | 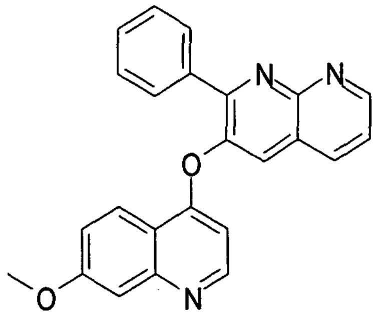 | 100 | 99 | |
| 57 | 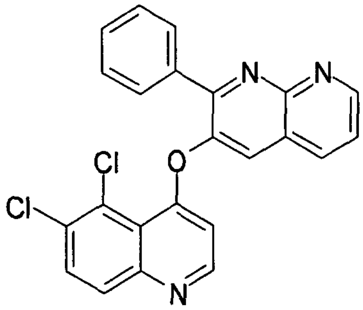 | 100 | 75 | |
| 58 | 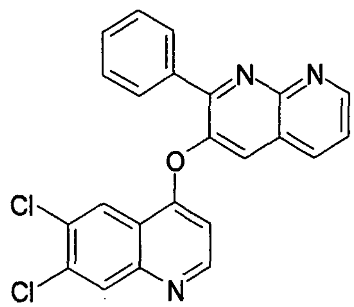 | 100 | 83 | |
| 59 | 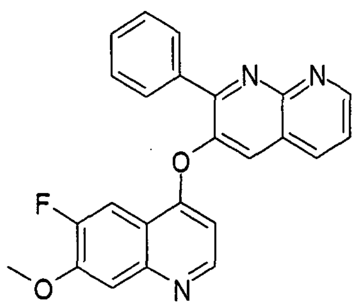 | | | 93 |

(continued)

| Compound | Molecular structure | TGFβ inhibition rate, % | | |
|---|---|---|---|---|
| | | 10 μM | 3 μM | 1 μM |
| 60 | 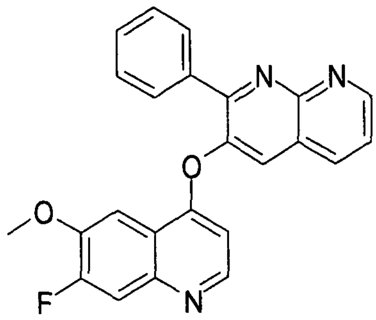 | 100 | 76 | |
| 61 | 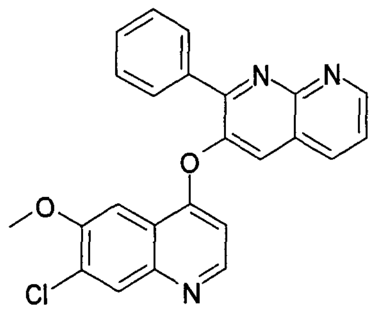 | 100 | 98 | |
| 62 | 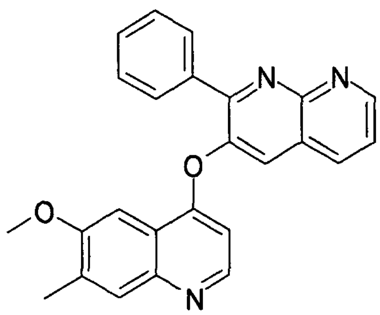 | 100 | 99 | |
| 63 | 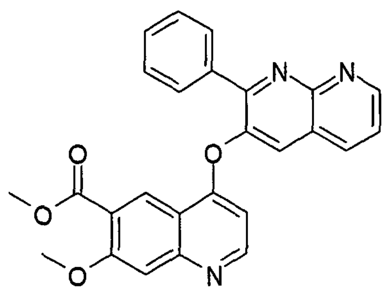 | 100 | 99 | |
| 65 | 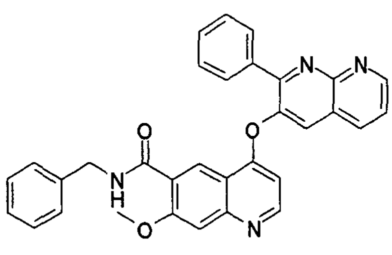 | 100 | 97 | |

(continued)

| Compound | Molecular structure | TGFβ inhibition rate, % | | |
|---|---|---|---|---|
| | | 10 μM | 3 μM | 1 μM |
| 66 | 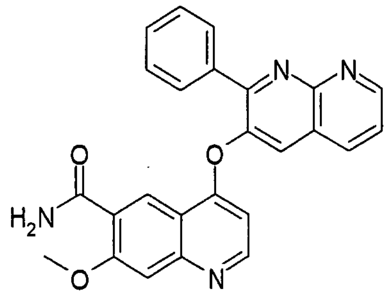 | | 100 | 97 |
| 68 | 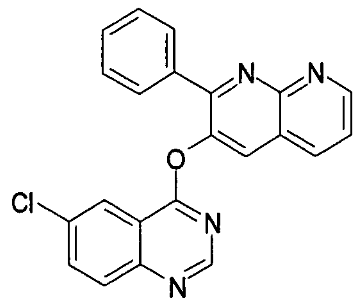 | 91 | 43 | |
| 69 | 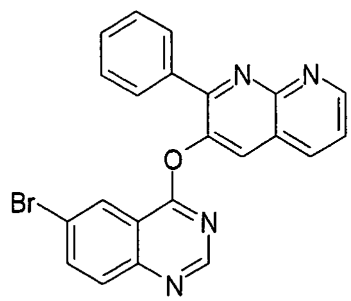 | 94 | 52 | |
| 70 | 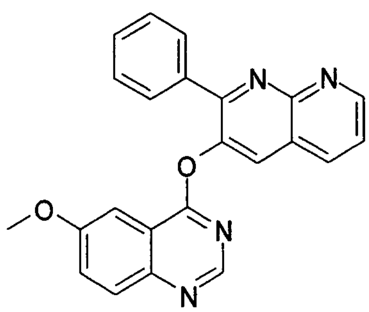 | 95 | 61 | |
| 71 | 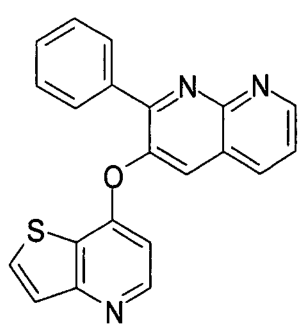 | 100 | 93 | |

(continued)

| Compound | Molecular structure | TGFβ inhibition rate, % | | |
|---|---|---|---|---|
| | | 10 μM | 3 μM | 1 μM |
| 72 | 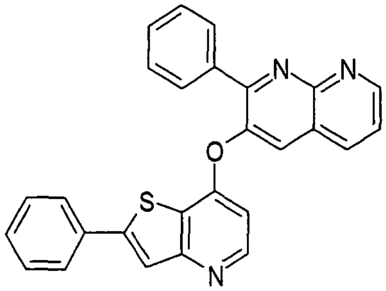 | | 99 | 81 |
| 73 | 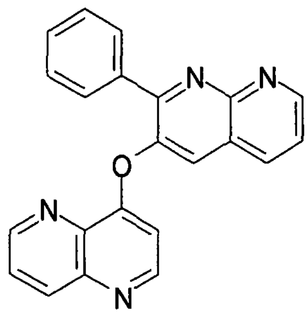 | 80 | 26 | |
| 74 | 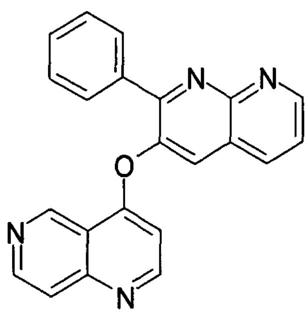 | 90 | 53 | |
| 75 | 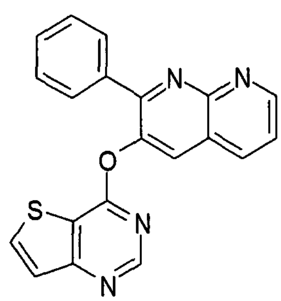 | 98 | 84 | |
| 76 | 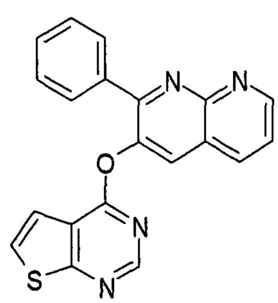 | 83 | 44 | |

(continued)

| Compound | Molecular structure | TGFβ inhibition rate, % | | |
|---|---|---|---|---|
| | | 10 μM | 3 μM | 1 μM |
| 77 | 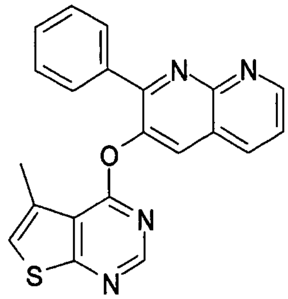 | 99 | 93 | |
| 78 | 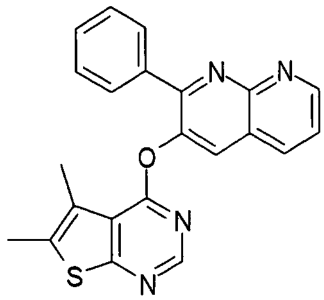 | 99 | 75 | |
| 81 | 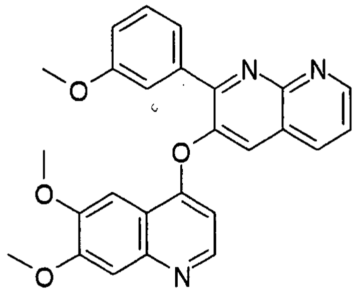 | 100 | 97 | |
| 82 | 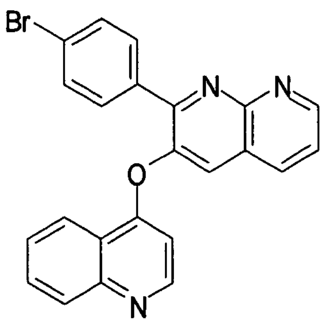 | 67 | 25 | |
| 83 | 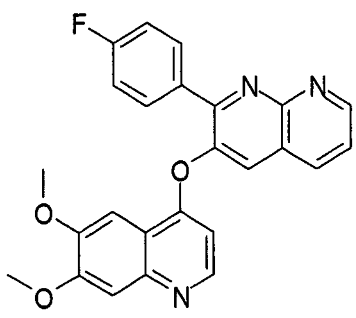 | 99 | 92 | |

(continued)

| Compound | Molecular structure | TGFβ inhibition rate, % | | |
|---|---|---|---|---|
| | | 10 μM | 3 μM | 1 μM |
| 84 | 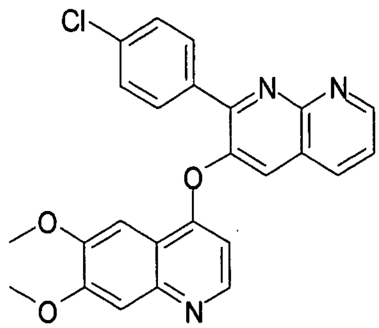 | 100 | 69 | |
| 85 | 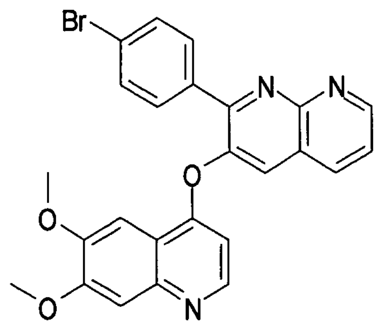 | 97 | 48 | |
| 86 | 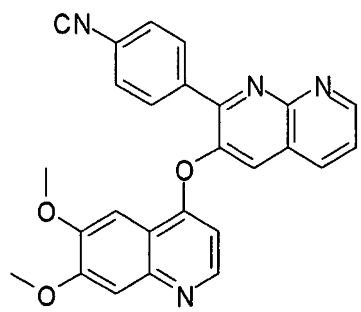 | 74 | 26 | |
| 88 | 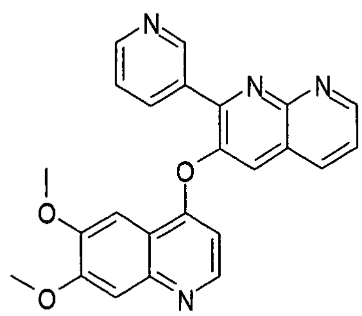 | 100 | 90 | |

(continued)

| Compound | Molecular structure | TGFβ inhibition rate, % | | |
|---|---|---|---|---|
| | | 10 μM | 3 μM | 1 μM |
| 89 | 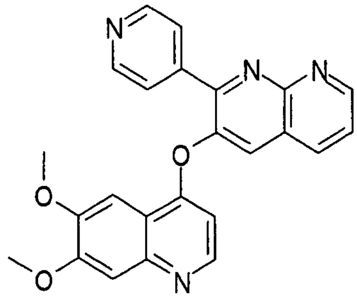 | 77 | 36 | |
| 90 | 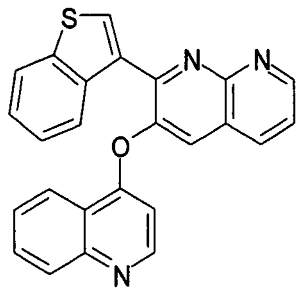 | 68 | 22 | |
| 91 | 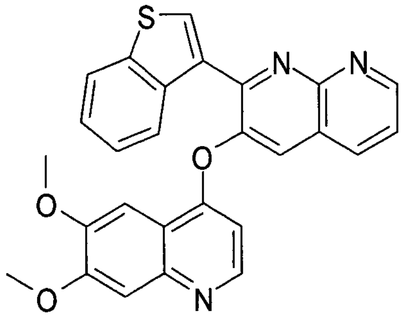 | 100 | 97 | |
| 92 | 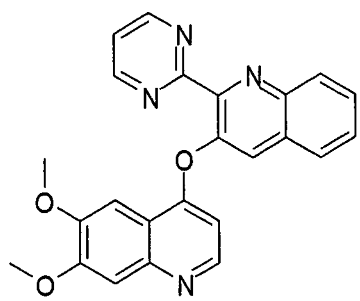 | | 98 | 62 |
| 93 | 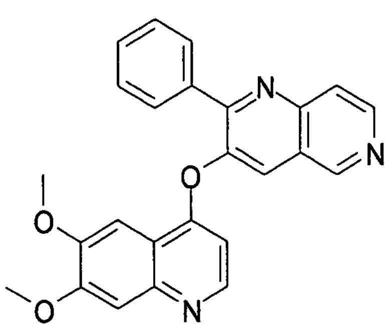 | 100 | 97 | |

(continued)

| Compound | Molecular structure | TGFβ inhibition rate, % | | |
|---|---|---|---|---|
| | | 10 μM | 3 μM | 1 μM |
| 95 | 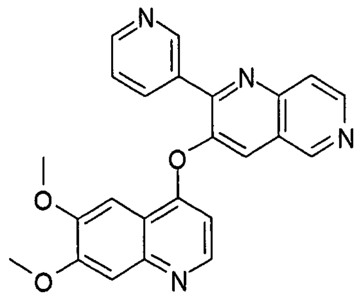 | 85 | 40 | |
| 97 | 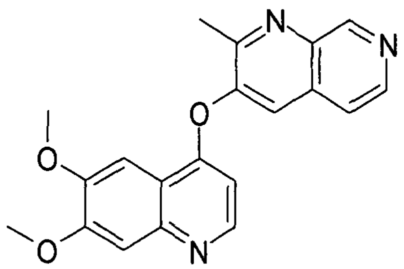 | 88 | 52 | |
| 98 | 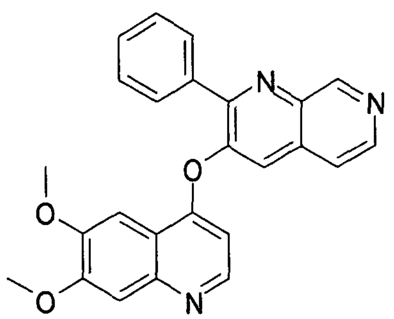 | 100 | 94 | |
| 99 | 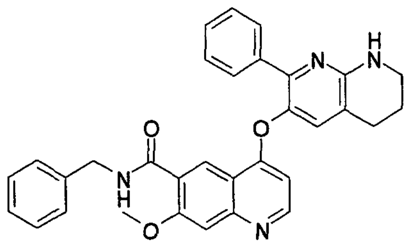 | 92 | 76 | |
| 100 | 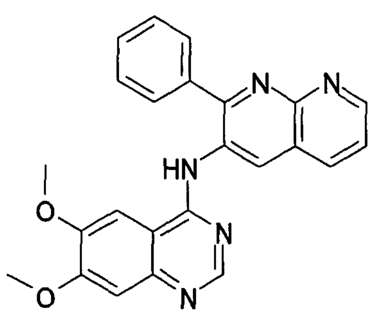 | 93 | 56 | |

(continued)

| Compound | Molecular structure | TGFβ inhibition rate, % | | |
|---|---|---|---|---|
| | | 10 μM | 3 μM | 1 μM |
| 101 | 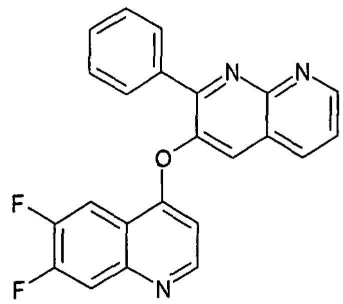 | 98 | 54 | |
| 102 | 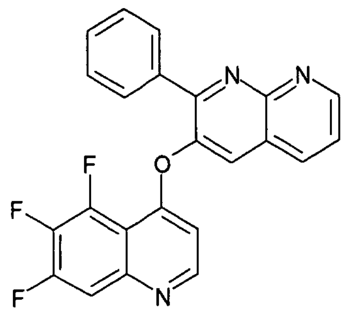 | 92 | 45 | |
| 103 | 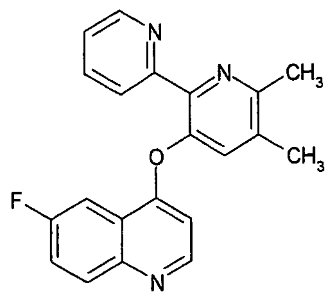 | 100 | 99 | |
| 104 | 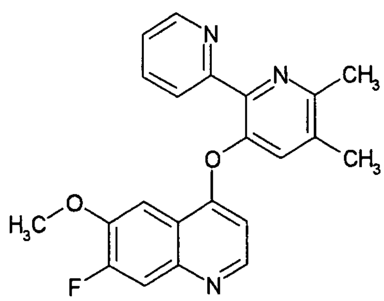 | 100 | 99 | |
| 105 | 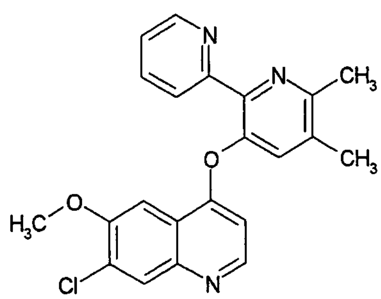 | 100 | 100 | |

(continued)

| Compound | Molecular structure | TGFβ inhibition rate, % | | |
|---|---|---|---|---|
| | | 10 μM | 3 μM | 1 μM |
| 106 | 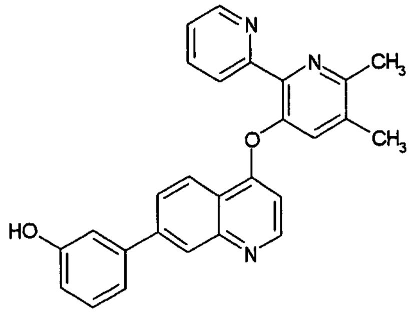 | 100 | 99 | |
| 107 | 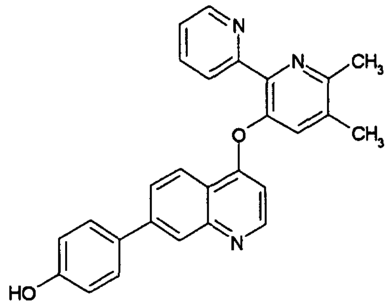 | 100 | 100 | |
| 108 | 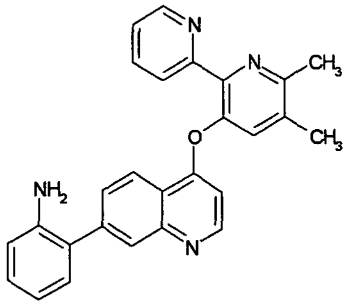 | 100 | 96 | |
| 110 | 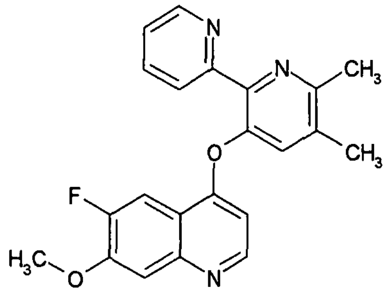 | 100 | 100 | |

(continued)

| Compound | Molecular structure | TGFβ inhibition rate, % | | |
|---|---|---|---|---|
| | | 10 μM | 3 μM | 1 μM |
| 111 | 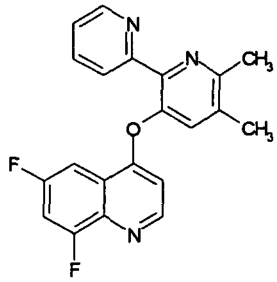 | 84 | 43 | |
| 113 | 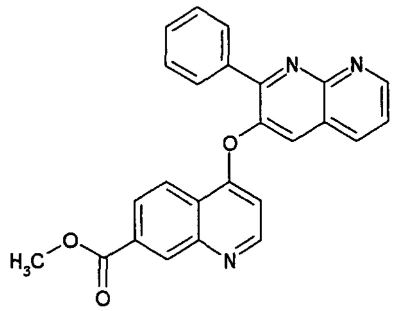 | 100 | 95 | |
| 115 | 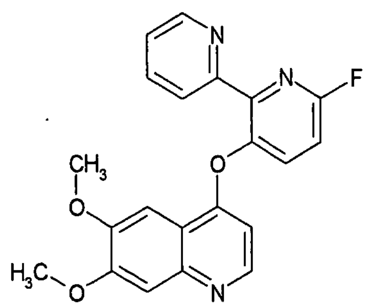 | 100 | 100 | |
| 116 | 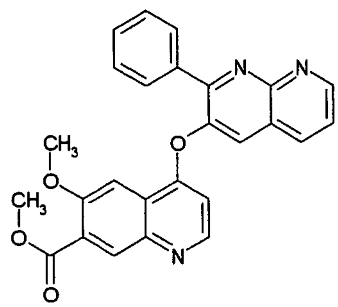 | 100 | 99 | |
| 117 | 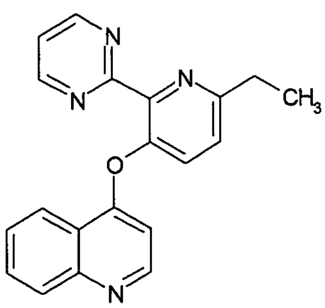 | 100 | 96 | |

(continued)

| Compound | Molecular structure | TGFβ inhibition rate, % | | |
|---|---|---|---|---|
| | | 10 μM | 3 μM | 1 μM |
| 118 | 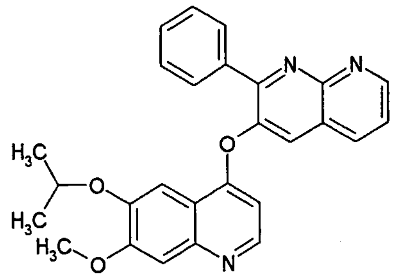 | 100 | 100 | |
| 119 | 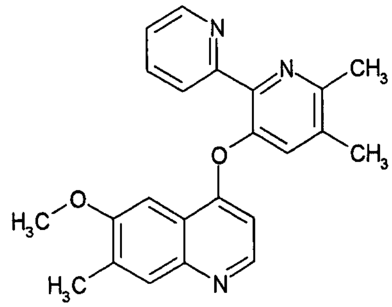 | 100 | 100 | |
| 120 | 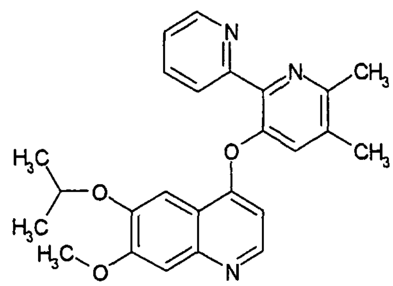 | 100 | 94 | |
| 121 | 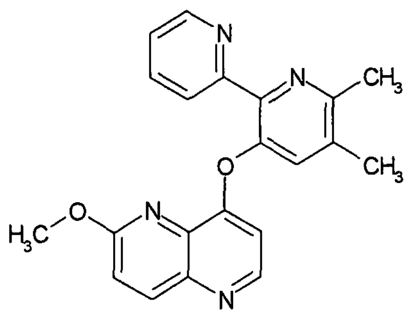 | 100 | 98 | |
| 123 | 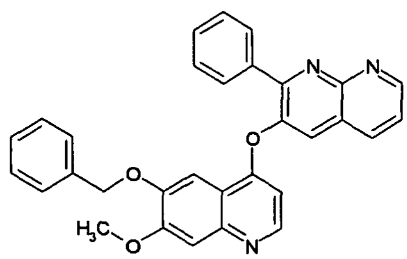 | 100 | 99 | |

(continued)

| Compound | Molecular structure | TGFβ inhibition rate, % | | |
|---|---|---|---|---|
| | | 10 μM | 3 μM | 1 μM |
| 124 | 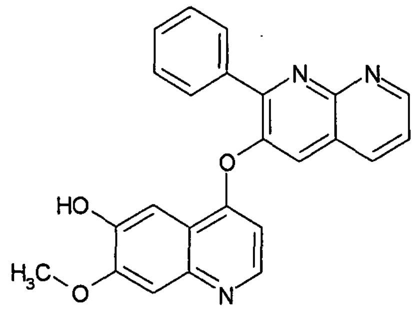 | 100 | 99 | |
| 125 | 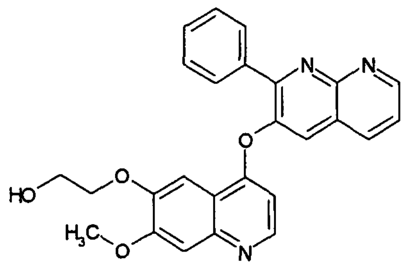 | 100 | 99 | |
| 126 | 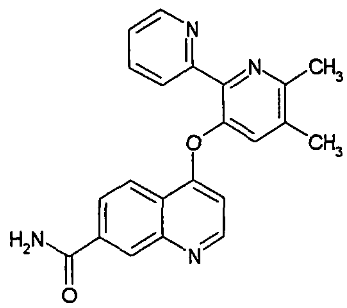 | 100 | 93 | |
| 128 | 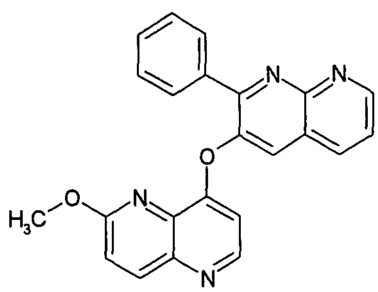 | 100 | 93 | |
| 130 | 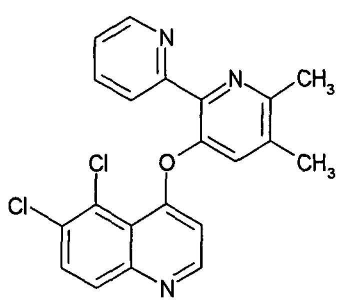 | 100 | 100 | |

(continued)

| Compound | Molecular structure | TGFβ inhibition rate, % | | |
|---|---|---|---|---|
| | | 10 μM | 3 μM | 1 μM |
| 131 | 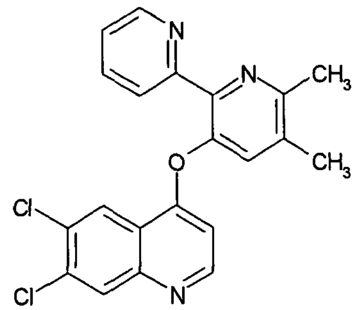 | 100 | 99 | |
| 132 | 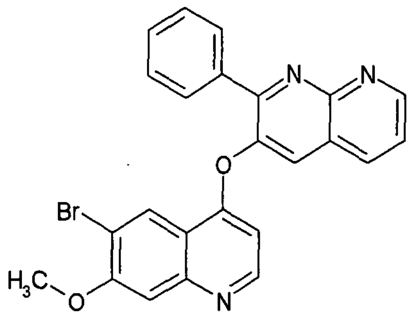 | 100 | 98 | |
| 133 | 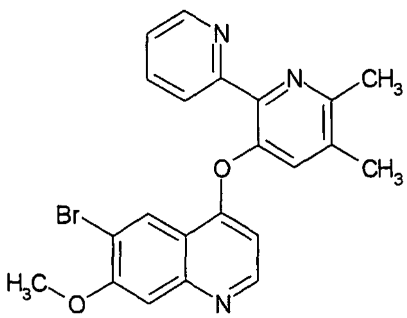 | 100 | 99 | |
| 136 | 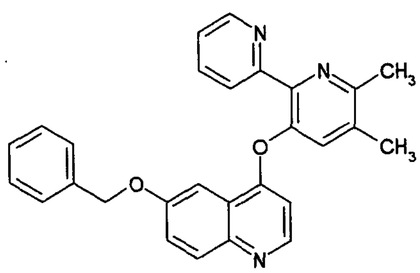 | 100 | 93 | |

(continued)

| Compound | Molecular structure | TGFβ inhibition rate, % | | |
|---|---|---|---|---|
| | | 10 μM | 3 μM | 1 μM |
| 137 | 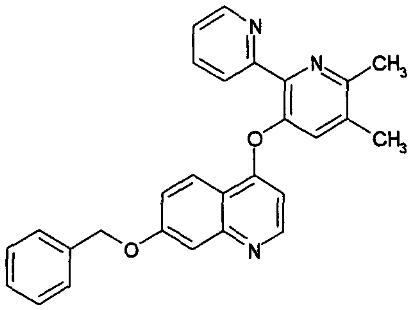 | 100 | 88 | |
| 138 | 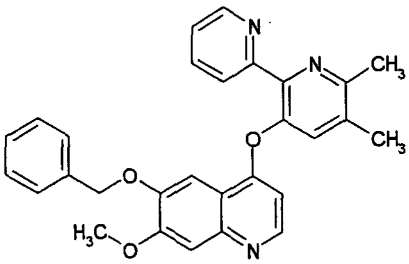 | 100 | 98 | |
| 139 | 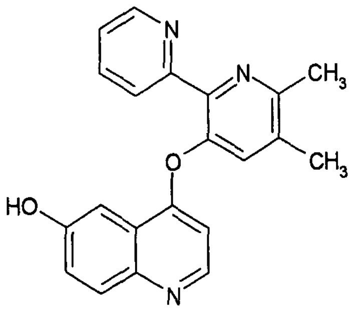 | 100 | 100 | |
| 140 | 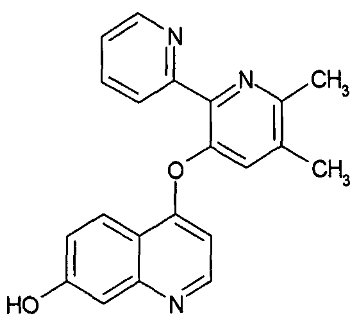 | 100 | 99 | |
| 141 | 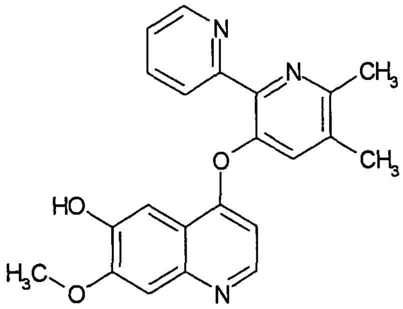 | 100 | 100 | |

(continued)

| Compound | Molecular structure | TGFβ inhibition rate, % | | |
|---|---|---|---|---|
| | | 10 μM | 3 μM | 1 μM |
| 142 | 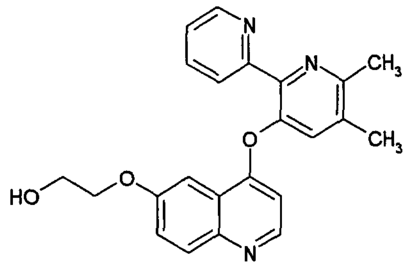 | 100 | 98 | |
| 143 | 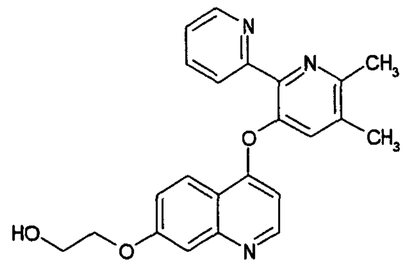 | 100 | 100 | |
| 144 | 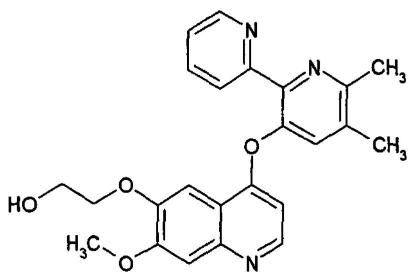 | 100 | 93 | |
| 145 | 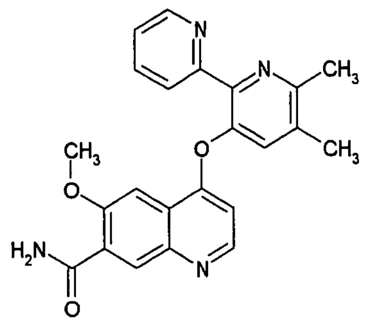 | 100 | 78 | |
| 146 | 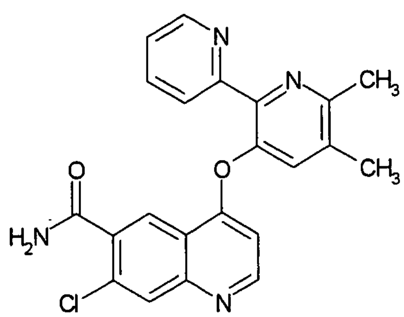 | 95 | 54 | |

(continued)

| Compound | Molecular structure | TGFβ inhibition rate, % | | |
|---|---|---|---|---|
| | | 10 μM | 3 μM | 1 μM |
| 147 | 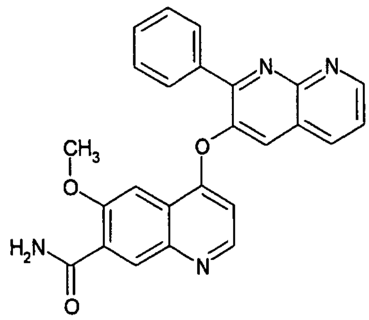 | 77 | 34 | |
| 148 | 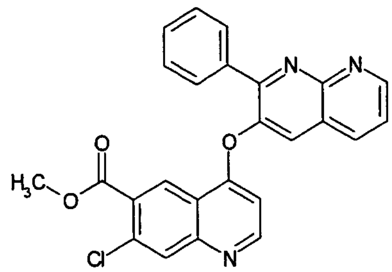 | 98 | 58 | |
| 154 | 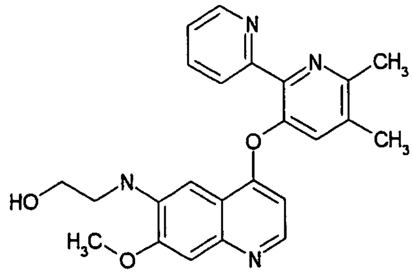 | 100 | 99 | |
| 155 | 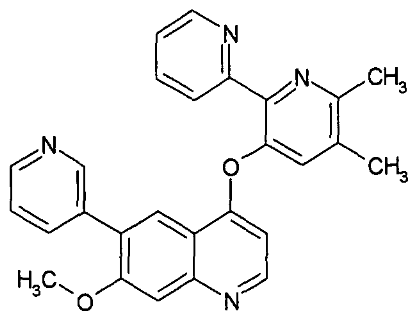 | 100 | 99 | |
| 156 | 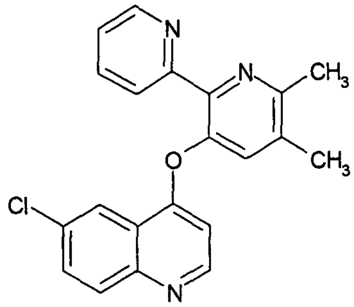 | 100 | 100 | |

(continued)

| Compound | Molecular structure | TGFβ inhibition rate, % | | |
|---|---|---|---|---|
| | | 10 μM | 3 μM | 1 μM |
| 157 | 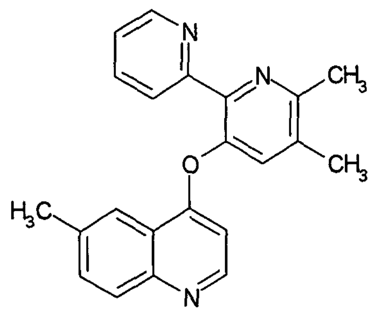 | 100 | 99 | |
| 158 | 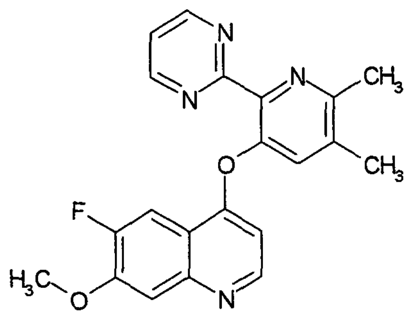 | 100 | 98 | |
| 159 | 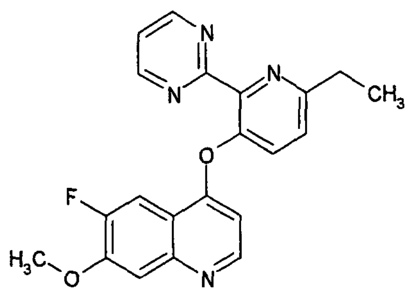 | 100 | 99 | |
| 160 | 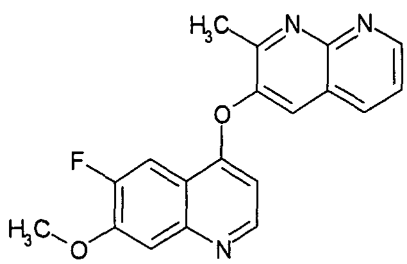 | 87 | 56 | |
| 161 | 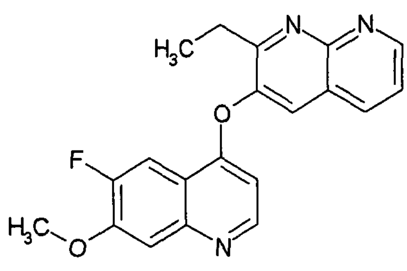 | 95 | 68 | |

(continued)

| Compound | Molecular structure | TGFβ inhibition rate, % | | |
|---|---|---|---|---|
| | | 10 μM | 3 μM | 1 μM |
| 162 | 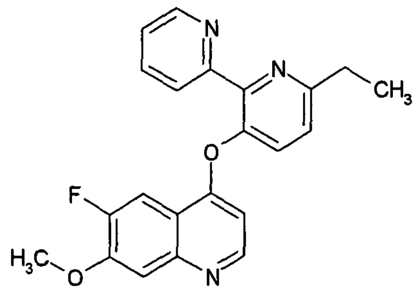 | 100 | 100 | |
| 163 | 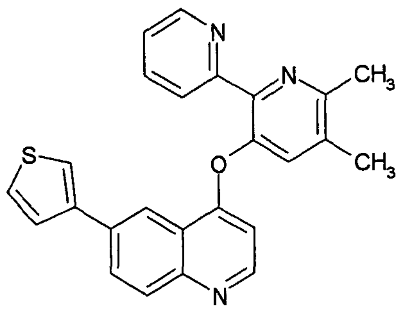 | 100 | 92 | |
| 164 | 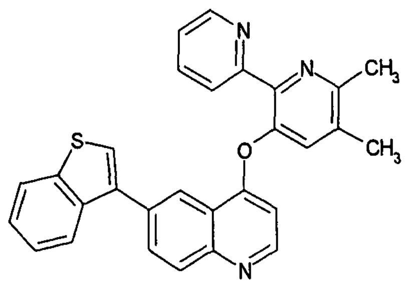 | 100 | 75 | |
| 165 | 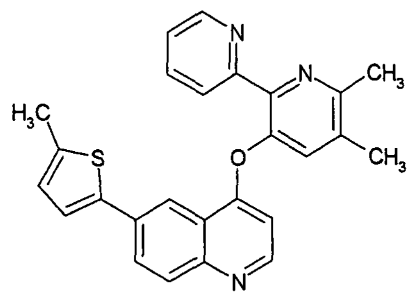 | 100 | 90 | |
| 166 | 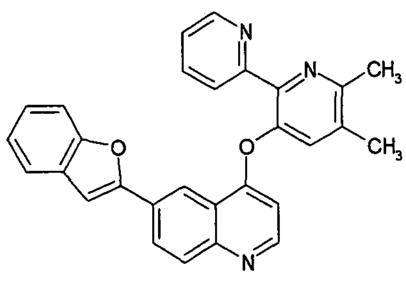 | 100 | 78 | |

(continued)

| Compound | Molecular structure | TGFβ inhibition rate, % | | |
|---|---|---|---|---|
| | | 10 μM | 3 μM | 1 μM |
| 167 | 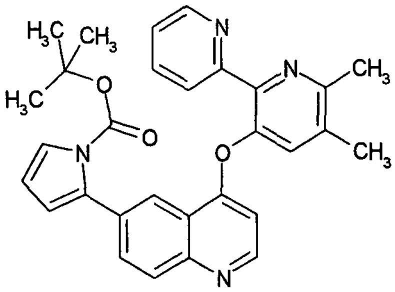 | 85 | 35 | |
| 168 | 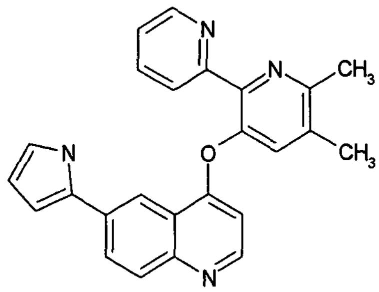 | 100 | 92 | |
| 169 | 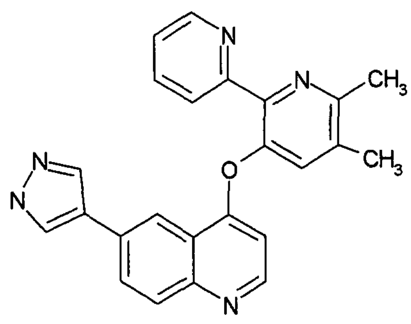 | 100 | 100 | |
| 170 | 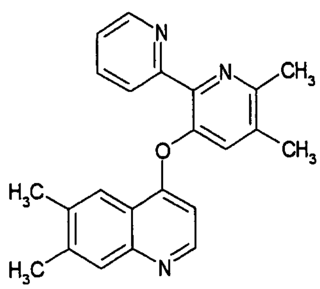 | 100 | 98 | |
| 171 | 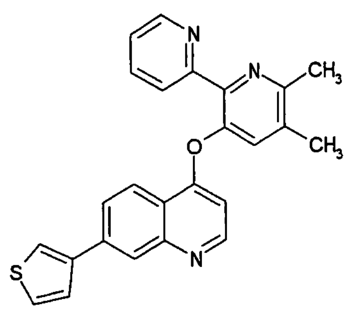 | 100 | 100 | |

(continued)

| Compound | Molecular structure | TGFβ inhibition rate, % | | |
|---|---|---|---|---|
| | | 10 μM | 3 μM | 1 μM |
| 172 | 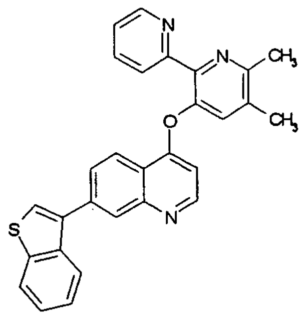 | 90 | 49 | |
| 173 | 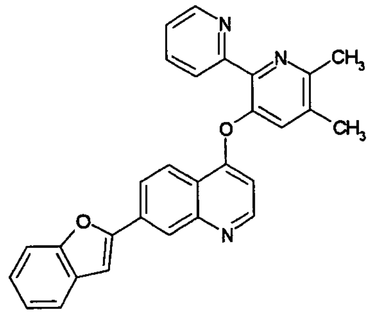 | 92 | 57 | |
| 174 | 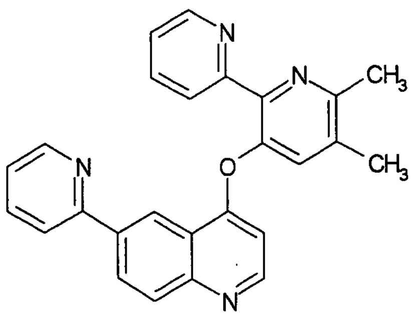 | 100 | 89 | |
| 175 | 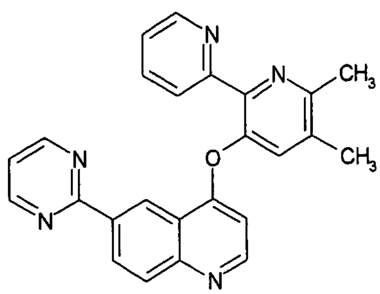 | 100 | 97 | |

(continued)

| Compound | Molecular structure | TGFβ inhibition rate, % | | |
|---|---|---|---|---|
| | | 10 μM | 3 μM | 1 μM |
| 176 | 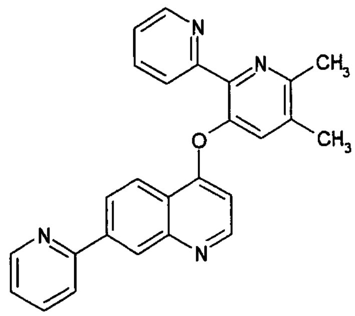 | 100 | 99 | |
| 177 | 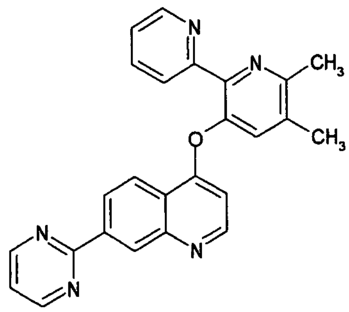 | 100 | 98 | |
| 178 | 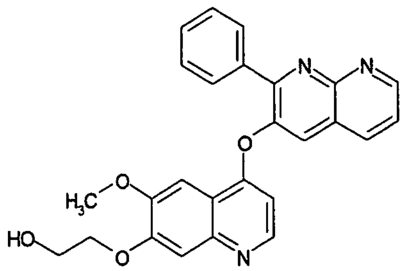 | 100 | 100 | |
| 179 | 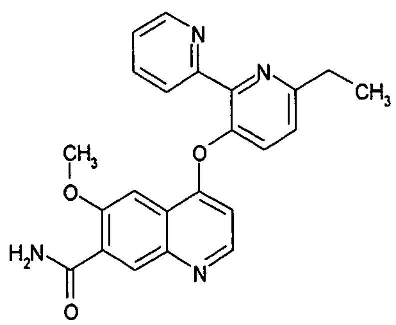 | 100 | 100 | |

(continued)

| Compound | Molecular structure | TGFβ inhibition rate, % | | |
|---|---|---|---|---|
| | | 10 μM | 3 μM | 1 μM |
| 180 | 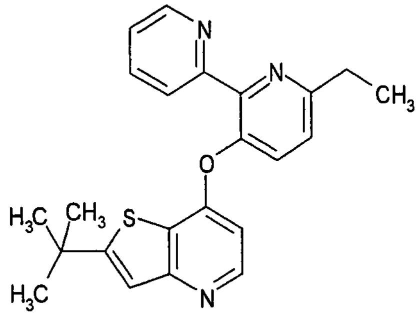 | 98 | 74 | |
| 181 | 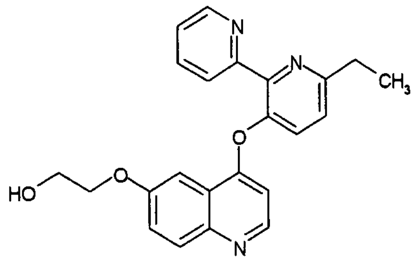 | 100 | 100 | |
| 182 | 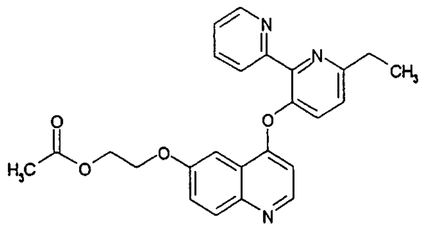 | 100 | 99 | |
| 183 | 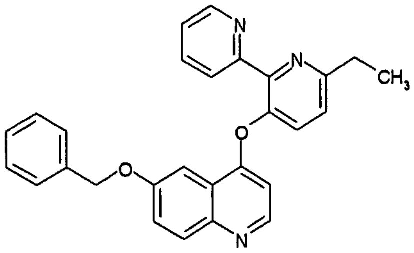 | 100 | 100 | |
| 184 | 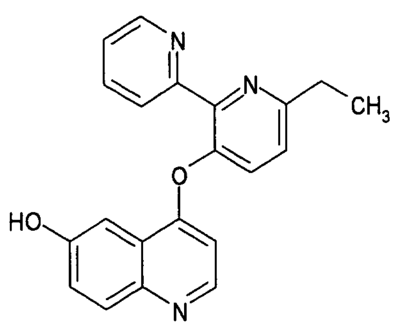 | 100 | 100 | |

(continued)

| Compound | Molecular structure | TGFβ inhibition rate, % | | |
|---|---|---|---|---|
| | | 10 μM | 3 μM | 1 μM |
| 185 | 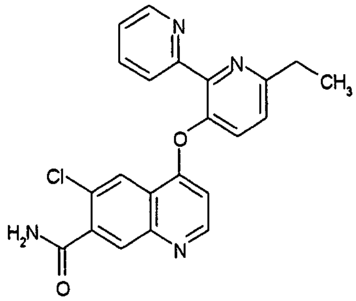 | 100 | 96 | |
| 186 | 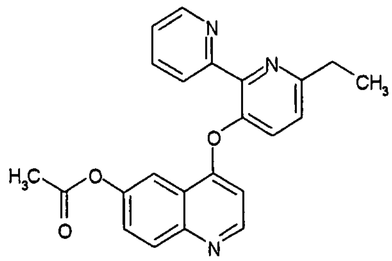 | 100 | 100 | |
| 187 | 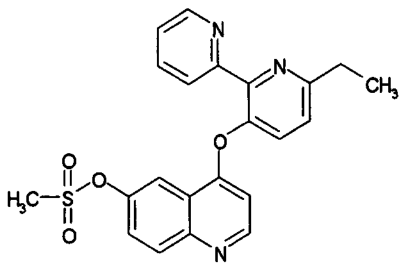 | 100 | 100 | |
| 188 | 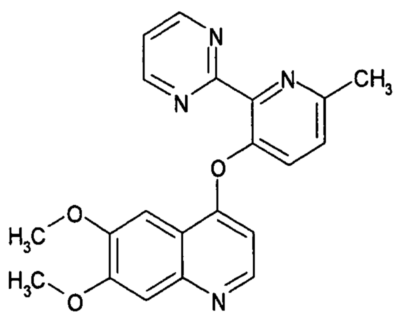 | 100 | 100 | |
| 189 | 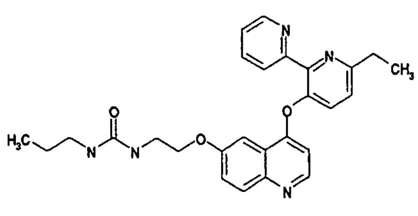 | 99 | 97 | |

(continued)

| Compound | Molecular structure | TGFβ inhibition rate, % | | |
|---|---|---|---|---|
| | | 10 μM | 3 μM | 1 μM |
| 190 | 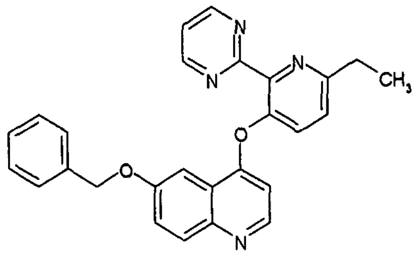 | 100 | 100 | |
| 191 | 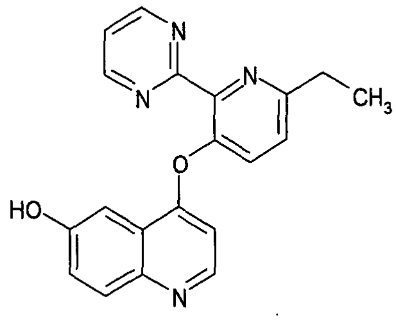 | 100 | 99 | |
| 192 | 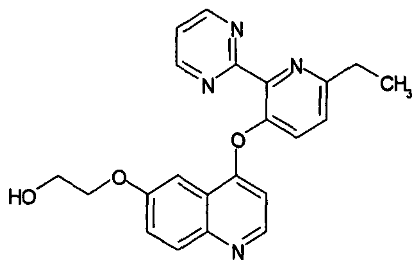 | 100 | 97 | |
| 193 | 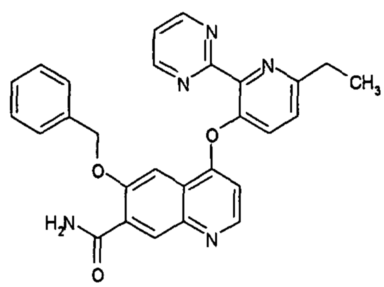 | | 80 | |
| 194 | 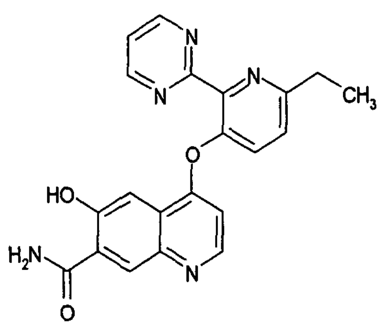 | | 60 | |

(continued)

| Compound | Molecular structure | TGFβ inhibition rate, % | | |
|---|---|---|---|---|
| | | 10 μM | 3 μM | 1 μM |
| 200 | 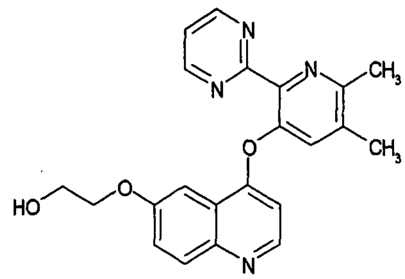 | | 85 | |
| 201 | 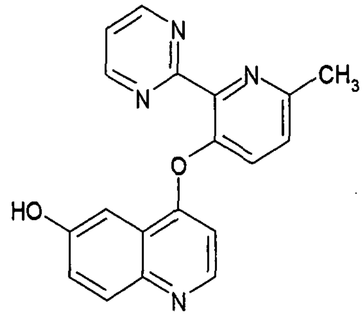 | | 82 | |
| 202 | 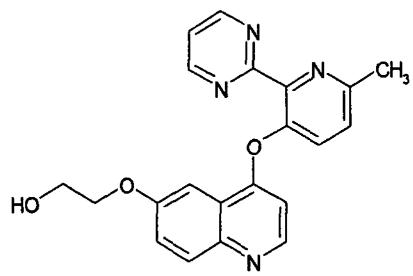 | | 86 | |
| 203 | 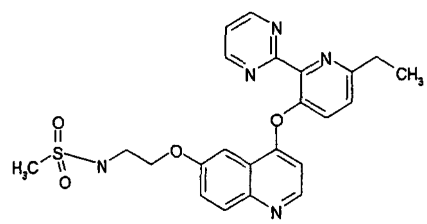 | | 79 | |
| 204 | 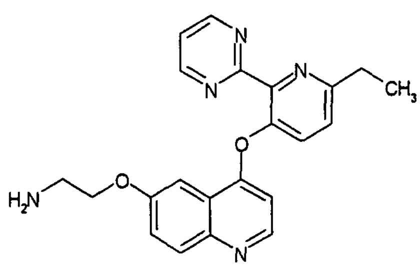 | | 84 | |

(continued)

| Compound | Molecular structure | TGFβ inhibition rate, % | | |
|---|---|---|---|---|
| | | 10 μM | 3 μM | 1 μM |
| 205 | 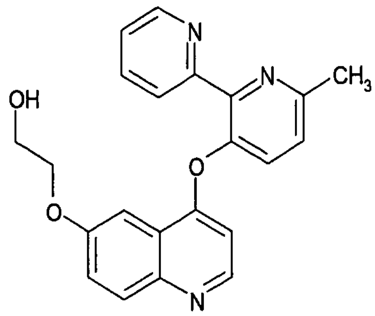 | | 98 | |
| 206 | 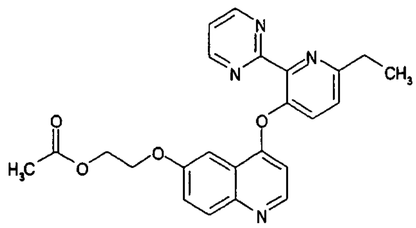 | | 89 | |
| 207 | 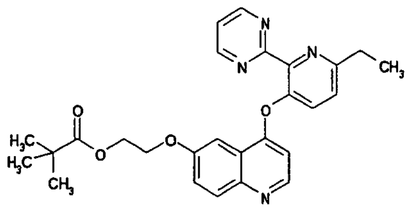 | | 93 | |
| 208 | 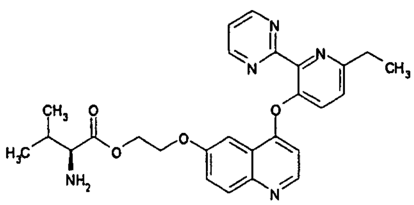 | | 75 | |
| 209 | 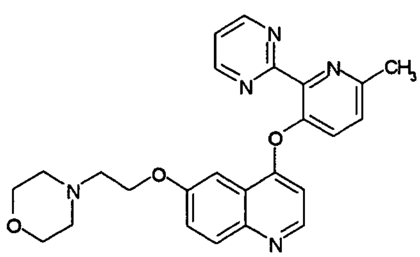 | | 54 | |

(continued)

| Compound | Molecular structure | TGFβ inhibition rate, % | | |
|---|---|---|---|---|
| | | 10 μM | 3 μM | 1 μM |
| 210 | 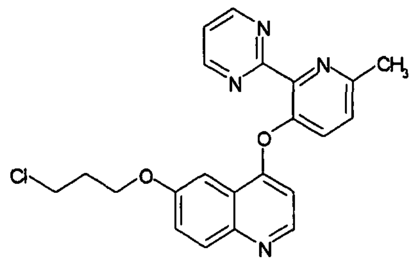 | | 87 | |
| 211 | 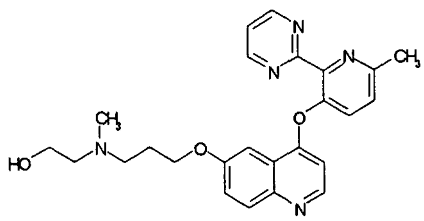 | | 54 | |
| 212 | 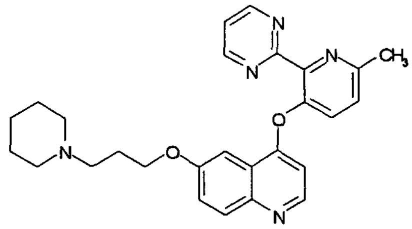 | | 55 | |
| 213 | 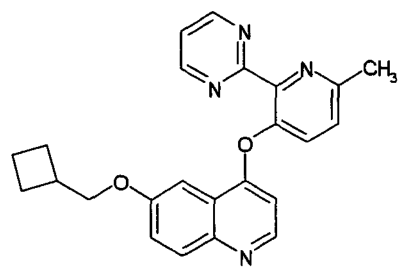 | | 98 | |
| 214 | 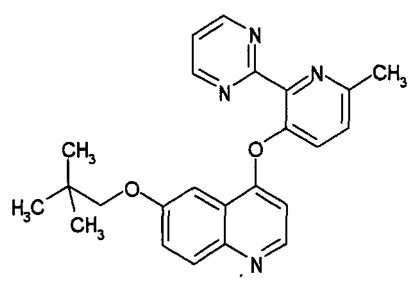 | | 90 | |

(continued)

| Compound | Molecular structure | TGFβ inhibition rate, % | | |
|---|---|---|---|---|
| | | 10 μM | 3 μM | 1 μM |
| 215 | 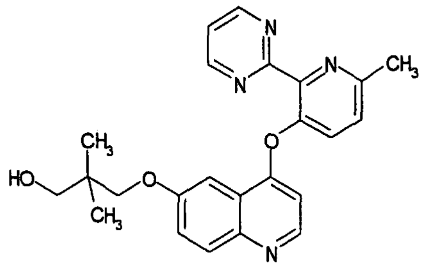 | | 79 | |
| 216 | 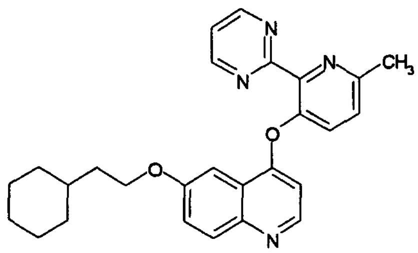 | | 84 | |
| 217 | 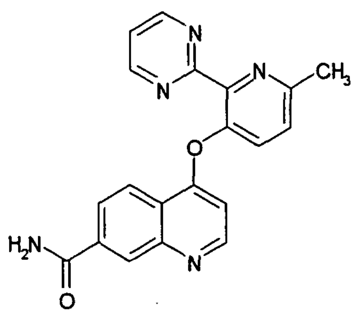 | 100 | 90 | |
| 218 | 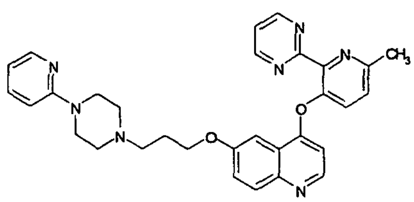 | 100 | 84 | |
| 219 | 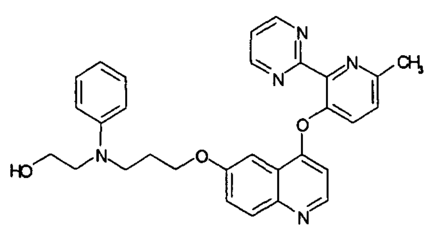 | 100 | 96 | |

(continued)

| Compound | Molecular structure | TGFβ inhibition rate, % | | |
|---|---|---|---|---|
| | | 10 μM | 3 μM | 1 μM |
| 220 | 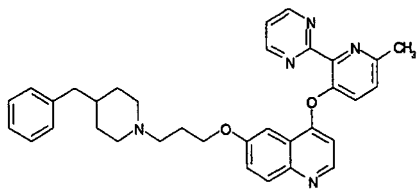 | 100 | 91 | |
| 221 | 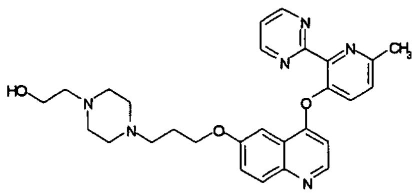 | 79 | 39 | |
| 222 | 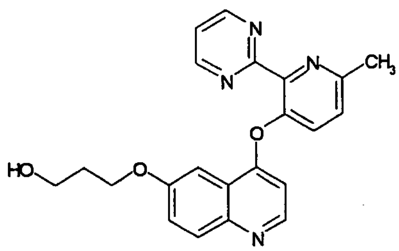 | 100 | 91 | |
| 223 | 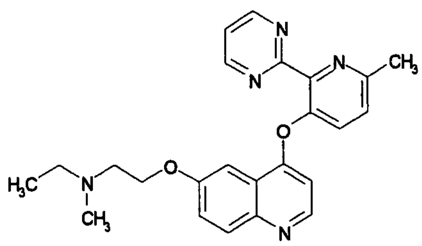 | 90 | 52 | |
| 224 | 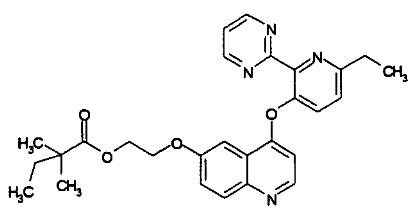 | 100 | 95 | |
| 225 | 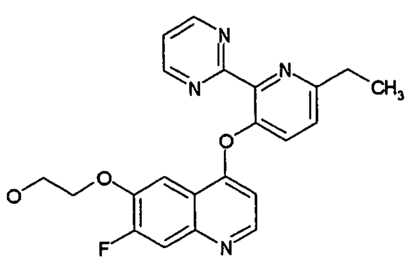 | 99 | 89 | |

Table 1B:

[Table 1B]

| Compound | Molecular structure | TGFβ inhibition rate, % | |
|---|---|---|---|
| | | 10 μM | 3 μM |
| r1 | 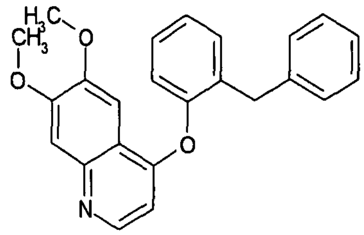 | 53 | 5 |
| r2 | 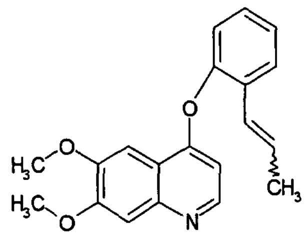 | 88 | 13 |
| r3 | 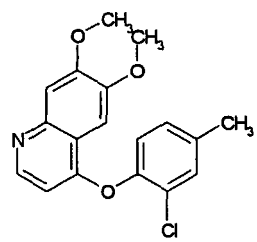 | 75 | 0 |
| r4 | 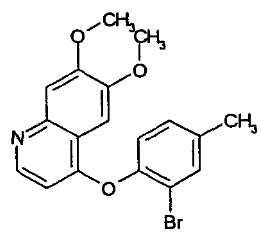 | 78 | 20 |
| r 5 | 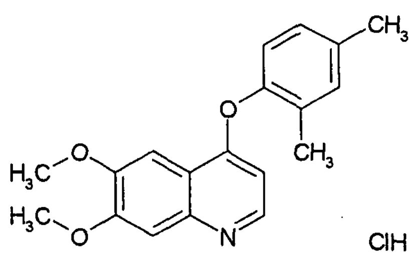 | 56 | 13 |

(continued)

| Compound | Molecular structure | TGFβ inhibition rate, % | |
|---|---|---|---|
| | | 10 μM | 3 μM |
| r6 | 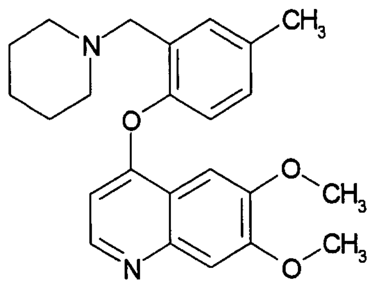 | 72 | 30 |
| r7 | 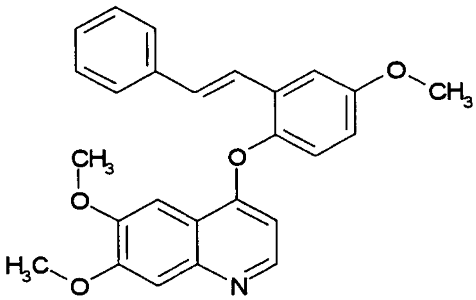 | 54 | 13 |
| r8 | 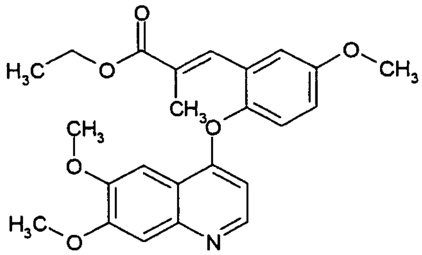 | 59 | 10 |
| r9 | 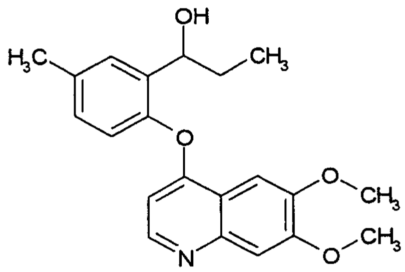 | 77 | 29 |
| r10 | 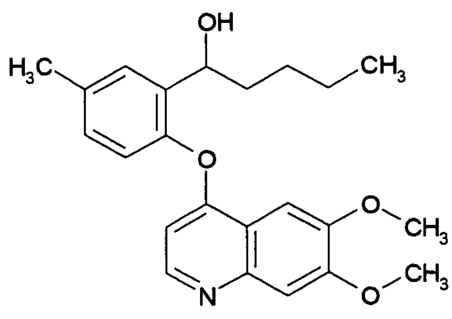 | 87 | 38 |

(continued)

| Compound | Molecular structure | TGFβ inhibition rate, % | |
|---|---|---|---|
| | | 10 μM | 3 μM |
| r 11 | 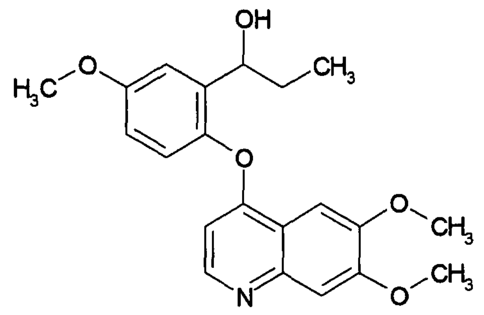 | 66 | 28 |
| r12 | 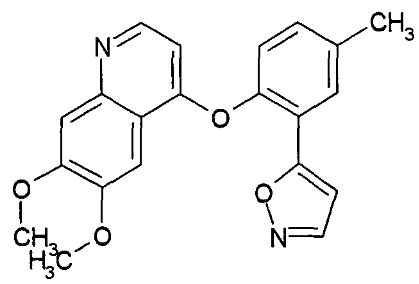 | 89 | 21 |
| r13 | 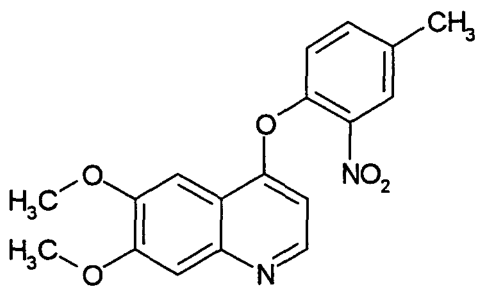 | 78 | 28 |
| r14 | 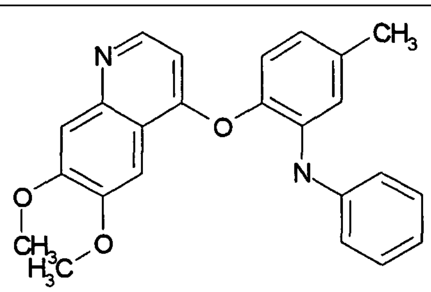 | 64 | 15 |
| r15 | 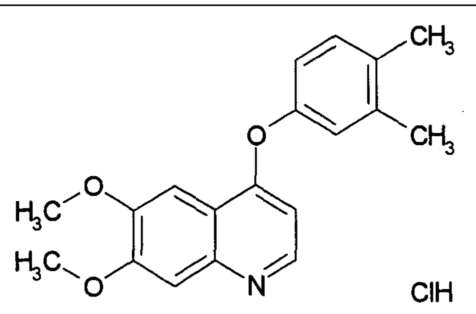 | 84 | 41 |

(continued)

| Compound | Molecular structure | TGFβ inhibition rate, % | |
|---|---|---|---|
| | | 10 μM | 3 μM |
| r16 | 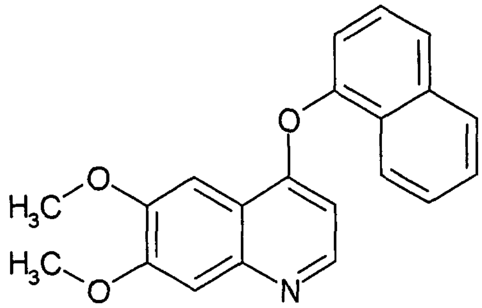 | 62 | 0 |
| r17 | 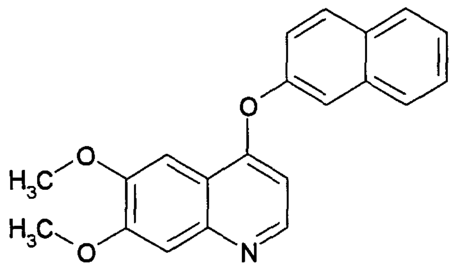 | 80 | 15 |
| r18 | 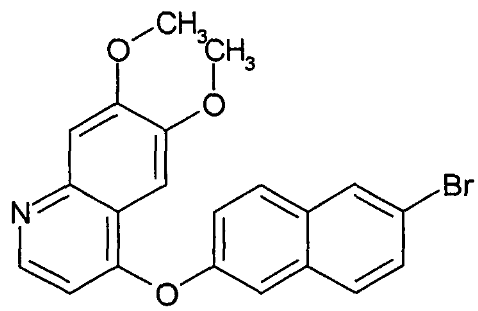 | 69 | 15 |
| r19 | 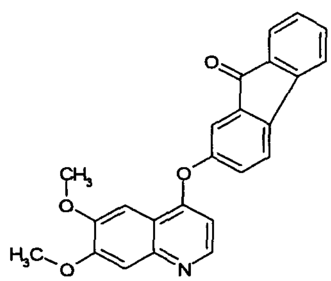 | 95 | 73 |
| r 20 | 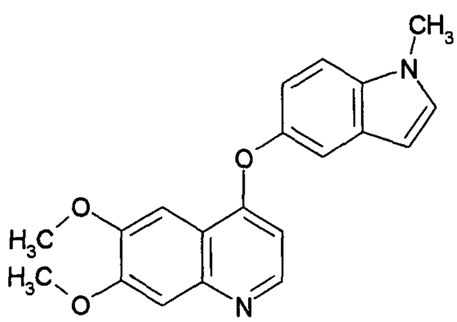 | 69 | 19 |

(continued)

| Compound | Molecular structure | TGFβ inhibition rate, % | |
|---|---|---|---|
| | | 10 μM | 3 μM |
| r 21 | 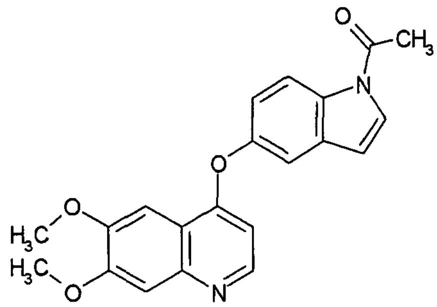 | 71 | 16 |
| r 22 | 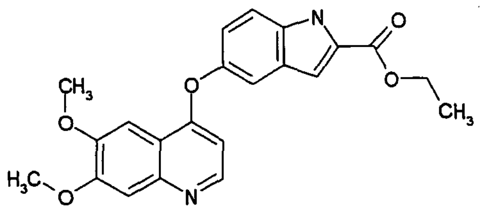 | 81 | 11 |
| r 23 | 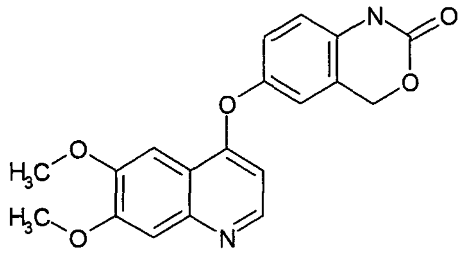 | 91 | 56 |
| r 24 | 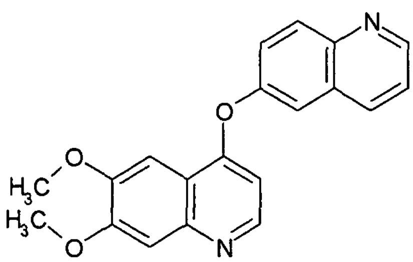 | 91 | 66 |
| r 25 | | | |
| r 26 | 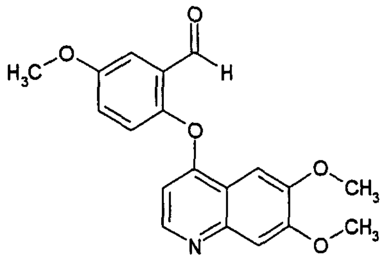 | 73 | 20 |

(continued)

| Compound | Molecular structure | TGFβ inhibition rate, % | |
|---|---|---|---|
| | | 10 μM | 3 μM |
| r 27 | 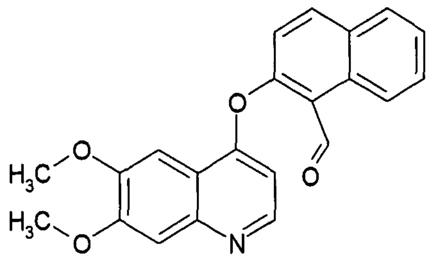 | 93 | 0 |
| r 28 | 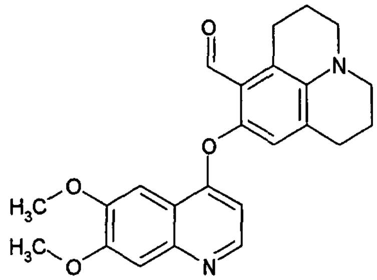 | 53 | 0 |
| r 29 | 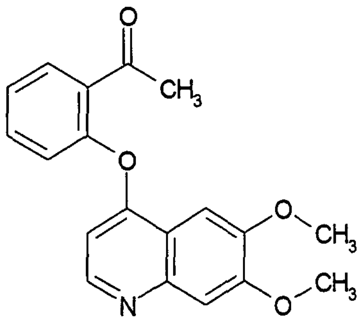 | 67 | 0 |
| r 30 | 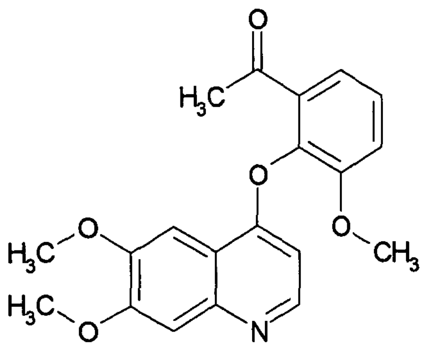 | 61 | 0 |

(continued)

| Compound | Molecular structure | TGFβ inhibition rate, % | |
|---|---|---|---|
| | | 10 μM | 3 μM |
| r 31 | 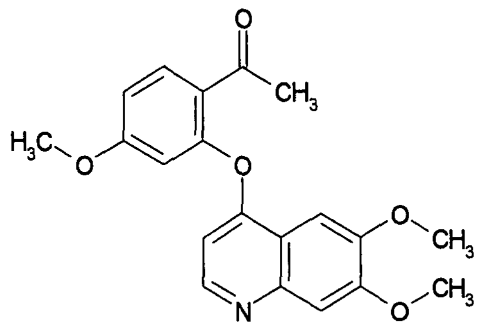 | 98 | 49 |
| r 32 | 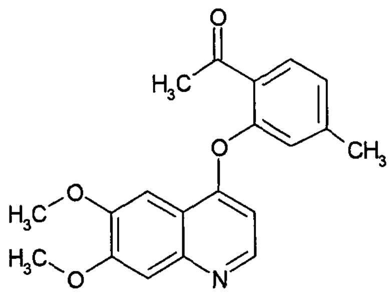 | 98 | 81 |
| r 33 | 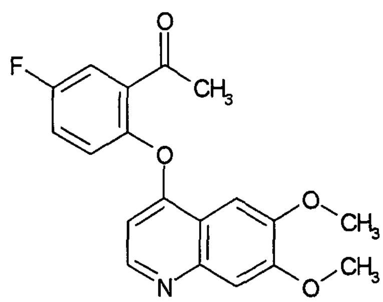 | 94 | 27 |
| r 34 | 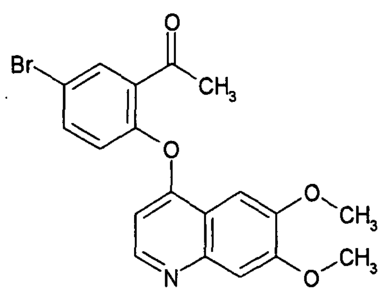 | 78 | 0 |
| r 35 | 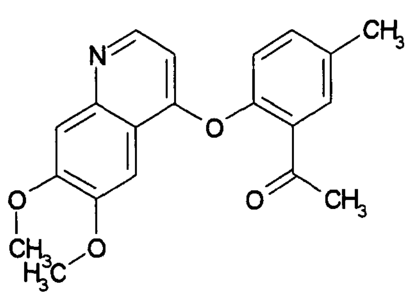 | 89 | 49 |

(continued)

| Compound | Molecular structure | TGFβ inhibition rate, % | |
|---|---|---|---|
| | | 10 μM | 3 μM |
| r 36 | 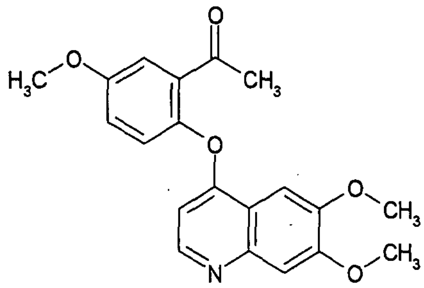 | 100 | 95 |
| r 37 | 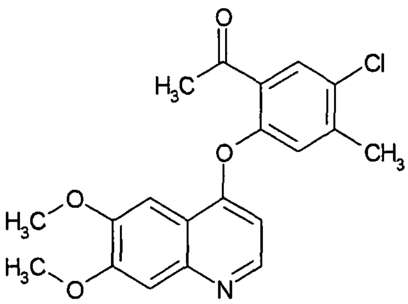 | 100 | 94 |
| r 38 | 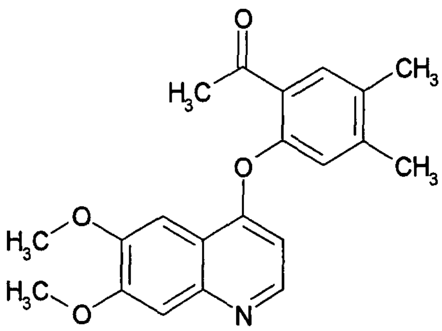 | 100 | 98 |
| r 39 | 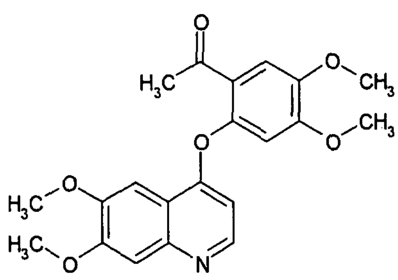 | 91 | 0 |

(continued)

| Compound | Molecular structure | TGFβ inhibition rate, % | |
|---|---|---|---|
| | | 10 μM | 3 μM |
| r 40 | 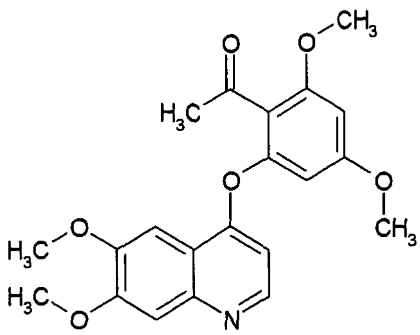 | 83 | 12 |
| r 41 | 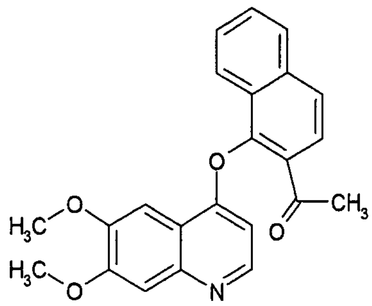 | 97 | 57 |
| r 42 | 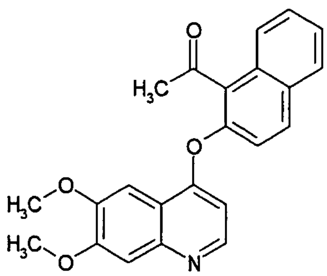 | 93 | 90 |
| r 43 | 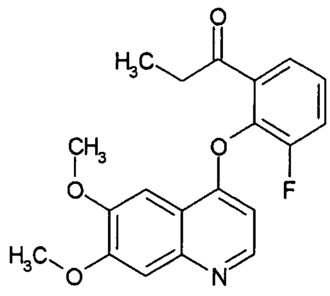 | 66 | 0 |

(continued)

| Compound | Molecular structure | TGFβ inhibition rate, % | |
|---|---|---|---|
| | | 10 μM | 3 μM |
| r 44 | 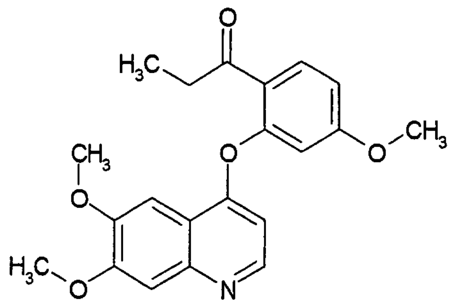 | 100 | 97 |
| r 45 | 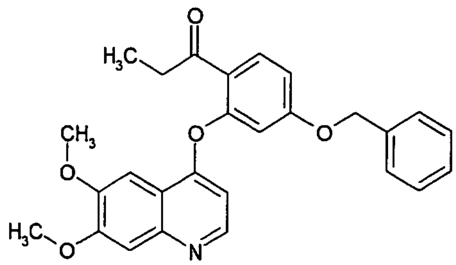 | 67 | 0 |
| r 46 | 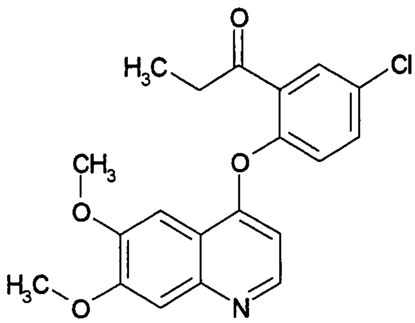 | 100 | 70 |
| r 47 | 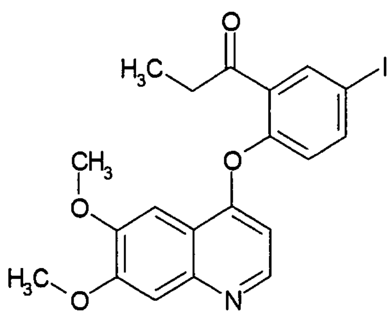 | 98 | 45 |

(continued)

| Compound | Molecular structure | TGFβ inhibition rate, % | |
|---|---|---|---|
| | | 10 μM | 3 μM |
| r 48 | 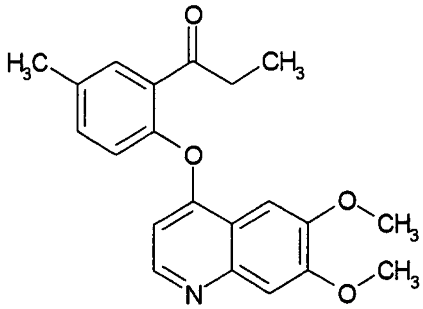 | 100 | 98 |
| r 49 | 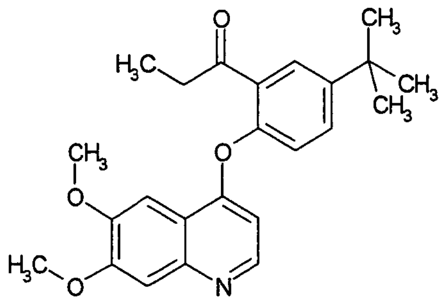 | 62 | 0 |
| r 50 | 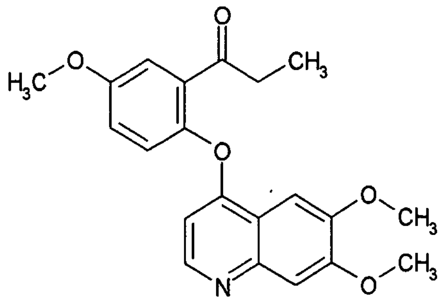 | 100 | 100 |
| r 51 | 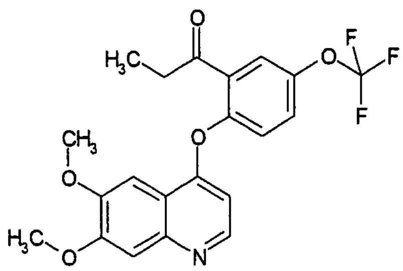 | 99 | 71 |
| r 52 | 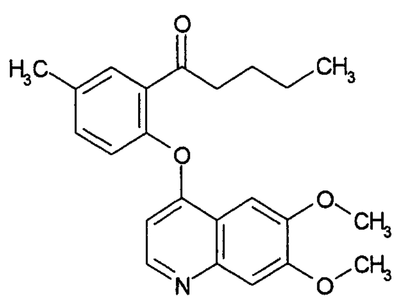 | 100 | 97 |

(continued)

| Compound | Molecular structure | TGFβ inhibition rate, % | |
|---|---|---|---|
| | | 10 μM | 3 μM |
| r 53 | 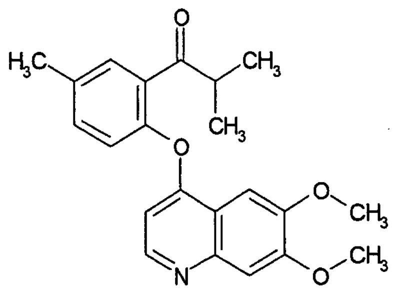 | 100 | 97 |
| r 54 | 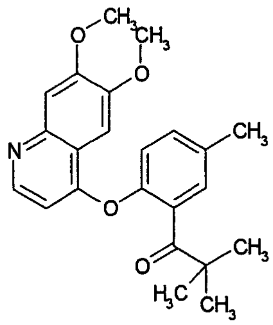 | 73 | 13 |
| r 55 | 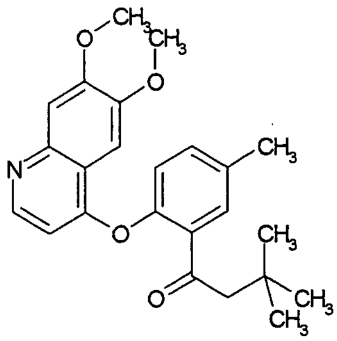 | 98 | 53 |
| r 56 | 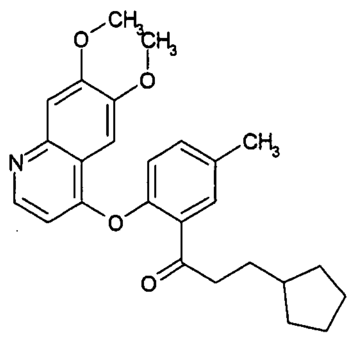 | 84 | 0 |

(continued)

| Compound | Molecular structure | TGFβ inhibition rate, % | |
|---|---|---|---|
| | | 10 μM | 3 μM |
| r 57 | 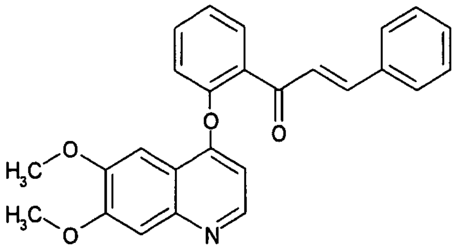 | 55 | 10 |
| r 58 | 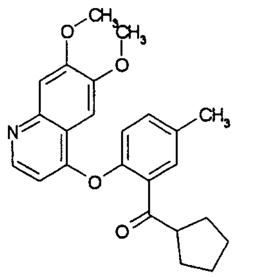 | 100 | 90 |
| r 59 | 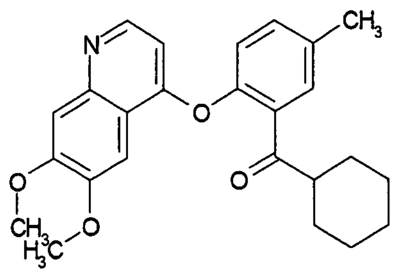 | 99 | 65 |
| r 60 | 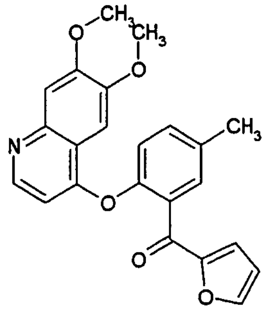 | 98 | 63 |
| r 61 | 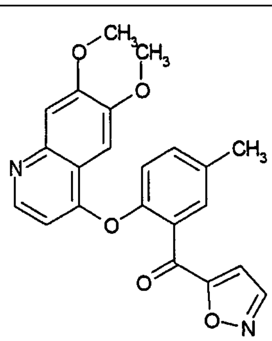 | 98 | 61 |

(continued)

| Compound | Molecular structure | TGFβ inhibition rate, % | |
|---|---|---|---|
| | | 10 μM | 3 μM |
| r 62 | 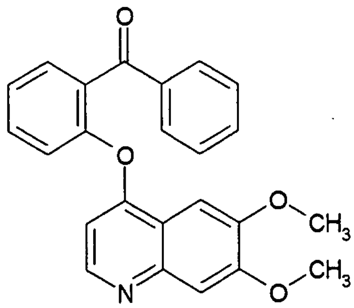 | 85 | 43 |
| r 63 | 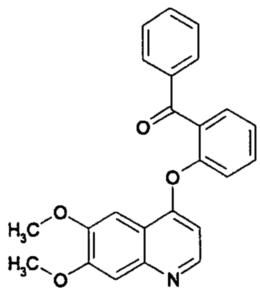 ClH | 91 | 57 |
| r 64 | 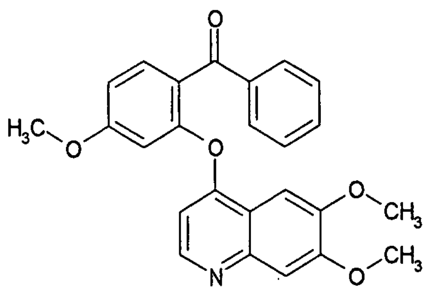 | 98 | 67 |
| r 65 | 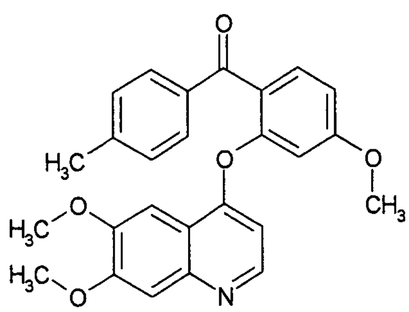 | 100 | 58 |
| r 66 | 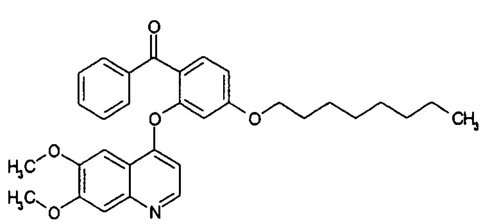 | 86 | 22 |

(continued)

| Compound | Molecular structure | TGFβ inhibition rate, % | |
|---|---|---|---|
| | | 10 μM | 3 μM |
| r 67 | 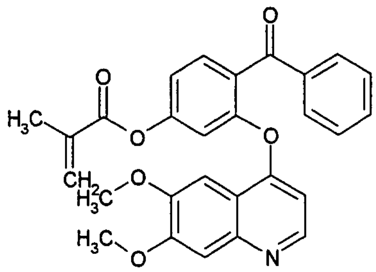 | 93 | 4 |
| r 68 | 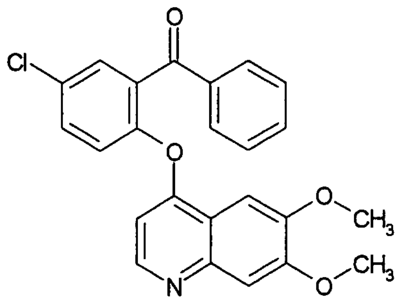 | 100 | 89 |
| r 69 | 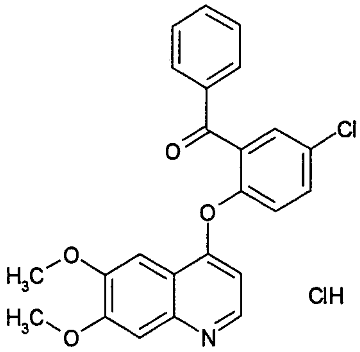 | 100 | 100 |
| r 70 | 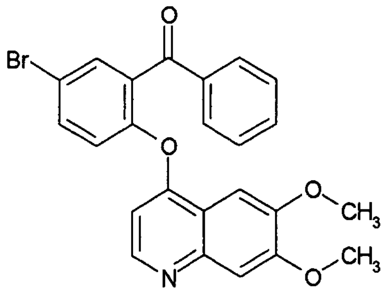 | 100 | 73 |
| r 71 | 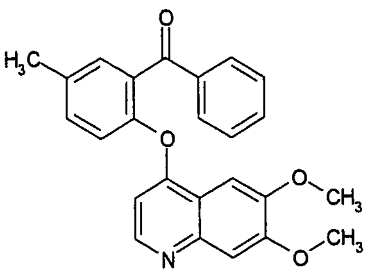 | 100 | 79 |

(continued)

| Compound | Molecular structure | TGFβ inhibition rate, % | |
|---|---|---|---|
| | | 10 μM | 3 μM |
| r 72 | 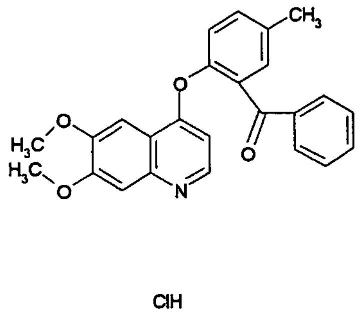 | 100 | 98 |
| r 73 | 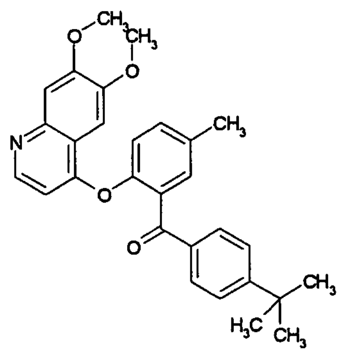 | 81 | 24 |
| r 74 | 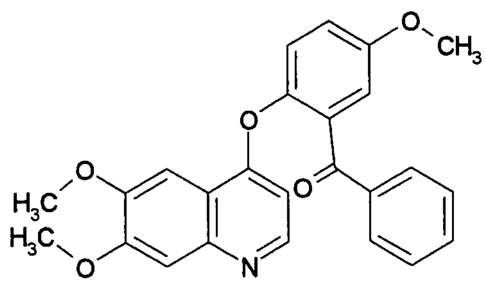 | 84 | 27 |
| r 75 | 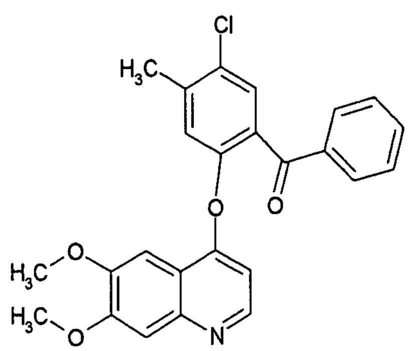 | 100 | 97 |

(continued)

| Compound | Molecular structure | TGFβ inhibition rate, % | |
|---|---|---|---|
| | | 10 μM | 3 μM |
| r 76 | 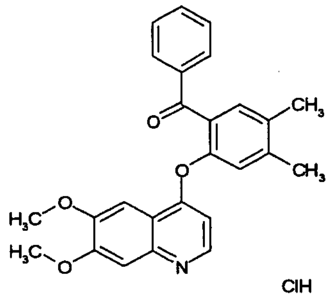 | 100 | 95 |
| r 77 | 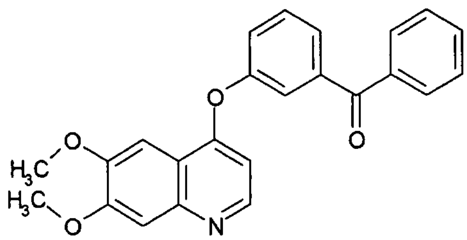 | 46 | 0 |
| r 78 | 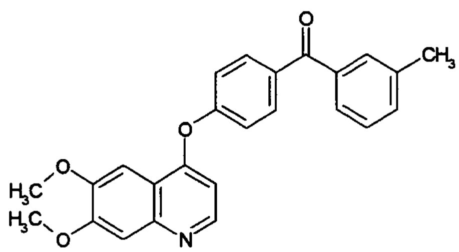 | 57 | 0 |
| r 79 | 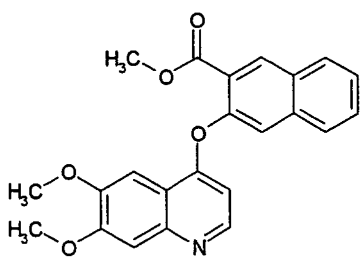 | 100 | 97 |
| r 80 | 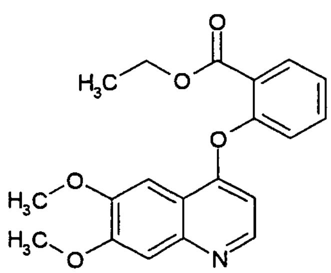 | 95 | 62 |

(continued)

| Compound | Molecular structure | TGFβ inhibition rate, % | |
|---|---|---|---|
| | | 10 μM | 3 μM |
| r 81 | 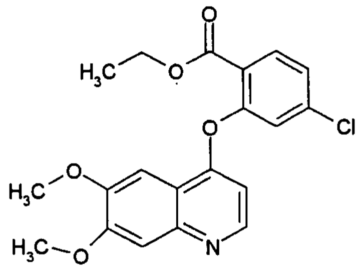 | 100 | 91 |
| r 82 | 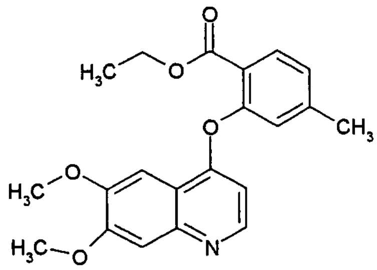 | 99 | 90 |
| r 83 | 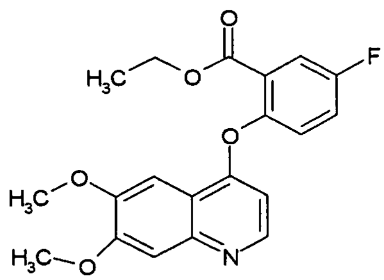 | 81 | 41 |
| r 84 | 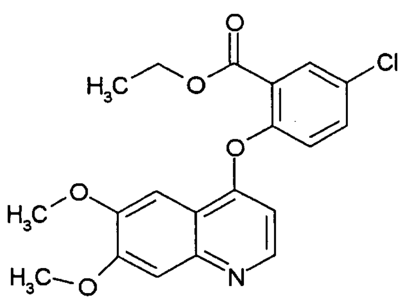 | 100 | 84 |
| r 85 | 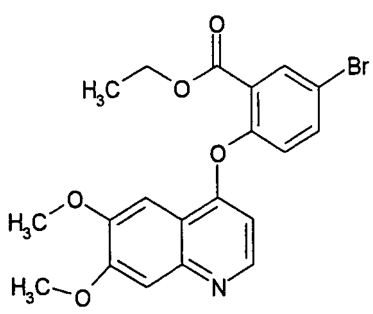 | 100 | 87 |

(continued)

| Compound | Molecular structure | TGFβ inhibition rate, % | |
|---|---|---|---|
| | | 10 μM | 3 μM |
| r 86 | 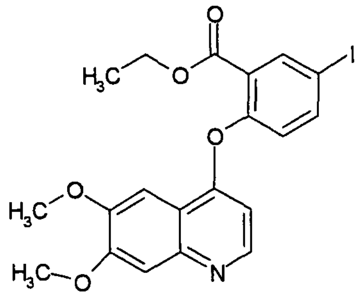 | 99 | 68 |
| r 87 | 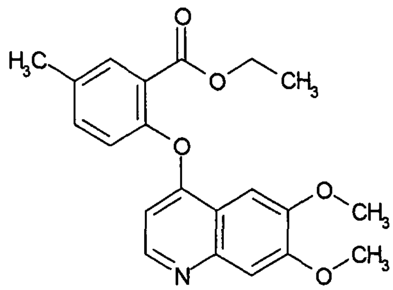 | 99 | 84 |
| r 88 | 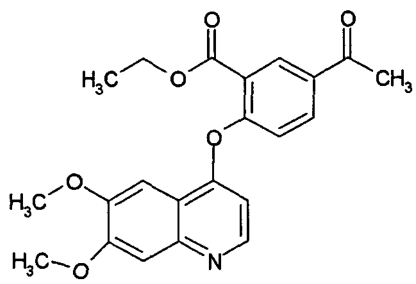 | 94 | 70 |
| r 89 | 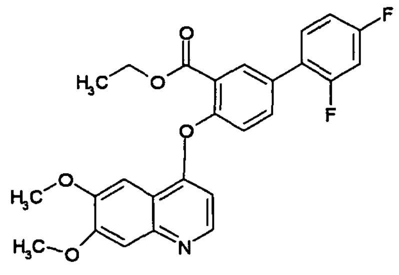 | 63 | 20 |
| r 90 | 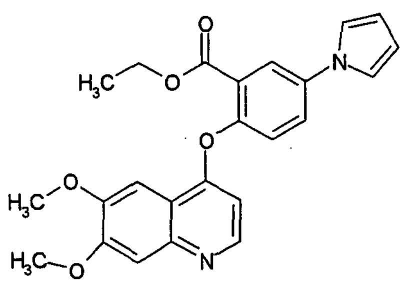 | 95 | 54 |

(continued)

| Compound | Molecular structure | TGFβ inhibition rate, % | |
|---|---|---|---|
| | | 10 μM | 3 μM |
| r 91 | 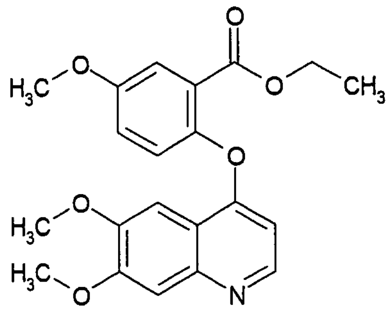 | 100 | 96 |
| r 92 | 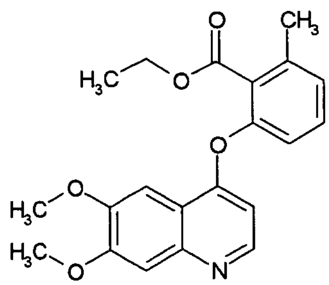 | 82 | 25 |
| r 93 | 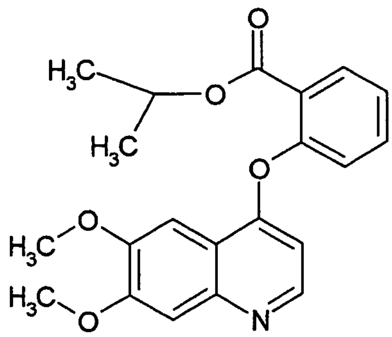 | 88 | 44 |
| r 94 | 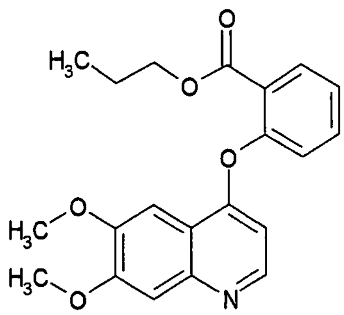 | 97 | 39 |
| r 95 | 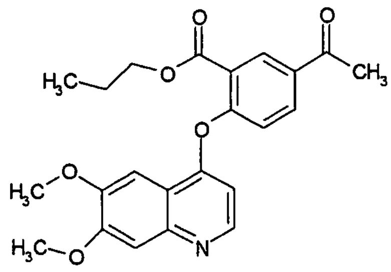 | 96 | 67 |

(continued)

| Compound | Molecular structure | TGFβ inhibition rate, % | |
|---|---|---|---|
| | | 10 μM | 3 μM |
| r 96 | 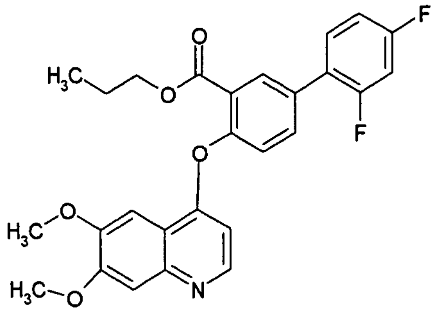 | 65 | 19 |
| r 97 | 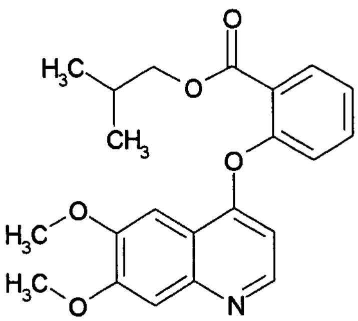 | 92 | 14 |
| r 98 | 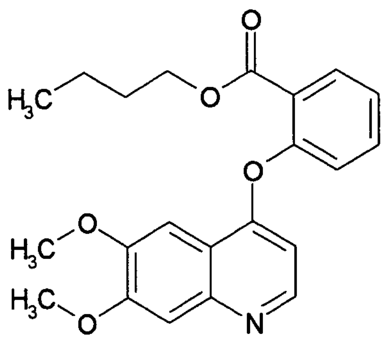 | 93 | 4 |
| r 99 | 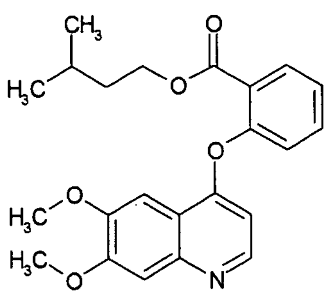 | 92 | 7 |
| r 100 | 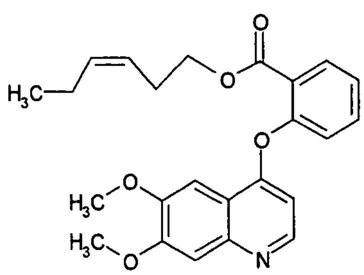 | 82 | 0 |

(continued)

| Compound | Molecular structure | TGFβ inhibition rate, % | |
|---|---|---|---|
| | | 10 μM | 3 μM |
| r 101 | 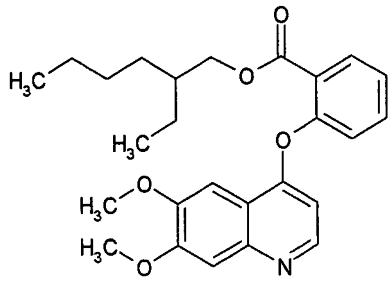 | 64 | 0 |
| r 102 | 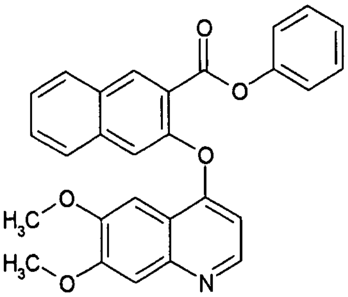 | 95 | 33 |
| r103 | 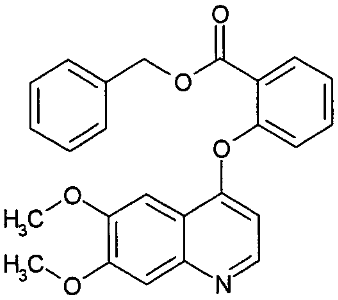 | 84 | 20 |
| r104 | 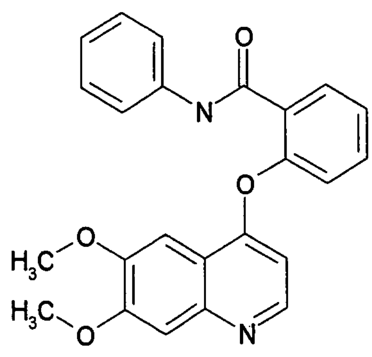 | 58 | 0 |
| r 105 | 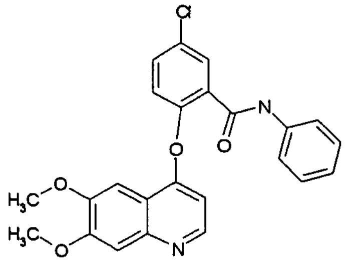 | 88 | 17 |

(continued)

| Compound | Molecular structure | TGFβ inhibition rate, % | |
|---|---|---|---|
| | | 10 μM | 3 μM |
| r 106 | 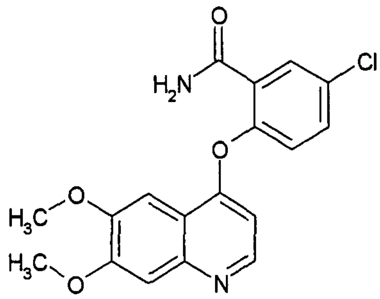 | 80 | 9 |
| r 107 | 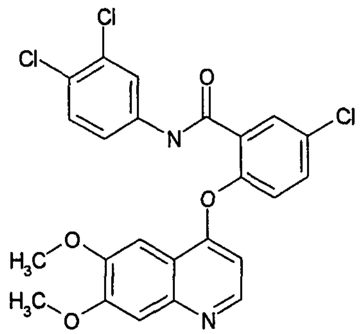 | 82 | 0 |
| r 108 | 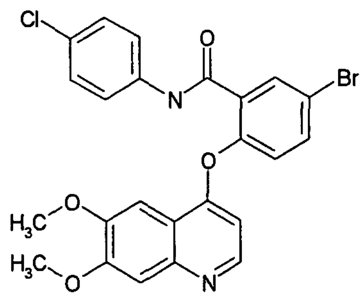 | 73 | 0 |
| r109 | 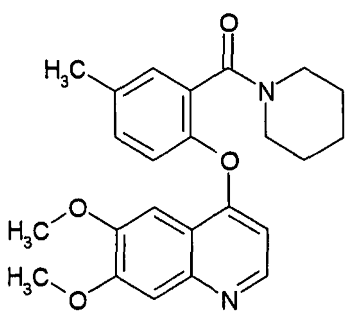 | 66 | 5 |
| r 110 | 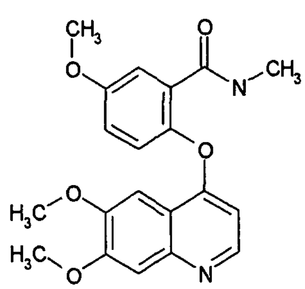 | 93 | 55 |

(continued)

| Compound | Molecular structure | TGFβ inhibition rate, % | |
|---|---|---|---|
| | | 10 μM | 3 μM |
| r 111 | 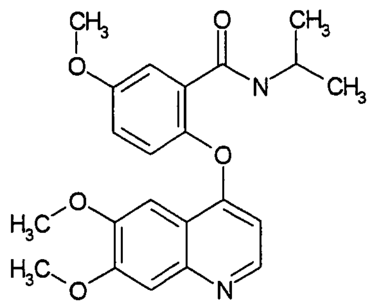 | 92 | 29 |
| r 112 | 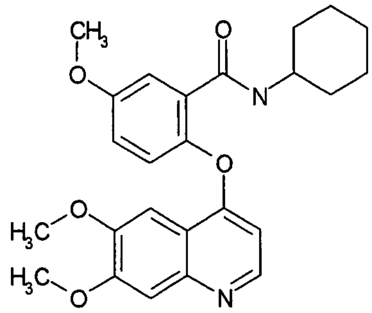 | 91 | 44 |
| r 113 | 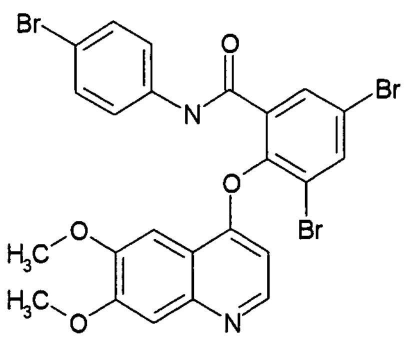 | 96 | 0 |
| r114 | 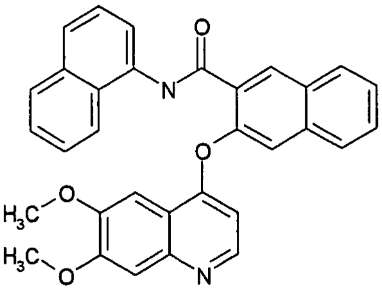 | 96 | 0 |
| r115 | 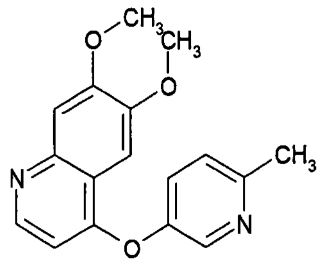 | 97 | 74 |

(continued)

| Compound | Molecular structure | TGFβ inhibition rate, % | |
|---|---|---|---|
| | | 10 μM | 3 μM |
| r116 | 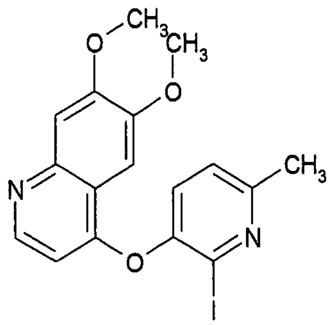 | 100 | 99 |
| r117 | 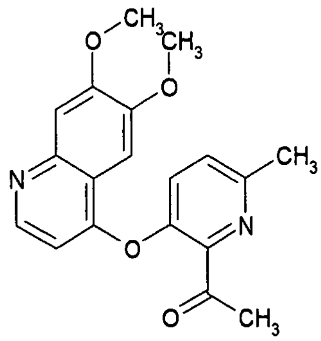 | 98 | 84 |
| r118 | 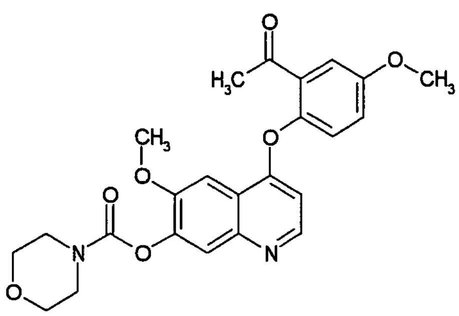 | 100 | 97 |
| r119 | 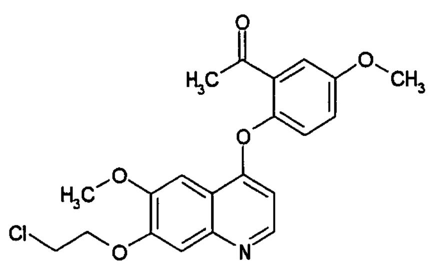 | 97 | 96 |
| r120 | 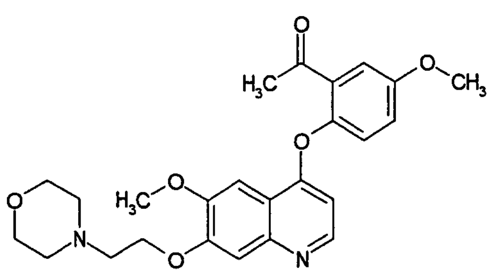 | 100 | 94 |

(continued)

| Compound | Molecular structure | TGFβ inhibition rate, % | |
|---|---|---|---|
| | | 10 μM | 3 μM |
| r 121 | 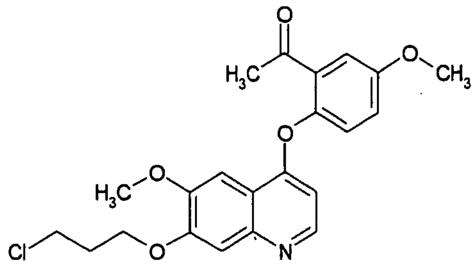 | 100 | 100 |
| r122 | 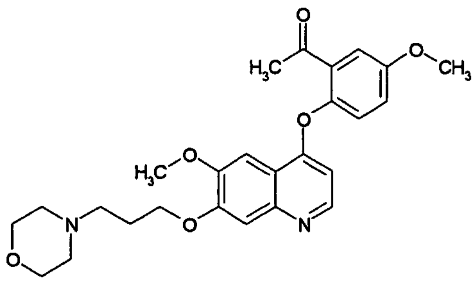 | 100 | 100 |
| r123 | 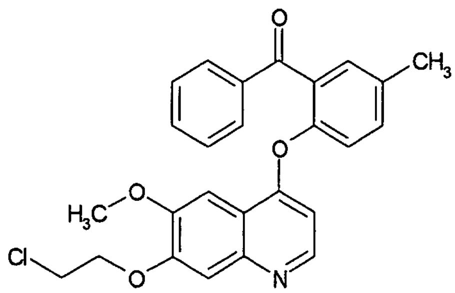 | 100 | 98 |
| r124 | 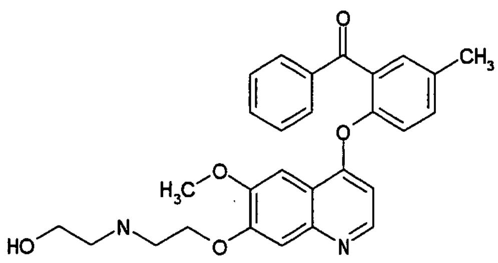 | 100 | 99 |
| r 125 | 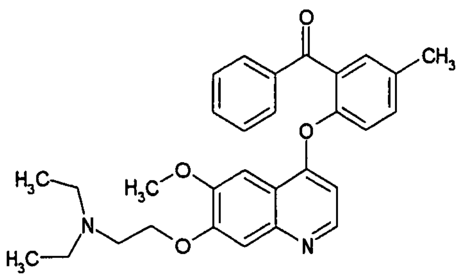 | 100 | 98 |

(continued)

| Compound | Molecular structure | TGFβ inhibition rate, % | |
|---|---|---|---|
| | | 10 μM | 3 μM |
| r 126 | 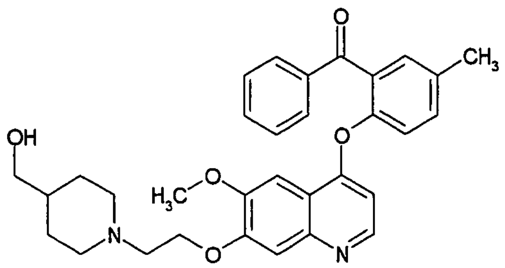 | 100 | 100 |
| r 127 | 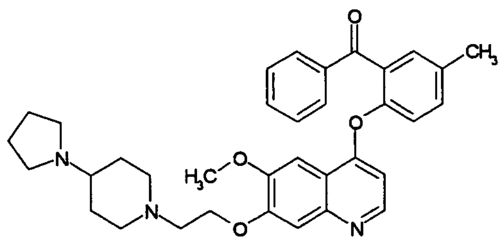 | 100 | 99 |
| r 128 | 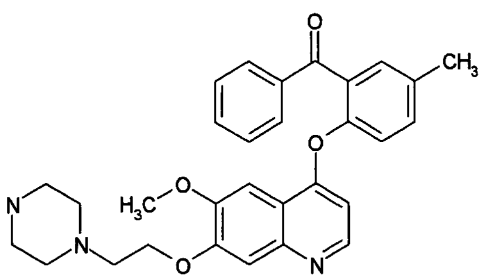 | 100 | 100 |
| r 129 | 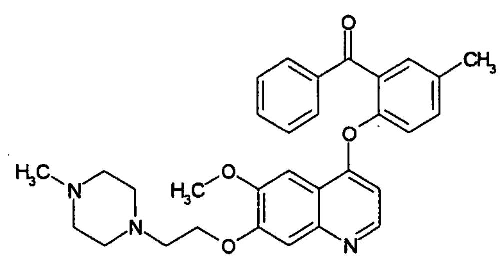 | 100 | 100 |
| r 130 | 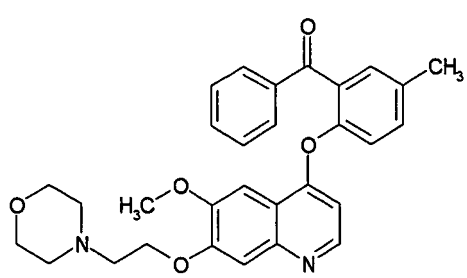 | 100 | 90 |

(continued)

| Compound | Molecular structure | TGFβ inhibition rate, % | |
|---|---|---|---|
| | | 10 μM | 3 μM |
| r 131 | 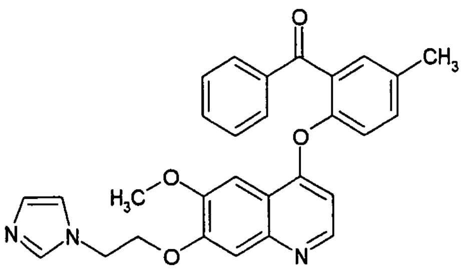 | 100 | 96 |
| r 132 | 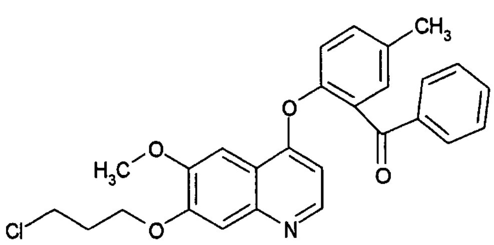 | 99 | 72 |
| r 133 | 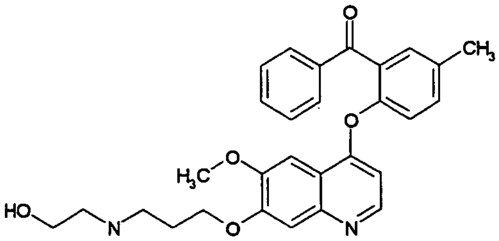 | 100 | 100 |
| r 134 | 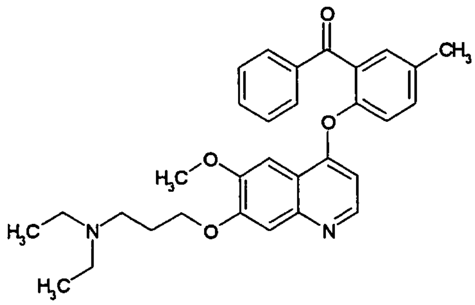 | 100 | 100 |
| r135 | 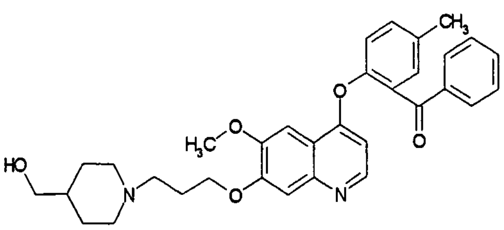 | 100 | 100 |

(continued)

| Compound | Molecular structure | TGFβ inhibition rate, % | |
|---|---|---|---|
| | | 10 μM | 3 μM |
| r 136 | 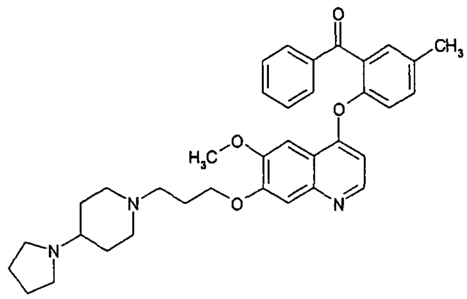 | 100 | 90 |
| r137 | 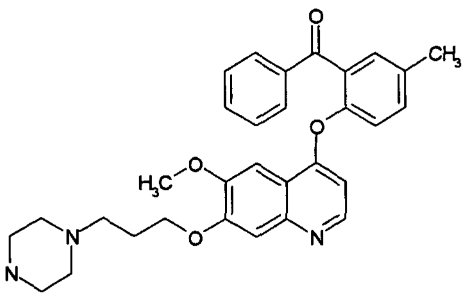 | 100 | 100 |
| r138 | 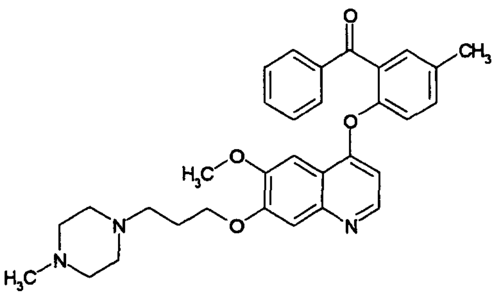 | 100 | 96 |
| r139 | 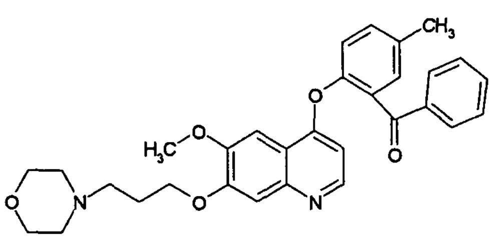 | 100 | 99 |
| r140 | 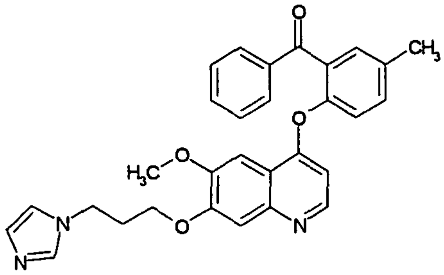 | 100 | 85 |

(continued)

| Compound | Molecular structure | TGFβ inhibition rate, % | |
|---|---|---|---|
| | | 10 μM | 3 μM |
| r 141 | 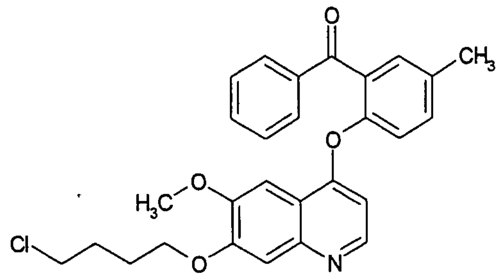 | 89 | 55 |
| r142 | 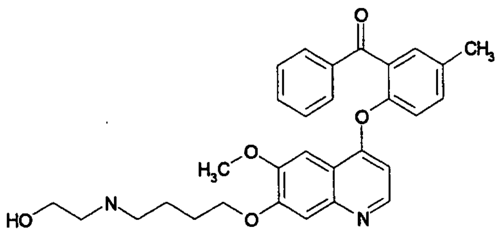 | 100 | 100 |
| r143 | 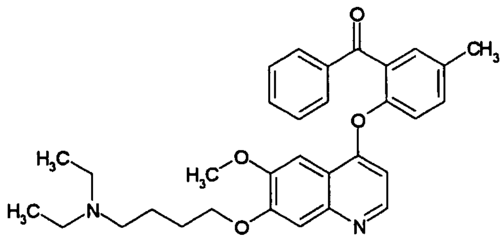 | 100 | 97 |
| r 144 | 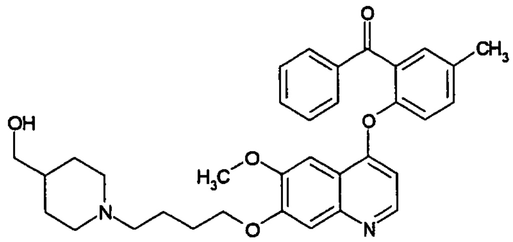 | 100 | 99 |
| r 145 | 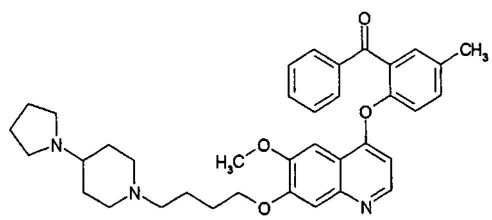 | 100 | 87 |

(continued)

| Compound | Molecular structure | TGFβ inhibition rate, % | |
|---|---|---|---|
| | | 10 μM | 3 μM |
| r 146 | 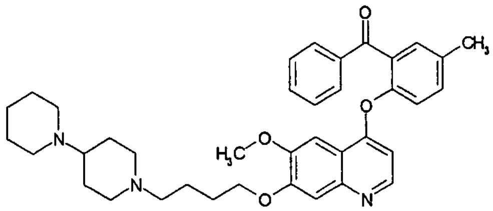 | 100 | 64 |
| r 147 | 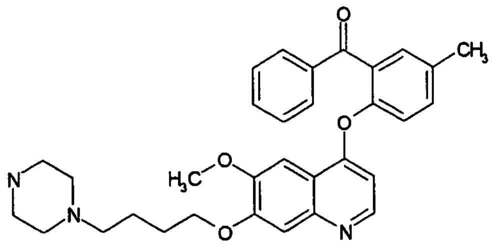 | 100 | 98 |
| r 148 | 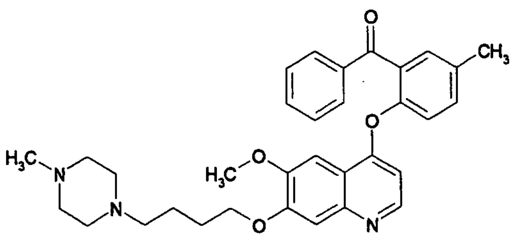 | 100 | 99 |
| r 149 | 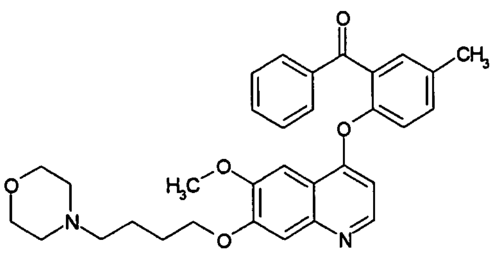 | 100 | 96 |
| r 150 | 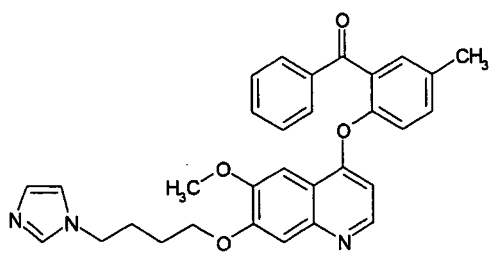 | 100 | 97 |

(continued)

| Compound | Molecular structure | TGFβ inhibition rate, % | |
|---|---|---|---|
| | | 10 μM | 3 μM |
| r 151 | 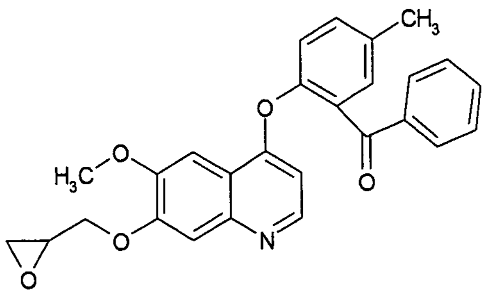 | 100 | 95 |
| r 152 | 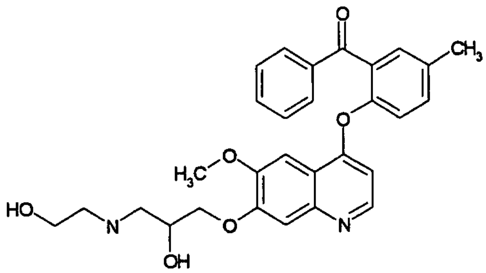 | 100 | 99 |
| r153 | 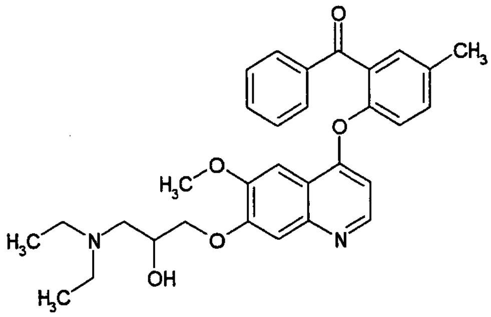 | 100 | 100 |
| r154 | 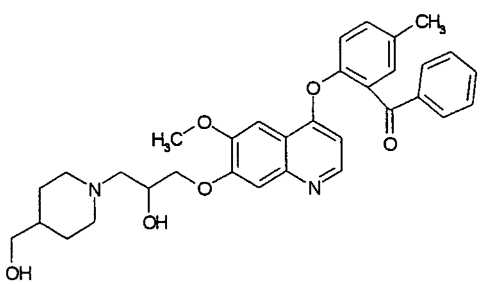 | 100 | 99 |
| r155 | 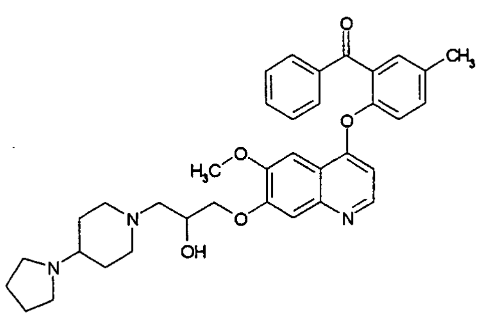 | 100 | 100 |

(continued)

| Compound | Molecular structure | TGFβ inhibition rate, % | |
|---|---|---|---|
| | | 10 μM | 3 μM |
| r 156 | 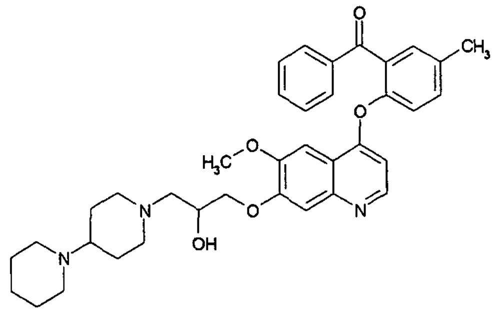 | 100 | 99 |
| r 157 | 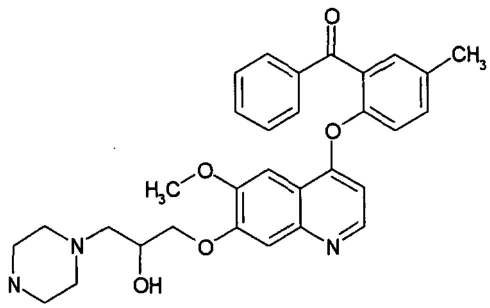 | 100 | 100 |
| r 158 | 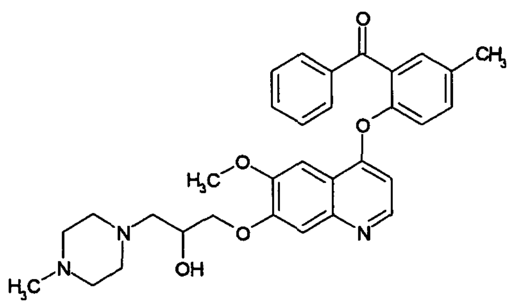 | 100 | 100 |
| r 159 | 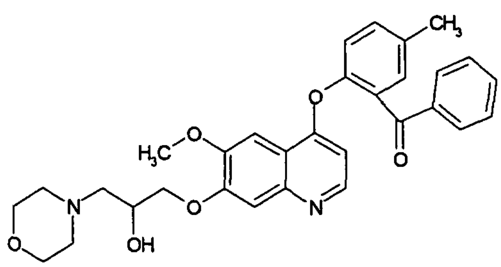 | 100 | 98 |
| r 160 | 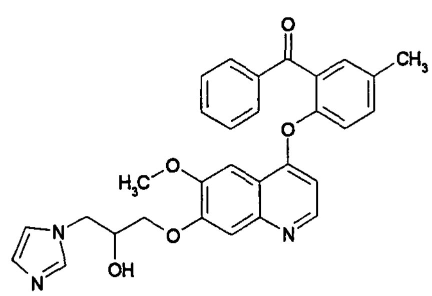 | 100 | 98 |

(continued)

| Compound | Molecular structure | TGFβ inhibition rate, % | |
|---|---|---|---|
| | | 10 μM | 3 μM |
| r 161 | 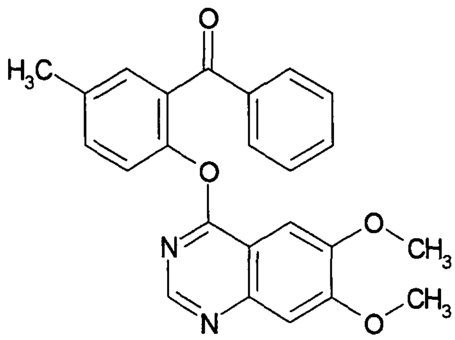 | 83 | 25 |
| r 162 | 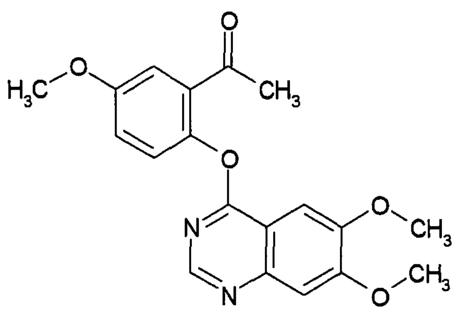 | 54 | 5 |
| r163 | 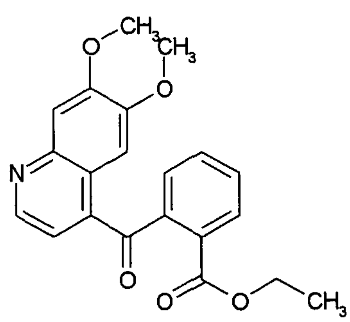 | 67 | 3 |

| Compound | Molecular structure | TGFβ inhibition rate, % | | |
|---|---|---|---|---|
| | | 10 μM | 3 μM | 1 μM |
| r 164 | 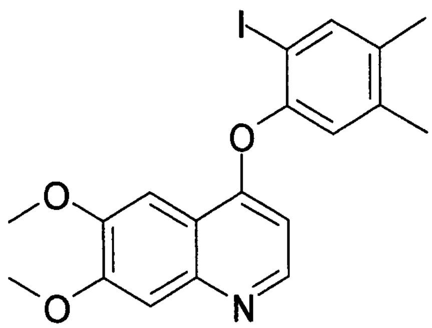 | 98 | 54 | |
| r165 | 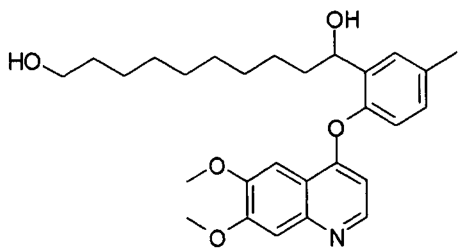 | 89 | 38 | |
| r 166 | 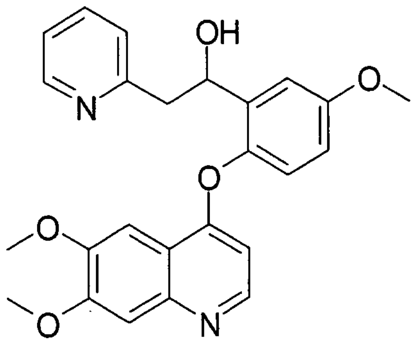 | 67 | 24 | |
| r167 | 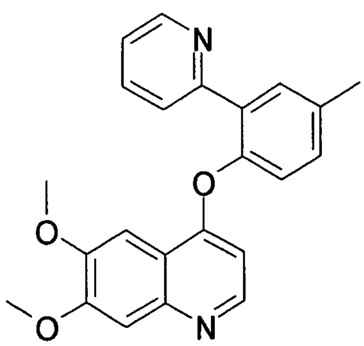 | 100 | 98 | |

(continued)

| Compound | Molecular structure | TGFβ inhibition rate, % | | |
|---|---|---|---|---|
| | | 10 μM | 3 μM | 1 μM |
| r168 | 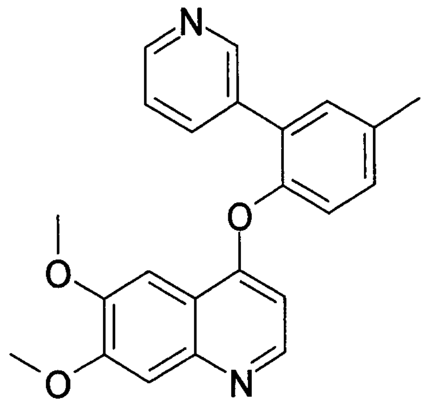 | 74 | 22 | |
| r 169 | 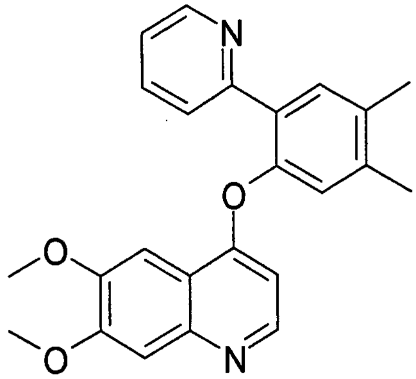 | 100 | 99 | |
| r170 | 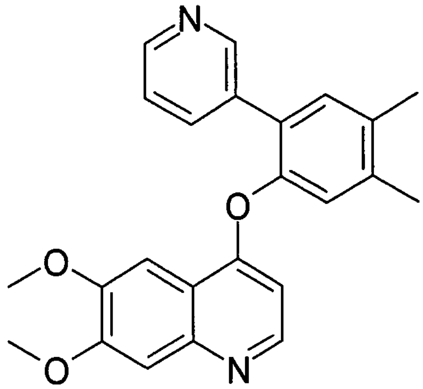 | 100 | 76 | |
| r 171 | 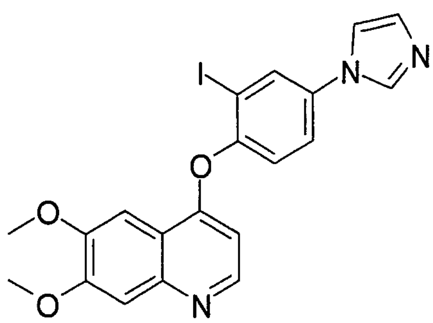 | 76 | 11 | |

(continued)

| Compound | Molecular structure | TGFβ inhibition rate, % | | |
|---|---|---|---|---|
| | | 10 μM | 3 μM | 1 μM |
| r172 | 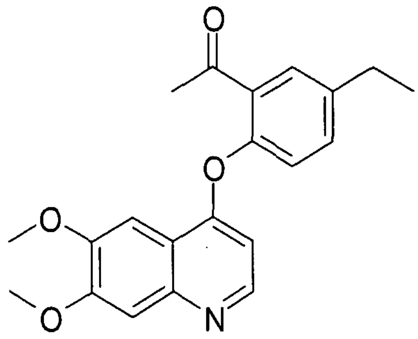 | 99 | 82 | |
| r 173 | 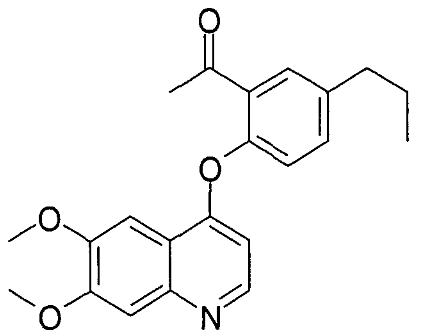 | 100 | 83 | |
| r 174 | 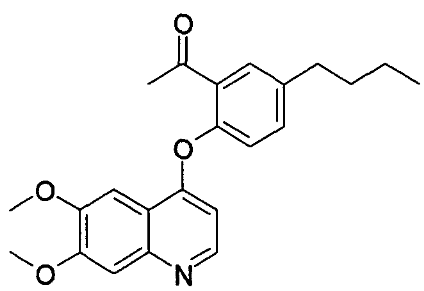 | 96 | 57 | |
| r175 | 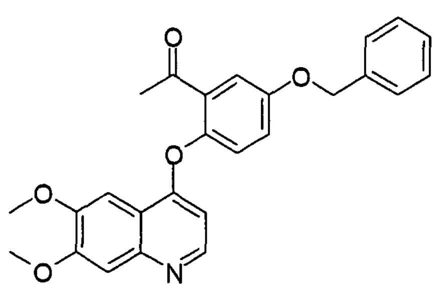 | 60 | 13 | |

(continued)

| Compound | Molecular structure | TGFβ inhibition rate, % | | |
|---|---|---|---|---|
| | | 10 μM | 3 μM | 1 μM |
| r176 | 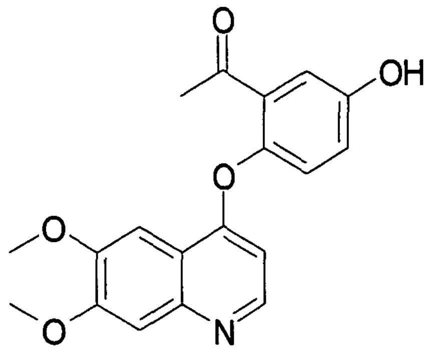 | 67 | 32 | |
| r177 | 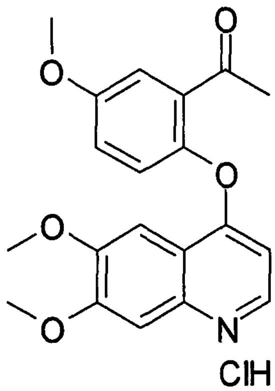 | 100 | 94 | |
| r178 | 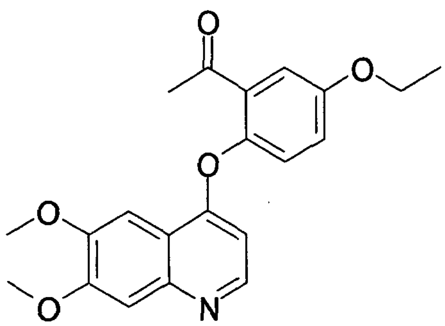 | 97 | 66 | |
| r 179 | 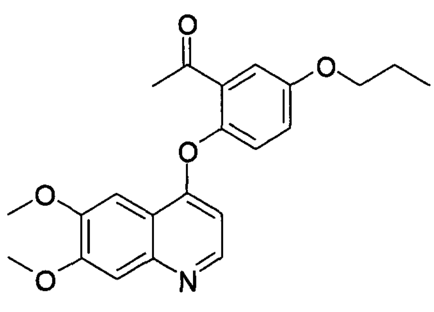 | 68 | 18 | |

(continued)

| Compound | Molecular structure | TGFβ inhibition rate, % | | |
|---|---|---|---|---|
| | | 10 μM | 3 μM | 1 μM |
| r180 | 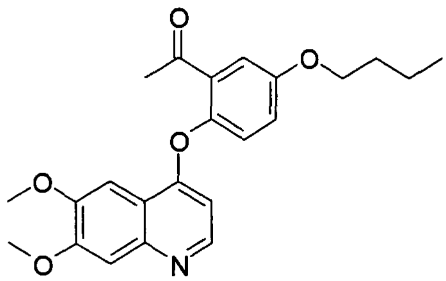 | 55 | -2 | |
| r 181 | 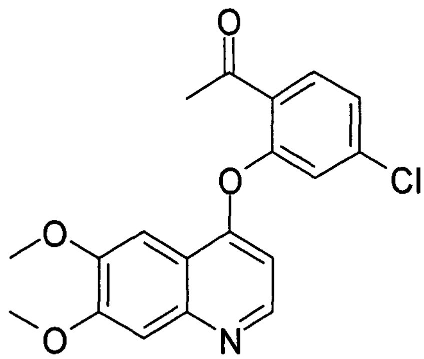 | 82 | 42 | |
| r182 | 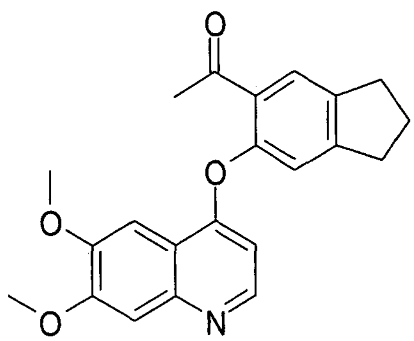 | 96 | 57 | |
| r183 | 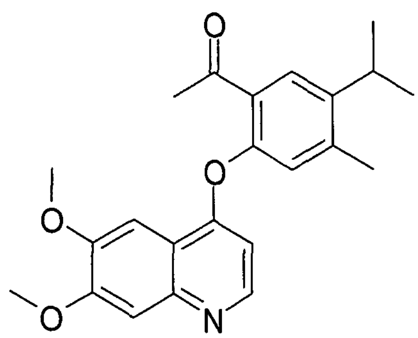 | 100 | 82 | |

(continued)

| Compound | Molecular structure | TGFβ inhibition rate, % | | |
|---|---|---|---|---|
| | | 10 μM | 3 μM | 1 μM |
| r 184 | 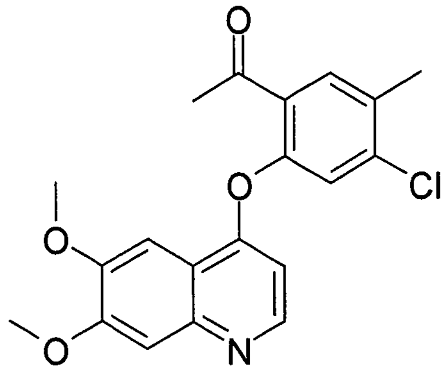 | 100 | 100 | |
| r 185 | 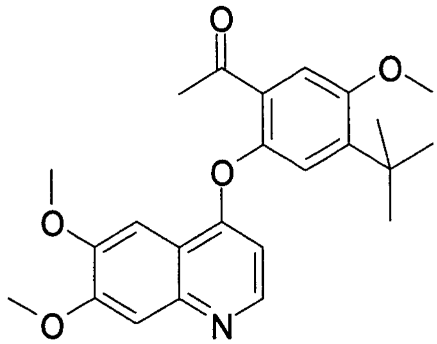 | 59 | 11 | |
| r 186 | 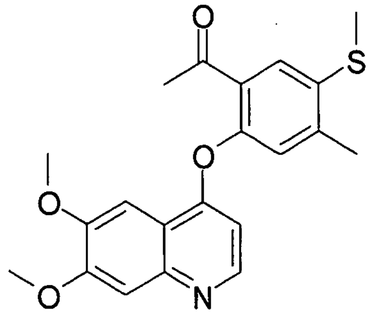 | 100 | 76 | |
| r 187 | 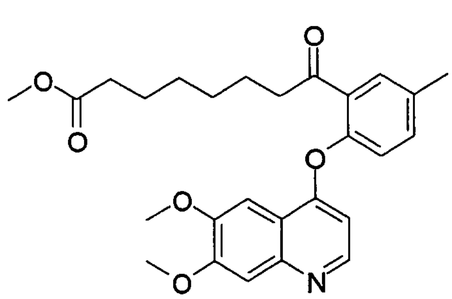 | 91 | 35 | |

(continued)

| Compound | Molecular structure | TGFβ inhibition rate, % | | |
|---|---|---|---|---|
| | | 10 μM | 3 μM | 1 μM |
| r 188 | 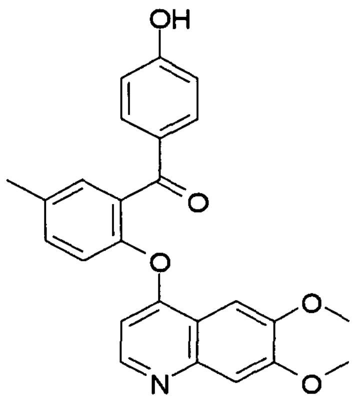 | 100 | 80 | |
| r 189 | 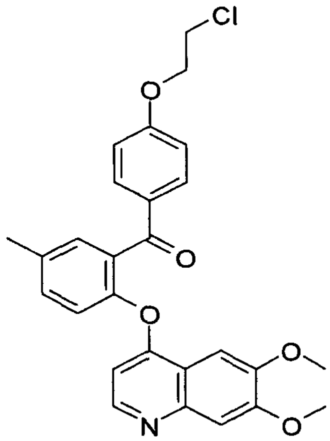 | 100 | 86 | |
| r 190 | 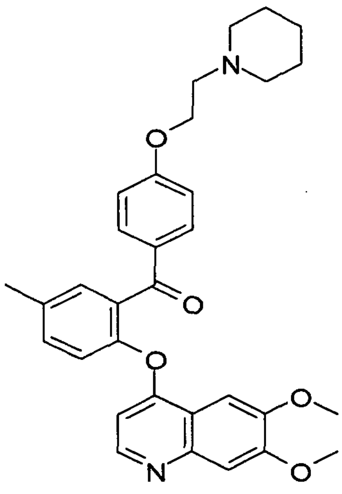 | 86 | 30 | |

(continued)

| Compound | Molecular structure | TGFβ inhibition rate, % | | |
|---|---|---|---|---|
| | | 10 μM | 3 μM | 1 μM |
| r 191 | 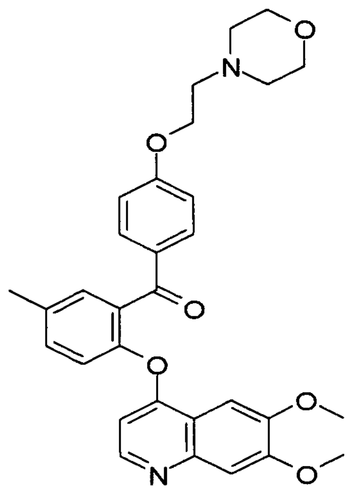 | 93 | 39 | |
| r 192 | 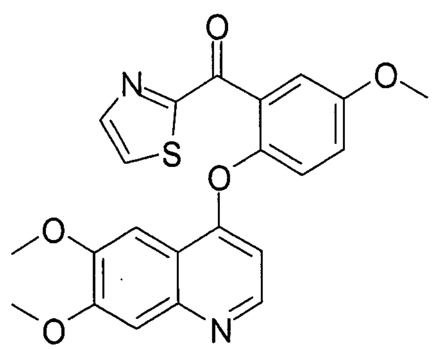 | 99 | 79 | |
| r193 | 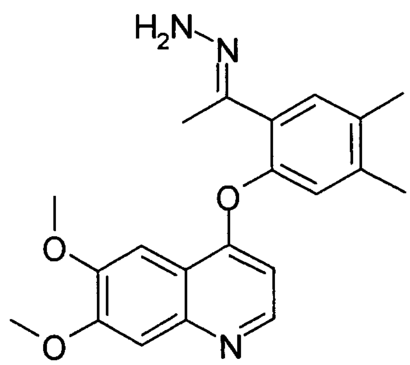 | 99 | 74 | |
| r 194 | 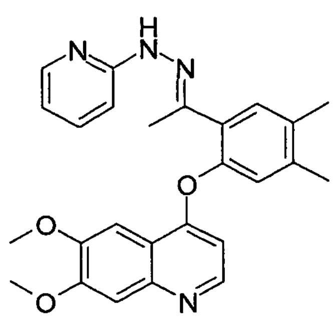 | 94 | 47 | |

(continued)

| Compound | Molecular structure | TGFβ inhibition rate, % | | |
|---|---|---|---|---|
| | | 10 μM | 3 μM | 1 μM |
| r 195 | 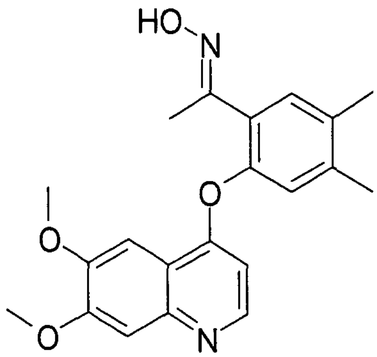 | 100 | 99 | |
| r196 | 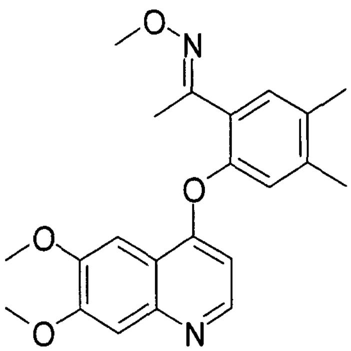 | 83 | 41 | |
| r 197 | 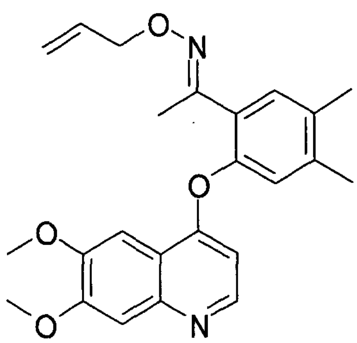 | 88 | 40 | |
| r198 | 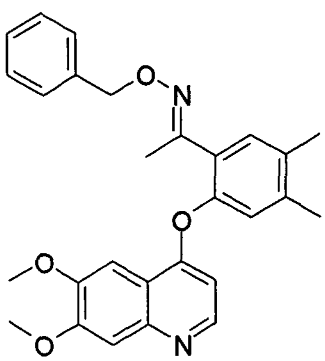 | 80 | 32 | |

(continued)

| Compound | Molecular structure | TGFβ inhibition rate, % | | |
|---|---|---|---|---|
| | | 10 μM | 3 μM | 1 μM |
| r 199 | 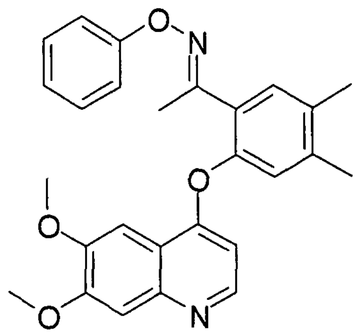 | 59 | 14 | |
| r 200 | 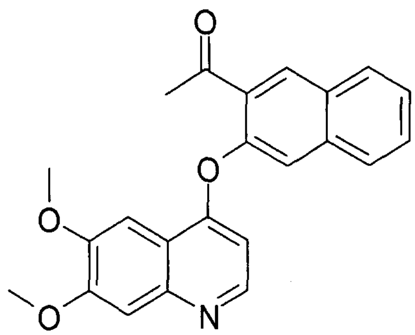 | 100 | 94 | |
| r 201 | 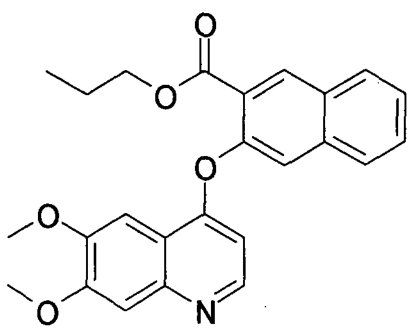 | 100 | 79 | |
| r 202 | 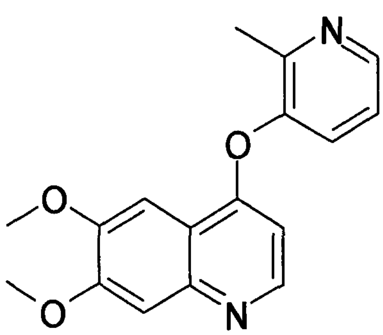 | 54 | 20 | |

(continued)

| Compound | Molecular structure | TGFβ inhibition rate, % | | |
|---|---|---|---|---|
| | | 10 μM | 3 μM | 1 μM |
| r 203 | 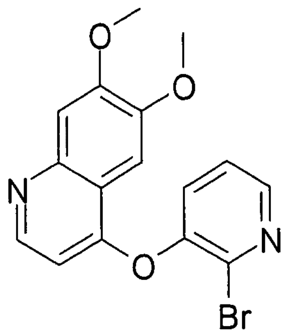 | 52 | 19 | |
| r 204 | 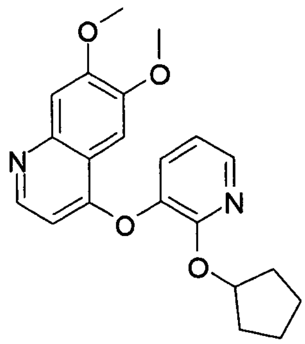 | 77 | 14 | |
| r 205 | 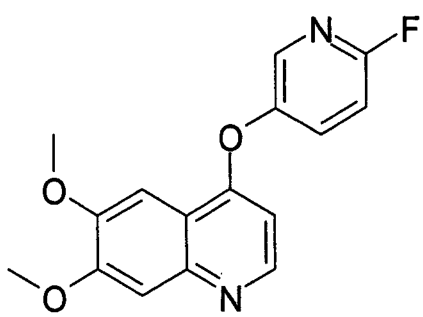 | 57 | 26 | |
| r 206 | 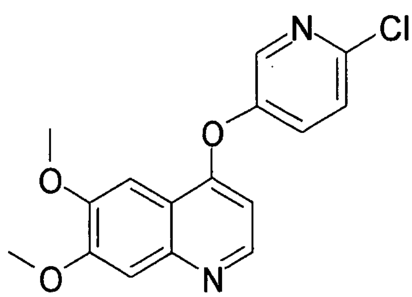 | 94 | 69 | |

(continued)

| Compound | Molecular structure | TGFβ inhibition rate, % | | |
|---|---|---|---|---|
| | | 10 μM | 3 μM | 1 μM |
| r 207 | 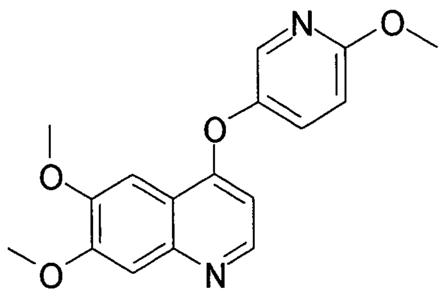 | 99 | 77 | |
| r 208 | 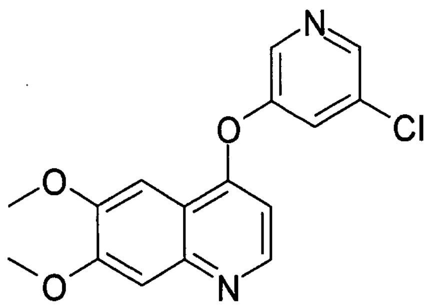 | 73 | 18 | |
| r 209 | 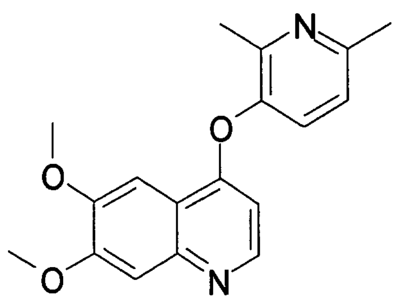 | 100 | 99 | |
| r 210 | 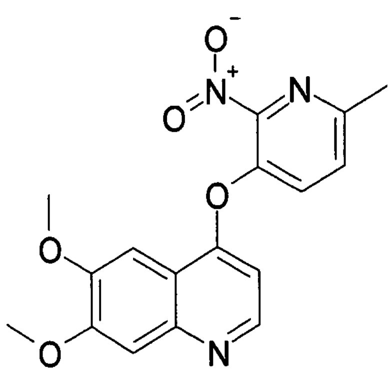 | 80 | 45 | |

(continued)

| Compound | Molecular structure | TGFβ inhibition rate, % | | |
|---|---|---|---|---|
| | | 10 μM | 3 μM | 1 μM |
| r 211 | 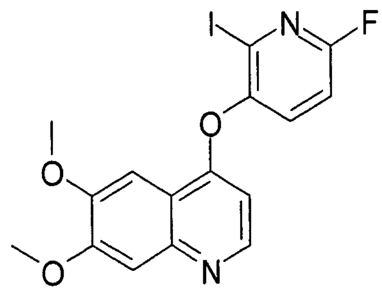 | 100 | 87 | |
| r 212 | 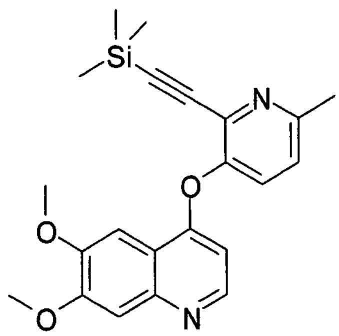 | 100 | 94 | |
| r 213 | 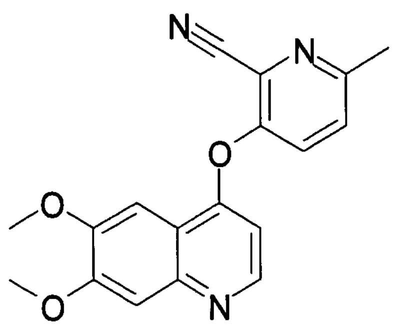 | 65 | 28 | |
| r 214 | 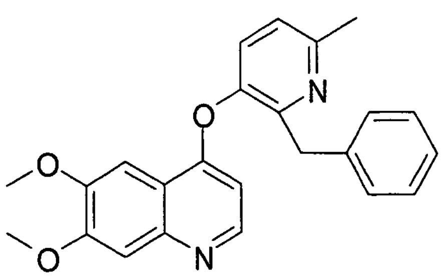 | 100 | 99 | |

(continued)

| Compound | Molecular structure | TGFβ inhibition rate, % | | |
|---|---|---|---|---|
| | | 10 μM | 3 μM | 1 μM |
| r 215 | 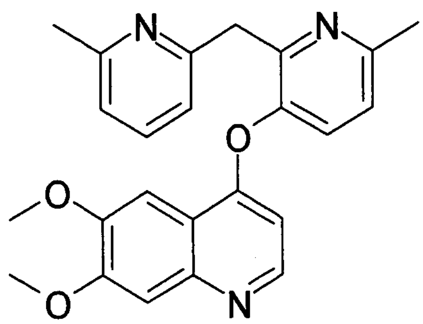 | 99 | 72 | |
| r 216 | 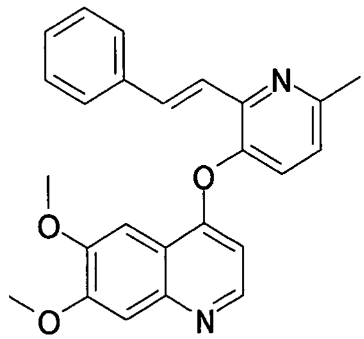 | 73 | 22 | |
| r 217 | 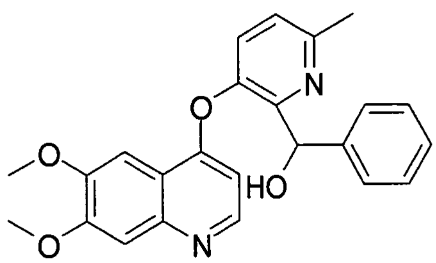 | 100 | 91 | |
| r 218 | 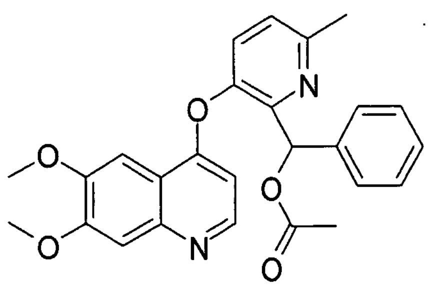 | 56 | 21 | |

(continued)

| Compound | Molecular structure | TGFβ inhibition rate, % | | |
|---|---|---|---|---|
| | | 10 μM | 3 μM | 1 μM |
| r 219 | 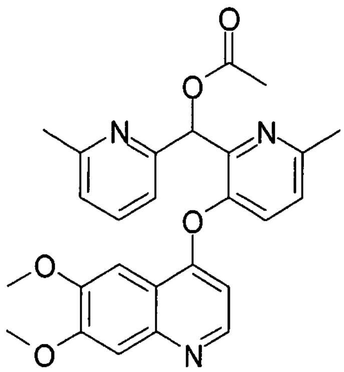 | 67 | 20 | |
| r 220 | 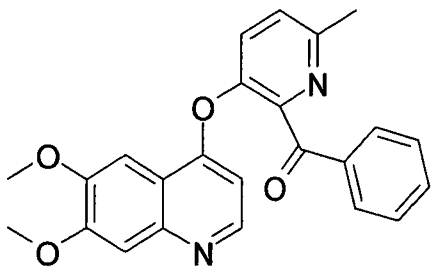 | 100 | 99 | |
| r 221 | 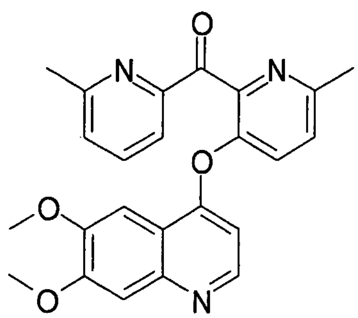 | 98 | 71 | |
| r 222 | 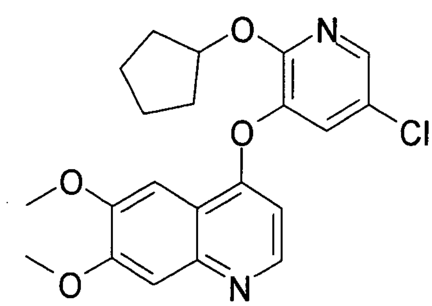 | 100 | 44 | |

(continued)

| Compound | Molecular structure | TGFβ inhibition rate, % | | |
|---|---|---|---|---|
| | | 10 μM | 3 μM | 1 μM |
| r 223 | 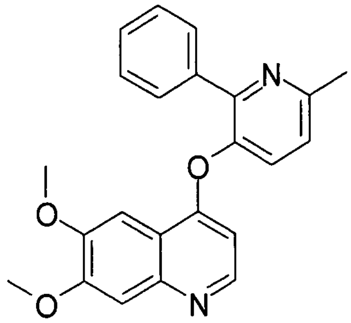 | 100 | 99 | |
| r 224 | 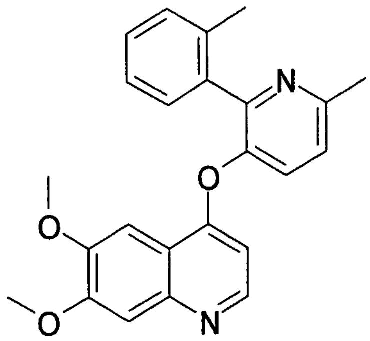 | 96 | 69 | |
| r 225 | 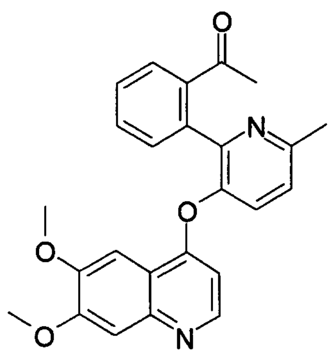 | 78 | 39 | |
| r 226 | 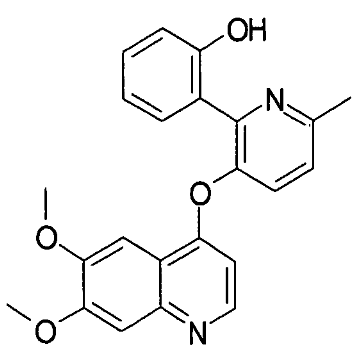 | 96 | 60 | |

(continued)

| Compound | Molecular structure | TGFβ inhibition rate, % | | |
|---|---|---|---|---|
| | | 10 μM | 3 μM | 1 μM |
| r 227 | 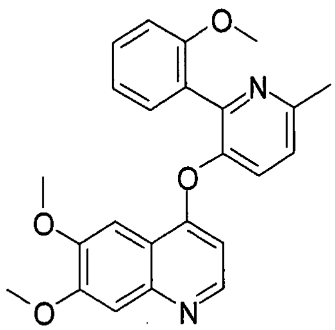 | 87 | 50 | |
| r 228 | 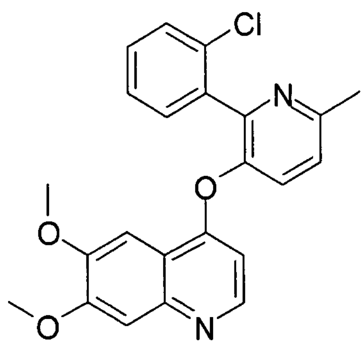 | 100 | 88 | |
| r 229 | 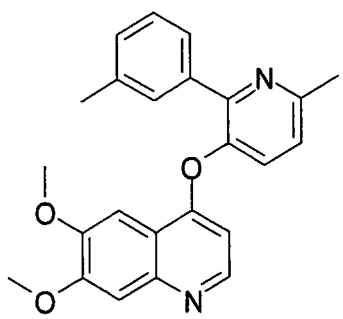 | 100 | 99 | |
| r 230 | 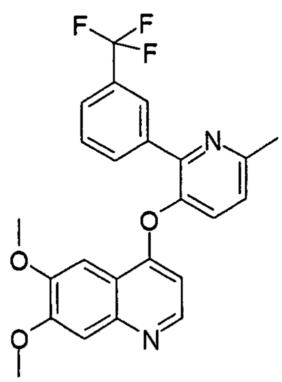 | 97 | 60 | |

(continued)

| Compound | Molecular structure | TGFβ inhibition rate, % | | |
|---|---|---|---|---|
| | | 10 μM | 3 μM | 1 μM |
| r 231 | 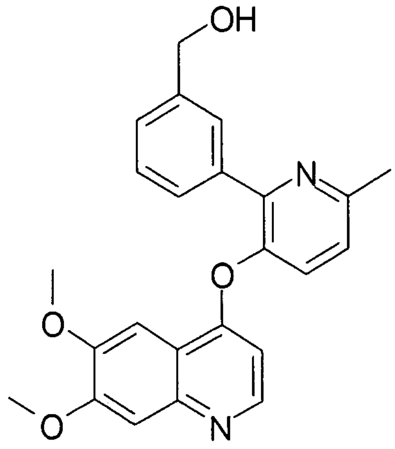 | 100 | 100 | |
| r 232 | 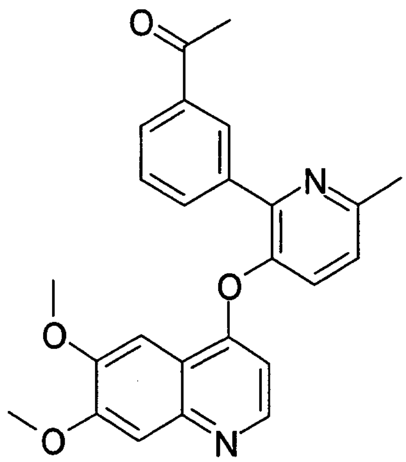 | 79 | 66 | |
| r 233 | 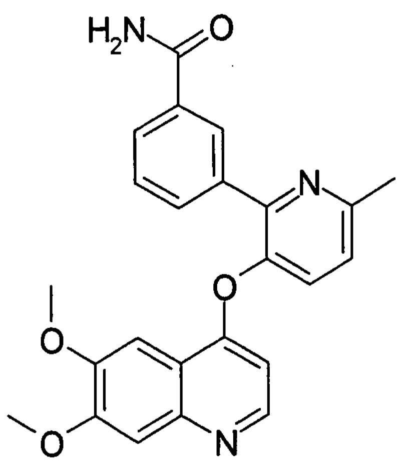 | 100 | 98 | |

(continued)

| Compound | Molecular structure | TGFβ inhibition rate, % | | |
|---|---|---|---|---|
| | | 10 μM | 3 μM | 1 μM |
| r 234 | 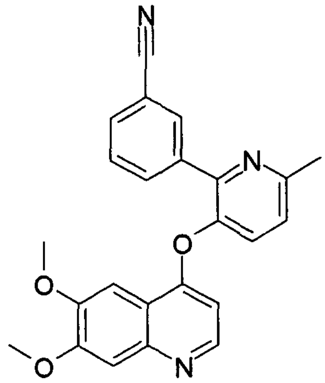 | 100 | 87 | |
| r 235 | 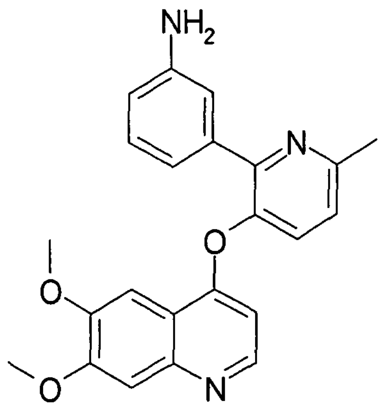 | 100 | 100 | |
| r 236 | 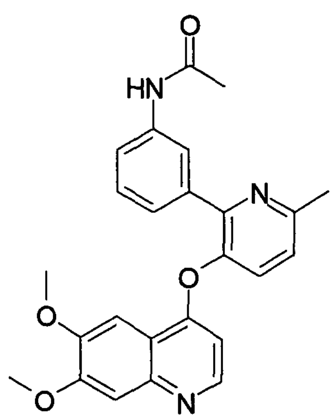 | 100 | 90 | |

(continued)

| Compound | Molecular structure | TGFβ inhibition rate, % | | |
|---|---|---|---|---|
| | | 10 μM | 3 μM | 1 μM |
| r 237 | 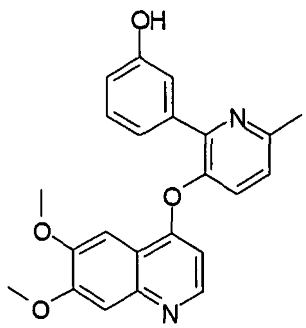 | 100 | 100 | |
| r 238 | 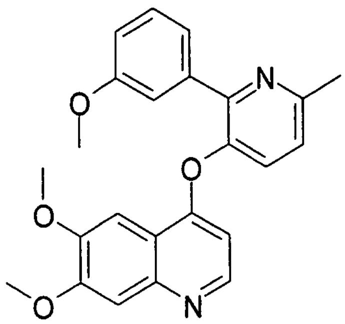 | 100 | 92 | |
| r 239 | 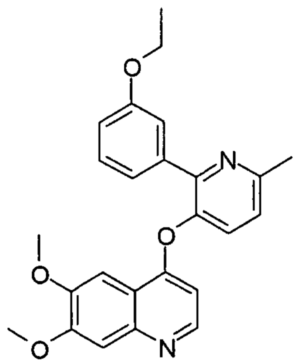 | 100 | 83 | |
| r 240 | 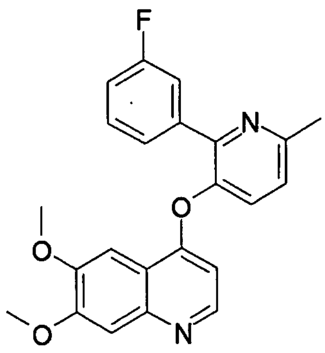 | 100 | 100 | |

(continued)

| Compound | Molecular structure | TGFβ inhibition rate, % | | |
|---|---|---|---|---|
| | | 10 μM | 3 μM | 1 μM |
| r 241 | 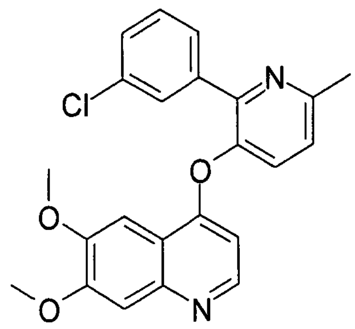 | 100 | 96 | |
| r 242 | 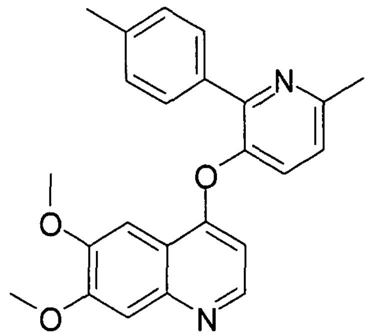 | 99 | 68 | |
| r 243 | 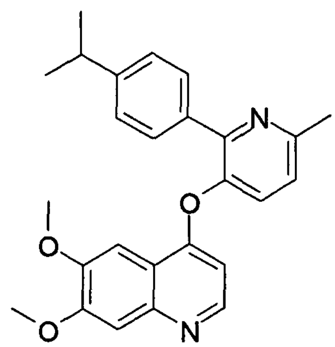 | 62 | -7 | |
| r 244 | 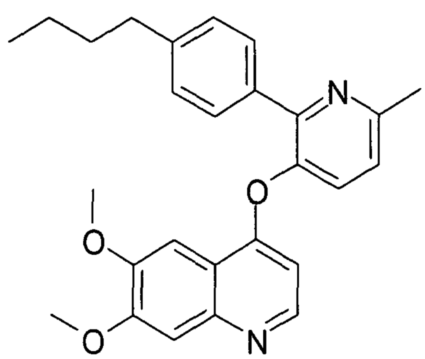 | 63 | -3 | |

(continued)

| Compound | Molecular structure | TGFβ inhibition rate, % | | |
|---|---|---|---|---|
| | | 10 μM | 3 μM | 1 μM |
| r 245 | 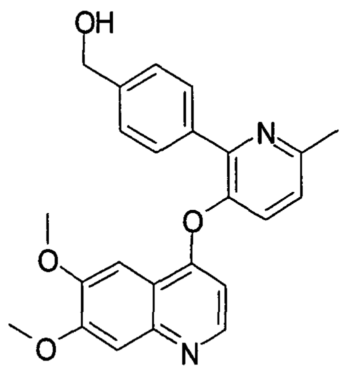 | 99 | 80 | |
| r 246 | 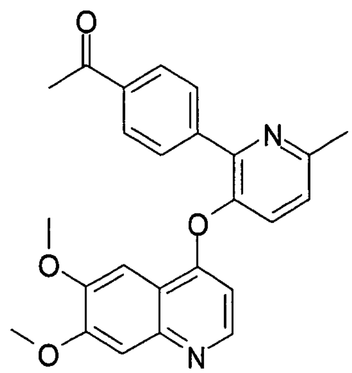 | 93 | 64 | |
| r 247 | 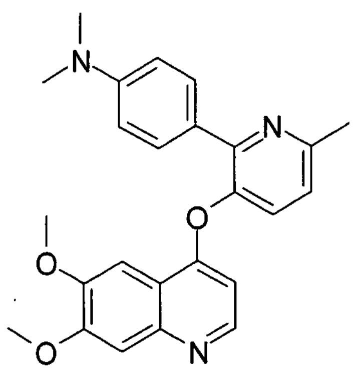 | 100 | 93 | |
| r 248 | 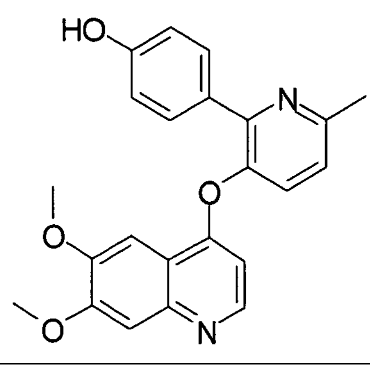 | 100 | 99 | |

(continued)

| Compound | Molecular structure | TGFβ inhibition rate, % | | |
|---|---|---|---|---|
| | | 10 μM | 3 μM | 1 μM |
| r 249 | 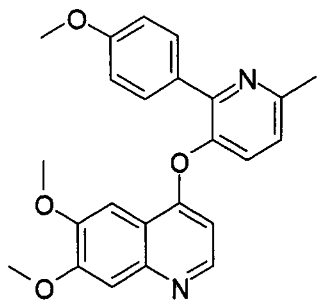 | 98 | 52 | |
| r 250 | 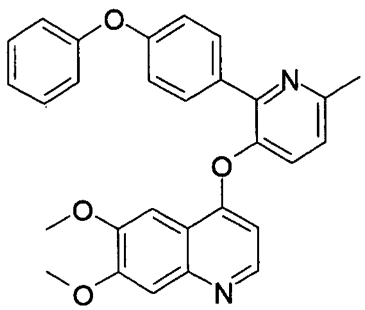 | 81 | 34 | |
| r 251 | 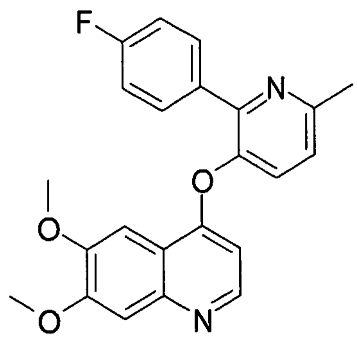 | 100 | 84 | |
| r 252 | 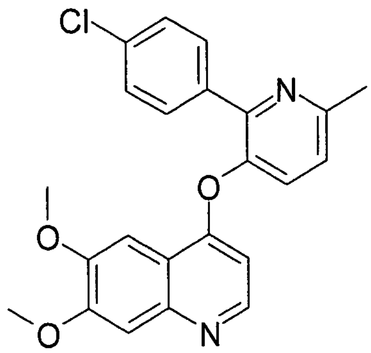 | 78 | 16 | |

(continued)

| Compound | Molecular structure | TGFβ inhibition rate, % | | |
|---|---|---|---|---|
| | | 10 μM | 3 μM | 1 μM |
| r 253 | 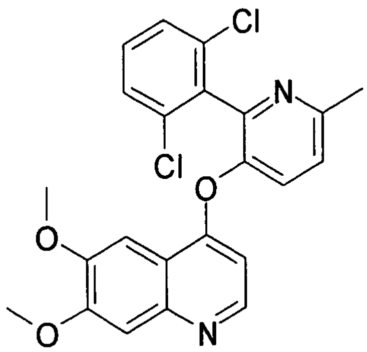 | 97 | 68 | |
| r 254 | 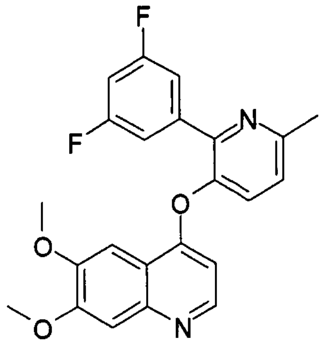 | 100 | 100 | |
| r 255 | 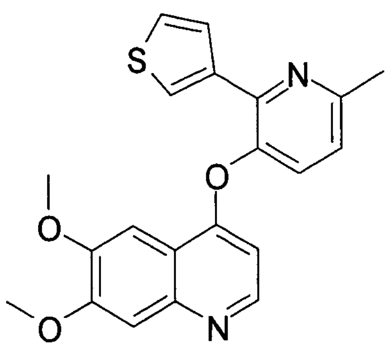 | 100 | 93 | |
| r 256 | 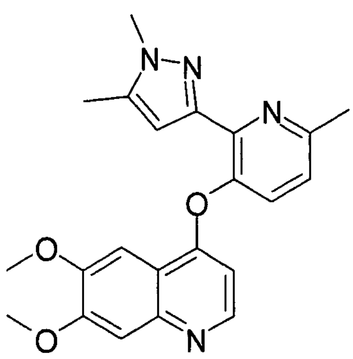 | 100 | 98 | |

(continued)

| Compound | Molecular structure | TGFβ inhibition rate, % | | |
|---|---|---|---|---|
| | | 10 μM | 3 μM | 1 μM |
| r 257 | 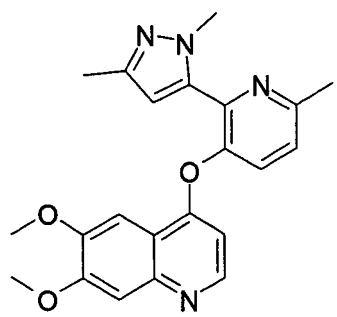 | 62 | 15 | |
| r 258 | 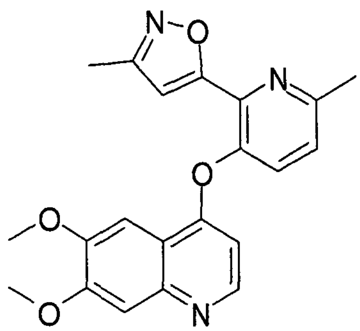 | 100 | 99 | |
| r 259 | 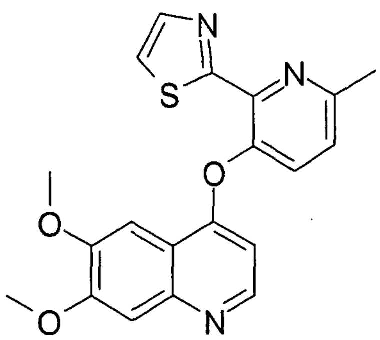 | | | 100 |
| r 260 | 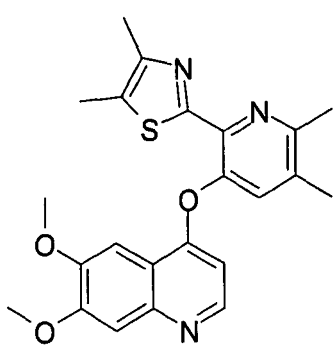 | 100 | 96 | |

(continued)

| Compound | Molecular structure | TGFβ inhibition rate, % | | |
|---|---|---|---|---|
| | | 10 μM | 3 μM | 1 μM |
| r 261 | 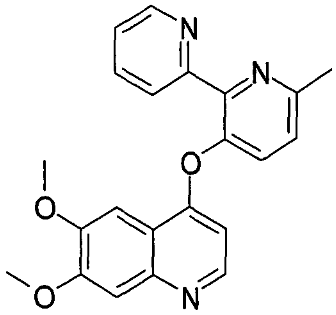 | 100 | 100 | |
| r 262 | 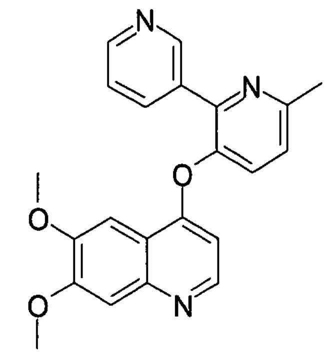 | 100 | 91 | |
| r 263 | 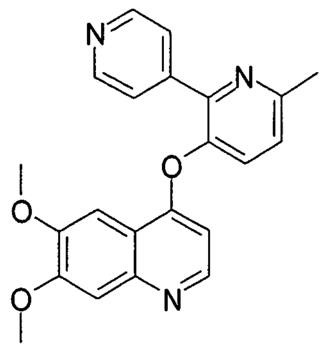 | 99 | 82 | |
| r 264 | 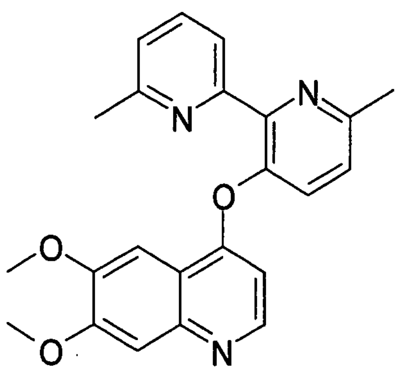 | 100 | 88 | |

(continued)

| Compound | Molecular structure | TGFβ inhibition rate, % | | |
|---|---|---|---|---|
| | | 10 μM | 3 μM | 1 μM |
| r 265 | 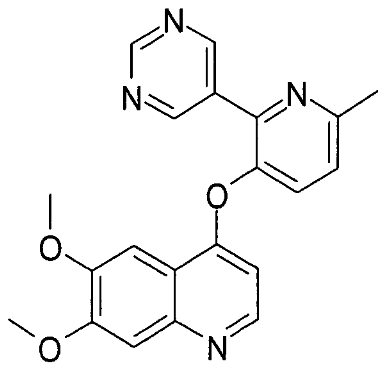 | 85 | 45 | |
| r 266 | 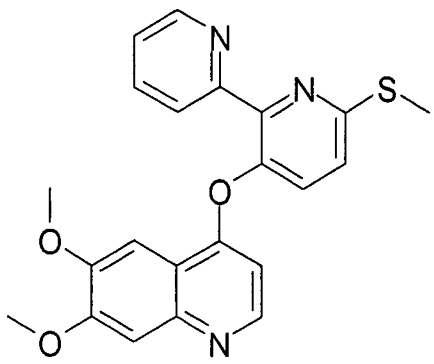 | 100 | 92 | |
| r 267 | 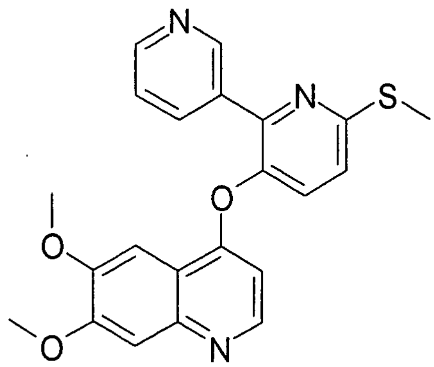 | 83 | 34 | |
| r 268 | 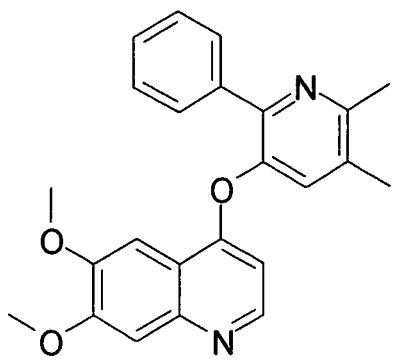 | 100 | 100 | |

(continued)

| Compound | Molecular structure | TGFβ inhibition rate, % | | |
|---|---|---|---|---|
| | | 10 μM | 3 μM | 1 μM |
| r 269 | 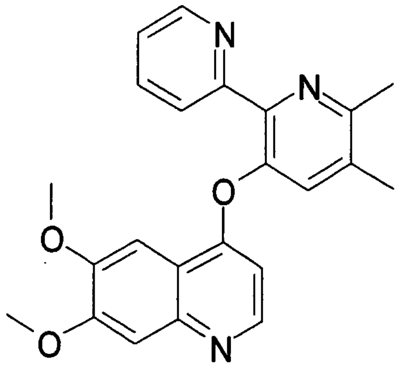 | | | 97 |
| r 270 | 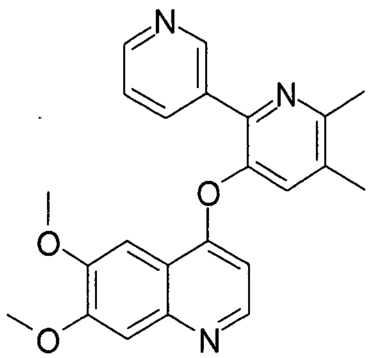 | | 100 | |
| r 271 | 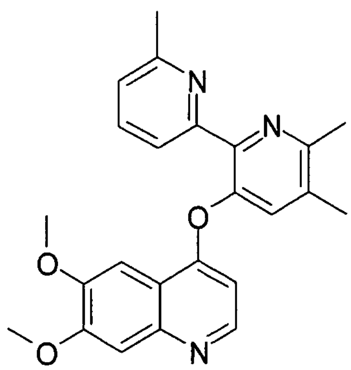 | 100 | 94 | |
| r 272 | 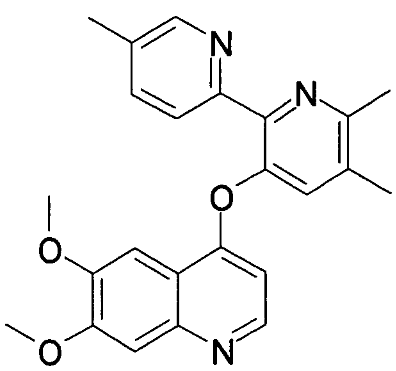 | 100 | 98 | |

(continued)

| Compound | Molecular structure | TGFβ inhibition rate, % | | |
|---|---|---|---|---|
| | | 10 μM | 3 μM | 1 μM |
| r 273 | 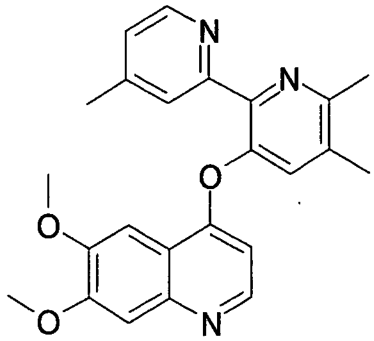 | 100 | 99 | |
| r 274 | 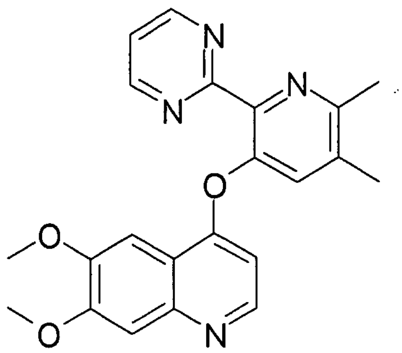 | | 97 | |
| r 275 | 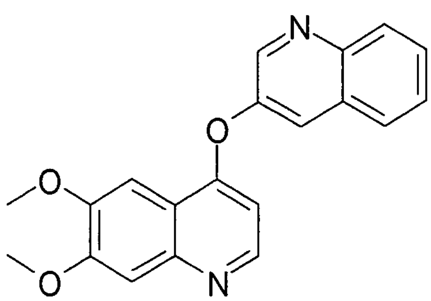 | 90 | 45 | |
| r 276 | 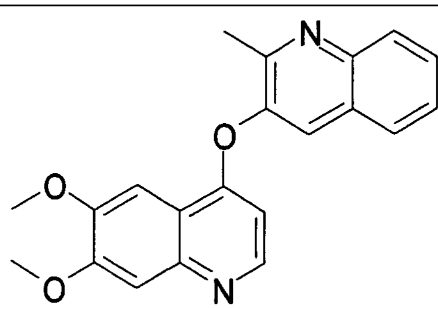 | 100 | 89 | |

(continued)

| Compound | Molecular structure | TGFβ inhibition rate, % | | |
|---|---|---|---|---|
| | | 10 μM | 3 μM | 1 μM |
| r 277 | 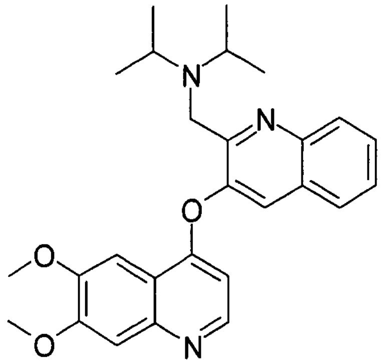 | 89 | 32 | |
| r 278 | 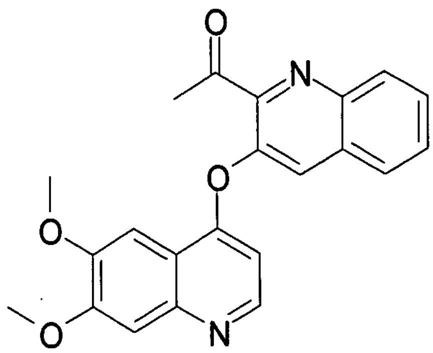 | 99 | 90 | |
| r 279 | 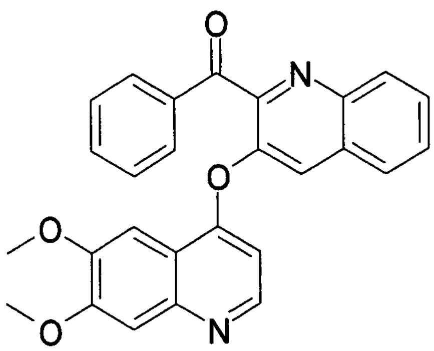 | 100 | 98 | |
| r 280 | 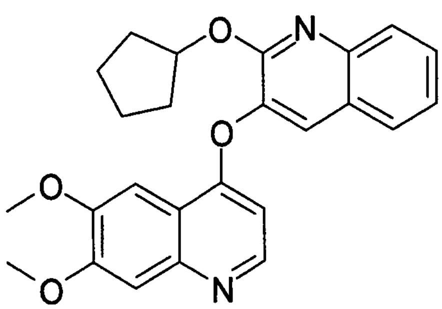 | 88 | 45 | |

(continued)

| Compound | Molecular structure | TGFβ inhibition rate, % | | |
|---|---|---|---|---|
| | | 10 μM | 3 μM | 1 μM |
| r 281 | 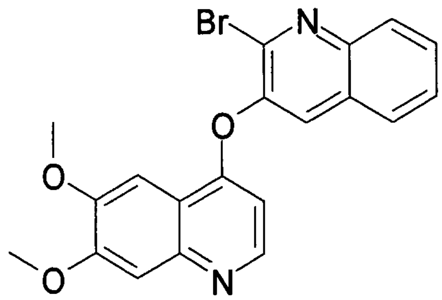 | 100 | 88 | |
| r 282 | 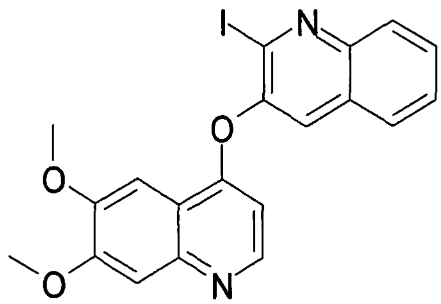 | 100 | 96 | |
| r 283 | 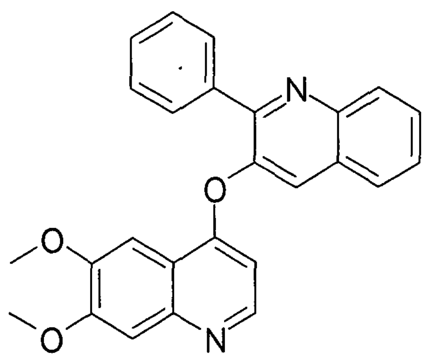 | 100 | 99 | |
| r 284 | 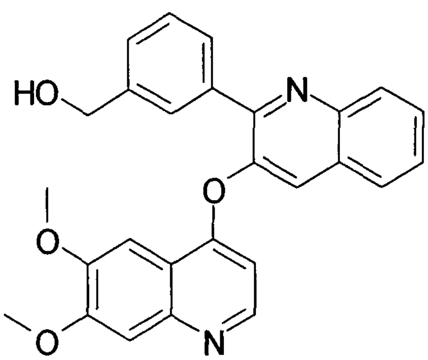 | 100 | 96 | |

(continued)

| Compound | Molecular structure | TGFβ inhibition rate, % | | |
|---|---|---|---|---|
| | | 10 μM | 3 μM | 1 μM |
| r 285 | 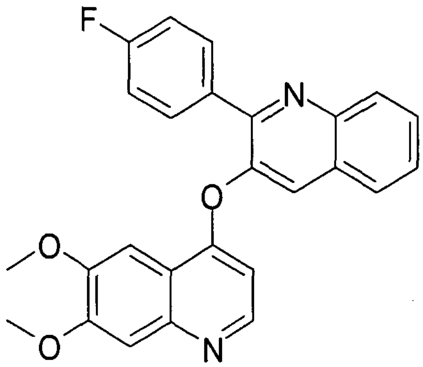 | 100 | 65 | |
| r 286 | 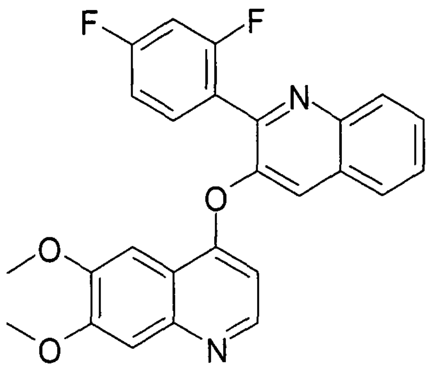 | 100 | 93 | |
| r 287 | 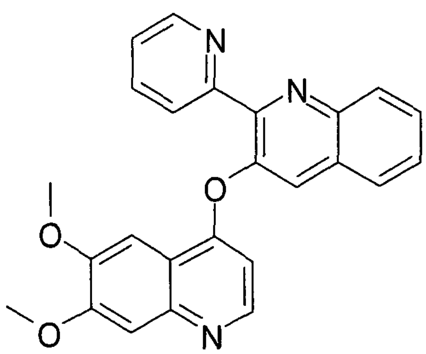 | 100 | 100 | |
| r 288 | 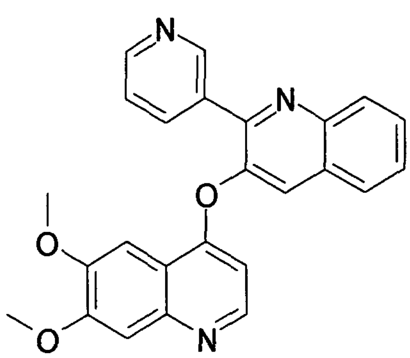 | 100 | 94 | |

(continued)

| Compound | Molecular structure | TGFβ inhibition rate, % | | |
|---|---|---|---|---|
| | | 10 μM | 3 μM | 1 μM |
| r 289 | 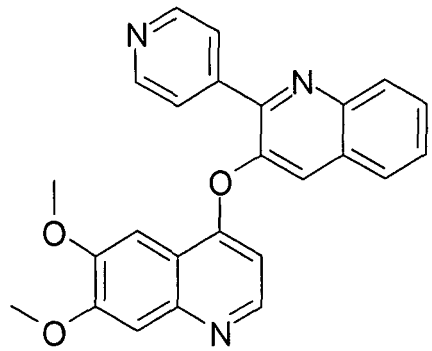 | 99 | 80 | |
| r 290 | 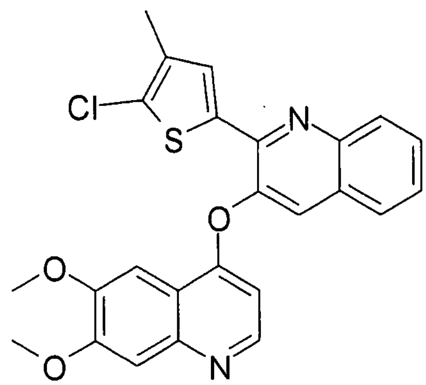 | 57 | 18 | |
| r 291 | 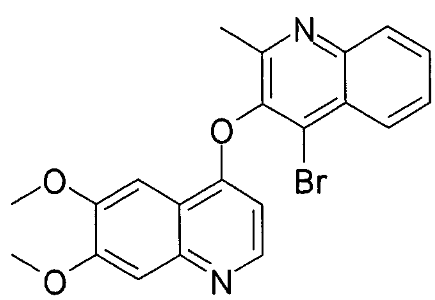 | 78 | 14 | |
| r 292 | 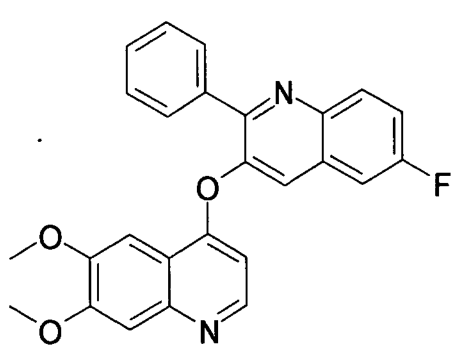 | 100 | 86 | |

(continued)

| Compound | Molecular structure | TGFβ inhibition rate, % | | |
|---|---|---|---|---|
| | | 10 μM | 3 μM | 1 μM |
| r 293 | 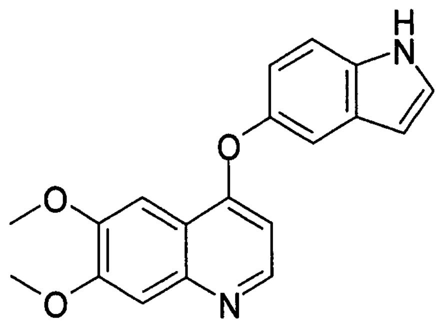 | 95 | 65 | |
| r 294 | 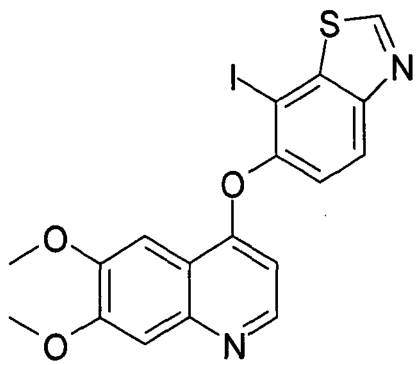 | 77 | 53 | |
| r 295 | 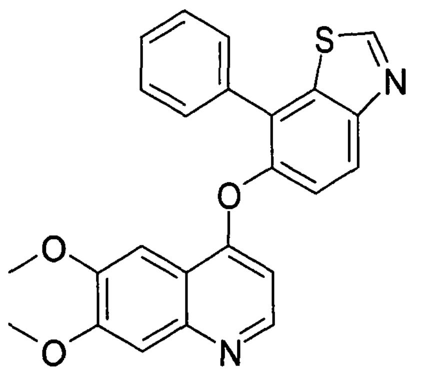 | 96 | 47 | |
| r 296 | 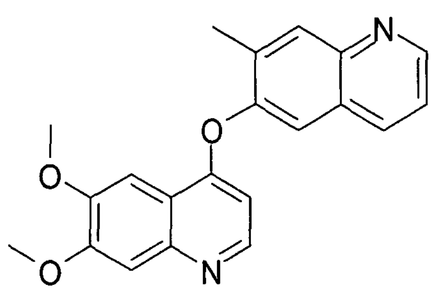 | 100 | 86 | |

(continued)

| Compound | Molecular structure | TGFβ inhibition rate, % | | |
|---|---|---|---|---|
| | | 10 μM | 3 μM | 1 μM |
| r 297 | 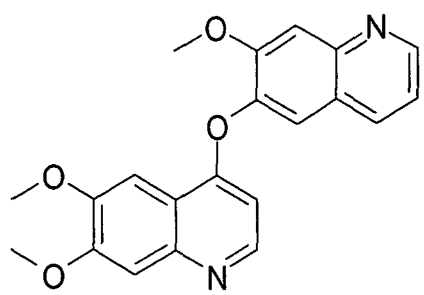 | 94 | 61 | |
| r 298 | 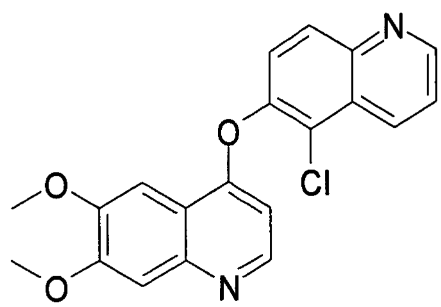 | 75 | 30 | |
| r 299 | 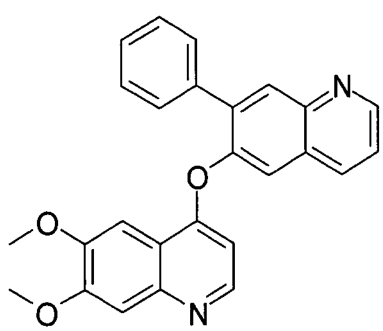 | 100 | 99 | |
| r 300 | 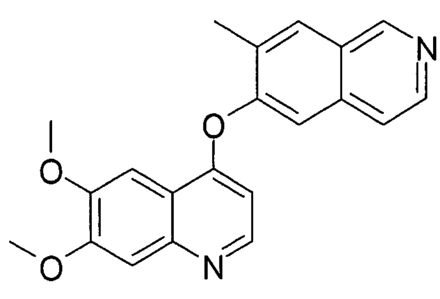 | 63 | 9 | |

(continued)

| Compound | Molecular structure | TGFβ inhibition rate, % | | |
|---|---|---|---|---|
| | | 10 μM | 3 μM | 1 μM |
| r 301 | 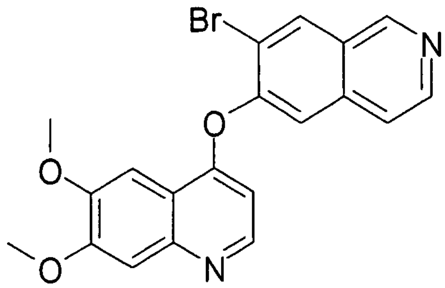 | 75 | 20 | |
| r 302 | 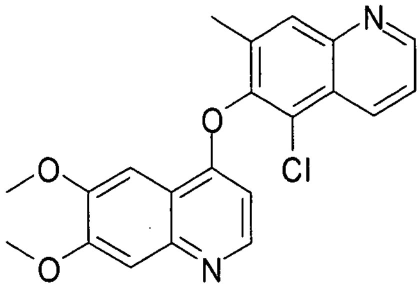 | 90 | 30 | |
| r 303 | 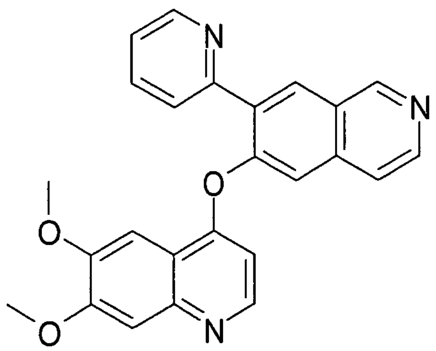 | | 96 | |
| r 304 | 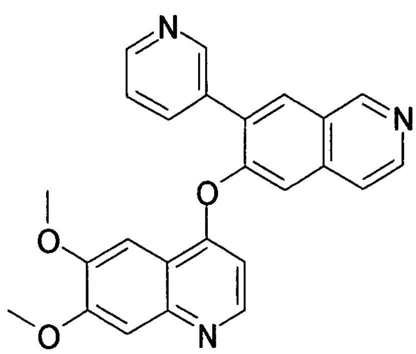 | 64 | 26 | |

(continued)

| Compound | Molecular structure | TGFβ inhibition rate, % | | |
|---|---|---|---|---|
| | | 10 μM | 3 μM | 1 μM |
| r 305 | 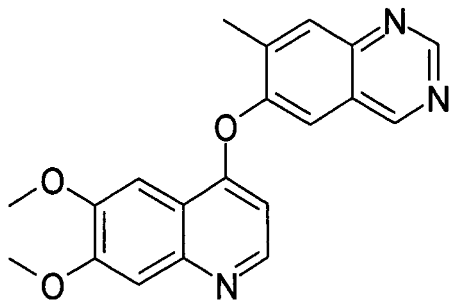 | 64 | 28 | |
| r 306 | 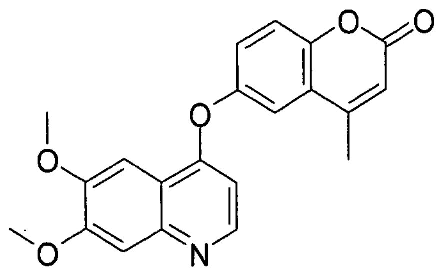 | 53 | 20 | |
| r 307 | 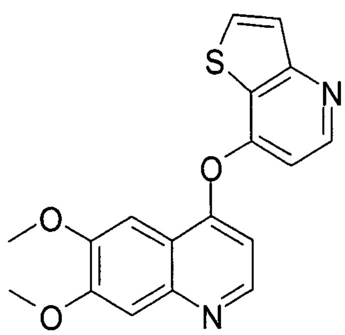 | 69 | 26 | |
| r 308 | 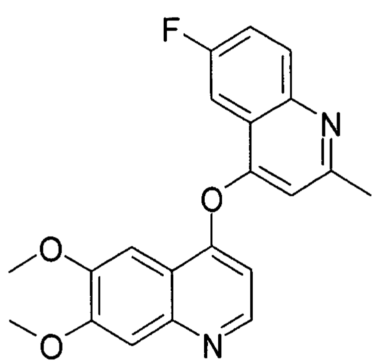 | 56 | 21 | |

(continued)

| Compound | Molecular structure | TGFβ inhibition rate, % | | |
|---|---|---|---|---|
| | | 10 μM | 3 μM | 1 μM |
| r 309 | 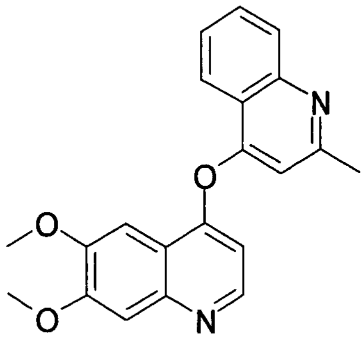 | 79 | 41 | |
| r 310 | 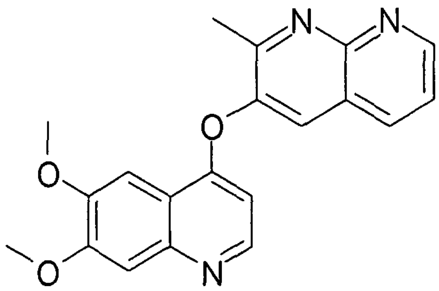 | 97 | 72 | |
| r 311 | 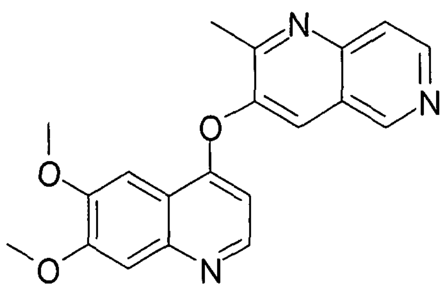 | 90 | 53 | |
| r 312 | 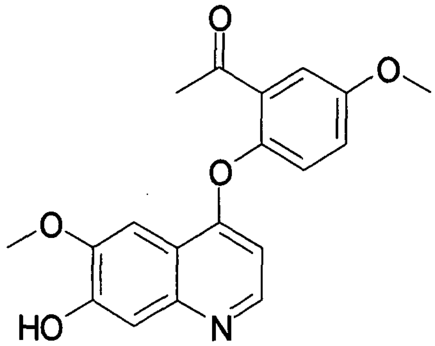 | 76 | 50 | |

(continued)

| Compound | Molecular structure | TGFβ inhibition rate, % | | |
|---|---|---|---|---|
| | | 10 μM | 3 μM | 1 μM |
| r 313 | 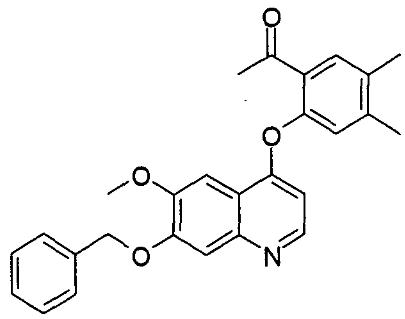 | 94 | 56 | |
| r 314 | 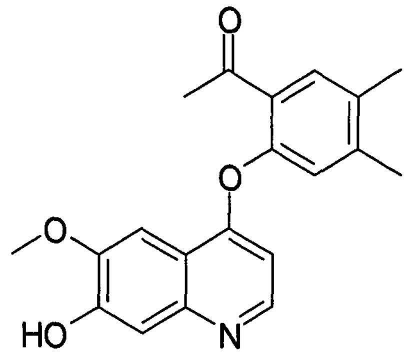 | 89 | 50 | |
| r 315 | 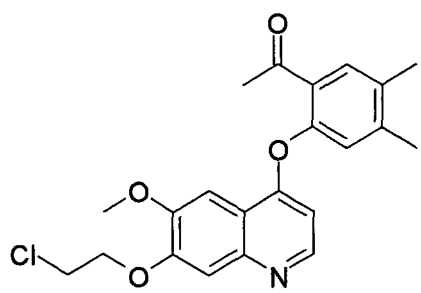 | 100 | 98 | |
| r 316 | 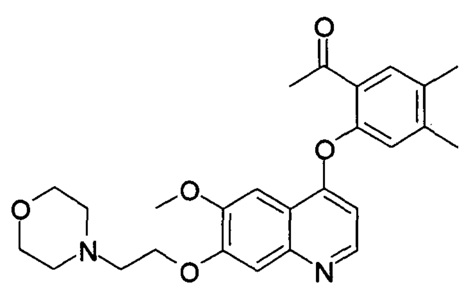 | 100 | 99 | |

(continued)

| Compound | Molecular structure | TGFβ inhibition rate, % | | |
|---|---|---|---|---|
| | | 10 μM | 3 μM | 1 μM |
| r 317 | 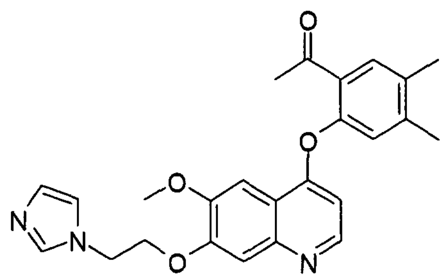 | 100 | 100 | |
| r 318 | 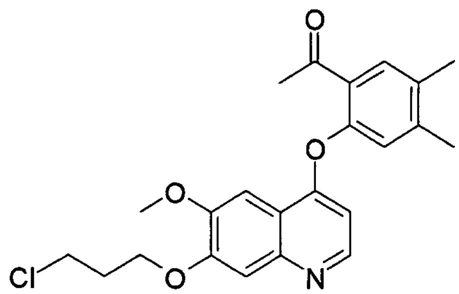 | 100 | 82 | |
| r 319 | 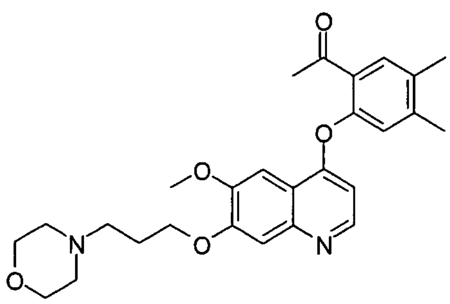 | 100 | 100 | |
| r 320 | 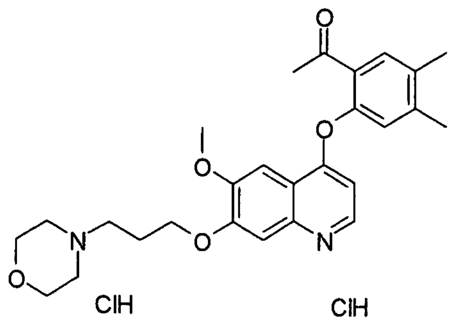 | 100 | 100 | |

(continued)

| Compound | Molecular structure | TGFβ inhibition rate, % | | |
|---|---|---|---|---|
| | | 10 μM | 3 μM | 1 μM |
| r 321 | 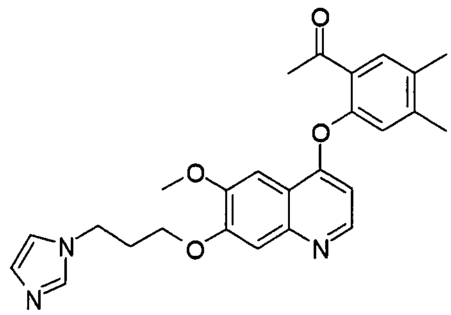 | 100 | 100 | |
| r 322 | 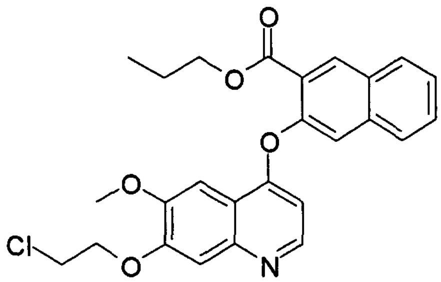 | 88 | 50 | |
| r 323 | 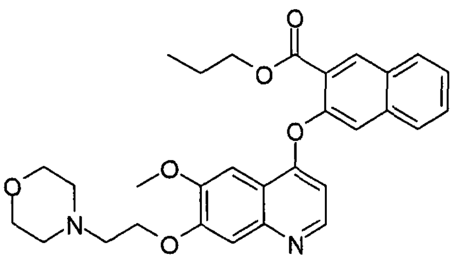 | 100 | 89 | |
| r 324 | 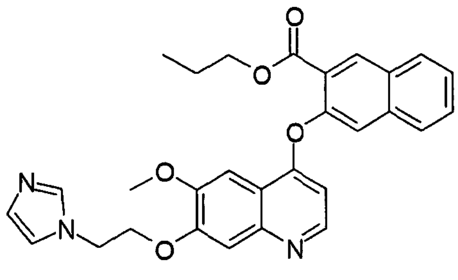 | 100 | 86 | |

(continued)

| Compound | Molecular structure | TGFβ inhibition rate, % | | |
|---|---|---|---|---|
| | | 10 μM | 3 μM | 1 μM |
| r 325 | 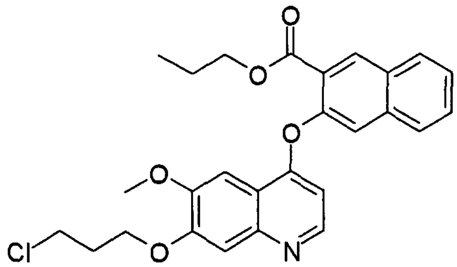 | 60 | 19 | |
| r 326 | 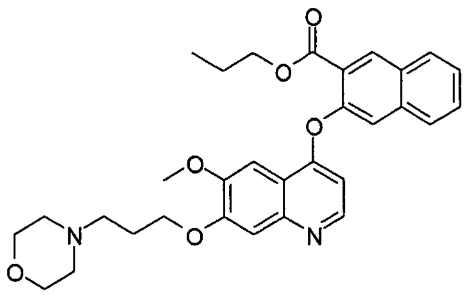 | 100 | 83 | |
| r 327 | 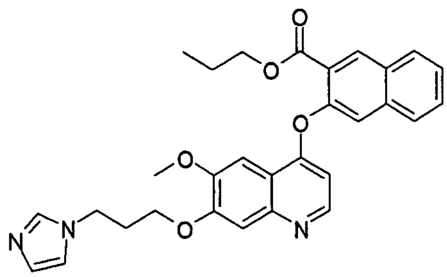 | 100 | 89 | |
| r 328 | 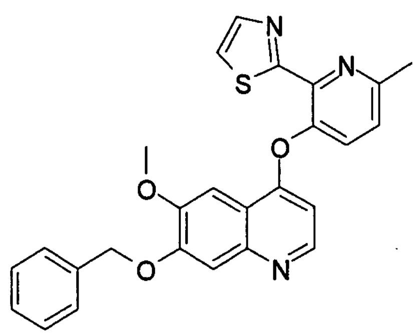 | 100 | 100 | |

(continued)

| Compound | Molecular structure | TGFβ inhibition rate, % | | |
|---|---|---|---|---|
| | | 10 μM | 3 μM | 1 μM |
| r 329 | 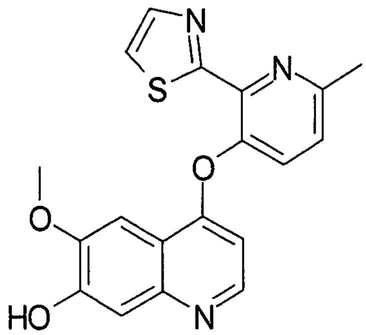 | 100 | 100 | |
| r 330 | 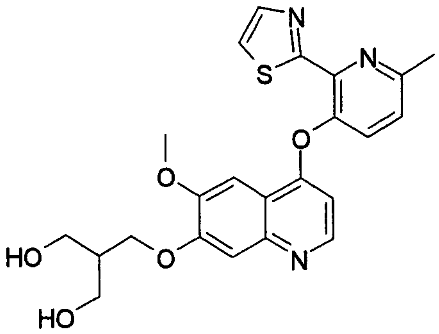 | 100 | 100 | |
| r 331 | 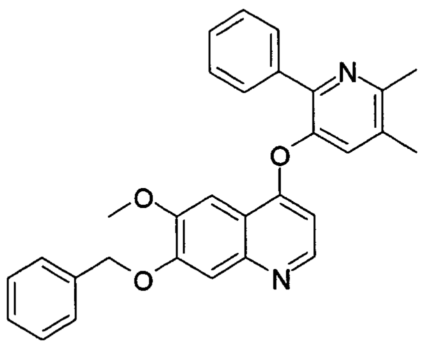 | 90 | 73 | |
| r 332 | 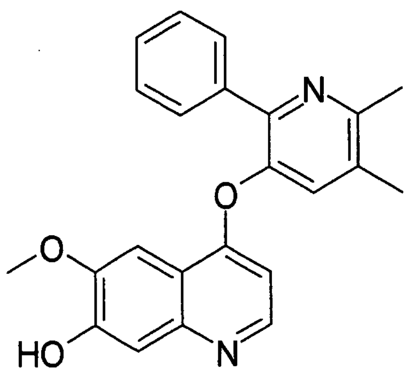 | 100 | 97 | |

(continued)

| Compound | Molecular structure | TGFβ inhibition rate, % | | |
|---|---|---|---|---|
| | | 10 μM | 3 μM | 1 μM |
| r 333 | 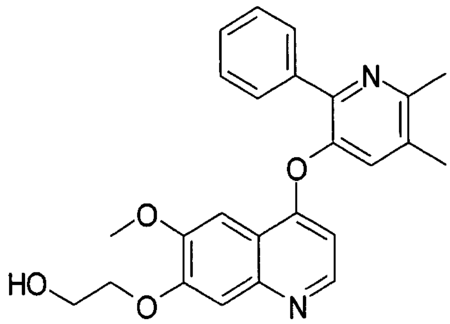 | | 100 | |
| r 334 | 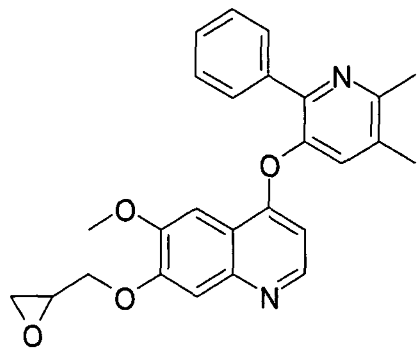 | | 99 | |
| r 335 | 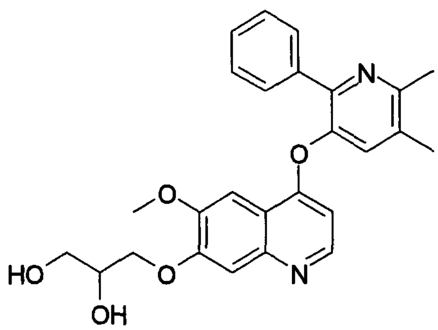 | | 100 | |
| r 336 | 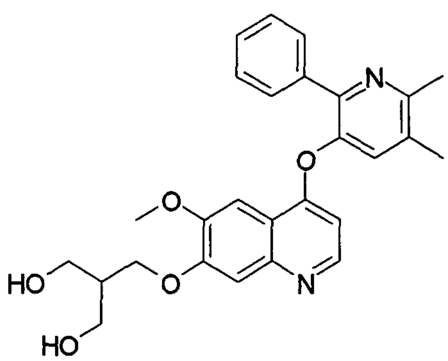 | | 100 | |

(continued)

| Compound | Molecular structure | TGFβ inhibition rate, % | | |
|---|---|---|---|---|
| | | 10 μM | 3 μM | 1 μM |
| r 337 | 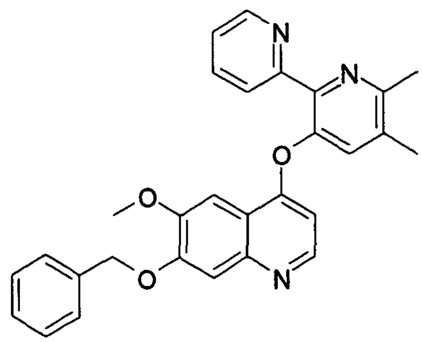 | | 98 | |
| r 338 | 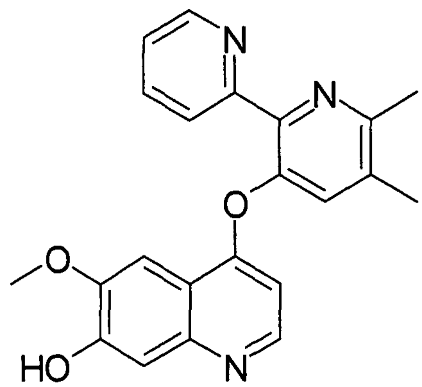 | 100 | 92 | |
| r 339 | 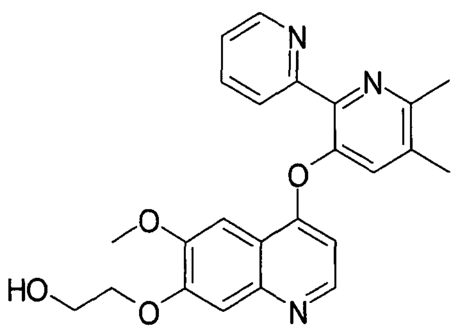 | | 100 | |
| r 340 | 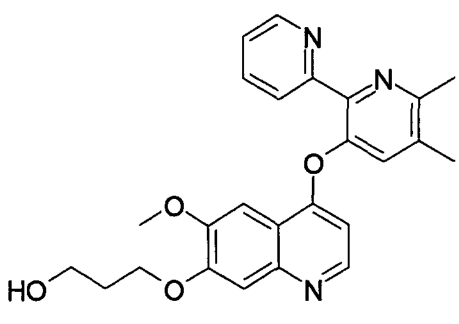 | | 99 | |

(continued)

| Compound | Molecular structure | TGFβ inhibition rate, % | | |
|---|---|---|---|---|
| | | 10 μM | 3 μM | 1 μM |
| r 341 | 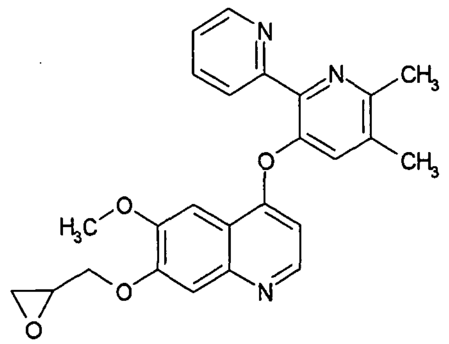 | | 99 | |
| r 342 | 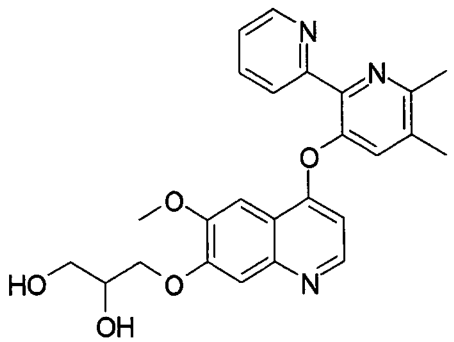 | | 89 | |
| r 343 | 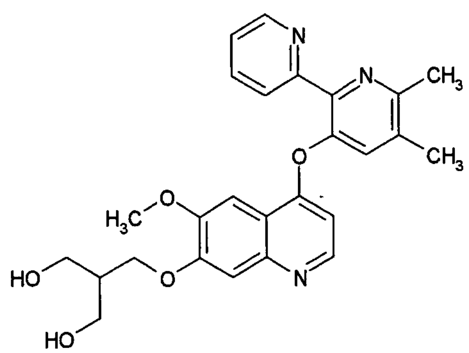 | | 93 | |
| r 344 | 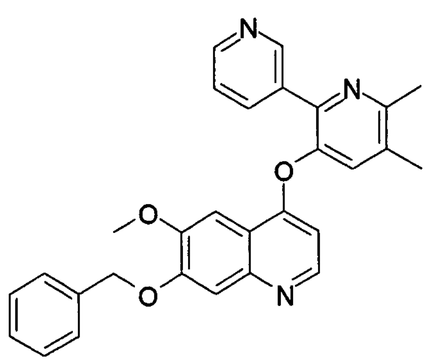 | | 92 | |

(continued)

| Compound | Molecular structure | TGFβ inhibition rate, % | | |
|---|---|---|---|---|
| | | 10 μM | 3 μM | 1 μM |
| r 345 | 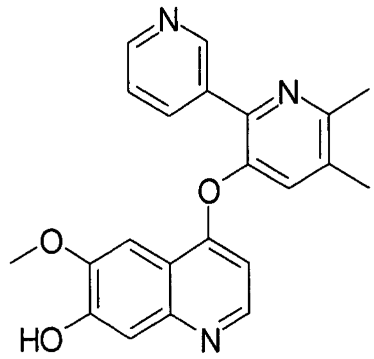 | 93 | 61 | |
| r 346 | 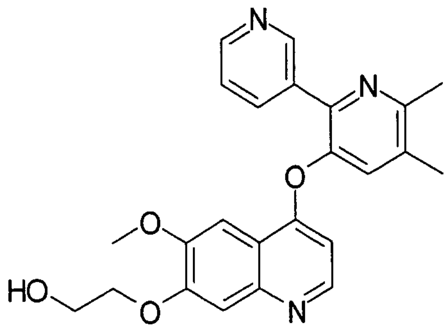 | | 99 | |
| r 347 | 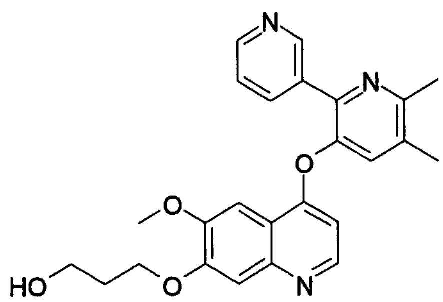 | | 100 | |
| r 348 | 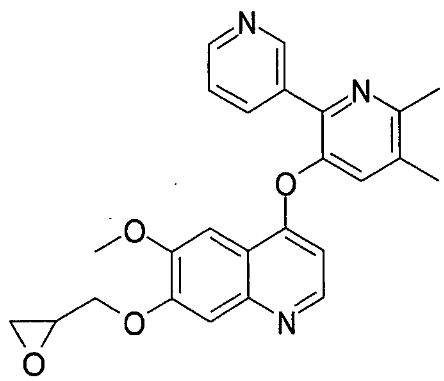 | | 76 | |

(continued)

| Compound | Molecular structure | TGFβ inhibition rate, % | | |
|---|---|---|---|---|
| | | 10 μM | 3 μM | 1 μM |
| r 349 | 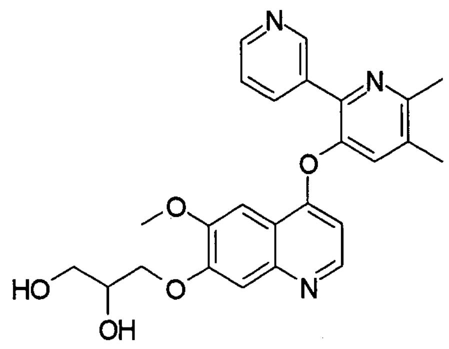 | | 96 | |
| r 350 | 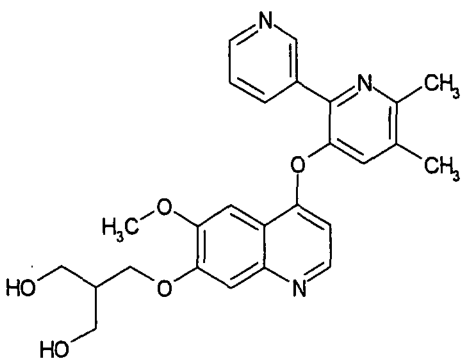 | | 96 | |
| r 351 | 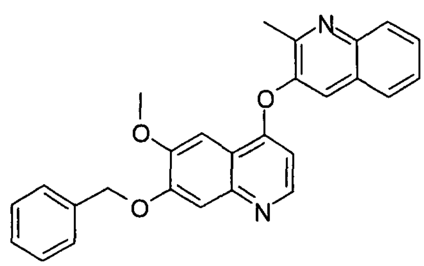 | 90 | 42 | |
| r 352 | 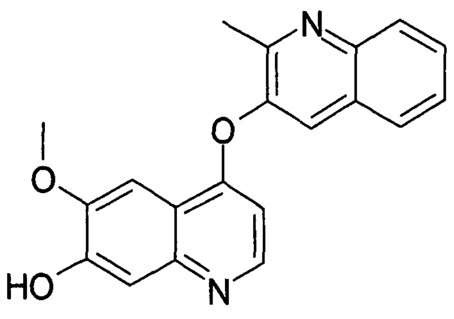 | 68 | 14 | |

(continued)

| Compound | Molecular structure | TGFβ inhibition rate, % | | |
|---|---|---|---|---|
| | | 10 μM | 3 μM | 1 μM |
| r 353 | 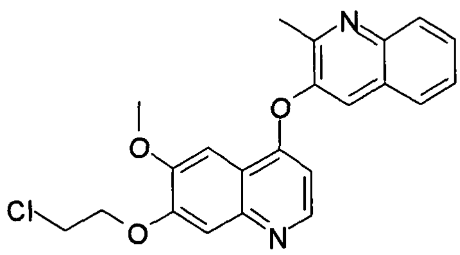 | 86 | 54 | |
| r 354 | 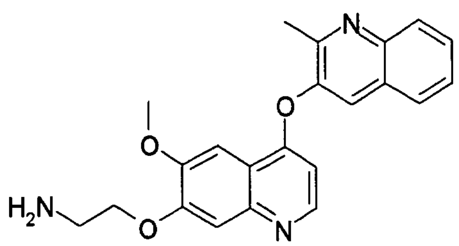 | 100 | 100 | |
| r 355 | 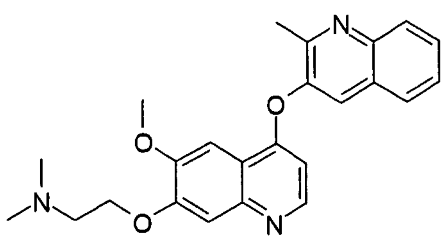 | 100 | 100 | |
| r 356 | 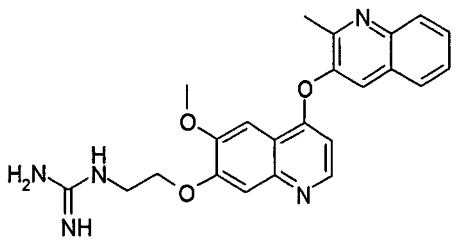 | 54 | -2 | |
| r 357 | 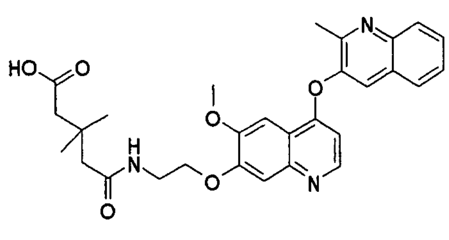 | 60 | 27 | |

(continued)

| Compound | Molecular structure | TGFβ inhibition rate, % | | |
|---|---|---|---|---|
| | | 10 μM | 3 μM | 1 μM |
| r 358 | 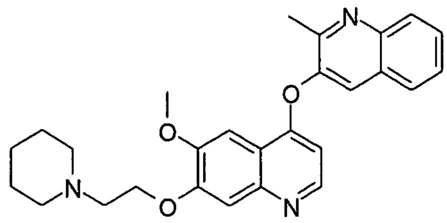 | 100 | 100 | |
| r 359 | 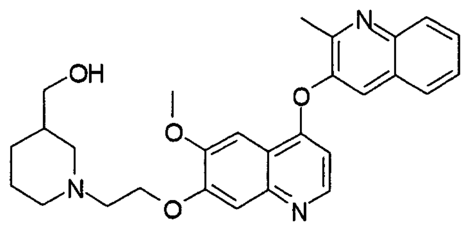 | 100 | 100 | |
| r 360 | 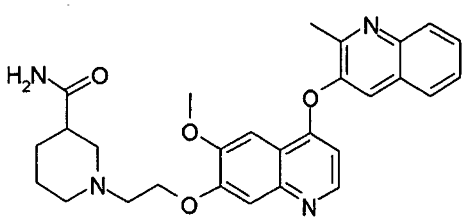 | 100 | 100 | |
| r 361 | 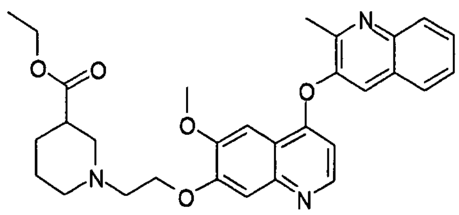 | 100 | 96 | |
| r 362 | 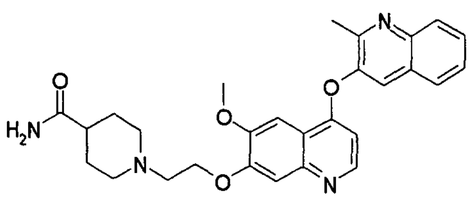 | 100 | 100 | |

(continued)

| Compound | Molecular structure | TGFβ inhibition rate, % | | |
|---|---|---|---|---|
| | | 10 μM | 3 μM | 1 μM |
| r 363 | 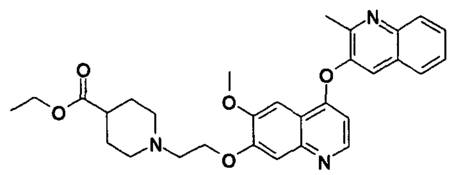 | 100 | 100 | |
| r 364 | 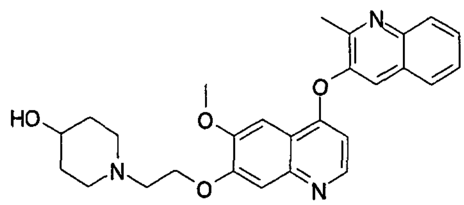 | 100 | 100 | |
| r 365 | 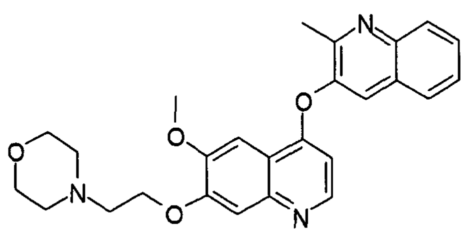 | 100 | 100 | |
| r 366 | 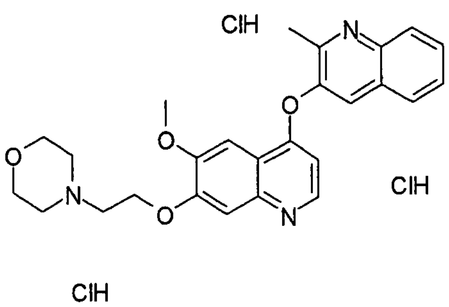 | 100 | 100 | |
| r 367 | 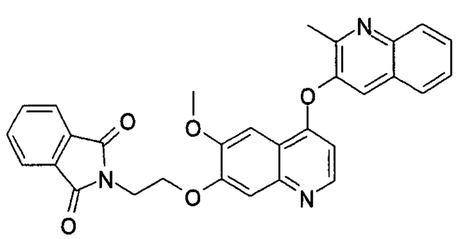 | 98 | 95 | |

(continued)

| Compound | Molecular structure | TGFβ inhibition rate, % | | |
|---|---|---|---|---|
| | | 10 μM | 3 μM | 1 μM |
| r 368 | 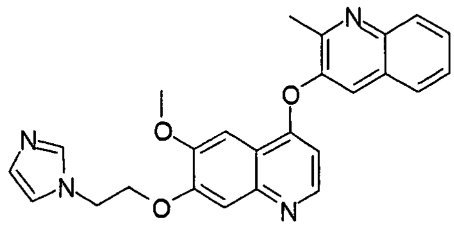 | 100 | 100 | |
| r 369 | 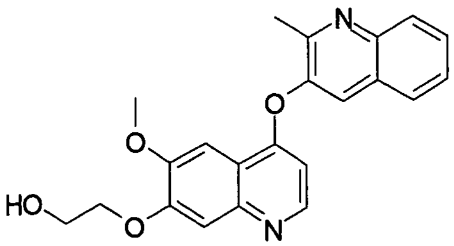 | 100 | 99 | |
| r 370 | 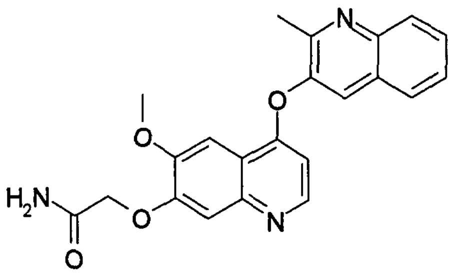 | 99 | 85 | |
| r 371 | 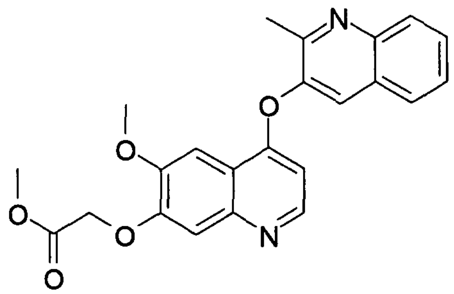 | 90 | 28 | |
| r 372 | 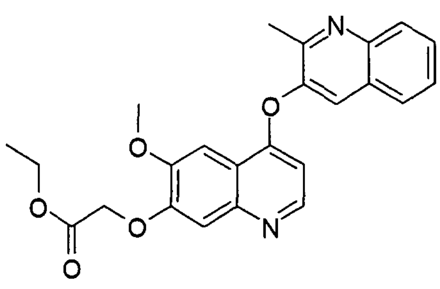 | 93 | 14 | |

(continued)

| Compound | Molecular structure | TGFβ inhibition rate, % | | |
|---|---|---|---|---|
| | | 10 μM | 3 μM | 1 μM |
| r 373 | 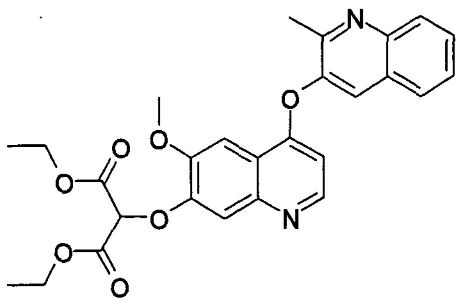 | 86 | 48 | |
| r 374 | 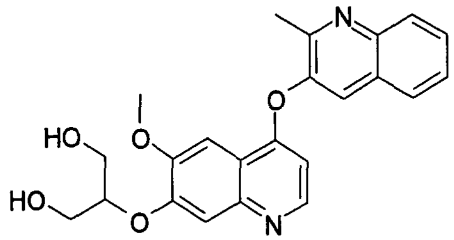 | 79 | 26 | |
| r 375 | 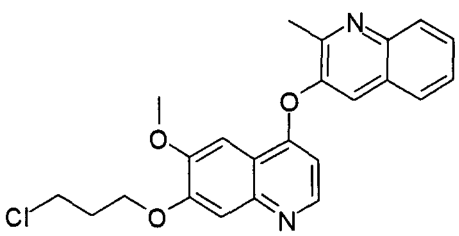 | 100 | 84 | |
| r 376 | 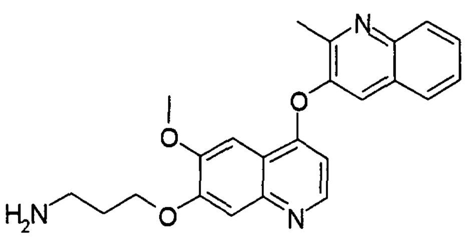 | 100 | 100 | |

(continued)

| Compound | Molecular structure | TGFβ inhibition rate, % | | |
|---|---|---|---|---|
| | | 10 μM | 3 μM | 1 μM |
| r 377 | 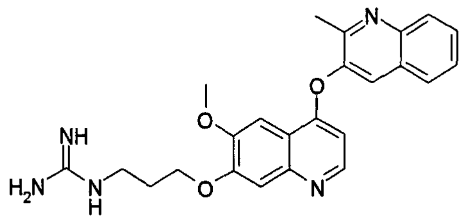 | 100 | 93 | |
| r 378 | 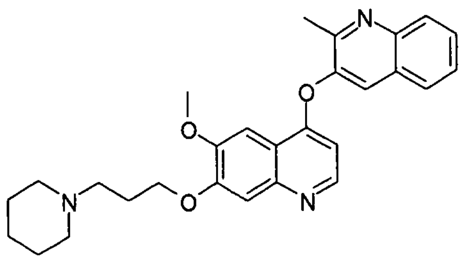 | 100 | 100 | |
| r 379 | 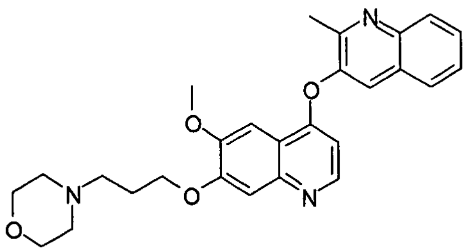 | 100 | 100 | |
| r 380 | 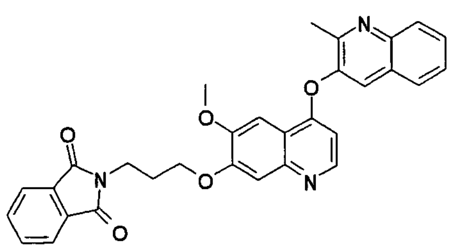 | 89 | 50 | |

(continued)

| Compound | Molecular structure | TGFβ inhibition rate, % | | |
|---|---|---|---|---|
| | | 10 μM | 3 μM | 1 μM |
| r 381 | 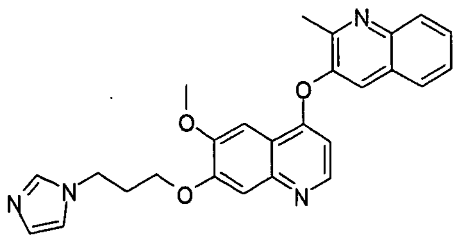 | 100 | 100 | |
| r 382 | 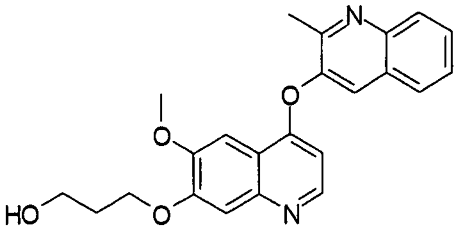 | 100 | 100 | |
| r 383 | 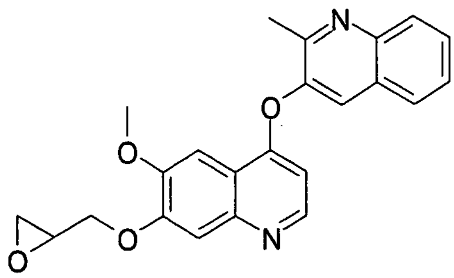 | 100 | 97 | |
| r 384 | 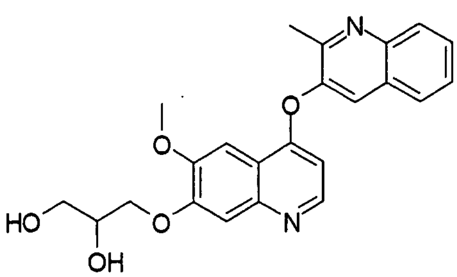 | 100 | 100 | |

(continued)

| Compound | Molecular structure | TGFβ inhibition rate, % | | |
|---|---|---|---|---|
| | | 10 μM | 3 μM | 1 μM |
| r 385 | 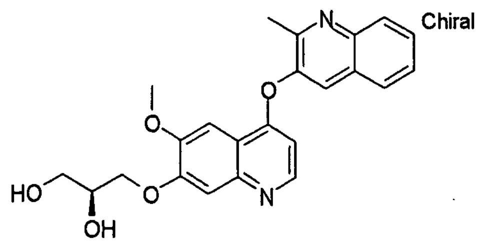 | 100 | 100 | |
| r 386 | 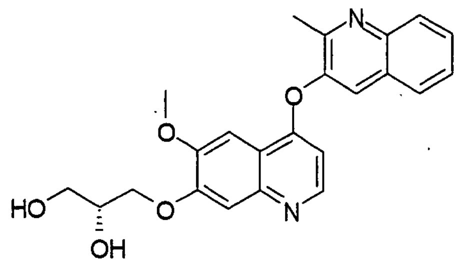 | | 99 | |
| r 387 | 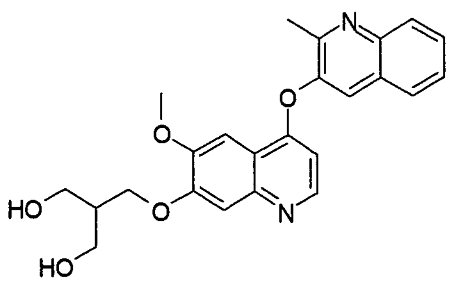 | 100 | 99 | |
| r 388 | 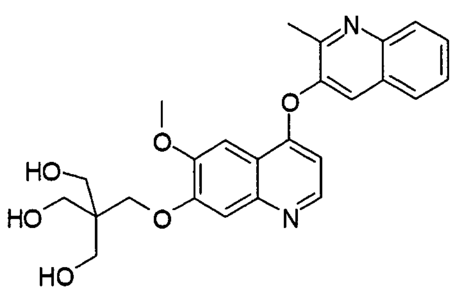 | 100 | 88 | |

(continued)

| Compound | Molecular structure | TGFβ inhibition rate, % | | |
|---|---|---|---|---|
| | | 10 μM | 3 μM | 1 μM |
| r 389 | 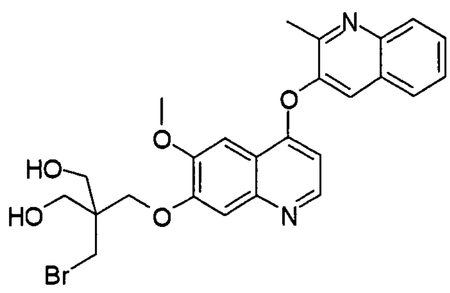 | 100 | 100 | |
| r 390 | 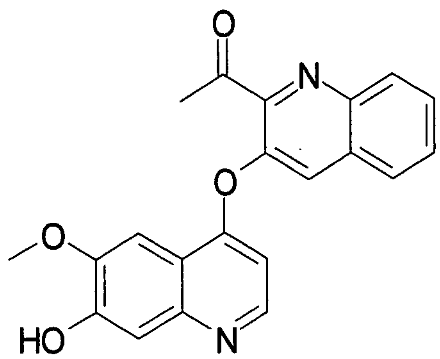 | 64 | 16 | |
| r 391 | 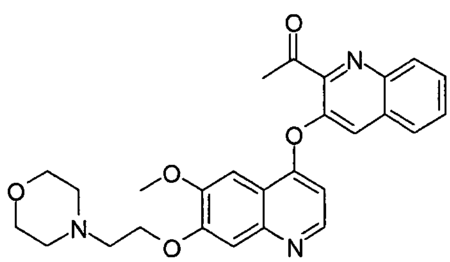 | 100 | 99 | |
| r 392 | 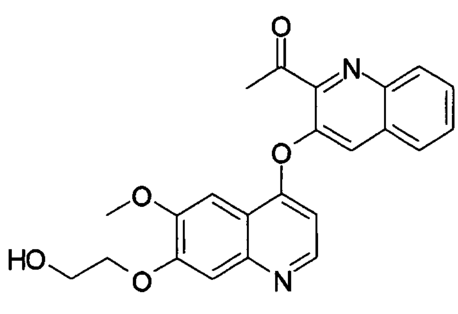 | 100 | 93 | |

(continued)

| Compound | Molecular structure | TGFβ inhibition rate, % | | |
|---|---|---|---|---|
| | | 10 μM | 3 μM | 1 μM |
| r 393 | 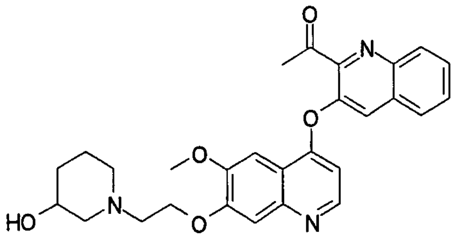 | | 100 | |
| r 394 | 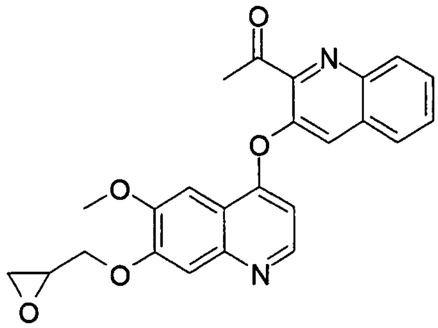 | 100 | 83 | |
| r 395 | 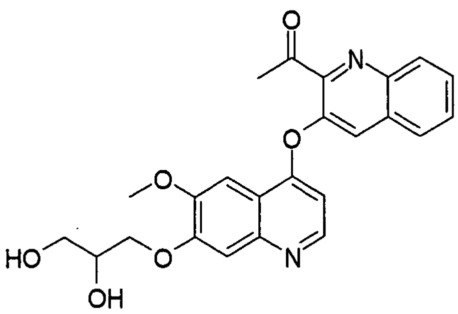 | 100 | 96 | |
| r 396 | 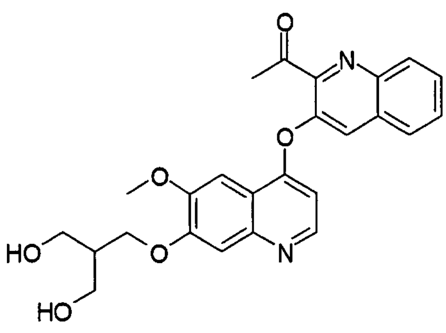 | | 96 | |

(continued)

| Compound | Molecular structure | TGFβ inhibition rate, % | | |
|---|---|---|---|---|
| | | 10 μM | 3 μM | 1 μM |
| r 397 | 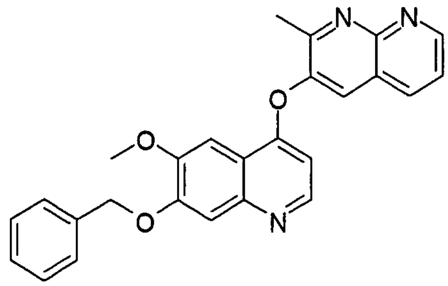 | 100 | 71 | |
| r 398 | 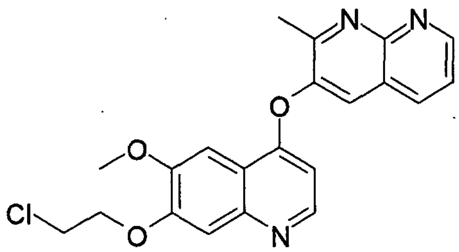 | 97 | 78 | |
| r 399 | 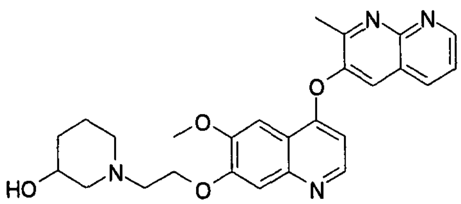 | 100 | 99 | |
| r 400 | 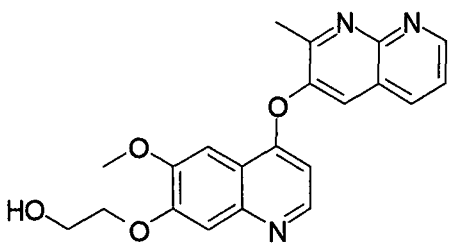 | 100 | 82 | |

(continued)

| Compound | Molecular structure | TGFβ inhibition rate, % | | |
|---|---|---|---|---|
| | | 10 μM | 3 μM | 1 μM |
| r 401 | 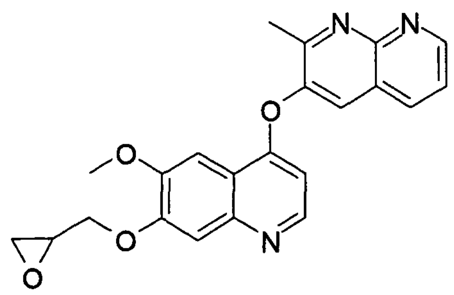 | 76 | 24 | |
| r 402 | 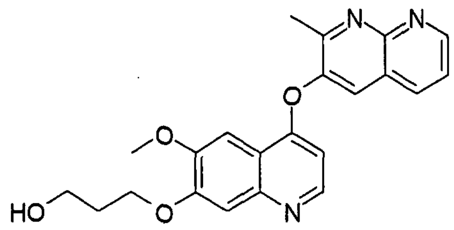 | 100 | 90 | |
| r 403 | 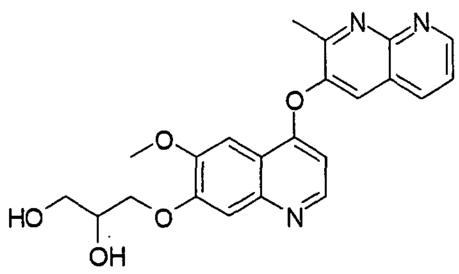 | 66 | 27 | |
| r 404 | 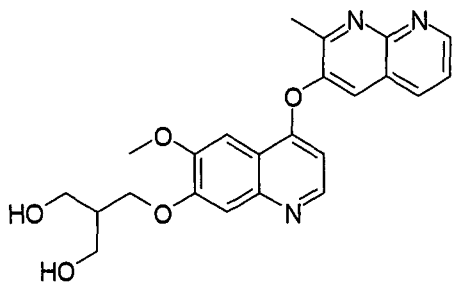 | 72 | 29 | |

(continued)

| Compound | Molecular structure | TGFβ inhibition rate, % | | |
|---|---|---|---|---|
| | | 10 μM | 3 μM | 1 μM |
| r 405 | 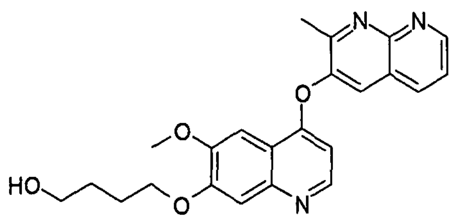 | 100 | 92 | |
| r 406 | 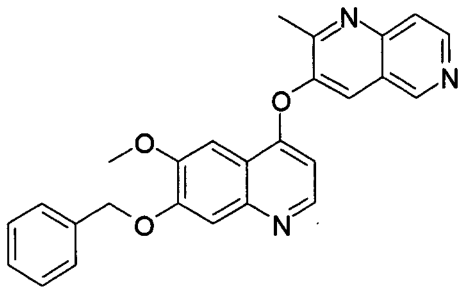 | 96 | 56 | |
| r 407 | 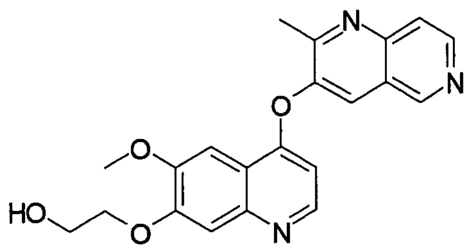 | 97 | 71 | |
| r 408 | 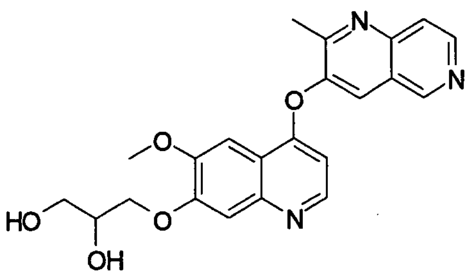 | 79 | 38 | |
| r 409 | 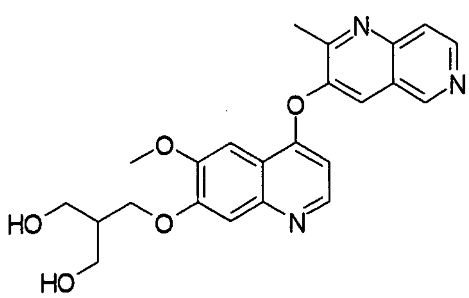 | 88 | 41 | |

(continued)

| Compound | Molecular structure | TGFβ inhibition rate, % | | |
|---|---|---|---|---|
| | | 10 μM | 3 μM | 1 μM |
| r 410 | 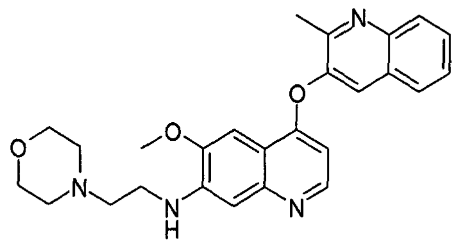 | 100 | 91 | |
| r 411 | 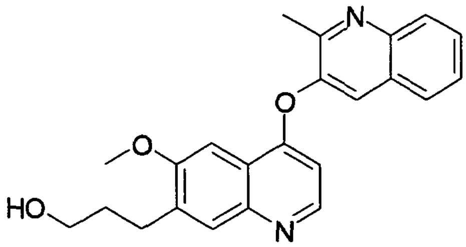 | 85 | 50 | |
| r 412 | 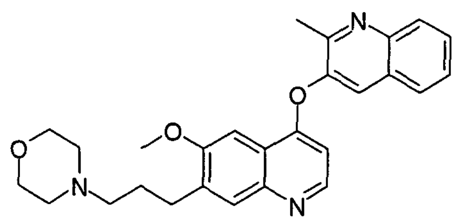 | 99 | 77 | |
| r 413 | 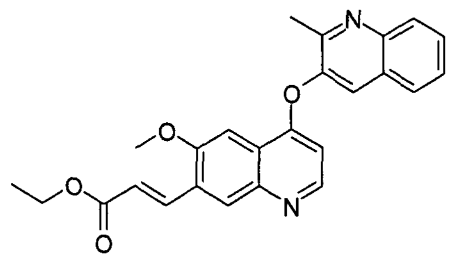 | 76 | 31 | |
| r 414 | 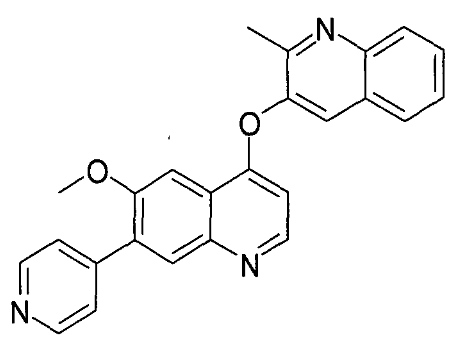 | 100 | 82 | |

(continued)

| Compound | Molecular structure | TGFβ inhibition rate, % | | |
|---|---|---|---|---|
| | | 10 μM | 3 μM | 1 μM |
| r 415 | 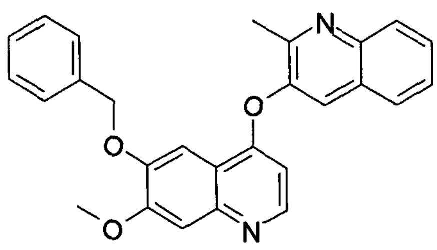 | 100 | 77 | |
| r 416 | 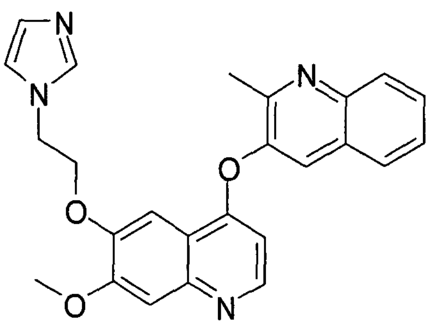 | 100 | 92 | |
| r 417 | 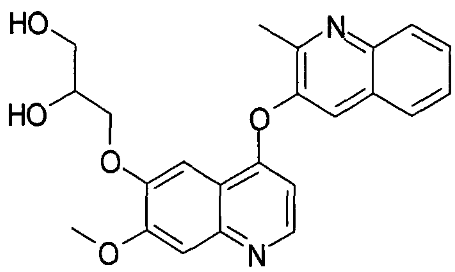 | 100 | 98 | |
| r 418 | 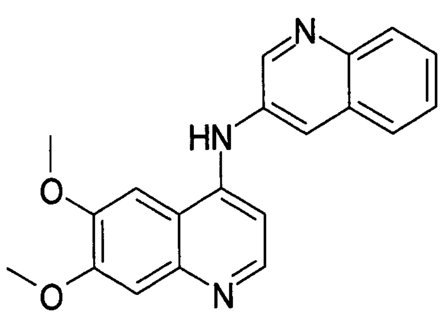 | 73 | 46 | |

(continued)

| Compound | Molecular structure | TGFβ inhibition rate, % | | |
|---|---|---|---|---|
| | | 10 μM | 3 μM | 1 μM |
| r 419 | 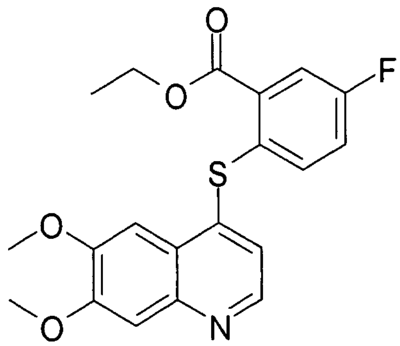 | 67 | 7 | |
| r 420 | 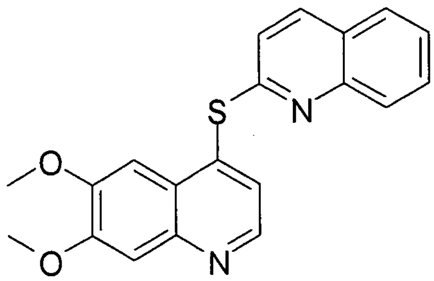 | 62 | 2 | |
| r 421 | 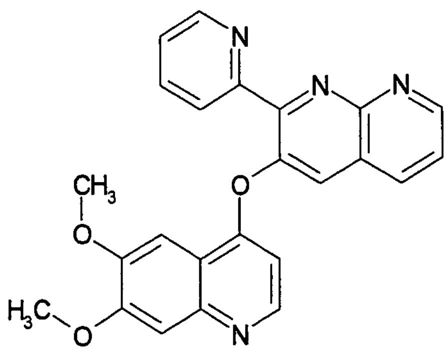 | 100 | 94 | |
| r 422 | 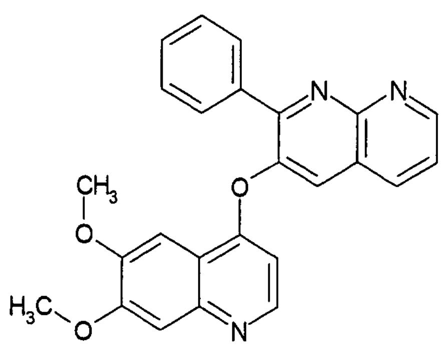 | 100 | 100 | |

(continued)

| Compound | Molecular structure | TGFβ inhibition rate, % | | |
|---|---|---|---|---|
| | | 10 μM | 3 μM | 1 μM |
| r 423 | 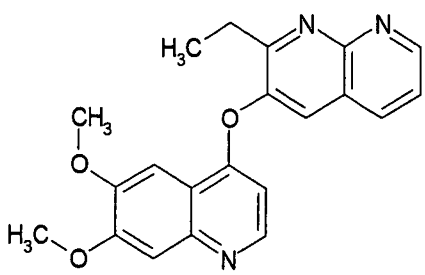 | 100 | 88 | |
| r 424 | 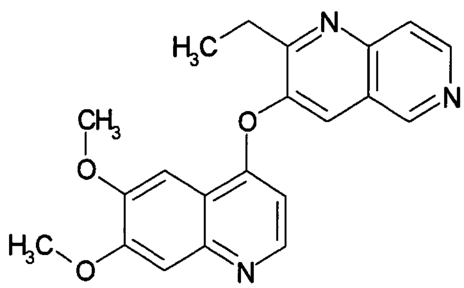 | 79 | 28 | |
| r 425 | 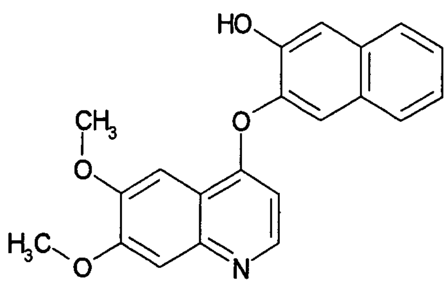 | 100 | 72 | |
| r 426 | 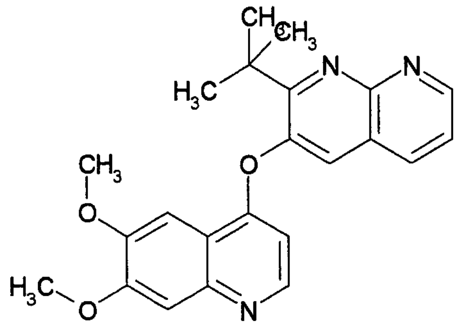 | | 100 | |

(continued)

| Compound | Molecular structure | TGFβ inhibition rate, % | | |
|---|---|---|---|---|
| | | 10 μM | 3 μM | 1 μM |
| r 427 | 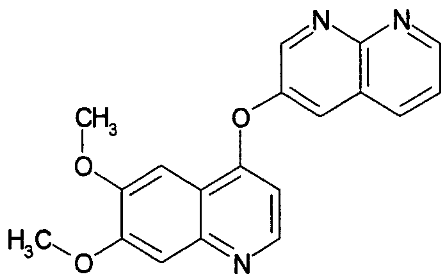 | 80 | 44 | |
| r 428 | 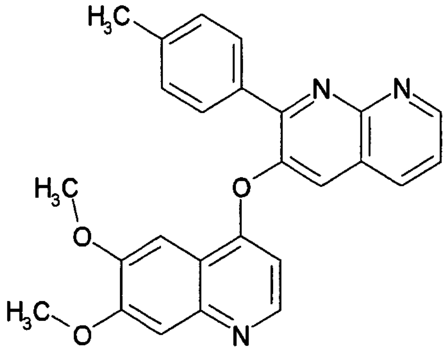 | | 67 | |
| r 429 | 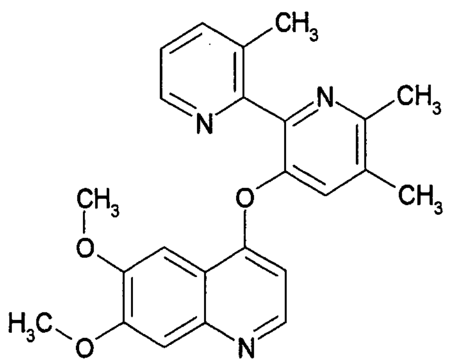 | 94 | 52 | |
| r 430 | 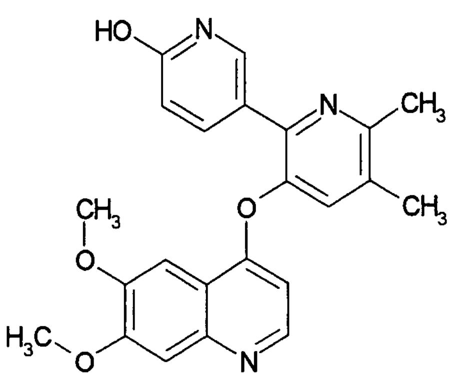 | 78 | 19 | |

(continued)

| Compound | Molecular structure | TGFβ inhibition rate, % | | |
|---|---|---|---|---|
| | | 10 μM | 3 μM | 1 μM |
| r 431 | 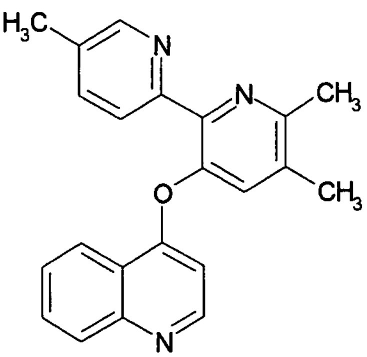 | 99 | 97 | |
| r 432 | 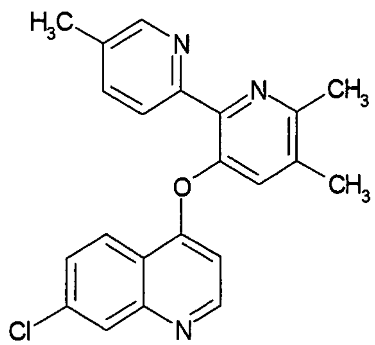 | 100 | 99 | |
| r 433 | 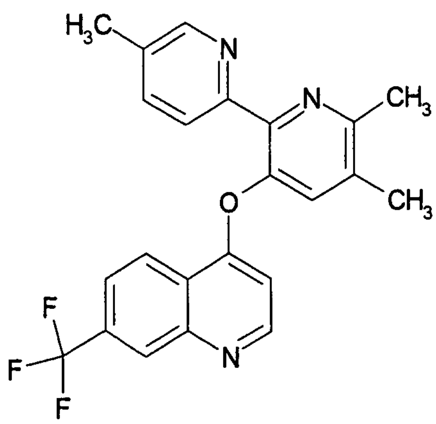 | 100 | 91 | |
| r 434 | 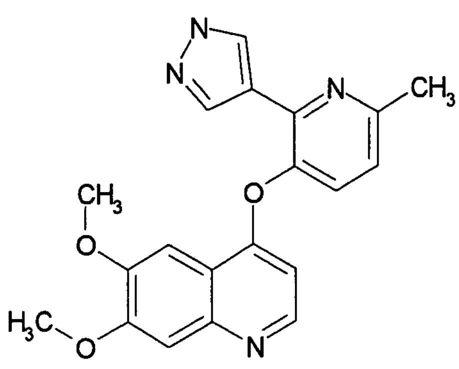 | 100 | 100 | |

(continued)

| Compound | Molecular structure | TGFβ inhibition rate, % | | |
|---|---|---|---|---|
| | | 10 μM | 3 μM | 1 μM |
| r 435 | 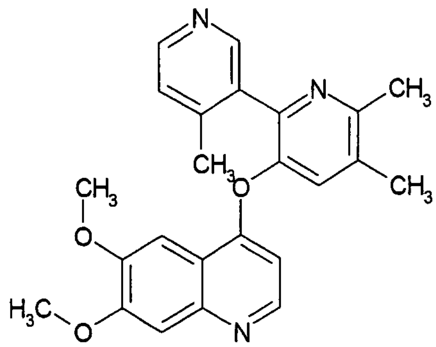 | 79 | 29 | |
| r 436 | 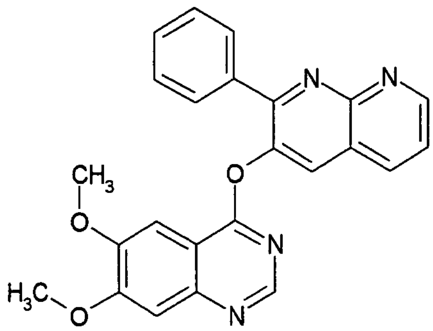 | 100 | 99 | |
| r 437 | 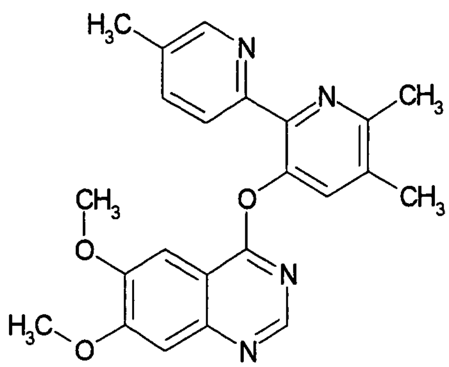 | 100 | 95 | |
| r 438 | 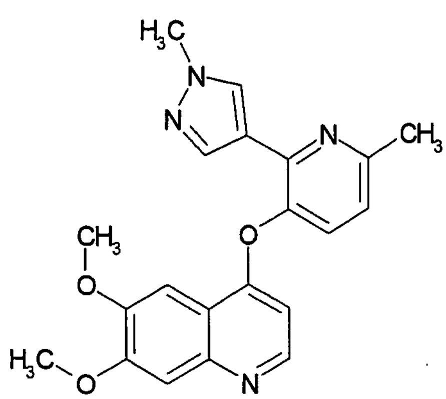 | 100 | 99 | |

(continued)

| Compound | Molecular structure | TGFβ inhibition rate, % | | |
|---|---|---|---|---|
| | | 10 μM | 3 μM | 1 μM |
| r 439 | 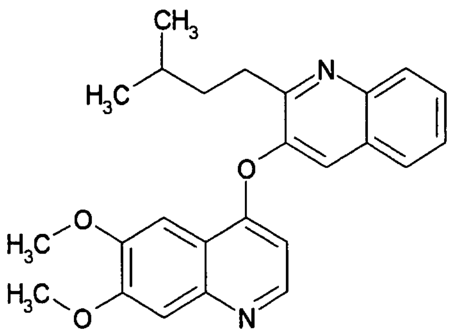 | 95 | 43 | |
| r 440 | 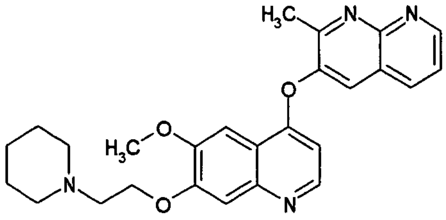 | 100 | 100 | |
| r 441 | 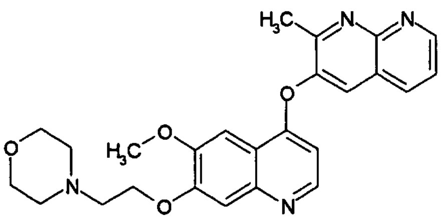 | 100 | 97 | |
| r 442 | 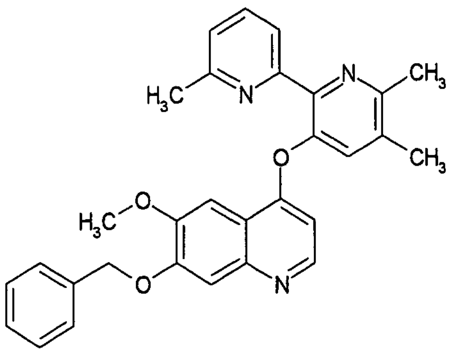 | 100 | 70 | |

(continued)

| Compound | Molecular structure | TGFβ inhibition rate, % | | |
|---|---|---|---|---|
| | | 10 μM | 3 μM | 1 μM |
| r 443 | 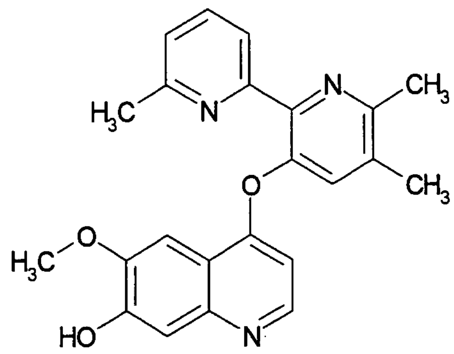 | 97 | 60 | |
| r 444 | 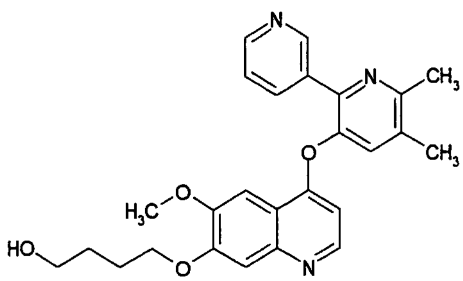 | | 100 | |
| r 445 | 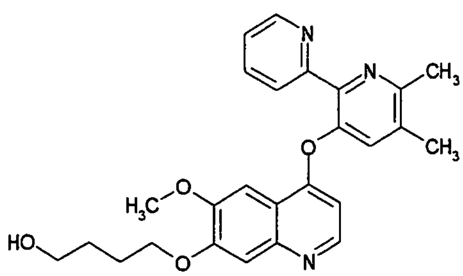 | | 99 | |
| r 446 | 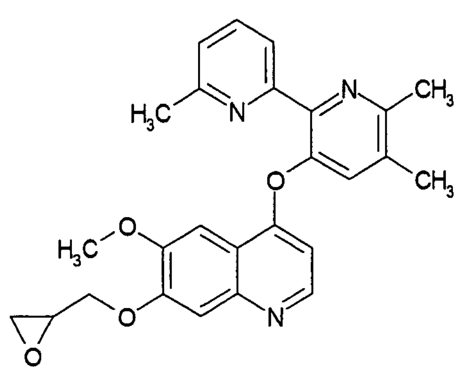 | 89 | 35 | |

(continued)

| Compound | Molecular structure | TGFβ inhibition rate, % | | |
|---|---|---|---|---|
| | | 10 μM | 3 μM | 1 μM |
| r 447 | 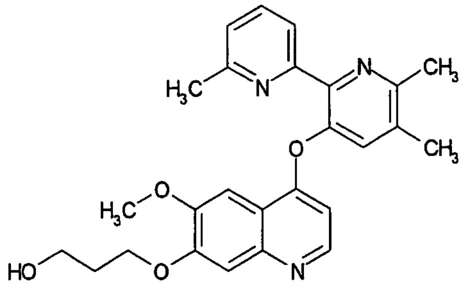 | 100 | 94 | |
| r 448 | 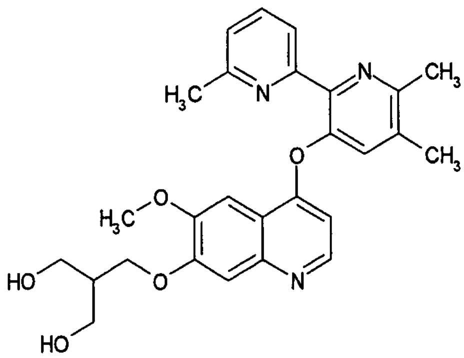 | 97 | 70 | |
| r 449 | 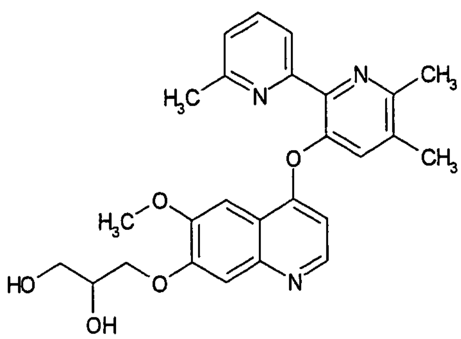 | 98 | 74 | |
| r 450 | 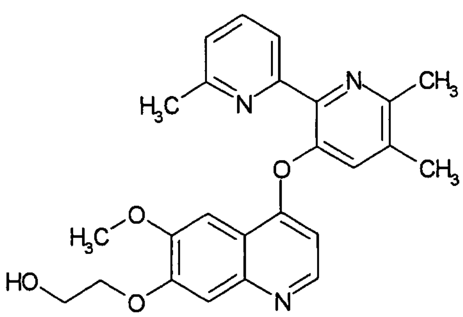 | 100 | 94 | |

(continued)

| Compound | Molecular structure | TGFβ inhibition rate, % | | |
|---|---|---|---|---|
| | | 10 μM | 3 μM | 1 μM |
| r 451 | 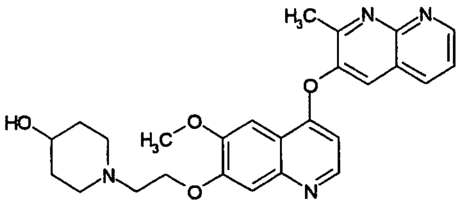 | 100 | 99 | |
| r 452 | 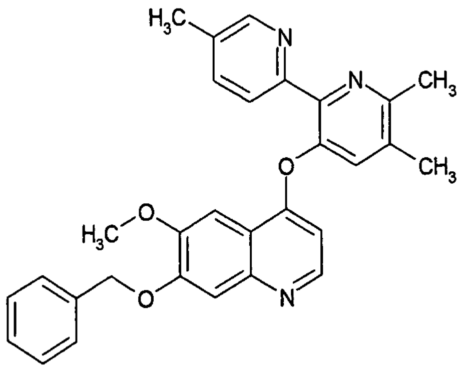 | 99 | 67 | |
| r 453 | 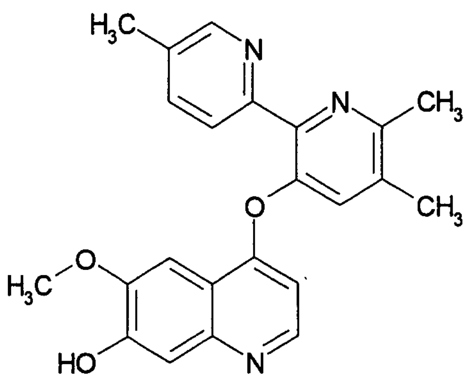 | 98 | 72 | |
| r 454 | 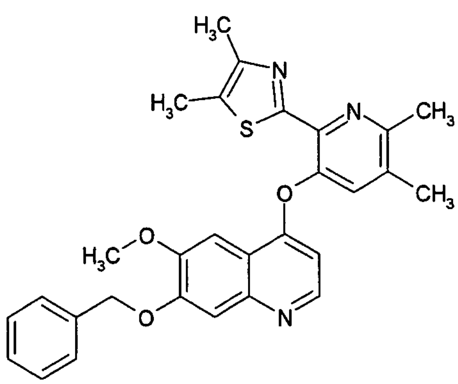 | 75 | 41 | |

(continued)

| Compound | Molecular structure | TGFβ inhibition rate, % | | |
|---|---|---|---|---|
| | | 10 μM | 3 μM | 1 μM |
| r 455 | 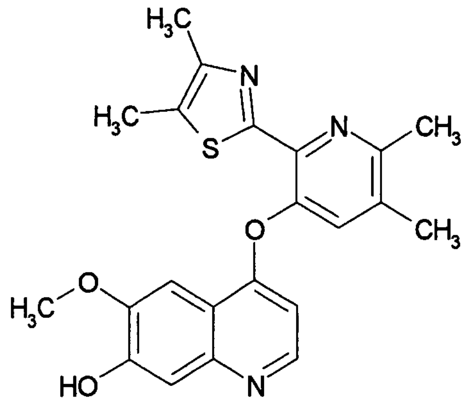 | 99 | 76 | |
| r 456 | 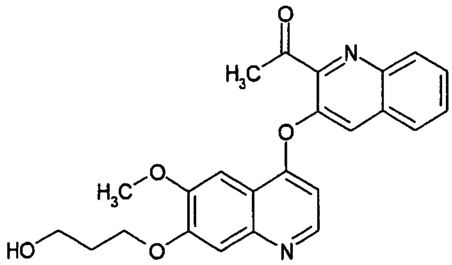 | 100 | 99 | |
| r 457 | 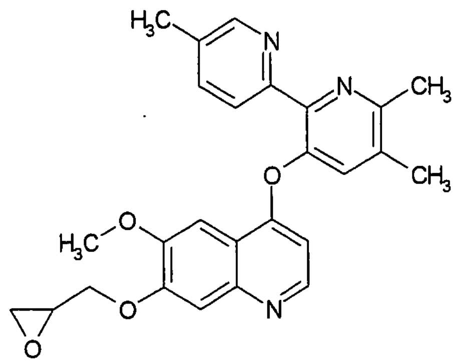 | 100 | 97 | |
| r 458 | 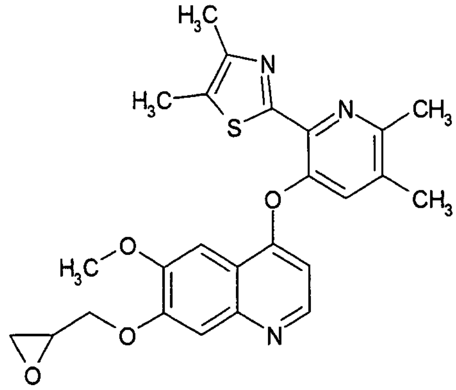 | 100 | 94 | |

(continued)

| Compound | Molecular structure | TGFβ inhibition rate, % | | |
|---|---|---|---|---|
| | | 10 μM | 3 μM | 1 μM |
| r 459 | 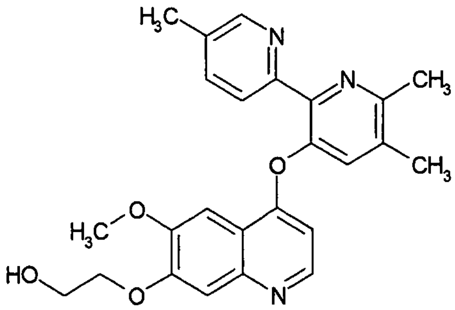 | | 98 | |
| r 460 | 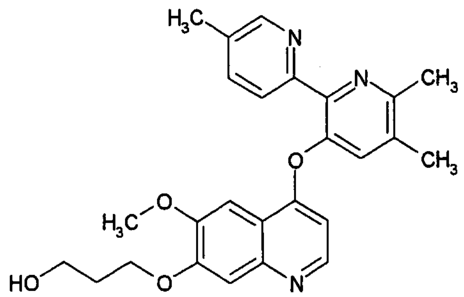 | | 97 | |
| r 461 | 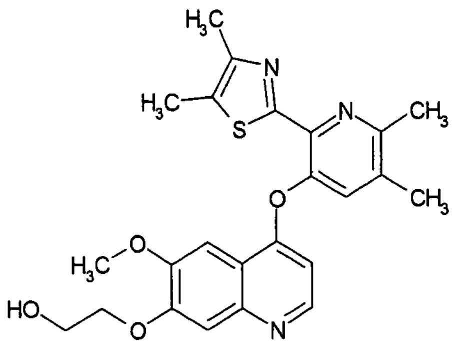 | | 82 | |
| r 462 | 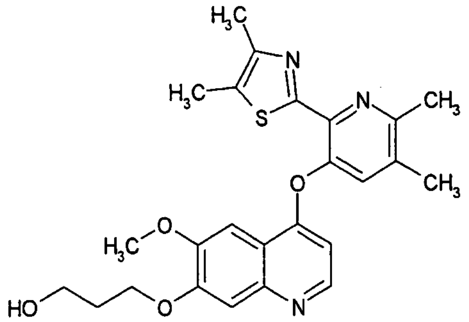 | | 99 | |

(continued)

| Compound | Molecular structure | TGFβ inhibition rate, % | | |
|---|---|---|---|---|
| | | 10 μM | 3 μM | 1 μM |
| r 463 | 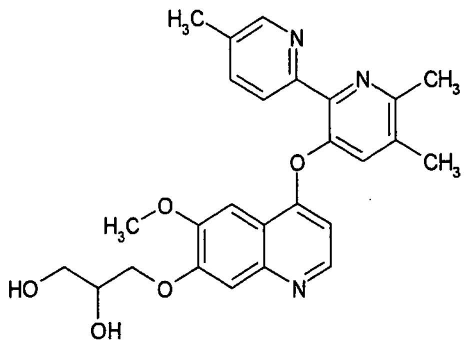 | 99 | 77 | |
| r 464 | 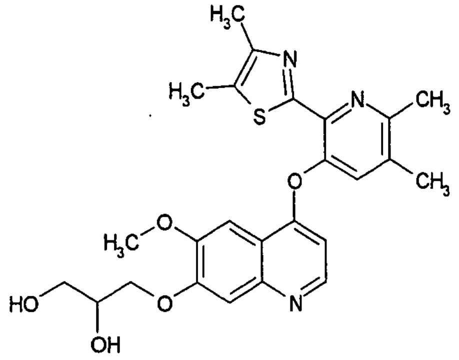 | 100 | 97 | |
| r 465 | 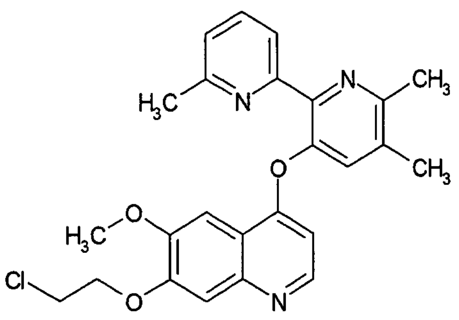 | 100 | 98 | |
| r 466 | 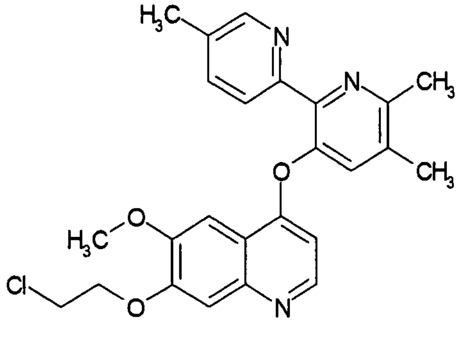 | 100 | 99 | |

(continued)

| Compound | Molecular structure | TGFβ inhibition rate, % | | |
|---|---|---|---|---|
| | | 10 μM | 3 μM | 1 μM |
| r 467 | 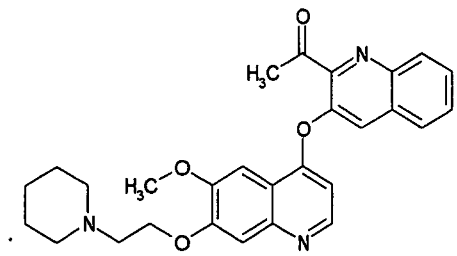 | | 100 | |
| r 468 | 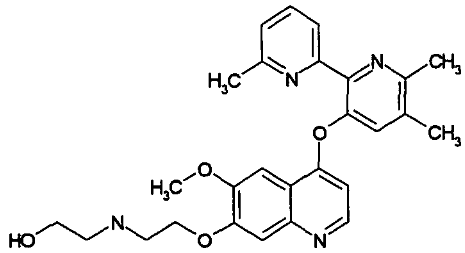 | | 99 | |
| r 469 | 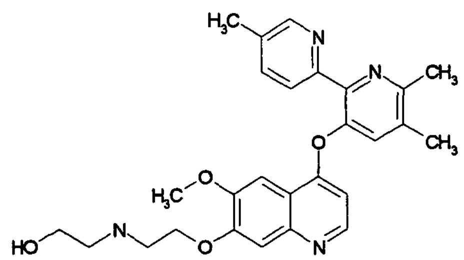 | | 100 | |

Test Example 2: Measurement of anti-fibrotic activity using mouse unilateral ureteral obstruction (UUO) model

The anti-fibrotic activity of the compounds related to the present invention was evaluated using a mouse unilateral ureteral obstruction (UUO) model which is a renal fibrosis model. Compound r384 produced in the working example was used as the test compound.

Seven week-old male BALB/c mice (available from Charles River Japan, Inc.) were purchased and were pre-raised before use in experiments. Regarding diets and drinking water, pellets CE-2 (available from CLEA JAPAN INC.) and sterile tap water were freely fed.

The mice underwent laparotomy under pentobarbital anesthesia, and the left renal urinary duct was ligated. Thereafter, the mice underwent suturing operation and were divided into a vehicle administration group (n = 7) and a test compound administration group (n = 7) using the weight as an index.

The test compound was weighed, and then one drop of 1 N HCl was added to the test compound to prepare a solution. The solution was then suspended in 0.5% carboxymethylcellulose (solvent). The suspension was forcibly orally administered twice a day with an oral sonde from the day of urinary duct ligation (5, 15, or 50 mg/kg). The solvent was administered to the vehicle administration group in the same manner as described just above.

After administration for 4 days, the mouse left kidney was removed, and the hydroxyproline content as an index of organ fibrosis was measured by the following method.

Renal pieces were first placed in 6 N HCl, were homogenized, and were then heated on a heat block at 130˚C for 3 hr to hydrolyze protein. Thereafter, the suspension of renal pieces was neutralized by the addition of an appropriate amount of 4 N NaOH. The neutralized suspension was centrifuged (1000 rpm, 5 min, room temperature) to obtain the supernatant as a kidney extract. A chloramine T liquid, a perchloric acid solution (a solution prepared by adding distilled water to

31.5 ml of 60% perchloric acid to adjust the total volume to 100 ml), and a p-dimethylaminobenzaldehyde solution (a solution prepared by adding methylcellulose to 20 g of p-dimethylaminobenzaldehyde to adjust the total volume to 100 ml) were added to the kidney extract, and the reaction was allowed to proceed at 60˚C for 20 min. The absorbance at 557 nM was then measured. The content of hydroxyproline was determined from the measured data based on a calibration curve for hydroxyproline prepared using control. Further, the content of hydroxyproline thus determined was corrected according to the weight of the homogenized kidney.

The results were as shown in Table 2. The values in the table are average of data on 7 mice for each group ± standard deviation.

The result showed that the content of hydroxyproline in the UUO treatment mouse kidney was increased as compared with the kidney of normal mice, suggesting that extracellular matrix proteins were accumulated within the kidney. In contrast, the content of hydroxyproline in the mouse to which the test compound had been administrated was lowered, as compared with that of the solvent administration group, indicating that the compound could suppress the accumulation of extracellular matrix proteins in the kidney.

Table 2:

| Administration group | Hydroxyproline content (mg/g) |
|---|---|
| Normal mice | 392.9 ± 10.8 |
| UUO treatment + solvent | 549.4 ± 15.2 ### |
| UUO treatment + compound r384 (5 mg/kg) | 514.4 ± 20.8 |
| UUO treatment + compound r384 (15 mg/kg) | 487.7 ± 10.9 ** |
| UUO treatment + compound r384 (50 mg/kg) | 425.7 ± 14.2 *** |

In the table, ### indicates that $p < 0.001$ in Student's t-test against the normal mouse group, and ** and *** respectively indicate that $p < 0.01$ and $p < 0.001$ in Student's t-test against UUO treatment + solvent group.

Test Example 3: Measurement of anti-fibrotic activity using mouse unilateral ureteral obstruction (UUO) model

The anti-fibrotic activity of a compound related to the present invention was evaluated using the same model as used in Test Example 2.

Compound r320 prepared in the working example was used as the test compound.

The experiment method and evaluation method were the same as those in Test Example 2, except that the compound administration was carried out by feeding a diet mixed with the compound for 10 days and the correction of hydroxyproline content was changed to the content of protein in the kidney extract.

The results were as shown in Table 3. The values in the table are average of data on five mice for each group ± standard deviation.

The result showed that the content of hydroxyproline in the UUO treatment mouse kidney was increased as compared with the kidney of normal mice, suggesting that extracellular matorix was accumulated within the kidney. In contrast, the content of hydroxyproline in the mouse to which the test compound had been administrated was lowered, as compared with that of the solvent administration group, indicating that the compound could suppress the accumulation of extracellular substrate in the kidney.

Table 3:

| Administration group | Hydroxyproline content (mg/g protein) |
|---|---|
| Normal mice | 6.7 ± 1.26 |
| UUO treatment + solvent | 12.7 ± 1.14 # |
| UUO treatment + compound r320 (0.1% mixed feed) | 9.44 ± 0.53 * |
| UUO treatment + compound r320 (0.3% mixed feed) | 9.19 ± 0.38 * |

In the table, # indicates that $p < 0.05$ in Student's t-test against the normal mouse group, and ** indicates that $p < 0.05$ in Student's t-test against UUO treatment + solvent group.

Test Example 4: Measurement of anti-fibrotic activity using mouse DMN hepatic fibrosis model

The anti-fibrotic activity of a compound related to the present invention was evaluated using a mouse DMN

hepatic fibrosis model as a liver fibrosis model. Compound r384 prepared in the working example was used as the test compound.

Six-week-old male BALB/c mice (available from Charles River Japan, Inc.) were purchased and were pre-raised before use in experiments.

The mice were divided into a vehicle administration group (n = 6) and a test compound administration group (n = 7) using the weight as an index. Thereafter, dimethylnitrosoamine (DMN) diluted with physiological saline was intraperitoneally administered at 15 mg/kg three times per week for 3 weeks to induce hepatic fibrosis.

The administration of the test compound was carried out from the first day of the DMN administration. The test compound was mixed at the dosage of 0.015, 0.03, and 0.06% in powder diet CE-2 (available from CLEA JAPAN INC.), and the mixtures were fed as a diet to each mouse group.

After 21 days, the mice underwent laparotomy under ether anesthesia, and blood was collected from the heart, followed by measurement of the level of hyaluronic acid in blood as an index of hepatic fibrosis using a hyaluronic acid plate (Chugai Pharmaceutical Co., Ltd.).

The results were as shown in Table 4. The values in the table are average of data on 6 mice for each group ± standard deviation.

The result showed that the blood hyaluronic acid level in the mouse liver to which DMN had been administered was increased as compared with the liver of normal mice, suggesting that extracellular matrix proteins were accumulated within the liver. In contrast, the blood hyaluronic acid level in the mouse to which the test compound had been administered was lowered as compared with that of the solvent administration group, indicating that the compound could suppress the accumulation of extracellular matrix proteins in the liver.

Table 4:

| Adminstration group | Blood hyaluronic acid content (ng/ml) |
|---|---|
| Normal mice | 113.8 ± 14.9 |
| DMN administration + solvent | 326.4 ± 47.1 ## 326.4 ± 47.1 ## |
| DMN administration + compound r384 (0.015% mixed feed) | 167.8 ± 25.0 * |
| DMN administration + compound r384 (0.03% mixed feed) | 108.5 ± 9.0 ** |
| DMN administration + compound r384(0.06% mixed feed) | 97.2 + 11.0** |

In the table, ## indicates that $p < 0.01$ in Student's t-test against the normal mouse group, and * and ** respectively indicate that $p < 0.05$ and $p < 0.01$ in Student's t-test against UUO treatment + solvent group.

Test Example 5: Measurement of anti-fibrotic activity using rat unilateral ureteral obstruction (UUO) model

The anti-fibrotic activity of the compounds described in the present specification was evaluated using a rat unilateral ureteral obstruction (UUO) model which is a renal fibrosis model. Compound 50 and compound 159 were used as the test compound.

Six week-old male SD rats (available from Charles River Japan, Inc.) were purchased and were pre-raised before use in experiments. Regarding diets and drinking water, pellets CE-2 (available from CLEA JAPAN INC.) and sterile tap water were freely fed.

The rats underwent laparotomy under pentobarbital anesthesia, and the left renal urinary duct was ligated. Thereafter, the mice underwent suturing operation and were divided into a vehicle administration group (n = 6) and a test compound administration group (n = 6) using the weight as an index.

The test compound was mixed in amounts of 0.03% and 0.1% in powder diet CE-2 (available from CLEA JAPAN INC.), and the mixtures were fed as a diet to each rat group from the first day of the ureteral obstruction.

After administration of the mixed feed for 7 days, the left kidney was removed, and the hydroxyproline content as an index of organ fibrosis was measured by the following method. Specifically, renal pieces were first placed in 6 N HCl, were homogenized, and were then heated on a heat block at 130˚C for 3 hr to hydrolyze protein. Thereafter, the suspension of renal pieces was neutralized by the addition of an appropriate amount of 4 N NaOH. The neutralized suspension was centrifuged (1000 rpm, 5 min, room temperature) to obtain the supernatant as a kidney extract. A chloramine T liquid, a perchloric acid solution (a solution prepared by adding distilled water to 31.5 ml of 60% perchloric acid to adjust the total volume to 100 ml), and a p-dimethylaminobenzaldehyde solution (a solution prepared by adding methylcellulose to 20 g of p-dimethylaminobenzaldehyde to adjust the total volume to 100 ml) were added to the kidney extract, and a reaction was allowed to proceed at 60˚C for 20 min. The absorbance at 557 nM was then measured. The

content of hydroxyproline was determined from the measured data based on a calibration curve for hydroxyproline prepared using control. Further, the content of hydroxyproline thus determined was corrected according to the total protein content of the kidney extract.

The results were as shown in Tables 5a and 5b.

The values in the tables are average of data on 6 rats for each group ± standard deviation. The result showed that the content of hydroxyproline in the UUO treatment rat kidney was increased, as compared with the kidney of normal rats, suggesting that extracellular matrix proteins were accumulated within the kidney. In contrast, the content of hydroxyproline in the rat administrated the test compounds was lowered as compared with the solvent administration group, for the group to which had been administered, , indicating that the compounds could suppress the accumulation of extracellular matrix proteins in the kidney.

Table 5a:

| Administration group | Hydroxyproline content (mg/g) |
|---|---|
| Normal rats | 4.47 ± 0.17 |
| UUO treatment + solvent | 8.24 ± 0.31 ### |
| UUO treatment + compound 50 (0.03% mixed feed) | 7.33 ± 0.67 |
| UUO treatment + compound 50 (0.1% mixed feed) | 4.67 ± 0.22 *** |

Table 5b:

| Administration group | Hydroxyproline content (mg/g) |
|---|---|
| Normal rats | 18.84 ± 1.05 |
| UUO treatment + solvent | 27.16 ± 2.65 ### |
| UUO treatment + compound 159 (0.03% mixed feed) | 22.47 ± 0.82 |
| UUO treatment + compound 159 (0.1% mixed feed) | 17.28 ± 0.87 *** |

In the tables, ### indicates that p < 0.001 in Student's t-test against the normal rat group, and *** indicates that p < 0.001 in Student's t-test against UUO treatment + solvent group.

Test Example 6: Measurement of cell growth inhibitory activity using A549 cells

Human lung cancer epithelial cells (A549) (available from ATCC) were suspended to a concentration of 3 × $10^4$/ml in a DMEM/F12 medium containing 10%FCS, and the suspension was inoculated in 100 μl portions in wells of a 96-well plate.

On the following day, a stock solution of the test compound which had been adjusted to 10 mM with DMSO was adjusted with a serum-free DMEM/F12 medium to a dose (60 μM) which was twice the evaluation concentration. After removing the medium inoculated in the 96 wells on the previous day, the solution was added in 50 μl portions to the plate. Next, a DMEM/F12 medium containing 10% serum was added in 50 μl portions to the wells, followed by culturing at 37°C for 40 hr.

The final concentration of the serum and the concentration of the evaluation compound were 5% and 30 μM, respectively. The compounds according to the present invention synthesized in the working example were used as the test compound. Compounds 261, 269 and 274 described in WO 2004/018430 were used as the comparative compound.

After 40 hr, the culture was removed, and 100 μl of a solution prepared by diluting counting kit-8 (purchased from Dojindo) with a serum-free F12 medium by 10 times was added, followed by culturing at 37°C for 20 to 30 min. After the confirmation of suitable color development, the absorbance at 450 nM was measured with ARVO (purchased from Wallach Bethold Japan). The cell growth inhibition in the test compound added wells were determined by presuming the absorbance value in the test compound-free wells to be 0% and the absorbance value in the cell-free wells to be 100%.

The results were as shown in Table 6. As is apparent from Table 6, the conventional compounds had a cell growth inhibition of about 30% to 45%, whereas the test compounds had substantially no cell growth inhibitory activity, indicating that the test compounds had a significantly improved potential as a medicament.

Table 6:

| Test compound | Cell growth inhibition rate (%) |
|---|---|
| Compound 261 of WO 2004/018430 | 44 |
| Compound 269 of WO 2004/018430 | 45 |
| Compound 205 | 9 |
| Compound 179 | -3 |
| Compound 181 | -7 |
| Compound 182 | -8 |
| Compound 184 | 8 |
| Compound 186 | -2 |
| Compound 187 | 0 |
| Compound 274 of WO 2004/018430 | 25 |
| Compound 192 | 3 |
| Compound 194 | -15 |
| Compound 200 | 0 |
| Compound 201 | 2 |
| Compound 202 | 8 |
| Compound 204 | -9 |
| Compound 206 | -1 |
| Compound 217 | 4 |
| Compound 222 | 6 |
| Compound 225 | 8 |

Test Example 7: Measurement of BMP signal inhibitory activity (in-vitro test)

The inhibitory activity of the compounds according to the present invention against BMP signals was studied using a cell line through which TGFb and BMP signals can be simultaneously observed.
Specifically, the cell lines capable of detecting the two signal was generated by transfecting reporter plasmids for detection of TGFb and BMP signals into a human hepatocarcinoma cell line HepG2(available from ATCC) and was used as evaluation cells. For detection of TGF-beta signal, the plasmid having four tandem binding sequences of Smad2/3, TGF-beta signal transducers, to the upstream of a luciferases gene were used as a reporter plasmid (p(SBE)4-Luc/hygro). For detection of BMP signal, the plasmid having twelve tandem binding sequences of Smadl/5/8, BMP signal transducers, to the upstream of a luciferases gene were used as a reporter plasmid (p(GCCG)12-Luc/neo), according to the method described in Mol Biol Cell. 2000 11(2): 555-65.
The test compound and TGFβ-1 (2 ng/ml) or BMP4 (20 ng/ml) were added to the cells, and the mixture was cultured for 4 hr. The compounds according to the present invention synthesized in the working example were used as the test compound. Compounds 261, 269 and 274 described in WO 2004/018430 were used as the comparative compound. After the culture, the luciferase activity of the cells was measured by chemiluminescence (Steady Glo (trademark) luciferase assay system, available from Promega).
Likewise, the luciferase activity was measured as control for the culture of cells with only TGFβ or BMP4 added thereto and the culture of cells with the addition of none of TGFβ, BMP4 and the test compound.
The inhibitory activity IC50 of the compounds against TGFβ and BMP4 was calculated based on the results of measurement, and the kinase selectivity for BMP4 was determined by the following method.
Kinase selectivity for BMP4 (times) = Inhibitory activity against BMP4 signal (IC50)/Inhibitory activity against TGFβ signal (IC50)
The results were as shown in Table 7.
As is apparent from the table, the selectivity of the conventional compounds was as low as about four times to ten times, whereas, for all the test compounds, the selectivity for BMP signal was 20 times or higher, indicating that the test compounds had a significantly improved potential as a medicament.
Table 7:

| Test compound | Selectivity for BMP (times) |
|---|---|
| Compound 261 of WO 2004/018430 | 4.1 |

(continued)

| Test compound | Selectivity for BMP (times) |
|---|---|
| Compound 269 of WO 2004/018430 | 11.9 |
| Compound 181 | 308 |
| Compound 182 | 195 |
| Compound 184 | > 428 |
| Compound 187 | 72 |
| Compound 274 of WO 2004/018430 | 6.7 |
| Compound 192 | > 176 |
| Compound 194 | 51 |
| Compound 200 | 75 |
| Compound 202 | 21.4 |

This application is based upon and claims the benefit of priority from the prior Japanese Patent Applications No. 45383/2004, filed on February 20, 2004, the entire contents of which are incorporated herein by reference.

## Claims

**1.** A compound of formula (I) or a pharmaceutically acceptable salt or solvate thereof:

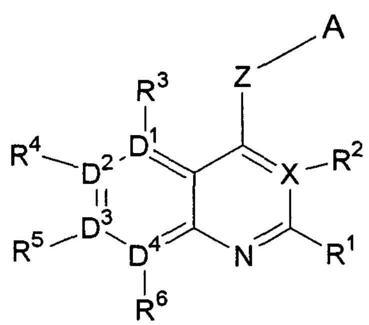

(I)

wherein
A represents a group of formula (a):

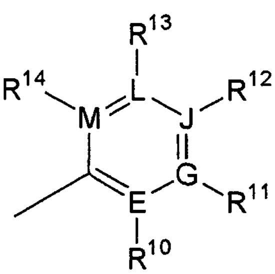

(a)

Z represents -O-, -N(-$R^Z$)-, -S-, or -C(=O)- wherein $R^Z$ represents a hydrogen atom or unsubstituted C1-4 alkyl,
$D^1$, $D^2$, $D^3$, $D^4$, X, E, G, J, L, and M, which may be the same or different, represent C or N,
$R^1$ to $R^6$ and $R^{10}$ to $R^{14}$, which may be the same or different, represent

(1) a hydrogen atom;
(2) a halogen atom;
(3) hydroxyl;
(4) cyano group;

(5) nitro group;
(6) C1-6 alkyl;
(7) C2-6 alkenyl;
(8) C2-6 alkynyl;
(9) C1-6 alkoxy;
(10) C1-6 alkylthio;
wherein (6) C1-6 alkyl, (7) C2-6 alkenyl, (8) C2-6 alkynyl, (9) C1-6 alkoxy, and (10) C1-6 alkylthio are optionally substituted by

(I) hydroxyl,
(II) a halogen atom,
(III) C1-4 alkoxy,
(IV) an oxygen atom,
(V) a saturated or unsaturated three- to nine-membered carbocyclic or heterocyclic group wherein the carbocyclic or heterocyclic group is optionally substituted by C1-4 alkyl, C1-4 alkoxy, a halogen atom, or a five- or six-membered carbocyclic or heterocyclic group, and the C1-4 alkyl group is optionally substituted by hydroxyl or phenyl,
(VI) amino group wherein one or two hydrogen atoms in the amino group are optionally substituted by C1-4 alkyl or a five- or six-membered carbocyclic or heterocyclic group, and the C1-4 alkyl group is optionally substituted by hydroxyl or C1-4 alkoxy,
(VII) -NHCONHR$^{VII}$ wherein R$^{VII}$ represents C1-4 alkyl,
(VIII) -OCOR$^{VIII}$ wherein R$^{VIII}$ represents C1-6 alkyl optionally substituted by amino, or
(IX) -NSO$_2$R$^{IX}$ wherein R$^{IX}$ represents C1-4 alkyl;

(11) -NR$^a$R$^b$;
(12) -CO-OR$^c$;
(13) -CO-NR$^d$R$^e$;
wherein, in groups (11) to (13), R$^a$, R$^b$, R$^c$, R$^d$, and R$^e$, which may be the same or different, represent a hydrogen atom or C1-4 alkyl, and the C1-4 alkyl group is optionally substituted by

(a) hydroxyl,
(b) a halogen atom,
(c) C1-4 alkoxy,
(d) a saturated or unsaturated three- to nine-membered carbocyclic or heterocyclic group optionally substituted by C1-4 alkyl, C1-4 alkoxy, or a halogen atom, or
(e) amino group wherein one or two hydrogen atoms in the amino group are optionally substituted by C1-4 alkyl, and

R$^d$ and R$^e$ together may combine with the carbon atoms to which they are attached represent a saturated three- to nine-membered heterocyclic group, and the heterocyclic group is optionally substituted by C1-4 alkyl, C1-4 alkoxy, or a halogen atom or may contain one or more additional heteroatoms;
(14) a saturated or unsaturated three- to nine-membered carbocyclic group;
(15) a saturated or unsaturated three- to nine-membered heterocyclic group;
(16) a bicyclic saturated or unsaturated eight- to twelve-membered carbocyclic or heterocyclic group;
(17) -OCOR$^k$ wherein R$^k$ represents C1-4 alkyl; or
(18) -OSO$_2$R$^L$ wherein R$^L$ represents C1-4 alkyl,

wherein (14) carbocyclic group, (15) heterocyclic group, and (16) bicyclic carbocyclic or heterocyclic group are optionally substituted by (i) hydroxyl, (ii) a halogen atom, (iii) cyano group, (iv) nitro group, (v) amino group wherein one or two hydrogen atoms in the amino group are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^9$R$^h$ wherein R$^f$, R$^g$, and R$^h$, which may be the same or different, represent a hydrogen atom or C1-4 alkyl, and
R$^{10}$ and R$^{11}$, R$^{11}$ and R$^{12}$, R$^{12}$ and R$^{13}$, and R$^{13}$ and R$^{14}$ together may combine with the carbon atoms to which they are attached represent a saturated or unsaturated five- or six-membered carbocyclic or heterocyclic group, and the carbocyclic or heterocyclic group is optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^9$R$^h$ wherein R$^f$, R$^g$, and R$^h$, which may be the same or different, represent a hydrogen

atom or C1-4 alkyl,
provided that, when $D^1$, $D^2$, $D^3$, $D^4$, X, E, G, J, L, and M represent a nitrogen atom, groups $R^2$ to $R^6$ and $R^{10}$ to $R^{14}$ which attach to the nitrogen atom are absent, and
provided that, when all of $D^1$, $D^2$, $D^3$, and $D^4$ represent a carbon atom,

I) at least one of $R^4$ and $R^5$ represents (4) cyano group, (5) nitro group, (12) -CO-$OR^c$, (13) -CO-$NR^dR^e$ wherein any one of $R^d$ and $R^e$ represents optionally substituted C1-4 alkyl, (14) carbocyclic group, (15) heterocyclic group wherein the heterocyclic group contains at least one substituent, or (16) bicyclic carbocyclic group or heterocyclic group, or
II) L represents a nitrogen atom, E, G, J, and M represent a carbon atom, $R^{10}$ represents a hydrogen atom, and $R^{14}$ represents (6) C1-6 alkyl group, (14) carbocyclic group, (15) heterocyclic group, or (16) bicyclic carbocyclic group or heterocyclic group.

**2.** The compound according to claim 1, wherein at least one of $D^1$ to $D^4$ represents a nitrogen atom.

**3.** The compound according to claim 1, wherein $D^1$ represents a nitrogen atom and, at the same time, all of $D^2$ to $D^4$ represent a carbon atom.

**4.** The compound according to claim 1, wherein $D^2$ represents a nitrogen atom and, at the same time, all of $D^1$, $D^3$, and $D^4$ represent a carbon atom.

**5.** The compound according to any one of claims 2 to 4,
wherein, in the group of formula (a), any one of E, G, J, L, and M represents a nitrogen atom and all the others represent a carbon atom.

**6.** The compound according to any one of claims 2 to 4,
wherein L represents a nitrogen atom and E, G, J, and M represent a carbon atom.

**7.** The compound according to any one of claims 2 to 6,
wherein
$R^{10}$ represents a hydrogen atom, and
$R^{11}$ and $R^{12}$ are selected from the group consisting of a hydrogen atom, a halogen atom, C1-4 alkyl, and C1-4 alkoxy.

**8.** The compound according to any one of claims 2 to 7,
wherein $R^{14}$ represents an optionally substituted saturated or unsaturated five- or six-membered carbocyclic or heterocyclic group.

**9.** The compound according to claim 8, wherein $R^{14}$ represents an optionally substituted unsaturated six-membered heterocyclic group.

**10.** The compound according to any one of claims 2 to 9,
wherein A represents a group of formula (a-1) or (a-2):

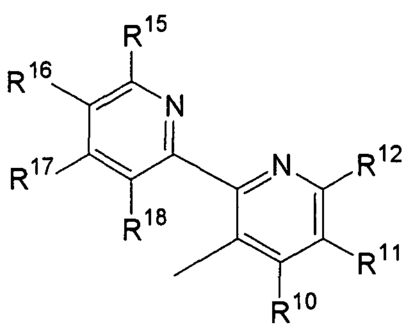
(a-1)

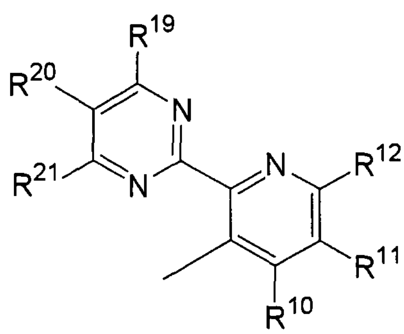
(a-2)

wherein
$R^{10}$ to $R^{12}$ are as defined in claim 1, and

$R^{15}$ to $R^{18}$ and $R^{19}$ to $R^{21}$, which may be the same or different, represent (0) a hydrogen atom, (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$
wherein R$^f$, R$^g$, and R$^h$, which may be the same or different, represent a hydrogen atom or C1-4 alkyl.

**11.** The compound according to claim 10, wherein, in the group of formula (a-1) or (a-2), $R^{15}$ to $R^{18}$ and $R^{19}$ to $R^{21}$ are selected from the group consisting of a hydrogen atom, a halogen atom, C1-4 alkyl, and C1-4 alkoxy.

**12.** The compound according to claim 10 or 11, wherein
$R^{10}$ represents a hydrogen atom,
$R^{11}$ and $R^{12}$ are selected from the group consisting of a hydrogen atom and C1-4 alkyl, and
$R^{15}$ to $R^{18}$ and $R^{19}$ to $R^{21}$ in the group of formula (a-1) or (a-2) are selected from the group consisting of a hydrogen atom, a halogen atom, C1-4 alkyl, and C1-4 alkoxy.

**13.** The compound according to claim 10 or 11, wherein
$R^{10}$ represents a hydrogen atom,
$R^{11}$ and $R^{12}$ are selected from the group consisting of
a hydrogen atom and C1-4 alkyl, and
all of $R^{15}$ to $R^{18}$ and $R^{19}$ to $R^{21}$ in the group of formula (a-1) or (a-2) represent a hydrogen atom.

**14.** The compound according to claim 12 or 13, wherein Z represents -O-, X represents a carbon atom, and $R^1$ to $R^3$ and $R^6$ represent a hydrogen atom.

**15.** The compound according to any one of claims 2 to 6,
wherein
$R^{10}$ represents a hydrogen atom, and
$R^{11}$ and $R^{12}$ together combine with the carbon atoms to which they are attached represent a saturated or unsaturated five- or six-membered carbocyclic or heterocyclic group.

**16.** The compound according to claim 15, wherein $R^{11}$ and $R^{12}$ together combine with the carbon atoms to which they are attached represent a unsaturated six-membered carbocyclic or heterocyclic group.

**17.** The compound according to any one of claims 2 to 4,
wherein A represents a group of formula (a-3):

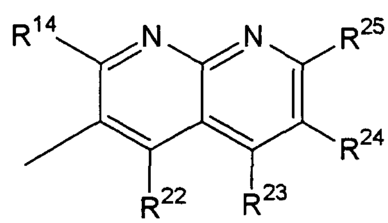

(a-3)

wherein
$R^{14}$ is as defined in claim 1, and
$R^{22}$ to $R^{25}$, which may be the same or different, represent (0) a hydrogen atom, (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein R$^f$, R$^g$, and R$^h$, which may be the same or different, represent a hydrogen atom or C1-4 alkyl).

**18.** The compound according to claim 17, wherein $R^{22}$ to $R^{25}$ in the group of formula (a-3) are selected from the group consisting of a hydrogen atom, a halogen atom, C1-4 alkyl, and C1-4 alkoxy.

**19.** The compound according to claim 17, wherein all of $R^{22}$ to $R^{25}$ in the group of formula (a-3) represent a hydrogen atom.

**20.** The compound according to any one of claims 17 to 19, wherein $R^{14}$ represents
optionally substituted C1-4 alkyl,
an optionally substituted saturated or unsaturated five- or six-membered carbocyclic or heterocyclic group, or
an optionally substituted bicyclic saturated or unsaturated nine- or ten-membered carbocyclic or heterocyclic group.

**21.** The compound according to claim 20, wherein $R^{14}$ represents unsubstituted C1-4 alkyl.

**22.** The compound according to claim 20, wherein $R^{14}$ represents an optionally substituted unsaturated six-membered carbocyclic or heterocyclic group.

**23.** The compound according to any one of claims 2 to 22, wherein X represents a carbon atom and both $R^1$ and $R^2$ represent a hydrogen atom.

**24.** The compound according to claim 23, wherein
$D^1$ represents a nitrogen atom and all of $D^2$ to $D^4$ represent a carbon atom, and
$R^6$ represents a hydrogen atom.

**25.** The compound according to claim 23, wherein
$D^2$ represents a nitrogen atom and all of $D^1$, $D^3$, and $D^4$ represent a carbon atom, and
$R^3$ represents a hydrogen atom or a halogen atom, and $R^6$ represents a hydrogen atom.

**26.** The compound according to claim 23, wherein all of $R^1$, $R^2$, $R^3$ and $R^6$ represent a hydrogen atom.

**27.** The compound according to any one of claims 2 to 26,
wherein $R^4$ and $R^5$, which may be the same or different, represent

(1) a hydrogen atom;
(2) a halogen atom;
(3) hydroxyl;
(6) C1-6 alkyl;
(9) C1-6 alkoxy;
(12) $-CO-OR^c$;
(13) $-CO-NR^dR^e$;
(14) a saturated or unsaturated three- to nine-membered carbocyclic group;
(15) a saturated or unsaturated three- to nine-membered heterocyclic group;
(16) a bicyclic saturated or unsaturated eight- to twelve-membered carbocyclic or heterocyclic group;
(17) $-OCOR^k$ wherein $R^k$ represents C1-4 alkyl; or
(18) $-OSO_2R^L$ wherein $R^L$ represents C1-4 alkyl,

wherein these groups are optionally substituted as defined in claim 1.

**28.** The compound according to any one of claims 1 to 27,
wherein Z represents -O-.

**29.** The compound according to any one of claims 1 to 28,
wherein X represents a carbon atom.

**30.** The compound according to claim 1 or a pharmaceutically acceptable salt or solvate thereof,
wherein formula (I) is represented by formula (100):

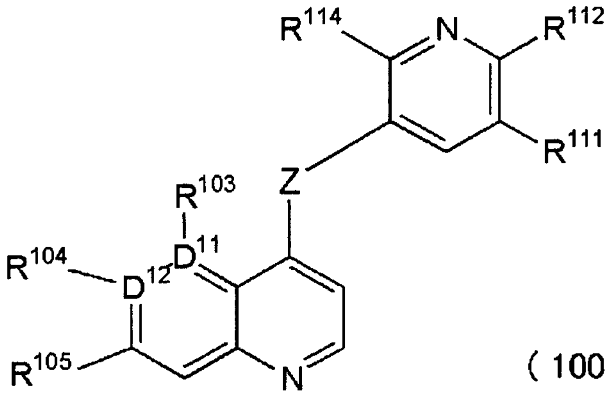

(100)

wherein
Z represents -O-, -NH-, -S-, or -C(=O)-,
any one of $D^{11}$ and $D^{12}$ represents a nitrogen atom, and the other represents a carbon atom,
$R^{103}$ represents a hydrogen atom or a halogen atom,
$R^{104}$ and $R^{105}$, which may be the same or different, represent

(1) a hydrogen atom ;
(2) a halogen atom;
(3) hydroxyl;
(6) C1-6 alkyl;
(9) C1-6 alkoxy;
(12) -CO-$OR^c$;
(13) -CO-$NR^dR^e$;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,
wherein these groups are optionally substituted as defined in claim 1,
$R^{111}$ and $R^{112}$, which may be the same or different, are selected from the group consisting of a hydrogen atom, C1-4 alkyl, and C1-4 alkoxy, and
$R^{114}$ represents
(14") a saturated or unsaturated five- or six-membered carbocyclic group;
(15") a saturated or unsaturated five- or six-membered heterocyclic group; or
(16") a bicyclic saturated or unsaturated nine- or ten-membered carbocyclic or heterocyclic group;
wherein (14") carbocyclic group, (15") heterocyclic group, and (16") bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-$OR^f$, or (xii) -CO-$NR^9R^h$ wherein $R^f$, $R^9$, and $R^h$, which may be the same or different, represent a hydrogen atom or C1-4 alkyl.

**31.** The compound according to claim 30, wherein Z represents -O-.

**32.** The compound according to claim 30 or 31, wherein $R^{103}$ represents a hydrogen atom.

**33.** The compound according to any one of claims 30 to 32, wherein
both $R^{111}$ and $R^{112}$ represent methyl, or
$R^{111}$ represents a hydrogen atom, and $R^{112}$ represents ethyl.

**34.** The compound according to any one of claims 30 to 33, wherein $R^{114}$ represents a group of formula (a-4) or (a-5):

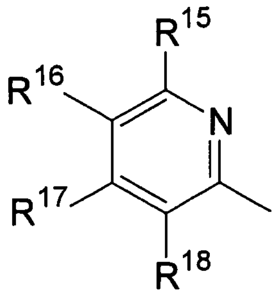
(a-4)
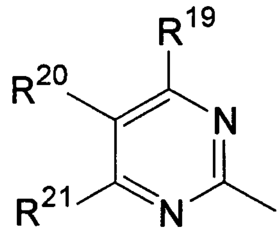
(a-5)

wherein
$R^{15}$ to $R^{18}$ and $R^{19}$ to $R^{21}$, which may be the same or different, are selected from the group consisting of a hydrogen atom, a halogen atom, C1-4 alkyl, and C1-4 alkoxy.

**35.** The compound according to claim 34, wherein all of $R^{15}$ to $R^{18}$ and $R^{19}$ to $R^{21}$ represent a hydrogen atom.

**36.** The compound according to any one of claims 30 to 35, wherein both $R^{104}$ and $R^{105}$ represent a hydrogen atom.

**37.** The compound according to claim 1 or a pharmaceutically acceptable salt or solvate thereof, wherein formula (I) is represented by formula (200):

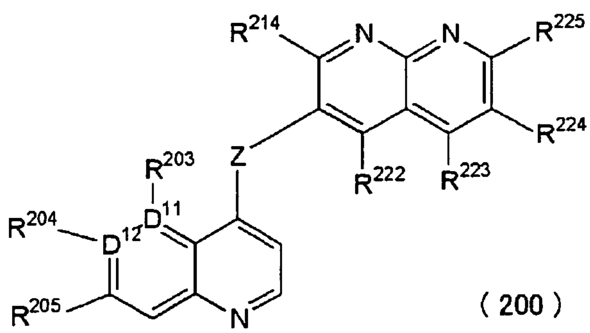
( 200 )

wherein
Z represents -O-, -NH-, -S-, or -C(=O)-,
any one of $D^{11}$ and $D^{12}$ represents a nitrogen atom, and the other represents a carbon atom,
$R^{203}$ represents a hydrogen atom or a halogen atom,
$R^{204}$ and $R^{205}$, which may be the same or different, represent

(1) a hydrogen atom ;
(2) a halogen atom;
(3) hydroxyl;
(6) C1-6 alkyl;
(9) C1-6 alkoxy;
(12) -CO-$OR^c$;
(13) -CO-$NR^dR^e$;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group, wherein these groups are optionally substituted as defined in claim 1),

$R^{222}$ to $R^{225}$, which may be the same or different, are selected from the group consisting of a hydrogen atom, a halogen atom, C1-4 alkyl, and C1-4 alkoxy, and
$R^{214}$ represents
unsubstituted C1-4 alkyl,
an optionally substituted unsaturated six-membered carbocyclic or heterocyclic group, or

an optionally substituted, bicyclic saturated or unsaturated nine- or ten-membered carbocyclic or heterocyclic group.

**38.** The compound according to claim 37, wherein Z represents -O-.

**39.** The compound according to claim 37 or 38, wherein $R^{203}$ represents a hydrogen atom.

**40.** The compound according to any one of claims 37 to 39, wherein all of $R^{222}$ to $R^{225}$ represent a hydrogen atom.

**41.** The compound according to any one of claims 37 to 40, wherein $R^{214}$ represents phenyl.

**42.** The compound according to any one of claims 37 to 40, wherein $R^{214}$ represents methyl or ethyl.

**43.** The compound according to any one of claims 37 to 42, wherein both $R^{204}$ and $R^{205}$ represent a hydrogen atom.

**44.** The compound according to claim 1, wherein
all of $D^1$ to $D^4$ represent a carbon atom,
$R^1$ and $R^2$ represent a hydrogen atom, and
$R^3$ and $R^6$, which may be the same or different, represent a hydrogen atom, a halogen atom, or C1-4 alkyl, and
$R^4$ and $R^5$, which may be the same or different, represent

(4) cyano group;
(5) nitro group;
(12) -CO-$OR^c$;
(13) -CO-$NR^dR^e$;
wherein, in groups (12) and (13), $R^c$, $R^d$, and $R^e$, which may be the same or different, represent a hydrogen atom or C1-4 alkyl, provided that at least one of $R^d$ and $R^e$ represents C1-4 alkyl, and the C1-4 alkyl group is optionally substituted by

(a) hydroxyl,
(b) a halogen atom,
(c) C1-4 alkoxy,
(d) a saturated or unsaturated three- to nine-membered carbocyclic or heterocyclic group optionally substituted by C1-4 alkyl, C1-4 alkoxy, or a halogen atom, or
(e) amino group wherein one or two hydrogen atoms in the amino groups are optionally substituted by C1-4 alkyl,

(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,

wherein (14) carbocyclic group, (15) heterocyclic group, and (16) bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-$OR^f$, or (xii) -CO-$NR^gR^h$ wherein $R^f$, $R^g$, and $R^h$, which may be the same or different, represent a hydrogen atom or C1-4 alkyl, and (15) heterocyclic group contain at least one substituent.

**45.** The compound according to claim 44, wherein
$R^4$ and $R^5$ represent
an unsaturated five- or six-membered carbocyclic or heterocyclic group wherein the carbocyclic or heterocyclic group is optionally substituted by C1-4 alkyl, C1-4 alkoxy, a halogen atom, amino group, or hydroxyl, or
a bicyclic unsaturated nine- or ten-membered carbocyclic or heterocyclic group wherein the carbocyclic or heterocyclic group is optionally substituted by C1-4 alkyl, C1-4 alkoxy, a halogen atom, amino group, or hydroxyl.

**46.** The compound according to claim 44 or 45, wherein L represents a nitrogen atom, and E, G, J, and M represent a carbon atom.

**47.** The compound according to any one of claims 44 to 46, wherein $R^{14}$ represents

(6) C1-6 alkyl; wherein the alkyl group is optionally substituted as defined in claim 1,
(14") a saturated or unsaturated five- or six-membered carbocyclic group;
(15") a saturated or unsaturated five- or six-membered heterocyclic group; or
(16") a bicyclic saturated or unsaturated nine- or ten-membered carbocyclic or heterocyclic group;
wherein (14") carbocyclic group, (15") heterocyclic group, and (16") bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein R$^f$, R$^g$, and R$^h$, which may be the same or different, represent a hydrogen atom or C1-4 alkyl.

**48.** The compound according to claim 1 or a pharmaceutically acceptable salt or solvate thereof, wherein formula (I) is represented by formula (300):

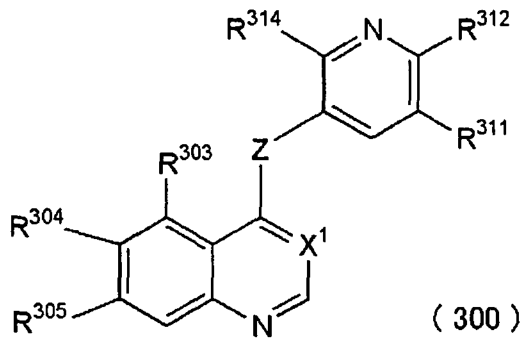

(300)

wherein
X$^1$ represents CH or N,
Z represents -O-, -NH-, -S-, or -C(=O)-,
$R^{303}$ represents a hydrogen atom, a halogen atom, or C1-4 alkyl,
$R^{304}$ and $R^{305}$, which may be the same or different, represent

(1) a hydrogen atom;
(2) a halogen atom;
(3) hydroxyl;
(6) C1-6 alkyl;
(9) C1-6 alkoxy;
(12) -CO-OR$^c$;
(13) -CO-NR$^d$R$^e$;
(14) a saturated or unsaturated three- to nine-membered carbocyclic group;
(15) a saturated or unsaturated three- to nine-membered heterocyclic group;
(16) a bicyclic saturated or unsaturated eight- to twelve-membered carbocyclic or heterocyclic group;
(17) -OCOR$^k$ wherein R$^k$ represents C1-4 alkyl; or
(18) -OSO$_2$R$^L$ wherein R$^L$ represents C1-4 alkyl,

wherein these groups are optionally substituted as defined in claim 1,
at least one of $R^{311}$ and $R^{312}$ represents C1-4 alkyl and the other represents a hydrogen atom or C1-4 alkyl, and $R^{314}$ represents an unsaturated six-membered heterocyclic group wherein the heterocyclic group is optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein R$^f$, R$^g$, and R$^h$, which may be the same or different, represent a hydrogen atom or C1-4 alkyl.

**49.** The compound according to claim 48, wherein
Z represents -O-,

$R^{303}$ represents a hydrogen atom, and
$R^{304}$ represents

(1) a hydrogen atom ;
(2) a halogen atom;
(3) hydroxyl;
(9) C1-6 alkoxy;
(17) -OCOR$^k$ wherein R$^k$ represents C1-4 alkyl; or
(18) -OSO$_2$R$^L$ wherein R$^L$ represents C1-4 alkyl,

wherein these groups are optionally substituted as defined in claim 1,
$R^{305}$ represents a hydrogen atom, a halogen atom, or -CO-NH$_2$, and
$R^{314}$ represents a group of formula (a-4) or (a-5):

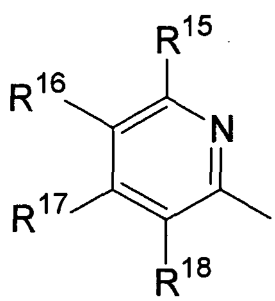
(a-4)

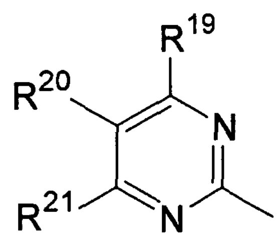
(a-5)

wherein
$R^{15}$ to $R^{18}$ and $R^{19}$ to $R^{21}$, which may be the same or different, are selected from the group consisting of a hydrogen atom, a halogen atom, C1-4 alkyl, and C1-4 alkoxy.

**50.** The compound according to claim 49, wherein $R^{15}$ to $R^{18}$ and $R^{19}$ to $R^{21}$ represent a hydrogen atom.

**51.** The compound according to any one of claims 48 to 50, wherein $X^1$ represents CH.

**52.** The compound according to any one of claims 48 to 51, wherein $R^{305}$ represents a hydrogen atom, a fluorine atom, or -CO-NH$_2$.

**53.** The compound according to any one of claims 48 to 52, wherein $R^{311}$ and $R^{312}$, which may be the same or different, represent C1-4 alkyl.

**54.** The compound according to claim 53, wherein both $R^{311}$ and $R^{312}$ represent methyl.

**55.** The compound according to any one of claims 48 to 52, wherein
$R^{311}$ represents a hydrogen atom, and
$R^{312}$ represents C1-4 alkyl.

**56.** The compound according to claim 55, wherein
$R^{311}$ represents a hydrogen atom, and
$R^{312}$ represents methyl.

**57.** The compound according to claim 55, wherein
$R^{311}$ represents a hydrogen atom, and
$R^{312}$ represents ethyl.

**58.** The compound according to any one of claims 48 to 57, wherein $R^{304}$ represents C1-6 alkoxy optionally substituted as defined in claim 1.

**59.** The compound according to claim 58, wherein $R^{304}$ represents C1-6 alkoxy substituted by hydroxyl.

**60.** The compound according to claim 58, wherein $R^{304}$ represents $-O(CH_2)m1-OH$ wherein m1 is an integer of 2 to 4.

**61.** The compound according to claim 58, wherein $R^{304}$ represents $-OC_2H_5-OH$.

**62.** The compound according to claim 48, which is selected from the group consisting of compounds 181, 188, 192, 200, 202, and 205.

**63.** The compound according to claim 1 or a pharmaceutically acceptable salt or solvate thereof, wherein formula (I) is represented by formula (400):

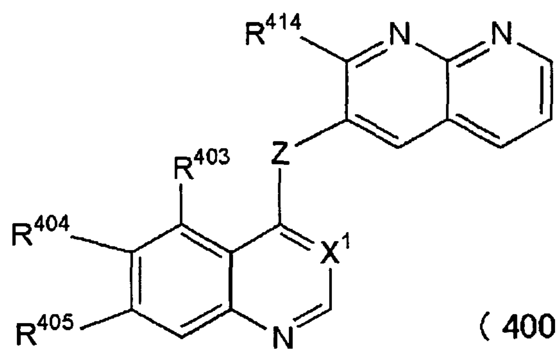

(400)

wherein
$X^1$ represents CH or N,
Z represents -O-, -NH-, -S-, or -C(=O)-,
$R^{403}$ represents a hydrogen atom, a halogen atom, or C1-4 alkyl,
$R^{404}$ and $R^{405}$, which may be the same or different, represent

(1) a hydrogen atom ;
(2) a halogen atom;
(3) hydroxyl;
(6) C1-6 alkyl;
(9) C1-6 alkoxy;
(12) $-CO-OR^c$;
(13) $-CO-NR^dR^e$;
(14) a saturated or unsaturated three- to nine-membered carbocyclic group;
(15) a saturated or unsaturated three- to nine-membered heterocyclic group;
(16) a bicyclic saturated or unsaturated eight- to twelve-membered carbocyclic or heterocyclic group;
(17) $-OCOR^k$ wherein $R^k$ represents C1-4 alkyl; or
(18) $-OSO_2R^L$ wherein $R^L$ represents C1-4 alkyl,
wherein these groups are optionally substituted as defined in claim 1, and
$R^{414}$ represents
(6) C1-6 alkyl;
wherein the alkyl group is optionally substituted as defined in claim 1,
(14") a saturated or unsaturated five- or six-membered carbocyclic group;
(15") a saturated or unsaturated five- or six-membered heterocyclic group; or
(16") a bicyclic saturated or unsaturated nine- or ten-membered carbocyclic or heterocyclic group;
wherein (14") carbocyclic group, (15") heterocyclic group, and (16") bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl.

**64.** The compound according to claim 63, wherein

Z represents -O-,
$R^{403}$ represents a hydrogen atom, and
$R^{414}$ represents
an unsaturated five- or six-membered carbocyclic or heterocyclic group wherein the carbocyclic or heterocyclic group is optionally substituted by C1-4 alkyl, C1-4 alkoxy, a halogen atom, amino group, or hydroxyl, or
a bicyclic unsaturated nine- or ten-membered carbocyclic or heterocyclic group wherein the carbocyclic or heterocyclic group is optionally substituted by C1-4 alkyl, C1-4 alkoxy, a halogen atom, amino group, or hydroxyl.

**65.** The compound according to claim 63, wherein
$X^1$ represents CH,
Z represents -O-,
$R^{403}$ represents a hydrogen atom,
any one of $R^{404}$ and $R^{405}$ represents C1-4 alkoxy substituted by hydroxyl, and the other represents unsubstituted C1-4 alkoxy, and
$R^{414}$ represents phenyl.

**66.** The compound according to claim 63, which is compound 178.

**67.** The compound according to claim 1, which is selected from the group consisting of compounds 1 to 27, 30, 31, 37 to 70, 73, 74, 81 to 179, and 181 to 225.

**68.** A compound of formula (II) or a pharmaceutically acceptable salt or solvate thereof:

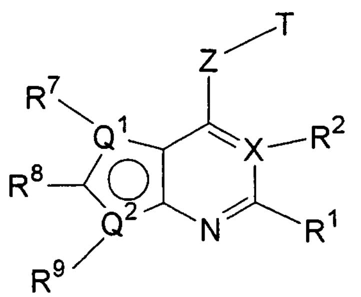

(II)

wherein
T represents a saturated or unsaturated five- or six-membered carbocyclic or heterocyclic group,
wherein group T is optionally substituted by groups (2) to (16):

(2) a halogen atom;
(3) hydroxyl;
(4) cyano group;
(5) nitro group;
(6) C1-6 alkyl;
(7) C2-6 alkenyl;
(8) C2-6 alkynyl;
(9) C1-6 alkoxy;
(10) C1-6 alkylthio;
wherein (6) C1-6 alkyl, (7) C2-6 alkenyl, (8) C2-6 alkynyl, (9) C1-6 alkoxy, and (10) C1-6 alkylthio are optionally substituted by

(I) hydroxyl,
(II) a halogen atom,
(III) C1-4 alkoxy,
(IV) an oxygen atom,
(V) a saturated or unsaturated three- to nine-membered carbocyclic or heterocyclic group optionally substituted by C1-4 alkyl, C1-4 alkoxy, or a halogen atom, or
(VI) amino group wherein one or two hydrogen atoms in the amino group are optionally substituted by C1-4 alkyl, and the C1-4 alkyl group is optionally substituted by hydroxyl or C1-4 alkoxy,

(11) $-NR^aR^b$;
(12) $-CO-OR^c$;
(13) $-CO-NR^dR^e$;
wherein, in groups (11) to (13), $R^a$, $R^b$, $R^c$, $R^d$, and $R^e$, which may be the same or different, represent a hydrogen atom or C1-4 alkyl, and the C1-4 alkyl group is optionally substituted by

(a) hydroxyl,
(b) a halogen atom,
(c) C1-4 alkoxy,
(d) a saturated or unsaturated three-to nine-membered carbocyclic or heterocyclic group optionally substituted by C1-4 alkyl, C1-4 alkoxy, or a halogen atom, or
(e) amino group wherein one or two hydrogen atoms in the amino groups are optionally substituted by C1-4 alkyl, and
$R^d$ and $R^e$ together may combine with the carbon atoms to which they are attached represent a saturated three- to nine-membered heterocyclic group

wherein the heterocyclic group is optionally substituted by C1-4 alkyl, C1-4 alkoxy, or a halogen atom and may further contain one or more additional heteroatoms;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,

wherein (14) carbocyclic group, (15) heterocyclic group, and (16) bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl,
two adjacent substituents on group T together may combine with the carbon atoms to which they are attached represent a saturated or unsaturated five- or six-membered carbocyclic or heterocyclic group, and the carbocyclic or heterocyclic group is optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl,
$Q^1$ and $Q^2$, which may be the same or different, represent C, S, O, or N,
X represents C or N,
Z represents -O-, $-N(-R^Z)-$, -S-, or -C(=O)- wherein $R^Z$ represents a hydrogen atom or unsubstituted C1-4 alkyl,
$R^1$, $R^2$, and $R^7$ to $R^9$, which may be the same or different, represent,

(1) a hydrogen atom;
(2) a halogen atom;
(3) hydroxyl;
(4) cyano group;
(5) nitro group;
(6) C1-6 alkyl;
(7) C2-6 alkenyl;
(8) C2-6 alkynyl;
(9) C1-6 alkoxy;
(10) C1-6 alkylthio;
wherein (6) C1-6 alkyl, (7) C2-6 alkenyl, (8) C2-6 alkynyl, (9) C1-6 alkoxy, and (10) C1-6 alkylthio are optionally substituted by

(I) hydroxyl,
(II) a halogen atom,
(III) C1-4 alkoxy,
(IV) an oxygen atom,
(V) a saturated or unsaturated three- to nine-membered carbocyclic or heterocyclic group optionally substituted by C1-4 alkyl, C1-4 alkoxy, or a halogen atom, or
(VI) amino group wherein one or two hydrogen atoms in the amino group are optionally substituted by C1-4

alkyl, and the C1-4 alkyl group is optionally substituted by hydroxyl or C1-4 alkoxy;

(11) $-NR^aR^b$;
(12) $-CO-OR^c$;
(13) $-CO-NR^dR^e$;
wherein, in groups (11) to (13), $R^a$, $R^b$, $R^c$, $R^d$, and $R^e$, which may be the same or different, represent a hydrogen atom or C1-4 alkyl, and C1-4 alkyl is optionally substituted by

(a) hydroxyl,
(b) a halogen atom,
(c) C1-4 alkoxy,
(d) a saturated or unsaturated three- to nine-membered carbocyclic or heterocyclic group optionally substituted by C1-4 alkyl, C1-4 alkoxy, or a halogen atom, or
(e) amino group wherein one or two hydrogen atoms in the amino groups are optionally substituted by C1-4 alkyl, and $R^d$ and $R^e$ together may combine with the carbon atoms to which they are attached represent a saturated three- to nine-membered heterocyclic group, and the heterocyclic group is optionally substituted by C1-4 alkyl, C1-4 alkoxy, or a halogen atom and may contain one or more additional heteroatoms;

(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group,

wherein (14) carbocyclic group, (15) heterocyclic group, and (16) bicyclic carbocyclic or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl, and
the five-membered ring part containing $Q^1$ and $Q^2$ in formula (II) represents an aromatic ring,
provided that, when X represents a nitrogen atom, $R^2$ is absent, and
when $Q^1$ and $Q^2$ represent an oxygen atom or a sulfur atom, $R^7$ and $R^9$ which attach to the oxygen atom or the sulfur atom are absent, and, when both $Q^1$ and $Q^2$ represent a nitrogen atom, any one of $R^7$ and $R^9$ is absent.

**69.** The compound according to claim 68, wherein T represents a group of formula (a):

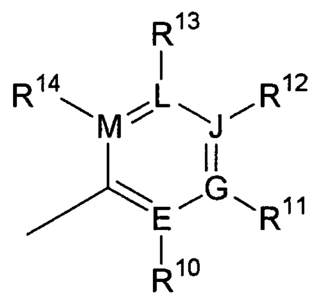

(a)

wherein
E, G, J, L, and M, which may be the same or different, represent C or N, and
$R^{10}$ to $R^{14}$, which may be the same or different, represent

(1) a hydrogen atom;
(2) a halogen atom;
(3) hydroxyl;
(4) cyano group;
(5) nitro group;
(6) C1-6 alkyl;
(7) C2-6 alkenyl;
(8) C2-6 alkynyl;

(9) C1-6 alkoxy;
(10) C1-6 alkylthio;
wherein (6) C1-6 alkyl, (7) C2-6 alkenyl, (8) C2-6 alkynyl, (9) C1-6 alkoxy, and (10) C1-6 alkylthio are optionally substituted by

(I) hydroxyl,
(II) a halogen atom,
(III) C1-4 alkoxy,
(IV) an oxygen atom,
(V) a saturated or unsaturated three- to nine-membered carbocyclic or heterocyclic group optionally substituted by C1-4 alkyl, C1-4 alkoxy, or a halogen atom, or
(VI) amino group wherein one or two hydrogen atoms in the amino group are optionally substituted by C1-4 alkyl, and the C1-4 alkyl group is optionally substituted by hydroxyl or C1-4 alkoxy;

(11) $-NR^aR^b$;
(12) $-CO-OR^c$;
(13) $-CO-NR^dR^e$;
wherein, in groups (11) to (13), $R^a$, $R^b$, $R^c$, $R^d$, and $R^e$, which may be the same or different, represent a hydrogen atom or C1-4 alkyl, and the C1-4 alkyl group is optionally substituted by,

(a) hydroxyl,
(b) a halogen atom,
(c) C1-4 alkoxy,
(d) a saturated or unsaturated three- to nine-membered carbocyclic or heterocyclic group optionally substituted by C1-4 alkyl, C1-4 alkoxy, or a halogen atom, or
(e) amino group wherein one or two hydrogen atoms in the amino groups are optionally substituted by C1-4 alkyl, and

$R^d$ and $R^e$ together may combine with the carbon atoms to which they are attached represent a saturated three- to nine-membered heterocyclic group, and the heterocyclic group is optionally substituted by C1-4 alkyl, C1-4 alkoxy, or a halogen atom and may contain one or more additional heteroatoms;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group;

wherein (14) carbocyclic group, (15) heterocyclic group, and (16) bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CQ-NR^gR^h$ wherein $R^f$, $R^g$, and $R^h$, which may be the same or different, represent a hydrogen atom or C1-4 alkyl, and
$R^{10}$ and $R^{11}$, $R^{11}$ and $R^{12}$, $R^{12}$ and $R^{13}$, and $R^{13}$ and $R^{14}$ together may combine with the carbon atoms to which they are attached represent a saturated or unsaturated five- or six-membered carbocyclic or heterocyclic group, and the carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) $-CO-OR^f$, or (xii) $-CO-NR^gR^h$ wherein $R^f$, $R^g$, and $R^h$, which may be the same or different, represent a hydrogen atom or C1-4 alkyl,
provided that, when E, G, J, L, and M represent a nitrogen atom, $R^{10}$ to $R^{14}$ which attach to the nitrogen atom are absent.

**70.** The compound according to claim 68 or 69, wherein $Q^1$ represents a sulfur atom, and $Q^2$ represents a carbon atom.

**71.** The compound according to claim 68 or 69, wherein $Q^1$ represents a carbon atom, and $Q^2$ represents a sulfur atom.

**72.** The compound according to any one of claims 69 to 71, wherein, in the group of formula (a), any one of E, G, J, L, and M represents a nitrogen atom, and all the others represent a carbon atom.

**73.** The compound according to claim 72, wherein L represents a nitrogen atom, and E, G, J, and M represent a carbon

atom.

**74.** The compound according to any one of claims 69 to 73, wherein
$R^{10}$ represents a hydrogen atom, and
$R^{11}$ and $R^{12}$ are selected from the group consisting of a hydrogen atom, a halogen atom, C1-4 alkyl, and C1-4 alkoxy.

**75.** The compound according to any one of claims 69 to 74, wherein $R^{14}$ represents an optionally substituted unsaturated six-membered heterocyclic group.

**76.** The compound according to any one of claims 69 to 75, wherein T represents a group of formula (a-1) or (a-2):

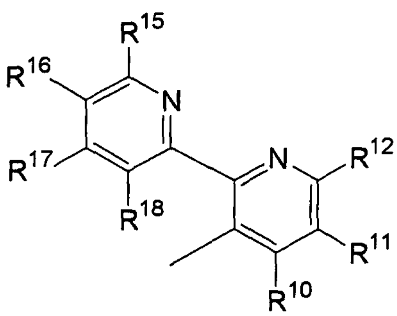
( a-1 )

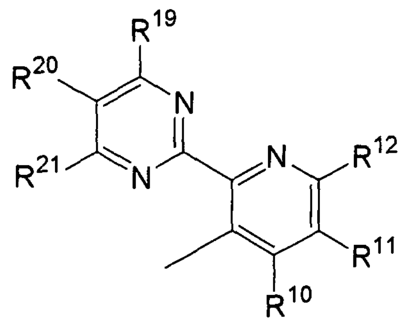
( a-2 )

wherein
$R^{10}$ to $R^{12}$ are as defined in claim 69,
$R^{15}$ to $R^{18}$ and $R^{19}$ to $R^{21}$, which may be the same or different, represent (0) a hydrogen atom, (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$
wherein R$^f$, R$^g$, and R$^h$, which may be the same or different, represent a hydrogen atom or C1-4 alkyl.

**77.** The compound according to claim 76, wherein, in a group of formula (a-1) or (a-2), $R^{15}$ to $R^{18}$ and $R^{19}$ to $R^{21}$ are selected from the group consisting of a hydrogen atom, a halogen atom, C1-4 alkyl, and C1-4 alkoxy.

**78.** The compound according to claim 76, wherein
$R^{10}$ represents a hydrogen atom,
$R^{11}$ and $R^{12}$ are selected from the group consisting of a hydrogen atom and C1-4 alkyl, and, in the group of formula (a-1) or (a-2), $R^{15}$ to $R^{18}$ and $R^{19}$ to $R^{21}$ are selected from the group consisting of a hydrogen atom, a halogen atom, C1-4 alkyl, and C1-4 alkoxy.

**79.** The compound according to claim 76, wherein
$R^{10}$ represents a hydrogen atom,
$R^{11}$ and $R^{12}$ are selected from the group consisting of a hydrogen atom and C1-4 alkyl, and, in the group of formula (a-1) or (a-2), all of $R^{15}$ to $R^{18}$ and $R^{19}$ to $R^{21}$ represent a hydrogen atom.

**80.** The compound according to any one of claims 76 to 79, wherein
$R^{10}$ represents a hydrogen atom, and
$R^{11}$ and $R^{12}$ together combine with the carbon atoms to which they are attached represent a saturated or unsaturated five- or six-membered carbocyclic or heterocyclic group.

**81.** The compound according to claim 80, wherein $R^{11}$ and $R^{12}$ together combine with the carbon atoms to which they are attached represent a unsaturated six-membered carbocyclic or heterocyclic group.

**82.** The compound according to any one of claims 76 to 79, wherein T represents a group of formula (a-3):

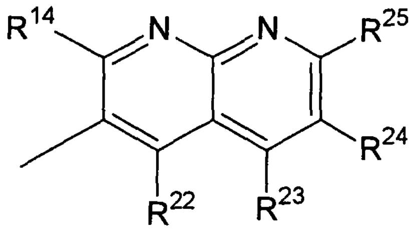

(a-3)

wherein
$R^{14}$ is as defined in claim 69,
$R^{22}$ to $R^{25}$, which may be the same or different, represent (0) a hydrogen atom, (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-$OR^f$, or (xii) -CO-$NR^gR^h$ wherein $R^f$, $R^g$, and $R^h$, which may be the same or different, represent a hydrogen atom or C1-4 alkyl.

**83.** The compound according to claim 82, wherein, in the group of formula (a-3), $R^{22}$ to $R^{25}$ are selected from the group consisting of a hydrogen atom, a halogen atom, C1-4 alkyl, and C1-4 alkoxy.

**84.** The compound according to claim 82, wherein, in the group of formula (a-3), all of $R^{22}$ to $R^{25}$ represent a hydrogen atom.

**85.** The compound according to any one of claims 82 to 84, wherein $R^{14}$ represents
optionally substituted C1-4 alkyl,
an optionally substituted saturated or unsaturated five- or six-membered carbocyclic or heterocyclic group, or
an optionally substituted bicyclic saturated or unsaturated nine- or ten-membered carbocyclic or heterocyclic group.

**86.** The compound according to claim 85, wherein $R^{14}$ represents unsubstituted C1-4 alkyl.

**87.** The compound according to claim 85, wherein $R^{14}$ represents an optionally substituted unsaturated six-membered carbocyclic or heterocyclic group.

**88.** The compound according to any one of claims 68 to 87, wherein X represents a carbon atom and both $R^1$ and $R^2$ represent a hydrogen atom.

**89.** The compound according to any one of claims 68 to 88, wherein $Q^1$ represents a sulfur atom, $Q^2$ represents a carbon atom, and $R^9$ represents a hydrogen atom.

**90.** The compound according to any one of claims 68 to 88, wherein $Q^1$ represents a carbon atom, $Q^2$ represents a sulfur atom, and $R^7$ represents a hydrogen atom.

**91.** The compound according to any one of claims 68 to 90, wherein both $R^1$ and $R^2$ represent a hydrogen atom.

**92.** The compound according to any one of claims 68 to 91, wherein
$R^7$ to $R^9$, which may be the same or different, represent

(1) a hydrogen atom ;
(2) a halogen atom;
(3) hydroxyl;
(6) C1-6 alkyl;
(9) C1-6 alkoxy;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group, wherein these groups are optionally substituted as defined in claim 68.

**93.** The compound according to any one of claims 68 to 92, wherein Z represents -O-.

**94.** The compound according to any one of claims 68 to 93, wherein X represents a carbon atom.

**95.** The compound according to claim 68 or a pharmaceutically acceptable salt or solvate thereof, wherein formula (I) is represented by formula (500):

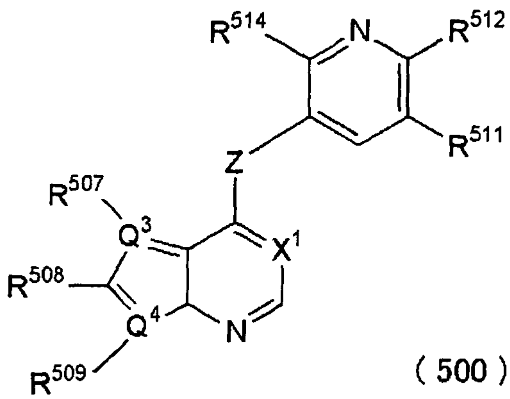

(500)

wherein
$X^1$ reprsents CH or N,
Z represents -O-, -NH-, -S-, or -C(=O)-,
any one of $Q^3$ and $Q^4$ represents a sulfur atom, and the other represents a carbon atom,
$R^{507}$ to $R^{509}$, which may be the same or different, represent

(1) a hydrogen atom ;
(2) a halogen atom;
(3) hydroxyl;
(6) C1-6 alkyl;
(9) C1-6 alkoxy;
(12) -CO-OR$^c$;
(13) -CO-NR$^d$R$^e$;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group wherein these groups are optionally substituted as defined in claim 68,
$R^{511}$ and $R^{512}$, which may be the same or different, are selected from the group consisting of a hydrogen atom and C1-4 alkyl,
$R^{514}$ represents
(14") a saturated or unsaturated five- or six-membered carbocyclic group;
(15") a saturated or unsaturated five- or six-membered heterocyclic group; or
(16") a bicyclic saturated or unsaturated nine- or ten-membered carbocyclic or heterocyclic group;
wherein (14") carbocyclic group, (15") heterocyclic group, and (16") bicyclic carbocyclic group or heterocyclic group are optionally substituted by (i) hydroxyl, (ii) a halogen atom, (iii) cyano group, (iv) nitro group, (v) amino group wherein one or two hydrogen atoms in the amino groups are optionally substituted by C1-4 alkyl, (vi) C1-4 alkyl, (vii) C2-4 alkenyl, (viii) C2-4 alkynyl, (ix) C1-4 alkoxy, (x) C1-4 alkylthio, (xi) -CO-OR$^f$, or (xii) -CO-NR$^g$R$^h$ wherein R$^f$, R$^g$, and R$^h$, which may be the same or different, represent a hydrogen atom or C1-4 alkyl.

**96.** The compound according to claim 95, wherein $Q^3$ represents a sulfur atom, and $Q^4$ represents a carbon atom.

**97.** The compound according to claim 95, wherein $Q^3$ represents a carbon atom, and $Q^4$ represents a sulfur atom.

**98.** The compound according to any one of claims 95 to 97, wherein Z represents -O-.

**99.** The compound according to any one of claims 95 to 98, wherein both $R^{511}$ and $R^{512}$ represent methyl, or $R^{511}$

represents a hydrogen atom while $R^{512}$ represents ethyl.

**100.** The compound according to any one of claims 95 to 99, wherein $R^{514}$ represents a group of formula (a-4) or formula (a-5):

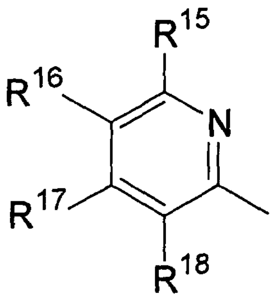

(a-4)

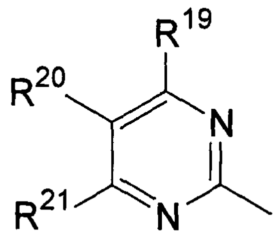

(a-5)

wherein
$R^{15}$ to $R^{18}$ and $R^{19}$ to $R^{21}$, which may be the same or different, are selected from the group consisting of a hydrogen atom, a halogen atom, C1-4 alkyl, and C1-4 alkoxy.

**101.** The compound according to claim 100, wherein all of $R^{15}$ to $R^{18}$ and $R^{19}$ to $R^{21}$ represent a hydrogen atom.

**102.** The compound according to any one of claims 95 and 98 to 101, wherein
$Q^3$ represents a sulfur atom,
$Q^4$ represents a carbon atom,
$R^{508}$ represents
a hydrogen atom,
C1-4 alkyl, or
a saturated or unsaturated six-membered carbocyclic or heterocyclic group,
wherein said carbocyclic or heterocyclic group is optionally substituted by (i) hydroxyl, (ii) a halogen atom, (v') amino group, (vi') C1-2 alkyl, or (ix') C1-2 alkoxy, and
$R^{509}$ represents a hydrogen atom.

**103.** The compound according to claim 102, wherein $R^{508}$ represents a hydrogen atom or phenyl.

**104.** The compound according to any one of claims 95 and 98 to 101, wherein
$Q^3$ represents a carbon atom,
$Q^4$ represents a sulfur atom, and
$R^{507}$ and $R^{508}$ are selected from the group consisting of a hydrogen atom and C1-4 alkyl.

**105.** The compound according to claim 104, wherein
both $R^{507}$ and $R^{508}$ represent a hydrogen atom or
both $R^{507}$ and $R^{508}$ reprsent methyl or
$R^{507}$ represent methyl while $R^{508}$ represents a hydrogen atom.

**106.** The compound of formula (I) according to claim 69 or a pharmaceutically acceptable salt or solvate thereof, wherein formula (I) is represented by formula (600):

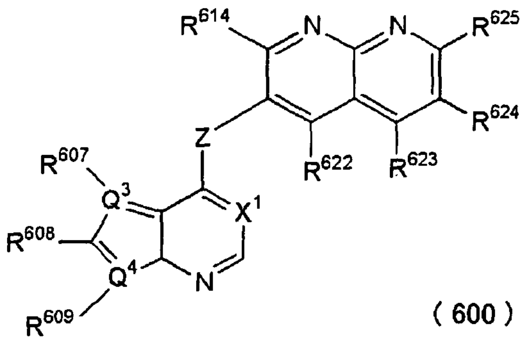

(600)

wherein
$X^1$ represents CH or N,
Z represents -O-, -NH-, -S-, or -C(=O)-,
any one of $Q^3$ and $Q^4$ represents a sulfur atom, and the other represents a carbon atom,
$R^{607}$ to $R^{609}$, which may be the same or different, represent

(1) a hydrogen atom ;
(2) a halogen atom;
(3) hydroxyl;
(6) C1-6 alkyl;
(9) C1-6 alkoxy;
(12) -CO-$OR^c$;
(13) -CO-$NR^dR^e$;
(14) a saturated or unsaturated three- to nine-membered carbocyclic group;
(15) a saturated or unsaturated three- to nine-membered heterocyclic group; or
(16) a bicyclic saturated or unsaturated eight- to twelve-membered carbocyclic or heterocyclic group, wherein these groups are optionally substituted as defined in claim 68,
$R^{622}$ to $R^{625}$, which may be the same or different, are selected from the group consisting of a hydrogen atom, a halogen atom, C1-4 alkyl, and C1-4 alkoxy, and
$R^{614}$ represents
unsubstituted C1-4 alkyl,
an optionally substituted unsaturated six-membered carbocyclic or heterocyclic group, or
an optionally substituted bicyclic saturated or unsaturated nine- or ten-membered carbocyclic or heterocyclic group.

**107.** The compound according to claim 106, wherein Z represents -O-.

**108.** The compound according to claim 106 or 107, wherein all of $R^{622}$ to $R^{625}$ represent a hydrogen atom.

**109.** The compound according to any one of claims 106 to 108, wherein $R^{614}$ represents phenyl.

**110.** The compound according to any one of claims 106 to 108, wherein $R^{614}$ represents methyl or ethyl.

**111.** The compound according to any one of claims 106 to 110, wherein
$Q^3$ represents a sulfur atom,
$Q^4$ represents a carbon atom,
$R^{608}$ represents
a hydrogen atom,
C1-4 alkyl, or
a saturated or unsaturated six-membered carbocyclic or heterocyclic group,
wherein said carbocyclic or heterocyclic group is optionally substituted by (i) hydroxyl, (ii) a halogen atom, (v') amino group, (vi') C1-2 alkyl, or (ix') C1-2 alkoxy, and
$R^{609}$ represents a hydrogen atom.

**112.** The compound according to claim 111, wherein $R^{608}$ represents a hydrogen atom or phenyl.

**113.** The compound according to any one of claims 106 to 110, wherein
$Q^3$ represents a carbon atom,
$Q^4$ represents a sulfur atom, and
$R^{607}$ and $R^{608}$ are selected from the group consisting of a hydrogen atom and C1-4 alkyl.

**114.** The compound according to claim 113, wherein
both $R^{607}$ and $R^{608}$ represent a hydrogen atom or
both $R^{607}$ and $R^{608}$ represent methyl or
$R^{607}$ represents methyl while $R^{608}$ represents a hydrogen atom.

**115.** The compound according to claim 68, which is selected from the group consisting of compounds 28, 29, 32 to 35, 71, 72, 75 to 78, and 180.

**116.** A pharmaceutical composition, comprising a compound according to any one of claims 1 to 115 or a pharmaceutically acceptable salt or solvate thereof as an active component.

**117.** The pharmaceutical composition according to claim 116, which can be used for the treatment or prevention of diseases for which TGFβ inhibition is effective therapeutically or prophylactically.

**118.** The pharmaceutical composition according to claim 117, wherein the disease for which TGFβ inhibition is effective therapeutically or prophylactically is a disease involving organ or tissue fibrosis.

**119.** The pharmaceutical composition according to claim 117, wherein the disease for which TGFβ inhibition is effective therapeutically or prophylactically is chronic renal disease, acute renal disease, hepatic fibrosis, cirrhosis, plumonary fibrosis, scleroderma, wound healing, arthritis, congestive cardiac disease, ulcer, ocular disorder, corneal problem, diabetic nephropathy, peritoneal sclerosis, arteriosclerosis, peritoneal adhesions, or subdermal adhesion.

**120.** The pharmaceutical composition according to claim 117, wherein the disease for which TGFβ inhibition is effective therapeutically or prophylactically is a malignant tumor.

**121.** The pharmaceutical composition according to claim 116, wherein can be used for ex vivo expansion cells.

**122.** A TGFβ inhibitor comprising a compound according to any one of claims 1 to 115 or a pharmaceutically acceptable salt or solvate thereof as an active component.

**123.** A method for treating or preventing a disease for which TGFβ inhibition is effective therapeutically or prophylactically, said method comprising the step of administering a therapeutically or prophylactically effective amount of a compound according to any one of claims 1 to 115 or a pharmaceutically acceptable salt or solvate thereof to a patient requiring the treatment or prevention of the disease for which TGFβ inhibition is effective therapeutically or prophylactically.

**124.** The method according to claim 123, wherein the disease for which TGFβ inhibition is effective therapeutically or prophylactically is a disease involving organ or tissue fibrosis.

**125.** The method according to claim 123, wherein the disease for which TGFβ inhibition is effective therapeutically or prophylactically is chronic renal disease, acute renal disease, hepatic fibrosis, cirrhosis, plumonary fibrosis, scleroderma, wound healing, arthritis, congestive cardiac disease, ulcer, ocular disorder, corneal problem, diabetic nephropathy, peritoneal sclerosis, arteriosclerosis, peritoneal adhesions, or subdermal adhesion.

**126.** The method according to claim 123, wherein the disease for which TGFβ inhibition is effective therapeutically or prophylactically is a malignant tumor.

**127.** A method for amplifying cells, comprising the step of adding a compound according to any one of claims 1 to 115 or a pharmaceutically acceptable salt or solvate thereof, in an amount effective for promoting cell growth, to intended cells in vitro to amplify the cells.

**128.** The method according to claim 127, wherein said intended cells are hematopoietic stem cells.

**129.** A method for inhibiting the action of TGFβ on cells, comprising the step of applying an effective amount of a compound according to any one of claims 1 to 115 to cells present in vitro or in vivo.

**130.** Use of a compound according to any one of claims 1 to 115, for the manufacture of a medicament used in the treatment or prevention of diseases for which TGFβ inhibition is effective therapeutically or prophylactically.

**131.** The use according to claim 130, wherein the disease for which TGFβ inhibition is effective therapeutically or prophylactically is a disease involving organ or tissue fibrosis.

**132.** The use according to claim 130, wherein the disease for which TGFβ inhibition is effective therapeutically or prophylactically is chronic renal disease, acute renal disease, hepatic fibrosis, cirrhosis, plumonary fibrosis, scleroderma, wound healing, arthritis, congestive cardiac disease, ulcer, ocular disorder, corneal problem, diabetic nephropathy, peritoneal sclerosis, arteriosclerosis, peritoneal adhesions, or subdermal adhesion.

**133.** The use according to claim 130, wherein the disease for which TGFβ inhibition is effective therapeutically or prophylactically is a malignant tumor.

**134.** The use of a compound according to any one of claims 1 to 115, for the manufacture of a TGFβ inhibitor.

**135.** The use of a compound according to any one of claims 1 to 115, for the manufacture of a promotor forex vivo expansion cells.

**INTERNATIONAL SEARCH REPORT**

International application No. PCT/JP2005/002610

A. CLASSIFICATION OF SUBJECT MATTER
Int.Cl$^7$ C07D401/14, 409/14, 405/14, 417/14, 471/04, 495/04, 519/00, 215/22, 239/88, 401/12, 405/12, 413/12, A61K31/4375, 31/444, A61P1/04, 1/16

According to International Patent Classification (IPC) or to both national classification and IPC

B. FIELDS SEARCHED

Minimum documentation searched (classification system followed by classification symbols)
Int.Cl$^7$ C07D401/14, 409/14, 405/14, 417/14, 471/04, 495/04, 519/00, 215/22, 239/88, 401/12, 405/12, 413/12, A61K31/4375, 31/444, A61P1/04, 1/16

Documentation searched other than minimum documentation to the extent that such documents are included in the fields searched

Electronic data base consulted during the international search (name of data base and, where practicable, search terms used)
CAPLUS(STN), REGISTRY(STN), MEDLINE(STN), CHEMCAST(STN)

C. DOCUMENTS CONSIDERED TO BE RELEVANT

| Category* | Citation of document, with indication, where appropriate, of the relevant passages | Relevant to claim No. |
|---|---|---|
| X | JP 2001-500890 A (Zenaca Ltd.), 23 January, 2001 (23.01.01), Claims; examples & WO 98/13350 A1 & AU 9743137 A & NO 9901423 A & NZ 334125 A & EP 929526 A1 & CN 1237963 A & MX 9902796 A1 & KR 2000/048575 A | 1-9,15-16, 23-29,44-47, 116-122, 130-135 |
| X | WO 2003/033472 A1 (Kirin Brewery Co., Ltd.), 24 April, 2003 (24.04.03), Claims; examples & EP 1447405 A1 & AU 2002/343997 A1 & JP 2003-536212 A & US 2005/049264 A1 | 1,7-9,23, 26-29,44-47, 116-122, 130-135 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 March, 2005 (17.03.05) | 05 April, 2005 (05.04.05) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2005/002610

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 2004/018430 A1 (Kirin Brewery Co., Ltd.), 04 March, 2004 (04.03.04), Claims; examples & AU 2003/257666 A1 | 1,7-29, 44-67, 116-122, 130-135 |
| P,X | WO 2004/063365 A1 (Kirin Brewery Co., Ltd.), 29 July, 2004 (29.07.04), Claims; examples (Family: none) | 1,7-29, 44-67, 116-122, 130-135 |
| X | YAGI M. et al., "Selective Inhibition of Platelet-Derived Growth Factor (PDGF) Receptor Autophosphorylation and PDGF-Mediated Cellular Events by a Quinoline Derivative", 1997, Vol.234, pages 285 to 292 | 1,7-9,23, 26-29,44-47 |
| X | JP 01-246263 A (Eli Lilly and Co.), 02 October, 1989 (02.10.89), Claims; examples & EP 326330 A1 & AU 8928728 A & BR 8900356 A & FI 8900423 A & ZA 8900626 A & CN 1034925 A | 1,7-9,23, 26-29,44-47 |
| X | JP 2002-536414 A (Astra Zeneca AB.), 29 October, 2002 (29.10.02), Claims; examples & WO 2000/47212 A1 & AU 2000/24475 A & NO 2001/03882 A & EP 1154774 A1 & BR 2000/08128 A & KR 2001/102044 A | 1,7-9,27-28, 44-47, 116-122, 130-135 |
| X | JP 2000-515114 A (Zenaca Ltd.), 14 November, 2000 (14.11.00), Claims; examples & WO 97/22596 A1 & AU 9711061 A & EP 873319 A1 & BR 9612043 A & CN 1205694 A & US 5962458 A & KR 2000/064455 A | 1,7-9,27-28, 44-47, 116-122, 130-135 |
| X | WO 95/15758 A1 (ROHNE POULENC ROBER PHARMACEUTICAL INC.), 15 June, 1995 (15.06.95), Claims; examples & AU 9513050 A & US 5480883 A & EP 871448 A1 | 1,7-9,27-28, 44-47, 116-122, 130-135 |
| X | WO 95/24190 A2 (SUGEN, INC.), 14 September, 1995 (14.09.95), Claims; examples & AU 9520968 A | 1,7-9,27-28, 44-47, 116-122, 130-135 |

Form PCT/ISA/210 (continuation of second sheet) (January 2004)

**INTERNATIONAL SEARCH REPORT**

International application No.
PCT/JP2005/002610

C (Continuation). DOCUMENTS CONSIDERED TO BE RELEVANT

| Category* | Citation of document, with indication, where appropriate, of the relevant passages | Relevant to claim No. |
|---|---|---|
| X | WO 2001/02409 A1 (VERNALIS RESEARCH LTD.), 11 January, 2001 (11.01.01), Claims; examples & AU 2000/55578 A & EP 1192164 A1 & JP 2003-503504 A | 68-70,72-75, 89,91-93, 116-122, 130-135 |
| X | JP 04-217685 A (BASF AG.), 07 August, 1992 (07.08.92), Claims; examples & EP 447891 A1 & DE 4008726 A & CA 2038521 A | 68-70,72-75, 89,91-93, 116-122, 130-135 |

Form PCT/ISA/210 (continuation of second sheet) (January 2004)

**INTERNATIONAL SEARCH REPORT**

International application No.
PCT/JP2005/002610

**Box No. II Observations where certain claims were found unsearchable (Continuation of item 2 of first sheet)**

This international search report has not been established in respect of certain claims under Article 17(2)(a) for the following reasons:

1. [X] Claims Nos.: 123-129
because they relate to subject matter not required to be searched by this Authority, namely:
Claims 123 to 129 pertain to methods for treatment of the human body by surgery or therapy and diagnostic methods.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200043366 A
- WO 2004018430 A
- WO 9717329 A
- WO 9847873 A
- WO 0050405 A
- WO 0300660 A
- JP 2004045383 A

### Non-patent literature cited in the description

- *International Review of Experimental Pathology,* 1993, vol. 34B, 43-67
- *Proc. Natl. Acad. Sci. USA,* 1995, vol. 92, 2572-2576
- *Laboratory Investigation,* 1995, vol. 74, 991-1003
- *Journal of Biological Chemistry,* 1987, vol. 262, 6443-6446
- *Proc. Natl. Acad. Sci. USA,* 1988, vol. 85, 1105-1108
- *Journal of Biological Chemistry,* 1988, vol. 263, 3039-3045
- *Journal of Biological Chemistry,* 1988, vol. 263, 16999-17005
- *Cancer Research,* 1989, vol. 49, 2553-2553
- *Journal of Biological Chemistry,* 1988, vol. 263, 4586-4592
- *American Journal of Physiology,* 1993, vol. 264, F199-F205
- *New Engl. J. Med.,* 1994, vol. 331, 1286-1292
- *Nature,* 1990, vol. 346, 371-374
- *Journal of the British Thoracic Society,* 1999, vol. 54, 805-812
- *Journal of Immunology,* 1999, vol. 163, 5693-5699
- *Human Gene Therapy,* 2000, vol. 11, 33-42
- *Proc. Natl. Acad. Sci. USA.,* 2000, vol. 97, 8015-20
- *Clinical and Experimental Pharmacology and Physiology,* 1996, vol. 23, 193-200
- *American Journal of Physiology,* 1995, vol. 146, 1140-1149
- *Biochem Biophys Res Commun.,* 1990, vol. 169, 725-729
- *Journal of Cellular Science,* 1995, vol. 108, 985-1002
- *Cornea,* 1997, vol. 16, 177-187
- *An international Journal of gastroenterology & Hepatology,* 1996, vol. 39, 172-175
- *J. Surg. Res.,* 1996, vol. 65, 135-138
- *Journal of Clinical Investigation,* 1993, vol. 92, 2569-76
- *Clinical Cancer Research,* 2001, vol. 7, 2931-2940
- *Cancer Res.,* 1999, vol. 59, 2210-6
- *Journal of Clinical Investigation,* 2002, vol. 109, 1551-1559
- *Journal of Clinical Investigation,* 2002, vol. 109, 1607-1615
- *Experimental Hematology,* 1998, vol. 26, 374-381
- *Org. Synth. Col.,* 1955, vol. 3, 272
- *Acta Chim. Hung.,* 1983, vol. 112, 241
- *J. Am. Chem. Soc.,* 1946, vol. 68, 1299
- *J. Am. Chem. Soc.,* 1946, vol. 68, 1305
- Dai Yuki Kagaku. Asakura Publishing Co., Ltd, 1967, vol. 17, 150
- *J Soc Biol.,* 2002, vol. 196 (1), 53-8
- *J. Boil .Chem.,* 1998, vol. 273, 21145-21152
- *Mol Biol Cell,* 2000, vol. 11 (2), 555-65